# DEMANDE DE BREVET EUROPEEN

(11) **EP 3 904 880 A1**
(43) Date de publication de la demande: **03.11.2021**
(21) Numéro de dépôt: 21173187.2
(22) Date de dépôt: 30.09.2017
(51) Int. Cl.: G01N 33/569, A61P 31/12, A61P 31/18, A61P 43/00, C12N 5/0783, A61P 37/04

(54) **MARQUEURS MEMBRANAIRES**

(30) Priorité: 30.09.2016 FR 1659440; 15.03.2017 FR 1752126
(62) Demande divisionnaire de: 17787640.6
(71) Demandeur: Centre national de la recherche scientifique, 75016 Paris (FR); Université de Montpellier, 34090 Montpellier (FR)
(72) Inventeur: BENKIRANE, Monsef, 34980 Saint Gely du Fesc (FR); PETITJEAN, Gael, 34090 Montpellier (FR); DESCOURS, Benjamin, 34090 Montpellier (FR)
(74) Mandataire: LLR

(57) **Abrégé**

L'invention concerne l'utilisation du marqueur de différentiation CD89 pour la détection de cellules réservoirs d'un virus de l'immunodéficience chez les mammifères.

SEQUENCE LISTING

<110>  Centre National de la Recherche Scientifique

<120>  Marqueurs membranaires

<130>  BR120813

<150>  FR1659440
<151>  2016-09-30

<150>  FR1752126
<151>  2017-03-15

<160>  39

<170>  PatentIn version 3.5

<210>  1
<211>  300
<212>  PRT
<213>  Homo sapiens

<400>  1

Met Val Leu Leu Trp Leu Thr Leu Leu Leu Ile Ala Leu Pro Cys Leu
1               5                   10                  15

Leu Gln Thr Lys Glu Gly Gly Lys Pro Trp Ala Gly Ala Glu Asn Leu
            20                  25                  30

Thr Cys Trp Ile His Asp Val Asp Phe Leu Ser Cys Ser Trp Ala Val
        35                  40                  45

Gly Pro Gly Ala Pro Ala Asp Val Gln Tyr Asp Leu Tyr Leu Asn Val
    50                  55                  60

Ala Asn Arg Arg Gln Gln Tyr Glu Cys Leu His Tyr Lys Thr Asp Ala
65                  70                  75                  80

Gln Gly Thr Arg Ile Gly Cys Arg Phe Asp Asp Ile Ser Arg Leu Ser
                85                  90                  95

Ser Gly Ser Gln Ser Ser His Ile Leu Val Arg Gly Arg Ser Ala Ala
            100                 105                 110

Phe Gly Ile Pro Cys Thr Asp Lys Phe Val Val Phe Ser Gln Ile Glu
        115                 120                 125

Ile Leu Thr Pro Pro Asn Met Thr Ala Lys Cys Asn Lys Thr His Ser
    130                 135                 140

Phe Met His Trp Lys Met Arg Ser His Phe Asn Arg Lys Phe Arg Tyr
145                 150                 155                 160

Glu Leu Gln Ile Gln Lys Arg Met Gln Pro Val Ile Thr Glu Gln Val
                165                 170                 175

Arg Asp Arg Thr Ser Phe Gln Leu Leu Asn Pro Gly Thr Tyr Thr Val
            180                 185                 190

Gln Ile Arg Ala Arg Glu Arg Val Tyr Glu Phe Leu Ser Ala Trp Ser
        195                 200                 205

Thr Pro Gln Arg Phe Glu Cys Asp Gln Glu Glu Gly Ala Asn Thr Arg
    210                 215                 220

Ala Trp Arg Thr Ser Leu Leu Ile Ala Leu Gly Thr Leu Leu Ala Leu
225                 230                 235                 240

Val Cys Val Phe Val Ile Cys Arg Arg Tyr Leu Val Met Gln Arg Leu
                245                 250                 255

Phe Pro Arg Ile Pro His Met Lys Asp Pro Ile Gly Asp Ser Phe Gln
            260                 265                 270

Asn Asp Lys Leu Val Val Trp Glu Ala Gly Lys Ala Gly Leu Glu Glu
        275                 280                 285

Cys Leu Val Thr Glu Val Gln Val Val Gln Lys Thr
    290                 295                 300

<210>  2
<211>  281
<212>  PRT
<213>  Homo sapiens

<400>  2

Met Gly His Pro Pro Leu Leu Pro Leu Leu Leu Leu Leu His Thr Cys
1               5                   10                  15

Val Pro Ala Ser Trp Gly Leu Arg Cys Met Gln Cys Lys Thr Asn Gly
            20                  25                  30

Asp Cys Arg Val Glu Glu Cys Ala Leu Gly Gln Asp Leu Cys Arg Thr
        35                  40                  45

Thr Ile Val Arg Leu Trp Glu Glu Gly Glu Glu Leu Glu Leu Val Glu
    50                  55                  60

Lys Ser Cys Thr His Ser Glu Lys Thr Asn Arg Thr Leu Ser Tyr Arg
65                  70                  75                  80

Thr Gly Leu Lys Ile Thr Ser Leu Thr Glu Val Val Cys Gly Leu Asp
                85                  90                  95

Leu Cys Asn Gln Gly Asn Ser Gly Arg Ala Val Thr Tyr Ser Arg Ser
            100                 105                 110

Arg Tyr Leu Glu Cys Ile Ser Cys Gly Ser Ser Asp Met Ser Cys Glu
        115                 120                 125

Arg Gly Arg His Gln Ser Leu Gln Cys Arg Ser Pro Glu Glu Gln Cys
    130                 135                 140

Leu Asp Val Val Thr His Trp Ile Gln Glu Gly Glu Glu Gly Arg Pro
145                 150                 155                 160

Lys Asp Asp Arg His Leu Arg Gly Cys Gly Tyr Leu Pro Gly Cys Pro
                165                 170                 175

Gly Ser Asn Gly Phe His Asn Asn Asp Thr Phe His Phe Leu Lys Cys
            180                 185                 190

Cys Asn Thr Thr Lys Cys Asn Glu Gly Pro Ile Leu Glu Leu Glu Asn
        195                 200                 205

Leu Pro Gln Asn Gly Arg Gln Cys Tyr Ser Cys Lys Gly Asn Ser Thr
    210                 215                 220

His Gly Cys Ser Ser Glu Glu Thr Phe Leu Ile Asp Cys Arg Gly Pro
225                 230                 235                 240

Met Asn Gln Cys Leu Val Ala Thr Gly Thr His Glu Arg Ser Leu Trp
                245                 250                 255

Gly Ser Trp Leu Pro Cys Lys Ser Thr Thr Ala Leu Arg Pro Pro Cys
            260                 265                 270

Cys Glu Glu Ala Gln Ala Thr His Val
        275                 280

<210>  3
<211>  287
<212>  PRT
<213>  Homo sapiens

<400>  3

Met Asp Pro Lys Gln Thr Thr Leu Leu Cys Leu Val Leu Cys Leu Gly
1               5                   10                  15

Gln Arg Ile Gln Ala Gln Glu Gly Asp Phe Pro Met Pro Phe Ile Ser
            20                  25                  30

Ala Lys Ser Ser Pro Val Ile Pro Leu Asp Gly Ser Val Lys Ile Gln
        35                  40                  45

Cys Gln Ala Ile Arg Glu Ala Tyr Leu Thr Gln Leu Met Ile Ile Lys
    50                  55                  60

Asn Ser Thr Tyr Arg Glu Ile Gly Arg Arg Leu Lys Phe Trp Asn Glu
65                  70                  75                  80

Thr Asp Pro Glu Phe Val Ile Asp His Met Asp Ala Asn Lys Ala Gly
                85                  90                  95

Arg Tyr Gln Cys Gln Tyr Arg Ile Gly His Tyr Arg Phe Arg Tyr Ser
            100                 105                 110

Asp Thr Leu Glu Leu Val Val Thr Gly Leu Tyr Gly Lys Pro Phe Leu
        115                 120                 125

Ser Ala Asp Arg Gly Leu Val Leu Met Pro Gly Glu Asn Ile Ser Leu
    130                 135                 140

Thr Cys Ser Ser Ala His Ile Pro Phe Asp Arg Phe Ser Leu Ala Lys
145                 150                 155                 160

Glu Gly Glu Leu Ser Leu Pro Gln His Gln Ser Gly Glu His Pro Ala
                165                 170                 175

Asn Phe Ser Leu Gly Pro Val Asp Leu Asn Val Ser Gly Ile Tyr Arg
            180                 185                 190

Cys Tyr Gly Trp Tyr Asn Arg Ser Pro Tyr Leu Trp Ser Phe Pro Ser
        195                 200                 205

Asn Ala Leu Glu Leu Val Val Thr Asp Ser Ile His Gln Asp Tyr Thr
    210                 215                 220

Thr Gln Asn Leu Ile Arg Met Ala Val Ala Gly Leu Val Leu Val Ala
225                 230                 235                 240

Leu Leu Ala Ile Leu Val Glu Asn Trp His Ser His Thr Ala Leu Asn
                245                 250                 255

Lys Glu Ala Ser Ala Asp Val Ala Glu Pro Ser Trp Ser Gln Gln Met

260                 265                 270

Cys Gln Pro Gly Leu Thr Phe Ala Arg Thr Pro Ser Val Cys Lys
        275                 280                 285

<210>  4
<211>  504
<212>  PRT
<213>  Homo sapiens

<400>  4

Met Glu Pro Ala Gly Pro Ala Pro Gly Arg Leu Gly Pro Leu Leu Cys
1               5                   10                  15

Leu Leu Leu Ala Ala Ser Cys Ala Trp Ser Gly Val Ala Gly Glu Glu
            20                  25                  30

Glu Leu Gln Val Ile Gln Pro Asp Lys Ser Val Leu Val Ala Ala Gly
        35                  40                  45

Glu Thr Ala Thr Leu Arg Cys Thr Ala Thr Ser Leu Ile Pro Val Gly
    50                  55                  60

Pro Ile Gln Trp Phe Arg Gly Ala Gly Pro Gly Arg Glu Leu Ile Tyr
65                  70                  75                  80

Asn Gln Lys Glu Gly His Phe Pro Arg Val Thr Thr Val Ser Asp Leu
                85                  90                  95

Thr Lys Arg Asn Asn Met Asp Phe Ser Ile Arg Ile Gly Asn Ile Thr
            100                 105                 110

Pro Ala Asp Ala Gly Thr Tyr Tyr Cys Val Lys Phe Arg Lys Gly Ser
        115                 120                 125

Pro Asp Asp Val Glu Phe Lys Ser Gly Ala Gly Thr Glu Leu Ser Val
    130                 135                 140

Arg Ala Lys Pro Ser Ala Pro Val Val Ser Gly Pro Ala Ala Arg Ala
145                 150                 155                 160

Thr Pro Gln His Thr Val Ser Phe Thr Cys Glu Ser His Gly Phe Ser
                165                 170                 175

Pro Arg Asp Ile Thr Leu Lys Trp Phe Lys Asn Gly Asn Glu Leu Ser
            180                 185                 190

Asp Phe Gln Thr Asn Val Asp Pro Val Gly Glu Ser Val Ser Tyr Ser

195                 200                 205

Ile His Ser Thr Ala Lys Val Val Leu Thr Arg Glu Asp Val His Ser
    210                 215                 220

Gln Val Ile Cys Glu Val Ala His Val Thr Leu Gln Gly Asp Pro Leu
225                 230                 235                 240

Arg Gly Thr Ala Asn Leu Ser Glu Thr Ile Arg Val Pro Pro Thr Leu
                245                 250                 255

Glu Val Thr Gln Gln Pro Val Arg Ala Glu Asn Gln Val Asn Val Thr
            260                 265                 270

Cys Gln Val Arg Lys Phe Tyr Pro Gln Arg Leu Gln Leu Thr Trp Leu
        275                 280                 285

Glu Asn Gly Asn Val Ser Arg Thr Glu Thr Ala Ser Thr Val Thr Glu
    290                 295                 300

Asn Lys Asp Gly Thr Tyr Asn Trp Met Ser Trp Leu Leu Val Asn Val
305                 310                 315                 320

Ser Ala His Arg Asp Asp Val Lys Leu Thr Cys Gln Val Glu His Asp
                325                 330                 335

Gly Gln Pro Ala Val Ser Lys Ser His Asp Leu Lys Val Ser Ala His
            340                 345                 350

Pro Lys Glu Gln Gly Ser Asn Thr Ala Ala Glu Asn Thr Gly Ser Asn
        355                 360                 365

Glu Arg Asn Ile Tyr Ile Val Val Gly Val Val Cys Thr Leu Leu Val
    370                 375                 380

Ala Leu Leu Met Ala Ala Leu Tyr Leu Val Arg Ile Arg Gln Lys Lys
385                 390                 395                 400

Ala Gln Gly Ser Thr Ser Ser Thr Arg Leu His Glu Pro Glu Lys Asn
                405                 410                 415

Ala Arg Glu Ile Thr Gln Asp Thr Asn Asp Ile Thr Tyr Ala Asp Leu
            420                 425                 430

Asn Leu Pro Lys Gly Lys Lys Pro Ala Pro Gln Ala Ala Glu Pro Asn
        435                 440                 445

Asn His Thr Glu Tyr Ala Ser Ile Gln Thr Ser Pro Gln Pro Ala Ser
    450                 455                 460

Glu Asp Thr Leu Thr Tyr Ala Asp Leu Asp Met Val His Leu Asn Arg
465                 470                 475                 480

Thr Pro Lys Gln Pro Ala Pro Lys Pro Glu Pro Ser Phe Ser Glu Tyr
                485                 490                 495

Ala Ser Val Gln Val Pro Arg Lys
            500

<210>  5
<211>  538
<212>  PRT
<213>  Homo sapiens

<400>  5

Met Ala Arg Ala Ala Ala Leu Leu Pro Ser Arg Ser Pro Pro Thr Pro
1               5                   10                  15

Leu Leu Trp Pro Leu Leu Leu Leu Leu Leu Leu Glu Thr Gly Ala Gln
            20                  25                  30

Asp Val Arg Val Gln Val Leu Pro Glu Val Arg Gly Gln Leu Gly Gly
        35                  40                  45

Thr Val Glu Leu Pro Cys His Leu Leu Pro Pro Val Pro Gly Leu Tyr
    50                  55                  60

Ile Ser Leu Val Thr Trp Gln Arg Pro Asp Ala Pro Ala Asn His Gln
65                  70                  75                  80

Asn Val Ala Ala Phe His Pro Lys Met Gly Pro Ser Phe Pro Ser Pro
                85                  90                  95

Lys Pro Gly Ser Glu Arg Leu Ser Phe Val Ser Ala Lys Gln Ser Thr
            100                 105                 110

Gly Gln Asp Thr Glu Ala Glu Leu Gln Asp Ala Thr Leu Ala Leu His
        115                 120                 125

Gly Leu Thr Val Glu Asp Glu Gly Asn Tyr Thr Cys Glu Phe Ala Thr
    130                 135                 140

Phe Pro Lys Gly Ser Val Arg Gly Met Thr Trp Leu Arg Val Ile Ala
145                 150                 155                 160

Lys Pro Lys Asn Gln Ala Glu Ala Gln Lys Val Thr Phe Ser Gln Asp
                165                 170                 175

Pro Thr Thr Val Ala Leu Cys Ile Ser Lys Glu Gly Arg Pro Pro Ala
            180                 185                 190

Arg Ile Ser Trp Leu Ser Ser Leu Asp Trp Glu Ala Lys Glu Thr Gln
        195                 200                 205

Val Ser Gly Thr Leu Ala Gly Thr Val Thr Val Thr Ser Arg Phe Thr
    210                 215                 220

Leu Val Pro Ser Gly Arg Ala Asp Gly Val Thr Val Thr Cys Lys Val
225                 230                 235                 240

Glu His Glu Ser Phe Glu Glu Pro Ala Leu Ile Pro Val Thr Leu Ser
                245                 250                 255

Val Arg Tyr Pro Pro Glu Val Ser Ile Ser Gly Tyr Asp Asp Asn Trp
            260                 265                 270

Tyr Leu Gly Arg Thr Asp Ala Thr Leu Ser Cys Asp Val Arg Ser Asn
        275                 280                 285

Pro Glu Pro Thr Gly Tyr Asp Trp Ser Thr Thr Ser Gly Thr Phe Pro
    290                 295                 300

Thr Ser Ala Val Ala Gln Gly Ser Gln Leu Val Ile His Ala Val Asp
305                 310                 315                 320

Ser Leu Phe Asn Thr Thr Phe Val Cys Thr Val Thr Asn Ala Val Gly
                325                 330                 335

Met Gly Arg Ala Glu Gln Val Ile Phe Val Arg Glu Thr Pro Asn Thr
            340                 345                 350

Ala Gly Ala Gly Ala Thr Gly Gly Ile Ile Gly Gly Ile Ile Ala Ala
        355                 360                 365

Ile Ile Ala Thr Ala Val Ala Ala Thr Gly Ile Leu Ile Cys Arg Gln
    370                 375                 380

Gln Arg Lys Glu Gln Thr Leu Gln Gly Ala Glu Glu Asp Glu Asp Leu
385                 390                 395                 400

Glu Gly Pro Pro Ser Tyr Lys Pro Pro Thr Pro Lys Ala Lys Leu Glu
                405                 410                 415

Ala Gln Glu Met Pro Ser Gln Leu Phe Thr Leu Gly Ala Ser Glu His
            420                 425                 430

Ser Pro Leu Lys Thr Pro Tyr Phe Asp Ala Gly Ala Ser Cys Thr Glu
        435                 440                 445

Gln Glu Met Pro Arg Tyr His Glu Leu Pro Thr Leu Glu Glu Arg Ser
    450                 455                 460

Gly Pro Leu His Pro Gly Ala Thr Ser Leu Gly Ser Pro Ile Pro Val
465                 470                 475                 480

Pro Pro Gly Pro Pro Ala Val Glu Asp Val Ser Leu Asp Leu Glu Asp
                485                 490                 495

Glu Glu Gly Glu Glu Glu Glu Glu Tyr Leu Asp Lys Ile Asn Pro Ile
            500                 505                 510

Tyr Asp Ala Leu Ser Tyr Ser Ser Pro Ser Asp Ser Tyr Gln Gly Lys
        515                 520                 525

Gly Phe Val Met Ser Arg Ala Met Tyr Val
    530                 535

<210> 6
<211> 427
<212> PRT
<213> Homo sapiens

<400> 6

Met Glu Trp Pro Ala Arg Leu Cys Gly Leu Trp Ala Leu Leu Leu Cys
1               5                   10                  15

Ala Gly Gly Gly Gly Gly Gly Gly Gly Ala Ala Pro Thr Glu Thr Gln
            20                  25                  30

Pro Pro Val Thr Asn Leu Ser Val Ser Val Glu Asn Leu Cys Thr Val
        35                  40                  45

Ile Trp Thr Trp Asn Pro Pro Glu Gly Ala Ser Ser Asn Cys Ser Leu
    50                  55                  60

Trp Tyr Phe Ser His Phe Gly Asp Lys Gln Asp Lys Lys Ile Ala Pro
65                  70                  75                  80

Glu Thr Arg Arg Ser Ile Glu Val Pro Leu Asn Glu Arg Ile Cys Leu
                85                  90                  95

Gln Val Gly Ser Gln Cys Ser Thr Asn Glu Ser Glu Lys Pro Ser Ile
            100                 105                 110

Leu Val Glu Lys Cys Ile Ser Pro Pro Glu Gly Asp Pro Glu Ser Ala
        115                 120                 125

Val Thr Glu Leu Gln Cys Ile Trp His Asn Leu Ser Tyr Met Lys Cys
    130                 135                 140

Ser Trp Leu Pro Gly Arg Asn Thr Ser Pro Asp Thr Asn Tyr Thr Leu
145                 150                 155                 160

Tyr Tyr Trp His Arg Ser Leu Glu Lys Ile His Gln Cys Glu Asn Ile
                165                 170                 175

Phe Arg Glu Gly Gln Tyr Phe Gly Cys Ser Phe Asp Leu Thr Lys Val
            180                 185                 190

Lys Asp Ser Ser Phe Glu Gln His Ser Val Gln Ile Met Val Lys Asp
        195                 200                 205

Asn Ala Gly Lys Ile Lys Pro Ser Phe Asn Ile Val Pro Leu Thr Ser
    210                 215                 220

Arg Val Lys Pro Asp Pro Pro His Ile Lys Asn Leu Ser Phe His Asn
225                 230                 235                 240

Asp Asp Leu Tyr Val Gln Trp Glu Asn Pro Gln Asn Phe Ile Ser Arg
                245                 250                 255

Cys Leu Phe Tyr Glu Val Glu Val Asn Asn Ser Gln Thr Glu Thr His
            260                 265                 270

Asn Val Phe Tyr Val Gln Glu Ala Lys Cys Glu Asn Pro Glu Phe Glu
        275                 280                 285

Arg Asn Val Glu Asn Thr Ser Cys Phe Met Val Pro Gly Val Leu Pro
    290                 295                 300

Asp Thr Leu Asn Thr Val Arg Ile Arg Val Lys Thr Asn Lys Leu Cys
305                 310                 315                 320

Tyr Glu Asp Asp Lys Leu Trp Ser Asn Trp Ser Gln Glu Met Ser Ile
                325                 330                 335

Gly Lys Lys Arg Asn Ser Thr Leu Tyr Ile Thr Met Leu Leu Ile Val
            340                 345                 350

Pro Val Ile Val Ala Gly Ala Ile Ile Val Leu Leu Leu Tyr Leu Lys
        355                 360                 365

Arg Leu Lys Ile Ile Ile Phe Pro Pro Ile Pro Asp Pro Gly Lys Ile
    370                 375                 380

Phe Lys Glu Met Phe Gly Asp Gln Asn Asp Asp Thr Leu His Trp Lys
385                 390                 395                 400

Lys Tyr Asp Ile Tyr Glu Lys Gln Thr Lys Glu Glu Thr Asp Ser Val
                405                 410                 415

Val Leu Ile Glu Asn Leu Lys Lys Ala Ser Gln
            420                 425

<210>  7
<211>  461
<212>  PRT
<213>  Homo sapiens

<400>  7

Met Ala Pro Val Ala Val Trp Ala Ala Leu Ala Val Gly Leu Glu Leu
1               5                   10                  15

Trp Ala Ala Ala His Ala Leu Pro Ala Gln Val Ala Phe Thr Pro Tyr
            20                  25                  30

Ala Pro Glu Pro Gly Ser Thr Cys Arg Leu Arg Glu Tyr Tyr Asp Gln
        35                  40                  45

Thr Ala Gln Met Cys Cys Ser Lys Cys Ser Pro Gly Gln His Ala Lys
    50                  55                  60

Val Phe Cys Thr Lys Thr Ser Asp Thr Val Cys Asp Ser Cys Glu Asp
65                  70                  75                  80

Ser Thr Tyr Thr Gln Leu Trp Asn Trp Val Pro Glu Cys Leu Ser Cys
                85                  90                  95

Gly Ser Arg Cys Ser Ser Asp Gln Val Glu Thr Gln Ala Cys Thr Arg
            100                 105                 110

Glu Gln Asn Arg Ile Cys Thr Cys Arg Pro Gly Trp Tyr Cys Ala Leu
        115                 120                 125

Ser Lys Gln Glu Gly Cys Arg Leu Cys Ala Pro Leu Arg Lys Cys Arg
    130                 135                 140

Pro Gly Phe Gly Val Ala Arg Pro Gly Thr Glu Thr Ser Asp Val Val
145                 150                 155                 160

Cys Lys Pro Cys Ala Pro Gly Thr Phe Ser Asn Thr Thr Ser Ser Thr
                165                 170                 175

Asp Ile Cys Arg Pro His Gln Ile Cys Asn Val Val Ala Ile Pro Gly
            180                 185                 190

Asn Ala Ser Met Asp Ala Val Cys Thr Ser Thr Ser Pro Thr Arg Ser
        195                 200                 205

Met Ala Pro Gly Ala Val His Leu Pro Gln Pro Val Ser Thr Arg Ser
    210                 215                 220

Gln His Thr Gln Pro Thr Pro Glu Pro Ser Thr Ala Pro Ser Thr Ser
225                 230                 235                 240

Phe Leu Leu Pro Met Gly Pro Ser Pro Pro Ala Glu Gly Ser Thr Gly
                245                 250                 255

Asp Phe Ala Leu Pro Val Gly Leu Ile Val Gly Val Thr Ala Leu Gly
            260                 265                 270

Leu Leu Ile Ile Gly Val Val Asn Cys Val Ile Met Thr Gln Val Lys
        275                 280                 285

Lys Lys Pro Leu Cys Leu Gln Arg Glu Ala Lys Val Pro His Leu Pro
    290                 295                 300

Ala Asp Lys Ala Arg Gly Thr Gln Gly Pro Glu Gln Gln His Leu Leu
305                 310                 315                 320

Ile Thr Ala Pro Ser Ser Ser Ser Ser Ser Leu Glu Ser Ser Ala Ser
                325                 330                 335

Ala Leu Asp Arg Arg Ala Pro Thr Arg Asn Gln Pro Gln Ala Pro Gly
            340                 345                 350

Val Glu Ala Ser Gly Ala Gly Glu Ala Arg Ala Ser Thr Gly Ser Ser
        355                 360                 365

Asp Ser Ser Pro Gly Gly His Gly Thr Gln Val Asn Val Thr Cys Ile
    370                 375                 380

Val Asn Val Cys Ser Ser Ser Asp His Ser Ser Gln Cys Ser Ser Gln

385                 390                 395                 400

Ala Ser Ser Thr Met Gly Asp Thr Asp Ser Ser Pro Ser Glu Ser Pro
                405                 410                 415

Lys Asp Glu Gln Val Pro Phe Ser Lys Glu Glu Cys Ala Phe Arg Ser
            420                 425                 430

Gln Leu Glu Thr Pro Glu Thr Leu Leu Gly Ser Thr Glu Glu Lys Pro
        435                 440                 445

Leu Pro Leu Gly Val Pro Asp Ala Gly Met Lys Pro Ser
    450                 455                 460

<210>  8
<211>  569
<212>  PRT
<213>  Homo sapiens

<400>  8

Met Lys Val Leu Leu Arg Leu Ile Cys Phe Ile Ala Leu Leu Ile Ser
1               5                   10                  15

Ser Leu Glu Ala Asp Lys Cys Lys Glu Arg Glu Glu Lys Ile Ile Leu
            20                  25                  30

Val Ser Ser Ala Asn Glu Ile Asp Val Arg Pro Cys Pro Leu Asn Pro
        35                  40                  45

Asn Glu His Lys Gly Thr Ile Thr Trp Tyr Lys Asp Asp Ser Lys Thr
    50                  55                  60

Pro Val Ser Thr Glu Gln Ala Ser Arg Ile His Gln His Lys Glu Lys
65                  70                  75                  80

Leu Trp Phe Val Pro Ala Lys Val Glu Asp Ser Gly His Tyr Tyr Cys
                85                  90                  95

Val Val Arg Asn Ser Ser Tyr Cys Leu Arg Ile Lys Ile Ser Ala Lys
            100                 105                 110

Phe Val Glu Asn Glu Pro Asn Leu Cys Tyr Asn Ala Gln Ala Ile Phe
        115                 120                 125

Lys Gln Lys Leu Pro Val Ala Gly Asp Gly Gly Leu Val Cys Pro Tyr
    130                 135                 140

Met Glu Phe Phe Lys Asn Glu Asn Asn Glu Leu Pro Lys Leu Gln Trp

145                 150                 155                 160

Tyr Lys Asp Cys Lys Pro Leu Leu Leu Asp Asn Ile His Phe Ser Gly
                165                 170                 175

Val Lys Asp Arg Leu Ile Val Met Asn Val Ala Glu Lys His Arg Gly
            180                 185                 190

Asn Tyr Thr Cys His Ala Ser Tyr Thr Tyr Leu Gly Lys Gln Tyr Pro
        195                 200                 205

Ile Thr Arg Val Ile Glu Phe Ile Thr Leu Glu Glu Asn Lys Pro Thr
    210                 215                 220

Arg Pro Val Ile Val Ser Pro Ala Asn Glu Thr Met Glu Val Asp Leu
225                 230                 235                 240

Gly Ser Gln Ile Gln Leu Ile Cys Asn Val Thr Gly Gln Leu Ser Asp
                245                 250                 255

Ile Ala Tyr Trp Lys Trp Asn Gly Ser Val Ile Asp Glu Asp Asp Pro
            260                 265                 270

Val Leu Gly Glu Asp Tyr Tyr Ser Val Glu Asn Pro Ala Asn Lys Arg
        275                 280                 285

Arg Ser Thr Leu Ile Thr Val Leu Asn Ile Ser Glu Ile Glu Ser Arg
    290                 295                 300

Phe Tyr Lys His Pro Phe Thr Cys Phe Ala Lys Asn Thr His Gly Ile
305                 310                 315                 320

Asp Ala Ala Tyr Ile Gln Leu Ile Tyr Pro Val Thr Asn Phe Gln Lys
                325                 330                 335

His Met Ile Gly Ile Cys Val Thr Leu Thr Val Ile Ile Val Cys Ser
            340                 345                 350

Val Phe Ile Tyr Lys Ile Phe Lys Ile Asp Ile Val Leu Trp Tyr Arg
        355                 360                 365

Asp Ser Cys Tyr Asp Phe Leu Pro Ile Lys Ala Ser Asp Gly Lys Thr
    370                 375                 380

Tyr Asp Ala Tyr Ile Leu Tyr Pro Lys Thr Val Gly Glu Gly Ser Thr
385                 390                 395                 400

Ser Asp Cys Asp Ile Phe Val Phe Lys Val Leu Pro Glu Val Leu Glu
                405                 410                 415

Lys Gln Cys Gly Tyr Lys Leu Phe Ile Tyr Gly Arg Asp Asp Tyr Val
            420                 425                 430

Gly Glu Asp Ile Val Glu Val Ile Asn Glu Asn Val Lys Lys Ser Arg
        435                 440                 445

Arg Leu Ile Ile Ile Leu Val Arg Glu Thr Ser Gly Phe Ser Trp Leu
    450                 455                 460

Gly Gly Ser Ser Glu Glu Gln Ile Ala Met Tyr Asn Ala Leu Val Gln
465                 470                 475                 480

Asp Gly Ile Lys Val Val Leu Leu Glu Leu Glu Lys Ile Gln Asp Tyr
                485                 490                 495

Glu Lys Met Pro Glu Ser Ile Lys Phe Ile Lys Gln Lys His Gly Ala
            500                 505                 510

Ile Arg Trp Ser Gly Asp Phe Thr Gln Gly Pro Gln Ser Ala Lys Thr
        515                 520                 525

Arg Phe Trp Lys Asn Val Arg Tyr His Met Pro Val Gln Arg Arg Ser
    530                 535                 540

Pro Ser Ser Lys His Gln Leu Leu Ser Pro Ala Thr Lys Glu Lys Leu
545                 550                 555                 560

Gln Arg Glu Ala His Val Pro Leu Gly
                565

<210>  9
<211>  532
<212>  PRT
<213>  Homo sapiens

<400>  9

Met Ala Pro Ser Ser Pro Arg Pro Ala Leu Pro Ala Leu Leu Val Leu
1               5                   10                  15

Leu Gly Ala Leu Phe Pro Gly Pro Gly Asn Ala Gln Thr Ser Val Ser
            20                  25                  30

Pro Ser Lys Val Ile Leu Pro Arg Gly Gly Ser Val Leu Val Thr Cys
        35                  40                  45

Ser Thr Ser Cys Asp Gln Pro Lys Leu Leu Gly Ile Glu Thr Pro Leu
    50                  55                  60

Pro Lys Lys Glu Leu Leu Leu Pro Gly Asn Asn Arg Lys Val Tyr Glu
65                  70                  75                  80

Leu Ser Asn Val Gln Glu Asp Ser Gln Pro Met Cys Tyr Ser Asn Cys
                85                  90                  95

Pro Asp Gly Gln Ser Thr Ala Lys Thr Phe Leu Thr Val Tyr Trp Thr
            100                 105                 110

Pro Glu Arg Val Glu Leu Ala Pro Leu Pro Ser Trp Gln Pro Val Gly
        115                 120                 125

Lys Asn Leu Thr Leu Arg Cys Gln Val Glu Gly Gly Ala Pro Arg Ala
    130                 135                 140

Asn Leu Thr Val Val Leu Leu Arg Gly Glu Lys Glu Leu Lys Arg Glu
145                 150                 155                 160

Pro Ala Val Gly Glu Pro Ala Glu Val Thr Thr Thr Val Leu Val Arg
                165                 170                 175

Arg Asp His His Gly Ala Asn Phe Ser Cys Arg Thr Glu Leu Asp Leu
            180                 185                 190

Arg Pro Gln Gly Leu Glu Leu Phe Glu Asn Thr Ser Ala Pro Tyr Gln
        195                 200                 205

Leu Gln Thr Phe Val Leu Pro Ala Thr Pro Pro Gln Leu Val Ser Pro
    210                 215                 220

Arg Val Leu Glu Val Asp Thr Gln Gly Thr Val Val Cys Ser Leu Asp
225                 230                 235                 240

Gly Leu Phe Pro Val Ser Glu Ala Gln Val His Leu Ala Leu Gly Asp
                245                 250                 255

Gln Arg Leu Asn Pro Thr Val Thr Tyr Gly Asn Asp Ser Phe Ser Ala
            260                 265                 270

Lys Ala Ser Val Ser Val Thr Ala Glu Asp Glu Gly Thr Gln Arg Leu
        275                 280                 285

Thr Cys Ala Val Ile Leu Gly Asn Gln Ser Gln Glu Thr Leu Gln Thr
    290                 295                 300

Val Thr Ile Tyr Ser Phe Pro Ala Pro Asn Val Ile Leu Thr Lys Pro
305                 310                 315                 320

Glu Val Ser Glu Gly Thr Glu Val Thr Val Lys Cys Glu Ala His Pro
                325                 330                 335

Arg Ala Lys Val Thr Leu Asn Gly Val Pro Ala Gln Pro Leu Gly Pro
            340                 345                 350

Arg Ala Gln Leu Leu Leu Lys Ala Thr Pro Glu Asp Asn Gly Arg Ser
        355                 360                 365

Phe Ser Cys Ser Ala Thr Leu Glu Val Ala Gly Gln Leu Ile His Lys
    370                 375                 380

Asn Gln Thr Arg Glu Leu Arg Val Leu Tyr Gly Pro Arg Leu Asp Glu
385                 390                 395                 400

Arg Asp Cys Pro Gly Asn Trp Thr Trp Pro Glu Asn Ser Gln Gln Thr
                405                 410                 415

Pro Met Cys Gln Ala Trp Gly Asn Pro Leu Pro Glu Leu Lys Cys Leu
            420                 425                 430

Lys Asp Gly Thr Phe Pro Leu Pro Ile Gly Glu Ser Val Thr Val Thr
        435                 440                 445

Arg Asp Leu Glu Gly Thr Tyr Leu Cys Arg Ala Arg Ser Thr Gln Gly
    450                 455                 460

Glu Val Thr Arg Lys Val Thr Val Asn Val Leu Ser Pro Arg Tyr Glu
465                 470                 475                 480

Ile Val Ile Ile Thr Val Val Ala Ala Ala Val Ile Met Gly Thr Ala
                485                 490                 495

Gly Leu Ser Thr Tyr Leu Tyr Asn Arg Gln Arg Lys Ile Lys Lys Tyr
            500                 505                 510

Arg Leu Gln Gln Ala Gln Lys Gly Thr Pro Met Lys Pro Asn Thr Gln
        515                 520                 525

Ala Thr Pro Pro
    530

<210>  10
<211>  1163

<212>  PRT
<213>  Homo sapiens

<400>  10

Met Thr Arg Thr Arg Ala Ala Leu Leu Leu Phe Thr Ala Leu Ala Thr
1               5                   10                  15

Ser Leu Gly Phe Asn Leu Asp Thr Glu Glu Leu Thr Ala Phe Arg Val
            20                  25                  30

Asp Ser Ala Gly Phe Gly Asp Ser Val Val Gln Tyr Ala Asn Ser Trp
        35                  40                  45

Val Val Val Gly Ala Pro Gln Lys Ile Thr Ala Ala Asn Gln Thr Gly
    50                  55                  60

Gly Leu Tyr Gln Cys Gly Tyr Ser Thr Gly Ala Cys Glu Pro Ile Gly
65                  70                  75                  80

Leu Gln Val Pro Pro Glu Ala Val Asn Met Ser Leu Gly Leu Ser Leu
                85                  90                  95

Ala Ser Thr Thr Ser Pro Ser Gln Leu Leu Ala Cys Gly Pro Thr Val
            100                 105                 110

His His Glu Cys Gly Arg Asn Met Tyr Leu Thr Gly Leu Cys Phe Leu
        115                 120                 125

Leu Gly Pro Thr Gln Leu Thr Gln Arg Leu Pro Val Ser Arg Gln Glu
    130                 135                 140

Cys Pro Arg Gln Glu Gln Asp Ile Val Phe Leu Ile Asp Gly Ser Gly
145                 150                 155                 160

Ser Ile Ser Ser Arg Asn Phe Ala Thr Met Met Asn Phe Val Arg Ala
                165                 170                 175

Val Ile Ser Gln Phe Gln Arg Pro Ser Thr Gln Phe Ser Leu Met Gln
            180                 185                 190

Phe Ser Asn Lys Phe Gln Thr His Phe Thr Phe Glu Glu Phe Arg Arg
        195                 200                 205

Ser Ser Asn Pro Leu Ser Leu Leu Ala Ser Val His Gln Leu Gln Gly
    210                 215                 220

Phe Thr Tyr Thr Ala Thr Ala Ile Gln Asn Val Val His Arg Leu Phe
225                 230                 235                 240

His Ala Ser Tyr Gly Ala Arg Arg Asp Ala Ala Lys Ile Leu Ile Val
                245                 250                 255

Ile Thr Asp Gly Lys Lys Glu Gly Asp Ser Leu Asp Tyr Lys Asp Val
            260                 265                 270

Ile Pro Met Ala Asp Ala Ala Gly Ile Ile Arg Tyr Ala Ile Gly Val
        275                 280                 285

Gly Leu Ala Phe Gln Asn Arg Asn Ser Trp Lys Glu Leu Asn Asp Ile
    290                 295                 300

Ala Ser Lys Pro Ser Gln Glu His Ile Phe Lys Val Glu Asp Phe Asp
305                 310                 315                 320

Ala Leu Lys Asp Ile Gln Asn Gln Leu Lys Glu Lys Ile Phe Ala Ile
                325                 330                 335

Glu Gly Thr Glu Thr Thr Ser Ser Ser Ser Phe Glu Leu Glu Met Ala
            340                 345                 350

Gln Glu Gly Phe Ser Ala Val Phe Thr Pro Asp Gly Pro Val Leu Gly
        355                 360                 365

Ala Val Gly Ser Phe Thr Trp Ser Gly Gly Ala Phe Leu Tyr Pro Pro
    370                 375                 380

Asn Met Ser Pro Thr Phe Ile Asn Met Ser Gln Glu Asn Val Asp Met
385                 390                 395                 400

Arg Asp Ser Tyr Leu Gly Tyr Ser Thr Glu Leu Ala Leu Trp Lys Gly
                405                 410                 415

Val Gln Ser Leu Val Leu Gly Ala Pro Arg Tyr Gln His Thr Gly Lys
            420                 425                 430

Ala Val Ile Phe Thr Gln Val Ser Arg Gln Trp Arg Met Lys Ala Glu
        435                 440                 445

Val Thr Gly Thr Gln Ile Gly Ser Tyr Phe Gly Ala Ser Leu Cys Ser
    450                 455                 460

Val Asp Val Asp Ser Asp Gly Ser Thr Asp Leu Val Leu Ile Gly Ala
465                 470                 475                 480

Pro His Tyr Tyr Glu Gln Thr Arg Gly Gly Gln Val Ser Val Cys Pro

                485                 490                 495

Leu Pro Arg Gly Trp Arg Arg Trp Trp Cys Asp Ala Val Leu Tyr Gly
            500                 505                 510

Glu Gln Gly His Pro Trp Gly Arg Phe Gly Ala Ala Leu Thr Val Leu
        515                 520                 525

Gly Asp Val Asn Gly Asp Lys Leu Thr Asp Val Val Ile Gly Ala Pro
    530                 535                 540

Gly Glu Glu Glu Asn Arg Gly Ala Val Tyr Leu Phe His Gly Val Leu
545                 550                 555                 560

Gly Pro Ser Ile Ser Pro Ser His Ser Gln Arg Ile Ala Gly Ser Gln
                565                 570                 575

Leu Ser Ser Arg Leu Gln Tyr Phe Gly Gln Ala Leu Ser Gly Gly Gln
            580                 585                 590

Asp Leu Thr Gln Asp Gly Leu Val Asp Leu Ala Val Gly Ala Arg Gly
        595                 600                 605

Gln Val Leu Leu Leu Arg Thr Arg Pro Val Leu Trp Val Gly Val Ser
    610                 615                 620

Met Gln Phe Ile Pro Ala Glu Ile Pro Arg Ser Ala Phe Glu Cys Arg
625                 630                 635                 640

Glu Gln Val Val Ser Glu Gln Thr Leu Val Gln Ser Asn Ile Cys Leu
                645                 650                 655

Tyr Ile Asp Lys Arg Ser Lys Asn Leu Leu Gly Ser Arg Asp Leu Gln
            660                 665                 670

Ser Ser Val Thr Leu Asp Leu Ala Leu Asp Pro Gly Arg Leu Ser Pro
        675                 680                 685

Arg Ala Thr Phe Gln Glu Thr Lys Asn Arg Ser Leu Ser Arg Val Arg
    690                 695                 700

Val Leu Gly Leu Lys Ala His Cys Glu Asn Phe Asn Leu Leu Leu Pro
705                 710                 715                 720

Ser Cys Val Glu Asp Ser Val Thr Pro Ile Thr Leu Arg Leu Asn Phe
                725                 730                 735

Thr Leu Val Gly Lys Pro Leu Leu Ala Phe Arg Asn Leu Arg Pro Met
            740                 745                 750

Leu Ala Ala Asp Ala Gln Arg Tyr Phe Thr Ala Ser Leu Pro Phe Glu
        755                 760                 765

Lys Asn Cys Gly Ala Asp His Ile Cys Gln Asp Asn Leu Gly Ile Ser
    770                 775                 780

Phe Ser Phe Pro Gly Leu Lys Ser Leu Leu Val Gly Ser Asn Leu Glu
785                 790                 795                 800

Leu Asn Ala Glu Val Met Val Trp Asn Asp Gly Glu Asp Ser Tyr Gly
                805                 810                 815

Thr Thr Ile Thr Phe Ser His Pro Ala Gly Leu Ser Tyr Arg Tyr Val
            820                 825                 830

Ala Glu Gly Gln Lys Gln Gly Gln Leu Arg Ser Leu His Leu Thr Cys
        835                 840                 845

Asp Ser Ala Pro Val Gly Ser Gln Gly Thr Trp Ser Thr Ser Cys Arg
    850                 855                 860

Ile Asn His Leu Ile Phe Arg Gly Gly Ala Gln Ile Thr Phe Leu Ala
865                 870                 875                 880

Thr Phe Asp Val Ser Pro Lys Ala Val Leu Gly Asp Arg Leu Leu Leu
                885                 890                 895

Thr Ala Asn Val Ser Ser Glu Asn Asn Thr Pro Arg Thr Ser Lys Thr
            900                 905                 910

Thr Phe Gln Leu Glu Leu Pro Val Lys Tyr Ala Val Tyr Thr Val Val
        915                 920                 925

Ser Ser His Glu Gln Phe Thr Lys Tyr Leu Asn Phe Ser Glu Ser Glu
    930                 935                 940

Glu Lys Glu Ser His Val Ala Met His Arg Tyr Gln Val Asn Asn Leu
945                 950                 955                 960

Gly Gln Arg Asp Leu Pro Val Ser Ile Asn Phe Trp Val Pro Val Glu
                965                 970                 975

Leu Asn Gln Glu Ala Val Trp Met Asp Val Glu Val Ser His Pro Gln
            980                 985                 990

Asn Pro Ser Leu Arg Cys Ser Ser Glu Lys Ile Ala Pro Pro Ala Ser
        995                 1000                1005

Asp Phe Leu Ala His Ile Gln Lys Asn Pro Val Leu Asp Cys Ser
    1010                1015                1020

Ile Ala Gly Cys Leu Arg Phe Arg Cys Asp Val Pro Ser Phe Ser
    1025                1030                1035

Val Gln Glu Glu Leu Asp Phe Thr Leu Lys Gly Asn Leu Ser Phe
    1040                1045                1050

Gly Trp Val Arg Gln Ile Leu Gln Lys Lys Val Ser Val Val Ser
    1055                1060                1065

Val Ala Glu Ile Thr Phe Asp Thr Ser Val Tyr Ser Gln Leu Pro
    1070                1075                1080

Gly Gln Glu Ala Phe Met Arg Ala Gln Thr Thr Thr Val Leu Glu
    1085                1090                1095

Lys Tyr Lys Val His Asn Pro Thr Pro Leu Ile Val Gly Ser Ser
    1100                1105                1110

Ile Gly Gly Leu Leu Leu Leu Ala Leu Ile Thr Ala Val Leu Tyr
    1115                1120                1125

Lys Val Gly Phe Phe Lys Arg Gln Tyr Lys Glu Met Met Glu Glu
    1130                1135                1140

Ala Asn Gly Gln Ile Ala Pro Glu Asn Gly Thr Gln Thr Pro Ser
    1145                1150                1155

Pro Pro Ser Glu Lys
    1160

<210> 11
<211> 351
<212> PRT
<213> Homo sapiens

<400> 11

Met Glu Thr Asn Phe Ser Thr Pro Leu Asn Glu Tyr Glu Glu Val Ser
1               5                   10                  15

Tyr Glu Ser Ala Gly Tyr Thr Val Leu Arg Ile Leu Pro Leu Val Val
            20                  25                  30

Leu Gly Val Thr Phe Val Leu Gly Val Leu Gly Asn Gly Leu Val Ile
        35                  40                  45

Trp Val Ala Gly Phe Arg Met Thr Arg Thr Val Thr Thr Ile Cys Tyr
    50                  55                  60

Leu Asn Leu Ala Leu Ala Asp Phe Ser Phe Thr Ala Thr Leu Pro Phe
65                  70                  75                  80

Leu Ile Val Ser Met Ala Met Gly Glu Lys Trp Pro Phe Gly Trp Phe
                85                  90                  95

Leu Cys Lys Leu Ile His Ile Val Val Asp Ile Asn Leu Phe Gly Ser
            100                 105                 110

Val Phe Leu Ile Gly Phe Ile Ala Leu Asp Arg Cys Ile Cys Val Leu
        115                 120                 125

His Pro Val Trp Ala Gln Asn His Arg Thr Val Ser Leu Ala Met Lys
    130                 135                 140

Val Ile Val Gly Pro Trp Ile Leu Ala Leu Val Leu Thr Leu Pro Val
145                 150                 155                 160

Phe Leu Phe Leu Thr Thr Val Thr Ile Pro Asn Gly Asp Thr Tyr Cys
                165                 170                 175

Thr Phe Asn Phe Ala Ser Trp Gly Gly Thr Pro Glu Glu Arg Leu Lys
            180                 185                 190

Val Ala Ile Thr Met Leu Thr Ala Arg Gly Ile Ile Arg Phe Val Ile
        195                 200                 205

Gly Phe Ser Leu Pro Met Ser Ile Val Ala Ile Cys Tyr Gly Leu Ile
    210                 215                 220

Ala Ala Lys Ile His Lys Lys Gly Met Ile Lys Ser Ser Arg Pro Leu
225                 230                 235                 240

Arg Val Leu Thr Ala Val Val Ala Ser Phe Phe Ile Cys Trp Phe Pro
                245                 250                 255

Phe Gln Leu Val Ala Leu Leu Gly Thr Val Trp Leu Lys Glu Met Leu
            260                 265                 270

Phe Tyr Gly Lys Tyr Lys Ile Ile Asp Ile Leu Val Asn Pro Thr Ser
        275                 280                 285

Ser Leu Ala Phe Phe Asn Ser Cys Leu Asn Pro Met Leu Tyr Val Phe
    290                 295                 300

Val Gly Gln Asp Phe Arg Glu Arg Leu Ile His Ser Leu Pro Thr Ser
305                 310                 315                 320

Leu Glu Arg Ala Leu Ser Glu Asp Ser Ala Pro Thr Asn Asp Thr Ala
                325                 330                 335

Ala Asn Ser Ala Ser Pro Pro Ala Glu Thr Glu Leu Gln Ala Met
            340                 345                 350

<210>  12
<211>  317
<212>  PRT
<213>  Homo sapiens

<400>  12

Met Thr Met Glu Thr Gln Met Ser Gln Asn Val Cys Pro Arg Asn Leu
1               5                   10                  15

Trp Leu Leu Gln Pro Leu Thr Val Leu Leu Leu Leu Ala Ser Ala Asp
            20                  25                  30

Ser Gln Ala Ala Ala Pro Pro Lys Ala Val Leu Lys Leu Glu Pro Pro
        35                  40                  45

Trp Ile Asn Val Leu Gln Glu Asp Ser Val Thr Leu Thr Cys Gln Gly
    50                  55                  60

Ala Arg Ser Pro Glu Ser Asp Ser Ile Gln Trp Phe His Asn Gly Asn
65                  70                  75                  80

Leu Ile Pro Thr His Thr Gln Pro Ser Tyr Arg Phe Lys Ala Asn Asn
                85                  90                  95

Asn Asp Ser Gly Glu Tyr Thr Cys Gln Thr Gly Gln Thr Ser Leu Ser
            100                 105                 110

Asp Pro Val His Leu Thr Val Leu Ser Glu Trp Leu Val Leu Gln Thr
        115                 120                 125

Pro His Leu Glu Phe Gln Glu Gly Glu Thr Ile Met Leu Arg Cys His
    130                 135                 140

Ser Trp Lys Asp Lys Pro Leu Val Lys Val Thr Phe Phe Gln Asn Gly
145                 150                 155                 160

Lys Ser Gln Lys Phe Ser His Leu Asp Pro Thr Phe Ser Ile Pro Gln
                165                 170                 175

Ala Asn His Ser His Ser Gly Asp Tyr His Cys Thr Gly Asn Ile Gly
            180                 185                 190

Tyr Thr Leu Phe Ser Ser Lys Pro Val Thr Ile Thr Val Gln Val Pro
        195                 200                 205

Ser Met Gly Ser Ser Ser Pro Met Gly Ile Ile Val Ala Val Val Ile
    210                 215                 220

Ala Thr Ala Val Ala Ala Ile Val Ala Ala Val Val Ala Leu Ile Tyr
225                 230                 235                 240

Cys Arg Lys Lys Arg Ile Ser Ala Asn Ser Thr Asp Pro Val Lys Ala
                245                 250                 255

Ala Gln Phe Glu Pro Pro Gly Arg Gln Met Ile Ala Ile Arg Lys Arg
            260                 265                 270

Gln Leu Glu Glu Thr Asn Asn Asp Tyr Glu Thr Ala Asp Gly Gly Tyr
        275                 280                 285

Met Thr Leu Asn Pro Arg Ala Pro Thr Asp Asp Asp Lys Asn Ile Tyr
    290                 295                 300

Leu Thr Leu Pro Pro Asn Asp His Val Asn Ser Asn Asn
305                 310                 315

<210>  13
<211>  290
<212>  PRT
<213>  Homo sapiens

<400>  13

Met Gly Ile Leu Ser Phe Leu Pro Val Leu Ala Thr Glu Ser Asp Trp
1               5                   10                  15

Ala Asp Cys Lys Ser Pro Gln Pro Trp Gly His Met Leu Leu Trp Thr
            20                  25                  30

Ala Val Leu Phe Leu Ala Pro Val Ala Gly Thr Pro Ala Pro Pro Lys
        35                  40                  45

Ala Val Leu Lys Leu Glu Pro Gln Trp Ile Asn Val Leu Gln Glu Asp
    50                  55                  60

Ser Val Thr Leu Thr Cys Arg Gly Thr His Ser Pro Glu Ser Asp Ser
65                  70                  75                  80

Ile Gln Trp Phe His Asn Gly Asn Leu Ile Pro Thr His Thr Gln Pro
                85                  90                  95

Ser Tyr Arg Phe Lys Ala Asn Asn Asn Asp Ser Gly Glu Tyr Thr Cys
            100                 105                 110

Gln Thr Gly Gln Thr Ser Leu Ser Asp Pro Val His Leu Thr Val Leu
        115                 120                 125

Ser Glu Trp Leu Val Leu Gln Thr Pro His Leu Glu Phe Gln Glu Gly
    130                 135                 140

Glu Thr Ile Val Leu Arg Cys His Ser Trp Lys Asp Lys Pro Leu Val
145                 150                 155                 160

Lys Val Thr Phe Phe Gln Asn Gly Lys Ser Lys Lys Phe Ser Arg Ser
                165                 170                 175

Asp Pro Asn Phe Ser Ile Pro Gln Ala Asn His Ser His Ser Gly Asp
            180                 185                 190

Tyr His Cys Thr Gly Asn Ile Gly Tyr Thr Leu Tyr Ser Ser Lys Pro
        195                 200                 205

Val Thr Ile Thr Val Gln Ala Pro Ser Ser Ser Pro Met Gly Ile Ile
    210                 215                 220

Val Ala Val Val Thr Gly Ile Ala Val Ala Ala Ile Val Ala Ala Val
225                 230                 235                 240

Val Ala Leu Ile Tyr Cys Arg Lys Lys Arg Ile Ser Ala Asn Pro Thr
                245                 250                 255

Asn Pro Asp Glu Ala Asp Lys Val Gly Ala Glu Asn Thr Ile Thr Tyr
            260                 265                 270

Ser Leu Leu Met His Pro Asp Ala Leu Glu Glu Pro Asp Asp Gln Asn
        275                 280                 285

Arg Ile
    290

<210>  14

<211>  1479
<212>  DNA
<213>  Homo sapiens

<400>  14
gtcaggttca tggttacgaa gctgctgacc ccaggatccc agcccgtggg agagaagggg      60

gtctctgaca gcccccaccc ctccccactg ccagatcctt attgggtctg agtttcaggg     120

gtggggcccc agctggaggt tataaaacag ctcaatcggg gagtacaacc ttcggtttct     180

cttcggggaa agctgctttc agcgcacacg ggaagatatc agaaacatcc taggatcagg     240

acaccccaga tcttctcaac tggaaccacg aaggctgttt cttccacaca gtactttgat     300

ctccatttaa gcaggcacct ctgtcctgcg ttccggagct gcgttcccga tggtcctcct     360

ttggctcacg ctgctcctga tcgccctgcc ctgtctcctg caaacgaagg aaggtgggaa     420

gccttgggca ggtgcggaga atctgacctg ctggattcat gacgtggatt tcttgagctg     480

cagctgggcg gtaggcccgg gggcccccgc ggacgtccag tacgacctgt acttgaacgt     540

tgccaacagg cgtcaacagt acgagtgtct tcactacaaa acggatgctc agggaacacg     600

tatcgggtgt cgtttcgatg acatctctcg actctccagc ggttctcaaa gttcccacat     660

cctggtgcgg ggcaggagcg cagccttcgg tatcccctgc acagataagt ttgtcgtctt     720

ttcacagatt gagatattaa ctccacccaa catgactgca aagtgtaata agacacattc     780

ctttatgcac tggaaaatga gaagtcattt caatcgcaaa tttcgctatg agcttcagat     840

acaaaagaga atgcagcctg taatcacaga acaggtcaga gacagaacct ccttccagct     900

actcaatcct ggaacgtaca cagtacaaat aagagcccgg gaaagagtgt atgaattctt     960

gagcgcctgg agcaccccca agcgcttcga gtgcgaccag gaggagggcg caaacacacg    1020

tgcctggcgg acgtcgctgc tgatcgcgct ggggacgctg ctggccctgg tctgtgtctt    1080

cgtgatctgc agaaggtatc tggtgatgca gagactcttt ccccgcatcc ctcacatgaa    1140

agaccccatc ggtgacagct tccaaaacga caagctggtg gtctgggagg cgggcaaagc    1200

cggcctggag gagtgtctgg tgactgaagt acaggtcgtg cagaaaactt gagactgggg    1260

ttcagggctt gtgggggtct gcctcaatct ccctggccgg gccaggcgcc tgcacagact    1320

ggctgctgga cctgcgcacg cagcccagga atggacattc ctaacgggtg gtgggcatgg    1380

gagatgcctg tgtaatttcg tccgaagctg ccaggaagaa gaacagaact ttgtgtgttt    1440

atttcatgat aaagtgattt tttttttttt aacccaaaa                           1479


<210>  15
<211>  1455
<212>  DNA
<213>  Homo sapiens

<400>  15
gccgagccag ccccttcacc accagccggc cgcgccccgg gaagggaagt ttgtggcgga      60

ggaggttcgt acgggaggag ggggaggcgc ccacgcatct ggggctgact cgctctttcg 120

caaaacgtct gggaggagtc cctggggcca caaaactgcc tccttcctga ggccagaagg 180

agagaagacg tgcagggacc ccgcgcacag gagctgccct cgcgacatgg gtcacccgcc 240

gctgctgccg ctgctgctgc tgctccacac ctgcgtccca gcctcttggg gcctgcggtg 300

catgcagtgt aagaccaacg gggattgccg tgtggaagag tgcgccctgg gacaggacct 360

ctgcaggacc acgatcgtgc gcttgtggga agaaggagaa gagctggagc tggtggagaa 420

aagctgtacc cactcagaga agaccaacag gaccctgagc tatcggactg gcttgaagat 480

caccagcctt accgaggttg tgtgtgggtt agacttgtgc aaccagggca actctggccg 540

ggctgtcacc tattcccgaa gccgttacct cgaatgcatt tcctgtggct catcagacat 600

gagctgtgag aggggccggc accagagcct gcagtgccgc agccctgaag aacagtgcct 660

ggatgtggtg acccactgga tccaggaagg tgaagaaggg cgtccaaagg atgaccgcca 720

cctccgtggc tgtggctacc ttcccggctg cccgggctcc aatggtttcc acaacaacga 780

caccttccac ttcctgaaat gctgcaacac caccaaatgc aacgagggcc caatcctgga 840

gcttgaaaat ctgccgcaga atggccgcca gtgttacagc tgcaagggga acagcaccca 900

tggatgctcc tctgaagaga ctttcctcat tgactgccga ggccccatga atcaatgtct 960

ggtagccacc ggcactcacg aacgctcact ctggggaagc tggttgccat gtaaaagtac 1020

tactgccctg agaccaccat gctgtgagga agcccaagct actcatgtat aaatgccatg 1080

tggagataga gccccagatg tttcagccat ctcagcccag gcaccagaca agtgggtgaa 1140

gaagccacct tggacatgta gccccagcag atgtgatata gagaagaaac aggaaacttg 1200

gctatattag tttcctaggg ctgcctgtga taaattatta caaactttat aaactaacac 1260

attgtgtgcc tatatcaaaa catcatggaa ggacaggcac agtggctcat gcctgtagtc 1320

ctagcacttt gggaggtgga gaaaggaaga tctcttgagc tcaggagttc aagatcagcc 1380

tgggcaacac agtgagacct catctccact aaaaataaaa aaaaattggc tggaaaaaaa 1440

aaaaaaaaaa aaaaa 1455

<210> 16
<211> 2618
<212> DNA
<213> Homo sapiens

<400> 16
tccacccaag agcaacctgg aactaagtta ttcggcaacg aactgttcca ctttgttgtg 60

aggcaataga tgtggaaatt ccctgacgag ggctctgtcc ctcatacttc ctgcggagct 120

tattgtcgta agaatatctg tcatcctgct aatgtgcatt gaaaggagag caacggggct 180

gaggccgtgt cagcacgatg gaccccaaac agaccaccct cctgtgtctt gtgctctgtc 240

tgggccagag gattcaggca caggaagggg actttcccat gcctttcata tctgccaaat 300

cgagtcctgt gattcccttg gatggatctg tgaaaatcca gtgccaggcc attcgtgaag 360

cttacctgac ccagctgatg atcataaaaa actccacgta ccgagagata ggcagaagac 420

tgaagttttg gaatgagact gatcctgagt tcgtcattga ccacatggac gcaaacaagg 480

cagggcgcta tcagtgccaa tataggatag ggcactacag gttccggtac agtgacaccc 540

tggagctggt agtgacaggc ttgtatggca aacccttcct ctctgcagat cggggtctgg 600

tgttgatgcc aggagagaat atttccctca cgtgcagctc agcacacatc ccatttgata 660

gattttcact ggccaaggag ggagaacttt ctctgccaca gcaccaaagt ggggaacacc 720

cggccaactt ctctttgggt cctgtggacc tcaatgtctc agggatctac aggtgctacg 780

gttggtacaa caggagcccc tacctgtggt ccttccccag taatgccttg gagcttgtgg 840

tcacagactc catccaccaa gattacacga cgcagaactt gatccgcatg gccgtggcag 900

gactggtcct cgtggctctc ttggccatac tggttgaaaa ttggcacagc catacggcac 960

tgaacaagga agcctcggca gatgtggctg aaccgagctg gagccaacag atgtgtcagc 1020

caggattgac ctttgcacga acaccaagtg tctgcaagta aacacctgga ggtgaaggca 1080

gagaggagcc aggactgtgg agtccgacaa agctacttga aggacacaag agagaaaagc 1140

tcactaagaa gcttgaatct actttttttt ttttttgaga cagagtctgg ctctgtcacc 1200

caggctggag tgcagtggag caatctcggc tcattgaacc tcttgggttc aagtgattct 1260

tgtgcctcag cctcccaagt agctggaatt acaggcacat accactgcac ccagctaatt 1320

tttgtatttt tagtagagat ggggtttcac tgtgttggcc aggctggtct cgaactcctg 1380

acctcaggtg atccacccac cttggcctcc caaagtgctg agattatagg catgagccac 1440

cacgcctggc cagatgcatg ttcaaaccaa tcaaatggtg ttttcttatg caggactgat 1500

cgatttgcac ccacctttct gcacataagt tatggttttc catcttatct gtcttctgat 1560

tttttatatc ctgtttaatt tcttccttca ttgttcttct ctttttttat ttattttatt 1620

tatttttatt tttattttta tttgagacag agtctcactc tgttgcccag gctggagtgc 1680

agtggcacga tctcggctca ctgcaacctc tgcctcctgg gttcaagtga ttctcctgcc 1740

tcggcctccc aagtagctgg gattgcaggc tcccaccatc acgcccagct acttttacag 1800

tatttttagt agagacgggg tttcatcaca ttggccaagc tggtctcaaa cttctgacct 1860

cgtgatctgc ccgcctcggc ctcccaaagt gctgggatta cagatgtgag ccactgcgcc 1920

cagccttctt tttatatttt taaatgtgtc ttccccaaat ataaatggtt ggtaagcatg 1980

ccaaatatat tcaataaccc ccctccttta tttttttttg ttgaagtgag gctctcccta 2040

tgttgcctaa gctggtcttg aactcctggt ctcaagcaat cctcctacct cagcctcctg 2100

ctgtgttcat ctacaaattg ataagagtga aagtcataat cctacaggag gattacccta 2160

tttatttcac aaaccctatt tctaccggat tttcatacaa ggaatacagg catgtgtttc 2220

acctcattaa tttatttttt cacttagttt tgatgatatt cacatatatt atcaagtgtg 2280

caaacattaa attcttgtgt acaaaactca aatggtcttc caaataattc cccattcttt 2340

tttcttataa actttcacag ctttaccctt gacagacttt actcaaggaa atctaagttg 2400

gtcatatgtg gctctttcac tgattgctat ttacttcatt gtccagtagc ttatgtatga 2460

aaatataatt ataaaatgta agggtcctac ttccagtgaa actgaaggga cttaggccca 2520

cttttatcct ttactgagag cttatctcta cttgataaaa tttctactgt attcttggct 2580

taactcaggt cctgtgatta aaaaaaaaat gcaaagta 2618

<210> 17
<211> 4201
<212> DNA
<213> Homo sapiens

<400> 17
tccggcccgc acccaccccc aagaggggcc ttcagctttg gggctcagag gcacgacctc 60

ctggggaggg ttaaaaggca gacgcccccc cgccccccgc gcccccgcgc cccgactcct 120

tcgccgcctc cagcctctcg ccagtgggaa gcggggagca gccgcgcggc cggagtccgg 180

aggcgagggg aggtcggccg caacttcccc ggtccacctt aagaggacga tgtagccagc 240

tcgcagcgct gaccttagaa aaacaagttt gcgcaaagtg gagcggggac ccggcctctg 300

ggcagccccg gcggcgcttc cagtgccttc cagccctcgc gggcggcgca gccgcggccc 360

atggagcccg ccggcccggc ccccggccgc ctcgggccgc tgctctgcct gctgctcgcc 420

gcgtcctgcg cctggtcagg agtggcgggt gaggaggagc tgcaggtgat tcagcctgac 480

aagtccgtgt tggttgcagc tggagagaca gccactctgc gctgcactgc gacctctctg 540

atccctgtgg ggcccatcca gtggttcaga ggagctggac caggccggga attaatctac 600

aatcaaaaag aaggccactt cccccgggta acaactgttt cagacctcac aaagagaaac 660

aacatggact tttccatccg catcggtaac atcaccccag cagatgccgg cacctactac 720

tgtgtgaagt tccggaaagg gagccccgat gacgtggagt ttaagtctgg agcaggcact 780

gagctgtctg tgcgcgccaa accctctgcc cccgtggtat cgggccctgc ggcgagggcc 840

acacctcagc acacagtgag cttcacctgc gagtcccacg gcttctcacc cagagacatc 900

accctgaaat ggttcaaaaa tgggaatgag ctctcagact tccagaccaa cgtggacccc 960

gtaggagaga gcgtgtccta cagcatccac agcacagcca aggtggtgct gacccgcgag 1020

gacgttcact ctcaagtcat ctgcgaggtg gcccacgtca ccttgcaggg ggaccctctt 1080

cgtgggactg ccaacttgtc tgagaccatc cgagttccac ccaccttgga ggttactcaa 1140

cagcccgtga gggcagagaa ccaggtgaat gtcacctgcc aggtgaggaa gttctacccc 1200
cagagactac agctgacctg gttggagaat ggaaacgtgt cccggacaga aacggcctca 1260
accgttacag agaacaagga tggtacctac aactggatga gctggctcct ggtgaatgta 1320
tctgcccaca gggatgatgt gaagctcacc tgccaggtgg agcatgacgg gcagccagcg 1380
gtcagcaaaa gccatgacct gaaggtctca gcccacccga aggagcaggg ctcaaatacc 1440
gccgctgaga acactggatc taatgaacgg aacatctata ttgtggtggg tgtggtgtgc 1500
accttgctgg tggccctact gatggcggcc ctctacctcg tccgaatcag acagaagaaa 1560
gcccagggct ccacttcttc tacaaggttg catgagcccg agaagaatgc cagagaaata 1620
acacaggaca caaatgatat cacatatgca gacctgaacc tgcccaaggg gaagaagcct 1680
gctccccagg ctgcggagcc caacaaccac acggagtatg ccagcattca gaccagcccg 1740
cagcccgcgt cggaggacac cctcacctat gctgacctgg acatggtcca cctcaaccgg 1800
acccccaagc agccggcccc caagcctgag ccgtccttct cagagtacgc cagcgtccag 1860
gtcccgagga agtgaatggg accgtggttt gctctagcac ccatctctac gcgctttctt 1920
gtcccacagg gagccgccgt gatgagcaca gccaacccag ttcccggagg gctggggcgg 1980
tgcaggctct gggacccagg ggccagggtg gctcttctct ccccacccct ccttggctct 2040
ccagcacttc ctgggcagcc acggccccct ccccccacat tgccacatac ctggaggctg 2100
acgttgccaa accagccagg gaaccaacct gggaagtggc cagaactgcc tggggtccaa 2160
gaactcttgt gcctccgtcc atcaccatgt gggttttgaa gaccctcgac tgcctccccg 2220
atgctccgaa gcctgatctt ccagggtggg gaggagaaaa tcccacctcc cctgacctcc 2280
accacctcca ccaccaccac caccaccacc accaccacta ccaccaccac ccaactgggg 2340
ctagagtggg gaagatttcc cctttagatc aaactgcccc ttccatggaa aagctggaaa 2400
aaaactctgg aacccatatc caggcttggt gaggttgctg ccaacagtcc tggcctcccc 2460
catccctagg ctaaagagcc atgagtcctg gaggaggaga ggacccctcc caaaggactg 2520
gagacaaaac cctctgcttc cttgggtccc tccaagactc cctggggccc aactgtgttg 2580
ctccacccgg acccatctct cccttctaga cctgagcttg cccctccagc tagcactaag 2640
caacatctcg ctgtggacgc ctgtaaatta ctgagaaatg tgaaacgtgc aatcttgaaa 2700
ctgaggtgtt agaaaacttg atctgtggtg ttttgttttg ttttttttct taaaacaaca 2760
gcaacgtgat cttggctgtc tgtcatgtgt tgaagtccat ggttgggtct tgtgaagtct 2820
gaggtttaac agtttgttgt cctggaggga ttttcttaca gcgaagactt gagttcctcc 2880
aagtcccaga accccaagaa tgggcaagaa ggatcaggtc agccactccc tggagacaca 2940
gccttctggc tgggactgac ttggccatgt tctcagctga gccacgcggc tggtagtgca 3000
gccttctgtg accccgctgt ggtaagtcca gcctgcccag ggctgctgag ggctgcctct 3060

tgacagtgca gtcttatcga gacccaatgc ctcagtctgc tcatccgtaa agtggggata 3120

gtgaagatga cacccctccc caccacctct cataagcact ttaggaacac acagaggtta 3180

gggatagtgg ccctggccgt ctatcctacc cctttagtga ccgcccccat cccggctttc 3240

tgagctgatc cttgaagaag aaatcttcca tttctgctct caaaccctac tgggatcaaa 3300

ctggaataaa ttgaagacag ccagggggat ggtgcagctg tgaagctcgg gctgattccc 3360

cctctgtccc agaaggttgg ccagagggtg tgacccagtt accctttaac ccccaccctt 3420

ccagtcgggt gtgagggcct gaccgggccc agggcaagca gatgtcgcaa gccctattta 3480

ttcagtcttc actataactc ttagagttga gacgctaatg ttcatgactc ctggccttgg 3540

gatgcccaag ggatttctgg ctcaggctgt aaaagtagct gagccatcct gcccattcct 3600

ggaggtccta caggtgaaac tgcaggagct cagcatagac ccagctctct gggggatggt 3660

cacctggtga tttcaatgat ggcatccagg aattagctga gccaacagac catgtggaca 3720

gctttggcca gagctcccgt gtggcatctg ggagccacag tgacccagcc acctggctca 3780

ggctagttcc aaattccaaa agattggctt gtaaaccttc gtctccctct cttttaccca 3840

gagacagcac atacgtgtgc acacgcatgc acacacacat tcagtatttt aaaagaatgt 3900

tttcttggtg ccattttcat tttattttat tttttaattc ttggaggggg aaataaggga 3960

ataaggccaa ggaagatgta tagctttagc tttagcctgg caacctggag aatccacata 4020

ccttgtgtat tgaaccccag gaaaaggaag aggtcgaacc aaccctgcgg aaggagcatg 4080

gtttcaggag tttattttaa gactgctggg aaggaaacag gccccatttt gtatatagtt 4140

gcaacttaaa ctttttggct tgcaaaatat ttttgtaata aagatttctg ggtaataatg 4200

a 4201

<210> 18
<211> 2869
<212> DNA
<213> Homo sapiens

<400> 18
agaattcttt ggcaggggcg accttagaat cctggggagg agcgagaatg gaatcccggg 60

gaggaacagg ggtggaatcc gggggcgggg gtcagaacgc caggaggggg cggggccgga 120

gccagggtcg gcttgactcg ggggagcagc gggtggatcc tgtgacgtca gcgggttcga 180

accgccggag ctgagcgaga ggccgggggt gccgagccgg gcggggagag ctgggccggg 240

agagcagaac agggaggcta gagcgcagcg ggaaccggcc cggagccgga gccggagccc 300

cacaggcacc tactaaaccg cccagccgat cggcccccac agagtggccc gcgggcctcc 360

ggccgggccc agtcccctcc cgggccctcc atggcccggg ccgctgccct cctgccgtcg 420

agatcgccgc cgacgccgct gctgtggccg ctgctgctgc tgctgctcct ggaaaccgga 480

gcccaggatg tgcgagttca agtgctaccc gaggtgcgag gccagctcgg gggcaccgtg 540

gagctgccgt gccacctgct gccacctgtt cctggactgt acatctccct ggtgacctgg 600

cagcgcccag atgcacctgc gaaccaccag aatgtggccg ccttccaccc taagatgggt 660

cccagcttcc ccagcccgaa gcctggcagc gagcggctgt ccttcgtctc tgccaagcag 720

agcactgggc aagacacaga ggcagagctc caggacgcca cgctggccct ccacgggctc 780

acggtggagg acgagggcaa ctacacttgc gagtttgcca ccttccccaa ggggtccgtc 840

cgagggatga cctggctcag agtcatagcc aagcccaaga accaagctga ggcccagaag 900

gtcacgttca gccaggaccc tacgacagtg gccctctgca tctccaaaga gggccgccca 960

cctgcccgga tctcctggct ctcatccctg gactgggaag ccaaagagac tcaggtgtca 1020

gggaccctgg ccggaactgt cactgtcacc agccgcttca ccttggtgcc ctcgggccga 1080

gcagatggtg tcacggtcac ctgcaaagtg gagcatgaga gcttcgagga accagccctg 1140

atacctgtga ccctctctgt acgctaccct cctgaagtgt ccatctccgg ctatgatgac 1200

aactggtacc tcggccgtac tgatgccacc ctgagctgtg acgtccgcag caacccagag 1260

cccacgggct atgactggag cacgacctca ggcaccttcc cgacctccgc agtggcccag 1320

ggctcccagc tggtcatcca cgcagtggac agtctgttca ataccacctt cgtctgcaca 1380

gtcaccaatg ccgtgggcat gggccgcgct gagcaggtca tctttgtccg agagaccccc 1440

aacacagcag gcgcaggggc cacaggcggc atcatcgggg gcatcatcgc cgccatcatt 1500

gctactgctg tggctgccac gggcatcctt atctgccggc agcagcggaa ggagcagacg 1560

ctgcaggggg cagaggagga cgaagacctg gagggacctc cctcctacaa gccaccgacc 1620

ccaaaagcga agctggaggc acaggagatg ccctcccagc tcttcactct gggggcctcg 1680

gagcacagcc cactcaagac cccctacttt gatgctggcg cctcatgcac tgagcaggaa 1740

atgcctcgat accatgagct gcccaccttg gaagaacggt caggaccctt gcaccctgga 1800

gccacaagcc tggggtcccc catcccggtg cctccagggc cacctgctgt ggaagacgtt 1860

tccctggatc tagaggatga ggagggggag gaggaggaag agtatctgga caagatcaac 1920

cccatctatg atgctctgtc ctatagcagc ccctctgatt cctaccaggg caaaggcttt 1980

gtcatgtccc gggccatgta tgtgtgagct gccatgcgcc tggcgtctca catctcacct 2040

gttgatccct tagctttctt gccaaggatc tagtgccccc tgacctctgg ccaggccact 2100

gtcagttaac acatatgcat tccatttgtg atgtctacct tggtggctcc actatgaccc 2160

ctaacccatg agcccagaga aattcaccgt gataatggaa tcctggcaac cttatctcat 2220

gaggcaggag gtggggaagg tgcttctgca caacctctga tcccaaggac tcctctccca 2280

gactgtgacc ttagaccata cctctcaccc cccaatgcct cgactccccc aaaatcacaa 2340

agaagaccct agacctataa tttgtcttca ggtagtaaat tcccaatagg tctgctggag 2400

tgggcgctga gggctccctg ctgctcagac ctgagccctc caggcagcag ggtcccactt 2460

accccctccc caccctgttc cccaaaggtg ggaaagaggg gattccccag cccaaggcag 2520

ggttttccca gcaccctcct gtaagcagaa gtctcagggt ccagaccctt ccctgagccc 2580

ccacccccac cccaattcct gcctaccaag caagcagccc cagcctaggg tcagacaggg 2640

tgagcctcat acagactgtg ccttgatggc cccagccttg ggagaagaat ttactgttaa 2700

cctggaagac tactgaatca ttttaccctt gcccagtgga ataggaccta aacatccccc 2760

ttccggggaa agtgggtcat ctgaattggg ggtagcaatt gatactgttt tgtaaactac 2820

atttcctaca aaatatgaat ttatactttg accaggaaaa aaaaaaaaa 2869

<210> 19
<211> 4006
<212> DNA
<213> Homo sapiens

<400> 19
tgccaaggct ccagcccggc cgggctccga ggcgagaggc tgcatggagt ggccggcgcg 60

gctctgcggg ctgtgggcgc tgctgctctg cgccggcggc gggggcgggg gcgggggcgc 120

cgcgcctacg gaaactcagc cacctgtgac aaatttgagt gtctctgttg aaaacctctg 180

cacagtaata tggacatgga atccacccga gggagccagc tcaaattgta gtctatggta 240

ttttagtcat tttggcgaca aacaagataa gaaaatagct ccggaaactc gtcgttcaat 300

agaagtaccc ctgaatgaga ggatttgtct gcaagtgggg tcccagtgta gcaccaatga 360

gagtgagaag cctagcattt tggttgaaaa atgcatctca cccccagaag gtgatcctga 420

gtctgctgtg actgagcttc aatgcatttg gcacaacctg agctacatga agtgttcttg 480

gctccctgga aggaatacca gtcccgacac taactatact ctctactatt ggcacagaag 540

cctggaaaaa attcatcaat gtgaaaacat ctttagagaa ggccaatact ttggttgttc 600

ctttgatctg accaaagtga aggattccag ttttgaacaa cacagtgtcc aaataatggt 660

caaggataat gcaggaaaaa ttaaaccatc cttcaatata gtgcctttaa cttcccgtgt 720

gaaacctgat cctccacata ttaaaaacct ctccttccac aatgatgacc tatatgtgca 780

atgggagaat ccacagaatt ttattagcag atgcctattt tatgaagtag aagtcaataa 840

cagccaaact gagacacata atgttttcta cgtccaagag gctaaatgtg agaatccaga 900

atttgagaga aatgtggaga atacatcttg tttcatggtc cctggtgttc ttcctgatac 960

tttgaacaca gtcagaataa gagtcaaaac aaataagtta tgctatgagg atgacaaact 1020

ctggagtaat tggagccaag aaatgagtat aggtaagaag cgcaattcca cactctacat 1080

aaccatgtta ctcattgttc cagtcatcgt cgcaggtgca atcatagtac tcctgcttta 1140

cctaaaaagg ctcaagatta ttatattccc tccaattcct gatcctggca agatttttaa 1200

agaaatgttt ggagaccaga atgatgatac tctgcactgg aagaagtacg acatctatga 1260

gaagcaaacc aaggaggaaa ccgactctgt agtgctgata gaaaacctga agaaagcctc 1320

tcagtgatgg agataattta tttttacctt cactgtgacc ttgagaagat tcttcccatt 1380

ctccatttgt tatctgggaa cttattaaat ggaaactgaa actactgcac catttaaaaa 1440

caggcagctc ataagagcca caggtcttta tgttgagtcg cgcaccgaaa aactaaaaat 1500

aatgggcgct ttggagaaga gtgtggagtc attctcattg aattataaaa gccagcaggc 1560

ttcaaactag gggacaaagc aaaaagtgat gatagtggtg gagttaatct tatcaagagt 1620

tgtgacaact tcctgaggga tctatacttg ctttgtgttc tttgtgtcaa catgaacaaa 1680

ttttatttgt aggggaactc atttggggtg caaatgctaa tgtcaaactt gagtcacaaa 1740

gaacatgtag aaaacaaaat ggataaaatc tgatatgtat tgtttgggat cctattgaac 1800

catgtttgtg gctattaaaa ctcttttaac agtctgggct gggtccggtg gctcacgcct 1860

gtaatcccag caatttggga gtccgaggcg ggcggatcac tcgaggtcag gagttccaga 1920

ccagcctgac caaaatggtg aaacctcctc tctactaaaa ctacaaaaat taactgggtg 1980

tggtggcgcg tgcctgtaat cccagctact cgggaagctg aggcaggtga attgtttgaa 2040

cctgggaggt ggaggttgca gtgagcagag atcacaccac tgcactctag cctgggtgac 2100

agagcaagac tctgtctaaa aaacaaaaca aaacaaaaca aaacaaaaaa acctcttaat 2160

attctggagt catcattccc ttcgacagca ttttcctctg ctttgaaagc cccagaaatc 2220

agtgttggcc atgatgacaa ctacagaaaa accagaggca gcttctttgc caagaccttt 2280

caaagccatt ttaggctgtt aggggcagtg gaggtagaat gactccttgg gtattagagt 2340

ttcaaccatg aagtctctaa caatgtattt tcttcacctc tgctactcaa gtagcattta 2400

ctgtgtcttt ggtttgtgct aggcccccgg gtgtgaagca cagacccctt ccaggggttt 2460

acagtctatt tgagactcct cagttcttgc cacttttttt tttaatctcc accagtcatt 2520

tttcagacct tttaactcct caattccaac actgatttcc ccttttgcat tctccctcct 2580

tcccttcctt gtagcctttt gactttcatt ggaaattagg atgtaaatct gctcaggaga 2640

cctggaggag cagaggataa ttagcatctc aggttaagtg tgagtaatct gagaaacaat 2700

gactaattct tgcatatttt gtaacttcca tgtgagggtt ttcagcattg atatttgtgc 2760

attttctaaa cagagatgag gtggtatctt cacgtagaac attggtattc gcttgagaaa 2820

aaaagaatag ttgaacctat ttctctttct ttacaagatg ggtccaggat tcctcttttc 2880

tctgccataa atgattaatt aaatagcttt tgtgtcttac attggtagcc agccagccaa 2940

ggctctgttt atgcttttgg ggggcatata ttgggttcca ttctcaccta tccacacaac 3000

atatccgtat atatcccctc tactcttact tcccccaaat ttaaagaagt atgggaaatg 3060

agaggcattt cccccacccc atttctctcc tcacacacag actcatatta ctggtaggaa 3120

cttgagaact ttatttccaa gttgttcaaa catttaccaa tcatattaat acaatgatgc 3180

tatttgcaat tcctgctcct aggggagggg agataagaaa ccctcactct ctacaggttt 3240

gggtacaagt ggcaacctgc ttccatggcc gtgtagaagc atggtgccct ggcttctctg 3300

aggaagctgg ggttcatgac aatggcagat gtaaagttat tcttgaagtc agattgaggc 3360

tgggagacag ccgtagtaga tgttctactt tgttctgctg ttctctagaa agaatatttg 3420

gttttcctgt ataggaatga gattaattcc tttccaggta ttttataatt ctgggaagca 3480

aaacccatgc ctccccctag ccatttttac tgttatccta tttagatggc catgaagagg 3540

atgctgtgaa attcccaaca aacattgatg ctgacagtca tgcagtctgg gagtggggaa 3600

gtgatctttt gttcccatcc tcttctttta gcagtaaaat agctgaggga aaaggagggg 3660

aaaaggaagt tatgggaata cctgtggtgg ttgtgatccc taggtcttgg gagctcttgg 3720

aggtgtctgt atcagtggat ttcccatccc ctgtgggaaa ttagtaggct catttactgt 3780

tttaggtcta gcctatgtgg attttttcct aacataccta agcaaaccca gtgtcaggat 3840

ggtaattctt attctttcgt tcagttaagt ttttcccttc atctgggcac tgaagggata 3900

tgtgaaacaa tgttaacatt tttggtagtc ttcaaccagg gattgtttct gtttaacttc 3960

ttataggaaa gcttgagtaa aataaatatt gtctttttgt atgtca 4006

<210> 20
<211> 3682
<212> DNA
<213> Homo sapiens

<400> 20
gcgagcgcag cggagcctgg agagaaggcg ctgggctgcg agggcgcgag ggcgcgaggg 60

cagggggcaa ccggaccccg cccgcaccca tggcgcccgt cgccgtctgg gccgcgctgg 120

ccgtcggact ggagctctgg gctgcggcgc acgccttgcc cgcccaggtg gcatttacac 180

cctacgcccc ggagcccggg agcacatgcc ggctcagaga atactatgac cagacagctc 240

agatgtgctg cagcaaatgc tcgccgggcc aacatgcaaa agtcttctgt accaagacct 300

cggacaccgt gtgtgactcc tgtgaggaca gcacatacac ccagctctgg aactgggttc 360

ccgagtgctt gagctgtggc tcccgctgta gctctgacca ggtggaaact caagcctgca 420

ctcgggaaca gaaccgcatc tgcacctgca ggcccggctg gtactgcgcg ctgagcaagc 480

aggaggggtg ccggctgtgc gcgccgctgc gcaagtgccg cccgggcttc ggcgtggcca 540

gaccaggaac tgaaacatca gacgtggtgt gcaagccctg tgccccgggg acgttctcca 600

acacgacttc atccacggat atttgcaggc cccaccagat ctgtaacgtg gtggccatcc 660

ctgggaatgc aagcatggat gcagtctgca cgtccacgtc ccccacccgg agtatggccc 720

caggggcagt acacttaccc cagccagtgt ccacacgatc ccaacacacg cagccaactc 780

cagaacccag cactgctcca agcacctcct tcctgctccc aatgggcccc agccccccag 840

ctgaagggag cactggcgac ttcgctcttc cagttggact gattgtgggt gtgacagcct 900

tgggtctact aataatagga gtggtgaact gtgtcatcat gacccaggtg aaaaagaagc 960

ccttgtgcct gcagagagaa gccaaggtgc ctcacttgcc tgccgataag gcccggggta 1020

cacagggccc cgagcagcag cacctgctga tcacagcgcc gagctccagc agcagctccc 1080

tggagagctc ggccagtgcg ttggacagaa gggcgcccac tcggaaccag ccacaggcac 1140

caggcgtgga ggccagtggg gccggggagg cccgggccag caccgggagc tcagattctt 1200

cccctggtgg ccatgggacc caggtcaatg tcacctgcat cgtgaacgtc tgtagcagct 1260

ctgaccacag ctcacagtgc tcctcccaag ccagctccac aatgggagac acagattcca 1320

gcccctcgga gtccccgaag gacgagcagg tccccttctc caaggaggaa tgtgcctttc 1380

ggtcacagct ggagacgcca gagaccctgc tggggagcac cgaagagaag cccctgcccc 1440

ttggagtgcc tgatgctggg atgaagccca gttaaccagg ccggtgtggg ctgtgtcgta 1500

gccaaggtgg gctgagccct ggcaggatga ccctgcgaag gggccctggt ccttccaggc 1560

ccccaccact aggactctga ggctctttct gggccaagtt cctctagtgc cctccacagc 1620

cgcagcctcc ctctgacctg caggccaaga gcagaggcag cgagttgtgg aaagcctctg 1680

ctgccatggc gtgtccctct cggaaggctg gctgggcatg gacgttcggg gcatgctggg 1740

gcaagtccct gactctctgt gacctgcccc gcccagctgc acctgccagc ctggcttctg 1800

gagcccttgg gttttttgtt tgtttgtttg tttgtttgtt tgtttctccc cctgggctct 1860

gccccagctc tggcttccag aaaaccccag catccttttc tgcagagggg ctttctggag 1920

aggagggatg ctgcctgagt cacccatgaa gacaggacag tgcttcagcc tgaggctgag 1980

actgcgggat ggtcctgggg ctctgtgcag ggaggaggtg gcagccctgt agggaacggg 2040

gtccttcaag ttagctcagg aggcttggaa agcatcacct caggccaggt gcagtggctc 2100

acgcctatga tcccagcact ttgggaggct gaggcgggtg gatcacctga ggttaggagt 2160

tcgagaccag cctggccaac atggtaaaac cccatctcta ctaaaaatac agaaattagc 2220

cgggcgtggt ggcgggcacc tatagtccca gctactcaga agcctgaggc tgggaaatcg 2280

tttgaacccg ggaagcggag gttgcaggga gccgagatca cgccactgca ctccagcctg 2340

ggcgacagag cgagagtctg tctcaaaaga aaaaaaaaag caccgcctcc aaatgccaac 2400

ttgtcctttt gtaccatggt gtgaaagtca gatgcccaga gggcccaggc aggccaccat 2460

attcagtgct gtggcctggg caagataacg cacttctaac tagaaatctg ccaatttttt 2520

aaaaaagtaa gtaccactca ggccaacaag ccaacgacaa agccaaactc tgccagccac 2580

atccaacccc ccacctgcca tttgcaccct ccgccttcac tccggtgtgc ctgcagcccc 2640

gcgcctcctt ccttgctgtc ctaggccaca ccatctcctt tcagggaatt tcaggaacta 2700

gagatgactg agtcctcgta gccatctctc tactcctacc tcagcctaga ccctcctcct 2760

cccccagagg ggtgggttcc tcttccccac tccccacctt caattcctgg gccccaaacg 2820

ggctgccctg ccactttggt acatggccag tgtgatccca agtgccagtc ttgtgtctgc 2880

gtctgtgttg cgtgtcgtgg gtgtgtgtag ccaaggtcgg taagttgaat ggcctgcctt 2940

gaagccactg aagctgggat tcctccccat tagagtcagc cttccccctc ccagggccag 3000

ggccctgcag aggggaaacc agtgtagcct tgcccggatt ctgggaggaa gcaggttgag 3060

gggctcctgg aaaggctcag tctcaggagc atggggataa aggagaaggc atgaaattgt 3120

ctagcagagc aggggcaggg tgataaattg ttgataaatt ccactggact tgagcttggc 3180

agctgaacta ttggagggtg ggagagccca gccattacca tggagacaag aagggttttc 3240

caccctggaa tcaagatgtc agactggctg gctgcagtga cgtgcacctg tactcaggag 3300

gctgagggga ggatcactgg agcccaggag tttgaggctg cagcgagcta tgatcgcgcc 3360

actacactcc agcctgagca acagagtgag accctgtctc ttaaagaaaa aaaaagtcag 3420

actgctggga ctggccaggt ttctgcccac attggaccca catgaggaca tgatggagcg 3480

cacctgcccc ctggtggaca gtcctgggag aacctcaggc ttccttggca tcacagggca 3540

gagccgggaa gcgatgaatt tggagactct gtggggcctt ggttcccttg tgtgtgtgtg 3600

ttgatcccaa gacaatgaaa gtttgcactg tatgctggac ggcattcctg cttatcaata 3660

aacctgtttg ttttaaaaaa aa 3682

<210> 21
<211> 5170
<212> DNA
<213> Homo sapiens

<400> 21
gtggccggcg gccggagccg actcggagcg cgcggcgccg gccgggagga gccggagagc 60

ggccgggccg ggcggtgggg gcgccggcct gccccgcgcg ccccagggag cggcaggaat 120

gtgacaatcg cgcgcccgcg caccgaagca ctcctcgctc ggctcctagg gctctcgccc 180

ctctgagctg agccgggttc cgcccggggc tgggatccca tcaccctcca cggccgtccg 240

tccaggtaga cgcaccctct gaagatggtg actccctcct gagaagctgg accccttggt 300

aaaagacaag gccttctcca agaagaatat gaaagtgtta ctcagactta tttgtttcat 360

agctctactg atttcttctc tggaggctga taaatgcaag gaacgtgaag aaaaaataat 420

tttagtgtca tctgcaaatg aaattgatgt tcgtccctgt cctcttaacc caaatgaaca 480

caaaggcact ataacttggt ataaagatga cagcaagaca cctgtatcta cagaacaagc 540

ctccaggatt catcaacaca aagagaaact ttggtttgtt cctgctaagg tggaggattc 600

aggacattac tattgcgtgg taagaaattc atcttactgc ctcagaatta aaataagtgc 660

aaaatttgtg gagaatgagc ctaacttatg ttataatgca caagccatat ttaagcagaa 720

actacccgtt gcaggagacg gaggacttgt gtgcccttat atggagtttt ttaaaaatga 780

aaataatgag ttacctaaat tacagtggta taaggattgc aaacctctac ttcttgacaa 840

tatacacttt agtggagtca aagataggct catcgtgatg aatgtggctg aaaagcatag 900

agggaactat acttgtcatg catcctacac atacttgggc aagcaatatc ctattacccg 960

ggtaatagaa tttattactc tagaggaaaa caaacccaca aggcctgtga ttgtgagccc 1020

agctaatgag acaatggaag tagacttggg atcccagata caattgatct gtaatgtcac 1080

cggccagttg agtgacattg cttactggaa gtggaatggg tcagtaattg atgaagatga 1140

cccagtgcta ggggaagact attacagtgt ggaaaatcct gcaaacaaaa gaaggagtac 1200

cctcatcaca gtgcttaata tatcggaaat tgaaagtaga ttttataaac atccatttac 1260

ctgttttgcc aagaatacac atggtataga tgcagcatat atccagttaa tatatccagt 1320

cactaatttc cagaagcaca tgattggtat atgtgtcacg ttgacagtca taattgtgtg 1380

ttctgttttc atctataaaa tcttcaagat tgacattgtg ctttggtaca gggattcctg 1440

ctatgatttt ctcccaataa aagcttcaga tggaaagacc tatgacgcat atatactgta 1500

tccaaagact gttggggaag ggtctacctc tgactgtgat atttttgtgt ttaaagtctt 1560

gcctgaggtc ttggaaaaac agtgtggata taagctgttc atttatggaa gggatgacta 1620

cgttggggaa gacattgttg aggtcattaa tgaaaacgta aagaaaagca gaagactgat 1680

tatcatttta gtcagagaaa catcaggctt cagctggctg ggtggttcat ctgaagagca 1740

aatagccatg tataatgctc ttgttcagga tggaattaaa gttgtcctgc ttgagctgga 1800

gaaaatccaa gactatgaga aaatgccaga atcgattaaa ttcattaagc agaaacatgg 1860

ggctatccgc tggtcagggg actttacaca gggaccacag tctgcaaaga caaggttctg 1920

gaagaatgtc aggtaccaca tgccagtcca gcgacggtca ccttcatcta aacaccagtt 1980

actgtcacca gccactaagg agaaactgca aagagaggct cacgtgcctc tcgggtagca 2040

tggagaagtt gccaagagtt ctttaggtgc ctcctgtctt atggcgttgc aggccaggtt 2100

atgcctcatg ctgacttgca gagttcatgg aatgtaacta tatcatcctt tatccctgag 2160

gtcacctgga atcagattat taagggaata agccatgacg tcaatagcag cccagggcac 2220

ttcagagtag agggcttggg aagatctttt aaaaaggcag taggcccggt gtggtggctc 2280

acgcctataa tcccagcact ttgggaggct gaagtgggtg gatcaccaga ggtcaggagt 2340

tcgagaccag cccagccaac atggcaaaac cccatctcta ctaaaaatac aaaaatgagc 2400

taggcatggt ggcacacgcc tgtaatccca gctacacctg aggctgaggc aggagaattg 2460

cttgaaccgg ggagacggag gttgcagtga gccgagtttg ggccactgca ctctagcctg 2520
gcaacagagc aagactccgt ctcaaaaaaa gggcaataaa tgccctctct gaatgtttga 2580
actgccaaga aaaggcatgg agacagcgaa ctagaagaaa gggcaagaag gaaatagcca 2640
ccgtctacag atggcttagt taagtcatcc acagcccaag ggcggggcta tgccttgtct 2700
ggggaccctg tagagtcact gaccctggag cggctctcct gagaggtgct gcaggcaaag 2760
tgagactgac acctcactga ggaagggaga catattcttg gagaactttc catctgcttg 2820
tattttccat acacatcccc agccagaagt tagtgtccga agaccgaatt ttattttaca 2880
gagcttgaaa actcacttca atgaacaaag ggattctcca ggattccaaa gttttgaagt 2940
catcttagct ttccacagga gggagagaac ttaaaaaagc aacagtagca gggaattgat 3000
ccacttctta atgctttcct ccctggcatg accatcctgt cctttgttat tatcctgcat 3060
tttacgtctt tggaggaaca gctccctagt ggcttcctcc gtctgcaatg tcccttgcac 3120
agcccacaca tgaaccatcc ttcccatgat gccgctcttc tgtcatcccg ctcctgctga 3180
aacacctccc aggggctcca cctgttcagg agctgaagcc catgctttcc caccagcatg 3240
tcactcccag accacctccc tgccctgtcc tccagcttcc cctcgctgtc ctgctgtgtg 3300
aattcccagg ttggcctggt ggccatgtcg cctgccccca gcactcctct gtctctgctc 3360
ttgcctgcac ccttcctcct cctttgccta ggaggccttc tcgcatttcc tctagctgat 3420
cagaatttta ccaaaattca gaacatcctc caattccaca gtctctggga gactttccct 3480
aagaggcgac ttcctctcca gccttctctc tctggtcagg cccactgcag agatggtggt 3540
gagcacatct gggaggctgg tctccctcca gctggaattg ctgctctctg agggagaggc 3600
tgtggtggct gtctctgtcc ctcactgcct tccaggagca atttgcacat gtaacataga 3660
tttatgtaat gctttatgtt taaaaacatt ccccaattat cttatttaat ttttgcaatt 3720
attctaattt tatatataga gaaagtgacc tattttttaa aaaaatcaca ctctaagttc 3780
tattgaacct aggacttgag cctccatttc tggcttctag tctggtgttc tgagtacttg 3840
atttcaggtc aataacggtc ccccctcact ccacactggc acgtttgtga gaagaaatga 3900
cattttgcta ggaagtgacc gagtctagga atgcttttat tcaagacacc aaattccaaa 3960
cttctaaatg ttggaatttt caaaaattgt gtttagattt tatgaaaaac tcttctactt 4020
tcatctattc tttccctaga ggcaaacatt tcttaaaatg tttcattttc attaaaaatg 4080
aaagccaaat ttatatgcca ccgattgcag gacacaagca cagttttaag agttgtatga 4140
acatggagag gacttttggt ttttatattt ctcgtattta atatgggtga acaccaactt 4200
ttatttggaa taataatttt cctcctaaac aaaaacacat tgagtttaag tctctgactc 4260
ttgcctttcc acctgctttc tcctgggccc gctttgcctg cttgaaggaa cagtgctgtt 4320

ctggagctgc tgttccaaca gacagggcct agctttcatt tgacacacag actacagcca 4380

gaagcccatg gagcagggat gtcacgtctt gaaaagccta ttagatgttt tacaaattta 4440

attttgcaga ttattttagt ctgtcatcca gaaaatgtgt cagcatgcat agtgctaaga 4500

aagcaagcca atttggaaac ttaggttagt gacaaaattg gccagagagt gggggtgatg 4560

atgaccaaga attacaagta gaatggcagc tggaatttaa ggagggacaa gaatcaatgg 4620

ataagcgtgg gtggaggaag atccaaacag aaaagtgcaa agttattccc catcttccaa 4680

gggttgaatt ctggaggaag aagacacatt cctagttccc cgtgaacttc ctttgactta 4740

ttgtccccac taaaacaaaa caaaaaactt ttaatgcctt ccacattaat tagattttct 4800

tgcagttttt ttatggcatt tttttaaaga tgccctaagt gttgaagaag agtttgcaaa 4860

tgcaacaaaa tatttaatta ccggttgtta aaactggttt agcacaattt atattttccc 4920

tctcttgcct ttcttatttg caataaaagg tattgagcca ttttttaaat gacatttttg 4980

ataaattatg tttgtactag ttgatgaagg agtttttttt aacctgttta tataattttg 5040

cagcagaagc caaatttttt gtatattaaa gcaccaaatt catgtacagc atgcatcacg 5100

gatcaataga ctgtacttat tttccaataa aattttcaaa ctttgtactg ttaaaaaaaa 5160

aaaaaaaaaa 5170

<210> 22
<211> 3249
<212> DNA
<213> Homo sapiens

<400> 22
caagcttagc ctggccggga aacgggaggc gtggaggccg ggagcagccc ccggggtcat 60

cgccctgcca ccgccgcccg attgctttag cttggaaatt ccggagctga agcggccagc 120

gagggaggat gaccctctcg gcccgggcac cctgtcagtc cggaaataac tgcagcattt 180

gttccggagg ggaaggcgcg aggtttccgg gaaagcagca ccgccccttg gcccccaggt 240

ggctagcgct ataaaggatc acgcgcccca gtcgacgctg agctcctctg ctactcagag 300

ttgcaacctc agcctcgcta tggctcccag cagcccccgg cccgcgctgc ccgcactcct 360

ggtcctgctc ggggctctgt tcccaggacc tggcaatgcc cagacatctg tgtccccctc 420

aaaagtcatc ctgccccggg gaggctccgt gctggtgaca tgcagcacct cctgtgacca 480

gcccaagttg ttgggcatag agaccccgtt gcctaaaaag gagttgctcc tgcctgggaa 540

caaccggaag gtgtatgaac tgagcaatgt gcaagaagat agccaaccaa tgtgctattc 600

aaactgccct gatgggcagt caacagctaa aaccttcctc accgtgtact ggactccaga 660

acgggtggaa ctggcacccc tcccctcttg gcagccagtg ggcaagaacc ttaccctacg 720

ctgccaggtg gagggtgggg caccccgggc caacctcacc gtggtgctgc tccgtgggga 780

gaaggagctg aaacgggagc cagctgtggg ggagcccgct gaggtcacga ccacggtgct 840

ggtgaggaga gatcaccatg gagccaattt ctcgtgccgc actgaactgg acctgcggcc 900

ccaagggctg gagctgtttg agaacacctc ggccccctac cagctccaga cctttgtcct 960

gccagcgact cccccacaac ttgtcagccc ccgggtccta gaggtggaca cgcaggggac 1020

cgtggtctgt tccctggacg ggctgttccc agtctcggag gcccaggtcc acctggcact 1080

gggggaccag aggttgaacc ccacagtcac ctatggcaac gactccttct cggccaaggc 1140

ctcagtcagt gtgaccgcag aggacgaggg cacccagcgg ctgacgtgtg cagtaatact 1200

ggggaaccag agccaggaga cactgcagac agtgaccatc tacagctttc cggcgcccaa 1260

cgtgattctg acgaagccag aggtctcaga agggaccgag gtgacagtga agtgtgaggc 1320

ccaccctaga gccaaggtga cgctgaatgg ggttccagcc cagccactgg gcccgagggc 1380

ccagctcctg ctgaaggcca ccccagagga caacgggcgc agcttctcct gctctgcaac 1440

cctggaggtg gccggccagc ttatacacaa gaaccagacc cgggagcttc gtgtcctgta 1500

tggcccccga ctggacgaga gggattgtcc gggaaactgg acgtggccag aaaattccca 1560

gcagactcca atgtgccagg cttgggggaa cccattgccc gagctcaagt gtctaaagga 1620

tggcactttc ccactgccca tcggggaatc agtgactgtc actcgagatc ttgagggcac 1680

ctacctctgt cgggccagga gcactcaagg ggaggtcacc cgcaaggtga ccgtgaatgt 1740

gctctccccc cggtatgaga ttgtcatcat cactgtggta gcagccgcag tcataatggg 1800

cactgcaggc ctcagcacgt acctctataa ccgccagcgg aagatcaaga aatacagact 1860

acaacaggcc caaaaaggga cccccatgaa accgaacaca caagccacgc ctccctgaac 1920

ctatcccggg acagggcctc ttcctcggcc ttcccatatt ggtggcagtg gtgccacact 1980

gaacagagtg gaagacatat gccatgcagc tacacctacc ggccctggga cgccggagga 2040

cagggcattg tcctcagtca gatacaacag catttggggc catggtacct gcacacctaa 2100

aacactaggc cacgcatctg atctgtagtc acatgactaa gccaagagga aggagcaaga 2160

ctcaagacat gattgatgga tgttaaagtc tagcctgatg agaggggaag tggtggggga 2220

gacatagccc caccatgagg acatacaact gggaaatact gaaacttgct gcctattggg 2280

tatgctgagg ccccacagac ttacagaaga agtggccctc catagacatg tgtagcatca 2340

aaacacaaag gcccacactt cctgacggat gccagcttgg gcactgctgt ctactgaccc 2400

caacccttga tgatatgtat ttattcattt gttattttac cagctattta ttgagtgtct 2460

tttatgtagg ctaaatgaac ataggtctct ggcctcacgg agctcccagt cctaatcaca 2520

ttcaaggtca ccaggtacag ttgtacaggt tgtacactgc aggagagtgc ctggcaaaaa 2580

gatcaaatgg ggctgggact tctcattggc caacctgcct ttccccagaa ggagtgattt 2640

ttctatcggc acaaaagcac tatatggact ggtaatggtt acaggttcag agattaccca 2700

gtgaggcctt attcctccct tccccccaaa actgacacct ttgttagcca cctccccacc 2760

cacatacatt tctgccagtg ttcacaatga cactcagcgg tcatgtctgg acatgagtgc 2820

ccagggaata tgcccaagct atgccttgtc ctcttgtcct gtttgcattt cactgggagc 2880

ttgcactatg cagctccagt ttcctgcagt gatcagggtc ctgcaagcag tggggaaggg 2940

ggccaaggta ttggaggact ccctcccagc tttggaagcc tcatccgcgt gtgtgtgtgt 3000

gtgtatgtgt agacaagctc tcgctctgtc acccaggctg gagtgcagtg gtgcaatcat 3060

ggttcactgc agtcttgacc ttttgggctc aagtgatcct cccacctcag cctcctgagt 3120

agctgggacc ataggctcac aacaccacac ctggcaaatt tgattttttt tttttttcca 3180

gagacggggt ctcgcaacat tgcccagact tcctttgtgt tagttaataa agctttctca 3240

actgccaaa 3249

<210> 23
<211> 4720
<212> DNA
<213> Homo sapiens

<400> 23
atgctgacaa tcttcttcct tcccctggcc acctctctgc ccacttgctt cctcagtacc 60

ttggtccagc tcttcctgca acggcccagg agctcagagc tccacatctg accttctagt 120

catgaccagg accagggcag cactcctcct gttcacagcc ttagcaactt ctctaggttt 180

caacttggac acagaggagc tgacagcctt ccgtgtggac agcgctgggt ttggagacag 240

cgtggtccag tatgccaact cctgggtggt ggttggagcc ccccaaaaga taacagctgc 300

caaccaaacg ggtggcctct accagtgtgg ctacagcact ggtgcctgtg agcccatcgg 360

cctgcaggtg cccccggagg ccgtgaacat gtccctgggc ctgtccctgg cgtctaccac 420

cagcccttcc cagctgctgg cctgcggccc caccgtgcac cacgagtgcg ggaggaacat 480

gtacctcacc ggactctgct tcctcctggg ccccacccag ctcacccaga ggctcccggt 540

gtccaggcag gagtgcccaa gacaggagca ggacattgtg ttcctgatcg atggctcagg 600

cagcatctcc tcccgcaact ttgccacgat gatgaacttc gtgagagctg tgataagcca 660

gttccagaga cccagcaccc agttttccct gatgcagttc tccaacaaat tccaaacaca 720

cttcactttc gaggaattca ggcgcagctc aaaccccctc agcctgttgg cttctgttca 780

ccagctgcaa gggtttacat acacggccac cgccatccaa aatgtcgtgc accgattgtt 840

ccatgcctca tatggggccc gtagggatgc cgccaaaatt ctcattgtca tcactgatgg 900

gaagaaagaa ggcgacagcc tggattataa ggatgtcatc cccatggctg atgcagcagg 960

catcatccgc tatgcaattg gggttggatt agcttttcaa aacagaaatt cttggaaaga 1020

attaaatgac attgcatcga agccctccca ggaacacata tttaaagtgg aggactttga 1080

tgctctgaaa gatattcaaa accaactgaa ggagaagatc tttgccattg agggtacgga 1140

gaccacaagc agtagctcct tcgaattgga gatggcacag gagggcttca gcgctgtgtt 1200

cacacctgat ggccccgttc tgggggctgt ggggagcttc acctggtctg gaggtgcctt 1260

cctgtacccc ccaaatatga gccctacctt catcaacatg tctcaggaga atgtggacat 1320

gagggactct tacctgggtt actccaccga gctggccctc tggaaagggg tgcagagcct 1380

ggtcctgggg gccccccgct accagcacac cgggaaggct gtcatcttca cccaggtgtc 1440

caggcaatgg aggatgaagg ccgaagtcac ggggactcag atcggctcct acttcggggc 1500

ctccctctgc tccgtggacg tagacagcga cggcagcacc gacctggtcc tcatcggggc 1560

cccccattac tacgagcaga cccgaggggg ccaggtgtct gtgtgtccct tgcccagggg 1620

gtggagaagg tggtggtgtg atgctgttct ctacggggag caggccaccc cctggggtcg 1680

ctttggggcg gctctgacag tgctggggga tgtgaatggg gacaagctga cagacgtggt 1740

catcggggcc ccaggagagg aggagaaccg gggtgctgtc tacctgtttc acggagtctt 1800

gggacccagc atcagcccct cccacagcca gcggatcgcg ggctcccagc tctcctccag 1860

gctgcagtat tttgggcagg cactgagcgg gggtcaagac ctcacccagg atggactggt 1920

ggacctggct gtgggggccc ggggccaggt gctcctgctc aggaccagac ctgtgctctg 1980

ggtgggggtg agcatgcagt tcatacctgc cgagatcccc aggtctgcgt ttgagtgtcg 2040

ggagcaggtg gtctctgagc agaccctggt acagtccaac atctgccttt acattgacaa 2100

acgttctaag aacctgcttg ggagccgtga cctccaaagc tctgtgacct tggacctggc 2160

cctcgaccct ggccgcctga gtccccgtgc caccttccag gaaacaaaga accggagtct 2220

gagccgagtc cgagtcctcg ggctgaaggc acactgtgaa aacttcaacc tgctgctccc 2280

gagctgcgtg gaggactctg tgacccccat taccttgcgt ctgaacttca cgctggtggg 2340

caagcccctc cttgccttca gaaacctgcg gcctatgctg gccgccgatg ctcagagata 2400

cttcacggcc tccctaccct ttgagaagaa ctgtggagcc gaccatatct gccaggacaa 2460

tctcggcatc tccttcagct tcccaggctt gaagtccctg ctggtgggga gtaacctgga 2520

gctgaacgca gaagtgatgg tgtggaatga cggggaagac tcctacggaa ccaccatcac 2580

cttctcccac cccgcaggac tgtcctaccg ctacgtggca gagggccaga aacaagggca 2640

gctgcgttcc ctgcacctga catgtgacag cgccccagtt gggagccagg gcacctggag 2700

caccagctgc agaatcaacc acctcatctt ccgtggcggc gcccagatca ccttcttggc 2760

tacctttgac gtctccccca aggctgtcct gggagaccgg ctgcttctga cagccaatgt 2820

gagcagtgag aacaacactc ccaggaccag caagaccacc ttccagctgg agctcccggt 2880

gaagtatgct gtctacactg tggttagcag ccacgaacaa ttcaccaaat acctcaactt 2940

ctcagagtct gaggagaagg aaagccatgt ggccatgcac agataccagg tcaataacct 3000

gggacagagg gacctgcctg tcagcatcaa cttctgggtg cctgtggagc tgaaccagga 3060

ggctgtgtgg atggatgtgg aggtctccca cccccagaac ccatcccttc ggtgctcctc 3120

agagaaaatc gcacccccag catctgactt cctggcgcac attcagaaga atcccgtgct 3180

ggactgctcc attgctggct gcctgcggtt ccgctgtgac gtcccctcct tcagcgtcca 3240

ggaggagctg gatttcaccc tgaagggcaa cctcagcttt ggctgggtcc gccagatatt 3300

gcagaagaag gtgtcggtcg tgagtgtggc tgaaattacg ttcgacacat ccgtgtactc 3360

ccagcttcca ggacaggagg catttatgag agctcagacg acaacggtgc tggagaagta 3420

caaggtccac aaccccaccc ccctcatcgt aggcagctcc attgggggtc tgttgctgct 3480

ggcactcatc acagcggtac tgtacaaagt tggcttcttc aagcgtcagt acaaggaaat 3540

gatggaggag gcaaatggac aaattgcccc agaaaacggg acacagaccc ccagcccgcc 3600

cagtgagaaa tgatcccctc tttgccttgg acttcttctc ccccgcgagt tttccccact 3660

tacttaccct cacctgtcag gcctgacggg gaggaaccac tgcaccaccg agagaggctg 3720

ggatgggcct gcttcctgtc tttgggagaa aacgtcttgc ttgggaaggg gcctttgtct 3780

tgtcaaggtt ccaactggaa acccttagga cagggtccct gctgtgttcc ccaaaggact 3840

tgacttgcaa tttctaccta gaaatacatg gacaataccc ccaggcctca gtctcccttc 3900

tcccatgagg cacgaatgat ctttctttcc tttctttttt tttttttttc ttttcttttt 3960

tttttttttg agacggagtc tcgctctgtc acccaggctg gagtgcaatg gcgtgatctc 4020

ggctcactgc aacctccgcc tcccgggttc aagtaattct gctgtctcag cctcctgagt 4080

agctgggact acaggcacac gccacctcgc ccggcccgat ctttctaaaa tacagttctg 4140

aatatgctgc tcatccccac ctgtcttcaa cagctcccca ttaccctcag gacaatgtct 4200

gaactctcca gcttcgcgtg agaagtcccc ttccatccca gagggtgggc ttcagggcgc 4260

acagcatgag aggctctgtg cccccatcac cctcgtttcc agtgaattag tgtcatgtca 4320

gcatcagctc agggcttcat cgtggggctc tcagttccga tttcccaggc tgaattggga 4380

gtgagatgcc tgcatgctgg gttctgcaca gctggcctcc cgcgttgggc aacattgctg 4440

gctggaaggg aggagcgccc tctagggagg gacatggccc cggtgcggct gcagctcacc 4500

cagccccagg ggcagaagag acccaaccac ttctattttt tgaggctatg aatatagtac 4560

ctgaaaaaat gccaagacat gattattttt ttaaaaagcg tactttaaat gtttgtgtta 4620

ataaattaaa acatgcacaa aaagatgcat ctaccgctct tgggaaatat gtcaaaggtc 4680

taaaaataaa aaagccttct gtgaaaaaaa aaaaaaaaaa 4720

<210> 24
<211> 1921

<212>  DNA
<213>  Homo sapiens

<400>  24
gaagcacaca ggaaaaggag cttagctgct ggtgctgctg gcaagatgga aaccaacttc      60

tccactcctc tgaatgaata tgaagaagtg tcctatgagt ctgctggcta cactgttctg     120

cggatcctcc cattggtggt gcttggggtc acctttgtcc tcggggtcct gggcaatggg     180

cttgtgatct gggtggctgg attccggatg acacgcacag tcaccaccat ctgttacctg     240

aacctggccc tggctgactt ttctttcacg gccacattac cattcctcat tgtctccatg     300

gccatgggag aaaaatggcc ttttggctgg ttcctgtgta agttaattca catcgtggtg     360

gacatcaacc tctttggaag tgtcttcttg attggtttca ttgcactgga ccgctgcatt     420

tgtgtcctgc atccagtctg ggcccagaac caccgcactg tgagtctggc catgaaggtg     480

atcgtcggac cttggattct tgctctagtc cttaccttgc cagttttcct ctttttgact     540

acagtaacta ttccaaatgg ggacacatac tgtactttca actttgcatc ctggggtggc     600

acccctgagg agaggctgaa ggtggccatt accatgctga cagccagagg gattatccgg     660

tttgtcattg gctttagctt gccgatgtcc attgttgcca tctgctatgg gctcattgca     720

gccaagatcc acaaaaaggg catgattaaa tccagccgtc ccttacgggt cctcactgct     780

gtggtggctt ctttcttcat ctgttggttt ccctttcaac tggttgccct tctgggcacc     840

gtctggctca aagagatgtt gttctatggc aagtacaaaa tcattgacat cctggttaac     900

ccaacgagct ccctggcctt cttcaacagc tgcctcaacc ccatgcttta cgtctttgtg     960

ggccaagact tccgagagag actgatccac tccctgccca ccagtctgga gagggccctg    1020

tctgaggact cagccccaac taatgacacg gctgccaatt ctgcttcacc tcctgcagag    1080

actgagttac aggcaatgtg aggatggggt cagggatatt ttgagttctg ttcatcctac    1140

cctaatgcca gttccagctt catctaccct tgagtcatat tgaggcattc aaggatgcac    1200

agctcaagta tttattcagg aaaaatgctt ttgtgtccct gatttggggc taagaaatag    1260

acagtcaggc tactaaaata ttagtgttat tttttgtttt ttgacttctg cctataccct    1320

ggggtaagtg gagttgggaa atacaagaag agaaagacca gtggggattt gtaagactta    1380

gatgagatag cgcataataa ggggaagact ttaaagtata aagtaaaatg tttgctgtag    1440

gttttttata gctattaaaa aaaatcagat tatggaagtt ttcttctatt tttagtttgc    1500

taagagtttt ctgtttcttt ttcttacatc atgagtggac tttgcatttt atcaaatgca    1560

ttttctacat gtattaagat ggtcatatta ttcttcttct tttatgtaaa tcattataaa    1620

taatgttcat taagttctga atgttaaact actcttgaat tcctggaata aaccacactt    1680

agtcctgatg tactttaaat atttatatct cacaggagtt ggttagaatt tctgtgttta    1740

tgtttatata ctgttatttc actttttcta ctatccttgc taagttttca tagaaaataa    1800

ggaacaaaga gaaacttgta atggtctctg aaaaggaatt gagaagtaat tcctctgatt 1860

ctgttttctg gtgttatatc tttattaaat attcagaaaa attcaccagt gaaaaaaaaa 1920

a 1921

<210> 25
<211> 2429
<212> DNA
<213> Homo sapiens

<400> 25
ctcttttcta agcttgtctc ttaaaaccca ctggacgttg gcacagtgct gggatgacta 60

tggagaccca aatgtctcag aatgtatgtc ccagaaacct gtggctgctt caaccattga 120

cagttttgct gctgctggct tctgcagaca gtcaagctgc agctccccca aaggctgtgc 180

tgaaacttga gcccccgtgg atcaacgtgc tccaggagga ctctgtgact ctgacatgcc 240

agggggctcg cagccctgag agcgactcca ttcagtggtt ccacaatggg aatctcattc 300

ccacccacac gcagcccagc tacaggttca aggccaacaa caatgacagc ggggagtaca 360

cgtgccagac tggccagacc agcctcagcg accctgtgca tctgactgtg ctttccgaat 420

ggctggtgct ccagacccct cacctggagt tccaggaggg agaaaccatc atgctgaggt 480

gccacagctg gaaggacaag cctctggtca aggtcacatt cttccagaat ggaaaatccc 540

agaaattctc ccatttggat cccaccttct ccatcccaca agcaaaccac agtcacagtg 600

gtgattacca ctgcacagga aacataggct acacgctgtt ctcatccaag cctgtgacca 660

tcactgtcca agtgcccagc atgggcagct cttcaccaat ggggatcatt gtggctgtgg 720

tcattgcgac tgctgtagca gccattgttg ctgctgtagt ggccttgatc tactgcagga 780

aaaagcggat ttcagccaat tccactgatc ctgtgaaggc tgcccaattt gagccacctg 840

gacgtcaaat gattgccatc agaaagagac aacttgaaga aaccaacaat gactatgaaa 900

cagctgacgg cggctacatg actctgaacc ccagggcacc tactgacgat gataaaaaca 960

tctacctgac tcttcctccc aacgaccatg tcaacagtaa taactaaaga gtaacgttat 1020

gccatgtggt catactctca gcttgctgag tggatgacaa aaagagggga attgttaaag 1080

gaaaatttaa atggagactg gaaaaatcct gagcaaacaa aaccacctgg cccttagaaa 1140

tagctttaac tttgcttaaa ctacaaacac aagcaaaact tcacggggtc atactacata 1200

caagcataag caaaacttaa cttggatcat ttctggtaaa tgcttatgtt agaaataaga 1260

caaccccagc caatcacaag cagcctacta acatataatt aggtgactag ggactttcta 1320

agaagatacc tacccccaaa aaacaattat gtaattgaaa accaaccgat tgcctttatt 1380

ttgcttccac attttcccaa taaatacttg cctgtgacat tttgccactg gaacactaaa 1440

cttcatgaat tgcgcctcag atttttcctt taacatcttt tttttttttg acagagtctc 1500

aatctgttac ccaggctgga gtgcagtggt gctatcttgg ctcactgcaa acccgcctcc 1560

caggtttaag cgattctcat gcctcagcct cccagtagct gggattagag gcatgtgcca 1620

tcatacccag ctaatttttg tattttttat tttttttttt tagtagagac agggtttcgc 1680

aatgttggcc aggccgatct cgaacttctg gcctctagcg atctgcccgc ctcggcctcc 1740

caaagtgctg ggatgaccag catcagcccc aatgtccagc ctctttaaca tcttctttcc 1800

tatgccctct ctgtggatcc ctactgctgg tttctgcctt ctccatgctg agaacaaaat 1860

cacctattca ctgcttatgc agtcggaagc tccagaagaa caaagagccc aattaccaga 1920

accacattaa gtctccattg ttttgccttg ggatttgaga agagaattag agaggtgagg 1980

atctggtatt tcctggacta aattcccctt ggggaagacg aagggatgct gcagttccaa 2040

aagagaagga ctcttccaga gtcatctacc tgagtcccaa agctccctgt cctgaaagcc 2100

acagacaata tggtcccaaa tgactgactg caccttctgt gcctcagccg ttcttgacat 2160

caagaatctt ctgttccaca tccacacagc caatacaatt agtcaaacca ctgttattaa 2220

cagatgtagc aacatgagaa acgcttatgt tacaggttac atgagagcaa tcatgtaagt 2280

ctatatgact tcagaaatgt taaaatagac taacctctaa caacaaatta aaagtgattg 2340

tttcaaggtg atgcaattat tgatgaccta ttttattttt ctataatgat catatattac 2400

ctttgtaata aaacattata accaaaaca 2429

<210> 26
<211> 2114
<212> DNA
<213> Homo sapiens

<400> 26
agaacatttc tttttcactt cccctttcag actccagaat ttgtttgccc tctagggtag 60

aatccgccaa gctttgagag aaggctgtga ctgctgtgct ctgggcgcca gctcgctcca 120

gggagtgatg ggaatcctgt cattcttacc tgtccttgcc actgagagtg actgggctga 180

ctgcaagtcc ccccagcctt ggggtcatat gcttctgtgg acagctgtgc tattcctggc 240

tcctgttgct gggacacctg ctcccccaaa ggctgtgctg aaactcgagc cccagtggat 300

caacgtgctc caggaggact ctgtgactct gacatgccgg gggactcaca gccctgagag 360

cgactccatt cagtggttcc acaatgggaa tctcattccc acccacacgc agcccagcta 420

caggttcaag gccaacaaca atgacagcgg ggagtacacg tgccagactg gccagaccag 480

cctcagcgac cctgtgcatc tgactgtgct ttctgagtgg ctggtgctcc agacccctca 540

cctggagttc caggagggag aaaccatcgt gctgaggtgc cacagctgga aggacaagcc 600

tctggtcaag gtcacattct tccagaatgg aaaatccaag aaattttccc gttcggatcc 660

caacttctcc atcccacaag caaaccacag tcacagtggt gattaccact gcacaggaaa 720

cataggctac acgctgtact catccaagcc tgtgaccatc actgtccaag ctcccagctc 780

ttcaccgatg gggatcattg tggctgtggt cactgggatt gctgtagcgg ccattgttgc 840

tgctgtagtg gccttgatct actgcaggaa aaagcggatt tcagccaatc ccactaatcc 900

tgatgaggct gacaaagttg gggctgagaa cacaatcacc tattcacttc tcatgcaccc 960

ggatgctctg gaagagcctg atgaccagaa ccgtatttag tctccattgt cttgcattgg 1020

gatttgagaa gaaaatcaga gagggaagat ctggtatttc ctggcctaaa ttccccttgg 1080

ggaggacagg gagatgctgc agttccaaaa gagaaggttt cttccagagt catctacctg 1140

agtcctgaag ctccctgtcc tgaaagccac agacaatatg gtcccaaata accgactgca 1200

ccttctgtgc ttcagctctt cttgacatca aggctcttcc gttccacatc cacacagcca 1260

atccaattaa tcaaaccact gttattaaca gataatagca acttgggaaa tgcttatgtt 1320

acaggttacg tgagaacaat catgtaaatc tatatgattt cagaaatgtt aaaatagact 1380

aacctctacc agcacattaa aagtgattgt ttctgggtga taaaattatt gatgattttt 1440

attttcttta tttttctata aagatcatat attactttta taataaaaca ttataaaaac 1500

aacattctgt ttaccttttc aaggctgtat tggttggagt gtagactgaa ctgcctgggg 1560

tctgtttctc ttcagtgatg agactcttag gaaggcagga atggatagga taggggaggg 1620

agaggagaga tggggattta gaatgtagag tgagtgcccc ttttcttaaa actgaataca 1680

gtcacgcacc acataatgat gtttagttca acaacagact gcatatatga tggtgatccc 1740

ataaaattat aataccatat ttctattgta ccttttctat tcctatgttt agatatatga 1800

gtacttacca ttgtgttaca attgcctaaa gtattcagta cagtagcatg ctgtacaggt 1860

ttgtagccta ggggcaatag gctatacgct acagcctagg tgtgtagtag gccacaccat 1920

ttaggtttgt ataagtacct gctatgatgt tcacacaaca aaattgcctg catttctcaa 1980

aatgtatccc catatttcaa caatgcatga ctgtactctt ctgccaatga ccttgtattc 2040

ttgtttccat gtcttcttct ctttcctcct atggcaaata aaacactgtt ttgcaacaca 2100

aaaaaaaaaa aaaa 2114

<210> 27
<211> 46115
<212> DNA
<213> Homo sapiens

<400> 27
   acatgataat tttcaaagaa agagtctttc tttcaaggaa agtcaggttc atggttacga 60
   agctgctgac cccaggatcc cagcccgtgg gagagaaggg ggtctctgac agcccccacc 120
   cctccccact gccagatcct tattgggtct gagtttcagg ggtggggccc cagctggagg 180
   ttataaaaca gctcaatcgg ggagtacaac cttcggtttc tcttcgggga aagctgcttt 240
   cagcgcacac gggaagatat cagaaacatc ctaggatcag gacaccccag atcttctcaa 300
   ctggaaccac gaaggctgtt tcttccacac agtactttga tctccattta aggtaaggtc 360
   ccccctccag ggtgggatga gggaaaaaga gggcaggcag gggaggcggt ggactgtggg 420
   ttcccaaatc caagctggca gacacggggc attggcatgt aacaggtttc tgctgagacc 480
   tgctgtccgt gtgggcacca caccacacat agaatgaccc ggtaccagag aagtatgttt 540
   gcgatctcat tcaccctttt gtaagtggat cggacgctga agccatcata gcatgttaga 600
   tgccttgggt ttataaaggt gatagcatta ggagtaattt gacagtgctt tctgcttttt 660
   ctgtgtgctt gctagaaaat gtacacacct acacatggct agagttctct gtttattaga 720
   aagcgctggt tcagttagtc aatgagctca cgcccagggc gagctgactg ccagccgggg 780
   agccgctttt ggggctggga ccccagcctg cttgtagtgt ttctcagtgg ttttcaaagg 840
   ctggcgggac tcagaatgtt ccagatggct ggtgaaacct caggtccctc ctagcagctt 900
   tattcacagt agccaagagg tggagacagc cctcgtaccc atctatagat gaatggaaaa 960
   atataatgtc catccacaca gtggaatatt acgcagccat gaaaaggaac aagcagcttt 1020
   attcacaata gccaagaggt ggagacagcc ctcataccca tctatagacg aatggataaa 1080
   tataatgtgg tccagccaca cggtggaata ttacacagcc atgaaaagga acaagcagct 1140
   ttattcacaa tagccaagag gtggagacag ccctcatacc catctataga cgaatggata 1200
   aatataatgt ggtccagcca cacagtggaa tattacacag ccatgaaaag gaacaagcag 1260
   ctttattcac aatagccaag aggtggagac agccctcata cccatctata gatgaatgga 1320
   gaaatataat gtggtccagc cacacagtgg aatattatac agccatgaaa aggaacaagc 1380
   agctttattc acaatagcca agaggtggag acagccctcg tacccatcta tagatgaatg 1440
   gagaaatata atgtggtcca gacacacagt ggaatattat acagccatga aaaggaacaa 1500
   gcagctttat tcacaatagc caagaggtgg agacagccct cataccccca tctatagatg 1560
   aagggagaaa tataatgtcc atccacacag tggaatatta cacagccatg aaaaggaaca 1620
   agcagcttta ttcacaatag ccaagaggtg gagacagccc tcatacccat ctatagatga 1680
   atggaaaaat ataatgtcca tccacacagt ggaatattac acagccatga aaaggaacaa 1740
   gcagctttat tcacaatagc caagaggtgg agacagccct cataccccat ctatagatga 1800
   atggagaaat ataatgtggt ccagccacac ggtggaatat tacacagcca tgaaaaggaa 1860
   taagcagctt tattcacact agccaagagg tggagagagt cctcataccc atctatagat 1920
   gaatggagaa atataatgtg gtccagccac acggtggaat attacacagc catgaaaagg 1980
   aacgaggctc tgccacaggt ggcaatgtgg gtgaatgctg aggacatcat gcttcctgag 2040
   agaggccaga tacaaaaaga tacatagtgt ctgtatgatt ccatttattc cctatgtcca 2100
   gatacaggca aatccacaga gacagaaagt ggatgagtgg ttgccacagg ctggggaaga 2160
   ggaaggacgg gtctttaatg ggtctggagt ctccttttgg ggtcgtgaca atgttctaga 2220
   agatagaagt ggtggttttt tttgagacag agtctcgctc tgtcgcccag gctggagtgc 2280
   agtggcgcga tctcagctca ctgcaacctc cacctcccgg gttcaagcaa ttctcctgcc 2340
   tcagcctcct gagtagctgg gactacaggc acccgcaact acgcctggct aatttttgta 2400
   tgtttagtag agactttgta tgtttagtag agtagtagag attttgtatg tttagtatgt 2460
   tttgtatgtt ggtcaggctg gtctcgaact cctgacctca agcaatccac ccccctcagc 2520
   ctcccaaagt gctgggatta taggcgtgag ccaccgcgcc tggccaatga aaatattaat 2580
   caataaatgt taagagcaca gatttcccaa cgttctgact caggcatctt cctggaccac 2640
   agcccctaca ggcaagtgtg actgtgtgtg gtgagcccat gagctgaaca caggctctgt 2700
   ccgaaaggag actctagaga ttgcctgttg ccttggaggt gggctgcggt ccctctgccc 2760
   aggcaggaag gggctccgag gcctctggaa cctcatttta ccttttccct gctgccctca 2820
   gtcccgtccc cagcctcaag gggctcagct ttttccaggc tcaccccttc catttataat 2880
   ttggagacaa gcaaggtcca ctgacgctaa cgccatttgg ttctttaaaa gatgttaggc 2940
   cgggcgcggt ggctcacgcc tgtcatccca gcactttggg aggccaaggc gggtggatca 3000
   tgaggtcagg agttcgagac cagcctgacc aacatggtga aacaccatct ctactaaaaa 3060
   tagaaaaatt agctgggtgt ggtggcggac accggtaatc ccatctactg gggaggctga 3120
   ggcaggagaa tcgcttgaac gtgggaggtg gaggttgcag tgaaccaaga tcgcgccact 3180
   gcactccggc ctggacaaca agagtgagac tccatctcag aaaaaacaaa acaaaacaaa 3240
   acaaaaaacc agaaaagaca gctgccaccc cagagcacag tgtaggagag ccagcctttc 3300
   tgggatgcgg cagaaagttc cagtgcagga ggctgaggtc ctgtaccccc ttgttacggt 3360
   aactgcagcc acttaggaat ttccgtgtgt tgatttcatc attgtaatta tacttccttc 3420
   cactgctttg tcatctgagc ttcctggtgt gccgcagagg atgtgttttc ctggtttgtg 3480
   gatacagctc gagccaagaa cgggctaaac ccccagggca gcaactcttt cttttctttt 3540
   cttttttttt tttttttttg agacggagtc tcgctctgtc gtccaggctg gagtgcagtg 3600
   gcgccatctc ggctcactgc aagctccacc tcccgggttc aactgattgt cctgcctcag 3660
   cctcccgagt agctgggatt ataggcacac tccaccacgc ctggctaatt tttgtatttt 3720
   tagtagagac ggggtttcac catgttagcc aggctggtct tgaactcctg acctcgggca 3780
   atctgcccac ctctgcttcc caaagtgctg ggattactgg catgagccac tgcacccagc 3840
   cggcagggct tctttcaaaa gggaacggct gacacacaca cagatgcatg cacatacatg 3900
   cacacataca cactgatgca aacacatttg tacacataca ttcacacatg catgcacata 3960
   aatatgcact tctaggcaca tgcatgcaca catacagact catgcatgta tatttgtgta 4020
   tgcacatata tttatgcaca tgtgtgtaca cgtggaatgc atgcacagat acatatgcat 4080
   tcaaacacaa agactgtgtg tacctgcatg catgaaaaca cacaaacaca gccgggcacg 4140
   gtggctcaca cctgtaatct cagcacttgg cgaggaaaag gcgggcggat cacctgaggc 4200
   aggagtccga gaccagcctg gccaacatgg tcaaatctct actaaaaaat acaaaaagtg 4260
   ggctggacgc ggtggctgac gcctgtcatc ccagcacttt gggaggctga ggtgggtgga 4320
   tcacctgagg tcaggagttc gagaccagcc tggccaacac ggtgaaaccc cgtctctgct 4380
   aaaaatacaa aaattagccg gacatggtgg tgggtgccta taatcccagc tactcgggag 4440
   gctgaggcag gacaatcggt tgaacctggg aggcggaggt tgcagtgagc ccagatcacg 4500
   ccattgcact ccagcctggg caacaagagc aaaactctgt cacaaaaaat aaataaataa 4560
   ataaataata caaaaattag ccaggcatgg tggcgagtgc ctgtaatccc agctactcag 4620
   gaggctgagg caggagagtc gcttgaacct gggaggcaga ggttgcaatg agccgagatc 4680
   atgccactgc actccagcct ggacgacaag ggcgagtctc catcaggaaa aaaaaaaaaa 4740
   gaaaacacac aaacacttat gtgtgcacac agatacacac gggcacaaaa atgtacacac 4800
   gtgcaagcac acacacacac agacacatgt acacagtacc cgtgtgtgca caggcatgca 4860
   tgcagtcctg caaatgtgca aacagacatg gctacataga ggcatgcaca catatagacc 4920
   catgtacaca cacacgaata ttcgtgcaca catgtagaag cacactctga tgtgcatgca 4980
   tgtgcaaagg cgcatttgca cacagacaca tatgcacact caccaccgct ttccctccag 5040
   aagcagctcc ttctgctgtg agcaccaggc tctggaaggg gcaagcatcc ttcattaccc 5100
   gcaacccagt ttcacagcca gtccccgctg cctgactctc gtttctcctg cagcaggcac 5160
   ctctgtcctg cgttccggag ctgcgttccc gatggtcctc ctttggctca cgctgctcct 5220
   gatcgccctg ccctgtctcc tgcaaacgaa ggaaggtaag aactggagaa aaaatgcacg 5280
   tgccacctgg ggagcggtgg gggtagacag acacacaatg tcagcgtgcc gtccttcagg 5340
   gaaacttttc atgctgagct catggcagag tctcatgcag tggtcgggaa tgactcagac 5400
   acttccctgt acccgtcacc aagttccctg taatgcagca tcttgcaaaa cggtaatacg 5460
   acctcacagg cagggacctg atcgtgacac agatgccatg tgaggtgttt tgatgaaact 5520
   cacacgctgg gatcaacagc accaataaca atttccagtt tccttcattg tttatcttac 5580
   tttttcattt tcttattatt tttattttgg agacagggtc ttactgtctt gcccaggctg 5640
   gagtgcaata gtgtgatctc ggctcactgc aacctctgcc tcccggattc aagcgattct 5700
   tctgcctcag cctcccgagg agctgagatt acagtcgcgc accaccatac ccggctaatt 5760
   tctgtatttt tgatagagac gggatttcac tatgtcggtc aggctggtct agaactgctg 5820
   accacaagtg atccgtctgc cttggcctcc caaagtgctg ggattacagg tgtgagccac 5880
   agcgcccggc cttttttttc attggttttt acagtctatt actgcagggt ttcactttac 5940
   cttgaatttc ttttaacttt aatttgcttt tcattcttta acttcttttt tttttttttt 6000
   tttgacacag agtttcattc tggtcgccca ggctggagtg taatagcgtg accatggctc 6060
   actgcaacct ctacctgctg ggttcaattg attctcctgt ctcagcctcc caagcacctg 6120
   ggattacagg tgtccgccac cacgcccagc taatttttct gtttttacta gagacggggt 6180
   ttcaccgtgt tagacaggat ggtctcgatc tcctgacctc atgatccgcc tgcctcgggg 6240
   ttgggattac acactttggg aggccaaggc aggtggacga tcacaaggtc aggagttcgt 6300
   gaccagcctg actaacacgg tgaaaccccg tctctactaa aaatacaaaa atcagctggg 6360
   cgtggtggcg ggcgcctgta atcccagtta ctcgggaggc tgaggcagga gagtcgcttg 6420
   aacccgggag gaggaggttg cagtgagcct agatcacgcc attgcactcc agcctgggcg 6480
   acagagtgag actccgtctc aaaatgaatg aatgaatgaa tgaatttctt ataagaattt 6540
   ttttccccag acagtttgtt tttaaggata aattatcctt ctaagttaga agaaaataat 6600
   gccagaagtc tagacattct tatgctttgg tcccgcttat caaaccaagg ttgctgacct 6660
   tgataaccac tcaagatcct tacagtttat aaagtcattt cctcaagttt tctaagtggc 6720
   cgatcagaga taaaccctag agaaatagtt gatgtatgtt tctagctttg ggtgaccagc 6780
   aaaatgtgat agaatattgc cttttactgg ccgggtgcaa cggctcacgt ctgtaatccc 6840
   agcactttgg gaggctgagg cgggtggatc acttgaggtc aggagttcaa aaccagcttg 6900
   gccatcatgg tgaaaccccg tctgtactaa aaatacaaaa aaattagctg ggcgcggtgc 6960
   tgtgcacctg taatccctgc tactcaggag gctgaggcag gagaatcact tgaacccggg 7020
   aggcggaggt tgccgtgagc caagatcacg ccattgcact ccaacctggg tgacagagcg 7080
   aggctccatc tcaaaaaaaa aaaaaaaaaa agaatattgc ctttaacatc tttgtacagg 7140
   tcatttatga aatatcttga gctctgtgat ggctaagaga gaccttcttt ttctttcttt 7200
   cttttttttt tttttgagac ggagtttttt tgtttttttg agatggagtc tcgctctgtt 7260
   acccaggctg gagtgcagtg gcacgatctc agctcaccgc aacctccgcc tcccgagttc 7320
   cagtgattct cctgcctcag cttcctgatt agctgggatt acaggcgccc gccaccacgc 7380
   ccaactaatt tttgtatttt tagtcgcgac ggggtttcac cgtgttagcc aggatggtct 7440
   tgatcacttg acctggtgat ccgcccacct tggcctccca aagtgctggg atgacgggcg 7500
   tgagccacca cgcccgtttg ctttatgact tctaccagct cacagaagtc tcctgtgtac 7560
   atagaactcc acttcccagc caggctcagt aactcacgtc tgtgatccca gcactttggg 7620
   aggctgaggc aggcagatca tgtgaggttg ggagttcgag accagcctgg ccaacatggt 7680
   gaaaccccat ctctagtaaa aatacaaaaa ttagccgagt gtggtggcag gcacctgtta 7740
   tcccagctgc acagcaggct gacacaggat aatcgcttga acccgggagg cggaggttgt 7800
   agtgagccga gatcgcgcca ctgaattcca gcctgggcga cagagtgaga ctccgtctca 7860
   aaacaaacaa gcaaacaaaa atacccatta caatgttgtt ttaagattgt tgtatatcaa 7920
   ctgggcatgg tggctcatgc ttgtaatccc agcactttga gaggccgagg cggacagatc 7980
   acgaggtcag gagatcgaga ccatcctggc caacatggtg aaaccccatc tctagtaaaa 8040
   atacaaaaat tagccgggcg tggtggtggg ctcctgtaat cctagctact cgggaggctg 8100
   aggcaggaga atcgcttgaa cccgggaggt agaggttgcg gtgagctgag atcgtgccac 8160
   tgcactccag cctgggtgac aagagcaaaa ctccgtctca aaaaaataaa aaatttaaaa 8220
   aaataaagaa ctcgacctcc caaaggtatt ggctaactcc acgggcaaaa aaaccatacc 8280
   cattacaatg ctgttttaag attgttgacc tggtgcggtg gctcatgcct gtaatcccag 8340
   cactttggga ggctgaggcg gacgaatcat gaggtcagga gatcgagacc atcctggcta 8400
   acacggtgaa acgccatctc tactaaaaat acaaaaaaaa aaaaattagc cgggcgtggt 8460
   ggcgggcgcc tgtagtccca gctactcggg aggctgaggc aggagagttg cttgaaccag 8520
   gaggcggagc ttgcagtgag ctgagatcgt gccactgtag tccagccagg gcgacaagag 8580
   tgaaactcca tgtcaaaaaa tttaaaaaaa ttaaataaaa gaactccacc tcccaaaggt 8640
   attggctaac tccacgggca aaaaaaaaaa accatacccc ttacaatgcc gttttaagat 8700
   tcttacgtat ctcttcgaac tccaacctgt caccgtttta gatccaaacc caccaatcac 8760
   gaacctaagg atgaaagcaa aggctcagca gttgacctgg gaccttaaca gaaatgtgac 8820
   cgatatcgag tgtgttaaag acgccgacta ttctatgccg gtaaatcata ctctctattg 8880
   tttttttatt tttattttat ttatttatgt atttatgtat ttatttattt tttgagacgg 8940
   agtcttgctc tgtcgcccag gctggactgc ggtgacccga tctccgctct ctgcaacctc 9000
   cacctcccag gtccatgcca ttctcctgcc tcaacctccc gagtagctgg gactacaggc 9060
   gcccgccacc atgcccggct aatttttttg tatttttagt agagatgggg tttcactgtg 9120
   ttagccagga gggtctcgat ctcctgacct cgtgatctgc ccgcctcggc ctcccaaagt 9180
   gctgggatta caggcgcgag tcaccgcgca tggcccagac tctctaatgt tgacgaacaa 9240
   gacgtttccg tcttctgcag gaatctcaga accaatactg ctcccatcgc tggtgtcatg 9300
   actacctggt ttctgccccg aagtcaggtg tgggatttga agtgactttg gagggtctgg 9360
   ttccccgtcc gcgggacatc taagatggca cacactggac agggtggatg tgaaagtgaa 9420
   atgaggctaa gctatgactg gtgcgaaacc caaccccacg ctgggcgtgg tggctcacgt 9480
   ctgtaatccc agcactttgg gaggctgagg cgggcggatc atgaggtcag gagttcgaga 9540
   ccagtctggc caacacggtg aaacgctgtc tctgctaaaa atacgaaagt tagccgggca 9600
   cagtggctca caccagcact ttgagaggct gaggcggatg gatcacctgg ggccaggagt 9660
   tcgagactag tctgggcaac atggtgaaac cccgcctcca ctaaaaatat aaaaattagc 9720
   tgggcatggt ggtgggcgcc tgtaatccca gttacttggg aggctgaggc aggagaatcg 9780
   cttgaacccg ggaggcagag gttgcattga gccgagactg tgccactgca ctccagcctg 9840
   ggggacgaga gcaagacttc atgtcaaaaa agaaaaagaa aaattagccg gatgtggtgg 9900
   cacatgcctg taataccagc tattcaggag gctgaggcag gagaattgct tgaacctggg 9960
   aggccgaggt tgcactgagc cgagattgtg ccactgcact ccagcctggg tgacagagtg 10020
   agactccatc tcaaaacaaa aacaaaaaca aaagcaaaaa caaaacaaaa gtgtgtgctc 10080
   aggaaacaag gtcctcatca cgaaatcctt ccaaatcccc catcttgtca tcacctgcgt 10140
   tctcagggtt tgagaacagc gccagacctc atggggtggc ccaggtgaca ctgtgagcta 10200
   tttacaagtc agtgtcttat gggaaaggag cacgtttccc tgagaaccta tttggtcccc 10260
   tccaagagct atgttcgttc aatacaattc aaatcacggc ccttcatgcg tctgctcggg 10320
   ccaccattat aaaatcctac ccccagctcc aaatacagtc ccattgaact ttgtgatttt 10380
   ggagagtaga gataaaacag tctagaatcc cagagcgatt ttaccatacc atggcaaact 10440
   gactctcaac tttagaaaca caaatgctga aaaaaaaact aaggaaattt tgaaaaagaa 10500
   ggtgaatgaa ggagaacctg ccttacctat atcaaaaggc actgaaaagt tcatacccaa 10560
   tgtgcggatt gctataagaa tacacaagta ggccgggcgt ggtggctcac gcctgtcatc 10620
   ccagcacttt ggaggccgag gcgggtggat cacgaggtca ggagatcgag accctcctgg 10680
   ctaacacggt gaaaccccgt ctctactaaa aatataaaaa ttagccgggc gtggtggctg 10740
   gtgcctgtag tcccagctac tcgggaggct gaggcaggag aatggcttga acctgggagg 10800
   cagaagcttg cagtgagccg agatcgcgcc actgcactcc agcctgggcg acagagcgag 10860
   actctgtctc aaaaaaacaa aaacaaaagc aaacaaaacg aagaatatac aagtagatta 10920
   atgaaatgtg gccgggtgcg gtggtgaggc aggagaattg cttgaacccg agaggtggag 10980
   gttgcagtga gctgagatcg caccactgaa gtctagcctg ggcaagcgga gtgaggccct 11040
   gtcttaacaa aaaaacaaag aaacaaaaaa caaacaaaca agaaaaaaac aaagcaaaac 11100
   aaacagaaaa gtatttcact aatatttact gctaagtggg attattttta ttcaagcttt 11160
   tgtatcttta gaaaaaaatt gtggccgggc gcggtggctc acgcctgtca tcccagcact 11220
   ttgggaggcc gaggagggtg gatcacgagg tcaggagatc gagaccctcc tggctaacac 11280
   ggtgaaaccc cgtctctact aaaaatataa aaattagccg ggcgtggtgg cgggcgcctg 11340
   tagtcccagc tacttgggag gatgaggcag gagaatggca tgaacccggg agggagaggc 11400
   tacagtgagc cgagatcgcg cccctgcact ccagcctggg cgacagagcg agactccctc 11460
   tcaaaaaaaa aaaaaacaga aaaaagtctt ggccgggcac ggtggctcac gcctgtaatc 11520
   ccagcacttt gggaggctga ggcaggcgga tcacatgagg tcaggagttc gagaccagcc 11580
   tgaccaacat ggtgaaaccc tgtctctacc cagaaaaata ctttaaaaat tagctgggcg 11640
   tggtggcggg cacctgtaat cccaggtgct cgggaggttg aagcaggaga atggcttgaa 11700
   cccgggaggg agaggctgca gtgagccgag atcacgccac tgcactccag cgtgggcgac 11760
   gagagcgaaa ctctgcctca aaaaaaatct gaacatcatt agcgtcaaat taagcatggt 11820
   ctgtcagcag ccatcatagt cctatgtctc tcttaggcag tgaacaatag ctattgccag 11880
   tttggagcaa tttccttatg tgaagtgacc aactacaccg tccgagtggc caacccacca 11940
   ttctccacgt ggatcctctt ccctgagaac agtgagaaaa atgttcattg tttgtttatt 12000
   ctctattccc tccctccttc cctctctccc tccctctcgc cttcgctgtg tcttttttct 12060
   tttctttttc tctttctttc tttctttctt tctttctttc tttctttctt tctttctttt 12120
   tctttctttc tgtttctgtt tctttcttcc tttctttttc tttctttctt tccttctttc 12180
   ctctctttct tttctttctc tttccctccc tcccttcttt cttttcttca cttccttccc 12240
   tccctccttc tctccttttc ctccctcctc cttcccaccc tacttcctct ctctccttcc 12300
   ttccctttcg ttttcttttc ctccctgcct cactcccttc cttccttctc tccctcctct 12360
   tttccttcct tcttccctcc ttccctttct ctctctctct ctttctctct ttccccctcc 12420
   cctcccttcc cctcctctcc cctctccttt cctgacacgg tcttgctctg ttgcccaggc 12480
   tggagcgcag tggtgcaatc acagcttact gcaggcttca cctcctgagc tcaaacaatc 12540
   cttctgcctc agcctcccac gtaggtggaa ctacacccat gtaccaccat gcccacctaa 12600
   ttttttaaac accttttttt ttttttagac agagtctcac tctgtccacc aggctggagt 12660
   gcactggcgc gatctcggct cactgcaacc tctggctccc gggttcaagc gattctcctg 12720
   cctcagcctc ccgagtagct gggattatag gcgcccaccg ccacgcccag ctaattcttg 12780
   tatttttagt agagatgggg tttcaccatg ttggtcaggc tggtctcaaa ctcctgactt 12840
   cgtgatctgc ccgcctcggc ctcccaaagt gctgggagta caggggtgag ccaccgcgcc 12900
   cagccttaaa tactttttgt agagatgggg tgtcgctatg ttacctgagc tggtctcaaa 12960
   ctcctaggct caagtgatcc tcccgcctca gtctcccaaa gtgcagggat tgtaagcatc 13020
   agccaccacg cctggcctct tctttgtttt ttaattaatt agaagtccat ggtttatttt 13080
   tttgttttgt tttgtttgtt tttctgtgat ggagtcttgt tcttgtcgcc caggctggag 13140
   tgcagtggtg cgatttcagc ttaccgcaaa ctccgcctcc cgggttcaag cgattctcct 13200
   gcctcagcct cacaagtagc tgggattaca ggcacccgcc accaagcccg gctaaatttt 13260
   gtattttaag tagagacggg gcttcaccat gttggtctcg aactcctgac cttgtgatcc 13320
   acccgcctca gcctcccaaa gtgctgggat gacaggcggg agccactgtg ccgggcctca 13380
   atttaattat ttttaagttt aatggatttc cagggagtta tgccgagtcg gaaaaaaaag 13440
   accactccag agcgtgacac acacagtgat ttcattaata caactgtctt gaaatggcaa 13500
   aattttacaa atagaaaaca gtctcctggg ttgcagggat ataagcagga gtgagagctg 13560
   agagaggtgg gtttggctgt aaaagtgctc cttggggagg ccgggcgcag tggctcacgc 13620
   ctgtaatccc agcactttgg gaggccgagg cgggcggatc acctgaggtc aggagttcga 13680
   gaccagcctg gccaacatgg tgaaaccccg tctctactaa aaacacaaaa attagccggg 13740
   catggtggct cacgcctgta atcccagcac tttgggaggc cgaggcaggc ggatcacctg 13800
   aggtcaggaa ttcgagacca gcctggctaa catggtgaaa ccccatctct actaaaaata 13860
   caaaattagc caggggtggt ggcacatgcc tgtagtccca gctactcagg aggctgaggc 13920
   aggagaatca cttgaacccg ggaggcggag attgcggtga gctgagatcg tgccattgct 13980
   ctccagcctg ggcaacaaga gtgaaattcc atctaaaaaa aaaaaaaaat taaaaagaaa 14040
   ttagcggccg ggcccagtgg ctcacacctg taatcccagc actttgggag gccgaggtgg 14100
   gtggatcgcg aggtcaggag atcgagacca tcccggctaa catggtgaaa ccccatctct 14160
   actaaaaata caaaattagc caggggtggt ggcacatgcc tgcagtccca gctactcagg 14220
   aggctgaggc aggagaatca cttgaacctg ggaggcggag gttgtggtga gccattgagc 14280
   tccagcctgg gcaacaagag tgaaactctg tcttaaacac acacacagat gcacacacac 14340
   gcacacacac acaaacaatg aaagaaaaac atctgtagtc ccagcagttt gggaggctga 14400
   ggcgggtgga tcacttgagg tcaggaattc gcgaccagtc tgggcaacat ggtgaaaccc 14460
   catctctact aaaaatacaa aaattagctg ggtgtggtag tgggtgcctg ttgtcccaga 14520
   tacttgggag gctgaggcag gagaattgct tgaacccggg aggtggaggt tgcagtgagc 14580
   cgagatggcg ccattgtact ccagcctagg cgacggagcg agactctgta aaaatgaata 14640
   aataaatcat taaaagaata tgcaggatct ctccatatta ttattatttt ttacaacggc 14700
   ttgtgtatct accatggtct caaaatacaa aaggcactct aaaaaggaat tagagtttta 14760
   tttttttttt tgagacggag tctcgctctt gtcacacagg ctggagtgca gtgacgtggc 14820
   ctgggctcac tgcaacctct gcctcctggg ttcaagaaat tctcctgcct cagcctccct 14880
   agtagctggg attacagcgg cccgacatca cgcccggcta atttttgtat ttttagtaga 14940
   ggcagggttt caccatgtta gccaggccgg tctcgaactc cggacctcag gtgatcctcc 15000
   tgcctcggcc tcccaaagtg ctgggattac aggcgtgagc caccatgcct gggtttgttt 15060
   ttttattttt ttgagacgaa ggctcactct gtcgcccagg ctggagtgca gtggcgtgat 15120
   ctcagctcca tgcaacctct gcctcctggg ttcaagaaat tctcctgcct cagcctcccg 15180
   agtagctggg attacagcgg cccgacatca cacctggcta attttttata tttttagtag 15240
   agactgggtt tcaccatgtt agccaggctg gtctcgaact ccggacctcg ggtgatccac 15300
   ctgcctcggc ctcccaaagt gctgggatca caggcgcgtg ccaccagaca cagctaattt 15360
   ttgcattttt agtagagatt gggtttctcc atgttggcca ggctggtctc gaactcctga 15420
   cctcatgtga tccacccgcc tcggcctccc aaaatgctgg ggtgacaggc gtgagccacc 15480
   gcgcccggtc ccgattcgag ttctctttca tgtttgtgaa cccaggtggg aagccttggg 15540
   caggtgcgga gaatctgacc tgctggattc atgacgtgga tttcttgagc tgcagctggg 15600
   cggtaggccc gggggccccc gcggacgtcc agtacgacct gtacttgaac gttgccaagt 15660
   aggtgtgccc gtgggcagag gccgggctgt ccctggtgcg ggtgccatcg gcgtggggtc 15720
   gtcccccaac cttaccgctt accgcagcag gcgtcaacag tacgagtgtc ttcactacaa 15780
   aacggatgct cagggaacac gtatcgggtg tcgtttcgat gacatctctc gactctccag 15840
   cggttctcaa agttcccaca tcctggtgcg gggcaggagc gcagccttcg gtatcccctg 15900
   cacagataag tttgtcgtct tttcacagat tggtgagtag cccgggacac tccctcccac 15960
   cctcagttct gtgataccac ggctttagcg ccaggccaga tcccacggga ccacgtggct 16020
   cccaacgcag acgttggcct ctcacatttc cagaggctgg acgttggagg tcagcgtgct 16080
   ggctggctgg gctcctcggg aggtctcttc ctggcttgga gagagggtca tcttctcact 16140
   gtgtcttcac gtggtgtaga gagagagaga gatgaagttc tggggtctct tgttagaagg 16200
   gcactaacac catcatgggt cccatcatgg gtcataggat ccctcaccat gggtcatggg 16260
   accccccatc atgggttcca tcatgggtcc catcatgggt catagaatcc cccatgatgg 16320
   gtttcatcat gagtcataga accccctatc atggattcca tcatgggtca tgggaacccc 16380
   catcatgggt cccatcatgg ttcataggat cccccatcat gggtttcatc atgagtcata 16440
   gaacccccta tcatggattc catcatgggt catgggaacc cccatcatgg gtcccatcat 16500
   tggtcatagg accccctatc atgggttcca tcatgggtca tgggacaccc cccatcatgg 16560
   gttccaccat gagtcatggg atccctcatc gtgggttcca tcatgggtca tgggaccccc 16620
   catcatgggt tccatcatgg gtaataggac ccccccatca tgggttccac catgagtcat 16680
   gggatccctt atcatggatt ccatcatggg tcatgggacc ccctatcatg gattccatca 16740
   tgggtcatgg gaccccccca tcatgggtcc catcatgagt catgggatcc ctcatcatgg 16800
   gtttcatcat gggtcatagg acccccatca tggattccat catgggtcat gggaacctcc 16860
   atcatgggtc ccattatggg tcatagaatt ccccatcatg ggttccatca tgagtcatgg 16920
   gaccccccat catgggttcc acatgggatc cctcatcatt ggttccatca tgggtcatag 16980
   gatcccccat cataggtccc atcatgggtt ccacatggga tcccccatca taggtcccat 17040
   catgggtcat gggacccccc ccatcatggg ttccatcacg ggtcatggga ccccccccat 17100
   catgggttcc atcacgggtc atgggacccc ccccaatcat gggttccatc atgggtcatg 17160
   ggaaccccca tcatgggttt catcatgggt cataggatcc cctatcatgg gttccatcat 17220
   gggtcatggg acccccccca tcacgggttc catcatgggt catgggaacc cccatcatgg 17280
   gtttcatcat gggtcatagg atcccctatc atggattcca tcatgagtca tgggatccct 17340
   catcatgggt tccatcatgg gtcatgggac cccccccccc atcatgggtc gtgggacccc 17400
   cacccccatc atgggtcatg ggagccccca ttgtgagtca tggaatccct catcatgggt 17460
   ctcatcatgg gccatgggtc ccaccatggg tcatgggagc ccccatcatg agtcatggaa 17520
   tccctcatca tgggtcatgg gtcccatcat gggtcatggg agcccccatc atgggtcatg 17580
   ggtcccatca tgggtcacgg gagcccccat catgggggtc caccctcata acttcagccc 17640
   acgccagtca cctcccaaag accccacctt ctaacaccgc ccaaccaggg gttagagctt 17700
   cagtggagga atttgggaca gaaggacaca cactttcagt ccacgatacc cgagctccga 17760
   ggaacctccc aggtggtgag aatgtcaata tgcccagagc tgacgtgccc tgaacccaag 17820
   ggcagggtgc tcggatgctt cagaagagga gggggaaacg aggaagagga ggagaaggac 17880
   aaggagaaga gaagggagga gaggaagaac atgagcaggg ggaggaggag gagaaggaaa 17940
   agaaacagag gaaaaggagg gggaggaggt agagatggag agggaaggaa gaggaggaag 18000
   agaagaaaac ggagaaagag gagggggagg aggaggagga agagaaaatg gagaaagagg 18060
   agggggagga ggtggagatg gggaggggag gaagaggagg aagagaagaa aacaggaggg 18120
   ggaggaagag aagaaaatgg agggaaagga gggggaggag gtggagatgg ggagagaagg 18180
   agcaggagga ggagaagaaa gaagaggaag aaaggaggag gagagggtgg aggaggaaga 18240
   gggtgaagga gagagggagg agaatggaga ggaggaggag gcgggggagg gaagagaagg 18300
   aggtgaggag gaggaagggg aggatagaga aggtggggta gaagggtggt gaggtgggga 18360
   gggagaagga gggggaggag gagagaggag gactgggggg aggaaggggg aggaggggag 18420
   agaggaggag ggaggaggga gaaggagggg gagaggatgg aggaagggga gcagatggag 18480
   gaaggcgagc aggaggggga ggaaaaagga ggggcaggag gaagaaggag cgggaggaga 18540
   ggaggaggag gaggaggaga atgggcaggg aggaggagag ggaagaaaag gagggggagg 18600
   aggaaggagg gggaggaggg tggaggggga ggagggtgga gggggaggag ggtggagggg 18660
   gaggaggaag gagggagaag gaggggagag gatggaggaa gaggagcagg agggggagga 18720
   aaaaggagga ggggcaggag gggaggagga gaaaggagag tggaggagga gggtgacaag 18780
   gagaatgggg aggagagagg agtggtagaa aaaggagaga gaggaagagt aggagcggag 18840
   ggggaggagg ggaggggagg ggaggggaga aagaccagga gggtaggagg agggggagga 18900
   ggaggagggg gccagggctg gacttccttc caggggccca ggggagaccc agccaggcag 18960
   ccggacacac agggctgggc tggtagtcgg gtcacacctg aggatgtgga agtgcctggg 19020
   gttcccggga agtggaggat gtcctgggcc actgggagag gcggcacatc ccctgggggg 19080
   cggaggtggg ggaggtgaga agccaggcag gggccaggaa gtggaagctt caggaggatg 19140
   tgtgcaggtc aaagtgtagg agtctgcatc ttgaatgatg cagagagggc ctccaaggct 19200
   ccagtgtgct gcaggtgggc agagggggca tgggaagtag gggttggccc tgggcagggg 19260
   tggggagtgg gccaggctgc ccagcggggc tgagcctaga gatggaaggg gcagggacaa 19320
   agatgtgcag ctgccagtcc ttggaaaagc tgaacgcctg gtacttcaga gaacaaaagg 19380
   gtggttctga aggctgctcc caggacagtg gggctccgag ggtgcaaccc caaggctcac 19440
   tcctcccagt gcccccaacg cggctcagtc ctgtgtctct gcctgtagag tttctttgtt 19500
   ccctcttcct tcctggtgtt tttctctccc gctctccaaa tgcataggag aagtaatttg 19560
   aagtatctcc agaaaaaaaa agagaaaaag aaaaagaatt gatttcttgt actcctaaat 19620
   cctaaaagtg tttttctcgt tgctagagat attaactcca cccaacatga ctgcaaagtg 19680
   taataagaca cattccttta tgcactggaa aatgagaagt catttcaatc gcaaatttcg 19740
   ctatgagctt cagatacaaa aggtaaactt tcaccccgcc cccagccccc ccacccccgt 19800
   ggacatccct tatttttggt aagtcgcact ctggggcctt gaaacgggca acaatctcct 19860
   ctgataacgt cacagaaggc atggatcatt aaaaaacaaa aacaaaaaca aaaggccggg 19920
   cgctgtggct cacgcctgtc atcccagcac tttgggaggc tgaggcaggt ggatcacaag 19980
   gtcaggagat cgagaccatc ctggctaacg cggtgaaacc ccgtctctac taaaaatacg 20040
   aaaaaaaatt agctgggcgt ggtggcacgg tcttgtagtt ccagctactg gggaggccga 20100
   ggcaggagaa ttgcttgaac ccaggaggtg ggggttgcag tgagccgaga tcgcgccatt 20160
   gcactccagc ctgggcgaca gagcgagact ccgtctcaaa aaaaaaaaaa aagtttgaga 20220
   ctgtatgtgg tctgttgttt tttattttta ttatttctat tgttatgtgg ttttttaatt 20280
   ttttcttgaa ctttcttttt tcttttgtag tgatctacag attcaatgca agcttcccag 20340
   atatttttga tccacagttc gttggatcag ttgaacataa atattgattt atttattgag 20400
   ccactctggc tctgtagccc agactggagt gcagtggctt aaatcttgac tcactgcaac 20460
   ctctgcctcc cgggttcaag tgattctcct gcctcagcct cccgagtagc tgggattatg 20520
   ggcacccatc accacaccca gctaagtttt tgtattttta gtagaaacag agtttcatca 20580
   tgttggccag gctggtctca aactcctgac ctcaggtgat ccacccgcct cggcctccca 20640
   aagtgctggg attacaggtg tgagccttcg tgcgcagcct atgtgttata tttagtttgt 20700
   attttatttt attgtatttt attatttatt tatttatttt tgagacggag tcctgctctg 20760
   tcacccaagc tgtttgtttg tttgtttgtt tgtttattat ttttgagacg gagtcctgct 20820
   ctgttgccca ggctggggtg cagtggcatg atctcagctc actgtaacct ccgcctctcg 20880
   ggttcaacca attctcagct tcagcctcct gagtagctgg aattacaggc acctgccacc 20940
   atgcctggct aatttttttt tgtattttta gtagagatgg ggtttcacca tcttgtcctg 21000
   gctggtcttg aactcctgac ctcatgatcc acccgcctca gcttcccaaa gtgctgggat 21060
   tacaggcgtg aggcactgtg ccccgccata tatttattat ttatgctcaa atactaatta 21120
   tttcatatgc aatttttctg taagtctaaa tctgctaaaa aacgttaggt ctattaattt 21180
   cttttatatt accaagtgtt ttttagccaa tctgtttggg tttttttttt ttttaagaaa 21240
   ataaatggct gggtgcagta gcttacgcct gtaatcccag cactttggga ggccgaggca 21300
   ggacgatcac gaggtcagga gatagagacc atcctggcta acatggtaaa accccgtctc 21360
   tactaaaaat acaaaaaaaa attagctggg cgtggtggcg ggcgcctgta gtcccagcta 21420
   cttgggaggc tgaggcagga gaatggcgta aacccagaag gcggagcttg cagcgagctg 21480
   agattgtgcc actgcactcc atcctgggca acagagtgag actccgtctc aggagaaaaa 21540
   aaaaaaaaga aaataaatac agcacagact tctttctttc actgatttga gggagcaggc 21600
   atagctgcag ccacaggcag agtcgtagct agtctgatgt tgcaccccct acctagttcg 21660
   ctggcctggc ataggctgtc tgtggctacc cctgagtgca tctggacaca gtctgggagg 21720
   acggtgggtc ttgtttgtcc accggcctca caaagccccc tcccaccaag gtcttgccac 21780
   agtggcccat aagaaacctt ttgggccagg cgcgatgggg catgcctgta atcccagcac 21840
   tttgggaggc cgaggtgggc agatcatgag gtcaagagat caagaccatc ctggccaaca 21900
   tggtgaaact ttgtttccac taaaaataca aaaaattagc caggcgtggt ggcacgcacc 21960
   tgtagtccca gctactcggg agactgaggc aaaagaatca cttgaatccg ggaggcggag 22020
   gttgcagtga gccgagatca cgccactgca ttccagcctg ggtgacggag tgagactccg 22080
   tctcataaat aaataaacaa acaaaccttt tggtcaggtg ctatttactc ctaagctcat 22140
   tattttgccc ccactgctgc ccgaaggcct tcccagagcc ctcactgttt tgctggtttt 22200
   cctggaggga gaaatttgag tttgggagga ggaggctttc agggacggtc cagacactca 22260
   aaagtttgct tgcttttgtg ttgcagagaa tgcagcctgt aatcacagaa caggtgagtg 22320
   ttccctaccc ccagccgctg tacttgacat tgcaaagggt gagttttatt attattaaga 22380
   ataaaatgat aaaaaatatt aataattctt attaataaaa taatgaaaat attattaata 22440
   ataaatgtta ttattcaatg ttcagtgact ttcattggac agactcttga gtgtcaccct 22500
   tactgcgatc ttgcaaaatt gggatatttc acatccccaa attgagggat gggaaaagga 22560
   agagtcaggg atgacacctc ccaaggtgtg agagccagat gctatggctg gccaggtgct 22620
   gtccaaacga ggtccaccca tttgccccag attccttacc ctgggccagg cagccccagt 22680
   ccagcaggaa caagctacca aaccatagct ccacccagca gagacaagca ttcagacagg 22740
   tggcccagac ctcagacaga ggaccctccc ccagcccctg cctgggatct gcaggaacag 22800
   acagccggac cacagcattc tctgtcccct ctctctctgt ccatctcttt ccccgtatct 22860
   ctttctctgt ctctgaatct ctctgccttt ctccctccct ctctttctct ctctctcgtc 22920
   tctatctccc tccccctctc tgtatctccc tccctttttc cctccctcta tctttctggc 22980
   tctctccctg tgtctatctc tccctgtccc catctctctc tgtgtctctt tccctccctc 23040
   tctcattctc cttccctctc tctatctttc tctccctgtc tctatctccc gctccgtgtc 23100
   tgtctctatc tttctccctt tctccctcca tctctctctc cctgtctctc tctccccgtt 23160
   cccatctctc tctctgtgtc tctgtctctc cctctctcat tctccctccc tcttttattc 23220
   tccctccctc tctttctctc tctctctctc tctcctggtc tctgtctccc ccctccccat 23280
   ctctttctct gtgtctgtct ctgtaacctt ctccatttct ccctccatct ctctgtctct 23340
   ccctgtgtct atctcccact gtccctgtct gtttctccgt gtctgtctct gtatctctct 23400
   ccctttctcc ctccatcttt ctctctctcc cccggtctct atcttccctt ctccccatct 23460
   ctctctctct gtctgtctct gtatctctct ccctttctcc ctccatcttt ctctctctct 23520
   cccggtctct atctccccct ctccccgtct ctttctccgt gtctgtctct gtgtctctct 23580
   ccctttctcc ctccctccat ctttctctct ctctcccggt ctctatttcc tccctcccca 23640
   tctctttctc tgtgtctgtc tctgtatctc tctccctttc tccctccatc tttctctctc 23700
   tctcctggtc tctatctccc cctctccctg tctctctgtg tctgtctctg tatctctctc 23760
   cctttctccc tccctctctt tatctctctc tctctctctg cctctatctc ccactgtcca 23820
   aatctgtgtc tctgtctctc cctctttcat tctccctctc tttctctatc tctctccttg 23880
   tctatatctc cccctctccc agtctctctg tatctctgta tctctctccc tttctccttc 23940
   cctctcttca tctttctttt tgaaacggag tttttctctt gttgcccagg ctggagtgcg 24000
   atggcacgat ctcggctcac tgcaacctcc gcctcccagg ttcaagcgaa tctactccct 24060
   cagcctccca agtagctggg attacaggca ctcgccacca tgcccaacta actttttttt 24120
   ttttttgtat ttttagtaga gacagggttt cactagtggg ccaggctggt ctcgaactcc 24180
   tgacctcagg cgatccacct gcctcatcct ctcaaagtgc tggaattaca ggtgtgagcc 24240
   accgtgcccg gcccctctct ttatctttct agctctctcc ctgtctctct ctcccttccc 24300
   ctctctgtct ctctctccct tcccctctct gtctctctct cccttcccct ctctgtctct 24360
   ctctcccttc ccctctgtct ctctctccct tcccctctct gtctctctct cccttcccct 24420
   ctctgtctct ctctcccttc ccctctgtct ctctctccct tcccctctct gtctctctct 24480
   cccttcccct ctctgtctct ctctcccttc ccctctctgt ctctctctcc cttcccctct 24540
   ctgtctctct ctcccttccc ctctctgtct ctctctccct ttccttctct gtctctctct 24600
   cccttcccct ctctctctct ccattcccct gtctctatgt ctctcccttt ctctctccca 24660
   ttatttctct gtgattgtct ctttctttct ctgcctctgt ctgtctgtcc ccctgtatta 24720
   gtccattgtc acactgctga taaacatata cccaagactg ggtaatttat aaacaaaaga 24780
   gggttcctgg actcacagtt ccatgtggct ggggaggcct cacgatcacg gcagaaggtg 24840
   aaggaggagg aaaggcatgt cttacatgtc agcgggcaag acagaatgag acagtcgccg 24900
   ggcgcagtga ctcatgcctg taatcacagc accttgggag gacgaggtgg gtggaccacg 24960
   aggtcaggag ttcaagacca gcctggccaa catggtgaaa cctagctcta ctaaaaataa 25020
   aaaaaattac ccagacgtgg tggtggcagg cgcctgtggt cccagctact cgggaggctg 25080
   aagcaggaga atcgcttgaa cccgggaggc agaggttgca gtgagttgag atcgcgccac 25140
   tgcactccag cctgggtgac agagtgagac tccgtctcga gaaaaaaaaa aaaaaaaaaa 25200
   aaatgagagc cgagagaaag gtttccctta ttaaaccatt agatctcgtg agacttattc 25260
   actaccccga gaacagtgtg gggggaaact gcccccatga ttcagttatg tcctacccag 25320
   tccctctcac aacacgtggg agttataagg gctacaattc aagatgagat ttgggtgggg 25380
   acacagccaa actacatcac ccactctctc tgtctctctg cttctgtttt cctctctgtc 25440
   tctgtttttc tttccctctc tctgtctctt tgtatctctg tctctctctc tctgtctccg 25500
   tttctatctc tgtctctctc tgtccatcac ccactctgtc tctttgtctc tctatgtctc 25560
   tctgtttctg tttttctgtt tttctctttc cctctctctt tgtatctatg tctttctgtc 25620
   tctccgtttc tgtctctctc tctgtccatc acccactctg tctgtctttg tctatgtctc 25680
   tctgtttttc tgtctctttt tctctttccc tctctgtctc ccctctctgt gtctctttgt 25740
   atctccgtct ctccctgtct gtttctatct ctgtctctct ctgtccatca cctactctgt 25800
   ctctttgtct ctgtctgttt ctgtttttct ttctttttcc ctctgtctcc cttctctgtg 25860
   tctctttgta tctctgtctc tctctgtttc tatctgtctc tctctgtcca tcacccacta 25920
   tctctgtcta tgtctgtttt tctgtctctg tttttctctc tccctctctc tgtctcccct 25980
   ctctgtgtct ctttgtatct ctttctctct ctctgtctct ccctgtctgt ttctatctcc 26040
   gtcctcctgt attagtccat tttcatggac taatgaggat ccttcccgct tctcccagct 26100
   cctggggact ctggggtccc tgggcttgtg gccatatcac tccactctct gcctccgtct 26160
   ccacatggcc ttctcctctg catccgtgtc tcctttttat ttatttattt atttattttg 26220
   agacagagtc tcgctctgtc acccaggctg gagtgcagtg gtgcgatctc agctcactgc 26280
   aacctccgcc tcccaggttc aagcgattcc cctgcctcag cctcccgagt agctgggact 26340
   acaggtgcac accaccacgc ctggctaatt tttgtattat tagtaaagat gggattttac 26400
   cttgttgctc aggttgacct cgaactcctg acctcaggtg atctacccac ctcagcctcc 26460
   caaagtgctg ggattacagg tgtcagccac cgcgcccggc cacgtctcct cttcttataa 26520
   ggatattggt cattgcattt agggcccccc ctgatccaaa atgacatcat ctcaatctat 26580
   atcttaacga catctgccat gaacaggtat ttcatcataa ggccccattg taaggtgctg 26640
   ggggctaaga ctgcaacata tgaattttgg gggatcacaa tgcagcctca ttcttttgta 26700
   cccatcaaat atgaatggtc ttttcttctt tttttttttt ttttgagacg gagtctcgct 26760
   ctgtcaccca ggctggagtg cagtggcgca atctcggctc actgcaagct ccgcctcccg 26820
   ggttcccgcc actctcctgc ctcagcctcc tgagtagctg ggacctcagg cgcccgccac 26880
   cgggcccggc taattttttg tatttttagt agagacgggg tttcactgtg gtctcgatct 26940
   cctgaccttg tgatctgccc gccttggccc tccaaagtgc tgggattaca ggcgtgagcc 27000
   accgcgcctg gccgagctat ttcatcataa ggccccactg taaggagctg ggggctaaga 27060
   ctgcaacata tgaattttgg gggatcacaa tgcagactca ttcttttgta cccatcaaat 27120
   atgaatggtc ttttcttcta cttttttgtt tggtttttaa tttcagacag gctctcactc 27180
   tgtcacccac gctagagtgc agtggcacag tcacggctca ctgcaatctc tgcctcctgg 27240
   gctcaaggga tcctcctgcc tcagcccccc aagtagctga aactacaggc gtttgccacc 27300
   aggtcagctc atttaaaaaa aaatttggct gggcgcggtg gctcacgctg gtaatcccag 27360
   ccctttggga ggccgaggcg ggtggatcat gaggtcagga aatcaagacc atcctggcta 27420
   acatggtgaa accctgtctc tactaaaaaa aaaaatacaa aaaattagct gggcatggta 27480
   gcgggtgcct gtagtcccag ctactcggga ggctgaggca ggagaatggc atgaacccgg 27540
   gaagcggagc ttgcagtgag ccgagatcac gccactgcac tccagcctgg gggacagagt 27600
   gagactccat ctcaaaaaaa aaaaaatttt tttttggctg gacgtggtgg ctcacactgg 27660
   taatcccagc actttgggag gctgaggcgg gtggatcacc ggaggttggg agttcgagac 27720
   cagcctgacc aacatggaga aaccccgact ctactaaaaa tacaaaatta accgagcgtg 27780
   gtggtgggcg cctgtaatcc cagctactcg ggaggctgag gcaggagaat cgcttgaatc 27840
   tgggaggcgg aggttgcggt gagccgagtt ggtgccactg cactccagcc tgggcaacaa 27900
   gagtgaaact ctgtctcaaa acaaaacaaa acaaattttc ttctagagat ggggtcttgg 27960
   tttgttgccc aggctggtct caaactccta agctcgaggt atccccccac ctcagcctcc 28020
   ctggtagctg ggaccaaaga catgtgtcac cacgtctggt aatttttaca gttttttttt 28080
   tttggtaaag atggggtctt gctatgttgc ccaggctggt gtcaaactcc tgggcttaag 28140
   gaattctccc acctcagcct ccaaaagtgc tgtggtgaga ggcataaacc gtagcaccca 28200
   gcccctcttt tcttctttta tttatttatt tatttattta tttatttatt tagagacaga 28260
   gtctcactct gttgcccagg ctgcagtgca gtggtgccat ctcgcctcac tgcaacctcc 28320
   acctctcagg tttaagctat tctcctgcct cagcctcctg agtagctggg attacaggtg 28380
   cctgccacca tgccaggcta atttttgtat ttttagtaga gacagggttt caccatgttg 28440
   gccaggctgg tctcgaactc ctgacctcag gtgatccacc tgccttggcc tccctaagtg 28500
   cccaggtgag agtcatgagc caccatacct ggcccctctt ctttattttc tttcaaacca 28560
   caggtcagag acagaacctc cttccagcta ctcaatcctg gaacgtacac agtacaaata 28620
   agagcccggg aaagagtgta tgaattcttg agcgcctgga gcacccccca gcgcttcggt 28680
   gagtgggctg tgcggggtgc gcggggtgag cggggtgagc ggggtgcgcg gggtgagcgg 28740
   ggtgcgcggg gtgagcgggg tgcgcggggt gagcggggtg agcggggtga gccgggtgcg 28800
   cggggtgagc cggctgcgcg gggtgagcgg ggtgagcggg gtgagcgggg tgagcggggt 28860
   gcgcggggtg agcggggtga gcggggtgcg cggggtgagc ggggtgagcc gggtgcgcgg 28920
   ggtgagccgg gtgagcgggg tgcgcggggt gagcggggtg agcggggtgc gcggggtgag 28980
   ccgggtgcgc ggggtgagcg gggtgagccg ggtgcgcggg gtgagcgggg tgagcggggt 29040
   gagccgggtg agccgggtga gccgggtgcg cggggtgagc cgggtgcgcg gggtgagcgg 29100
   ggtgcgcggg gtgagcgggg tgcgcggggt gagccgggtg cgcggggtga gcggggtgcg 29160
   cggggtgagc ggggtgcgcg gggtgagccg ggtgcgcggg gtgagcgggg tgagcggggt 29220
   gagccgggtg ccccgggtga gcggggtgcg cggggtgagc cgggtgcgcg gggtgagcgg 29280
   ggtgagcggg gtgcgcgggg tgagccgggt gcgcggggtg agccgggtgc gcggggtgag 29340
   ccgggtgagc ggggtgagcg gggtgcgggg tgcgcggtgc gcggggtgag cggggtgcgc 29400
   ggggtgagcc gggtgcgcgg ggtgagcggg gtgagccggg tgccccgggt gagcggggtg 29460
   cgcggggtga gccgggtgcg cggggtgagc ggggtgcgcg gggtgagcgg ggtgcgcggg 29520
   gtgcgcggcg tgagccgggt gcgcggggtg cgcggggtga gcggggtgcg cggggtgcgc 29580
   ggggtgcgcg gggtgagcgg ggtgcgcggg gtgcgcggcg tgagccgggt gcgcggggtg 29640
   agcggggtgc gcggggtgag cggggtgcgc ggggtgagcc gggtgcgcgg ggtgagccgg 29700
   gtgagccggg tgcgcggggt gagcggggtg cgcggggtga gccgggtgag ccgggtgcgc 29760
   ggggtgagcc gggtgcgcgg ggtgagcggg gtgcgcgggg tgagccgggt gcgcggggtg 29820
   agcggggtgc gcggggtgag cggggtgcgc ggggtgagcg gggtgcgcgg ggtgagccgg 29880
   gtgcgcgggg tgagcggggt gagcggggtg agccgggtgc cccgggtgag cggggtgcgc 29940
   ggggtgagcc cggtgagccg ggtgcgcggg gtgagccggg tgcgcggggt gagccgggtg 30000
   agcggggtga gcggggtgcg cggtgcgcgg ggtgagcggg gtgcgcgggg tgagccgggt 30060
   gcgcggggtg agcggggtga gccgggtgcc ccgggtgagc ggggtgcgcg gggtgagccg 30120
   ggtgcgcggg gtgagcgggg tgcgcggagt gagcggggtg cgcggggtgc gcggcgtgag 30180
   ccgggtgcgc ggggtgcgcg gggtgagcgg ggtgcgcggg gtgcgcgggg tgcgcggggt 30240
   gagcggggtg cgcggggtgc gcggcgtgag ccgggtgcgc ggggtgagcg gggtgcgcgg 30300
   ggtgagcggg gtgcgcgggg tgagccgggt gcgcggggtg agcggggtgc gcggggtgag 30360
   ccgggtgcgc ggggtgcgcg gggtgcgcgg ggtgcgccgg gtgcgcgggg tgagccgggt 30420
   gcgcggggtg agcggggtgc gcggggtgcg cggggtgcgc ggggtgagcc gggtgcgcgg 30480
   ggtgagcggg gtgagccggg tgcgcggggt gagcggggtg agccgggtgc gcggggtgag 30540
   cggggtgcgc ggggtgagcc gggtgcgcgg ggtgagcggg gtgcgcgggg tgagccgggt 30600
   gcgcggggtg agcggggtgc gccgggtgag cggggtgagc ggggtgagcc gggtgcgcgg 30660
   ggtgcgcggg gtgcgccggg tgcgcggggt gagccgggtg cgcggggtga gcggggtgcg 30720
   cggggtgcgc ggggtgcgcg gggtgagccg ggtgcgcggg gtgagcgggg tgagccgggt 30780
   gagcggggtg cgcggggtga gccgggtgcg cggggtgagc ggggtgcgcg gggtgagccg 30840
   ggtgcgcggg gtgcgcgggg tgagccgggt gcgcggggtg agcggggtgc gcggggtgag 30900
   cggggtgcgc ggggtgagcc gggtgcgcgg ggtgagccgg gtgcgcgggg tgcgcggggt 30960
   gcgcggcgtg agccgggtgc gcggggtgcg ccgggtgcgc ggggtgagcg gggtgagccg 31020
   ggtgcgcggg gtgagcgggg tgcgcggggt gagcggggtg cgccgggtga gccgggtgcg 31080
   cggggtgagc cgggtgcgcg gggtgagcgg ggtgcgcggg gtgagccggg tgcgcggggt 31140
   gagcggggtg cgccgggtga gcggggtgcg cggggtgagc ggggtgcgcc gggtgagcgg 31200
   ggtgcgcggg ctgagcgggg tgcgccgggt gagcggggtg cgcggggtga gcggggtgcg 31260
   ccatcctggg tcacggaaac actcctctcc tgcaaaggag aggagattca ctcccccagt 31320
   ttctgtgacc ccaaaaagga ccctgaaccc gacggtgaac tcacagcttg ctcttactca 31380
   cgagaggaga cgtggagggg aaacaaggtc gtcccactga cagacacccc ctgggccttg 31440
   taataaagac cgaggcgggc ggatcacaag gtcaggagat cgagaccatc ccggctaaca 31500
   cggtgaaacc ccgtctctac aaaaaatgta acaaattagc caggcgtggt gatgggcgcc 31560
   tgtagtccca gctactcggg aggctgaggc aggagaatgg cgtgaacccg ggaggcggag 31620
   gttgcagtga gccgagatcg cgccactgtg ctccatccag cctgggcgac aagagcgaga 31680
   ctccatctca acaacaaaac aaaaggatcg cctcagagta gaacttctgg ccgggcacgg 31740
   tgactcatgc ctgccatccc agcactttgg gaggctgagg tgggtagatc acctgaggtc 31800
   aggagttcga gaccagcctg accaacatgg aaaaaccctg tctctactaa aaatagaaaa 31860
   attaggtcag gtgctgtggc ttaagcctgt aacctcagct accagggagg ctgaggcagg 31920
   agaatcgctt gaattgtgtt ccctcaaaat tcgtgtgttg aagctttgat cccccaggac 31980
   ctcagaatgt gactgtgttt ggagttgggg tgtttaaaga ggcgattaag gtaaaatgag 32040
   gtcattaggg tgggccctaa tctaacagga ctggggtcct tataagaaga ggagatgagg 32100
   acacagacac acacagaggg acgaccctgt gggacacagg gagaagacgg cgtctccaag 32160
   cccaggagag gggcctcagg aggaaccagc actgcccaca cctggatctc agacctccag 32220
   cctccagggc tgtgggagaa tcaatgtgtt ttgtttctaa gccgcccagc ctctggtatt 32280
   ctgtgacagc agcctgagat ggactaagcc atctcataag aagagacgag gacacagaca 32340
   cacacagagg gacgaccctg tgagggcaca gggagaagac ggcgtctcca agcccaggag 32400
   aggggcctca ggaggaacca gcactgccca cacctggatc tcagacctcc agcctccagg 32460
   gctgtgggag aatcaatgtg ttttgtttct aagccgccca gcctctggta ttctgtgaca 32520
   gcagcctgag atggactaag acatctcata agaagaggag atgaggacac agacacacac 32580
   agagggacga ccctgtgagg gcacagggag aagacggcgt ctccaagccc aggagagggg 32640
   cctcaggagg aaccagccct gcccacacct ggatctcaga cctccagcct ccagggctgt 32700
   gggagaatca atgtgttttg tttctaagcc gcccagcctc tggtattctg tgacagcagc 32760
   ctgagatgga ctaagacatc tcataagaag aggagatgag gacacagaca cacacagagg 32820
   gacgaccctg tgagggcaca gggagaagac ggcgtctcca agcccaggag aggggcctca 32880
   ggaggaacca gcactgccca cacctggatc tcagacctcc agcctccagg gctgtgggag 32940
   aatcaatgtg ttttgtttct aagccgccca gcctctggta ttctgtgaca gcagcctgag 33000
   atggactaag ccatctcata agaagagacg aggacacaga cacacacaga gggatgaccc 33060
   tgtgagggca cagggagaag acggcgtctc caagcccagg agaggggcct caggaggaac 33120
   cagcactgcc cacacctgga tctcagacct ccagcctcca gggctgtggg agaatcaatg 33180
   tgttttgttt ctaagccgcc cagcctctgg tattctgtga cagcagcctg agatggacta 33240
   agacatctca taagaagagg agatgaggac acagacacac acagagggat gaccctgtga 33300
   gggcacaggg agaagacggc gtctccaagc ccaggagagg ggcctcagga ggaaccagcc 33360
   ctgcccacac ctggatctca gacctccagc ctccagggct gtgggagaat caatgtgttt 33420
   tgtttctaag ccgcccagcc tctggtattc tgtgacagca gcctgagatg gactaagcca 33480
   tctcataaga agagacgagg acacagacac acacagaggg acgaccctgt gaggacacag 33540
   ggagaagacg gcgtctccaa gcccaggaga ggggcctcag gaggaaccag cactgcccac 33600
   acctggatct cagacctcca gcctccaggg ctgtgggaga atcagtgttt tgtttctaag 33660
   ccgcccagcc tctggtattc tgtgacagca gcctgagatg gactaagcca tctcataaga 33720
   agagacgagg acacagacac acacagaggg acgaccctgt gagggcacag ggagaagacg 33780
   gcgtctccaa gcccaggaga ggggcctcag gaggaaccag ccctgcccac acctggatct 33840
   cagacctcca gcctccaggg ctgtgggaga atcaatgtgt tttgtttcta agccgcccag 33900
   cctctggtat tctgtgacag cagcctgaga tggagtaaga catctcataa gaagaggaga 33960
   tgaggacaca gacacacaca gagggacgac cctgtgggac acagggagaa gacggggtct 34020
   ccaagcccag gagaggggcc tcaggaggaa ccagccctgc ccacacctgg atctcagacc 34080
   tccagcctcc agggctgtgg gagaatcaat gtgttttgtt tctaagccgc ccagcctctg 34140
   gtattctgtg acagcagcct gagatggact aagccatctc ataagaagag acgaggacac 34200
   agacacacag agagggacga ccctgtgagg acacagggag aagacggcat ctccaagccc 34260
   aggagagggg cctcaggagg aaccagcact gcccacacct ggatctcaga cctccagcct 34320
   ccagggctgt gggagaatca atgtgttttg tttctaagcc gcccagcctc tggtattctg 34380
   tgacagcagc ctgagatgga ctaagacatc tcataagaag aggagatgag gacacagaca 34440
   cacacagagg gacgaccctg tgaggacaca gggagaagac ggcgtctcca agcccaggag 34500
   aggggcctca ggaggaacca gccctgccca cacctggatc tcagacctcc agcctccagg 34560
   gctgtgggag aatcaatgtg ttttgtttct aagccgccca gcctctggta ttctgtgaca 34620
   gcagcctgag atggactaag ccatctcata agaagagacg aggacacaga cacacacaga 34680
   gggacgaccc tgtgagggca cagggagaag acggcgtctc caagcccagg agaggggcct 34740
   caggaggaac cagccctgcc cacacctgga tctcagacct ccagcctcca gggctgtggg 34800
   agaatcaatg tgttttgttt ctaagccgcc cagcctctgg tattctgtga cagcagcctg 34860
   agatggacta agccatctca taagaagaga cgaggacaca gacacacaca gagggacgac 34920
   cctgtgaggg cacagggaga agacggcgtc tccaagccca ggagaggggc ctcaggagga 34980
   accagccctg cccacacctg gatctcagaa ctccagcctc cagggctgtg ggagaatcaa 35040
   tgtgttttgt ttctaagccg cccagcctct ggtattctgt gacagcagcc tgagatggac 35100
   taagacatct cataagaaga ggacatgagg acacagacac acacagaggg acgaccctgt 35160
   gggacacagg gagaagacgg ggtctccaag cccaggagag gggcctcagg aggaaccagc 35220
   cctgcccaca cctggatctc agacctccag cctccagggc tgtgggagaa tcaatgtgtt 35280
   ttgtttctaa gccgcccagc ctctggtatt ctgtgacagc agcctgagat ggactaagac 35340
   atctcataag aagaggagat gaggacacag acacacacag agggacgacc ctgtgggaca 35400
   cagggagaag acggggtctc caagcccagg agaggggcct caggaggaac cagccctgcc 35460
   cacacctgga tctcagaact ccagcctcca gggctgtggg agaatcaatg tgttttgttt 35520
   ctaagccgcc cagcctctgg tattctgtga cagcagcctg agatggacta agacatctca 35580
   taagaagagg agatgaggac acagacacac acagagggac gaccctgtga ggacacaggg 35640
   agaagacggc gtctccaagc ccaggagagg ggcctcagga ggaaccagcc ctgcccacac 35700
   ctggatctca gacctccagc ctccagggct gtgggagaat caatgtgttt tgtttctaag 35760
   ccgcccagcc tctggtattc tgtgacagca gcctgagatg gactaagcca tctcataaga 35820
   agagacgagg acacagacac acacagaggg acgaccctgt gagggcacag ggagaagacg 35880
   gcgtctccaa gcccaggaga ggggcctcag gaggaaccag ccctgcccac acctggatct 35940
   cagacctcca gcctccaggg ctgtgggaga atcaatgtgt tttgtttcta agccgcccag 36000
   cctctggtat tctgtgacag cagcctgaga tggactaagc catctcataa gaagagacga 36060
   ggacacagac acacacagag ggacgaccct gtgagggcac agggagaaga cggcgtctcc 36120
   aagcccagga gaggggcctc aggaggaacc agccctgccc acacctggat ctcagaactc 36180
   cagcctccag ggctgtggga gaatcaatgt gttttgtttc taagccgccc agcctctggt 36240
   attctgtgac agcagcctga gatggactaa gacatctcat aagaagagga catgaggaca 36300
   cagacacaca cagagggacg accctgtggg acacagggag aagacggggt ctccaagccc 36360
   aggagagggg cctcaggagg aaccagccct gcccacacct ggatctcaga cctccagcct 36420
   ccagggctgt gggagaatca atgtgttttg tttctaagcc gcccagcctc tggtattctg 36480
   tgacagcagc ctgagatgga ctaagacatc tcataagaag aggagatgag gacacagaca 36540
   cacacagagg gacgaccctg tgggacacag ggagaagacg gggtctccaa gcccaggaga 36600
   ggggcctcag gaggaaccag ccctgcccac acctggatct cagaactcca gcctccaggg 36660
   ctgtgggaga atcaatgtgt tttgtttcta agccgcccag cctctggtat tctgtgacag 36720
   cagcctgaga tggactaaga catctcataa gaagaggaca tgaggacaca gacacacaca 36780
   gagggacgac cctgtgggac acagaagacg gggtctccaa gcccaggaga ggggcctcag 36840
   gaggaaccag ccctgcccac acctggatct cagacctcca gcctccaggg ctgtgggaga 36900
   atcaatgtgt tttgtttcta agccgcccag cctctggtat tctgtgacag cagcctgaga 36960
   tggactaaga catctcataa gaagaggaca tgaggacaca gacacacaca gagggacgac 37020
   cctgtgaggg cacagggaga agacggcgtc tccaagccca ggagagaggc ctcaggagga 37080
   accagccttg cccacacctg gatctcagac ctccagcctc cagggctgtg ggagaatcaa 37140
   tgtgttttgt ttctaagccg cccagcctct ggtattctgt gacagcagcc tgagatggac 37200
   taagacacct cataagaaga ggagatgagg acacagacac acacggagga acaaccctgt 37260
   gaggacacag ggagaagacg gcatctccaa gcccaggaga gaggcctcag gaggaaccag 37320
   ccctgcccac acctggatct cagacctcca gcctccaggg ctgtgggaga atcaatgtgt 37380
   tttgtttcta agccgcccag cctctggtat tctgtgacag cagcctgaaa cggactaaga 37440
   catcccataa aaaggagatg aggacacaga cacacacgga gggacgaccc tgtgggacac 37500
   agggagaaga cggcgtctcc aagcccagga gaggggcctc aggaggaacc agcactgccc 37560
   acacctggat ctcagacctc cagcctccag ggctgtggga gaatcaatgt gttttgtttc 37620
   taagccgccc agcctctggt attctgtgac agcagcctga gatggactaa gccatctcat 37680
   aagaagagac gaggacacag acacacacag agggatgacc ctgtgagggc acagggagaa 37740
   gacggcgtct ccaagcccag gagaggggcc tcaggaggaa ccagcactgc ccacacctgg 37800
   atctcagacc tccagcctcc agggctgtgg gagaatcaat gtgttttgtt tctaagccgc 37860
   ccagcctctg gtattctgtg acagcagcct gagatggact aagacatctc ataagaagag 37920
   gagatgagga cacagacaca cacagaggga cgaccctgtg agggcacagg gagaagacgg 37980
   cgtctccaag cccaggagag gggcctcagg aggaaccagc cctgcccaca cctggatctc 38040
   agacctccag cctccagggc tgtgggagaa tcaatgtgtt ttgtttctaa gccgcccagc 38100
   ctctggtatt ctgtgacagc agcctgagat ggactaagcc atctcataag aagagacgag 38160
   gacacagaca cacacagagg gacgaccctg tgaggacaca gggagaagac ggcgtctcca 38220
   agcccaggag aggggcctca ggaggaacca gcactgccca cacctggatc tcagacctcc 38280
   agcctccagg gctgtgggag aatcaatgtg ttttgtttct aagccgccca gcctctggta 38340
   ttctgtgaca gcagcctgag atggactaag ccatctcata agaagagacg aggacacaga 38400
   cacacacaga gggacgaccc tgtgagggca cagggagaag acggcgtctc caagcccagg 38460
   agaggggcct caggaggaac cagccctgcc cacacctgga tctcagacct ccagcctcca 38520
   gggctgtggg agaatcaatg tgttttgttt ctaagccgcc cagcctctgg tattctgtga 38580
   cagcagcctg agatggagta agacatctca taagaagagg agatgaggac acagacacac 38640
   acagagggac gaccctgtgg gacacaggga gaagacgggg tctccaagcc caggagaggg 38700
   gcctcaggag gaaccagccc tgcccacacc tggatctcag acctccagcc tccagggctg 38760
   tgggagaatc aatgtgtttt gtttctaagc cgcccagcct ctggtattct gtgacagcag 38820
   cctgagatgg actaagccat ctcataagaa gagacgagga cacagacaca cacagaggga 38880
   cgaccctgtg agggcacagg gagaagacgg cgtctccaag cccaggagag gggcctcagg 38940
   aggaaccagc cctgcccaca cctggatctc agacctccag cctccagggc tgtgggagaa 39000
   tcaatgtgtt ttgtttctaa gccgcccagc ctctggtatt ctgtgacagc agcctgagat 39060
   ggactaagac atctcataag aagaggacat gaggacacag acacacacag agggacgacc 39120
   ctgtgggaca cagggagaag acggggtctc caagcccagg agaggggcct caggaggaac 39180
   cagccctgcc cacacctgga tctcagacct ccagcctcca gggctgtggg agaatcaatg 39240
   tgttttgttt ctaagccgcc cagcctctgg tattctgtga cagcagcctg agatggacta 39300
   agacatctca taagaagagg agatgaggac acagacacac acagagggac gaccctgtgg 39360
   gacacaggga gaagacgggg tctccaagcc caggagaggg gcctcaggag gaaccagccc 39420
   tgcccacacc tggatctcag acctccagcc tccagggctg tgggagaatc aatgtgtttt 39480
   gtttctaagc cgcccagcct ctggtattct gtgacagcag cctgagatgg actaagacat 39540
   ctcataagaa gaggacatga ggacacagac acacacagag ggacgaccct gtgggacaca 39600
   gaagacgggg tctccaagcc caggagaggg gcctcaggag gaaccagccc tgcccacacc 39660
   tggatctcag acctccagcc tccagggctg tgggagaatc aatgtgtttt gtttccaagc 39720
   cgcccagcct ctggtattct gtgacagcag cctgagatgg actaagacat ctcataagaa 39780
   gaggacatga ggacacagac acacacagag ggacgaccct gtgagggcac agggagaaga 39840
   cggcgtctcc aagcccagga gagaggcctc aggaggaacc agccttgccc acacctggat 39900
   ctcagacctc cagcctccag ggctgtggga gaatcaatgt gttttgtttc taagccgccc 39960
   agcctctggt attctgtgac agcagcctga gatggactaa gacacctcat aagaagagga 40020
   gatgaggaca cagacacaca cggaggaaca accctgtgag gacacaggga gaagacggcg 40080
   tctccaagcc caggagagag gcctcaggag gaaccagccc tgcccacacc tggatctcag 40140
   acctccagcc tccagggctg tgggagaatc aatgtgtttt gtttctaagc cgcccagcct 40200
   ctggtattct gtgacagcag cctgaaacgg actaagacat cccataaaaa ggagatgagg 40260
   acacagacac acacggaggg acgaccctgt gggacacagg gagaagacgg cgtctccaag 40320
   cccaggagag gggcctcagg aggaaccagc cctgcccaca cctggatctc agacctccag 40380
   cctccagggc tgtgggagaa tcaatgtgtt ttgtttctaa gccgcccagc ctctggtatt 40440
   ctgtgacagc agcctgagat ggactaagac atctcataag aagaggagat gaggacacag 40500
   acacacacca agggacaacc ctgtgggaca cggagaagac ggggtctcca agcccaggag 40560
   agaggcctca ggaggaacca gccctgccca tgcccatctc ttgatcttgg acctgcagct 40620
   tccaggactg tgggagaatc aattcctttt ttctttttct tttttctttt ttttcctctt 40680
   tttctgagat ggagtctcac tctgtcgcca ggctggagtg cagtggcgtg atcttggctc 40740
   actgcaacct ccacctcgca ggttcaggcc attctcctgc ctcagccttc tgagtagctg 40800
   ggatgacagg cgcccgccac cacacccggc tactgtttgt atttttatta gagacgggct 40860
   ttctggccat gttggccagg ctggtctcat actcccgacc tcaggtgatc cgcccgcctt 40920
   ggcctcccaa agcgctggga ttacaggcgt gagccaccgc gcccggccaa attccttttt 40980
   tctgaagcca ccaagctgtg ggactttttt atggcagtcc cagcagacag atccaccctc 41040
   gttccaaata aggttatcgt cataggttct gggggtgagg tcatagatgt atgttttttt 41100
   ttttttttgg agagagagtc tctctctatt gcccaggctg gagggcagtg gcgcgatcac 41160
   ggctcactgt agcctcgacc tcctgggctc aagcgatcct ctcgggaggc taagagagga 41220
   gaatcgtttg aatccgggag gcagaggttg cagtgagctg agatagcacc acagcactgc 41280
   agagcgagac tctgtctcaa aaaaaaaaaa aagaaaaaag aaaaaaaata ggccgggtgc 41340
   gtggctgacg cctgtaatcc cagcactttg ggaggccgag gcgggtggat cacttgaggt 41400
   caggagttca agaccagcct aacgaagatg gtgaaacccc atctctatga gaaatacaaa 41460
   aattacccgg gcgtggtggc gggcgcctgt agtcccagct actcgggagg caggggcagg 41520
   agaatcgctt gaacctggga ggtggaggtt gtgagctgag atcgtgccac tgcactccag 41580
   cctgggcgac aaaacgagac tccatctcaa aaaaaaaaaa aaaagaaaaa gaaaaagaaa 41640
   aaattaacac acacacacaa attccatgat aaacacaaaa tcaagttcaa agcacgcacg 41700
   ccagtgctgc ctctgctgcc cctccatgct gcccacaccc aaagcacctg ccggcctgct 41760
   gggcacagtg gaagtccctg gcgctccccg tgtcccgagc accaggcaga cagggatccc 41820
   tggtggtctt tccgctccct tagggttcca gactggggct gggaggtccg gaagtcgctc 41880
   ccggtcctgg tactcaggtg gcctgcaggt ggcccgcagg tggtcacggt ctctgtgcag 41940
   gtggcactac tggggtgtcc ccccctggac gccaccccat atggcagcca cctctctgct 42000
   tcccagggcc ccggggagag cttacagtcc ctggtccccc caggacggcc cccggtctgt 42060
   gaccctctca ccctttaccc ctagagtgcg accaggagga gggcgcaaac acacgtgcct 42120
   ggcggacgtc gctgctgatc gcgctgggga cgctgctggc cctggtctgt gtcttcgtga 42180
   tctgcagaag gtgagccctc gagggcgtcc gcgagcgtcg cttgtttcca gtgtgaccct 42240
   gaaagttatt cacagaacca tcctgagaat tatcattatt atttttgtga tggagtctcg 42300
   ctctgtctcc caggctggag tgcagtggca cgatctgggc tcactgcagc ctctgcctcc 42360
   tgggttcagg cgatcctcct gcctcagcct cccaagtagc tgggattaca ggcacccagc 42420
   aacacaccca gctaattttt gtatttttag tagagacggg gttttgccat gttggccagg 42480
   ctggtcttga attcctggac tcaggtgatc cacctgcctc agcctcctaa agtgctggga 42540
   ttacaggcgt aagccacctc gcctggccca tattattatt attgttatga ttattattat 42600
   tttttgagtc ttgctctgtc acccaggctg gagtgcagtg gctcgatctc ggctcaccgc 42660
   agcctccacc tcccgggttc aagtgattct cctgcctcag cctcccgagt agctgggatt 42720
   acaggtgtgc aacactgcac ccaactaatt cttgtatttt tagtggagac ggggtttcac 42780
   catgttggcc acactggtgt tgaactcctg gcctcaagtg atcccccagc ctcggcctcc 42840
   caaagtgctg ggattacagg cgtgagccac cgcacctggc cccttgagaa ttattcacaa 42900
   aagaccaggg gccaggtcct ctctgctagc tgccccctgc caggaggtct gtagagccaa 42960
   agctgagggc tctggtggga ccaagggtgt ccccagggtg gaggaagcag ggccggtcct 43020
   cccctgccct gcccagcggg cctgacacag tcagagggcg aaaggccagg ctttctggtc 43080
   gggaaggggc ctgggcgaac gtcacagctg tccacttgga tgggccagga tccgtcatgc 43140
   agaccaactc gaggtttttg gtcaccagct gggtcacccc aggtcctgtc tccttttttt 43200
   tagagaagga gttttgcttt tgttgcccag cctggagtgc aatggcgcca tctcggttca 43260
   ctgcaacctc tgcctcctgg gttcaagcga ttctcctgcc tcagcctccc tgagtagctg 43320
   ggattacaag gatgtgccac cacgcctggc taattttgta tttttagtag agatggggtt 43380
   tctccgtgtt ggtcaggctg gtcttgaact cctgacctca tgtgatccac ccgcctcggc 43440
   ctcccaaagt gctgggatga caggcgtgag ccaccatgcc cggataattt tgtattttca 43500
   gtagagacgg agtttctccg tgttgatcag gctggtcttg aactcctcac ctcagttgat 43560
   ctgcccgcct gggcctccca aagtgctggg atgacaggcg tgtgccacca tgcccagata 43620
   attttgtatt tttagtagag atggagtttc tccatgttgg tcaggctgtt ctcgaactcc 43680
   tgacctcagt tgatctgccc gcctgggcct cccaaagtgc tggcattaca ggcttgagcc 43740
   accatgacca gccaggccct gtttccttcc tgctgggcat cagagcttca caatctgtca 43800
   aatgggtgcc atggtgactt ctgtctcccg ggggaagggg gaaaagggga gggggagggg 43860
   gaaggggaag agggggaggg agggggaagg ggaggagggg gaagggaggg gggaaaggag 43920
   ggggaaggga ggagggggaa gggaagaggg ggaggagggg gaaggggagg aggaggaggg 43980
   ggaggagagg cgagggggag ggtgggggag ggggaagggg gaggaggaga ggggaggatg 44040
   agtggggagg gggaaggagg gagaggggga gggggagagg gaggggtagg ggggaagggg 44100
   tggaggaggg aggaaaaggg ggaggaagag ggggacgagg cccttcctgc aagctgtctc 44160
   aggtcgtaaa ctcagtgacc tgaggcacca ggggtctgtc tttgcagctg cacccctggg 44220
   tggctccggt accagcgccc tactccttta attagacacc agcgcctgcc tatgatgatg 44280
   gtcgggggcg tgtcagggcc tcaggggccg ggaaaataga gacccctcga gtagatgact 44340
   tgagtctttt gctctgtcct ggcactgtct gtcctggaca cgctgtgtcc cagatgatga 44400
   gctggtcggt tttgggttca gagctgggcc atttctcttt cctccgaggt atctggtgat 44460
   gcagagactc tttccccgca tccctcacat gaaagacccc atcggtgaca gcttccaaaa 44520
   cgacaagctg gtatgttgtt ttttctgcct tgggacgggt ctggaggcgt ggtggccact 44580
   ttgggaggcc caggcgggcg gaccacttga ggccaggaac tggagaccag cctggccaac 44640
   atggagaaac tccgtgtcta ctaaaaatac aaaattagcc gggtgtggtg gcgggcgcct 44700
   gtaatcccag ctactcggga ggctgaggga ggaggatcac ttgaacccgg gaggtggagg 44760
   ctgcagtgaa ctacgatcga gccactgcac tccatctggg cgacaagagt gaaatggcct 44820
   ggactaggag gctggacacc tgtctgctgg cgctggtggg cggctgggaa aagctgcagg 44880
   atgaagggag tggggggaca ctgggctccc agcccaccgt gatcacgggc cgtctccgca 44940
   ccctgcacac cagagcagag cagatttttt tttttttttt tagatggagt tttgctcttg 45000
   ttgcccaggc tagagtgcaa tggtgcgatc ttggctcacc acaacctcca ccttcccagt 45060
   ccaaggttca agcgattgtc ctgcctcagc ctcccgagta gctgggacta caggcacgca 45120
   ccgacacgcc cagctaattt tgtattttta gtagagacgg ggtttcacca tgttggccag 45180
   gatggtctcg atctcctgac cttgtgatcc ccctgcctcg gcctcccaaa gtgctgggat 45240
   tacaggcatg agccaccatg cccagcctag ttttcaacaa gtttttagtg atacctgtgt 45300
   ccctaagaga aaggaagggc agaggaaaag gaggcagaca tctctgtcag agttttttgt 45360
   tttgttttgt tttgtttgtt tttgtttttg agacgcagtt ttgttcgttg ccccggctgg 45420
   agtgcaatgg tgcaatctcg gctcactgca acctccacct tcccggttga aggttcaagc 45480
   gattctcctg cctcagcctc ccaagtagct gggactacag gcatgcacca acacacccag 45540
   ctaattctgc atttttagta gagaaggggt ttcaccatgt tggccaggat ggtctggatc 45600
   tcctgacctt atggtccgct cgcctcggcc tcccaaagtg ctgggattac aggtgtgacc 45660
   caccgcgcct ggcccaaagt gctgggatga caggcgtgag acaccatgcc tggcccacag 45720
   agcagatctg agatgggaca ggcccccgca gatcaggacg tgggctctgt tatctggggg 45780
   gtggccgact caccctgcct cctctcgtct ctgcaggtgg tctgggaggc gggcaaagcc 45840
   ggcctggagg agtgtctggt gactgaagta caggtcgtgc agaaaacttg agactggggt 45900
   tcagggcttg tgggggtctg cctcaatctc cctggccggg ccaggcgcct gcacagactg 45960
   gctgctggac ctgcgcacgc agcccaggaa tggacattcc taacgggtgg tgggcatggg 46020
   agatgcctgt gtaatttcgt ccgaagctgc caggaagaag aacagaactt tgtgtgttta 46080
   tttcatgata aagtgatttt ttttttttta accca 46115

<210> 28
<211> 24252
<212> DNA
<213> Homo sapiens

<400> 28
gccgagccag ccccttcacc accagccggc cgcgccccgg gaagggaagt ttgtggcgga 60

ggaggttcgt acgggaggag ggggaggcgc ccacgcatct ggggctgact cgctctttcg 120

caaaacgtct gggaggagtc cctggggcca caaaactgcc tccttcctga ggccagaagg 180

agagaagacg tgcagggacc ccgcgcacag gagctgccct cgcgacatgg gtcacccgcc 240

gctgctgccg ctgctgctgc tgctccacac ctgcgtccca ggtaggggct gggtcccgaa 300

cgcctgcgcc tggaacaggg tctaattgag ggggttgggg gttgtcagag gatgagttgg 360

aggaatgcgg ttcagtcctc agcatcctcc ctaatcaaat aatagtaatt ctcgtgcttt 420

gtgcaacgcc acgcggcgca gtacctggca ctcagtagct aaggaaatat tagtggagca 480

aaggcattta gctttacata atttagtgag tgcttttttt tttttttttt tttttttgac 540

agagtctcac tctgtcgccc aggctggagt gcagtggcgc gatctcggct cactgcaacc 600

tctgccgccc gggttcaagc gattctcctg cctcagcctc ctgggtagct gggattacag 660

gcgcctgcca tcgcgcccgg ctaatttctg tatttttagt ggagaccggg tttcactctg 720

ttagccagga tggtctcaat ctcctgacct cagatgatcc acctgcctcg gcctcccaaa 780

ctgctgggat tacaggcgtg agccaccgcg cccggcccaa gtgttctttt tttaaatgga 840

gttcttcgaa gcctcttcct tgcaatttca aactaggcga tgggacttta ttaatttcgt 900

ttcgcagagg aaactagggc acagagaggt tagataactg gcctaaaatc acacagccag 960

tgcttgaata cgcaggatct gaccctgcag cgccccccag cgctctccac gctgctgggt 1020

ctccccctct gagaaacggg gggacaggac ccccttttac aaaaggccca aagggaggct 1080

gactgagcgg cgcagagcca gtgctggaga cccgggactg tccctcagga cctttccctc 1140

tcactgaggc gactctcact tacttccccg gaaaatgtgg ggggctctgg gtcgaggaat 1200

tcgagaagga actgagtcag ggcgggtggc cacagggtgt tggggccgcg atgaataacc 1260

cggaaagcgc tcgagaccgc gggaggccgg gaatgagtaa cagctccggg atactccgaa 1320

cgcgcagctg gaaagggatg tccgggaagg cccggaggtc ggggaccggg cctagggact 1380

gggctgcaat ctcggggcgg agcctggggc ggggagagag tgtcggggag gagccagagg 1440

gcggggctgg aacctcaagg aagagctacg ggagaggtta cagaccgagg aagagctagg 1500

agcggggcta gaacctcgag gcggagccag agggcggggt tataacctcg aggcgtaacc 1560

agagggcgga gttataacct cgggaggagc tagaaggtgg gactagactc ttgaggggca 1620

ggattataac ctcggggagg agctagaggg cggggctgga acctcaagga ggggctaggg 1680

gcggggttat aagcttgggg aggagctacg gggctgggct aggacctcaa ggggccaagg 1740

ggcgggggtt acgacctcgg ggaggagcta cagggtgggg ctgaaacctc aagaagggac 1800

tggggcggag ttatgacctc aggggaaaac taggggcggg gagagctaaa gggcgaagtt 1860

agaatctcaa ggaggagcta gagggaggga ctaaagcctc gaggaggagc taggggcggg 1920

gttatggcat cggggaggag ctagggggcg ggaagataag gtcaggaaga gctaaagggc 1980

ggggctagaa cctcgacgat gagtctggag cggggccaaa acctagttgg agagctggag 2040

ggcggagaca gaacttcgcg ggaaactaga gtatctaaca atagaaactc cggagggctg 2100

atggggccgg gcctagaatt tgggaataaa ttagtgggcc gggagaggct ctggagccgg 2160

gtagaaccag ggggagtgag tggagggata gttccattag ggcactggga gtgacggtat 2220

aacataaaga tcgacgcggg tggggcaagg ctagaacgtc cccagcagaa ctggagaggc 2280

gtaatcgacc gagggccggt gcggtgagaa agacctaata ggagcaggaa tagaacgtac 2340

tgatagagag ggcggggata cgactgtcag gatatacgcc tcacgaggac agaatgaaag 2400

gaaaaacggg ccaaggcagg actttgggaa aggacttgtg ggcagggata gaacgttcag 2460

ttagtggggt ggaggtagaa cgtggacaac ggacagactg gaagtaccgc cggccggaac 2520

ccagcagaac atggacacga atctgaatgg acggggcctg gagacttggt gttggtattg 2580

ggacatgcag gggtgagcgg gggtcttgga gctaagcgta gttaacctct cctctcctct 2640

ccctccttcc cccagcctct tggggcctgc ggtgcatgca gtgtaagacc aacggggatt 2700

gccgtgtgga agagtgcgcc ctgggacagg acctctgcag gaccacgatc gtgcgcttgt 2760

gggaaggtga gcttcccccc aacccacaca cccctccagc gcagcatgga acgcgagtga 2820

ttgagaaacc ttcctcacca gtaactggga catgcaaact aaaaacaaac aaacaaggag 2880

agcccatttc acacccacca gattggcaag catttaaaaa tctgacaata cccgggttag 2940

taaggatgtg gagttgcaga ccgtgtctgt ggtgggggag taaattggca catcaaccac 3000

ttagagcgat tcggcaatat ctagtccaga tgaagattaa catatgctac agctcaactc 3060

ctcgccatgc agaaatgcca aagaaccttg gccacatata cccagggaca catgtatacg 3120

aatgttctta gcagcaatgt ttgtgacatg gaaaattgga aacaggctgg gcactggctc 3180

acacctgtat ttccagcact ttgggaggca gaggcggatg aattgcttga ggtcaggagt 3240

tcaagaccag gctggccaac atggtgaaac acccgtctcc acacacacac aaaaaaggaa 3300

aaattagccg agtgtggtgg cacgcacctg taatctcagc tacttgggag gctgagacag 3360

gagaattgct tgaacctggg aggtggaggt tgcagtgagc tgagatcatg ctactgcact 3420

ccagcctggg cgacagagcg agatcctgtc aaaaaaatga acgaaagaga gaaaggtggg 3480

ccgggcatgg tggctcacac ctgtaatccc agcactttgg gaggctgagg caggtggatt 3540

acctgaggtc aggagttcag gaccagcctg gccaacatgg tgaaaccctg tctctactaa 3600

aaaatacaaa aattagccgg gcttggtggc aggcaccagt tatcccagct actcgggagg 3660

ctgagacagg agaattgttt gaacccagga ggcggaggtt gcagtgagct gagattgtgc 3720

cattgcactc cagcctgggc gacaagagca aaactccatc tcaagaaaga aagaaagaaa 3780

ggaaaggaag agagagagag agaaagagag agaggaaggg agggagggac ggagagaggc 3840

agggagggag ggagggagga gggaggaagg aaggggagaa agaaagaaaa gaagaaaatg 3900

gaaacgactt tatgtcagtc agcaagtcag cagcagaaga gaaacactgt ggtaataatt 3960

acataatgga gcgctctact gcagcttaaa gaaggaaata gagctgcaga taccaatgtg 4020

gacacatctc agaaacctaa ggttgagagg gaaaaaaatg ttgcagaata tgatacgttt 4080

tttataagct ttaagaacac atgaaacaac agtatatgaa gccaagcaca gtagctcaca 4140

tttgtacccc tagctactca ggaggctaag gcagaaggat tccttgagcc cagaagttcg 4200

aggttgtggt gagctatgat tgccccagct gggacaacag agaccctgtt tctaataaat 4260

aaataaataa aataaagtta catttgaggc atctcagtac tgagatcact aagctgaggg 4320

tgtgggggag ggatagatgg atccagaaag tcccagttgt agtgctgtca ctacaagagc 4380

aggagtggga cacagtggct cacacctgta attccagcac tttgggaggc caaggtggga 4440

ggatcacttg aggccaggag tttgagatca gcctgggcaa catagtgaga tgccatctgt 4500

ataaaaaatt taaaaattag cagggtgtgg tgatgtaagc ctgtggttcc agctactcgg 4560

gaggccgaag caggactgct tgagcctggg aggtcaaggc tccagtgagc tatgattgca 4620

ccactccagc ctgggtgaca gagcaagacc ctgtctcccg gaaaaaaaaa ttaattaatt 4680

aattaattga aataaaatag agcaggctcc ttgttggggt gggggtggag gcaaggttaa 4740

ctctagaaaa agacagagtt cgactcaaat aacagaagag gcctagcaga gatcagccct 4800

gggagtggat gaccttgaga ccaggagtgt cagaggctgg ttgagctgag aggagcctct 4860

ggggtaatga agatcccctc cttgcagaag gagaagagct ggagctggtg gagaaaagct 4920

gtacccactc agagaagacc aacaggaccc tgagctatcg gactggcttg aagatcacca 4980

gccttaccga ggttgtgtgt gggttagact tgtgcaacca gggcaactct ggtgagtagg 5040

gcagcccttg ccatccccaa ccccaagcca tccccagctc aacctcatca tcatcccaat 5100

cctattctta actttacatc agctgaaccc caactccatg ccttatccaa gtcccaaccc 5160

caacccaacc catctctagc tcagctccat ccccaactca ttcttgaccc catatccaac 5220

cccaactcaa cttcacctcc aatcctatgc ccaacttcat gttaccctaa cactgttccc 5280

aactcaaacc cagccctgtt ccaagcacag ttccaacccc atccccaaat caaccccatc 5340

cttatctcag actacccaaa tccccacccc aacgacacaa ttactttcat ccttaaccca 5400

atccccatcc tgcatgtttg tatgtatggt tgttatgtat aacttgcaac tctgccctta 5460

acaagctcac acgcatgcat gagacataga tgagatgtct cagtgatgtt tgtccatctg 5520

actgggcagg agatggtcct gcccaatcca ttgggacggg agatggtcca agcaatagat 5580

actgaggggc caggattcaa gcaaagactt gacgttctta agggaagtcg aaagagggga 5640

agattaaatg ggccgcagta ggctgggaga atcctctgca atagatggtc tctgacttgg 5700

gcagtgaaaa aacaaatgtg aggaaatgtc cataataggc tgggtgtggt ggctcatgcc 5760

tgtaatccaa gcactttggg agaccaaggc gggagaaccg cttgagccca agagtttaag 5820

accagcctgg gcaacaacca taagcccctt tgtcgaattt catggcttca ggctctgatc 5880

attttaagaa aaagacttag aaaaggtcaa attttcctaa attcccacag gagaggaagg 5940

gtggggaagg aatgggtcca tggtgcatgg atccatccgt gaaagagatt cctctttctg 6000

gacctggtga ggaaggaaat agggtaaggc cagtctagtc atggttggtg acatcagtga 6060

gcagagtgct gcgtttgcac tgggttttct ttgttcttca ttcatcctga ttctgcctgg 6120

ttttcatcca tcctgacctc aaacactaat ccgctggtgg ggccaaatca gatgtttgtt 6180

ttgaggaagt gacgagagga tgtgggcttc ccgtctcggt gagatgtgct taaggaaagc 6240

accaatactt gggtctaagt agtgcattca atcagagatg atagcgaaaa tatttaacaa 6300

tattggtcag tgctgggaca aactcctgga gtccgctgct gggcctggtg ttgccccttt 6360

ccatgatggg tcacagggac atgaagggtc ctggggagag agttggcagt ggtcagggat 6420

ccaccaggag cttagagcag cagctatcaa ttcaggaatc taataactga gatacctata 6480

tcatcccttt ttcttttctt ttttctttct tttttttttt gtttcctttt gtttttgttt 6540

tttttttttg tttgtttgtt cgtttgagat ggagtttcac tcttcttgcc caggctggag 6600

tgcaatgcca cgatctcggc tctctgcaac ctccgcctcc taggctcgag cgattcttct 6660

gcctcagcct cccaagtagc tgggattaca ggcatgcgcc accacgccca gctaattttg 6720

tatttttagt agagatgagg tttctccatg ttggtcaagc tgatcttgaa ctcctgacct 6780

caggtgatct gcccgcctca gcctcccaaa gtgctgggat tacagacgtg agccaccgtg 6840

cccggcctca tccccatttc taatgccatt gtaccctgct tgtggcaact tgcatagttg 6900

gttaaactgc actcagtact tacatgatgc tgggaacaca gcaggaccca agacagcctt 6960

gtgccctgcc tgactccatg ggactcacag tcctgtgtgt gtgtgtgtgt gtgtgtgtgt 7020

gtgtgtgtgt gtgtgtgtgt gtgtgtaggg gtgacagacc catccatcac cagacagtga 7080

ctgattatta ggttaatgca aaagtaattg tggcttttgc cattaaaagc aatggcagaa 7140

accacaatta cttttgcacc aacctaatag atttgtctgg gctatgaagg gggaaacaca 7200

ggcagagggg tcagggctgg gatgagggaa gagtaggagc tgtgggaccc tagaggagat 7260

acctgactca ggtcagggaa ggtttcctag gggaagcaac agctaagctg gatcttgaag 7320

gatgagtagg aatcagacta ttaaacagct aatggaggct gggtgcagtg gctcacgcct 7380

gtaatcccaa cactttggga ggcccaggtg ggaggattgt ttcagaccag cctaggcaac 7440

atgactctac aaaaaaatta aaaaattagc cagatgtgat ggtacatgcc tgtgatccca 7500

gctccttggg aggttgaggc gggaggatca ctatagccca ggtggtggag gctgcagtaa 7560

gctatgatcg caccactgta ccccagcctg ggtgacagag cgagatcctg caaaaaaaaa 7620

aaaaaacagt ggaaagggtt ttccagcaga gggagcagca gatacaaagg tggccaaaag 7680

cagtgtggct ggacaaagga aacagtacag tggggcgaga tgagctggaa agcttggcag 7740

gggcatgtta cacaggggtt tctagacatt gtcctgggag cactggggca tcacagaaga 7800

ttttaaacag cgaatgtcat gatcgcaatt atttatttat ttatttttga gatggagttt 7860

cgctcttttt gcccaggctg gagtgcaatg gtgcgatctc agctcactgc aacctccgcc 7920

tcctaggttc aagtgattct cctgcctcag cctcccaaga aactgagatt acaggcaccc 7980

gccaccatgc ccggctaatt tttttgaatt tttagtagag atggggtttc accatgttgg 8040

ccaggctggt ctcgaactct tgacctcgtg atccacccac ctcggcctcc caaagtgctg 8100

ggattacaga cgtgagccac cgcgcccggc ccccatttat atttttaaca aattattctg 8160

acctcaggac agaagataga ctgaaaggaa gcaagagtag atgaggggaa accaacttgg 8220

gggtgggcga ggttgtccag gtggagaaga gggcagcagg actagggtgt caggtgtgca 8280

gaggaagaaa agtggataga tttaagagct attcaggcta ggcacagtgg ctcacgcctg 8340

taatcccagc actttggaag gccaaggcag gaggactgct tgatcccagg agttggaggc 8400

tgcagtgatc acgccactgc actccagcct aggcggcaga gcaaaaccct gtctcaacca 8460

aagccgaaca agagctatta agaagcaggc cttagtggtg gattagatgt gggaggggac 8520

ctgcaattct ggtcatgatg gagtcacttg tatcagacca acccttctgc caatgacagt 8580

tgcaaatgct ggacaaataa taaacaatta ttggaagtca ctggagagca gcaaatgaaa 8640

ggagaaactt ggctgggcaa agtggttcat gcctgtaatc ccagcacttt gggaggccaa 8700

gacaggtgaa ttgctcgagc tcatgagttc aagaccagcc tgggcaacat ggcgaaaccc 8760

cgtctctact aaaaatacaa aagttagcca ggcgtggtgg cacatgcctg tggtcccagc 8820

tacttgggtg gctgaggcac aagaattgct tgaacccggg aggtagaggt tacagtgagc 8880

caagatcaca cgactgcact ccagcctggg cgacagcgcg agactccatt tcaaaaaaaa 8940

aaaaaagaaa aaagaaagaa aggagaaact tgaggcgcta caatctctga aataacagaa 9000

gtgtagaatt tgggttccac agacaacctg gcttttcccc ggagcgcact tgccaatctg 9060

ctgctgtgga agggaaatag agcccagacg gaaagagagt cgctagactg agaagacgga 9120

ggttggagtt tggggctgcc agaatgtctg gaagatgaga gttaaaatcc tggaatggat 9180

gggaccacag aaaagggagt tcccaacctg caaacaaatc tccctcaagt cattggcaag 9240

ctctacatgc acagggtgag agtccaagaa acttggaaga gaaaaatagc aactgggaag 9300

ctacaaaagc gaacaaggct tccagcagac atgtgatgct ggggacacag gttgccaagc 9360

tgaagaggcc ttggtaagca tctcaggctt tccccctaag accaccccag gggtaagggc 9420

caaagtgaaa tagaccagcc ctaaacaagc ctaggtccag tccttgacag gaatgaaaac 9480

ctgactctct gtggaagaag aaaacatcgt ttaggccctt gacagatttt tatctagaat 9540

gtctggcgtc tgatcaaaat ttacaagata gctaggcatg gtggcatgcg tctgtagtcc 9600

caactacttg gaaggatttc ttgagcctga gagttctggg ctgtagtgtg ctatatcaac 9660

cgggtgtctg cactaagttt ggcatcaata tggtgatctc ccaggagcag gggaccacca 9720

ggttgtctaa ggaggggttg aagcagccca ggtcagaaat ggagcaggtc aaaactcctg 9780

tgatgatcag tagcaggatt atgcctatga atagccactg cacaccagcc tggacaacat 9840

agcaagaccc catctttaaa aaaaaaaaaa accaggccgg gcatggtggc tcaagcctgt 9900

aatcccagca ctttgggagg ccgaggtggg aggattacaa ggtcaggagt tcatgaccac 9960

actggccaac atggtgaaac cccttctcta ctaaagatac aaaaaaaaaa aaaattagcc 10020

gggtatggtg gcacacacct gtaataccag ctacttggga gactgaggca ggagaattgc 10080

ttgaaccagg gaggtggagg ttgcagtgag ccgagatcac accattgcac tccagcctga 10140

gcgagagggc aagactctgt ctcaaaacaa aacaaaacaa aacaaaacaa aacgcaagac 10200

tctgtctcaa aacaaaacaa aacaaaaaac aaaaaataaa aaacagtaac aaaaaacctt 10260

acaaggtatc ctgaagaaaa taaaagggac caattgacca gaaaccaaga gaaaaatcca 10320

actgtagaaa cagaattgta gcaactcaga tattggatta taggacagga aacatgaaaa 10380

ttgctacaat cgatatattc aggaaaacag aagaaaggat atgttttagc acctggaatc 10440

cattttttaa aagagtccaa cagaaaatat agaacagtga ctcaaatgaa gaactcggca 10500

gttatgcttg atagaggtca gaaacagcag agcaaaagga ttaaggagct agaagacaga 10560

acagtagaag catgtcggtt gatgcacaga tgtggaagat aagggggtct gccaggtttc 10620

tgccccgggg actggatggg gccgccttgg acgcgtggac ttcaagaaaa agagcagttg 10680

tgggagaaga tggggagttt tgtttaggac aggtgtttga ggtatagcca tggggcctcg 10740

aagagacagt tgcgtgtctg gagctcagag agaagtctag actagagaga gatttgtgag 10800

tgatgagcag atagatggaa atgaagtcat gtcaatattt accccatgag agagtctata 10860

ctgtgagaga taaaaagggc ttaaaacaga gccctggcca ggtgcggtac tcacgtctgt 10920

aatcccagca ctttaggagg ccgaagcggc aggatcattt gagtccagga gttcgagact 10980

agcctgggta acatagcaag accttgtctc tacaaaaata caggaatcag ccaggtgaac 11040

tggcacatgc ctgtagtctc agctactcag taggctgaga tgggagaatc acctgagctc 11100

agggaggtcg aggtggcagt gagccactgc actccagcct tgggacagag caagacccta 11160

tctcaaaaaa aaaaaaaaag aaaagaaaag aaaaaggaaa atagccctaa ggatcattgg 11220

catttacaag agttcacacc agagacccat gagtgaccac ccagagtggg cagaactaga 11280

aatggggaat tcaaggggca gtgggagctc agagcaggta cccgttccag gctgatgggt 11340

cagggagggc ttcctagagg aggtgaaatt gaagatgaca gctgaagaat gaataggagt 11400

tagagaaagt agggctcaag gaaaaggtgt tctaggtatg aacagctatg ttggcaatgg 11460

tgagaatttg tgttttcatt ttcatggaag tttgtgtgag aagacgtggg tggccttgta 11520

gttatttttc tttgaggggg acgattgggc tttggatgta aatgattcgc ctaattacag 11580

ctagcggcct gccagttgag aaccttggct gacatggtcc tctcactttt ttaagacttg 11640

gcctggactc agcttagatg tgtcttgggt caggacgggg agggcatgcc aagcatggga 11700

aatggtgtga gccaagggct gggtagagga atgactctgg tgtttccagg gaaaagtaac 11760

ggatccagtt ggccaacacc acaaggttcg tgcaaaaggc gaagtgatca ggaatcagtc 11820

aagtgagtct ttgatccctg ggacaattag gtagtgtatt atctattgct tatctattat 11880

ctattgctgc attacagatt gcagcaataa aataagcatg tgttatttca caaagtttct 11940

gaaggtcaaa aaccctggag tggcttagct aggtggttct ggctcaggat ccttcatgag 12000

cctgcagtcg agatgtcagc tggggctgca gtcatctgaa ggctcctaag gtagcttcac 12060

tcacatgaat gctcccctcc catggctttg gcacaggcct cagttctgcc acgtgaactt 12120

tcccatggtg ctacttgagt gtcctcacaa catggctgct gcttgcccca gaaaaagtga 12180

tccaagtgag agagccaggc atgtgttcta tgatctagcc acagaaggca tacaccatca 12240

tttccataat atccagttga ctacataggg catccttatt cttttcttct tctttttttt 12300

ttttaattta aagacagagt cttgctatgt tgcccagact agtctcaaac tcccagactc 12360

aagcaatcct cccacctcgg cctcccaaaa tgctgggatt acaggcatga gctgctgcac 12420

ccagccagcc ctattcttta agggcatgaa taccagggag cagagattat tgggggccat 12480

tttggagaca ggctaccaca gggaacaata gaagggtttt aagtaattgc tcattcattc 12540

attcatttaa caaatatgca ttgaatatct tatctgtccc tggccatgtg ccaggtggtg 12600

ttgggacaca gtagtgatca agacagcccc tggccctgcc ctcctggggg ctcacagtcc 12660

agtagaggag acatacctgt caccagacag tgaccaccca gagtgggatg ggggagtaca 12720

ggggtgtgtg ggagcctggt ggatcaggaa gggcttccta gaggaggtga aattagagct 12780

gacacctgaa gaatgagtag gagttaggga aggcagagag caaaggaaag gtgttctaga 12840

cagaaacaac ggcatatgca aaggcctgga tgccagggaa catgtgtcgt cgcagactgt 12900

gggaagtcag tgtggagcag agtctcaggg tgtagtggag ggagacgagg caggagaggt 12960

gctcaggccc cagtatttga taggctttga atcccatgct gaggatcttg gaatttattt 13020

tcagggcagt agggagctat ggaagagtct tgagcagagg agagacaggg tcagatatga 13080

gtattagaaa tatccttcta agggcccgag tgcagtggct catgcctgta atcccagcac 13140

tttgggaggc caaggcatgt agatcacttg aggttaggag ttcgagacca gcctggccaa 13200

catggtgaaa ccctgtctct actaaaaata caaaaattag cccagcgtga tggtgcacgc 13260

ctgtagtccc agctattcgg gaggctgagg caggagaatc acttgaaccc ggggagtgga 13320

ggttgcagtg agccaagatg gtgccactgc gttccagcct gggtgacgga gtgagactct 13380

gtctcgggaa aaaaaaaaaa aaaaaggaaa gaaatgtttc tagggccatg tggagatgct 13440

gcactggggt gagtaggggg tgacaagaat ggaaacccag aggctaggga ggtagctgga 13500

gttgaaggag gtctgagctg gactggtcca tggggctgga aaggaggggg tggattctag 13560

aaagattcag caggcagaat aggcagggct caatgactgg ctgcagggtg ggataaggag 13620

gaattgattt tgagtgagtc cttgcagagc tggggctcca ggactgtccc catggagtct 13680

cactcccctc cccctcttcc aaacaggccg ggctgtcacc tattcccgaa gccgttacct 13740

cgaatgcatt tcctgtggct catcagacat gagctgtgag aggggccggc accagagcct 13800

gcagtgccgc agccctgaag aacagtgcct ggatgtggtg acccactgga tccaggaagg 13860

tgaagaaggt gagccccaac ctgctggcaa ctcctcctcc ctgctctgct cctccctaag 13920

actgcactta acaaacaacc ccaaagtaac agggatgtca gcaagggact aagttctctc 13980

atacacaaga ggtccagtga taggaagtcc agggccacaa ggacaggtcc agagtcacca 14040

ggaacccaga tgccttctca tttcttttta tttatttatt tatttatttt ttgagacaga 14100

gtcttgctct ctgtcaccca ggctggagtg caatggcatg atcttggctg gctgcaacct 14160

ccgcctccca ggttcaagtg attctcctgc ctcagcctcc tgaatagctg ggattacagg 14220

tgtgcatcac cacgccttgc taatttttgt atttttagta gagatggggt ttcaccatgt 14280

tggtcaggct ggtctcaaac tcctgacctt gtgatccacc cgcctcaacc tcccaaagtg 14340

ctgggattac aagcgtgagc caccacgccc ggccaccctc ttgtttctta cacattactt 14400
ccattctcaa aatgcctcat agtctaaaat ggctgccaga gctccgggtc tcacatctaa 14460
gttccaggaa ggaggaagaa ggaaagaagg gacaaaaaag ggggcactcc tccttttgta 14520
agggttctcc tttctggaag tcccataaag cacttctatt tatatcacat tagtccgaac 14580
ttcatcacat gactacctgg agaggctgga atatgtagtt ttcgacaaga tctatgactg 14640
ttctagataa aatgagggtc ttatgatgaa gggtgaagta gagaatggtt gaatgcctgc 14700
tatttcgggc aaatcccatc ttagtccctc attcacaatc tgacatctga cctctgtccc 14760
ctggccccca tagggcgtcc aaaggatgac cgccacctcc gtggctgtgg ctaccttccc 14820
ggctgcccgg gctccaatgg tttccacaac aacgacacct tccacttcct gaaatgctgc 14880
aacaccacca aatgcaacga gggcccaagt aaggaacggg agacacaggc aaggcctggg 14940
gtcgggcagg ggcatgcact caggcagaca gctgcgcagt cactctctgg caatcaagtc 15000
ctctctgggc ctctgtttgc ttttctgaaa aatgggagta tcaggccttt tttttttttt 15060
tttttttttt gagacggagt ctcactctgt tgcccaggct ggagtgcaat ggcgtgatct 15120
ccgctcactg taaacttcgc ctcacgagtt caagcgattc tcctgcttca gcctcctgag 15180
tagctgggat tacaggtgcc caccatcacc cctggctaat ttttctattt ttagtagaga 15240
tgggggtttc accatgttgg ccaggctggt ctcgaactcc tgacctcaag tgatctgtct 15300
gcctcagcct cacaaagtgc tggggtaaca ggcatgagcc actgcaccca gccaggcctt 15360
tcttcaaaga agaacttcgt aaggattcaa tgatactatc agccctcgcc aagacctgtg 15420
cttcctgtct cttttgtctc tctcaaattt tcccactcct cccctgctcc actgccacca 15480
cctgggctga gccacgctga cttcttgccc aggttgttgc aatgacctcc tctctggtct 15540
ccctgcttcc actcctgcca ccctacagcc aaggctcaac ccagcagcga gtgatttttt 15600
ggggggtatg ggggggacag ggtctcgctc tgtcacccag gctggagtgc actggcatga 15660
tcatggctca ctgcagcctc aaactcctgg gctcaggaat tcttcccacc taagctactg 15720
gagtagctgc gactacaggt gcatgccacc gtacccagct aattttgtat ttttggtaga 15780
gatggggtct tgctttgttg cccaggctgg tcttgaactc ctgggcttgg cctcccaaag 15840
tgttaggatt acaggcatga gccaccatgc accaccaatt ttttattttt taatgtaaat 15900
cagatcatgt caccctcctt tctcaggcac ttcaatgact tccccttcct cttagaataa 15960
gatccaaaga ccttattctg ttggcctatg taatctggca cctgtctgcc tcacagtctt 16020
tgtttagatt cgtttttggg gttttgtgtt ttctttagac atagggtctt gctctgccac 16080
ccaggctggg gtgcagtggc acaatcacgg ctcactgcca cctcaaactc ctgggctcca 16140
gtgatcctcc cacctcagct tcccaagtag ctgggactac agatgcgcat cattgcaccc 16200
agctacattt tttttttttt tctgagatgg agtcttgcgc tgttgcccag gctggagtgc 16260

agtggcgcga tcttggctca ctgcaagctc cgcctcccgg gttcacgcca ttcccctgcc 16320

tcagcctccc gagtggctgg gactacaggc acccaccacc atgcctggct aatttttgta 16380

tttttagtag agacagggtt tcactgtgtt agccaggatg atctcgatct cctgacctca 16440

tgatccgcct gactcagcct cccaaagtgc tgggattaca ggcgtgagcc accacgcccg 16500

gccgcaccca gctaaatttt taaaactttt gtagagatgg ggtctcacta tgttgccggg 16560

gctggtatca aactcccagg ctcaagcaat ccttctgcct tgacctccca aagtgctggg 16620

attacaggca tgagccgctg cacctggcct tcatttagtt tctttctttc ttcctttctt 16680

tcttttttta gatggagtct cgctctgtca gccaggctgg agggcagtgg cgtgatcttg 16740

gctcactgca acctccgcct cccaggttca agcgattctc ctgcctcggc ctcccaaagt 16800

gctgggatta caggcatgag ccgctgtgct gacgttcatt tggtttctat aatcaccaaa 16860

gcccatctgg tctcatggcc cttgcagatg aatatcctcc cttagaacac atcttcccca 16920

agagttcacc ctgctggcaa cttctcatcc attaggcctc agctttaatg tatcatcttc 16980

agggatgctt tcactgtccc tcccctccag tgtaatctag atccctgtct ctattaccca 17040

gcactgtcaa cagatagaaa tgttccctat ctgtcctgtc caatattaca gccaccactg 17100

tatgtggtca gtgagcactt gaaatgtgaa ctgaatttta agattcgatt taatattaat 17160

ttatttaaat gtaaacaacc acatgtggcc agtggctacc agattgggta gtgtagctct 17220

aaattgtaaa ttctggctgg gcacagtggc tcacacctgt aatcccagca cttttggagg 17280

cggaggcggg agggtcgcct gaggccagga gtttgagaca agcctaggca acatagcaag 17340

actccgtctc aaaaaaaatt ttttagtaat aataatagta ataacttgta agttctggcc 17400

aggtacagtg gctcacacct gtactccgag aacattggga ggctgaggca ggcagattgc 17460

ttgatcccag gagtttgaga ccagcctgag taatgtgtca aaaccccatc tctacaaaaa 17520

acagaaaaat tggctaggca tggtggtgca cgcctgtagt cccagctact caggaggctg 17580

atgtggaaga atctcttgag ccggggtggt cgaggctgca gtgagctaca attgcgccac 17640

tgcactccag cctgggcaac aaactgagac cctgtatcaa aaataaataa ataagttgta 17700

acttttgtga gagcaaggac agtttcatct tacatagttt tacccagcac ccagcacggt 17760

gcctggccag aaatgggact catggggagt tgaaagttgg ggtttctctc aagccctcga 17820

aaaccatccc cctccaaatg ccattcagac gcctgaccac aaggtggctc catgacatct 17880

ctggctcgga gtggaagtcc tggggaggtc attcaaggaa gtggagatcc aagcactaat 17940

tttcttggtc tctgtgtctc tggtcttctc ctaagtcctg gagcttgaaa atctgccgca 18000

gaatggccgc cagtgttaca gctgcaaggg gaacagcacc catggatgct cctctgaaga 18060

gactttcctc attgactgcc gaggccccat gaatcaatgt ctggtagcca ccggcactca 18120

cggtgaggcc ctctccgagg ctgggaggga acacttattg ggggtggaga gttccgcaag 18180

agattgactt ccagttaagc tggagttaac tgtggtgggt ctccctgtgg aaaaggtggg 18240

atttccttca ggtgggtgaa tattattgta aggaattagg acttgtccat caggatgtgg 18300

acatttttgc catggaaaag tggaattccg gctgggtgcg gtggctgaag cttgtactct 18360

cagcactttg ggaggctgag gccggaggat ctcttgaggc caggagtttg agaccagcct 18420

gggcaacata gcaagacccc catctctaca aaaagaaaaa tggaattctt tggtggattt 18480

ctcgtgggtt gtggggattt gctgaagaca tcttgggctt tcatcctgtg gtgtcctgga 18540

aaggtctctg tattccagtg gttctcaaac tttttggtct caacttcttc atgcttttaa 18600

aaattatttt tggccaggtg tggtggctca cgcctgtaat tgcaacacct tgggagggtg 18660

aggaggagaa ccacttgagg tcaggagtat gagagcaagc ctggccacca tggtgaaacc 18720

ctgtctctac aaaaaattac aaaaattagc tgggtgtggt ggcgtgcacc tataacccca 18780

gctactcagg gggctgaggc acgagaatca cttgaaccca ggaggaggag gttgcagtga 18840

gtggggatca ccccactgca ctccagcctg gatgacagag ggagactttg tctcaaaaaa 18900

aaaaaaaaac tctttattgt ttatatcagt gatttctata gataaatgcc acattaaaat 18960

taaaactgag aaccaggagt ggtggctcac gcctgtaatc ctagcacttt gggaggccga 19020

ggtgggtaga tcacctgagg tcgggagttc aagaccagcc tgaccaacat ggtgaaaccc 19080

catctctact aaaaatacaa attagtcggg catggtggca catgcctata atcccagcta 19140

ctcgggaggc tgaggcagga gaatcacttg aacctgggag gcagaggttg cggtgagcca 19200

agattgtgcc attgcactcc agcctgggca acaagagtga aactccgtcc caaaaaataa 19260

aataaaataa aattaaatta aaattaaaac tgagaaatct tttttttttt tttttgaaat 19320

ggagtcttgc tctgtcgcca ggctagagtg cagtggcatg gtctcagctc actgcaacct 19380

ccccctcctg ggttcaagca atcctcctgc ctcagcttcc tgcatagctg ggattacagg 19440

catgagccac catgcccagc ttatttttgt aattttagta gagacggggt ttcaccatgt 19500

tggccaggat ggtctcgatc tcctgacctc atgatctgcc tgcctcggcc tcccaaagtg 19560

ctgggattac agatgtgagc caccgtgccc agccaaaact gagaaatctt taaagattta 19620

tttatgaatt catcttataa caataaaaac tcattacaca ttaagataaa taacatttta 19680

atgaaaaata agtatagttt ccaaagaaaa aatgtaatga tagccaggca tggtggctca 19740

tgcctataat cccaatactt tgggaggcca aggtgggcgg gtcacttaag cctagggatt 19800

tgagaccagc ctgggcaaca tagcaagatc ctgtctctac agaaaataca aaagttagct 19860

gggcatggtg gtgcatgcct gtagtcccag ctgcttggga ggctgaggtg ggagggtcac 19920

ttgagcctgg gaggtggagg ttgcagtgaa cggagattgc accactgcac tgcggcctgg 19980

gcaacagagc gagaccctgt cttaaaaaaa caaacaaaga aacaacaaaa aaaaaaccag 20040

ccaggcgcgg tggctcacgc ctgtaatacc agcactttgg gaggccaagg cgggcagatc 20100

acaaggtcag gagttcgaga ccagcctggc caatatggtg aaaccccgtc tctactagaa 20160

atacaaaaat tagctgggca tggtggtcgc gcctgtagtc ccagctactt gggaggctga 20220

ggcagaagaa ttgcttgaac ccgggaggca gaggttgcag tgagccgaga tcgtgccact 20280

gcactccagc ctgggtgaca gagtgagact ccatctcaaa aaaaaaaaca aaaaaaaagc 20340

gtaatgagaa aactggcatt attttgctgt gttgcacatc tctttaatgt ccagcttaat 20400

agaagacagc tgggtcctca tggaagcttc tgctttcagt ctcttgcaat atcacatgtc 20460

ctggagtttt tggaaaagcc cattgtacac ttatgagagt gaaaaagcca tgtgaaatca 20520

cagatctcct gaaagggtct ctggggtccc tgagccacac tttaagaacc gctgctcgct 20580

tcctttcttt cttttttttt ctttcctttt ctttctctgt ctctctttcc ctttctttct 20640

ttctttcttc cttccttcct cccttccttc cttctttccc ttctccttcc ttccttcctc 20700

cctctctctc tctttctttc ttttctttcc cttcttccct tcctcccttc cttcttccct 20760

ccctccctcc ctcccttcct tcctttcttt ctttttattt gttttttgaa acagggtctc 20820

actctgttac ccaggctgga gtgcagtggc gtgatcataa ctcaccacag cctccatctc 20880

ctgagctcaa gtgatcctct catctcagcc tcccaagtag ctgggactac agctgttttt 20940

ttctttttta tgtttgtaga aacagagttt tgctatgttg tccaggctgg tctggaactc 21000

ctgggctcaa gctatcctct ctccttggct tcccaaggtg ttgagattac aggcatgagc 21060

caccacacct ggccctgaga actgctgctt tctctaggtg gtagtgaagg tggcacccac 21120

tgcaaggtgg caggtcacct actggagaat tccagtcctg ggcccaggag ctggaagtct 21180

cactccgtct tctctttcct cagaaccgaa aaaccaaagc tatatggtaa gaggctgtgc 21240

aaccgcctca atgtgccaac atgcccacct gggtgacgcc ttcagcatga accacattga 21300

tgtctcctgc tgtactaaaa gtggctgtaa ccacccagac ctggatgtcc agtaccgcag 21360

tggggctgct cctcagcctg gccctgccca tctcagcctc accatcaccc tgctaatgac 21420

tgccagactg tggggaggca ctctcctctg gacctaaacc tgaaatcccc ctctctgccc 21480

tggctggatc cgggggaccc ctttgccctt ccctcggctc ccagccctac agacttgctg 21540

tgtgacctca ggccagtgtg ccgacctctc tgggcctcag ttttcccagc tatgaaaaca 21600

gctatctcac aaagttgtgt gaagcagaag agaaaagctg gaggaaggcc gtgggccaat 21660

gggagagctc ttgttattat taatattgtt gccgctgttg tgttgttgtt attaattaat 21720

attcatatta tttattttat acttacataa agattttgta ccagtggaca aggccaggta 21780

tgcccttctt tgtagtgtct atctttggga ggataaatgg tgggggacac cttttcacag 21840

gctcctggaa tcaggggtca tgtttgaggc ccctgcctag gttggaagtc ctcctggggg 21900

ctgcctccac atctatttct ggactccaaa tgctgacaga gcaggagcac tgccatcttg 21960

aacaaacacc accattctta gttcctcttg attaaaaatc ggctaaatcc ggacgtggtg 22020

gcgggtgcct gtagtcccag ctattcggga ggctgaggca ggagaatggc gtcaatccgg 22080

gaggtggagc ttgcagtgag ccgagattgc accactgcac tccagcctgg gtgacagagc 22140

aagactctgt ctcaaaaaaa aatcagctaa atccagcccc aaaacatcag cctaatggct 22200

accatcagca taaccagaaa cattccaacc ctaagataaa cccctctctg accagaaaca 22260

tgccaacccc cagatagcct cccttccgac cagagacttt ccaaccccac aataaacttt 22320

tcctcacatg aaaacattcc gaacctagat aagcaccccc ttccaaaact cttaaatatc 22380

cttagtctgt aaaaaaaaaa aaaaaaaagg gctctctaac ctaactcagc caaaagcccc 22440

cctcaggttg gttttctcta aaataaacct gtccttcacc gtcaagccac atttcatgtt 22500

tctttccttt tcctttttct tttcttttct tttctttttt tgagatggag tttcactctt 22560

gttgcccagg ctggagtgca atgacacaat ctcagctcac cacaacctcc acctcctagt 22620

tcaagggatt ctcctgcctc agcctcccaa gtagctggga ttacaggcat gcaccaccac 22680

acccggctaa ttttgtattt ttagtagaga cagggtttct ccatgttgtt caggctggtc 22740

tcaaactccc aacctcaggt gatctgccca ccttggcctc ccaaagtgct aggattacag 22800

gcatgagcca ccgtgcctgg cctcctcttt ctttaattct tacaaatgca agcttccaga 22860

tcacacactg aacactaacc ttctagtctt ctcctaaact tcaaaggttg caactatttt 22920

gttgttctaa ggagacatcc ccacatttcc cagcatgctt tgggaaagga gacggataga 22980

agataacctg atggccagaa ctccctgcat gcactgcagt gaggaataag gacaggagtg 23040

cacttcccct gatgtgctgg ggaccgggga taaaggctgg gctctatttc ccgggatgcc 23100

tcaggactgt cagaaaaaga gacagaacta ccatttcaat ggcactctag gagcacaata 23160

ttgaaaaatt catgttcttc tcatcatttg ctaaggcaac ccatggagac aaaaattaca 23220

ttccccatca aatcttgtgg ctaatgaaga aaaaataaga tatcttcggc tggctgtggt 23280

ggttcacgcc tgtaatccca gcactttggg aggccgaggt gggcagatca cttgaggcag 23340

gagtttgaga ccagcctggc caatttggca aaaccccgtc tctactaaaa ttacaaaaat 23400

taaccgggta aggtggtggg tgcctgtaac tccagctact cggaaggctg aggcaggaga 23460

atcgcttgaa cccaggagga ggaggttgta gtgagccaag atcacaccac tgcatgacac 23520

agtgagacta tccccaaaaa aaaaaaaaaa aaaaaaaaga catcttccat ggtatttggg 23580

ggcaggtgag ggacaaggtt cttgtttagg gctttctcta atgacatatc accacctgtc 23640

actgtagtgg cttaaagaag tgaggtctgt acttctgcct cttgaatatg agtgactctg 23700

tgactgcttt gaccagtaga atgtggtgga agttttgctg tgctatgttc tggaaccaag 23760

ttttaagaaa ctttcatttt tcactttctg aatcttagaa cgctcactct ggggaagctg 23820

gttgccatgt aaaagtacta ctgccctgag accaccatgc tgtgaggaag cccaagctac 23880

tcatgtataa atgccatgtg gagatagagc cccagatgtt tcagccatct cagcccaggc 23940

accagacaag tgggtgaaga agccaccttg gacatgtagc cccagcagat gtgatataga 24000

gaagaaacag gaaacttggc tatattagtt tcctagggct gcctgtgata aattattaca 24060

aactttataa actaacacat tgtgtgccta tatcaaaaca tcatggaagg acaggcacag 24120

tggctcatgc ctgtagtcct agcactttgg gagggtgaga aaggaagatc tcttgagctc 24180

aggagttcaa gatcagcctg ggcaacacag tgagacctca tctccactaa aaataaaaaa 24240

aaattggctg ga 24252

<210> 29
<211> 17328
<212> DNA
<213> Homo sapiens

<400> 29
tccacccaag agcaacctgg aactaagtta ttcggcaacg aactgttcca ctttgttgtg 60

aggcaataga tgtggaaatt ccctgacgag gggctctgtc ctcatacttc ctgcggagct 120

tattgtcgta agaatatctg tcatcctgct aatgtgcatt gaaaggagag caacggggct 180

gaggccgtgt cagcacgatg gaccccaaac agaccaccct cctgtgtctt ggtgagtttc 240

agagtaaaag tgggttagag gggaagatag agaaatccca aaataatcag ggtgtctctt 300

aacagtgtga ctaggagatt ttagtggctg ccaaggagat tctgatctcc ttagtggaaa 360

ggccgtcttt gtcaatgtat ctataacttt gtctctaccc aagcccaagc tagcttgtgg 420

ggctcaaggt ttaatatttg tattaaacct atagtgtgtt atctgggatt catgatggtc 480

ccaaggttct tatcaaggag agacttagag gctggaatct gaaaggtaaa aataaagaat 540

gaacctcaaa actgtgattg ttgtggaagg aaaacatatg atagaacccc atatagaaat 600

atggttacta gtattttgtt gaagattttt gcatttatgt tcaacaaaga tattatccag 660

aagttttctg tttttgttgt atctctgcca cattttgtta tcaggataat gttggcctca 720

tagaatgagt tggggaggag tccctcctcc aggatttttt tcaatagttt cagtaggaat 780

aatactagct cttcttggcc gggcgcagtg gctcacacct gcaatcccag cactttggga 840

ggccaaggca ggcggatcac aaggtcagga gatcaaaacc atcctggcca acatggtgaa 900

accctgtctc tactaaaaat acaaaaaaat tagccaggcg tggtggcggg cgcctgtagt 960

cccagctact cgtgcggctg aggcaggaga atggcatgaa cctgggaggc agagcttgca 1020

gtgagccaag atcatgccac tgcactccag cttgggcgac acagcgagac tctgtctcaa 1080

aaaaaaaaaa aaatgccagc tcttctttat atatctggtg ggattgagct gtgaatccat 1140

ctggtactgg tctttttctg gtctgtcatt acagagggtg atttgtcgta aaggttggaa 1200

atggaagctt gatttttcat aaatctctct cttccagtgc tctgtctggg ccagaggatt 1260

caggcacagg aaggtaagtg tcctgtaaat ctctcccagc ccctttagac cctcttggga 1320

gctctaggat aaagaaattg aagaatagcc tgaagcacca ttcttatttt aatccccatt 1380

ctagttgttt ctgctgtgct tctcttgcat aatttctatc tcactttgtt atctccaaac 1440

ccttcagact cattaatgct caggcctgga tttatagtta gtccttgcct gtgttagact 1500

gtccatgaag gatctgtaat ttactgaatg ctcaaactgc aagaatgagg aagtcaggag 1560

tcatctgccc aatatccttc cttatgctga ttctattttg ttttagcaac ccacttcctc 1620

ccgtcacttc atttaaaagg atgctgccat agtctaaccc tactgaacac tctagcattc 1680

tgtagtacta ctgcagtact aagcatgagg cagtcttagt gtactactga atattctgcc 1740

accccaacta ctactgcctt agcctcctaa tgggtgtgag ccccacgtcc atccatgtct 1800

tctctcttcc agctccttct aaagcctgaa ttatttgtgt gttgaacaat actcattctt 1860

cctatccatg agcatggaat gtttttccat ttgtttgtgt catctatgat ttctttgacc 1920

agtgttttgt agttctcctt gcagagatct ttcacctccc tggttagctg tattcccagg 1980

tattttattc tttttgcagt aattgtgaat ggattctatt cttgatttgg ctctcagctt 2040

agatgttttt ggtgtatagg aatgctactg atttttatat attgattttg tatcctggaa 2100

ctttgctaaa gttgtttatc agattaagaa gtgtttgggc agagactgtg gttttctagg 2160

tatagaatca tatcacctgc aaacagggat agtttgactt cctttcttcc tatttggatg 2220

ccttttattt ctctcttgcc tgattgctct agctaggacc tccagtacta tgttgaacag 2280

aagtagtgag agacggcatc catgtctttt gccagttttc aaggggaata cttccagctt 2340

ttgcccattc aatatgatgt tgactgtgag tttgtcatac atcattctta ttattttgaa 2400

atatgtttct tcaatgccta gtttgtcgag gttttttagc ataaagggat gttgaatttt 2460

atcaaaagct ctattgagag gattatgtgt gtggggaggg ttgttctatt tatgtgatga 2520

atcatattta agatttgtgt atattggccg ggcactgtgg ctcatgcctg taatcccagc 2580

actttgggaa gccaaggctt gtggatcatg aggtcaggag atcgacacca tcctggctaa 2640

cacggtgaaa cctcgtctat actaaaaaac acaaaaaaat tagccaggcg tggtggtggg 2700

cacctgtagt cccagctgct cgggaggctc aggcaggaga atggcgtgaa tccaggaggc 2760

ggagcttgca gtgagccaag ttcacgccac tacactccag cctgggcaac agagcgagac 2820

tcctatatcg aatcaacctt gcatcccaga aataaagcct acctgatggt ggtggattag 2880

ctttctgatg tgctgctgga tagtttgcta gtattttgtt gaggattttt gcatttatgt 2940

tcaacaagga tattgtcctg aagttttctg gttttgttgt gtctctgcca tgtttttgca 3000

tcaagatgat gctggtctca tagaatgagc tggggaggca ttcctcctcc tgaatatttt 3060

tggaacgttt cagtaggtat agtaccagct cttctttata tatcagatgg gattcagctg 3120

tgagtctgtc tggtactggg ctttttctgg tctgtaggat ttttattact gattcaattt 3180

tggagctcat tattggtctg ttcatgtatt caatttattc ttggtttgat ctcaggaggg 3240

tgtatgtgtc caggaatttc tccatttatt ctggattttc tagtttgtgt gcatagaggt 3300

gttcataata ttctctgatg attgtatttc tgtggggtga gtggtaatat accctttgtt 3360

gtttctaatt gtgtttatcc ggatcatctc tcttttcttc tttattagtc taactagtca 3420

tctgtcttac taattttttc aaaaattcta ctcctcgatt tgctgatctt ctgaatgctt 3480

tttcgtgtct caatctcctt cagctcagct ctgattttgg ttatttcttg tttcctatga 3540

gctttggggt tgatttcctc ttggttctct tagttcctct tgttatgatg ttaggttgtt 3600

aatttgaact ttttctagct ttttgaagtg ggagtttagt gctataaact tcccccttaa 3660

cactgcctta gctgtgtccc agagattctg ctatatttac ccaaaaattc cagaacagac 3720

tgcttaattt ccatgcattg tacagttttg agtggttttc ttagtattta ttcctatttt 3780

tattccactg tgctctgatt tcgcttttct ggatttgcta aggattgttt tttttttttt 3840

tgaaatggag tcttgctctg tcgcccaggc tggagtgcag tggcgcaatc taggctcact 3900

gcaagctccg cctcccaggt tcacaccatt ctcctgcctc agcctcctga gtagctgaga 3960

ctacaggtgc ccgccacccc gcccggctaa ttttttttgt atttttagta aagacggggt 4020

ttcactgcgt tagccaggat ggtctcgatc tcctgacctc atgatccgcc agccttggct 4080

tcccaaagtg ctgggattac aggtgtgagc caccgtgccc agcctgctaa ggattgctgt 4140

atgtctgatt gtatgattga ctttagagta tgtgccatga ggcaatgaaa acaatgtaga 4200

ttctgttgtt ttgggggtgg agagttctgt agatgtctgt caggtccatt tgatccactg 4260

ctgagttcag gtcctgaata tctctgtttg cctcaatgat ctaatactgt cggcgggatg 4320

ttaaagtctc cccctattat tgtgtggttg tctaagtctc ttcgttggtc tctcagaaca 4380

cgctttatga atccgggtgc ttccatgtta ggtgcatata tatttaggat agttaggtct 4440

tcatgctctt tttttaattt tttttttctt tttctttttg attcagcagt tgggctatta 4500

cacactcctt agcagattcc gacttccgtg gccactgtcc tgctatggtc ttcatgttga 4560

attgaaccct ttaccatgat ttaatgccct tctttgcctt ttttgatctt tgttggtata 4620

aagtctgttt tgtctgaaat tttaatagca gctcctgcat tttttttttt ttttggcttt 4680

ccatttgctt ggtagatttt tctccatttc tttactttga gcctatggat gtcattgcat 4740

atgagatggg tttcttatag gcagcataat gttgagtctt gctttttttt tttttttttt 4800

ttttgagatg gagtctcact ctgtcaccca ggctggagtg cagtggtatg atcttgactc 4860

actgcaactt ctgcctccca ggttcaaatg attctcctgc ctcagcctcc caaatagctg 4920

ggattacagg tgtgtaccac cacgcccagc tatttttttt ttttattaga gatgggattt 4980

catcacattg gccaggctaa tgtcgaactc ctgacctcaa atgatccacc cacctcagcc 5040

tcccaaagtg ccagggttac aggcgtgagc cactgcacct ggcctcttgc ttctttatcc 5100

aacttgccac tctctgcatt ttaattagga caattagtcc atttacattc aaagttagca 5160

tttacatgtg cagatttttt cctgtcatca tgttgttagc tggtttggtt attatgcaga 5220

cttgtttgtg tggttgtttt atagtgtcac tggtttatgt acgtaagtgt gttttctatt 5280

ggctggtgat ggtcttttct ttccatattt agcgttcctc ttaggacctc ttgtaaggca 5340

ggcctgatgg taataaattc cctcagcatt tgcttgtctg taaaggatct tatttctcct 5400

tcacttatga aacttagttt ggctgggaat gagattcttg gttggaaatt cttttcataa 5460

gaacattaaa tataggcccc caatctcttc tggattgtag agtttctgct gaaaggtctg 5520

ctgttagctc gatggcattc cctttgtagg tgacctgccc cttctttttt gctgcctttt 5580

cacatttttt tttttttttt tgagactgag tcttgctctg ttgcccaggc tgccaggctg 5640

gagtgcaatg gcgtgatctc ggctcactgc aagctccgcc tcccggattc acgccattct 5700

cctgcctcag cctccccagt agctgggact acaggtgccc accaccacgc ccagctaatt 5760

ttttatattt ttttagtaga gatggggttt caccatgtta gccaggatgg tctcaatctc 5820

ctgacctcgt gatccgcccg ccttggtctc ccaaagtgct gggattacag gcgtgagcca 5880

ccgcgcccgg ccgacatttc tttctttcat ttctaccttg aagaatctga tgattttgtg 5940

tcttggggat gctcttcttg tgtagtattt tgcaggggtt ctctgtttcc tgcatttgat 6000

tcttggcctc tctagtgacg ttggggaagt tttcatgaac aatacactga aatatgtttt 6060

ccatgttcct tgctttctcc ccatctcttt cggggatgcc aatgggctat ttggtctctt 6120

ttcatgatcc catatttctt agaggttttg ttcattaatt tttattcttt ttcttcattt 6180

ttgtctgact gaattagttc agagagccag tattcatgct ctgagattct ttccttattt 6240

tgctttattt tgccattaat acttgtgatc gcattatgaa atctcgtagt gtgtttttca 6300

gctccatcag atccgtttgg ttctttcaaa atgaccatct catagattag cccctctgtc 6360

attttattgt aatctttagg ttccttgcat tgggtttcaa ctttctcctg aatcttgatg 6420

accttaattt ctatccatat tctgaattct atttctgtca tttcagccag gtaaagagcc 6480

cttgctggga agcttgtgtg gtaatttgga ggaaggaaga cactgttgct ttttgagttg 6540

atggagttct tgctcagttc tttctcatcc atgtgggcta atgttccttt gagtgtgctg 6600

caatttgaat tttttctttt tttcttttaa ccgtgatgta atttgagcac agtcagtaga 6660

cttcttttct ggatggtttc agagggctgg ggcttcgcac agggtcttta tttatagcta 6720

aattcttgtc cttggtttca cagggaggta tattagcgag catttttggt gttgaagttt 6780

gggctgcaat ccggtaaatg atgcttcagc acaacggcca gtaggtcatt cctcatgatt 6840

gccgctgtgc tccctctcac gctctgaaag tgcgggctcc tctcccaccc aagtgctggc 6900

tgcagatctg ggctcggcac tcccaggctg cacatcacag ctctggggtg agctcagact 6960

ttatgttccc tccgtggctt gggggcagca ggggaaggga ccttagcagc ggttgtggca 7020

gacggccttt cacttgtccc ttggaactcc accccagaga gatgtggagc cactatcagt 7080

gcgatgagcc aagagtgagg cgactgcact gtgggtccaa gctaggggcc ctgcctagtg 7140

atgagcaggg gggacaggtg ggtcacaggg gtgacagact ggcctcttct ccttagggca 7200

gtttgccgga ggtgtggttg aagcactcag agtctttgct ccttccccag tctgagggca 7260

gcaaggccag taccaccgca gtggcagcgg cagagtgacc ttccgttgcc tctgggagct 7320

ccgccacaga gaaacgcaga ctcacagctg ctgggaacgc tccgccagag ggtggggctg 7380

ttgtgccgcg gaatcaagct ggggcttgtt gaagagcagg gggttgaggg ctcacaggga 7440

gaggagactg agctcctctc cgtatggcga ctgcggtgtg ctggaagcat gaatgaaggc 7500

cgggcgcggt ggactcacgc ccggaatccc agcactttgg gaggccgagg cgggcggatc 7560

acgaggtcgg gagatcgaga ccatcctggc taatctggtg aaaccccgtc tctactgaaa 7620

acacaaaaaa tgagccgggc gtggtggcgg gcgcctgtag tcccagctac tcgggaggag 7680

gatgaggctg aggcaggaga atggtgtgaa cccgggaggc gcagcttgca gtgagccgag 7740

atcgggccac tgcactgcag cgtgggcgac agagcgagac tccgtctcaa ataaataaat 7800

aaataaataa ataaataaat aaataaataa ataaataaat tgaagcatga atgaaggccc 7860

caggctcctt gcttcttccc cagatcacgg gcagcagagg cagaaccctt gccatggcag 7920

tggcagaggg gctgtcagtt gcctctggga gccactcccc agggaaacac gagccaccac 7980

cagtgagtgt gctgagggcg gggcagctgc tctgcactcc cgagctgggg gctctgcctg 8040

gtaaagtggg ggtgggagct cacggggaag agagactgga ctcctctctg tctgatggct 8100

gtggcataat gaccgggccc ccacacatga aaaagaattc tgggaactca aaaagccagt 8160

gtgtccccac catggacccc ttggattgtg tttcaaattt ctcctcaatt tcgaagagcg 8220

tcctggccat ccagattctg aattctataa ccctcgtttc attcatctca atgtagctaa 8280

gaaccagatt tctggggaac tatcgagttg ccagagttct tgtgttgatt cttttttttt 8340

tttctttttt ttttcttttt tgtggcagag tcttactctg tcgcccaggc tggagtgcag 8400

tggcacgatc tcagctcact gctgcaacct tcacctcccg ggttcaagcg attctcctgt 8460

ctcagcctcc tgagtagctg ggattacagg tgtgtgccac cacgcccggc taatttttgt 8520

atgtttagta gagcccgggt tttgtcacgt tggtcaggct ggtctcaaac tcttgacctc 8580

cggtgatctg cccacttcag cctcccaaag tgctgggatt acaggtgtga gccaccgcgc 8640

ccggccttgg tgttgattct ttctcttgtg tgagggctgg tgttccttta actgtgatgt 8700

cggttgagta cagtcgcttg gcttcatttc tgggtgtttt cagatgccag gactctgcac 8760

aggatcttta tttgtggctg aatttttccc ttcattgtat actggcaaaa tttttcagtg 8820

ttgtattttg aagtgtgatc cagtaggtgg cacttaaaag ggttggccag catacaggat 8880

cttagccaca aggctctttt gtagttttgt ttcgtttttt gtttcgtttt ttgacacagg 8940

gtcttgttct gtcgcccagg ctggagcaca gtggcacaat ctcggctcac tgcagcctct 9000

acctatcagg ctaagtgatc ctcctgcctc agcctcctga gtagctggga ctacaagcac 9060

gcagcaccat aaagagaaaa tttttgtaat tttttttttt tttttgtaga gatggggttt 9120

caccatgttt accaggctgg tctcaaactc ctgggctcaa gcaatctgcc tgccttggcc 9180

ccctaactct tgtattttga caaagtcggc agtagtgctc tgtggttgtg aggaggggtg 9240

actccctcgc ctggtccatt cttgggcctt ggaggagcct cctacagtca ctggctctgc 9300

acccactgtt tcctttgtta ggattgttct gcccacgggg ctccctcagg cagggcatgg 9360

tgggcagaca ggctgtatcc ttccccggcc agccctatgg agggaggacc accccgcacc 9420

tctgcaggct gatgaaatca ggtgtttcac ccctctgaac gttctgagaa tgagggctcc 9480

tcacggcttg gtcgccacct aacgtggtga gtccttctca gcaaggtgga ttggagccac 9540

atgatctgcc atctcagtgc ttcccagggg aacacagagc tactgggcat ggtggctcac 9600

acgtgtaatc ccagtacttt aggaggccga gatgggcaga ttgcgaagtc aggagactga 9660

gaccatcctg gataacatgg tgaaaccccg tctctactaa aaatacaaaa aaaaattagc 9720

caggcgtggt ggcgggcgcc tgtagtccca gctactcggg aggctgaggc agaagaatgg 9780

tgtgaacccg ggaggcggag cttgcagtga gccaagatca caccactgca ctccagcctg 9840

ggcaacagag tgagacttca tctcaacaaa aaaagaaaag aaaagaaaaa cacagagctg 9900

cacaccccac agagttcagg cagaaggggg tctgcagcgc tggaagaccc agcaagcctg 9960

gcccgtctgg ctgcaagtgg caggggtggg tggagtcacc cacttcacca tctgggtgct 10020

ttccagggaa gcatgcagcc acgaccccgg gcagagttca ggcagaagct gggccactat 10080

gctggaaact ggccttgagc cttgtggagt aacggcaggt ggagccatct cactgctccc 10140

acgcaccatg cccgtggcct ctgcgggggc tgtggtaacg gcacccgact gctctggggt 10200

caatgcctgc ggaggtcccc ctggcttcag tgttgcctct gcaaaaaccc cagttgcagc 10260

caggtgcggt ggctcacgct tgtaatccca gcactttggg aggccgaggc aggtggatca 10320

cttgaagtca ggagttcaag accagcctgg ccaacatggt gaaaccccgt ttctactaaa 10380

aatacaaaaa ttatccaggc atggtggtgg gcacctgtaa tcccagctac tcggaaggct 10440

gaggcagcag aattgcttga acccgggagg cggaggagct gagattgcac cactgcactg 10500

cagcctgggc gacaaaacaa gactctgtct cgaaaaataa taacaataaa aaataaagat 10560

ggcaaccata gacactggag actactagat gggggggaag aaagggggtt gaaaaactgc 10620

ctattgggta ctatgctcag tacctgggtg acaggatcaa tcgtactcca aacctcagca 10680

tcacaaatta tttaaatttt tctctttttt taattttttt gttgttgttg ttgagacgaa 10740

gtctcactct gttgcccagg ctggagtgca gtggtgtgat ctcggctcac tgcaagctct 10800

gcctcccagg ttcacgccat tctcctgccc caatctcccg agtagctggg actacacgcg 10860

cccgccacca cgtcctgcta attttttgta ttttcagtag acacggggtt tcaccgtgtt 10920

agccaggatg gtcttgatat cgtcaccttg tgatccaccc gcctcggcct cccaaagtgc 10980

tgggaataca ggcgtgagct accgcacccg gcctaaattt ttttttaaat aaagaatggt 11040

aggttcttca caccctaatg tatttttact tctcccacag agaaggaaag gaatggcttc 11100

cccatggcaa gccacctcag tctgggcttt cttttcttcc aggggacttt cccatgcctt 11160

tcatatctgc caaatcgagt cctgtgattc ccttggatgg atctgtgaaa atccagtgcc 11220

aggccattcg tgaagcttac ctgacccagc tgatgatcat aaaaaactcc acgtaccgag 11280

agataggcag aagactgaag ttttggaatg agactgatcc tgagttcgtc attgaccaca 11340

tggacgcaaa caaggcaggg cgctatcagt gccaatatag gatagggcac tacaggttcc 11400

ggtacagtga caccctggag ctggtagtga caggtaagga aacatccagg gtccacagcc 11460

ctggtgtgat ttttttctta tttttaatag agtatttttc aagaagtttt agatttacaa 11520

acaaaaaaaa attgatgatt gcttcagaga gttctcagcc atctggcacc ccacttcccc 11580

cagagttaac atcttacatt agtatggcac atttcttacc attaatgaac aaatatcgac 11640

acattcccag ctacagtcta cagtttattt acattttctt agtttttacc tgatagtctt 11700

tctctgttcc aggatcccat tcaagatttc acattgcggc tgggagtggt ggctcacgcc 11760

tgtaatccca acacttaggg aagccgaggc gggtggatca cccaaggtca ggagttcgag 11820

accagcctgg ccaacatggt gaattccccg tctctactga aaatgcaaca atcgctgggc 11880

gcggtggctc acgcctgtaa tcccaacact ttgggtggct gaggtgggtg gatcacctga 11940

ggtcaggagt tcgagaccag cctggccaac acagtgaaac ctcgtctcta ctaaaaatgg 12000

aaaaaattgg ccaggcctgg tggcacacgc ctgtaatccc agctacttgg gaggctgagg 12060

caggagaatc gcttgaaccc aggaggcaga ggttgcagtg agccaagatc acaccactgc 12120

actccaggct gggcgacagg gcgagactcc atctcacaca cacacacaca aaaagatttc 12180

acattgcatt caggtgtcat gtatctttat tttttttttt tttttttttt ttgagatgga 12240

gtcccgctgt gttgcccagg ctggagtgca gtggcacaat ctcggctcac tgcaagctcc 12300

aacctcccgg gttcacgcca ttctcctgcc tcagcctccc gagtagctgg gactacaggc 12360

gcccgccacc acgcctggct aattttttgt atttttagta gagatagggt ttcactgtgt 12420

tagccaggat ggtctcaatc ttctgacctc gtgatccgcc cgcctggcct cccaaagtgc 12480

tgggattact ggcgtgagcc accacgcccg gcccccgaaa atgctgggat tacaggcatg 12540

agccaccgca cctggcctcc caaagtgctg ggattccagg cgtgagccac cgtgcccggc 12600

aggtgtcatg tatctttagg tttgtcttgg ctgtcacagc ttctcagatg ttgctggttt 12660

tccatgacct tgtcagtttt gagggtagtg gtccattatt ttcaagggta ctcccactac 12720

tggaaattgt ccgatgtttt gctcatgact agactgagtt atgggtcatt gcaggcaaga 12780

ccacagaagc aaagtgccat ttcatctcct catagcaaag gtttaaactg tccatgggaa 12840

catgactgtg gatgttgagc tggctgttgt tgaaagcctg gctgaagtag taactgtggc 12900

cagacaccgt ggctcgtgcc tgtaatccca gcactttggg aggctgggcg ccgtggctca 12960

cgcctgtaat cccagcactt tgagaagccg agatgggcag atcacttaag cccaggagac 13020

cagcctgggc aacatagtaa gaccccatct gtacaaaaaa tcaaaaaatt agctgggcat 13080

ggtggcaccc acctgtagtc tcagttactt gagaggctga gatggtagga tcacctgagc 13140

ctgggaggtc gaggctgcag tgagccgtga ttatgccact gccctcagcc tgggcgacag 13200

agtgagaccc tctctaaaat aaataaattc taaaaaagaa aaaagaggct gggcactgtg 13260

gttcacgcct gtaatcccag cactttggga ggctgaggca ggtggatcac ctgaggtcag 13320

ggattcaaga ccagcctgac caacatggag aaacctcatc tttactaaaa atacaaaaat 13380

tagctgggcg tggtggcggg tgcctgtaat cccagctact cgggaggctg aggcaggaga 13440

ctcacttgaa cctcggaggt ggaggttgca gtgagctgag atcgtgccac tgcactgcag 13500

cctcagtgac agagtgagac tccatctcaa aaaacaataa taggctgggc acagttgctc 13560

atgcctgtaa tcccagcact ttgggaggcc aaggtgggca aatcacctga ggtcaggagt 13620

tcgagaccag cctgaccaac atggagagac cccgtctcta ctaaaaatac aaaaattagc 13680

tgggcgtggt ggtacgcacc tgtaatccca gtttctcggg aggctgaggc aggagaattg 13740

cttgaacccg ggagacggag gttgcagtga gctgagatca cgccactgca ctccagcttg 13800

ggcaataaga gcgaaactcc atctcaaaaa aatatataat aataacaata ataagaagaa 13860

gaaaagaata aaggagaaaa ggtctttcta atagctcact cttttctctc ttaggcttgt 13920

atggcaaacc cttcctctct gcagatcggg gtctggtgtt gatgccagga gagaatattt 13980

ccctcacgtg cagctcagca cacatcccat ttgatagatt ttcactggcc aaggagggag 14040

aactttctct gccacagcac caaagtgggg aacacccggc caacttctct ttgggtcctg 14100

tggacctcaa tgtctcaggg atctacaggt gctacggttg gtacaacagg agcccctacc 14160

tgtggtcctt ccccagtaat gccttggagc ttgtggtcac aggtaggtac cgcccagtcc 14220

agccctgtgt ctgggttggc tgtccagggc cttgccaccg ggcaggaata tgaagacgtg 14280

cactgagagt gaagtgaaga gaggcaaagg ctctcactcc aggacagtgg agagagaaag 14340

gcttccccac cacactttcc gctttcactt cctcgctaga gttctccaga cagggttcat 14400

tgaaaactta gtctgtggag aacagaaggg ctaactcagt ttgtttcatt ttatttattt 14460

cattttattt tccgggatag agtcttgctc tttcgccaag gctggagtgc agtggcacga 14520

tctcgactca ctgcaacctt cgcctcccag gttcaagcaa ttctcctgcc tcagcctcct 14580

gagtagctgg gaccacacag acagggtttc accatgttgg ccaggctggt ctcgaactcc 14640

cgacctcagg tgatccacct gcctcggcct cccaaagtgc tgggattaca ggcgtgagcc 14700

accgcgcctg gccaggctgc acacattctt attaggattc caccttgttc tggtgttgta 14760

gagatgtgat taggtattta gtgaattcac caagtgagga gagaatgaaa agaaaacaca 14820

acctgcctgg ccgggcgtgg tggcgtgagc ctgtcgtccc agctactcag gaggctgagg 14880

caggagaatc acttgaaccc aggaggcagc tgttgcagtg agccaagatc acgccattgc 14940

actccagcct gggtgacaga acgagactcc acctcaagaa aaaaaaaaaa aacatggttg 15000

ggcacgatgg ctcacgcctg taatctgagc acattgggag gctgaggcag gtggatcacc 15060

tgaggtcggg agttcgagac cagcctggcc aacatagtga aaccccatct ccactaaaaa 15120

tacaaaaatt aaccaggcgt ggtggtggtg ggcgcctgta atcccagcta cttgggaggc 15180

tgaggcagga gaatcacttg accagggagg cggaggttgc agtgagccga gatcacgcca 15240

ctgcactcca gcctgggcaa cagagtgaga ctccatctca aaaaaaaaaa aaaaaaaaaa 15300

cacacacaac ctgcccataa tcacctcctt cccagtttat agcacttccc tgggaagcac 15360

agttccttgc ccgtgaacac agtcttgctg actgatcagt gtggtgctgg cgaagcatga 15420

gctcattgag gggatgcttg agggagtccc attttggcaa gcgaaaagga aaatgagctc 15480

ccgtttcagg gctctggggt tgggatggaa tggaacacaa ccaccaacca ttcatctcct 15540

tgaattgtgt ctccagactc catccaccaa gattacacga cgcagaactt gatccgcatg 15600

gccgtggcag gactggtcct cgtggctctc ttggccatac tggttgaaaa ttggcacagc 15660

catacggcac tgaacaagga agcctcggca gatgtggctg aaccgagctg gagccaacag 15720

atgtgtcagc caggattgac ctttgcacga acaccaagtg tctgcaagta aacacctgga 15780

ggtgaaggca gagaggagcc aggactgtgg agtccgacaa agctacttga aggacacaag 15840

agagaaaagc tcactaagaa gcttgaatct actttttttt ttttttgaga cagagtctgg 15900

ctctgtcacc caggctggag tgcagtggag caatctcggc tcattgaacc tcttgggttc 15960

aagtgattct tgtgcctcag cctcccaagt agctggaatt acaggcacat accactgcac 16020

ccagctaatt tttgtatttt tagtagagat ggggtttcac tgtgttggcc aggctggtct 16080

cgaactcctg acctcaggtg atccacccac cttggcctcc caaagtgctg agattatagg 16140

catgagccac cacgcctggc cagatgcatg ttcaaaccaa tcaaatggtg ttttcttatg 16200

caggactgat cgatttgcac ccacctttct gcacataagt tatggttttc catcttatct 16260

gtcttctgat tttttatatc ctgtttaatt tcttccttca ttgttcttct ctttttttat 16320

ttattttatt tatttttatt tttattttta tttgagacag agtctcactc tgttgcccag 16380

gctggagtgc agtggcacga tctcggctca ctgcaacctc tgcctcctgg gttcaagtga 16440

ttctcctgcc tcggcctccc aagtagctgg gattgcaggc tcccaccatc acgcccagct 16500

acttttacag tatttttagt agagacgggg tttcatcaca ttggccaagc tggtctcaaa 16560

cttctgacct cgtgatctgc ccgcctcggc ctcccaaagt gctgggatta cagatgtgag 16620

ccactgcgcc cagccttctt tttatatttt taaatgtgtc ttccccaaat ataaatggtt 16680

ggtaagcatg ccaaatatat tcaataaccc ccctccttta tttttttttg ttgaagtgag 16740

gctctcccta tgttgcctaa gctggtcttg aactcctggt ctcaagcaat cctcctacct 16800

cagcctcctg ctgtgttcat ctacaaattg ataagagtga aagtcataat cctacaggag 16860

gattacccta tttatttcac aaaccctatt tctaccggat tttcatacaa ggaatacagg 16920

catgtgtttc acctcattaa tttatttttt cacttagttt tgatgatatt cacatatatt 16980

atcaagtgtg caaacattaa attcttgtgt acaaaactca aatggtcttc caaataattc 17040

cccattcttt tttcttataa actttcacag ctttaccctt gacagacttt actcaaggaa 17100

atctaagttg gtcatatgtg gctctttcac tgattgctat ttacttcatt gtccagtagc 17160

ttatgtatga aaatataatt ataaaatgta agggtcctac ttccagtgaa actgaaggga 17220

cttaggccca cttttatcct ttactgagag cttatctcta cttgataaaa tttctactgt 17280

attcttggct taactcaggt cctgtgatta aaaaaaaaat gcaaagta 17328

<210> 30
<211> 45779
<212> DNA
<213> Homo sapiens

<400> 30
acttccttct tttccttcac ctcggcccgg ctctgaaagg atcgggcccc tccggcccgc 60

acccaccccc aagaggggcc ttcagctttg gggctcagag gcacgacctc ctggggaggg 120

ttaaaaggca gacgcccccc cgccccccgc gcccccgcgc cccgactcct tcgccgcctc 180

cagcctctcg ccagtgggaa gcggggagca gccgcgcggc cggagtccgg aggcgagggg 240

aggtcggccg caacttcccc ggtccacctt aagaggacga tgtagccagc tcgcagcgct 300

gaccttagaa aaacaagttt gcgcaaagtg gagcggggac ccggcctctg ggcagccccg 360

gcggcgcttc cagtgccttc cagccctcgc gggcggcgca ggtccgggcc cggcagggac 420

ggggaggggc agagcgggac cggtcggggc tggggaccag ccgcgcgggc ttctgaacgg 480

gcacgctgtc ggagccggag gctcgtctag tcccgagccc cagcgggccg cggcggctcg 540

cggggccgcc ttcccgggac agcagccggg gcccgggcgc cggcgcgggg gaggacgctg 600

ccctccccgg ccgggcgcgc agctcgcagc cctcgctccc tcccccgggg cgcgcaggcg 660

ggcgctcgct cgcagagaag gtgccggcgg ggccggagcc ccgaagcctc cccgggcggc 720

gggaagacgg gaggagggga aagggaagga gggagggggt cgggctgccg ggagggcggg 780

ggcagcggcc gggactgcgg gccgggagcg agcccagccg cccgcgccgt gtggagcccg 840

ggcggggcag gtgggcagcg gcgggggcgg ctccgcgcgg ccgggctcgg ccgtgctgcc 900

cctcgcccct gctcgcgcgg tcctctctcc cgcgcggtct gtcggtctgc gcgccgcctc 960

gctcctcgct cctcgctctc cccctctttc tccccctctc gcctctccgt gtctctggct 1020

ctctgcctgg ctcccttggg tctgtttctc tctcctgccg cctctctctg gccgcccctg 1080

gctttatttc tcgcgcgctt ggggtctctc ccagtctccg tctctccatt tctcctgggg 1140

ggcggggagg gggggtctcc aaaaaccgcg gcggcggcgg cggccgctcc aggcgcccgt 1200

tccggagtcg gggggaggcc cagccgggag gggggaaggg ggggagcctt agtcatttcc 1260

ccgctccagc ctgctcccgc ccgagcgcgc actcacggcc gctctccctc ctcgctccgc 1320

agccgcggcc catggagccc gccggcccgg cccccggccg cctcgggccg ctgctctgcc 1380

tgctgctcgc cgcgtcctgc gcctggtcag gtaagcaccc ccccgctccc caccgctgca 1440

ctccccaaac tgcgggctca ggcctctcag actggcactg ggaccccttc ggctaatgcc 1500

gagcagtaat agggggagag acgcctgtaa ccagggttat agatgcagaa actgaggccc 1560

agagagggcc agcgacatgc ccaaggtcac acagccaagg gtagaaccca agtctcttct 1620

atgagagttt gtctctgtgg actctgactc tgtgcactgt gctgggcact gagacaaaag 1680

agccgagttg cttcactcag cccctgcccg gtccaatggg gaaggctgac tgacagtggc 1740

tttcagagca gggccgtccg gcttccccag aatctgtccc catggacata cgactttcca 1800

catgtttgac ttcagggtgt gaggggagga ggtgagggca gaggtcctcc cgatgctgcc 1860

tggaatggaa cagagacaag ctgttgcaat tagggaagag tgagccctcc ctctggtgcg 1920

cagagtaaac cggtgttagt gtgtgcctgg gagagaggga gccgggaggc cgcggtaacc 1980

ccagagcgag ctgctgcgag caacagatcc ttatgtaaaa acacggtgga ttgtccctgt 2040

ggttgccaaa gcctctccct ggtggtcttg ctccttgagg cgctcccagc tcccccttcc 2100

tcctgccttc cccgagcttc caggcagctc cctgcagttc aggtgctccg cggggatgtt 2160

acctgtggct ctgaccatgt ctttctgttg gcctacatca aacacctggc tgtcgccggc 2220

aggcgggctc tgaaggctac tgtgcatgtt caccaaaggg tgaggtcacc attcaggagt 2280

cttgcttctt aaatgggttt tgcaaaacca tgggtctcct gggagggcac cgggtggggg 2340

gcggggggag cagagaaatg gcattaaggg agttgggagg gagagaaaaa tggggatctt 2400

gttttttgtc gtcttgaaac tgtgttgtag gcagtggggt ctctctgtac ctcccaccct 2460

tgaggaggga ggtaaagatg agctcagtgt ccaacacccc cttttgcgtt tgactagaga 2520

tggtaaccat ctgatagcag ggagaaaggg agaactagca aagagtgggt ggaaggagaa 2580
gtatgggggg tagacagctg cttctctctg ccctgcagtt tcccctgaaa cccaggaatt 2640
cacataggca tccccccacc ccaccccacc ctccaccccc agttcttttc agttatagtt 2700
gcgtccttgc tgggctgaag ggagcactga tacagttcac gtctctccag tgagtggcat 2760
ctcctatctg ctccttcctg cctgagctgg ccatttgtac cctgcaaaag tccgagttcc 2820
tgccctgcca tccttcctgg cctggaaatg cacaagtgtc actcacaagt gcttaatttt 2880
cctgaagcag ggaattgggc ccgagttaat ttctaaatgt ctattccaga cccttctccg 2940
accaggaggc tgtggttcag tccctggcaa acgagcagtg acgctgatta gcaagtgctc 3000
cgatgggcag cctgcctttc ctggggcacc ggctgggaac accaacacct ttcttgtgag 3060
cacatctccc ccttttgcct ataaaaacag tctataagat ggtaaagatt gggccataaa 3120
acttccgttc caatccattc agcatcaata gcctgaaaaa gtgggagcag cagaggggcc 3180
aagattagct ttgaaataaa taaatcttag gaggtaagaa tgaatttctt ggtcgagggt 3240
ggtggggaag ggatgaggtg cgaccatcat agctcaagtc tcccattatc cttcagtatc 3300
tgttcttcgg ggtcctggtc acaccatggg cctgggcagg gaagtcacct ggtttggctg 3360
aaggatgggg cctcaggccc ctccccctat tgctccagac atggaattta caacaaagaa 3420
gtagagagac aggatatgag ggtaggaagg tacaaggagg cagttgtcca attttctaag 3480
aaaatggatt tgttcgagtg agaaactaaa atgtcaggtt taggaaggcg gctttggatt 3540
gaatgactac tagtgtctca ggcagaactg tcagcctggg aggtgtcaca tgattcctgt 3600
gtcttcacat gatcactgtt ttgggggggt ggggccaatc atgtgaatta tcctgggtgg 3660
gggaggcaag agaagaagtt tctaaagatt gcttttagca caaatgctgc caaagggaaa 3720
ttctaaaaag atcccagtga agtcaccaac ctatgttttt ggacccaatg tgaggcctgt 3780
ttcctaaaac aactgcactg agggagattc tgaggctggg agaagggcaa gggactgagg 3840
cctggggaca cctggcacct gattttgtgt cagcagggcc tgacgctgaa cccgcaggat 3900
gcctgcttgg ccccaggtag gccttgagac ctctgagtca ccccccaggc cgacctcaga 3960
taaactccca ttcatttctt ctctctggcc tggaggaggc caagccaagt tgcagttcag 4020
gctctgggga gcagcggggc cccccagtga acctctcacc gtttggtaga gggtggcact 4080
ctgggggctc ctctgtactt tcctaaatgg taaagttaat ctcacttcct ctttcgtaag 4140
aagagaaaac ggggcgggag tgctttggct caacacatca ggctgttgga agttccttta 4200
aagccatagt tttctctgag tactttctgg cctttaccaa cccctcggaa actcacggta 4260
attcacgtct gtacttgcag tcatgcagca cctatgcagt gctggtgtga accatgtgtt 4320
gagagaaggg aggaaaacca acattcatta gatagttaat ggcgttccag gccggatgct 4380
gctcctgcgt cgtctcaccc gcaagacatg gattggctct tctccccatt ctgcagatgc 4440

agaaaatcga gtcccaaact ggggaactaa cttataagtg atagagagga gccaggattt 4500

tggatgctgg ccttcctgct gggtggcttc tagggaagaa gctcaatgac ttaatcagga 4560

tgatttagtc caagctggag agggagtgag agaggcccct ttctggcctt gggtggagga 4620

ctgtagaaga gttctttctc tgggcagaag gttttgatga agtcacaggc atttctctga 4680

aagcctgctg agggtggaaa tactgacagc cccttggtcc tcaccagtaa caacacatga 4740

gaggtgatgt ggctcctgtg agagcccccc atctgggctg ctgagctctg gagcccccag 4800

agctggaata ttcctccctg catcttcctg gtgccctttg cgacagatgg agagggggtg 4860

tgcgagagga agggagtcta aaagtgttgt acggatccca cacgcgctcg cttgctgctt 4920

ccaatggctc cgtgcgacag gttattttct tatttgtagt cttggactga cggaaggttt 4980

cccccctctc tctctctttt taaatgtgct cagatttctg gcctgtcaac acaactcaga 5040

tcatgtcact ccccagctca aaactgttga gtgggtccaa gttttcttta aaaaaaaaaa 5100

acaaaaaaac acaaagctgc cgtgaggcct gggcttcctc cctgccaggc ctgatttcca 5160

gccacatgtt ttccaacaag ctgacctgtt gagatgctgc aggataacca ggcttcctcc 5220

ctgcagacca gagctttgct ggctactcag ccacacgagc accccaaaga ccctgagaag 5280

ccctgcaatg tagaatcccg agctgggtca catttactcc agtgttccct gaacttatga 5340

acgagaggca tttgatttgt agtatgcccg ttagcatgtg gcctctctcc gtttggtaaa 5400

cggttttcta cagatccatg ccctcagctg aactctcagc tctgtgcctt ggcccgtgct 5460

gttcctgcca tcagaaatgc cctccctgta cctccttgtc ttcctgtgtc tgaatcctac 5520

tcatcctccg aggttcaatt taatgtgcct cctggggaga ggtcagagga gcctctccac 5580

gcagacacac accctggcct agcgctaaga tggtgctaag acccctgggt gttgatctga 5640

gccctgctac tgacttggac aatttgcagg aatctgtgtc tcattttgct catctgtaaa 5700

agaggaatgg tattaaccac tctattgcaa ggattaagtt aattaatgcg tgtataatgc 5760

tggaagccat gccttgtatg tagtaagcac tcaatacata ttaaccagtg ttgtaattag 5820

catggcttta ttctggtttg attcctcttt tttaacaatt gggacggatt ttcataaaac 5880

atctaacaca gttgcttaca catagggggt acttaaaaaa cagcaaaggc tgttcagtga 5940

acaaatgtcc ttcagcttga tattttcctt gtagcttttt tttttctttt tttttttttt 6000

tgagatggag tttcactctt gttgcccagg ctggagtgca atggcgtgat ctcggctcaa 6060

cgcaacctcc gcctcctggg ttcaagcgat tctcctgcct cagcctcccg agtagctggg 6120

attacaggga tgcgccacca tgcctggctg attttgtatt tttagtagag acaaggtttc 6180

tccgtgttgg tcaggctggt ctcgaactcc tgaccccagg tgatccacct cccaaagtgc 6240

tgggattaca ggctgagcca ccgcaccacc cagctgtcct tgtagctttt atgctgttgg 6300

gggatggaca agttgagtag aaacccaggc tagagtgatt taattgcctg ttactaggga 6360

ctgagtgctg tgggcacagc ctgcctctag gtgtctctat ctggatccac aggtctgcag 6420

ttgtggattc ccggacctgc aaagcagtag ggggcctgag agagccctct gtgctttcat 6480

ccagtgcctt atggtgcggc cagaagaaca gagagaaaaa aactggccgg gcccacgcgg 6540

gagatccatg gcaaggcccg gctggaacgc caagcctcct gtggtccagg ccaaggcttt 6600

taaaaactgg gctgtgctgg aactttccac tgtttcagcc agaaatctga caggatcccg 6660

aattccccgg gaaaacacta aagtaatact aataggaacc atctgggttc tctgcttcct 6720

gggctcagca cttggtgtgt gtgaccctgg tggtactcac agccacctgg cgatgcacgt 6780

cgttatcttc ggacccgttg gatctgtgag gaaactgagg ctctgaatgg tgaagcagct 6840

tgcccaaggc cacaccgtaa gcgccagatg tggataacca ctgtcggcct aatcacagag 6900

cctatgtggt taactgttgt gccattcctt agccgtcact gaggcttgct tgacaggtag 6960

ggaaactgag gcccagttgt gctggcaccc tgctccctta gtgctcctct catttttgtt 7020

tttgtttttt cttcctttct ttctttcttt tttttttttt tttttttttt ttgagacgga 7080

gtcttgctct gtcgcccagg ctggagtgca gtggtgcgac ctcggctcac tgcagcctct 7140

gcctcctggg tccaagcaat tctcctatct cagcctcccc agtagctagg actacaggcg 7200

cacaccgtca cgcccagcta atttttgtgt ttttagtaga gactgggttt caccatattg 7260

atcaggctgg tctcaaactc ctgacctcag gtgatcctcc cgcctcggcc tcccaaagtg 7320

ctgggattac aggcgtgagc cactgcaccc ggcctcctct cgtatttcta tcagtgcacc 7380

ttcctatgag gtgggggcag tgtgtgtccc caaggtgtgt ccctgtcccc atactagaga 7440

aggaagcatc tattttcaca gctgaaactg ctttcctgtt aactgctggt gttcctggga 7500

tgggtattct gatgaagccc tccacgagat ggattttccc tgccaggtgt cttgccaggc 7560

cctcagtcca gagagatgca aaacagctga ggtggaagtc ggaaatgctg cagaattggt 7620

tctttgtgtc tgtaagattt cctggcacat tcgttcgggg gcgcagggtg ttagatggct 7680

gcagattagc gccaagagcc caaggagatc aggtgctcgg atcaggaggg aaattggaag 7740

ccgggagtga gcgatgggcc tcccagccag gagggacggc tggagcaggt gggcggtgat 7800

accagcgccg tttctacagc tccattcttc aggccgaccg tcggtgttct ccagaaatgg 7860

aagcccgtgg tggaaggaaa tactaggtac acgatgtgct cggcacaggc tgagccttga 7920

acatgtgtta gctcttcctt atctttttat tttggaaaat ttcaaacata tgcaaaatga 7980

gaaggaatga tgtaatgagc ctcagctttg acactgatca actctatacc atgcttgtcc 8040

cctgccccta tttttgctag ggagaggaga atcggggagt atataaagca agtgctaaac 8100

atggggtatc atttcaccca ccgatactct ctccacatcc cttcatgtga gctgttacct 8160

gaccctcacc acgagccaaa cagtatggcc cttcatcccc actttacaga taaggcaact 8220

gaggcttccc aaggccaagt ggggctcatc cttaggccac acagccagtg agaggtaaag 8280

ccaggctcca ctcctcactt tacccttaac cactttgcag tcattattag ggtcatttta 8340

attgtaaata ttcattcaac aaatattttt gagcacctac tatgtgccag ggatggctct 8400

aggtgcttgg aggatacaaa tgtgaacaag acagacaaaa acacctgctc tcgtgcagca 8460

gacattctag tgaggaagag aaaaacacac ccaagtaaac aaatgatgcc aggtagctgg 8520

ggagaaaagc aggaggaggg aagggagaag gttagtcagg acagagggag ggccgctcta 8580

aggagcagag gcctcaagaa aatgtacaat taaggccagg tgcggtggct cacgcctata 8640

atcccagcac tttgggaggc tgaggtgggc agatcacctg aggtcaggag tttgagacca 8700

gcctggccaa catggcgaaa tcccatctcc actaaaaata caaaaattag ccgggcatgg 8760

tggcgtgcgc ctggaatccc agctactctg gaggctgcgg caggagaatc gcttgacccc 8820

aggaggcaga ggttgcagtg agccgagatt gcaccactgc actcccgcct gggtgacaga 8880

gcaagactct gtctcaaaaa aaaaaaaaaa aaaaaagaaa agaaagaaaa gaaaatggag 8940

gagtagccac gggggatagc aagagccctc catgtgggag gaatggcaga tgcaaaggcc 9000

ctgaggcagg aatgagcttg tatgttccag gaattgcaaa gaggccagtg gggctggagt 9060

gaaagtagaa gaggtggggg agtgagcaga ggtcagggcc agaccagcag gatctggctc 9120

attgagcacc taacatggag catgtctggc ttgtgagagg atcattatgc attctctcat 9180

ttaattctca ccacgggttc tgtaagatgg gaaataccgt taatccattg gacagatgaa 9240

gaaactgaga ctcagaaagg taaagtgact tgcctaaggt cacacagctt ttatgtgcag 9300

aactaggatt taggtccctg aatgctgcag catccccatt gctgctttcc gcagccgtcc 9360

ttgccccctt cgggctcttc acccatagca gctggagtga atcaagtcat ctttggttct 9420

gctggaaacc tttttcggtt tccagcatgg catttctgga ccacgtctgt ctcttcatcc 9480

tcacctctta ccactctcct gcctccttgg gcttcagcct tggcagcgtc ctttctggcc 9540

cttcaccagc ttctttccca cccgagggcc tttgcactgg ctgtctcttc tacggagcca 9600

tcttccctgg tcttcacttc ctttgggtct cagctgccac ctcccttcct tggatgctgt 9660

cccaaccacc ccattccaaa agaggttatc gcttgtgatt ctctatcact gggccaaatc 9720

tgcctgttgc ctagtgctcc tagccctttc tatattaatt catttcatta tttcagccct 9780

gtctcctcca ctaggatata agctgcacga gagatgggct atgtctctta ctcactgttg 9840

ggcctcccag gacctcagca cagggcctga cacacagtag gtgggttaat agatactgat 9900

tgaatgaaag aacaaggtgg gtactaaaat ccccctttta aagaggaggc ttggagttat 9960

gaggttgtac gagcaaagct tattccctaa ctaccacagt cccccccctg cagccagggc 10020

cgggatcata ccacaattcc ctgcccatca agctgtcatg aatagcccta agatatcagg 10080

aagcacaaag atatcaatgc caggagggat aggaggaggc ggccaagaaa ccccggaatt 10140

tgtacttaaa attctggcta cgcaaactgc ttgcccaact ttagacacca tgatgaatga 10200

tcaggtgctg caaccatgaa acatgacgta attagtgctg cactcttggc tgggggttgg 10260

cttggaggcc ccacctcgag gttagcctcc cccagagggt cactgcacga ggcagtctgg 10320

tgcggggtgg gggttacaac agagggaggt ggaatcagac cactcggcag ccctgtgacc 10380

atggccaaat ggttgtacct cccaaagctt cacatctggt cctccgtgaa atgggggtgg 10440

cgacagccct gtcttactgg gctcctgcag aggttccatg aggcctgtct tgctctgggc 10500

agcgtcgatg cttattagct attgttactg ctgttatgtg attgtcatcg ttttatggtt 10560

tgggaatttt aaaagctcca tcttttgcca gcggggagac ctggaaccca ctcctctgcc 10620

ccctcagcct cagtttcccc ttctgtgacc gggaggcttg gctcggctcc tctcttgcag 10680

gtcccttcca gccctgatgc tccagcctgc atgtcctcct ggccctttgt cctgatgact 10740

ccacagactc ctctgggagg agaggccatg gaggggcttc tgctctgggg cacattcctg 10800

aattggagtc cagcatgtgc cgcttactgg ctgtgtgctc tggggcaggt gtcttcccag 10860

ctctgagcct ccgtttcccc atctgcaaaa tggaaccgct aacactgttt cctcccaaat 10920

gggtgactgt tgagggttaa ggaattaaga gccgtggaaa gtgcttttgc aagctgcaaa 10980

atgctctata aaaggaatca gtgaatagtg tcagcttggg gaaaaatccc gtcagtccgg 11040

agactgtctg ctgccggcag tgtgctgaaa ccctggcaaa tgtcgtttaa taaatgcgtg 11100

ttgcttttaa gagggccttt atctcagctg ggaaaatgtg tgcgcatcct tggcaacctg 11160

agaatccaga agcctgaact ggaggggacc tgtcacaggg agaggcgatc gcatggggtg 11220

gctccagcct ccagtgttgg aatcccacct ctgccactta cctacctctc cagactctgc 11280

ttcctcatct gtaggagggg acagtgacaa tagcacctgc cactctgggc caggatgtcc 11340

tgagggctaa atgaaggctt ctggttatct ggcctaaccc atgcatttcc cccatgaaag 11400

tgtggctgtg cagcaccagg gcctgtctca gggcaatggt tggggcctgg agattgggag 11460

gacctcccaa cctcagcctc tccaggcctt actccaggcc tcagtttctt cctctgcaaa 11520

atgaggagga ggaaaggtcc ttaggttgtc aaggcaccaa gcctagtaga tgtcaatgtc 11580

ctctacctgc tctctcagcc tctgccggtc ccccgaagcc tgttaattct aaaggatatt 11640

tacacagatt tgcagttctt ttcctgccac tttgcaaaca gggtcacact gcaagcatcg 11700

cttcatcacc tccaggtttc atctgaaccc agcaaatgct ccatagggct cccagtgccg 11760

cctgtccccg ctgcctcctt gccagcagtg tgctcagcct gcttacaggc ccatgccaga 11820

ggggcctcat gcatgcatca aggcagagct ggttctgcct cggagaggat cccgagcctc 11880

tcaggcttaa ggacgtggtc atctgtcgcc cccttcatct ttagacaccc atctgcatgg 11940

tgacagctgc tctgcattga gtacttactc tgtgccaggc acgctagccc gtttcatcct 12000

cagaacaacc ctagtattgt tatcatcatc atcatcatca gacccattct acagataaag 12060

agccagaggt ccagagtggg cgagacattc cctggagtct catgggtagc agagttggaa 12120

tccaaatcaa aacatacttt cagccagtgc caggcactgc tccaaccctg gggaagaaaa 12180

ctcagttcct gctctcaaag gacttataag tgagtctatg acagggagat gagcgctttt 12240

aaaaaattaa caaggaaatg cgtagctttt tgagtgagga tgacatatcg aacaatgaag 12300

cagagccagg gacattgagg tgggtagtat tttagacaga atggccaagg aaggcctctc 12360

agataggcct gaaggagagg agcgagccag acatgtagat atttagggat gagagtgcca 12420

ggcagaggca atggcgagtg caaaggtcat gtggtgagtg gagaatctgg cacgattggg 12480

ggaaagcaag gctgtaggcc cctggggcca ctgtaaaaat tctgggtttc actctgagtg 12540

cagtgggggc tgtgggaggg ttttgcacaa aggaggaacg tcacctgact taggttttag 12600

aggcttctct ggctgctggg ggaatgtggc tggtagagag gtgaggcaga ggcagggagg 12660

tcagcgtgga ggttcagtca gggtggtaag aagaggtcag ggtctggcca tgtgttggaa 12720

gttgagccag caggatctac taacagatgg gttgtggaac acggatgaaa gggagtcaag 12780

gaaatgtctc attctgagtc cagcacttct aaccacagag cctctgacct ggggagggag 12840

ggggctgcat ttcctgttta caggtgagga aacaggctta gagaaaagca actttgccaa 12900

gcaccgtgct acagacaaga cataaaggtc atctggcccc agaacctgag ctctggacag 12960

tcttagcacc ttagaagttt acctactgca tggatggggc caccagagtc tgccccttcc 13020

ctgcagggat gtggcacagc actgagttct cagagctacc cagggcaagg gtgaaggagt 13080

ggggaccctg agcccctggg ccagctgtca gcttatttac caggccacac gcatccccat 13140

ttttggtggc ttcaatgcag gtagcagttt ctatcacagc acctggtttg tccctgccca 13200

atgggtgtat ccagtggtgc cggcagaatg ttcccagtgc tggcctgctc ttcagatggg 13260

caaccgaaac catccttcag ggcgctaggt tagcccagtc ccaggtcctc cccatctcag 13320

cttccttaca gccagtgagc atgttccaag gactctaccc tgagtcctgt cacctctgcg 13380

tctttgctca tgtggttccc tcctcttgga atgccttcct tcatcccagc ctctgtcagc 13440

ctttaagacc tagctcaggg gccaccagct ccaggaagtc ttcctgggtt tcactctctt 13500

ccagaacccc atccctaaac ctcctcttgc aacaccgatc gccatgtgcg tggtcattgc 13560

tcatgtccag gacggcccca catgctaaga tagtctcaag ggcaagggcc tgtcttactt 13620

ggctctgttt tctgtggtat ctaggacagg gcctggcagc agagtgaggt ttgagagggc 13680

atgaatgaat cattctgact ccagaggaaa tcactgtgta gtcctggctg ttggagcccc 13740

aagctctctt cttgggaact gtggcatcca tgcagccatc cactggtgca gccccggcca 13800

tgaatgcagc cccagctacc tttcgccttc ctgcatgggg tcatgggctc cagcagatgg 13860

ttctgggcta gcctgttgac agctgtgccc tgtggccagc acatagtaag taggtgctct 13920

ggaaaggtta gcacctccta tcaagggcac gaccctagac agataccaca cagggtgttg 13980

accgccagga gcccatggca gacttgaggg aagtgtgtct cctcacatcc tccatcaccc 14040

ttgtgcttgt ggaaacttac acagcaacct catctattcc aggcacatgc ccagctggac 14100

acactcccga acacaggcac acggacacct ggggtgcaca caaatagtgg catgtgcata 14160

cctccctgct tgtcaatgca cacaccctca tagtcacatt tacgcataca gcactctgtc 14220

acacacgtgc caccccccac acacaccctc tacacacaca tacacaccac actcacgtgc 14280

atgccgctcc tgcatcactc tcccacacac tcacaccctc ccacacatgc ccaggccacc 14340

atgattcaga cactctcctg cacacaattc acatgcccac gcccatgatc cctagccccc 14400

cattcagaca cagtcctgtg ttgcactctg acatattccg gcacacaggc accgtctctt 14460

tcacactcct ccttgtgccc acccatgtgt acatgtgtgt gcagatgctt atgctctgta 14520

cacgcataca tgtatgtaca cacgtgtgtt ctcgtgtgca tactcttatg caccctcagc 14580

acactcatgt tcacatgttc cattgtacag ccctgtacta catcctgtgt gcatccgctt 14640

taatgtacac tcatgttcac gtgttctatt gcacagccct gtactatatc ctgtgtacat 14700

cctctttaat gcactcttaa tgttcacatg ttctctgcat agccctttat tacatcctgt 14760

ttgcatccgc cttaatgcac atttatgttc acatgtgcta ttgcaaggtc ctgtactaca 14820

tcctgtgagc accctcttta atgcacactc ttgcactgca tactctggca cacagcatta 14880

aatcacaccc agaagatagt agaggcagcc cccgatgagg agctgctata agtgcggggt 14940

ggaaaatcgc agccttctga ggatggtgaa taacaataat gatgagggaa gaagggagcc 15000

gggtatcact gcagccactt tagaatgttg agccctgtgt actgccagag ctccagccat 15060

agcctcccag accctggggg tccttgagta caaacaaaga atctacaaag aatccaaaca 15120

ggtggcctcc tgcctgtccc tgtggcttgt gctgtggtga cccactgctt ccgggggatg 15180

ggcaagctcc catgcagtgg cagtgagcac ccgcctgggc aaggctggtt cttgggaaat 15240

gtgcctgttg gcttccttct ccccacccct tgagacaggc ttctggaaga ctcacacccc 15300

agggtaaggc agccctcccc atccccgcct caagcttcag ttctaagttg attttttttt 15360

ctattagaaa aaacatccat gtgggcaggg cgcagtggct catgcctgta atcccagcac 15420

tttgggaggc tgaggcgggc agatcgcctg aggtcaggag tttgagacca gcctggccaa 15480

catggtgaaa ctccatctct actaaaaata caaaaattag ccaggtgtgg tggcaggcgc 15540

ctgcaacccc agctacttgg gaggctgagg caggagaatt gcttgaaccc gggaggtgga 15600

ggttgcagtg agccgagatt gtgccattgc actccagcct gggtgatgag agtgagactt 15660

cgtctcaaaa gaaaaaaaaa gtatccatgc taattttttt caccctttat gaaatatttt 15720

catcttttaa tctcaaaagc cacacacatg aacattgttt tttaagaaat caaatatatt 15780

ttctaaatcc cagtggtcag ttctcagccc ccatcttatt gcatctctca gtggcattca 15840

atgcaggtgg tcacaccctc cttggccaat tcaaagaaga gaaaggagat gtggcgggag 15900

aagcaggtca caggaagggg ccgggagatg ggatctaaga tgccagcagg agcctcttgc 15960

ccaggggggt taggtacatt ccagatgtaa tcatttgagt gatttctttg aaatccatct 16020

ttgcctcgag ggtggaagct ccatgaggcc agggacacag ccatttcaca gcccttagct 16080

cagggcctgg cacaggaaag gatccagaat gcagcactga ccgcacttgc tgtggacact 16140

gagttcctct tacagcaaac atatggtgac cacccactgt gctgactgct ccccttagga 16200

aacgtgttcg tgtgttttgt ttcattactt tttaactttt ttccaaaacc aataaatgtt 16260

cactgcaaaa aaatacaata tatgatgtat tataaagtaa taagaaaaca tgccgacccc 16320

atcctacctc agtcccattt cccagatgca acacaccaca gcatgccatg tttaacttat 16380

catccttgca gacctttttc tctagacatg cgattgacca atcaacaaag atttatcgag 16440

cgtctacact gtgccaacca cagtagcagg agctggggaa acagtgaaca aaacagacta 16500

aatccctgga ggagaggttc atttaaacag tacacatacg gtaacataca tcattacatg 16560

tacaggtttt ttaaaaaaca aaatgagata ctataaatat tcttttgtgt tcatccaatt 16620

tagagaagct gaaacaatgc ccaaagtagg aggggaaaaa aagagaaaaa cctcatgagg 16680

tttagattca tgatccaaag agagttacat tttatagctt tgtttaatac cctccagttg 16740

tttatatata gttttatttt acaaagtcat gatcatacag agggtataga tttttctcca 16800

tctttttttt tcttacaaaa gaaatgcaaa cagaaaacat aaacaagagc aaaatcccat 16860

gtaaatctac cactcaatca tggctaatct ttaatatcat tgtttttttc taaaattggg 16920

agtttgggat cacactggat ctactacttg gtaacagact ctgatcttgt aacacatttc 16980

cattgtaatt ctataccatc attttcatgg ctatagactt ggcctatagc tgtgaaagta 17040

gattcaccat aatggatcag gccaatctcc tttccttgga tattaggata ttttcaattt 17100

tttattttca tacagacact taagtgaatg aagcaaaatg tctgcacata ttcttagtta 17160

cctaaaggta tgatgttaga ttgtttttct tcactcatat ttttactaca tcagaatgtt 17220

gattacagtt atctttgatg gtatgatgtg agattattac tttgtttcca tacttatttt 17280

ctctaaatgc cttgcaatga acaggtttta tttgcataaa cacaatatta tttaaaatga 17340

tgccatgaaa atttctatat gaccaccgtt ttcatggtaa tggcttgtta cggctgcctg 17400

taattccaca taggggatag gccgtaattc acttaaccat cccctatcat tgaccggctg 17460

ggctttttct aatgtcttta agatcgttat acaggaaacc tactccatat ctggggttac 17520

gtcctcaggg agcagcccag caaaggctgg tgaacagatt tcacaccttt ggttcatcct 17580

gccaagctgc tttcccgaaa agatgttcca atttcccctc ccaccggcag tgcgtgggaa 17640

tgcctgccca gccgccctcc tctccctgag acggttcagg cccctggaca gtcgcatcct 17700

ctgctaggag ggcaagagaa agcaggaaat aatttggcca gcaagtgacc ccagacccag 17760

agccaggagt gatttttaac aagcctgatt ctcagaacag aacctgcaga gaaaaagaaa 17820

gcaagcaagg aaaaaaaaaa aaaaacccct ctctactgga aaaaacatac acatacacag 17880

attaaaataa tatttggcac ttacgtcgca cttttaaaat tcttcaaacg ctttataaac 17940

actagctaat taattaagcc gcctaaatct ggctgcagtc ggtggagaga gctcgagagg 18000

agctaaaaaa aaaatggggg agccagcgct acacaactcc agattttatt gtcgcctgcc 18060

tggggtgtga ttacaggcct ccggctgcac cacaagaata gccctgctcc aacatgctgc 18120

acgcctgact ccgcagcctc tcctgcgcct cctgctccct caccaactgc tgactcacag 18180

ccccttgtgg tgttattgat aaggataata ataactgcaa gccaggctga gtcccgggct 18240

ccgggcctgc accctcccct ggatatgcca gtatcaactc cacctaacac atgaagagag 18300

aggagatttg cccagggtca tggagccaac gacatccttc cttccttcat tcattaccgc 18360

attcattctg catattttta tcgatgacac tgtgttttag gcaggaggtt ggaaacagtc 18420

ccttgtggat cctacatcct actattggga atgtgacaat ccagagaaaa tcaaatatgt 18480

gaataataca gctatgtgat aataatggga ataagaatta aaacttaccc agcacctatg 18540

cagcaggcat accaccgtgt cagggtcctc atgactgtgc tggggtggat gccggaaccc 18600

cacttcatgg atgatggaat gggggtccag ggaaggtggg tacagaggtg gctaagaggg 18660

gagccatgct gtcacccagg cctgctggcc ctagcaactg tgcttccccg aggcagcctt 18720

tccagttggt ccagtctcga ggctgaaata cagacctatc cggaaagttc ctagctcagt 18780

gctgctggtg ctgcctctga tgtccccgcc tggatgggcc tcccgggcct cccacacatt 18840

tggccaccag cctcacccct ctcaggccgt ctgatgctgg ctgggcctct gtccactgtg 18900

ggttctgtct gtccactgtg ggttctgttt ttttctcctg tgccatgaag atcttgccat 18960

gttcaacttc aaatggaaat gaaatgagaa gctggacttc ctgataataa aagcagttga 19020

ggtcttaagt ggttgatcag gaaaaggaag tcgaaatggt aatatttaca cccaaagcag 19080

gtggagtggt cttaggcact ctgagactcc actcacctgc catgagaacc gggccccaag 19140

ggtgatccct tgccagagtc aagactgggt gaggctggga atgggagaaa gaggcaggac 19200

aatgggcaga tagccctggg cttcctgcag tgagggcaga taggggtctg tgagcctctt 19260

tccctggctg ccctgcccct gccagtgggg tggtgccagg ccatgggttg gtgggcaaat 19320

tcacagggtc tctctcctcc ccagccagag ccagaggggc acagcagctc tggacatctg 19380

ccgcagcctt gtctgatgta caaggcaaag gtggcccagg tcagggcagg acaaggtcac 19440

atgaagggtc agtggagagc ccaactgggg ccccaggtca cctgtcacct gcataccagg 19500

tagctgccac tcctctgtct acctggttct ctccttctcc cctattctag ctcgctctct 19560

cactctgcgt ctgtctctca ggcatgtgtt tctctgaggt ctttatgatc atgatctttc 19620

ttttttttgt ttcttgtacc tcaccttgtt cagaatcgag gccctggctc tgccttgctc 19680

tgaactgttc tcacttggtc cctatccatc ttcaacgcat ctcatgacaa gctcgtcctg 19740

ccctcttcct gtctgggctc cccccgtgca ccccaatctc tccacccagg gtccctgtct 19800

tgtgtgcccc agtgtctctg attggggctc aggctcctgc agccaacctc tacaattcct 19860

tccagctgcc catttgggag cccttgaggc ctggactggg tccagctcca gtgtgtcccc 19920

atcacagtga ggcctggatc acagggcatc agcaggtgtt ggtgagagtg aagtttgagc 19980

cacagccacc tccttgttta ttcaatgtat agtaacaaca acttcctcag ggcccatctc 20040

agggctaagt gacttctatg tgtgatctcg ttttcttttc agaacaaagc agaggcctgg 20100

gggctgttct acccatcctg tagataagaa aactgagact caaaactgat ggataaatta 20160

aggtcccaca agaacaaggg gcgagccagg tttcaaacca ggtccgatga ctctaaagac 20220

cagatgctgc atttggacgc tgtccccagc cttggcttat tttatcctcc cagagatcca 20280

tcgaggtggg gtcatagtaa ctattttaca gatgaagaaa ccaaggctga gagcagtaag 20340

ggcaaactaa gggcagctct tgcatttgtg tgacactttg ctagccaggc tcagtcctgg 20400

gttccaggtg gggactcaaa cacactggca cgagtctaca ttggatattc ttggttacct 20460

gagtggtggc ccaaggacac agctataggc cactgatcca gaactcaaac tacattctcc 20520

tactgcccaa accgctgagt acagcctccc ttctcggggc ctgcatcctg ggccaggcca 20580

gaaacagaat cttaacacct tgtacagccc cagatatgcc aggcccctac ctctgagtct 20640

ttgcccaggc tgttccctct gtctggaata ccaggctccc ttctggctca ttcagattag 20700

aattagccca gctcagatgt gactcttcca gagagggtcc aggcattcaa acccactctc 20760

tgccatgtca tttagctttc tgtgcctcag tgtccccact tagaatacag gctcatgttg 20820

caggtttgtt gtgagggtca aatgagatga tacatgcact atactgatct cacagcctgc 20880

ttctggtgtg catccagtca atgaacgtca ttgataaaca cttgaggaaa cacagaggat 20940

cacgtaagga tgaaaaaatg actgctttgt gctcctttcc aggagtggcg ggtgaggagg 21000

agctgcaggt gattcagcct gacaagtccg tgttggttgc agctggagag acagccactc 21060

tgcgctgcac tgcgacctct ctgatccctg tggggcccat ccagtggttc agaggagctg 21120

gaccaggccg ggaattaatc tacaatcaaa aagaaggcca cttcccccgg gtaacaactg 21180

tttcagacct cacaaagaga aacaacatgg acttttccat ccgcatcggt aacatcaccc 21240

cagcagatgc cggcacctac tactgtgtga agttccggaa agggagcccc gatgacgtgg 21300

agtttaagtc tggagcaggc actgagctgt ctgtgcgcgg tgagtacagc gtgggcctcc 21360

tttgcctctg gtttgtgaca gtaactcaat aataacaccc tccattcttt gagcaatgat 21420

caggtgtggt ggtaggtgct cctttccatg aattgatgtc tccccctttt acaaataagg 21480

gaagttgagg cctgaagacg tcatgctatt tgctgaaggc tccacagctg gtaaagggtg 21540

ggaacatctg catccttcat ctgcctggct gtgtagccct ggcctcttca aagctgaccc 21600

agccctttca ttccacagat gaggaaactg acagatagtg tgacttgccc aggcacagga 21660

ccagacctca cctgctgcct ccagagagca cttccctctc agggcagccg agtctcctct 21720

atgtgacagg cactcactga gaacctactg tgtgccaggc tccgccaagc ttgtgttctc 21780

atcccttcca ataagagatc cacccagggc agggagcaga gagggtgtgt ttcctttatt 21840

tcatcaccaa acaccaactg agaagattcc taaaaggcac agatttactg tttaatctga 21900

tggccctggt gagaggagaa gccagagggt tgggcatttt ctgtgggtct tttagaaaaa 21960

cacttgtgtg atttagggac tgattcatgc gtcactaaag gctctgaaca aagctgggtc 22020

atcatagaat gtctctcacc ccagggagga ggtctccctt ctaggtgact ttgggttcct 22080

ccgttaaggc agcaaagatg ctcattttgt atttactgcc agagccccag gatccttgtt 22140

ctaactccga atcctgggac attagtacag gaaggaccct cagaccatcc agcccaaccc 22200

ctagttacag gtggaaactt gaagtcagga gatgacaggg gccttgtcca cagtcatagg 22260

cccagttagt gacaggatct ggaccagagc ccacatctga ttccagggag gctccttcat 22320

tagccattgt ttacatagaa gagcctggtg catcataggt gccgactgaa agctccttga 22380

acagaactgc taagcaggaa gccagcactt ctcctttcca tcagtttcct aaaggttcag 22440

aatattcaca ttcagaccgt taggcccact ggtgattgga aaaccgctcc caaatgtaag 22500

ccatatcatt tgatactgag atttcatatc aggatagaga cctgctccat tgtgtaatag 22560

ctacagaata ctccagctta tggaaatacc agatttattt taccatcttc cattgatgga 22620

tacgtaactt gtccccaaac ttgaactatt aaaacagtcc tgcagtggat gaccttatac 22680

atgtgtgacc ttactcaggt gggagacgcc cactgcaagt gccattgcta gatcagaggg 22740

ggtgtgtgtt ttaatgtgtg tggatatggc ctaatttccc tcctagacat tataacaatc 22800

aagtctcccc acaaccgctg gacataggca gggagggtgg ctttatcctg attgtgcaga 22860

tgttacagga aaagacgttg aagttcccag atgatgcatc tcgtgcctag acaatcacag 22920

caaatacgtg gctcaggtgt gacccagatc tgctgactcc tagcagagtg ctcactcctc 22980

tttgccatgc tgtcagccta aatgatgccc tggatgagaa aggagggagg tgggaggggg 23040

ccagggagca gcagctggat tggatcctca tcctggcact ttctcctttt ataacaagta 23100

cttatgaagg aagagccatt tcttgccccc tttacagata aggactctga ggctcagaga 23160

ggagacctgt gtggtgagcc agtgggggag ccaaggtttg atccccacca gcctgactca 23220

cagcccctgt ctctgtaaca ctcagctatg cttgtcaaag tgtggagaaa tgctccaccc 23280

tactcattcc aaactcttgc agttctatag caataagcat aggctgaggc aggagaatgc 23340

ttgaacccag gagacggaga ttacagtgag ccgagatcgc accactgtac tccagcctgg 23400

gtgatagagc aagattctgt cttgaaagaa aaaacaaaac aagtcctatg ggagtaggag 23460

ggactggccc ccatcagaaa ggtcctgcag atgtctcatg atatgggaac ttgttcctaa 23520

aggacaggtc tcagcacatc actgagtagc atccatggag cagagttggg cctcctgcag 23580

cccagcagac cacgtgccca cctactcacg gtgctgagga ctaaggatga ggagatgagg 23640

catttcctgg tgggtgcttg gggctcattg gagcaagagt ttatattatc ccctcaggct 23700

gtcaggagcc cactgacttc agtttatggc tatagggtaa ccagatctgg gtaccctgga 23760

gggaggcttc tccctagaac tgcatgaagt cagggatctc aaagggaaaa agggaaggtg 23820

gtggtcagtg tcagggttca tgaagttcag gcacttgctc aattgattga catgagagct 23880

tggggaacac tggaaatgcc cattgggtac atgagggatg aggagacagt gccttcgctt 23940

ttctcagcct cagtttcctc atctgtagaa tggtgataat cagggactcc tttggcagag 24000

tggaaaggat ttgagttcct gtgtataaaa cacccaatgc agagtagaca cccccaaatc 24060

aaggtttttt gtttgtttgt ttgttttttc tttttttttg agatggagtt tcactcttgt 24120

tgcccaggct ggagtgcaat ggcaccatct cggctcactg caacctccac ctcctgggtt 24180

caagcgattc tgcttcaacc tcccaagtag ctgggattac aggggcaccc accaccacac 24240

cagctttttt tttttttttt tttttttgta tttttagtag agacatggtt tcatcatgtt 24300

ggcccggctg gtcccaaact cctgacctca ggtgatcccc ccacctcggc ctcccaaagt 24360

gctgggatta caggtgtgag ccactgcgcc ctgccatcaa ggtgttattt taagatgtgt 24420

taccttgggc aacccagttc cccttgctga gcctcagttt ccccatgcgt agaacgtcga 24480

taattgtacc aacctttcca ggatgttatg aggatgcaat gagcctgggg cgacgataga 24540

ccttagtaag caatggggtt ttcccaaggt catgtgatct tgggcaaatc ctggcttccc 24600

ttgagtcctc agcttccctg tctatgaaat gatgatgatg attgccaacc ctcacaggtt 24660

ctgtgaggat tggagaggat gtacagagcc tcggaatggt ggcggtgttc gggttatcac 24720

tgagctcctg cagaagtgtg gttaggagag tgtggccaga gtccaactgc ctgggccaca 24780

tcccagctct gccacctcct ggctctgtga cctgggacaa ggcactccac caccatgggc 24840

ctcagtgtcc tcacttgtca gtgataacac caacctcaca gaccaggagt tgggaccgaa 24900

tgagttaaat cctgtagcgt gcttagggca gcaggcgcct ggcctgcagt caatgttcaa 24960

cagatgtgag ctgtgtgatg ctgaagccat gcaacctcag atgggttagt gctcctccag 25020

gcctcaagga taaatggtcc ttaggatgta ctcaggtagt tttcaatctt ggctgctctt 25080

taggatcatc tgggaaacat tgaaataaat cccaggctcc accacagaaa tgcctcttca 25140

attggcctgg catgggccta ggcatctaaa gtttttaatt aatccagaag attctaatac 25200

agagtcagga ctgaaaaccc ctgggataga cacccagagg tcacggagga caagttggaa 25260

aagatggtcc tcttggggtg ccagatggga gaacagcttt tcagggtgtg gatgcttctg 25320

aacaacgtgc acccgggaca gctctttgga ggccccagtg gcctggccag accccctctt 25380

atgatacagg cctcgggggc caaggtaggg gccccagaca aagccctgct cagatccagc 25440

ctcagtgtgg ggaaaccttc catcctgaaa atgtttttgt ctaaatgacc gccggaatcc 25500

cagcaaatcc ttccaattcg gccccagaac cctccctgcc tcgacacacc aagatgtcac 25560

ggaggagtct gttccctcct cctttaaaat gcttccagct tctgtaaatg gctcttctgg 25620

agggaggcgc ctgtgatctt gtgttttgaa agccaattcg ccaaacttgt ttcctgaggg 25680

gattgtttag aaaaggggag agaatcctca cttggtggtt tctgctcctt gtatcatttg 25740

ggtttgtcat gagccacagg gagcagggtt tgagcctttt cttggtgttt ctgctctgct 25800

ttgttctgct gcctgaagga taaatgtgta gattcggaag cagtaatgac cagacctcct 25860

gataacagtc acaggggtca tttattaagc acctgctgca taccaggctc tgtgcttgca 25920

ttcatcctcc ctcttcttaa caggtggggg tttgtatcca cttttggccc tgggcctctc 25980

tgagctttgt cctaaaaggg gctcacacga ggatcccctc acagagatgg agtgaggatt 26040

aaagagctga cgggcatgga gggagcagcc agtgtccagc tccagagaag ccctggataa 26100

atgggaactg attgcagccc ctcagccctg tggggacatc caggccgctg gcctcaatga 26160

cttgacttct tcaagtgaca ccctccttac tgtgccctca aaatgtttga tgggtgtgag 26220

ggagaagtta atatttacaa atgcaagtat gtgtgatatg tgcttcctgg tgactgcgat 26280

aggaggtatg gggtaatgat tagcaatttg gagacacgtt tacgacccat ctgcagggag 26340

aaacagcccg cgttctgatt cagccatgtg tgtgctttca ggacccagga agtgcttctg 26400

cctgcttggg cgccctgtgt ctgtgtaatt aattgataat taaccacttt ggctttctcc 26460

acacttggtg atctttagtg tctatctaaa actgacacca aagaagaccg cagattcaga 26520

aaagtatccc ttcctttcaa atttagcact tggcacagat tagaccctgc tggaatattt 26580

gtggagaaaa tgaaaaatat gaagtaaatt cataaatacc tgaagagaga gctttggata 26640

tgaataaagt ctgggttaaa gttagatctg tttttgctta tttttgtgaa gcatcttctg 26700

tgtttatcca aagcaaaact gcttctgttg cctcatttaa tcctcgctgc atcccagaaa 26760

ggagaaatgc tgctgttcca ttttacagac aaggagctgg gtgtgccagg ccgcccctct 26820

catgggctga gcggcctggt tccagctctc atggtgatgc cacgttgctg cttactccct 26880

gcactgacct ctataaacgc tctctctgag gttggcactg ttgccagggg aggcagggta 26940

gaaacagtta ataccaagga tgagatggat cgttaatttt gatcctgtgc ccaaattgct 27000

ggaagtcacc ctgtgtggag tggagaagtt tgaactcagg cctggacttc cctctgggcc 27060

ctgcccctac actgtctgac ctcacatggg tcctcacctc tcccaacctt tgcatcctgc 27120

tctgtaaaat gacaataagg acaccgccct cattaatcct tccacattat tggaaggata 27180

aaaaataatg tctcagaagg ttcttaacgt gtcacacact tatcgttagt gattgtcatc 27240

tgagtcatgt ctcctgatta tcgatggtcc ttttgtagcc aaaccctctg cccccgtggt 27300

atcgggccct gcggcgaggg ccacacctca gcacacagtg agcttcacct gcgagtccca 27360

cggcttctca cccagagaca tcaccctgaa atggttcaaa aatgggaatg agctctcaga 27420

cttccagacc aacgtggacc ccgtaggaga gagcgtgtcc tacagcatcc acagcacagc 27480

caaggtggtg ctgacccgcg aggacgttca ctctcaagtc atctgcgagg tggcccacgt 27540

caccttgcag ggggaccctc ttcgtgggac tgccaacttg tctgagacca tccgaggtag 27600

aagaccctca cccagcccaa gcccacacct gaccgccaag cccacccccc accaccctcc 27660

actcatccag ctctactctt gctccagggc ttgaaatgcc tggaacctaa attcctaact 27720

gcccacctcc catgccccta gatgtgccag ccacttacta acattttaat ggcagtggct 27780

gggtgaccac accacccacc aggtgccaag gactgtgcta gccgtttcat ttatttcact 27840

gatagcaaac acagttaatg agtacctact atgaactaag cctctatgtg cttggtgatt 27900

ttctttattt tgctacagtt acaacattcc aactgcatgc caggggtggt gcttatagat 27960

ttccctcatt cttagcctag taggagctac cagtccttga gcacctactg tgtgctgggc 28020

gttgtgctta gtaatttcat tcatatgcaa agagcaccct caatgtctga gcacctgatg 28080

cctgcctagc tctgtcctgg tgatgacctc accgtggtgg gggagctacg ttatggagcc 28140

ctccctgtga tctgtctgtg ccacaaggtc agagcttctg ccctgtgctg tttcagttcc 28200

acccaccttg gaggttactc aacagcccgt gagggcagag aaccaggtga atgtcacctg 28260

ccaggtgagg aagttctacc cccagagact acagctgacc tggttggaga atggaaacgt 28320

gtcccggaca gaaacggcct caaccgttac agagaacaag gatggtacct acaactggat 28380

gagctggctc ctggtgaatg tatctgccca cagggatgat gtgaagctca cctgccaggt 28440

ggagcatgac gggcagccag cggtcagcaa aagccatgac ctgaaggtct cagcccaccc 28500

gaaggagcag ggctcaaata ccgccgctgg tgaggcctct atttcagctg acccagcttt 28560

tttaaacttt tagttttgtg ggttttttaa atgttaaaag tagtaattca tgcttattat 28620

agaacaatct agaagtctat aaatataaag tagaatttta atgtcagctc catttcccca 28680

aatcccacac tccaagagtc accactggta agagtttgat gcacatcttg atagatcttt 28740

tgacatagat ttatatcaca ggaggaaaga gggagagagg gggcccatct gttcatggtg 28800

tcctgcctct tgccttcttc acctgacagt tgtcatgctc cccattcctc actggcccag 28860

cccctcttcc ctactctttc tagcccctgc ctcatctccc tggctgccat tgggagcctg 28920

ccccactgga agccagggtg gagtcattca gggtgtttgc agcttgtctc acatacagtc 28980

agtgcttcag tgaacatcct tgtgcatgtt ccatcccaca tgtgactatt tctgtgtgat 29040

tgcccaagga acactttagg ccaaaatgca tactcatgta aggaggagat aatgctgtcg 29100

ctcccctgag agagtgtgag ggtgtcaagg tatcattttc tcagcccttg caacgtggtg 29160

ttggtgtaca tctcttttgt tttcaaatgg tggcacctga ttggcatttc agtgcagcca 29220

tggaatattt catcacctgt tatcaatgtg gctgatgccc tgtacatagt agaacccttg 29280

aatggattag aatggaagcc atttctgtcc ttcatgcacc cttcagggaa gctctcaaag 29340

acagccttgg ggctgtgtca aagtggccct ttggtttcac caatgccagg ttttcttagg 29400

atgtgtaggg catcggccaa tgctgggttt tcatagtacg tgtggagcat tggccaatgc 29460

cgggttttcc taggacgtgt agggcatcgg ccaatgccag gttttcatgg gatgtgtagg 29520

gcattgggca atgctaggtt ttcatgggat gtgtagggca ttgggcaatg ccaggttttc 29580

atgggatgtg tagggcattg ggagacagga tgtgtggctt tccacgccag ctatgttgca 29640

tacttggctg tgtgaccttg gacaagtcag tgcctctctc tgggcctcca cttgcttctg 29700

gctgtgttga aggggatgag cttcatgacc cctcagctct gagtctcagc tggtaggggc 29760

catgattatc ttgctcagca ttgtagcttc agggcctgac cacagtaagc actcagtaaa 29820

tgctttttga gtgaaagagg ataaaagagt cagttggttg gctggttggt tggttggttg 29880

gttggttggt tggttggttg gttggttaca taatccttag ttagccaatg aggtagagat 29940

gactgttggc cccagtaaca gaagaagcag ccagggtttg gagggaggct taactgactt 30000

gtccagggtc acacagctac ttagtagcat agatttgaaa gcacttttct gtggcttaga 30060

gccctgcttc tgatgaacca ctgtgttcta gtctatgacc cccttacctg tgctgtgggc 30120

agtcaagcct ccattgtatt ttcaagagtc ctggttcaga gcccctatgc taactcctga 30180

gcccagtgtg catggcccag gtgctgggca cagaccggtc gtcagcccct gagctgtgca 30240

gagcctgctg gccgcactgc ccttcttcca gctgctgatt cagtttccag tggatctggg 30300

gtgccctcat gaccgctctg tggttcactc cagatgagaa ggaacgtttc tgttcctcag 30360

gagtgagatt tctgcctgta tcctgcagca ggggttcctc cgtagctgtc agctacaaat 30420

atattccccc ctggcacctc agcatggttt ttgtgctgtt catggatgag tctcgtgggc 30480

aagtgtgtac agacccatga gtgtgcccat gtggctcgag acatctaaga aggtccagcc 30540

agatgttctc agttaatgat gcctgcttag tggtgaaaag cagtggtggg tttggttttc 30600

tgtttttaat ctgcatacgt gaagcctcta ttccatgtgg tccctagaga acactggatc 30660

taatgaacgg aacatctata ttgtggtggg tgtggtgtgc accttgctgg tggccctact 30720

gatggcggcc ctctacctcg tccgaatcag acagaagaaa ggtgggtgca ttcccctctt 30780

cctccctaag ggtttgtccc tggactgtcc tcggagggag acgccattag gtgctttggg 30840

ttaaggacat cagcttctgc cagtagcaag aagtccagag gtagtggtgc tagggctggg 30900

gcagtggcct caccatgtta gagtctgggt gggtcactct gtggcttccc cctcatggtt 30960

acaagatgtt catcactgcc ccaggccccg tgtcctcaag gggccactac tgagcaaggg 31020

agctgccagc ctgggaagag aggaggcagg gactcttctt acctgtctca gggaggacat 31080

cgtctttccc aggagcctcc cggcagactt ctgctttcct ggtacaagtc taggttacat 31140

gaccagtccc tggacaggag gccagaggga atggagataa tagcgccacc atttgattgg 31200

gaggacttaa caagtgattg catgggaaat gttagggaag taacctggca cacaagtgct 31260

caatgtatgt cagccctgat tattacccgt attgtatgtg agcccagaaa tatgagccac 31320

ctccctagaa agccccctca cagggcaagg tgcatagcct ttgactgcac agcaggaggg 31380

cacagggtca aacccaggac accgcttccc cttgtttgtg tgcacgtgtt acgtgtgtgt 31440

ctgtgtgtgt gtgctggttg ttgggagggt tggttgttgc tgtggcaggg cctctgactt 31500

tgaagtcaga caaaccaggg ttccagaccc tggttatata ctgtaaacaa tcaatttggc 31560

ctctacaagc ctcagtttcc ccatctgtaa aatgggttta gcaacgcctg tcttggagaa 31620

gttacaagat ccaaataagc ccatgtgaaa taacgggtct gacacattct aggatcgcca 31680

taaatacatg ttctctttcc cactcttgga gaagacaatt ttaagctggt atttctgcaa 31740

ccttttttta ttacaagaga tagaaataca tttcacatct caacccaaca catacacaca 31800

ccctttgtat ataaaattaa aactgatatt ttcttttttg tcatattctt ttttctgttt 31860

aaaatgctgg ttgtgaccca ccgcctccaa agggacaagt gctgtcccga gctgcctttc 31920

tgtggcctgg cacagctgag cttggttggg cacattgtac actaatcacg catcccttca 31980

gtagctcctt cacctctgcc tcccaagttt ccctccctcc tgggctggct ctgagatact 32040

ggccaagcct gatgcttctg tctgggaggg ttggagtcag gccctgcttc tccagaatct 32100

gcaagacgtc ccaccaagac gtctacccaa tgggttccct aaagttcctg gagagcccca 32160

cacagggagg actcctcctt ggatggggaa gcaggtccct tccccaccaa gggtgacagc 32220

ccatcccatg ggatgaagca ttcatagcag ccgatagtga aatcctggcc atcctttctg 32280

agtctcaggc ccaggcagca cgccagctcc tggcacacgt gagctcatta ctgtgggctt 32340

aggtgttgca gaggaatgga gtttgcaggg ctgcagctct ggtaaaggcc acccagcccg 32400

agtggcaggt ctgtgtgcag agcctagctc tcaaacacag gctccagtga cagggacggc 32460

tagtatcccc cagcagttcc aatgtgccag gcactgctca ctgtgctttc cctgtagtaa 32520

cagtcagtgc tcaccacaga cctcatgggt gacattacta accccacttt acagatgagg 32580

aaactgaggc atgggaaagt tataaaactt gttcagggcc tcacagccag gaggtggtgg 32640

ggccagaatt taaacccaag gaccatgtct aggaccacct tgcacactac ctccctccgc 32700

cataactaga ccccccttct ccctcattag gccctggagg ggtctgccca gcctctcaat 32760

gctcacagtg aaatgtcctg atttcaactc aaccactacc cagctgtgtg accctgagga 32820

gtcacctagt ctctctggtg taaaaggggg tgactggtgt cctatcactg gattcccaga 32880

gatgacttgt gtccagcact ttgcaacctg tcagcctcca ctcagacttt cggtgctgag 32940

cacagcttcc aactggtaca cctcatctaa agatccagga cctgcccagg cagcaagcgt 33000

taacccagca gcaagaattc ttgcactgtg gcagcctcca gcctattaaa gtgcggtgca 33060

gagtgcattt gggtgtgcat gttgcttttt aattacaatt ttacagcaaa atgggagtca 33120

aacggctgct gaaccctgga ggcttctctg tgggttaccc catatcacag gtttaaaacc 33180

tctgaaccag atggacactt tctttccttc caagatcctt tttttggaaa gtggtggcat 33240

gtaaataaat ggatgctcag cccaaccctg caagttatag aagctgagag agaggaagtg 33300

acccacccgg ggtcacatgg tgagtgagtg ggtagcagac ccggggttca cacatgatcc 33360

ccgatttgca gccctgctag aatctgtttt caagccacga agggcactga ttggacctta 33420

ggcttgttgg agggtctccg ctgaggtttg tggttgaatg gaggtgagac ccagacagag 33480

gtagggcggt tgtcgcctcc caggctgaag gctgcttttg gacccctggc aacttccaga 33540

agtggaaaag caggaccatc tagcttactc cttcccaggc ttccttggtg gaagaggatg 33600

cccactgggt gggcatgggg gtctcctgtg gttccaagga tgtgattaca gcatttcctc 33660

tccatgtccc tggaggcaaa ccttttgcca aaaaatagtt acataagaaa agtgtgcttc 33720

tagttaaaca actggctttt gtttcttttg tctttcagcc caggctccca cttcttctac 33780

aaggtaagtg catcattggt ccagaccctc ttgcagttat tatttggtta tttgacagcc 33840

ccccagacta caaagcataa tccatgtcca ctgacctcac caatgtgttg gtcaacatgt 33900

ctctttctct cctttgtaac ctcctcacac tgggtcacgc tgtttttaat tattgtccca 33960

aatgaggggt ttttttggtg cacagaggtg ctaatttctt gcctgctctt cccagacaac 34020

gagacccttg tctaatccca tgtggactgg ggcagagacc tcccgggctt tctgtcctca 34080

cagacatcct gcacacgcca ccggatgcat cctcccagat ccaagttgta tactttactc 34140

tttcctttgt ttagtaaata tttattgagt accttggcgt gctgtgaccc tggaatgccg 34200

ggatgagtag gatagaatct gtgccctctg caagctcaca gcctggtcag aggctcagac 34260

ggtgacatta catcactgat gtacgtcacc gatggcactt tagtttgttg attcacattt 34320

ttagtgactt gagtccctac tatgtgccag ccctgctcag gaccagctgg tgatatagca 34380

gtaaacaaga atcagcccag gctttcatgg ggctcagacg ccggtgagag aaacaggcat 34440

aaacgcagaa ataaagtgat tcatggtgtc atatcaggtg gtgacaactc ctaccctgca 34500

aggagatgga ggtgaatggt ggggggctgt cttaggcaga ggaagggact gcctctgaaa 34560

aggggatgca aacgggtgcc tgaaactagt gagctatgtt ggacaggagc atgctgggcc 34620

agagaggagg cctgtgtgct gggccagagg gaaggagagg aagaagataa ggtcaggaag 34680

acccaggtcc tcggcatccc ggtgtccttt gcagatgccc ctgcagtcag aggtgttacg 34740

aactattctg tgaaaagcct ttatcggctt tttggaaggc ctctgatctt catccccagc 34800

agagcagagg cagcacacca tcatctgcca tgtgaaccca tctgcataga accatgtgga 34860

catccgtgct aagagtgata aataaaacta taaatagctt catgttggtt cacaccaggt 34920

tttgccagca actgcatagt gccaatcttt tgatggttta gagctttctg ggtttcagaa 34980

ttacagaata ggacatgatg atctggagtg gggcctgtgg gtgttagaag gactcgggat 35040

tttatccgca gtgggatgag gaaccaaaag atggacacga tctgactttt gttttaaaag 35100

tgtcgcctgg ctacatgtgg agcccagact acaggaggtg aggatgtgta ggtgaccata 35160

gcggtactgg tccaagcaga aaaagatggt gacctggacc aaagtggtgg gatgggctga 35220

gaagtgctca gattctggat atattttgaa ggtagacaca agaggacttg ctgatagatt 35280

ggctgtaggg ttaaggacca cctcaaggtt gggggcagtg gagtgctgta tcctgagttg 35340

gggggtgcag gtgatgggtg gagtgatggg gcaaatatca ggcaaagcag ttgcatatag 35400

caggagcagc tcttctgcca gagaaggctc tgggaagcat caggtgcccc gctcctgccc 35460

agcagtgtga gtccaaggta ctcttgggat aaagccttgc ttcttgctct gatctccaca 35520

gggggcctcc tggtctgagc cctgcccagc ctcctggcct tttcctcccc tgaacaagca 35580

gagccgcctc tccagcaaaa ctaggctctt cactgccttc tgggcacatg tgttttcctt 35640

ttgtcatgct tgtgcctgtc acactcccac acctaggtca ctcttccaag ggcaagactc 35700

gtttatatcc tttgccaaaa aaccttcagt ggctctacct gtccctccag ataaagtcca 35760

gatgcccccc tgaggctcct gggccccttg tggtccagtt gccttgaaca gtttcacttc 35820

tagccacccc ctgtcctcac atttcacggc tcaaccacac tgtgctcccc ccttccctcc 35880

tcccacagtc tgccctcctc ccccttgcac actgccctca ctgctggggt gtgctccctg 35940

cccctgctca tgcactaccc agggtccccc agctccaggg gtggcaggca cagtgggctc 36000

tgtgaatggc accccctgga gttatgcaat gtggtagcta gagtgggacc cctcctgtca 36060

cctgctcacc tcctgcttct cctgagatca gcaagggcac gacatcctcc agggtcggag 36120

ggcttcctgt gaaccctcct ggtgccgcag cgtggacacc ctggtgtgtt caaggtgtct 36180

ctgcacctgt cccatttcat aacccacccc tcaaggccag ggccaatgtc cctgaggttc 36240

actgcaaata gctgctaaac ttggcaccat gcctggaatg ggataggtgc ttagtaaagg 36300

tttactgact gaacaagtga acaaacgaat gaatgaatgg atcagtgcat accatcctaa 36360

accactgtag cacgtcccac tctgccttct ctgaatccct aaaatgctgc ttcccagtgg 36420

ctacacagcc ccatctagga ggatctctct tttttgtttt ttttgttgtt atttttttat 36480

tttttatttt tgagacggag ttttgctctt gttgcccatg ctggagtgca gtggtgcgat 36540

cttggctcac cacaacctcc gcttcctagg ttcagatgat tctcctgcct cagcctcctg 36600

agtagctggg attacaggca tgcgccacca cccccggcta attttgtatt tttagtagag 36660

atggggtttc tccttgttgg tcaggctggt cttgaactcc cgacctcagg taatccacct 36720

gcctcggcct cccaaagtgc tggaattaca ggcgtaagcc actgcgcccg gccctgttgt 36780

cattattttt tacttgtctt acctccccga ttagacaatg agtttctgga agaataggac 36840

attgtcgata atagtaacag caatagcagc taccatttat tgagcactta ctgtgctcct 36900

ggcactatct atgcctttat gtgtattaac ccacttgtga ttttaattct tgcaggaacc 36960

ctatgattta tcactgtccc aagttcatag aacagtgttt ttcaaacttg agtgtgctac 37020

agaatcccca aaggacttgt taaaacacag attactgggc cccaccccca aagtccctga 37080

ttgagaatct ctatttccac aagttctcag gtggtgctga tgctgttgtt ccccagccac 37140

actttgggaa cccctgttct agaaggttct tattccctat cctatggttg aggaaattga 37200

ggctcagaga agtcgtgaca ttcccgaggt cctgcggcta ggaagcaccg gaatgatgac 37260

ttcagcctcg acatctcttg tactaaagct agtgccttca tcactgttcc catgcttctt 37320

gctgtctatt tcttcttccc cagagaaacc aaccgttttc tgcctgtttt ttatttttcc 37380

agcgtgagcc taccaaggga gccatgagat cacttgctcc ctttcatctc tctaaaccaa 37440

agcaatagct agtgagtgct aatggtggtt tcccatgtag cagccagctt cggctttggc 37500

ttgctgggat cagacatttt ccagaggacg ttggtgtgag taacagctga gcacctcagg 37560

gaggctgcaa gcggggaggc tgccactttg agctcaaggg gttcttcagg ttgcagggct 37620

ggtggaggga ttggctctct ctaactccct gggttaggaa tgtacagaca gaatattgtc 37680

tgtaatcttc cccagctcag atgagagaaa aatgtgtctc tgctccctta atggggcact 37740

gatcattcga tcagccattt attcacttgt cagacattca ctgcatttat tcttggaata 37800

tttagggctt ccctgagtct ttcatttatt cgtcgggact gagcctaact cagtgccaag 37860

ccttgagcag agggttgcaa aggggagcag aaaaggccca cccctggtct catggcactt 37920

aagagccagt gagaaagatt cattcattca ttcattcatt cattcattca gcaaatacat 37980

atggactgct gacgatgtgc cagcccagtt cttggtgctg gggcttcagc agtgaatgag 38040

actggcccag ttcctccctg tgtgaagctc agtgtccgct gaggagacag gttaccagac 38100

aagctggaaa gttaataagg gatgtggtgc agctgctgac tagataaaat gaagccaggt 38160

atgggataga gcagatcaag gaggtggggt ctggggcgag cagggagggc ttgtctgcaa 38220

ggtgacattt agaagatcac tgaaggatga ggaggttcca gccaagggaa tatctgggac 38280

aagactgcac caatcaaagg gaacagccag tgccaagacc ctgagacggg agcagtcaga 38340

tgcatgcaca gaacgcagag gaggccagag gagctggagc agagcagagg caggagaggg 38400

atggagccta gagcagttgg ccgagctgag ccttggagct ggtgtcactt gaccaccttg 38460

gagtggttat agaggggttg agcattctga gcagaaaagt aacatgacat cacttatgca 38520

tttcagatag ggagaatgga gggcagggaa gcaggaatag aggtggggag atcaaggagg 38580

aggttcttca ggaatcaggg gagttcggat ggtggagagg atggggtggc tggctttgat 38640

ggatattttg gaggtggagc cgacgagccc taggttggat tagatgagag ctgtaaagga 38700

agagagagaa gaatcaggac tgactcccac ttctccagcc tgagcacagg atgaccgatg 38760

gtagagctat tcactgaaat aggaaaatcc aggggagaca cagatatggg gaaaatgata 38820

attggttatt gtctgaaatt gctttgaaag agaagaaccc aatgttttga gaatggagtt 38880

tgggggttca gtgaatattt ccccaagaaa aggctgttta aaaatagcat ctgaaggaag 38940

aaaaggatta aagcagatta agaggcagca gcatggagca tgcaaaggcc ctggggcagc 39000

ctgactggca ttcattccaa gtcactgttt ttgagcacat gtggtgagga agcatggcag 39060

gcaggcaggc agacagcaat agaaaccaga tatgcagata gacaggagat ctgagattgt 39120

catggagggg cagcccagcc agcgcagcac tgtcagcaga gccatcacaa ggtgacacca 39180

tagttgagtc ttgaaggaaa caggaaagtt agccaagagg aaaaatgatc aaaggggtcc 39240

ttcgaggctg ctattgaagc atgaagtaca taacatcaaa tgccaccccc cccccgaaat 39300

atctggtggg cagatgagtg ggtacatgag ggcagggcag tgaattagcc tggaggcagc 39360

acgtcagacc acgtgggact ctgagcaggg cgatgagttg gacttgaacc agccagccag 39420

tgggaagctc caggagggct ggaggccagt agtgtgggga cagatctgcg tgatcttccc 39480

cagcagacgt gcccctgctc ctggagggtt ctttctgtgg ccacgtgggg cttgtcatgt 39540

ctcagaactg ctttcagagc caagcactca tgccaaatcc attaataacc ctgctgccaa 39600

gccccactgc tcatttctga cattatccat gctaacatcc ccggggttgc cggcttgaga 39660

atttacacaa cttctgccaa gccagtggct ctggtttgct catcaccttg accgtgaccc 39720

tccagtgagt caagtcatga cctccaatta cagaagcaag tgcacgaaca acttctttga 39780

ttaacttgga catcttggtc tttttctttt ctttctttaa tgatgataca tagatgttta 39840

taatacagaa catttggaga atatgggaaa ctaaaaagga gaaaaaaaat accatgcaaa 39900

gaccactgca gtttgcattt tgctatgttt ccttccagtg ttatccacac atttttcata 39960

attgagataa tcctacagag aattttcctt tttcacttat cattttaatg taagcaattt 40020

taacttaaaa aaaaaacctc ctcataaacc ttttaaatct ttatctgtgg atttatccta 40080

atttccctaa ccagtcccaa ttggcagagc cctaagacat aaacctcctc ccccatccag 40140

cccttgacaa atgcctttgt gttgaaagct ttttctatat tttgaattcc cccaaaaaaa 40200

agtgcaatta tgagtcaaag gatgaggaca tcttccaagc tcttgatttt gccagctgct 40260

tcccaaaagg attgtactgg tttgcctctc ccaccagtga cgtatgaaag ctccagttca 40320

tttcaccata gcttccccag caccaagtat tataattttt aaagatcctt gccaatagag 40380

taggttaaaa aaatgatagt gcattgctta gaattccttt ttagtgagat tgaccccttt 40440

gtccaaacat ttgatatgca gttgtatttc tgttatgagt ccttcgttca tgtcctcaac 40500

ccatatagaa aatggagcct aaatgttatt cttatcagtt tgcatgagca cttgagatag 40560

tgagggtatt tttatctgtg tgtctctttc ctttttaggt tgcatgagcc cgagaagaat 40620

gccagagaaa taacacaggt acagtccttg gtgagatgcc cttcctggga aactccgtgg 40680

cgtggttgct tcacatcaac cggaattgca gatctggttc taaattaaga ctccttgtgg 40740

ggtggagggt ggaggatctt acactcctag ctttccccat gtcctgtgca tattccttct 40800

ctctactgca gccaagagtg gctgttgcag cctcatgact cagtgcgacc cattgcacaa 40860

ctccccaggg gcaccattca caatccgtcc tatgaaaatg gtgccctgga gtcaggctgc 40920

agctccctta gtgtggatcg tgccttccca tctctctaca tttgcccagg cccatccctc 40980

taccaggagc gccactccca gctcactgcc cgctcctcac agccaggctt ttctatgctg 41040

gaactgcaaa gcccagcctg ccagggcctc tcccaccttt gctgatggaa agaccccatg 41100

tcattaatta cctcacacaa gggcacactc actcaagctg gaggtttctg gtcagcattg 41160

ctgagatgat tttgcagcaa cacggtttct gagaaaccta gccctgctta tcaccggctg 41220

atgctgtcag accttgaagg cagagggctg ctcagcatct tgtaacaaga caggccctgc 41280

tctgaacccg gaacgagagc gcgccagctg tccttgacat ttccaaggca gttgtcatct 41340

ttttagatgt aatctgttga aggagtcggg ctggtgaaag gcgcagcatt tgtagaccaa 41400

aatgccttgg ccctgccctt gtgacccatg gggtccccc gcagccccgg acatgagctc 41460

gagttgtaat taagaaaaaa caatttccag tcatgagcga gggagatctt ctcccccatc 41520

attttacttc tggacttcct cctggaatct tctgggggaa aaaaatgatg gtgagaagaa 41580

tgggtcatgg aaacctctac gaaattcaca gcagagactt actgggcacc cactgtgtgc 41640

cacgcactgg acagtgagcc agacagtcac agcccagctg gcagagtgcc tgcgattcca 41700

gtgccaggtg ccagccctgg ccagggaggg agttgcacgc tgtcaacacg gatttcccag 41760

acagaagcaa atgagagcac gcagggctca ggacagagga gctgtgtgct aagagcacag 41820

tcgtagagtc caacacactt tggttctttg ccagccggac aactttgtcc actttattga 41880

acctcccttg gtctcagtgt cttcagccgt aaagtggaat agataatcct tcccaagcag 41940

ggttgtggta agagtggatg gggtgatcta tgtgattgtg tgctgcttgt aagccctgag 42000

aactgaaatc agccattacc gtgcaccaac taggaggtgt gccggttggt ggtagcttta 42060

ttctgttcta tcacgcaggg gccagtcaca gagggagcaa agtttatcat tcatgatggg 42120

cgcttttgag gcggcagtgg caggttgata ggattagcac atgggaggta tccaagtttg 42180

ctaagtggtt aatgattgcg aacatttatt gaagacagat gctcaatagg taccaggctc 42240

tgttctaaac gctttacctg gataatctca ttccatcctc acagcaaccc tggcagtaga 42300

cactattatc atcccctctt tacagatgga gaaactgagg cccagagagg ttaagtgaat 42360

tgctcaaaat ccctgagcta gaaccaggat tcaaacccag gctttctgac tccagaacgt 42420

ttcccgcttc actaactggc gatctcgttc ttatactgcc cctgttctct gtctccctgt 42480

ccccagcttt gccctgagtg aaggtgaaat tattgccatc cactcatata ttcattcaat 42540

atggatcaga cttcttatgt gccagccttg gtttctgaat cataacaaat gcccagtgaa 42600

tatttgtgga ataaacgcat acattaatga atgtccccac acgtaccacc atcagcagca 42660

cctcctcagg cccccaaaca gacatggtcc tgggtagcgg gccctgtgat ggaaaacaaa 42720

catgggctga ggaagcacaa agaaggaaca tggaccctgc caggagaggg gagagccagg 42780

gagggtctta gtatgagcca tgcttcatct gaaatttaca tggtggattg ggatttgtta 42840

caaggagaag caggacagac accctcgagg gatgaacatg agaaatgggt gaagactgca 42900

ggtggttcag gctaggagga ggcaggaacg ggaggaggtg acactgaggc ggggctgtga 42960

ggagactgca ctgtgcgggt cagaaagacc cttcaggcag ggtgaggagg agggcagatg 43020

ggaaggggca aggctggagg caagtaacgt tggcaagaga tgaggggaac atgacttatg 43080

gctgagccag tgtgggccga gaggacacag aagcatccag acttggtatt cagtttgagt 43140

gagtgggcca ggggctatag aatgaccttc tcatgacttt ctctttgggt atcttaaatc 43200

caggacacaa atgatatcac atatgcagac ctgaacctgc ccaaggggaa gaagcctgct 43260

ccccaggctg cggagcccaa caaccacacg gagtatgcca gcattcagac cagcccgcag 43320

cccgcgtcgg aggacaccct cacctatgct gacctggaca tggtccacct caaccggacc 43380

cccaagcagc cggcccccaa gcctgagccg tccttctcag agtacgccag cgtccaggtc 43440

ccgaggaagt gaatgggacc gtggtttgct ctagcaccca tctctacgcg ctttcttgtc 43500

ccacagggag ccgccgtgat gagcacagcc aacccagttc ccggagggct ggggcggtgc 43560

aggctctggg acccaggggc caggtggctc ttctctcccc cacccctcct tggctctcca 43620

gcacttcctg ggcagccacg gccccctccc cccacattgc cacatacctg gaggctgacg 43680

ttgccaaacc agccagggaa ccaacctggg aagtggccag aactgcctgg ggtccaagaa 43740

ctcttgtgcc tccgtccatc accatgtggg ttttgaagac cctcgactgc ctccccgatg 43800

ctccgaagcc tgatcttcca gggtggggag gagaaaatcc cacctcccct gacctccacc 43860

acctccacca ccaccaccac caccaccacc accactacca ccaccaccca actggggcta 43920

gagtggggaa gatttcccct ttagatcaaa ctgccccttc catggaaaag ctggaaaaaa 43980

actctggaac ccatatccag gcttggtgag gttgctgcca acagtcctgg cctcccccat 44040

ccctaggcta aagagccatg agtcctggag gaggagagga cccctcccaa aggactggag 44100

acaaaaccct ctgcttcctt gggtccctcc aagactccct ggggcccaac tgtgttgctc 44160

cacccggacc catctctccc ttctagacct gagcttgccc ctccagctag cactaagcaa 44220

catctcgctg tggacgcctg taaattactg agaaatgtga aacgtgcaat cttgaaactg 44280

aggtgttaga aaacttgatc tgtggtgttt tgttttgttt tttttcttaa aacaacagca 44340

acgtgatctt ggctgtctgt catgtgttga agtccatggt tgggtcttgt gaagtctgag 44400

gtttaacagt ttgttgtcct ggagggattt tcttacagcg aagacttgag ttcctccaag 44460

tcccagaacc ccaagaatgg gcaagaagga tcaggtcagc cactccctgg agacacagcc 44520

ttctggctgg gactgacttg gccatgttct cagctgagcc acgcggctgg tagtgcagcc 44580

ttctgtgacc ccgctgtggt aagtccagcc tgcccagggc tgctgagggc tgcctcttga 44640

cagtgcagtc ttatcgagac ccaatgcctc agtctgctca tccgtaaagt ggggatagtg 44700

aagatgacac ccctccccac cacctctcat aagcacttta ggaacacaca gagggtaggg 44760

atagtggccc tggccgtcta tcctacccct ttagtgaccg cccccatccc ggctttctga 44820

gctgatcctt gaagaagaaa tcttccattt ctgctctcaa accctactgg gatcaaactg 44880

gaataaattg aagacagcca gggggatggt gcagctgtga agctcgggct gattccccct 44940

ctgtcccaga aggttggcca gagggtgtga cccagttacc ctttaacccc cacccttcca 45000

gtcgggtgtg agggcctgac cgggcccagg gcaagcagat gtcgcaagcc ctatttattc 45060

agtcttcact ataactctta gagttgagac gctaatgttc atgactcctg gccttgggat 45120

gcccaaggga tttctggctc aggctgtaaa agtagctgag ccatcctgcc cattcctgga 45180

ggtcctacag gtgaaactgc aggagctcag catagaccca gctctctggg ggatggtcac 45240

ctggtgattt caatgatggc atccaggaat tagctgagcc aacagaccat gtggacagct 45300

ttggccagag ctcccgtgtg gcatctggga gccacagtga cccagccacc tggctcaggc 45360

tagttccaaa ttccaaaaga ttggcttgta aaccttcgtc tccctctctt ttacccagag 45420

acagcacata cgtgtgcaca cgcatgcaca cacacattca gtattttaaa agaatgtttt 45480

cttggtgcca ttttcatttt attttatttt ttaattcttg gagggggaaa taagggaata 45540

aggccaagga agatgtatag ctttagcttt agcctggcaa cctggagaat ccacatacct 45600

tgtgtattga accccaggaa aaggaagagg tcgaaccaac cctgcggaag gagcatggtt 45660

tcaggagttt attttaagac tgctgggaag gaaacaggcc ccattttgta tatagttgca 45720

acttaaactt tttggcttgc aaaatatttt tgtaataaag atttctgggt aataatgag 45779

<210> 31
<211> 43093
<212> DNA
<213> Homo sapiens

<400> 31

agaattcttt ggcaggggcg accttagaat cctggggagg agcgagaatg gaatcccggg 60

gaggaacagg ggtggaatcc ggggggcggg gtcagaacgc caggaggggg cggggccgga 120

gccagggtcg gcttgactcg ggggagcagc gggtggatcc tgtgacgtca gcgggttcga 180

accgccggag ctgagcgaga ggccgggggt gccgagccgg gcggggagag ctgggccggg 240

agagcagaac agggaggcta gagcgcagcg ggaaccggcc cggagccgga gccggagccc 300

cacaggcacc tactaaaccg cccagccgat cggcccccac agagtggccc gcgggcctcc 360

ggccgggccc agtcccctcc cgggccctcc atggcccggg ccgctgccct cctgccgtcg 420

agatcgccgc cgacgccgct gctgtggccg ctgctgctgc tgctgctcct ggaaaccggt 480

gagacgagcg gacggccccc tgccctcgcg cggaccccct ccaaccttac cttccgagct 540

ggggaagccg agcaccttcc ccgcccaccc ccggctcccc gagccccctc tcgcgtgccc 600

ccttcctggc tggccccaca gactccgacc ccctacacgt ccgattctct cgaggagact 660

ctgacccctc ctttctgcat ctctctcacc accaccccca tccttcttaa tccaattgcc 720

catcctacgc ctgcttgtct gtccccctcc ccatttctcg gattcccgcg ccgaaagggt 780

taaatcccag gaaggcctgg tgccccctcc ccaccccgcg ccgagtagtt cacccggatc 840

tgggtctctg tctcctctac ccctctcagg aatgtgggga atgttccccc cacctaccgc 900

agtaaggtct cgaggaagag gcgagaccac ctccctcccc tatttttcct tcttcccggc 960

ttgggcgtct gaggcccgga gagaactgag gacaagcgga cctgattcgg gtcccttgga 1020

gggctaagga taagagttct ggcgacttag ggggaagggc ctgagctcgg agctccaacc 1080

ccgaacctca gggagttggg ggttctgact ccgtggactc cagaaaaatt aggggccggg 1140

ggctccgtct tcagggtccc tggtagaatg gaggctaggc gctaagtcta actgggatct 1200

aggtaaaact gggggcgaga gaggatcaaa cttgtagctt ctggatttaa ttgtgggcag 1260

ggggcttcgt tttcagggtt cccggcagaa ccgggggaca ggagactcga tctcgtgggc 1320

ttcagactta attgtaggca gaggaacgtc ttctgagatc caaacagaag tgcgagcagc 1380

gggccccgtc tcttgggctc cagatatgat caagggtcga aggctctgtc ttgaggctgg 1440

agtctcagtc gcctgagtcc cagatagaac gggaggcccc gagtcccaga ggctgggaga 1500

ctcctccctt tccctatgaa tggtcggggg cgacggagac gtcgccgggg aagttttcct 1560

ggtttcactc tcgccgagcc agggaggggc ggggcgggcg ctcctggctc cgggttccgg 1620

actccccggt ttcgggacct tatctatcct ctggtagcgc ctttgtcctc agagccatag 1680

caggaggtgg acgagtggag ccgccgccct tatcccccgt ccctcccatt gctatgcctt 1740

ggaccccctg gcgtccttcc atccacccca ctaggggagg agagcggccc ccctggggag 1800

ggaggggctg ggagggaacc cttgaaagag gcgctggccc cgccccttgc aggtgacccg 1860

gttgtgtatt cccagcgaca aactctggga tgctagagtt tcccgagggt ttgcgcagaa 1920

gagagctcag ggggatcttg ggggaacccc ggccacctct ctggcctcat ttcccccagg 1980

atggctctca ctggcctccc agctttgacc cagagctggg aactccgctt ccatcccctg 2040

cctctccctt caggcagaaa taggcagatg agatcagaac tggcacctct gggtgaattc 2100

tccgaaccca ccagaccctg agcctactgt tagcttggtg catgatctca ccgaaggctc 2160

cagcttgccc tgggaaacag gtcctgtttc tgtttccatc ttagagatga ggaaacaggc 2220

tcagcaaagg caagcagcct tctcaaggtc tcacagcagg tcccggggaa gccgggctcc 2280

ggttcttcag agccccgcac cgggtgggct ccaggaagtg cctctgagtt tccatttcct 2340

ccctcgtaaa aaaactgaga taacaatcct ttccctgcgg accatccgga aggctcccag 2400

gccccactga ttccctgtga aacctcttag cccctggaga aggagctgtc ggcccctttc 2460

ttgaccaccc ccaccccaat ccttcgcctc tgctgtccac tggctggccg gctggcatgt 2520

gctcgctctc cctctctctc tctctcccct ccccctccct gttttcctcc ttccctcact 2580

ttctttcccc ctcttcatct ctcttcgttt ctcacccttt ctctctccat ccctctccaa 2640

gctctttctc tcttctcctc tccccatctc tctctctcct ctccgtcttt ctctgtatac 2700

ctgtctagct ccactgcctc ccccgcttgg cctcctcggg gaatgcgggt gcaccacctg 2760

ggcccccagc tgggccggct cccctccccc agctttcttc cctgcccatc cgtgtgtcca 2820

tgtccgccag gcagccccac gtagtggagg gggaggggag cagaggacgg gcagcctggc 2880

tgtctcctct cattagggaa gggggactgg gcagaggaga agttgggggg tgggtatata 2940

gagaggctgg agctacaagg atgggtacag aggccaatag agggaagcgg tcagacacag 3000

atttaggaat ggggtcagag acacgtgtag gaatggaatc agaggataga aggggagtga 3060

aatgccggca cagggtcggg ggagtgaaat accgacacag ggatgaggct gaaagacact 3120

ggaatgggac ggaggagggt gggggaaggg agctagacat agataccaga tgaaggcagg 3180

gacaggaaaa ggaaaggggt ttggtgacag gcagggagcc ccaaagactc cagctgtggg 3240

acagcagagg tgccgtgacc cacggaggtc agggtacagg gggcctcaag gccagcgaca 3300

tgtgggggcc acagggagtg atgaacacag aagggacagg gagctgggca catgggccag 3360

ctcccccgcc cctgcctggg acgctgttac atttttaatg tcccagcagt gctgacaaat 3420

ggagcaggaa ggggaggaga agaggggacc cagcaagatg ggccaggctg tgagaaggtg 3480

tgtcagaaat atggggagac agggcactgg agacccagga ggcagggaga ctccacagtc 3540

agagacagaa acagagacgt agaccagaag gcttgtgaga agagaacccc aggaatcaag 3600

gcagagagcc aggttccaga cacagggaga cccagacgga gaaagagacc ggagatgtca 3660

gacatacaga ggaaagagga agagagtgac taacggggac agggtatctt ctcaggctaa 3720

tgaaaatgtt ctataattgc tcgggtgatg gatggctaac tctgaatata caaaagccac 3780

tgaactgtac actttaaatg ggtagattgt atggtatggg aataatgtct caataaagct 3840

gttacaaaga gacagagaat ataagaggca cagagcagag accagagact ctgggggaca 3900

cagataagag tgacagaggc caggcacatt ggctcatacc agtaattcca gcactttggg 3960

aggctgacgc aggagaatcg tttgaaccga gttcaaggct gcagtgagcc ctgattgcac 4020

tactgcactc cagcctgggc cacagagcaa gaccctgtct caaaagagaa aagagtaaca 4080

gagaccccaa gagagaccct gggaattgga gacacagagg gacagggacc ctaagaacta 4140

gacacagaga gagatagaag aggccccgga agacacagat aagagacgct aagagacagc 4200

agggatacca ttaaaagact ttcaagaagc caggcatggt gactcatgcc tataatccta 4260

gcattctggg aggtccaggc aggtgtatca cttgagctca ggagttcgag accagcctgg 4320

gcaacacggc gaaaccctgt ctctgcaaaa aatttaaaaa ttagccaaag gtggtggtgt 4380

gtgcctatag tcccagctac ttggaaggct gaagtgggag gatcacatga gccaggaagg 4440

ttgaggctgc agtgagctga gatcacacca cagtactcca ccctgggcaa caaagcaaga 4500

tcctatctca aaaaaacaaa acaaaacaca cggattagag gaggccaggg ggcaggtgca 4560

gaaatccagg cacagacaga cagccctaca tcctggctgc tgcagccgct gctcactccc 4620

tgaaccagct cctggatttc cgctgtgggg gttgggcact gtgattaata caattaatta 4680

actgacaagg tgactgaggc agcagctgag gccagtactc aaagcctctg gccttcaagg 4740

gccttctcta gtcccgacgt gccttgccat gggaggctgg gtcgctgtgc tttatagatc 4800

tgcgggggaa gccaggccct ggggagccag ccaggcagga gtccaggagc ccagctcctc 4860

ctcccctaga cccaggtgtc cagcttcagg ccccgcctcc ctcagaccca gaagcccagg 4920

ccccagcctc tcctccctca gacccaggag tccaggcccc agccctcttc cctcagaccc 4980

aggagtccag gcccccatcc ctaatccctc agacctggga gttcaggccc ccatccctgc 5040

tctctcagac ccaggagtcc aggccaccag tgcttcctcc ctcagaccca ggagtccagg 5100

cccccatcct tcccctctca gacccaggag tccaggccct tagtccctcc tccctcagac 5160

ccaggggtcc agcctcagtc ccctgctccc tcagacccag gagcccaggc cccctgctct 5220

tcctccctca aacctgggag tctaggcctc cagctcctcc tccctcagac ccaggaatcc 5280

aggcccccag ccccctcctt tctcagaccc aggagtccaa gcccccaacc tctcctccag 5340

ggttccatag gtcactctgc accctcctcg ctgccttggt ctacccatcc ctggatctcg 5400

gggtctcaag ctgtgtctgt ccccattgtt ctcccatggg ctcacactac accctgggca 5460

gcctccactc tgccaccaca ggaagcctgc aggtgcagtc cacagggaga ggaagtgtgt 5520

ctggggaccc agctcacact tcatgaagct gtcaccagtc tggcttgccc agtgacactg 5580

agggaggctg gtgttcccat ggccacagtc cctggctatg gtgctggcc atgtcctggc 5640

caagttcatg gcacagcccc tgcctagcag tgtgctggat gatgggggct cactccaggc 5700

tgaccaacct cctggctgta tctcccttgc ctccacctga catgcagagc gtctgggcag 5760

cggccctcac ccctgtcccc tttacctccg cagccctgtc agcctccaag tcccaaccct 5820

cccagcttct ccctgtcacc cccaccgctg gtcctgcttt cccattccaa gttctgagtg 5880

tgtttccaat ctcgccatcc tccgtcccca cagccccgac cctgatcccg gcgcctcccc 5940

ccgagacccc tgccccactg agggctcttt ctttttcttt tttttctttt ttttgagaca 6000

agagtctcac tctgttgccc aagctggcaa tagtgtctca ctctctcact ctgtcttcca 6060

agctggtaca gtggtgtgat cttggctcac tgcaacctcc gcctcccagg ttcaagcaat 6120

tcttgtgcct cagcctccca agtagtgggg attacaggca ggtgccacca tgcccagcta 6180

atttttgtat ttttagtaga gacgggctct caccgtgttg cccaggctgg tctcctgagc 6240

tcaggtgagc cactgcaccc agcctgtgag ggttttttct ttctcttttt gttttttttg 6300

tttttttttt gagacatggt agccaccacc tgtaatccca gctactcagg aggctgaggc 6360

aggagcccga gacagagcaa gactccgtct caaaaaaaaa aaaaaacaga aaagacttgg 6420

gcttgtttcc tgagggctct gtgcagagga gggcgtgttc tgcactttct agcaggatcc 6480

ttctagctgt gcataggaac aaaccacaag gacaactgca gtcctccagg tgggaagtgg 6540

cagtggatta gaccagggca gggactgggg aggcgtcaga ggtggtctaa ctagataaat 6600

gtcaaagaag gaggtgacag ggtttcctga tagattgctt atgaggtgag agagaaagag 6660

gtgaaaatgc cttaaaaatg gactgtaggc aggacgcggt ggctcacgcc tgtaatccaa 6720

gcactttggg aggccaaggc aagaggatcg ctttagttca ggagtttgag aacagcctag 6780

gcaacaaaca gtgagacccc ttctctacaa aaaaaaattt tttttaatta gtcaagtatg 6840

gtggtgcccg cctgtagtcc caactactga gaaggctgag gtggtaggat tgcttgaacc 6900

caagaggttg aggctgcggc aagctgggat cctgcactcc accctgagca acggagtgag 6960

atcctatccc agaaaaaaaa aaaaggaatg ttaaccccac agggcctgaa ctatcctatt 7020

cattcatttg gaattagaaa ttcagaatgg ggaacttaca gcaatgggat tttttttttc 7080

agacggagtc tccctctgtc cccccagctg gagtgcagtg gcgcgatctc agctcactgc 7140

aagctctgcc tcccagattc acgccattct cctgcctcag cctcccgagt agctgggact 7200

acaggggcct gccaccacgc ccagctaatt tttatatttt taatagagac gtggtttcac 7260

cgtgttagcc aggatggtct caatctcctg acctcgtgat ctgcctgcct tggcctccca 7320

aagtgctggg tttacaggcg tgagccacca cagctggccc tttttttttt tgagatggag 7380

tctcactctg tcccccaggc tggagtgcag tgctgcaatc tcagctcact acaacctccg 7440

cctgctaggt tcaagtgatt ctcctgcctc agcctcctga atagctggga ttacgggggc 7500

ccgccaccac gcccggctaa tttttgtatt tttagtagag acggggtttc accatgttgg 7560

ccaggctggt ctcaaactcc tgacctcgtg atccgccctc ctcggcctcc caaagtgttg 7620

gaattacagg cgtgagccat tgcgcccagg gattttagaa cctcattcat tcgttattca 7680

gcaagtactg agcatctact aggtaccaat ctctcttgca gatactgagg gtacaggttg 7740

tgagcaaaac agacagaaga tccctgccct cgtggcgctg gcagtcagtt tccaggagga 7800

tgtgatatat ctagtgtgtg agatggtgag aagcactatg cagacagaga acgtaacagg 7860

gcaaaagggc ggaagagcag tcaaccaggg aggggagagg aggtggcagc tgaggtggag 7920

tatctcgggg taaaagaacc ccccgaggag tgtgccccac aagaagctaa aagtggtggg 7980

tttgtttgag accgagtcct cactctgtca cccaggctgg agtgcagtgg cgcaatctca 8040

gctcactgca acctccacct cctgagttca agcgactctc cctaaagtag ctggaattac 8100

aggcatgcag ctaatttttg tatatttagt agagacaggg tttcactgtg tcggccaggc 8160

tggtctcgaa ctcctggcct caagtgatcc atccaccttg gtctcccaaa gttctgggat 8220

tacaggcttg agccaccgcc ccatcctaaa gtcggttttt ttgttttaat cctccaaatc 8280

cagtagtgct cagatgccat cctaaatgga ggattggagg ggagcctccc ttagaaggaa 8340

gatctgggcc cctggaagcc acaggactgc tccttaagtc aagaactctg aatctggcca 8400

ggtgcagtgg cttatgccta taatcccaac actttgggag gccgaggcgg gtggatcact 8460

tgaggtcagg agtccgagac tagcctggct gacatggcga aaccccatct ctactaaaat 8520

tacaaaaatt agctgggtat ggtggtacat gcctgtaatc ccagctactt gggaggctga 8580

gacaggagaa tcacttgagc tggggaggcg gaggctgtag tgagcccaga ttgcgccact 8640

gcactccagc ccgggcaaca gagtgagact ccacctcaaa aaaaattttt ttaaaaagaa 8700

ctctgagttg ccgggcgcgg tggctcacgc ctgtaatccc agcactttgg gagtcccagg 8760

cgggcggatc acgaggtcag gagatcgaga ccatcctggt taacatggtg aaaccccatc 8820

tctactaaaa aaattagccg ggcgtcgtgg cgggcacctg tagtcccagc tactcgggag 8880

gctgaggcag gagaatgggg tgaacccggg aggcggaggt tgcagtgagc cgagatcgcg 8940

ctgctgcact ccagcctggg tgacagagca agactccgtc tcaaaaaaaa aaaaaagaac 9000

tctgagtcag cctggcgcag tggctcacac ctgtaatctc agcactttgg gaggccgagg 9060

cgggcagatc acgaggtcag gagatcgaga ccatcctggc taacacggtg aaaccccgtc 9120

tctactaaaa atacaaaaaa ttagcagagc gtggtggcgg gcgcctgtag tcccagctac 9180

tcgggaggct gaggcaggag aatggcatga acctgggagg cggaggttgc agtgagccga 9240

gatagtggca ctgcactcca gcctgggcga aagagtgaga atctgtctca aaaaaaaaag 9300

aaaaaaactc tgaatcccag agacccaggg acttggagga ggtgaagggt gtggggctcc 9360

agggacctgg ggctcagctt ttcaggtctc ctcctccagg aagctcttct ttcgttcatc 9420

gtccctccct ccttcagaca ggtcaagcct cttcaggttc ctcccatacc acccctactc 9480

cgtcttccag gcattcttgg tgataattat ttgtgcaata tttgtacccc ctgcaatctg 9540
gatgtcaacc ctggaaaaca agcacctgat ttgtcctgtt acttctgcag catccacact 9600
tagcaacatt cattcacttg gcagtgttta ctgagcacct actacatgct aggccctgtt 9660
gtaggtgctg gggatacagt aggggaaaaa cacagacaaa cctcagggct ggagagggca 9720
gaccctctag cacccaggac ctgtttgttg agtaactatt tgttttttgg atttgaccta 9780
taagatgtga aagaacagcc agattcttcc aggttctgcc ttttcctgat ccctccctct 9840
ctccctacta ccaaatccct tccattttaa aattttaaat cctggtgggg cacagtggct 9900
cacgcctata atcccagcag tttgggaggc taaggtaggc agatcacctg aggtcgggag 9960
ttcgaggcca gcctggccaa gatggtgaaa ccctgtctct actcaaaata caaaaattag 10020
ctgagtgtgg tgacatatgc ctgtaatccc agctactcgg gaggttgagg cagaattgct 10080
tgaacccaga ggcagaggtt gcagggagcc aagatggcgc cactacactc tggcctgggt 10140
gatacagcga gaatgtgttt caaaaaaata aaatcctaag acctattagc ctcctcccca 10200
acactcagtc tcagtcttaa cctcttgttc ttttcattgc caactttttg tgtcattttg 10260
ctgtctggtc atctatctct gctcagccca ccaccttccc ctcaggcctc cttggatgaa 10320
gttaagtcat tctctatttg aagaacccgc aacggctccc acttgccaca aagaggaaac 10380
taacttcctc ggccccagcc tactttgctg tctctgcagt tcacacaggc ccaaatggct 10440
ttccttgacc ctcattgagt ggccttcccc ccttgcctag ctaggaaggg gccagagcgc 10500
ttaaccaccc tgatcccaac cttagtgcct ttgggggcca ggcgggtgat aagtcccagc 10560
acctggggaa tctggaggag ccggaaggga gtgtgccttg tgggaggggg gtgatgtcct 10620
tcctcagccc cacccagctg ttgcccccag gagccacata tccggacttc tgtctggagc 10680
tttagacagc aatctccagg ccttctgtgt gggctgctac ttcccaactg aaataacact 10740
ctgtgggcca aagcaaacat gcaccacagt cagatctggc ctgcagcagg cggctgaccc 10800
ctgctgctct gctcacctgg gccacgcccc gtgaacatcc cccatgagca agtccttccc 10860
tggaactggc acaaggaccc ctggagggtg gctgaaacat ccccatttta cagatggagg 10920
aagttgggct tggagacaag gctgccagct ggaaagggtg tcagcgttaa ataacaagat 10980
tcagaaaata ggattaagta tagagtttat tcaagcctaa agcttgagga tggccacccc 11040
ggagtgtgga ttcaagttgc cccaaataaa ccctttgact agctgtgatt acaagtggac 11100
tttttttttt tttttttgag acagggtctc accctgtcac tcagtctgga gtgcagtgtg 11160
tgatcatggc tcactgcagc gtcagtttct cgggctcctg gccttgagtg attctcctgc 11220
cacagcctcc ctagtagctg ggactacagg ccctagccat cttgcccagc tatttttttt 11280
ttttgagacg gagtcttgct ctattgccca ggctggagtg tagtggcaca atctctgctc 11340

actgcaacct ccacctcccg ggttcaagca atcctcctgc ctcagccccc ctagcagcta 11400
ggattacagg cacctgccac catgcccggg taatgtttat ttttttagta gagatgggat 11460
ttcgccatgc tggccaggct ggtctcgaac tcctgacctc aggtgatcca cctgctttgg 11520
cctcccaaag tgctgggatt acaggcgtga gccatcgtgc cgggtttttg tttttgtttt 11580
ttttttttta agagatgaag tctcactata ttgtccaggc tggtctcaaa ctcctggcct 11640
caagtgatcc tccacccact tcggcctccc atagtgctgg gattacaggc atgagccaac 11700
gcacccagtc tgcaagtgga tttttaagga acaaagaaga gagagttcct aaattgttac 11760
caagaattta cattgaaaca gcataaggta tctgcaggtc tggctcagaa aaaaagataa 11820
cagaaggtat tgattggcta tgcactgttt attgtatcac aaattccagg aacaagaagc 11880
taatggggag ggcagctgtg caggaaccac tgcctcgggc atgggtggag gaggcgtgtg 11940
accgaagtcc cctattcact gtacagtctc cctgggcctg atacatttcg catagctcag 12000
actgatctga gctatttttc ttttctcaag ttgggtgggg gtggcccctc tggactggaa 12060
aagcctcatt tttcccccaa gtgacacacc tgagtttgtt tgacttgttt cccccaagtg 12120
acacacctga gtttgtggga cttgtgtgga gatgatgtat ttatgtcaca cttcagtctt 12180
cgctgatggc tttttttttt atatttattt acattttttc ttttaaggta ggcagggcct 12240
cattctatcg cccatgttgg agtgcagtgg cgaggtctct gggttcaagt gattcccctt 12300
cctcagcctc ccaagtagct gggaatacag gggcccgcca ccacgcccag ctaatttttg 12360
tatttttggt agagacgggg tttcaccata ttggccaggc tggtctcgaa ctcctgacct 12420
cagggtgatc catccgcctc ggcctcccaa agtgctggga ttacaggcat gagccgcagc 12480
acccagcctt ttttctttct ttcttttctt tttgtttccc ctcccgagac cgagtctctc 12540
tctgtcaccc aggctggagt gcagtggtat gatctcagct tactgcaacc tccacctccc 12600
gggttcaagc aattctccca ccttagcctc ctgagtagct gggattacag gtgcctgcca 12660
ccacacccag ataatttttc tatttttagt agagatgggg ttttgccatg ttggccagtc 12720
tggtctcaaa ctcctggccc caagtgagcc tcctgcctcg gcctcccaaa gtgctgggat 12780
tacaggtgtg agccaccaca cctggcctta ttttattttt aatttctgtt cttgtattgc 12840
tataatttac tgatgatttc ttacccaaag atcctcggcc tgtccccacc caaggggact 12900
gccttccacc ggggacccct cataagagtt actcattagg atggtgttcc ctggggttcc 12960
tgtgtgtatg acgccagggc ctgtgcaccg cctccatggc ccatctccat cagacagagc 13020
tgggcagtgg tgttggccct cgaaatgagg cttgtgccct ggcctttcct ggcctggggg 13080
ctgagtgcca aggccacctg taattaaact gagcttccta tgtttactta gcaagcactg 13140
acctctcatt tcctggggcc ctatgctgag tgttccacag ctattcttta atctttaaac 13200
agcccttggg acgcaggcac tattattaac ctcacatcac agctggggaa actgaggcac 13260

agagaggtta agtggcctgc acaaggtcgc agagccagga ttcaaaccca tgaactatgg 13320

gccactcagt cgccatgctg tactgccgtg cacagcagag gaagatttgt tcttgttctt 13380

gtttatatta aaagtgagaa aacatgggcc acacgccgtg gctcacgcct gtaatcccag 13440

cactttggga ggccaaggcg ggtggatcac ctgaggtcag gagtttgaaa ccagcctgac 13500

caacatggtg aactctggtc tctactaaaa attcaaaaat cagccggacc gtagtggctc 13560

gcacctgtaa tcccagctac tcaggaggct gaggcaggag aatcgcttga acccaggagg 13620

cagaggttgc agtgagttga gaccgtgcca ctgcactcca acctgggtga cagagcaaga 13680

ctccgtctcc aaaaaaaaaa aaaaaaggtg agaaaccact taggctggct gagaactgca 13740

aacatgcgtc cacataaaaa cttgaacttg catgatccca gcagctttat ttatcagagc 13800

caaaaagttg aaacaacctc aatgcctatc agcggctgaa tggataaaca aaatgtggtc 13860

cgtccatgta gtggaatatt attgagcctt aaagaagagt gaagcgccga cacgtgttac 13920

agatggatga acctggaaaa caagatgcca agtgaaagaa gccagacaca aaggctacat 13980

agtctattac atgaaatgtc cagaacaggc acatcaacag agacagaaag ttgcatcgtg 14040

gctgggggaa tgggaagtga ctgcttaatg gataaggggg ttacttaggg aattataaaa 14100

atgcccaggc tgggcgcagt ggctcatgcc cgtaatccga acactttggg aggccgaggc 14160

aggtggatcg cttgagctca ggagcctaag caacatgatg aatccccatc tctacaaaaa 14220

atacaaaatt agctgggttt ggtggctcgc acctgtagtc tcagctactt ggggggctga 14280

ggtgggagga tcacttcggc cccagagacg gtttgcagtg agcagaaatc gcgccattgc 14340

acgccagcat ggacagagtg agatcctgtc tcaaaaaata ataccttaaa actgattata 14400

gtaacagatg taaaactcca aatatactta aagccactga attgtactgt attttttatt 14460

ttactttttt ataaaaaaat tatatatata tattgttttt tcttttcttt tcttttttca 14520

agacagagtc tcactttatc acccaagctg gagtgcagag gcgcgatctc ggctcactgc 14580

aacctccgcc tcctgggttc aagtgaacac gtccggctaa tttttatatt tttagtagag 14640

acagggtatt tcaccgtgtt ggccaggctg gtctcaaact cctgacctca agtggcccac 14700

ccacctcggt ctcccaaagt gctggaatta caggtgtgag ccactgcacc cagccttaat 14760

tgtatacttt aaatgggtga attgtgtagc gtaagaattt atatctcgat aaagctgttt 14820

aaaaaaaaaa aaaactacgt aaaaaatgag caaaggaccc gaagagacat ttttctaaag 14880

aagacataca ggcagccaac aaacatgaaa aaatgctcaa catcattaat catcagagaa 14940

atgcaaattt aaaccacaat gaggtgccat ctcacctcag tcagaatgtc tgttactaaa 15000

aagtctaaaa aaacagatgc tggcaaggat ttggagtaaa gggaccgctt acccactgtt 15060

gatgggaatg taaattagta cagcctctat ggcaaacagc atggagattt ctcaaagaac 15120

taaaaataga actaccattt ggtccagtat tccactgagt atcctcccaa aggaaaagac 15180

atcattatat caaaaagaca cctgcgcttg tatgtttatt gcagcactat ccacaacagc 15240

aaagatatgg aatcaactta agtgtccatc gatgaatgga ggattggata aagaaaatgt 15300

ggtctatatc tacaaagatt tcatgacgaa attgccaaaa gcagtgcagc aaaagcaaac 15360

attgacaaat gggatctaat taaactaaaa agcttctgca cagcaaaaga aactatcctc 15420

agagtgaaca gacaacctac agagtgggag aacatttttg caatctatcc atctgacaaa 15480

ggtctaacat ccagaaccta caaggaattt aaacaaattt acaagaaaag aaaaacctaa 15540

aaaagtgggc aaaggacacg aacagacact tctcaaaata agacagtcat gcagccaaca 15600

aacatgaaaa aaatgctcaa catcactgat cgttgagaaa tgcaaatcaa aacaatgaga 15660

taccatctca caccagtcaa aatggccatt atcagaaagt caagaaacaa cagatgcagg 15720

caaggttgtg gagaaatagg aatgctttta cactgttggt gggaatgtaa attagttgaa 15780

ctattatgaa gacagtatgg cgattcctca aagatctaga accagaaata ccatttgacc 15840

cagcaatccc attactggta tatacccaaa agaatataaa tcattctatt acaaagatac 15900

atgtatgcgt atgttcattg caggactatt cacaatacca aagacatgga ctcaatccaa 15960

atgcccatca tggatagact ggataaagaa aatgtacagg ccgggcactg tggctgacgc 16020

ctgtaatcct agcactttga gaggccgagg cggcggatca cgaggtcagg agatcgagac 16080

cattctgggt aacacggtga aaccccgtct ctactaaaaa tacaaaaaaa ttcactgtgt 16140

gtggtggcgg gcacctgtag tcccagctac tcgggaggct gaggcaggag aatggtgtga 16200

acccaggagg cagagcttgc agtgagccga gatggcacca ctgcactcag gcctgggcaa 16260

aagagtgaga ctccgtctca aaaaaaaaaa agaaaagaaa aaagaaacct gcacatgtat 16320

cctggaacaa taaaaaaaaa aaagaaagaa aatgtggtat atgtctacca tggaatacta 16380

cacagccata aaaaaggatg ggatcatgtc ttttgcacca acacggatgg aactggaggc 16440

cattatcctg agtaaaatag aaagtcaaat accacatgtt ctcacttata agtggaatct 16500

aaacagcaga tacacaaaga ttggaataat ggacagtgga gactacaaaa ggtgggaggg 16560

agggaaacag gtgagggttg aaaaattaac tgttggaggc tgggcacagt ggcgcacacc 16620

tataatccca gcactttggg aggccgacac gggtggatca cgaggtcagg cgttcgaggc 16680

cagcctggcc aacatagtga aaacccatct ctattaaaaa tattaaaaaa ttggccgggc 16740

gcggtggctc acgcctgtaa tcccagcact ttgggagccc gaggcggcag atcacgaggt 16800

caggagattg agaccatcct ggctaacatg gtgaaacccc gtctctacta aaaatacaaa 16860

aaaaaaaaaa aaaaaaatta gccgggcatg gtagtgtgcg cctgtaatcc cagctactcg 16920

ggaggctgag gcaggagaat tgcttgaaat tgggaggtgg aggttgcagt gagccgagat 16980

cacaccactg cactccggcc tgggcaacag tgcgagagtc tgtctcaaaa aaaagaaaaa 17040

ttgactgttg gaagccaggt gcattggtgc atgcctataa tcccagcact ttgggaggct 17100

gaggcgggct gctcactttg agttcaggag tttgagacca gcctgggcaa catggcaaaa 17160

ccctatctct acaaaaacaa aaattagccg ggcactggtg gcacatgcct gcagtcccag 17220

ccactcaagt tgcagtgagc caagattgtg ccactgcact tcagcctggg agacagagtg 17280

agaccctgtc tcaaaaaaga aaaagaaaaa ttaactgttg ggtacgctgt ttgctattca 17340

ggtgatagat atgctaaaag cccagacttc acctctatgc aatatatgta tgtaagaaac 17400

tggcacttgc acctgctaaa aatataataa ttttaaaatt tttattttaa taaaaaaata 17460

gaaacttagg gccaaggggg gagctgatca tttgaggtca ggagttcgag accagcctgg 17520

ccaacgtggt gaaagcccat ctctactaaa aatacaaaaa ttagccaggc atggtggcgg 17580

gcgcctgtaa tcccagctac tcaggaggct gaggcacaag aatcccttga accagggatg 17640

gaggttgcag tgaccaagat tgctccactg caccccagcc tgggcaacag agcgagactc 17700

agtctcaaaa aaaaaaaaga aacttgagaa aaaatgctat gtaggaggtc tgtaggtttc 17760

ataggaaggt gagagaaagt atattactga ggggggatga agacaatact cagcctttaa 17820

atgttttaaa atgtatttta tgaaagtttt tttttattga tttattttca gaggattccc 17880

cccccccaaa aaaaaatctt atgaaaattt tggggtgggc tgggtacagt ggctcacgcc 17940

tataatccca gcactttgag aggctgaggt gggaggatca cttgagtcca ggagttcaag 18000

accagcctgg gcaacatagc gagacattgt ctctaccaaa acaaacaaac aaaaaataca 18060

aattagccag gtgtggtggt acatgcctgt agtcccagct tgaacctggg aggtcaaggc 18120

tgcagtgagc tgggtggccc actgcactct agcctgggtg aaagtgaggc cctgtctgag 18180

aaaaaaaatg tagttgaaag aaaagaaaac ttttattttt ttattttctt ttttaatttt 18240

aaaggcaggg tctcactctg ttgcccaggc tgttcttgaa ctcctaggct caagcattcc 18300

tcccaccttg gcctcccaaa gtgctgagat tgaagtcttg agccactacc ctggccatca 18360

tgtcaacttt taaacataca aatacaggta agaatggcct ggtggccggg cgcggtggct 18420

cacgcctgta atcccagcac tttgggaggc tgaggcaagc ggatcacgag gtcaggagat 18480

tgagaccatc ctggctaaca cagtgaaacc ccatctctac taaaaataca aaaaagaaaa 18540

aaattagcca ggcatggtgg caggcgcctg tagtcccagg tactcaggag gctgaggcag 18600

gagaatggcg tgaacccagg aggcggagct tgcagtgagc cgagatcgcg ccactgcact 18660

ccagcctggt caacagagca agactccgtc tcaaaaaaaa aaagaagaat ggcctggagc 18720

ctccctcacc ttctccacct agacccactt gacaatgggt gacatatttt ggtttttgct 18780

gaaccattta ggagtaactt gagatcatga cattgcgact tcatatcttc tgggaaaaat 18840

agtgtgtgca cagccacaat ttaattatca cacctcggaa catgaacagc aattccaaaa 18900

tcatatgtaa tatgaactca tagtacacgt tatagttgta taatagaata gtcattttag 18960

tcttccctaa aatgtctttt ccaggtggct tttttggaat caggatgctg cgaagctgcc 19020

tacgttgcat gcggagcctg catttcccgt ggggccccct tcgtggtggc cctgcctgga 19080

ggtgtctggg tccctccccc acccgactac ttcactctct gtcctctctg cccaggagcc 19140

caggatgtgc gagttcaagt gctacccgag gtgcgaggcc agctcggggg caccgtggag 19200

ctgccgtgcc acctgctgcc acctgttcct ggactgtaca tctccctggt gacctggcag 19260

cgcccagatg cacctgcgaa ccaccagaat gtggccgcct tccaccctaa gatgggtccc 19320

agcttcccca gcccgaagcc tggcagcgag cggctgtcct tcgtctctgc caagcagagc 19380

actgggcaag acacagaggc agagctccag gacgccacgc tggccctcca cgggctcacg 19440

gtggaggacg agggcaacta cacttgcgag tttgccacct tccccaaggg gtccgtccga 19500

gggatgacct ggctcagagt cataggtgag cagggtaagg gcgggaggca aggaggtggg 19560

agggccgcgg tgtgggagca tccccttgcg ggtcagtttc tctcttggct tcagctgtga 19620

ggttcacatt ctctgtgggt ttccatccat cagcaaatgt tcattaagca cctaccgcgt 19680

gccagatgct tttctgtgtg ctgaggatgt ggcaggcaac caggcattct tgggaaaaac 19740

aaaaaaataa gtattggctg ggcgcagtgg cccacccctg taatcctagc actttggaag 19800

gccgagatgg gcagatcgcc tgaggtcagg agttcaagac cagcctggcc aacatggtga 19860

aaccctgtct ctacgaaaaa taaaaaatta gctgggtgtg gtggcccgtg cctataatcc 19920

cagatactca ggaggctgag tcaggagaat cgcttgaacc caggaggcag aggttgcagt 19980

gagccgagat tgtgccacca ctgcactcta gtctgggtga cagagcaaga ctcggtctaa 20040

aaaaaaaaaa agtattgagg ttgggtgtgg tggctcacga ctataatcca agcactttgg 20100

gaggctgagg caggtagatt gcttgagccc atggtggtgc gcagatgtgg tcccagctac 20160

ttgggaggct gaggtgggag gatcgcttga gaccaggaat ttgaggctgc ggtgagctgt 20220

gatcatgcca ctacactcta gcctgggtga cagagtgagg ccctatctct aataaataaa 20280

taaaagagta agtcaggcag ttaaaggggc catggaagtt ggagagggcc aagttgcaat 20340

tctagctaag gaggtctggg gatatttagg cagaaactag gagaggagga gccctctggg 20400

tatctaaggg aagagcgttc caggcagacg gaatggcaag tacaaaggcc ctgagacagg 20460

aatacctgga gtatttgagg gccaggaagg aagtcagggt ggctggagac atatgagtgg 20520

gggaaagtag gaagcaataa agtcaaggag aggatgggga cagattggca aaagccttgt 20580

ggactataag aagcctttgg cttttgccca gggaggataa gagctgtagt aggttcttag 20640

cagcagacag gtttgatctg gcttgggttc tcaccagctc ctcctggctg tgttgggagg 20700

ggaaagaatg ggagctgaga aaccacaggg taaggcaact acaaagtccg ggtggggccg 20760

ggtgctgtgg ctcacgccca taaccccagc actcagggag gccgaggtgg gaggatccct 20820

tgagcctagg aatttgagac catcctaggc aacatagtaa gactcttgtc tcaatgacaa 20880

aaaaaatttt tttttttttt gagacagagt tttgctcttg ttacccaggc tgtagtggag 20940

tggcgcgatc tcggctcact gcaacctccg cctcccaggt tcaaccaatt ctcctgcctc 21000

agcctcccaa gtagctggga ttacaggctc ccaccaccac acctagctaa atttttttgt 21060

atttttagta gagacggggt tgcaccatgt tggccaggct ggtcttgaac tcccgacctc 21120

aggtgatccg tccacctggg cctctcaaag tgctgggatt ataggcatga gccactgagc 21180

ctggccagga aagaaaaaaa aattgtttta attaaccagg ctcagtgact tacctgtaat 21240

ttcagctact cgagagactg aggtgggagg atcacctgag cccaggaggt tgaggctgca 21300

gtgagctgtg attgtgctac tgcactccat cctggacaag agagcgagac cctgtcttaa 21360

aacaagtcca gtccgggcag gaagtaaaac ggcttagaag taggggacat agtggcctca 21420

ttgagatagg atgtgcaggg aaggcgtctt agtcaagtcc tggagtctga gaaggagcta 21480

cctttgggca tagcagaggg gcacgggcac agaaacctgg gaggtgggca tgagatggga 21540

gctgggcgcg ttccaggaat ggaaaggggg ctggtgagga taggcaaggg cttgggtgac 21600

cctcagcggg gccacattac acgggcttca caggtggagg cgaggaggct ggactatgcc 21660

ctcagcatgg ttaagagcca tgggaggtgt caagccacag gagagatctg gacaaacgtt 21720

tgaggttgca aaaaatgacc ggcagcagta tgaggagtga ttggaggagt ggacggaggt 21780

agaagggagc tgggacgaga ggggagaaga ggcattgctg ggaagacgga tgaggcagtg 21840

gggggcagag ggaagcgggc ggcatatagt ggtgttttta gaagatagta ggtgctgggc 21900

ttgcccatgg attgaggggg aagcaggatc agagaggagt cccaggcctg gtgcggtggc 21960

tcacgcctgt aatcccagca ctttaggagg ccaaggtggg cagatcgctt tgaggtcagg 22020

agtttcagac cactctgggc aacatggtga ggccccatct ctacaagaaa aaaaaaaatt 22080

agctgggcat ggtagcacac acctgtagtc ccagctaccc aagaggctga ggtgggagga 22140

tcgcttgggc ctgggaagca gagattgcaa gtgagccaag attgcaccac tgcactccag 22200

cacgagaccc tgtctcaaaa aaaaaaaaaa aaaaatagga ctcccagcgg gaaggtgact 22260

gatacttggc aaaaatagag aaggcaggga agattttttt tgaaggatgc ttttttttct 22320

tttccttttt cttttttttt tttttctttt ttttgagaca aagtctcact acattgtcca 22380

ggctagactc gaactcctgg gctcagggga tcctcctacc tccgcctcct gcatagctgg 22440

gattacaggc atgagtcacc acatccagct acgaatgctc tttctttaaa tcttgaggct 22500

gggcgtggtg gctcactcct gtaatcccag cactttggga ggccgaggcg ggcggatcac 22560

gaggtcagga aatcgagacc atcctggcta acacggtgaa accccatctc tactaaaaat 22620

acaagaaatt agccaggcgt ggtggcgggt gccttagtcc cagctactcg ggagattgag 22680

gcaggagaat ggcttgaacc cgggaggcgg agcttgcagt gagccgagat cgcgccacta 22740

tactccagcc tgggcgacag agtgagactc tgtctggaaa aaaaaaaaaa tatgttggaa 22800

gtcccctggg agcccacctt ccctaggcaa cttcccagaa gccacgtcac tctggatgcc 22860

ttttcttccc ccttctcagc ccagagcgtc tgagaaggtc accctgtgct atccttgagt 22920

aaacacttcc ctttccagta acacatgaaa ccacagccgt tttccataat acaaaggaca 22980

gctcttctcc tccccaccct cacaaacgcc tctcttcttg tgcaatcatt tgttctcatc 23040

cagagataac ttcagggctg ggcacggtgg ctcacacctg taatcccagc actttgggag 23100

gctgagaggc aacaagagca aaactctgtc tcaaaataaa taaataaata atgagactca 23160

gaaaatatga ttaaggccca ggcgcagtgg ctcacgcctg taatcccagc actttgggag 23220

gccgaggcag gcggatcacg aggtcaggag attgagacca tcctggctaa cacggtgaaa 23280

ccctgtctct actaaaaata caaaaaaatt agccgggcgt ggtggcgggt gcctgtagtc 23340

ccagctactt gggaggctga ggcaggagaa tggcatgaac ccaggaggcg gagcttgcag 23400

tgagccgaga tcgcatcact gcactccagc ctgggcgaca gagcgagact ccatctcaaa 23460

aaaaaaaaaa aaaagaaaag aaaatatgat taagtgtagg gtttaatcaa gcccaaagct 23520

tgaggatgac cacctgggag catagattga agttgccctg aatatacact tcaagcaggg 23580

gccaaggcaa gcagctcttg caagtgagct tttgtttgtt tgtttgtttc ttggagagag 23640

ggtctccaaa actacaaata aaaattagct aggaatggtg gcgcacacct gtaatcccag 23700

ctactcagga ggctaaggca ggcgaattgc tgaaaccagc aagggcaggt tgcagtaagc 23760

tgagattgca ccactgcact gcatcctggg tgacagcaag actctgtctc aaattaataa 23820

aaaaaaaaga gagagagaga gataacctca agagcttccg tatacacact ggtgcagaag 23880

taaggaacaa aacagccaaa gcctctgtct tcatgatgcg tgagccacca aggaatagtt 23940

ggccagagtc ggcggtcagg aaagtgccct tgacgaagtg gggcttggag tggggtctgg 24000

agaaggaggc caaagcaatc caggcacaaa atcaccaacc aaaaccccaa agtgggtgga 24060

ggcagagact gaggagggcg tggatgggga gcatgtggag tggccagggg aggtggccat 24120

ggggaagtgg tgggctcggg ccctgagtca ctattctaac agcttggagg gcggccagga 24180

ggatgtgggg aggcttgttg gatgctccac gatggtcagg ggagaaggcg gtgggaaggg 24240

agagctggga agaagaggag agtcagaccg tgttgagaag ttcagaggtg gatacccagg 24300

tccagaaagc aacagaagat gtgagtctgg gtgggggaca cacatcataa gcatgagctg 24360

ctattcttcc tggagttttg tttttgtgat ttttttccat gttttccaag agaatgaggt 24420

ggaaagggta taaaaaaaag aaacggcttt gagattttct gcctttatca cacttccagt 24480

cattcaatca tctaatggtg attacaggaa aaggaacagg gggcctttcc agctggacct 24540

ttgaaccaca gtactcagtt actcgctcgg ccttgatttg cttttttttt ttcctttttt 24600

ttttttttct tttggagaca gagtctcact gtcacccagg ctggagtgca gtggcacgat 24660

ctcggctcac tacaacctct gcctcccagg ttcaagcaat tctcttacct cagcctcccg 24720

agtacctggg attacaggca cctgccacca cgcccagcta atttttgcat ttttagtaga 24780

gatggggttt caccacgttg gccacgctag tctagaactc ctgacctcag gtgatctgcc 24840

cacctgggcc tcccaaagtg ctgggattac aggcatgagc cactgcgccc atcctaattt 24900

ttgtattttc agtagagatg agttttcacc atgttggcca gactggtctc aaattcctgg 24960

cctcaagtga tccaccctcc tcagcctcct aaagtgctgg tattgcaggt gtgaaccacc 25020

acgcccgacc agcttttttt taaatctcag cctcctctag gcttttggac cctgtctctg 25080

catctctggc agggcgaaga gacagtatac cgaaatggcc aggccaggcg cctgtaacct 25140

cagagctttg gggagccgag gtgggaagat ggcttgtgct caggagtttg agatcagcct 25200

gggcaacata gcaagaactc atctctacaa aaaaatttaa aaattagccg ggcatggtgg 25260

tgcatgccta tggtcctagc tactctggag gccaaggcgg gaggatcact tgagcctagg 25320

aggtgcaggc tgcagcaagc tatgatcaca ccactgcact cgagaccagg tgacagagcg 25380

ggcccctgtc tctaaaaaat aaataaagta aaataaaatg ggcaacactt ggatagggca 25440

gtgcaggcca gacattaccc taagccattt acctatcaat gtattattta attcacacag 25500

caatcctagg ggtgggtact ttcattatcc ccattttaca aaacaagaaa cagctacaga 25560

gttgcaaaac cacttatccc aagccaggcc gctgataagc agcagagctg ggattggaac 25620

cccggcagtt agggcctaac caccctgctc ctctgctgag tgtttgttga atgactgccg 25680

gtgaggagtg acgcaccccc tctcctcctc tccccagcca agcccaagaa ccaagctgag 25740

gcccagaagg tcacgttcag ccaggaccct acgacagtgg ccctctgcat ctccaaagag 25800

ggccgcccac ctgcccggat ctcctggctc tcatccctgg actgggaagc caaagagact 25860

caggtgtcag ggaccctggc cggaactgtc actgtcacca gccgcttcac cttggtgccc 25920

tcgggccgag cagatggtgt cacggtcacc tgcaaagtgg agcatgagag cttcgaggaa 25980

ccagccctga tacctgtgac cctctctgta cgctgtgagt gtatcggggg tgacccctcc 26040

cccatcaaaa ctgcctacac tggcttctct aaagcactgt ctacattgtc tctgaatgtt 26100

gtctacgtgg gttccctgaa gccaaattct gcactacctt cctgccattg tctacactgg 26160

ctcccatggg caaatctaca tacttccccc tcattgtcta cattggcttt actgggttcc 26220

tgcatgctgt gcctagtctg tggtaacctg ggatgggctt gcaagagcca gtttgataca 26280

tttctttcca attccacatc caatgaagtc ttctcggtag cttgaaatca accttgggga 26340

atatttacac caaggaaatt ggcaaaccct agaaatgggg gcttctttta ttccccagag 26400

agttggttta ccagcaaaga gctgccccca acagtgtctt ttctggctcc catggacccc 26460

ggttgacact gcccctggac cgttagatca atcatgctgg gccatgtcca catgtctaca 26520

tgtctacact gcccaaccta acatcgtctc cacaccttcc catacttctg ccttgacttc 26580

cctaggtgcc atctatactg agtctcccag agtctctaca ccgacccctg atccttgata 26640

ttatttacat tgtatttatt atttattatt tattattata tttttatata ttatatatta 26700

tataaaatat ataaattata tattatatat taacatatat tttgtcatta tttattattg 26760

ttatttaata taatataatt acattatatt atatattaca ttacaggctc caccccccgg 26820

gccctgttga tcctgacccc tcccactggg ttttttgcta gctcttccag aaactgccta 26880

tactcacacc tcgtaatgtt acccagagag gccaccctgg cactcttcac acagaatccc 26940

tcaaatactg gcttaaccac ctcttccacc actgtccaaa atgaccacat atcccaactc 27000

aatgttgtct acactagctc cccaggcaat gcctccactt acacagattc cctaagccaa 27060

gtctacacca gctcccccag gcaacatgca tcacgtgcgc ccataagtgc aacagctccc 27120

ctgagcctcg tctgtgccac tttcctcccg ggacctacac tggttgccca aaccctgttt 27180

tgacatcctc cctccatgcc aaaaacacac acacaggccg ggtgcggtgg ctcacacctg 27240

caatcccagc actttgggag gccaaggcgg gcagatcact tgaggtcagg agttcgggac 27300

tagcctgggc aacatggtga aaccctgtct gtactaaaaa taccaaaatt agctggacgt 27360

ggtggtacat gcctgtaatc ccagccattt gggaggctga ggcggaggga tggcttgagc 27420

ccaggacgtg gagtttgcag taaaccagga tcaccccact gctctccatc ctgggcaaca 27480

gagcaagatc ctgtctcaaa aacacacaca cacacacaca cacattattt gtagtggctc 27540

ccttaggcac tgccagcact gaacaataac tcccacaaaa ttatccacag tggtttccct 27600

gaacagtgtc tacactgaca tcagcacaca acagcctgga ctgatttgcc ccagacagtt 27660

ttgactgctg tacctcctgc caacccgccc cgtttcttta gcattcctgt ctatctgcta 27720

acttgtccac ccgctctggg attctcctcc ttgctgatcc agtcccccca tttcccccag 27780

accctcctga agtgtccatc tccggctatg atgacaactg gtacctcggc cgtactgatg 27840

ccaccctgag ctgtgacgtc cgcagcaacc cagagcccac gggctatgac tggagcacgt 27900

gagtcacgtg gtctcagaac cctgggtctg agggaggcgg ggctgggggc ctggactcct 27960

ggatctaagg gaggaggggc tgggggcctg gactcctgga tctgagggag gaggggctgg 28020

gcctaaattc ctaagtcctc caggatgaag ggagcttgca ttttggcaat ttggggtctc 28080

cgggagtact taggtccctg gagcttggct cctggtgggt gtatctttag ggatgaggcc 28140

tgtgctcccc tctcgacctt ggtatccctc tcacctgacc ccacacccct ccaggacctc 28200

aggcaccttc ccgacctccg cagtggccca gggctcccag ctggtcatcc acgcagtgga 28260

cagtctgttc aataccacct tcgtctgcac agtcaccaat gccgtgggca tgggccgcgc 28320

tgagcaggtc atctttgtcc gaggtgagtg ggtcttgggg gccgtgtgtg gagacctggg 28380

tccgctcccc tggagttctg cccttcagga cttggggctg cactggggga ggctgcgccg 28440

ccgacctggg agggatgcag acctgcctgg ctgaggggag atgagggttg gccttcccct 28500

cgtggcctca gactggggtc tggatttggg gtgtcggggt aggtgaaggc agcagagttg 28560

gggggttgag gactttgctc ggggttccta gaaagtagag gcctgggtag ggtcctcacg 28620

aatagacccg aggaagctgc cctgggctcc agctcctgag aaagacgcac acctagagtc 28680

aggcatgcaa acttcaaaga gacagaaacc aagagctccc acccccagaa acacaatccg 28740

gccccaaggc acggagccaa agaggaaaag cacaaagcca gaccatcacc cagtgatgga 28800

aactgggtca aactcacaac tgcagggcca cacacctaga gcgcggccgg gactgttaac 28860

agagccatgc ttcctgggac agagagaccc ctgggtcctg gagatagggc cagagacccc 28920

tcctgtcaga gccacagctg gcaggatcca cagccacaga cactccctgt gctgagaaat 28980

tctgttgcag agatgcatca gaatccagac actcacctgg atggtaaccc tgcacgctca 29040

aacccattgt caccaagaca cacacccagg aggacagtgg caccagccaa gatagactga 29100

acgaagccag atgacacctg gaaagggaca ttcttttttt tttttttgga gatggagtct 29160

cgctctgtcg cccaggctga agtgtagtgg cgcaatctca gctcaccgca acctccgcct 29220

cccgggttca agcaattatc ctgcctcagc ctcctgagta gctgggacta tatgcgctca 29280

ccaccaaacc cggctaagtt ttgtattttt agtagagaca gggtttcacc atattggtca 29340

ggctggtctc gaactgctga cctcaggtga tccacccgcc tcggcctcac gaagtgctgg 29400

gattatatgc atgagccact gcgcctggcc agaaaaagac attcttagaa acacgccagg 29460

gcgacggtcc atgcagcaca cctgtgcaaa cacgcaatgt gcaggccccc cagaaacatg 29520

actgaaggtg tagggacaaa ctcccagaca ggggctgtcc ctgtcatgca gacagactag 29580

ccacggaggg acagaagcag acccccttac tgagtcataa tcttagaatg cacacacact 29640

tgcagggaca cccgagggaa aacagacacc tcttcaggaa tccagataca gccagggaga 29700

taggacaccc ctacgggaaa cactgtcaca gacagcaaac ccagagagac agctgtcccg 29760

ctctgggctc gcctggcgcg tgaggatgca cataagtccc cggaggaaga cgtcacacag 29820

acacgctggg ggcaaaacta atccaggaca gcaacggctg cacacggagg aatgtcatcc 29880

ctcactcaca gaccctcaga cggaaaatca aagacatcct gggaggagct tcttcccagg 29940

aaccctcccg aaactccccg aaactcctcc gaaattatcc agaaagtccg ttagggagat 30000

tctcattcaa caaatatgtg tttcctgttt gctaggcact cttgctgagg ttgggaatac 30060

aatggtgaat gagccacagt ccctgccctc agtagcacac ccaggggagg gtcagctcca 30120

cccgcccaga cagacaccga actggaaata cacccctaac ctctacccca gatacacctg 30180

acaagctgat cccctgccca cacacccagc aggtggctcc aaggtcagag cctgcagaac 30240

acagacgcaa tagagaaaga ctcagcgctg tggaaagaca cccccaccta cccacgacag 30300

tggccacagg gccaccccca gccaataaca ccattacaaa gacacgtgcc ttcagataat 30360

gcctgcagaa acgtgcatgc tccaagcact ctagaaatcc cgtctcaagc agtgagtgcc 30420

ccgacagcca cagccagagg agacacactc aaagctcctg gggtcactct cagagggtct 30480

ctgagccacg aacccacatc caggcacaca cagagtcgtc acagagacac agtatggagt 30540

ccacagcagc acgaacatag gatacgaacg ccgcaccaca ggacagatct atctttaggg 30600

agactcagag aactttttat ttttaataga gacagggtct cgctatgttg gccaggctgg 30660

tcttgaactc ctggcctcaa agcattcctc cagctttggc ctcccaaagt gctgggatta 30720

caggtatgag ccagcacact aagcctgatt cagagctttg aaccctagac atatgaacag 30780

actcagcttc gagttccaaa acaccaatgc cacctccaga aacagagaca agcctcccaa 30840

gataggctca acctggtcca gttagtgacc agcacaggag aaaaatctgt ttcactcctc 30900

aacccagtgt atagttagag acatatatac caccctaccc cctaccaaaa aagattttat 30960

cctccttttt ttaatgctat ttgtgagtca ctaagctgat ttgacagccc gctgggtcat 31020

gaccccctgt ttgaaaacaa cagaaacaga tgtccttcct gggtctcagc ccctcctgtg 31080

tgtcgaggtg tagaggtggg gtcaggtgtg ggtgaacccc ccacccccgt ctgtcccagt 31140

tatgccttgc atgcagggat gggggaagtg gatgtgggta ggttctggtt acattgactt 31200

ctgtccctgg cttggggccc aggagcccca tccctccctg ctcacactgc cctcctcagc 31260

acttcatggc ttcctctctc tctccgcctc cccactacct cccatccccc atccccacct 31320

gctcagcccc tgcagggagc actaaggaag tcccactgtc ccggtctgtg agaaccccat 31380

tttacacact ggggataagt ggcctagaga tgaggcagcc acggcacttg gaataaatgc 31440

tcccagccac tcaagagggc agaattatca ggaatctgtg gccctctgca gagagctttg 31500

ggatcagaca gcctcccact gaaacccagc tgtcctcccc cgagggggtg tggcccccaa 31560

gctcatagct ttgtgaggac cccacagcac attcagggag ggctgcgaag tcagatggca 31620

ccagggtcct ggccagtgac cggcggccca gatctattgc atccaaatag ccagcttcca 31680

ctcggctacg gcgatcgcca gacatgggag tttggtgttg ctagatctga tttgtccccc 31740

taagagaaac ctggattttt aagtgaaatc gcctgatttt tgaaaggttg gctagcaatt 31800

taaattttgc aaaacactaa cacctgggga ccaccggttg gcatccccct ccttgtacaa 31860

agtggtgctc aggctgtagc gaagggcgga ggggcttctg cttccccacc cccctcctcc 31920

tcctcctcca ttcttctgcc cagagtcacc ccctcattct ggtcactccc gctccctcca 31980

tccccgccta tcctgcatct cccccacccc ctcactcccc agagcgatcc tcgtgatctt 32040

gtgtctccct ttctctctct ctcacccctc cttctctctc tcctcagaaa cccccagggc 32100

ctcgccccga gatgtgggcc cgctggtgtg gggggccgtg ggggggacac tgctggtgct 32160

gctgcttctg gctggggggt ccttggcctt catcctgctg agggtgagga ggaggaggaa 32220

gagccctgga ggagcaggag gaggagccag tggcgacggg ggattctacg atccgaaagc 32280

tcaggtgttg ggaaatgggg accccgtctt ctggacacca gtagtccctg gtcccatgga 32340

accagatggc aaggatgagg aggaggagga ggaggaagag aaggcagaga aaggcctcat 32400

gttgcctcca cccccagcac tcgaggatga catggagtcc cagctggacg gctccctcat 32460

ctcacggcgg gcagtttatg tgtgacctgg acacagacag agacagagcc aggcccggcc 32520

ctcccgcccc cgacctgacc acgccggcct agggttccag actggttgga cttgttcgtc 32580

tggacgacac tggagtggaa cactgcctcc cactttcttg ggacttggag ggaggtggaa 32640

cagcacactg gacttctccc gtctctaggg ctgcatgggg agcccgggga gctgagtagt 32700

ggggatccag agaggacccc cgcccccaga gacttggttt tggctccagc cttcccctgg 32760

ccccgtgaca ctcaggagtt aataaatgcc ttggaggaaa acatcagggg tgtcttgtaa 32820

tacatcagac cccctccttg catgttgggg aaagagaggg tgggacaggg actcctaggt 32880

cccagggctg ggaagagctg atccacgccc ccggggaacg agagcctggc aggggttaga 32940

ctggggaagg ctgtggggct cggaccactt ggatccttga aagacgcatt ggatggccga 33000

gaaccgcaag gctcgtgcaa ctaggattca ggtcactgga ttggggactc agaggcctgg 33060

acatttggac tcctgggtcc caggacagag gctgcctttc tggatggtgt ggccagaccc 33120

atcagaggca ggagttctct ggactcctgg gccctcgggt ggaggtgggg gtggggacag 33180

caggggctca gccaggttcc ctgcgtcctg agagaatgag gccagagggc tgtgatccag 33240

tggagcctca gacctcaact cccccaggtt gctgggggtc tcgggagggg agggctgctc 33300

cccactccca ttcttagggg ccctgagggc atggggcagg cggggggtgc tggggaagtc 33360

ccacctctgg ccaccccgac tgtggctccc ctccttctcc atccttgccc ccagggatca 33420

ccccagggga cttttcagtg gctccctggg agtggcggca gctccctcca gctcctctca 33480

actggaaaag caaagacggg gggaggggcg gcccttggtc cttgaactcc caggctcagt 33540

aagggacagg tttttacccg cgtcacctct gctctcccaa gcctccatgc ctcctctgta 33600

acacggggca ggtgtttcca tcggcctcac aaggctgtcg tgggacccag taaggacatg 33660

cccgtgatgc cctcatgcag cctcattgac ctccacagac cccaccaagc cctgtgccag 33720

gcagtgctgg ggctgcagct gtggcctgca cagacccagt gccgtcctcc ggtgtgcacg 33780

gccataatga ccacaagaat agcagcagta tgtttaggat gaaggtgtgt gtgtggaccg 33840

aggggcagtc tctctctctc actcccccac cctctgaagc cctccagagg atgccaggct 33900

gtggaacggg agcagtggag atccaacagt ccagcgtcct ctggaagctt cctcctcatc 33960

tggagagtga ggccgatgca tgcgtgaccc ccacgggccc ttccatactt gttacttcct 34020

ctggttgttt gttgtctgta ctcacccacc caccccaccc ggacaaccgg agggcagggg 34080

cggggaattg ggtccgcctg gcacacagtg gatccttcag tgccatgctg agtcctcctt 34140

acacacccag caaacaccta ctgtgtgccg aacctcaggc acacagtcta gtatggaaat 34200

tggcaagtaa acgggtaacc actccaaaaa ctcttccatt aggaaagact tgagaaatgg 34260

aaacggaggg tgctagcaag gaaggagagg cccaggagca aacaggcttc tcgcttctcg 34320

accccttttc ctgtcttgcc tttttttttt tttttttttt tttttttttt gagacggtgt 34380

ctagctctgt tacccaggct ggagtgcagt ggtacgatct cggctcactg caccctccac 34440

ctcccaagtt caagcaattc tcctgtctca gcctcttgag taacgcaccc agcctttttt 34500

tttttaagaa gtggggtggt ctccctatat tacccagatt gggctcaaac tcctgggctc 34560

aagcgatcga tcctcccact tcagcctcca aaagtgctgg gatgacaggt gtgagtcact 34620

gtgcctggcc cctggctaat tttttttttt tttttttaag acggagtttc actcttatcc 34680

aggctggagt gcaatggcgc gatcttggct cacggcaacc tccgcttccc aggttcaagc 34740

aattctcctg cctcagcctc ctgagtagct gggattacag gcgcgcgcta gcatgcccag 34800

ctaacatttt gtatttttag tagagacagg gtttctccat gttggtcagg ctggtctcaa 34860

actcccaacc tcaggtaatc cacctgcctc ggcctcccaa agtgctggga ttacaggcgt 34920

gagccaacgc gcccggctag cccccggcta atttttaaaa ttttttctag agatgaggtc 34980

ttgtgctcgt tgccctggct ggtcttgaac aatccgcccg cctcgccctc ccaaagcgct 35040

gggattacag gcgtcagtca ctgccccaag ccctaacccc cttcctgata agtggaagga 35100

gattctagtc cccagaggtg attctggatc ccagaattct gtccccgcat tctctgtctt 35160

ctagccgccc ctggtcctgt ctcctctcct ctcatcttcc cactctgccc cgcctcattc 35220

tgccccagct acacagtcta cttgctgttc tttaaaaaca atacaaggca ggccgagcac 35280

ggcataatcc cagaacgttg ggaggccgag gcgggaggat cacttgagcc caggagttca 35340

agaccagcct gggcaacatg gcaaaacccc atctctacaa aaaaattcaa aaaaaaaaaa 35400

aaaaagctgg gcctggcggc acatgcctgt agttccagcc actccggagg gtgaggtggg 35460

aggatcgctt gagcccagga gattggggct acagttggtg gtgatcatgc cactgcactc 35520

tagcctgggt gacaaagcga gaccgtctca aaaaaaacca tagagcccca caagcctcct 35580

gcagggccgc tggctgtttg cgctgcctgc gataccccca gacactctca tgactcatcc 35640

ccggtgtctc tttcaggccc ttgctcagtt gtcaccttac agtaaggtag aggccctccc 35700

actaccttcg gggaccccac tttgcacctc ctatcacttc cgcactgtat atttttgtat 35760

cccccgtgac ttactcccat ctggcatgtg agctattgta ctggtttgct tactgttgtt 35820

ctccctgccc aaccagaatc aaagttccat gaaggtagac actttgtatg ttttgttctc 35880

cactcatctc ccatgcgggg cacacatcct cgctccttcc tcatgctcag cagtttgagt 35940

tgaatgaaag aatgtcccct gcccatggga gaggacatag acaggcaggc gatgatgtgg 36000

ggtgggatgg tcgcttggaa taaggagctg tggcttcctc ctggagaccc cctcacgctg 36060

tccctctcct tgcagagacc cccaacacag caggcgcagg ggccacaggc ggcatcatcg 36120

ggggcatcat cgccgccatc attgctactg ctgtggctgc cacgggcatc cttatctgcc 36180

ggcagcagcg gaaggagcag acgctgcagg gggcagagga ggacgaagag taagtgatgg 36240

gccctgagac ggggatggga gaggggtcga agaactgagg agtatggggt aggggtgagg 36300

gtgggagaaa attctccata atggctgtgt gaaacagaca gacctaaagg aactggggtc 36360

taaatggagt agggaggtat gggtttctct ctgctataaa gtatggcatt gaatttaaaa 36420

attagctggg ggccaggcac agtggcttac acctgccatc ccagcacttt tggaggctga 36480

ggtggggaga tcgatcgttt gagcccagga atttgagccc agtctgggta acataggaag 36540

actaccccca tctacaaaaa aaaaaaaaaa aaaaaaaaaa tggccacgca gggtggctct 36600

gaccgctccg ctctgcaaag actcctcgga gcccaggatc ctactacatt tttttttttt 36660

ttttgtgacg gagtctcact ctgtcaccga ggctggaata cagtgatgca atctcggctc 36720

actgcaagct ctgcctccca ggttaaagca attctcctgc ctcagcctcc ggagtagctg 36780

ggaatacagg cgcccaccac catgcccagc taattttttg gatttttagt agagacaggg 36840

tttcactgtg ttagccagga tggtctctat ctcctgacct tgggatcccc cctcctcaga 36900

ctccaaaagt gctgggatta caggcgtgag ccaccatggc tggccagttg ttaataaaag 36960

aagaaagggc ggttcctaaa ttgttatgga gaatttacat taaaataacg taagctattg 37020

attggctatc cattgctctt tgtatcacaa attccagaaa cctgaagata aagagtaaca 37080

ctggtcaggc agggtgggtc aggcagggtg gctcctgcct ctaatcccag cactttgttt 37140

tgtttttgtt tttgtttaag atggagtctt ggtctgtcgc cctggctgga gtgccaaggc 37200

gccatctcgg ctcactgcaa cctccgcctc cctggttcaa gcgattctcc agcctcagcc 37260

tctcgagtag ctgggattac agatgcgcgc caccacgccc ggctaatttt tgtattttta 37320

gtagagacgg agtttcacca tgttggccag gctactcttg aactcctgac ctcaagtgat 37380

ccacccgcct cggcctccca aagtgctaag atcacaagca tgagccactg cgcccagcca 37440

atgccagcac tttggaaggc caaggctctt gttgctagtg gttcgagacc agactgaaca 37500

tcaaagtgag acaccatctc taaaaaaata aaacaaacaa aattagccag gcaaagtggt 37560

gcacacctgt ggtccagctc ctcaggaggc tggggtggga ggtcacttga gcccaggagc 37620

tcaagtctgc aatgagctat gatcatgcca ctgcactcta gcccaggtga cagagcaaga 37680

ccctgtatct taaaaaataa aagactgggc acggtgcctc acacctgtaa tcccagcact 37740

ttgggaagcc aacccaagta gataacttga gctcaggagt tcgagaccag cctgggcaac 37800

atggcgaaac ctgtctacaa aaaaaataca aaaattaccc agacctggta gcccatgcct 37860

gtagtcccag ctacttgggt gggctgaggt gggggtgggg gcatgtggag gctgcagtga 37920

gctatgatca caccactgta ctccagtctg gccaacagag tgagaccttg taaaaaaaaa 37980

aaaagaaaag aaaaatagct cagagcagtc tgaggtatgc gagctatgca aaatttgttt 38040

ttttgagaca ggatctcact ctgtccctca ggctagagtt cagtggtata atctgagctc 38100

actgcaacct tcgcctcctg agctcaacca atcttcctac cgcagcctcc caagtaaatg 38160

ggaccacgta ccaccacacc tagctagttt ttgtattttt gtagagacag ggttttgcca 38220

tgttgcccag gctggtcttg aactcctgac ctcaggtgat ccacccacct cagcctccca 38280

aagtgctcgg attacaggcg tgagccacca tgcccagcct ggaatctgta tttaccacaa 38340

gctctctaag tttttctgat gcacaggaag gtctgttaca ccactgtctt aatcatctag 38400

ttagttcaaa ccaataactt gctgctgatc tcctctgtgt gcagctccat gctgtgaccc 38460

actgtgtccg agacggcctg gagctcacaa tatggaggag gagacaaaac tgtctccaga 38520

tagtgatggt gtagcatgac cagtgatggg atagggcagt tgagcaagct ctgagaagcc 38580

agagggggca tctgacccag gctggatggt caggtagggc ttcctggaag aggggacatc 38640

tgagctgaca gaatgcatcg taagcacctg tcattactgt atgctttggt ctgtgggtgt 38700

gtagaatgag ggacaggaac tttaacctaa tttgagggac aggaaggcac ttcatttatt 38760

cattcattct tttcacaaat atttactgaa ctcctcatag tggattctgg agacaccttg 38820

ctgacgaccc agatctttct tttttttctt ttcttttctt ttcttttttt ttctttttga 38880

ggtggagttt cgctcttttt tcccaggctg gagtgcaatg gcgcgatctc ggctcaccac 38940

aacctccacc tcccgggttc aagcaattct cctgcctcag cctcctgagt agctgggatt 39000

acaggcatgc gccaccacgc ccggctaatt ttgtattttt agtagggaca gggtttctcc 39060

atgttggtca ggctggtctc acactcctga cctcaggtga tctgcccacc tcggcctccc 39120

aaagtgctgg gattacaggc gtgagccacc acgcctggct gatctttctt tctttttttt 39180

tttttttttt ttttaatttg agatggcatc tcactctatt gcacaggctg gagtggagtg 39240

atctcatctc actgcaacct ctgcctcccg agttcaagcg attcttgtgc ctcagcctcc 39300

tgagtagctg ggattacagg cgtgcaccat catgcccagc taatttttgt attttttggg 39360

gagatggcat ttcaccatgt tggccaggct ggtctcgaac tgctgacctc agatgatccg 39420

cccgcctcag cctcccaaag tgctgggatt acaggcgtga gccactgccc ccagcccaac 39480

acagatcttt tttccctccc agagctcaca gtggggcatg gtaattataa ataacatttc 39540

aagtacaaat tcagtaagtg ccctgagggg gaagtacagc taggtatgtc cgtgtaacag 39600

gaattcctga tccactgggg aaacctgaag gcttccggga aaaagaggaa ttcatggaat 39660

gaataaaaac tcttactcca ggcaagaaaa gggggcaggg gaaagggaga cccaggtaga 39720

gggaacagca tgtgccataa ccccggagtc agagggatgc ctgggtctta atctccactt 39780

gtcctacctc ctaccccaca gcctggaggg acctccctcc tacaagccac cgaccccaaa 39840

agcgaagctg gaggcacagg agatggtgag cactttccct ggagcccaag ctatccccac 39900

ctccacaccc accccagggc tgggaggggc ctggcaggga gaagctggct gggtggtggg 39960

ggcagttcct gacctccccg cccctctccc actacagccc tcccagctct tcactctggg 40020

ggcctcggag cacagcccac tcaagacccc ctactttgat gctggcgcct catgcactga 40080

gcaggtagga gctcatggga agacaaaggt gggttggggg tctgggttga agggccaggg 40140

gtcaggcctg gcaaaagagg tcaaagacta aaggtcataa ctcaaggtca agggtgtgtc 40200

cccggttggt gcttaatgca ttgagatgtg ttcaggattc catgggggag gtcaggggtg 40260

aaaaggccca aggtcacaag gttagaattt aaagtcactt gggggctggg cacggtggct 40320

cacgcctata atcccagcac tttgggaggc caaggcagga ggatcacttg gggtcaggag 40380

ttcgagacca gcctgtgcaa catggtgaaa ccccgtctct actcaaaaca caaaaaatta 40440

gttgggtgtg gtggtgcgag cctgtaatct tagctacccg gggcgctgag gaggaagaat 40500

cgcctgaacc caggaggccg aggttacagt gagctaagat cacaccactg cactccagcc 40560

tgggcaacag agcaagattc cgtctcaaaa aaataaataa aaccaaataa ataaaatcac 40620

ctgggccggg catggtggct ctggtctata atcccagtac tttgggaggc caaggtagct 40680

ggaccccttg agcctaggag ctcgagacca gcgtgggcaa catgggctac ccagcaacat 40740

tttgcgtcta caaaaatact gaatttacct ggatgtggtg gcaaatgcct gtaggctgag 40800

gtgggcggat tgcttaagcc caggtggttg gggctgtgcc actacactcc agcctgggca 40860

tcagggtgag accctatctc aaaaaaaaaa aaaaagagga aaagtcatta ggtcaatgaa 40920

aattgattat ctggtcaggc atggtggctc acacctgtaa tcccagcact ttgggaggct 40980

gagacgggca gatcacctga cgtcaggagt tccagaccac cctggctaac aaggtgaaac 41040

cccttctcta ctaaaaacaa aaataagcca ggcatgggtg acatgctcct gtaatcccag 41100

ctattcagga ggctgaagca ggagaatcgc ttggacccag gaggcagaag ttgcaatgag 41160

ctgagaccac gccactgcac tccagcctag gcaacagagc aagactccgt ctcaaaaaag 41220

aaaaaggccg ggtgcggttg ctcacgcctg taatcccagc actttgggag gccaaggcag 41280

gtggatcacc tgaggtcagg agttccaaga ccagcctggc caacatggtg aaaccctgtc 41340

tctactaaaa attacaaaaa ttagctgggc gtggtggcgg gcgcctgtaa tcccagctac 41400

ttgggaggct gaggcaggag aatcgcttgg acccgggagg cagaggttgc agtgagccca 41460

gattgcacca ttgcacaaca gcctgggaga caagagcaaa actccgtctc aacaacaaaa 41520

aaaaacagaa tgaaagaaaa ttgattatct acatttgctg gatactgtgc ctgaagctgg 41580

gacaagacaa atcaagtcac tcccctcagt ggtctactgg aggtcagggg tcagacactg 41640

aggttgcgct caaggttcaa aggactgttt agagcccaag gttaagtatt tgaggtcaag 41700

actgaggtga aaggttcatg gggaaagatt tgcagagatc gagtgtcaaa agtgagtccc 41760

aggattaaca gggtgagttg cacgagctgg gctttgggtt aaggaagggc tgaatgaatg 41820

tcccagtgca atatggcctc aggggacaag gagtgaggtg gcatcttgtt ggcatgggga 41880

gagagcggtc aggattttgg ggtcaagagc agattggtaa tctgtgtcgt gcgtaggaag 41940

tgatcttgag ttcctgtcct ctctctacct ccaggaaatg cctcgatacc atgagctgcc 42000

caccttggaa gaacggtcag gacccttgca ccctggagcc acaagcctgg ggtcccccat 42060

cccggtgcct ccagggccac ctgctgtgga agacgtttcc ctggatctag aggatgagga 42120

gggggaggag gaggaagagt atctggacaa gatcaacccc atctatgatg ctctgtccta 42180

tagcagcccc tctgattcct accagggcaa aggctttgtc atgtcccggg ccatgtatgt 42240

gtgagctgcc atgcgcctgg cgtctcacat ctcacctgtt gatcccttag ctttcttgcc 42300

aaggatctag tgccccctga cctctggcca ggccactgtc agttaacaca tatgcattcc 42360

atttgtgatg tctaccttgg tggctccact atgaccccta acccatgagc ccagagaaat 42420

tcaccgtgat aatggaatcc tggcaacctt atctcatgag gcaggaggtg gggaaggtgc 42480

ttctgcacaa cctctgatcc caaggactcc tctcccagac tgtgacctta gaccatacct 42540

ctcacccccc aatgcctcga ctcccccaaa atcacaaaga agaccctaga cctataattt 42600

gtcttcaggt agtaaattcc caataggtct gctggagtgg gcgctgaggg ctccctgctg 42660

ctcagacctg agccctccag gcagcagggt cccacttacc ccctccccac cctgttcccc 42720

aaaggtggga aagaggggat tccccagccc aaggcagggt tttcccagca ccctcctgta 42780

agcagaagtc tcagggtcca gacccttccc tgagccccca cccccacccc aattcctgcc 42840

taccaagcaa gcagccccag cctagggtca gacaggtgga gcctcataca gactgtgcct 42900

tgatggcccc agccttggga gaagaattta ctgttaacct ggaagactac tgaatcattt 42960

tacccttgcc cagtggaata ggacctaaac atcccccttc cggggaaagt gggtcatctg 43020

aattgggggt agcaattgat actgttttgt aaactacatt tcctacaaaa tatgaattta 43080

tactttgacc agg 43093

<210> 32
<211> 66938
<212> DNA
<213> Homo sapiens

<400> 32
tgccaaggct ccagcccggc cgggctccga ggcgagaggc tgcatggagt ggccggcgcg 60

gctctgcggg ctgtgggcgc tgctgctctg cgccggcggc gggggcgggg gcgggggcgc 120

cgcgcctacg ggtgagtgcg accctcgggg cccgaggggc ggccggaggg ctgagggcgg 180

tgagggtcac ggctgaaagg ccccggaggt caacgccggg cggagggccg aagctggggt 240

gggtgctggg ggctgccctc gcgaggcccc gcgctcggcg gctgcggctt cccgcgcggg 300

atctctgggg cccggtactg gggccggttc gaggtgggac ccgggagctg cggggttttc 360

ccgaactccg ggtgcggcgg aggtggcggc agcgaagtaa gagggctgcg acttgtagtg 420

ggaagagccc aggaggctgg cgtccaggcg tgcgggggcg gggggcccga aacatgccca 480

cggcgtcaga gggccgtggg tcagcgcccg gaatgtcgcc tgtgagcccg ggaggaaatg 540

aggcgccaga gccggcgggg accacgaccg aagtccaggc gagctgagaa cgcgccgggg 600

ctctttgccc gaggccgcgc caatcctcac tgtctaaggg tggctcttgc gagtggaagt 660

tgaagggaac ctctaaacat ctcagtcctt gttgaaaaag gacgtgacgc tcatgacctc 720

ctctggcgtc gaagaaatcc tttgggaaat ccagaatagc atcccggcag atgctccccc 780

caccccactc tctttaataa accttatatt ttagggtaat tgtagattta tggaaaagtt 840

gcaaagatac ttcagagagt tgtgtatatc cctcaccctg tttggtaact tctagttaat 900

caacctggca gttgtgaaaa attgggagtt aatgacttcc ttaggagcag ctcctggaag 960

gaatggcctt ctggacgttt ttggtgacaa tgaaagcccc aaactgtata ggttttatta 1020

tcacattgac acaaacactt atatatgctt attgcatttt taccaatgtg ctttgaaatc 1080

caagttgaaa tgttgattag gaaaccttaa tgccctctat ggattcagtc aataaaatac 1140

atgctgccag gcagtgttta ggggctgagg atgcagcagt gagcaaaaca gatgaggtgc 1200

ctgccctcag acatatcggg gggtgggggt aaaatatatg atatgtctga gggtgttaag 1260

tagagaaaaa cagagcaggt gggaaggggt ttgccattgt aaaaagggtg gatcgagaag 1320

aaaaacaatg ttgctgaaag gtggcatttg agtagagacc tgagggagac ttgaagcttg 1380

tcagtcaata attattcagg tgtctgcctt atttagtact gagtcaggcc ctctaggttg 1440

acttagaacc tgagagtgta tagcaaaatt cttgccccaa aagggcctct agtttagttg 1500

ggtacacctg atttccgtaa tcatatgggg acattgcagt ccagagaagg gaaggaactt 1560

gtctaaagtt acccgtctca ttagtctcct gattttcaca gtgccactcc agcaccctcc 1620

ctcctacaga ctcactccca cacacacctg aaacaacagc tggccatcta gagaatactg 1680

gcaactccca acttcatcag tcctgtcaca atggtttgtc gttagcttgc ttatttagaa 1740

aacaaacttc tttccccatt aaaagtattg atagaattgc attgaggtat ggtgggaaaa 1800

aattagatgt ttccagtgag acttgagttt tagtggttct gatatttacc acctgtggga 1860

ctctgggctg gtcatttcct ctcttttgtg ctgcagttag ttttctcatc tgtgaaatga 1920

ggaggttgaa ctgcataaat tctaagagct cctccagctc taatcctcag agtgtttata 1980

aaagtgatta gattactagc ttagtttaac ctgtcccact caatggccca cattgtggca 2040

tgtcttaatt ataactagca tgtatatagc atttatgccc agcagttttc tagctgcttt 2100

acttgtacta tttcatttaa tctttaccaa taatataact tgtaggtgct gttattagtc 2160

ccattttgta cacgaaaaag ttgaggcaca gtgcgaccaa gacccttgcc caaattcgca 2220

tacctggcaa agtggaagag ctaggtttcc aaccctggca gcctgattcc agaggccatg 2280

cccttaacca ctgtactcat tgcttctgcc cagcagaaaa gacgagagtc cttagctgga 2340

cacctcccag ggtagcaggg caggacggct tgggacccac gaaagtatgt gcaagccatt 2400

tgttaagaag ctaatgatac tatattaggc cgggtgcagt ggctcatgcc tgtaatccca 2460

gcactttagg agactgaggc agatggatca cttgaggcca ggagtttgag cctggccaac 2520

atggcaaaac cccgtctact aaaaatacaa aaattagccg agtgtgggat tacatgcctg 2580

taatcccagc tactcaggaa gctgaggcat gagactcatt tgatcctggg aggcagaggt 2640

tgcagtgagg gaagatcagg ccactgcact tcagcctggg cgacagagca agactgtctc 2700

aaaacaaaaa aagaagcgaa tgatactaca ttggagactg gctttatagt ggctgggctg 2760

tgtggtccaa acaactagta ctataggcat tcaaaagaga aaaagctgac ggttggtagt 2820

gttatggaag aggtaaaact tgagctagat cttctggatg actgggattt gagtaggtgg 2880

agaatttatt ccaggtgggg gcaatagcag gagcagagac ccagaaatga gggcaagagt 2940

gctcctttgt ggtcaagaag gaacctggct tggcaggaga gtgcattggt aaagtcaatc 3000

caggcagaat agcttgattt gcccttgaaa gctctgagtc cggcacagca tgggaaaaat 3060

aacacagaag gaggaagcaa gcagggagac ttcactgggt aggggagccc ctggagtgtg 3120

aagctacata agaatctatg gttgcaggtc tccactagaa aaatgtgctg cgaacccttc 3180

ctgtccagga ccatgactta ctggtatctt cccacctggt ggggcacaac agagcatatt 3240

tgcatgattg gggtaactaa gaaagatgtt ggaagaaaaa aacgaacagg agttgatgcc 3300

tgagtggatg taagggaagg gacagatgac tcatatatga ctcttgtgat ttccttaact 3360

tttcccaaaa tgtgcatgcc tctgctagct gctgtgattg gacaggcgag agcaaaagct 3420

ataagctaag caaaatgcct tcttatgaaa tagtagattg tgttaactct taatttaaat 3480

aggatgttgt gctagatgac tgtacagact tgggatttga ggtttagata cagacatact 3540

tgggtccctt cctactccct tttctgaagt cagtggtgca atccccaaga catggttgaa 3600

attaggtgcc taagggaagc ttatcaggta gaagggatac ttgttctgtc tcaaaggcca 3660

gggacacata tcaattcaac agaaaaccaa agcaaccctg gccaggcaca gtggttcaca 3720

cctgtaatcc cagcactttg ggaggccgag gtgggtggat cacttgaggt caggagttcg 3780

agaccagcct ggccaacatg gcgaaaccca gtctctacta aaaatacaaa aattagccag 3840

gcgtggcagt gcacacctgt aatcccaact actcggaagg ctgaggcagg agaatcgctt 3900

gaacccagga ggcagagttt gcagtgagct aagatcacac cagcgcactc cagcctgggt 3960

aacagagcga gactccgtct ccaaaaaaaa aaaaaaaaag caactcttta gaattgtttt 4020

gtcttaaaga aataaagcag atagctaaca catagcactt gctgcctgtg ggcactattc 4080

taagcacatt gcttgtatta actcatttaa tcctcataat aaccctttgc aagaggttac 4140

tattattatc ctcatattag agatgagaaa actgagaaat agagaggtta agtaatttgc 4200

ctgctggcaa gtagtagagg tgggatataa actcaggcag tcaggctcca gagtctgtgc 4260

tcttaaacac tacactatgt tgaagttata cacgaagagc tgttagagac ctttacaagt 4320

agtataatca tcacttaatc atatatatgt ctaggcatgt tgtatacaca cacagttcac 4380

atgtatttgt atatatttaa acatttggag attaaagttt cagtgcctgt gtttttttca 4440

attttttatt ataaaataca cataaaattt accatcttaa tcatttttaa gtacacagtt 4500

cagtgttacg aaatacattc ataatgctgt gcaaccatca tcaccatcca tccccataac 4560

tctttttatc ttgtaaaact gaaactctgt acccattaaa caataactcc tcattttccc 4620

ctgcccctag cccctggcaa acaccattcc actttctatc tctatgattt tgactactct 4680

aagtatctca tatgaatgta atgatatact atttgtcttt tttgtgactg gcttatttct 4740

gttagcatag tgcccttatg gttcatggtg tatcatatgt cagaattttt ccttttttaa 4800

atttatatat atatatatat ttattatacc ttaagttcta gggtacattg cacaacgtgc 4860

aggtttgtta catatgtata catgtgccat gttggtgtgc tgcacccatt aactcgtcat 4920

ttacattagg tatatctcct aatgctatcc ctcccccatc cccccacccc acaacaggcc 4980

ccggtgtgtg atgttcccct tcctctgtcc aagtgttctc aatgttcaat tcccacctat 5040

gagtgagaac atgcagtgtt tggttttttt gtccttgcga tagtttgctg aaaatgatgg 5100

tttccagttt catccatgtc cctacaaagg agatgaactc atcctttttt atgactgcat 5160

agtattccat ggtgtatatg tgccacattt tcttaatcca gtctatcatt gttggacatt 5220

tgggttggtt ccaagtcttt gctattgtga gtagtgctgc aataaacata cgtgtgcatg 5280

tgtctttata gcagcatgat ttatactatt tgtctttttt gtgactgact tatttctgtt 5340

agcataatgc cctcatggtt catgttgtat catatgtcag aatttttcct ttttaaaagc 5400

tgactaatat tccattgtat gcatatacca tattttgctt atctgtttat ctgtcaatgg 5460

acacttaggt tgcttccatg ttttagcaat ttcacaatac tattatgaac ctgggtgtag 5520

aatatctctt tgagaccttg ttttcacttc ttttgggtat atacccagaa atggaattat 5580

tgtatcatat gataattcta tttgtaattt ttttgaggaa ccaccatact gttgtccata 5640

gtgaaatttt aaatttccac caactgtgca ccggggtttg aatttttcca tatcattgcc 5700

aacatttatt ttctgttttt tgatagtagc cattctaatg ggagtgaggt gaaatctcat 5760

tgtggttttg gtttgcattt tcctaaatat tagtgatgat gaacaccttc atgtgcttat 5820

tggctatttc cttatctttg ttggtgaaat atctatttaa gcctttgcct atttctgaat 5880

tgaatttttt tttattgttg agttttaggc attctctgta tattctggat attaatccct 5940

tatcagatat atgatttaca catatttttc cattctgtga gtcacttttt tctgtgaata 6000

ttttgatgca cacgttttaa ttttttccat gaaatccaat tcgtcaattt tttcttttgt 6060

tacctacatt gttggtgtca tatccaagaa attgttgctg aatccaatgg tatgaagctt 6120

ttgtcttatg ttttcttcta aaaattttat agttttagtc ttaaaatttc agtctctgat 6180

ccattttgaa tttttttttt acttggtatt gggtaagcgt ccaatttcat tctttttcat 6240

atggatatcc agttttccca gcaccatttg ttgaaaagat tgtctgcatt gagtggtctt 6300

ggcactgttg tcaaaatcat ttgaccatac atgtgatggt ttatttctgg actgtctatt 6360

ctattacatt gatcagtatg tctgtcttta tgccagtaca cactgttttg attattgcag 6420

ctttgtagta agttcagaaa tcaggaagtg tgagtcctac agctttgttt ttccgtttca 6480

agactgtttg gctattcagg gttccttgag atttcataag aattttagga tggatttttc 6540

tatttctgca aaactatgcc attaagattt tgataaggat tgaattgaat ctatatatca 6600

ctttggatag tattgacatc ttaacaatat taagtgttct aatacgtgaa catgggatgt 6660

gtttccatat catctacaaa cagagataat tttacatctt attttaactg gatgtctttt 6720

atctcttttt cttgcctaat tgctctgggt agaacttcaa gtactatgtt gaataggagt 6780

ggtgaaagtg ggcatctttg tttcattcct gaccctaaag gaaaagcttt cagtctttca 6840

ccattaaggg tgatgtttac tgtgggtttt tcatatataa cttttaagtc aatttcttta 6900

cactcctagt ttgttgagtg tttttatcat gaaaagtaaa ttttgtctaa tgctttttct 6960

gtatcaattg agatgatcag gtagtttttt cccctttatt ctgttaatgt ggtatattac 7020

attgatcaag ttttgtatgt taaaacatct ttgtattcca ggaataaatc ccacttggtc 7080

atggtctata atccttttga tatgcccgtg tattcacttt actgctattt tgttgagaat 7140

ttgtacatca atgttcataa ggcatatggg tctgtagttt tcttttcttg tagcgtcttt 7200

gtctggcttt ggtatcaggt aatgctggcc tcgtagaatg agttacgaag tttgagaagg 7260

tttggtacta gttctttaga tgtttggtag aattttatct gtgaagctat caggtctagg 7320

gcttttcttt gttgagagat ttttgattac tgattcaatc tccttactac ttataggtct 7380

gtttagattt cctatttctt tgtgatttag ttttggtggt tttgtgtttc tggaaatgtg 7440

tccatttcat ctaggctgtc caatttgtta gtgtacggtt gttcatagta ctctcttata 7500

acccttttta tttctctaga atcagtagtg attctagaga aatactcatt ttcatttttg 7560

aaaatgagta aagttctcat tttcattttt gattttagta atttcagtct tctttcttat 7620

tagtccatct agctaaaggt ttgtccgttt tgttgatctt ttcaagaagc ttttagtttc 7680

attgattttc tctatttttc tattcccttt ttcttttatc tctggtctaa tcttcattat 7740

ttccttcctt atgatagctt aagattagtt tgttcttttt ctagttacgc aagttgtaaa 7800

gttaaggttg ctgatttgag atctttcttg tttcttaata tgtgtttata gctataaatt 7860

tttccaatag tactgctttc gctgcatcgt ctacatttta gatgtttata tttacgcctt 7920

tcatcaaatt tgggaagttg tcatccatta tttctttatt ttcttttttt gagacagagt 7980

ctcactctgt cacccaggct ggagtgcagt agcacgatct cagctcactg caacctctgc 8040

ctcccaggtt caagcgatgc tcctgcctca gccacccaag tagctgggat tacagatgtg 8100

caccaccaca ctcagctaat ttttgtattt ttagtagaac tggggtattg ccatgttggc 8160

taggcttgtt tcgaactcct ggacccaggc aatctgcctg ccttggcctc ccaaagtgct 8220

gggatgagcc accacacccg gctcattatt ttttcaagta ttctttcttc ctctttctct 8280

ctcttctcct ggaaccccca taatgcatac actggtctgc tgatggtgta ccacaggtct 8340

cttagactct gttcactttt cttcaatctt tttttctttc tgttcctcgg actcaataat 8400

ttttattgcc ctaatttcaa gtttattgat tctttcttct gcctgctcaa atctaccttt 8460

gattcttttt agtgaatttt tcatttcagt tgtgcttttt ctgctccaga gtttcttttt 8520

ggtctctttt taggttttct atctctttat tggtattttc attttgctta catatcattt 8580

tcttgactct ctccacatct tcctttagtt ctgtgaacat ctgaagatat ttgtttcaga 8640

atctttcttt aatatatttg ccatcaggtc tttttcaggg acaggtttgc tgttgtttaa 8700

ctttttttct ttgggccata gtttcctggt ttttttgttt tgttttgttt tgtctgtctt 8760

gtgattcttt gttagaaact ggacatttaa atctggtttc tctggaaacc agattccccc 8820

ttcccccagg atttgctgtt ttttgttatt gttttgttta ttgtctttat ttttttgatt 8880

attgtaggct gtctgtgtgt tgagcatcaa cctgaagtgt aaacttagtc ttctcaggtt 8940

tttttctggg ctttccctga gcatgtgtgg tcactttctg tttttctctg tatatatagt 9000

tttgtttcat tttgttttgt tttgtttttg agacctagtc tcgctctgtt gcccaggcta 9060

gagtgcagtg gtgtgatcac agctcactgt aacctcgaat ccctgggctc aagtgatcct 9120

cccacttcag tctcctgagg agctggaact acagattcat gccaccacac ctagctaatt 9180

taaccaaaca aacaaaaaaa attgttttgg tggagatggg atctcgcagt gttgccaggg 9240

ctgatcttta actgctggcc tcaagtaatc ctcctgcctc ggcctaccga agtgctggga 9300

ttataggtgt gagtcaccat gcccagcctc tgtagttgtt taagaatttc taatctttaa 9360

tgcctggctc ccaaaagtgg aaaaggagaa aaatgaggcg acaaggaaag agcgccattc 9420

ttttacatct tctggaaatc actttagcta gatagggagg gcttgcaaca gtggggatag 9480

gtgcaaccac aatggctgcc agcctctttg tctgtacctc tgtgatcaga agcagcaatc 9540

agtaatcaga atacaaatcc ctgatatttg gaggacagag tacttctgcc cacatagccc 9600

acaagctgtg ggcagagggc tccaggaaca tgtgcacagc cgcctgccat ggggttgggg 9660

atgaaggatg ggcagctact actatgtaaa agctgacatt gattgaaatt tatggcaatt 9720

tgttgttcaa gcctttcctg ggaaattgca agccttcaat gtaagtgttt cctgatataa 9780

ctctgcagtc ttccaaaatt gttacatcaa acagactctg ccactaccat tgttatctag 9840

gtgaggagat agatgtctgg tgcttcctac tcttgcctta ttcccagaat ccatgtgtgt 9900

gtgtttttaa agtgtaggaa gtagaaaaat caaaagatat ggaggaaacc agaagagaaa 9960

aagaaaagga tagcagatag ccccaggaag cccaaaatgc aaaccagtta gaagatctga 10020

ccaaaggaca cttcaaaaca gatgggagaa tattaatccc atttgaaagg ttgaatatag 10080

ggcaggccat tgttttattt caaaactgag gttaaagtag gttttaaatt accttgaaaa 10140

taaattggag tacaattgaa atgaaccaca aataggtata tttttattat cagggcttta 10200

atatttgtgt gggagtggga ggtaggtggg tagggagagt actggaagtg ctaaactagg 10260

tttcttggat gtgtttctgg gaactgcaga ataaggcaga taatcctaca gtaaattgta 10320

gtctccagta ggaacaatgt ttttattgga ggctctctac ctgacaaatg atttgtcttc 10380

aaaaatatca ttgatatggt gttagaaagg ccaaaatgga atagccatac atgcttttaa 10440

tcacttaaaa tcctaacttt agcactgttg cctgagccat tggtgctccc tggtactagc 10500

tcagttttaa tgcagtgcaa taggaattct ttgtttccac aaggccttgt cctgctgtgg 10560

gctgctcctt cccacagttg gaccttggga aaaggtagag ttagaagagg gaatgatttt 10620

ttttatttca aattttatta tagattaaag ggtatatgca cagatttgct acatgggtaa 10680

actgcatgac gctgagactt ggggtccaac aatctgatca cccaggctgt aaccatggta 10740

cccaacaggt ggttcttcgg cccccaaacc cacctccctc cctcccttgt ctagtgatcc 10800

ccagtgtcta tcatttccat ctttatgatc atgtgtattc agtgtttaac acccacttat 10860

gagtgagaac atgcggtatt tggttttcta ttcttgcatt agatcactta ggataaaggc 10920

ctccagctcc acccatgttg ctgcaaaggg catggtttca ttctttttat ggctgcgtag 10980

tattccatgg tatatatgtg ccacattatc tttatccagt ccactgttgg tgggcactta 11040

ggttagttcc gtgtccttgc ttttgtgaat agtgctgcag tgaacataca ggtgcatgtg 11100

tctttatgat agaatgagtt attttccttt gggtatatat caagtaatgg aattgttggg 11160

ttgaatggta gttctatttt aagtcctttt tttttttttt tttttttttt gagacatagt 11220

cttgctctgt tgcccaggct ggggtgcagt ggtgcaatct tggctcactg caacctccac 11280

ctcctgggtt caagtgattc ttatgccaca gcctcctgag tttctggtat tacagttgta 11340

caccaccatg cctggctaat tttgtatttt ttagtagaga cggtgtttcg ttatgttgtc 11400

caggctggtc ccgaactcat gatctcaagt gatccaaccg cctcagcctc ccaaagtgct 11460

gggattatag gcgtgaacca ctgtgcctgg cctattttaa gttctttgag aaatctctaa 11520

agtgctttcc acggtggtgg aaccagtttg catttccacc aacagggtat aagcattccc 11580

tattttccac agcctcatca acatctgttt tctgactttt aaataatcac cagggaatga 11640

atgttgaggg tcaagggcct ttgtagactg ctgaccaagt agctgctatg tataaaattg 11700

actgttagag aaacctcaac tacagagtgt ctgtgagttt gatgttttga tgttttggga 11760

caaatggaat tttttgctca gttagggagg aaggtaacag tataaatatt gctgattatg 11820

taacaggccc agtgcactcc aaagtattag catagagtac aagatttaac tctatcattt 11880

ggtaaattac tattactata atccttaagt aattaataat ggttccgttc atcactttag 11940

aaaatttggg ggaattattt tcaaataatt ttaataaact tctatgttga aataattttt 12000

gatttacaga aaagttgcaa aggtgggaca gagggttcct gtatacccat cacccagttt 12060

ctgggggatg attttgatag tctcaggaga acttctcagg aaagtttaaa atgatgtttc 12120

acagttcctc ctctttctct tccctcttca gattagtcaa agctctagct ccagctgttt 12180

agtgtcagac ctcaggggaa caagggctgc taagtctctt tctcagtact tctttggaaa 12240

agctataaaa aaacaattac caggtcatgt tagcaagcag aattaaagat gagcttctta 12300

aacttactta tagaactgct gagattttga acaatctgat tttttgttgt tgtcattgtt 12360

tttgagacag ggtcttgctc cgtcacccag gctagagtgc agtggcatga tcttggctca 12420

ctgcagcggc atgatcttgg ctcactgcag cctcaaactc ccgggctcaa gtcatccttt 12480

tgcttcagcc ccccagtacc tgggactata ggcatgtacc agcatgtttg gctaactttt 12540

gtaacttttt tgtatagatg ggattttgct atgttgccgg ggctggtctc aaactcctgg 12600

gctcaagccg cttggtctcc caaagtgcca ggattacagg tgtgagccgc cgtgcccagt 12660

ctgaagagtc tgtatggagt gaggtgtgga tcccaaggtt agggaacccc aaattcctgg 12720

aagagaacaa cttggaaata ttagtattta agaatatcac tcttctctat ggaagtggtt 12780

aagaatgtga ccttggagcc acacttttca agtgacactc ccagctctgc cgtttactgt 12840

agccatggga tatggaacaa attacttcac ctgcttgttg cttcagttgc ctcatcttta 12900

acatgaggcc aataggccag gtgtggtggc tttatacctg taatcccaga actttgggag 12960

gccaaggtgg gcggatcact tgagctcagg agttcgagac cagcctggcc agtgtggcaa 13020

aaacccatct ctactaaaat tacaaaaatt agccaggcgt ggtggcatgt gcctgtagtc 13080

ccagtaacag gctaggtgaa tcacttcacc ccaggaggca gaggctgcag tgagctgaga 13140

tcgtgccact gcactccagc ctgggttaca aagtgagatt ctatctcaaa aaacaacaac 13200

aacaaaaaca tgaggctaat actagtccct atctccaaga ttgttgtgag gattaaatgg 13260

attaatatgt gtggaatatt tagaatagtc cctggcccag taaatgttag atgttgttaa 13320

ctcagttatt actgtcttgt cctctaggac ttgggagaat taagcaaacc attaaaaata 13380

ctgttgataa attcattttt ttttgtcctt gatttgaaca gaaactcagc cacctgtgac 13440

aaatttgagt gtctctgttg aaaacctctg cacagtaata tggacatgga atccacccga 13500

gggagccagc tcaaattgta gtctatggta ttttagtcat tttggcgaca aacaagataa 13560

ggtaagtttt ctgcaacatg ttacattgat gaggtaaagt gaacactatg aattggagcc 13620

aagaataagc caaattctag tcttacgtca aagtggaaat tgtattttag ggtgtgttta 13680

aatttcagtt ggggaaatta tagaagcaac tattgacaca agtgaacact taaaaatatt 13740

tgctaattat ttgacaatta aatgcaattg agattgctct gacaggaaat actagatatc 13800

tttttctcta aggtactcaa taaataatta tgttattgtt aaaatttgtt cctatgttcc 13860

tgcctgtagc tgtattgcct tgtatctata taacacttaa ttttggggaa tttttacatt 13920

taccatcttg ctaatcacat ggacaaatag atgtacaggc actatgtggt gtaaaagtaa 13980

acggccatat ggaaagtgaa aaagtaagtt gtcagtggca ggtgtgtttg agtagagggt 14040

tctgtaatgc ccgcggaaaa aggaggaaga ctttcaagat ttctagtcaa ctgatataag 14100

tgaatttttg ttaataggtt tggataattt caggtccttt atgataatct tattcaactt 14160

ttttttcaaa atctttttat accttctttc ggtgtgtatg tgtttgttgc agggtggcgg 14220

cggtgagtgg gttgggtgtt tggggttgaa tttgtccagg attttgagta ggcagaatga 14280

gaagttatta gcaacagtgc tttgcatcag tgaattgtag aagcaccaag ttcaggcagc 14340

tttggtggca ggaagtcagg agaactagct tctgattggc actgtcactc ttagctgact 14400

ctatggcctg gaataaattg cttaacctct ctgagcgttt gtttccttat gtataaagta 14460

tggctttact cacttcgctg gcttgctggg acacagaaaa taatgtatat atacaaaagt 14520

ttaatagtca ggatggaaca tgctcttcct ctggagggaa atttgctaat ctgccttcct 14580

ctcccttgtc ctgctgaggt gttacttatg gtttaggcgt taattgataa agaattgagg 14640

cttatcagaa gtatttgcat tttaaaagag gtcttctaaa tgtggaagcc aatgatacta 14700

ctagcagtga gactggagtt tgcctttaca tactgtcggc ttcctcttgt tcctcctaag 14760

gaagggcctt aggcatttgg agtcttagtg gtgatttttg gtaattgagc cttgagaaat 14820

tcacagaaag accctaagtg cctctggctg taagccctgt aatcacccaa gtgtctaact 14880

gaaaccctga gcagaggttg cttttcaggt cccttttcct taagttgtca aggatcaatc 14940

aggtagccag gctggtgttc tagcctagaa atggggctat ttcctcagag tatgtgcaga 15000

aatctagagt taaatgtaac tttaggagac agtagaatca gatagaattg tgcttgaaat 15060

tttatctctt cccgtttcaa gctgtttgat tttgggcaaa tcacttaacc tctctgagct 15120

cccattttct tgtttataaa atatggggtt gttttggcgg ggggcgggta ataatagtac 15180

caactgaata tggatggtgt gtgcattaaa ggagatgagg tttgtaatgc acctgacaca 15240

gagttagtgc taggtaattg ttagctttta ttattgttac tgttgttagg ttgagccatg 15300
tgaaattacc acttttatgg gtcaaaatgg ttgaatgttg tcagtattat ttgtcagcct 15360
aatattatca tttaatcttg tttcctccat tttacccctg aagaaaccag atagagacca 15420
aaaagggtga cctctgccta aggccagctc attcattgta gccatttgac agcattgatg 15480
gccatcagtc ctacatttgt gatgaacacg gattattcct tccagtcagt gataggagat 15540
atcctgtctg gacctttttc aagtttatca tcaggaggct gaaaatgaat ggcagaagta 15600
attttcctct cctctttctg tcctttgggt acagcattag catcattggg ccaaactgag 15660
gtatccctga agcatattga agcaatggca ttatgatagt ttgtacatta gggggcttag 15720
gaaaatgttc aaaggtccca agttgctaaa tgagataaac ccttgaggtc attttcaatc 15780
ttaagattgg aggctcatca agttgagaga cagggatttg cagaggattt ttggaaaaga 15840
aaagattagt gtaaatggca gccctccacc attttttagg cagtgtctaa tagttcctca 15900
gctggcaagt agaaggagga tctagcatca gattttagat atctttcatc actaagagat 15960
gggcaagtcc cattaggcaa actaatcaaa aggccaggca tccaaatagg aagatgctga 16020
agctaactcc ctttcttttt gagaaaggaa ttctggagtt gagaaaggtg cagtccagcc 16080
aagtgaacat tggtctgggg agatcagttc aggactcttg ccttggtttt tgataaattt 16140
tcaaagggaa tcagattccc tgcagggtgg agcataacat catacagcag ctaccagtgg 16200
gaatgattca ccaaagccaa aagtcacatg ggagccatca cacatacctc atcaagatct 16260
gatgggaggg ttgaacgtgg tgactcatgc ctgtaatccc agcactttgg gaggccgagg 16320
cgggctgatc acctgaggtc aggagttcaa gaccagcctt gccaacatgg tgaaaccctg 16380
tctctactaa aaatacaaaa gttagccagg cgtggtggtg tgcacctgta atcccaacta 16440
cttgggaggc tgaggcagga gaatcgcttg aacccaggag gcagtggttg cagtgagccg 16500
agatcatgcc actgcactcc agcctgggca acagagcaag actgtttaaa aaaaaaatct 16560
gatggaagga atttaggaat ttagtttctt gtgtgataca gagaggtgta cttgacttgg 16620
cagttgttga taatctgggt tcctacttta ttagtgccag tttaatattg tttaccttga 16680
actttgaaag aaattaaaaa aaaattattt gaaaaaattt tccctctact gtcccttttc 16740
tatgttatta ttcatatgcc tgaagcataa ggttcggatt tagatactaa ggcatttttt 16800
aattttttat gttattttat tttgaaatgg agtctgctct gtcacccagg ctggagtgta 16860
gtggtacgat ctcagctcac tgaaacctct gcctcctaag ttcaagcaat tcttgtgcct 16920
tagcctgcca aatagctgga actacaggtg catgccacca cgaccagcta attttttttg 16980
tatttttagt ggagacagtg ttttgccatg ttggccaggc tggtttccaa ctcctgacct 17040
taggtgatcc tgacctcagg tgaccttggc ctcccaaagt gctgggatta caggtgtgac 17100
ccaccacgcc tggccactaa ggcatttttt aaattaaaaa ttccattgtg ataatttttg 17160

tgtactaaaa ctccgtaacc ctctaactgg aacaatgtcc attataatat gaaaactggg 17220

attatcttca tgatgattct aatttgactg tatttgactg tacagtctaa tttgacattt 17280

gactgtacat tttcccctga tgtcttcaaa gggcaaatat agcttccaaa caagaattgc 17340

catggatcct tggataaggg tggcttcttc aaagatatat gggacttgtg cttagtaaaa 17400

ttgtgaggtt ggtaaaattt gtattcgaag ccttcctgcc accactacca tgatcctgga 17460

ggcaggtctt cagcttcaca tactcagaaa cttcatttcc tcagcacaca tgcatctctc 17520

aggggctgat tttttacttt gttgtgaaaa tggaatggta atggtgaagc attctaggaa 17580

gatttctgtt agcataactg gtgcatttca tccctgtcgg cagagcctat atgggaagcc 17640

aaatgttaag taggtttctt ggtgaggaat cagaaattag ttgtaaaaat atacttgtct 17700

aagaatagct ggaggcccta ctgagagtaa acagctttgc cagtgggaca gaaaaattca 17760

atgcccacat tcctgtatca ccctcttttt cctgcctgtc aggattttgc tcacagacat 17820

ttcttttctt gttcttccag ggctgaccaa ctccctttc agagtactgt cttgactgga 17880

cctaagagct attctggttc tataaagtga ttttgttttt gtgtccttgc tatgatttat 17940

tcattgttga cttggtagtt acttctcaga ggccatccat atgtcctggt tttgggggta 18000

tctcacagtt gccctcatgc tcagctggca gcaggcatgt gacatggcag aatagctctt 18060

tagggtagca gatagaacta actgtggcaa attttcctct tcatggccgt caacatgtag 18120

atgaaaggaa atactatctc tgacttgagt cagaagctat tgcaaaacag gaggtgctct 18180

ccttggagct ctgattaaaa gcagctccat tcttaattat tccaactctg tcagtttggg 18240

aaatgttaat ttcccggaga gcccattaat cttattattc tcagtcatag taaccagact 18300

ggtgatttat gtctctatga tgtctataaa ttggcttcct atggttccac aaagcttatc 18360

tacttttcag aatgagagtg tatatactca gcacatatag caatgtttga aatttataca 18420

tatatatgag gtagtcaaaa tagtcaaaca gtagagaaag aggtttaatg aaaagtctct 18480

cttcagccct caggccctcc tcctttactt ccttacccag aagtaaccat gattactgga 18540

tttttttttt gagacagggt ctcactgcat tgtccaggct agagtgcaat cttggctcac 18600

tgcagcctcc cgctcccagg ctcaagtgat cctcccacct cagcctcatg agtagctggg 18660

actataggca tgagccacca tacctggcca attttttttt gtacagatgg ggtttgccat 18720

ttttgcccag gctggtctcg aactcctggg cttaagtgat ctgcctgcct cagcctccca 18780

aagtgctggg attataggca tgagccactg agcctggcat gattcctgga ttcttaaagc 18840

atcccttttt aaccttaatt ttcctggtac agtgcatcta cttctaagtg aattttgata 18900

atcatattgt ttcctttaaa attgctttta gagagttgat atgttagcat tgtaggttta 18960

taggtaaatt tcatgtttat acctttaaaa tgtgggaaat tgttgtatct acaccagggt 19020

ttctcaatgg tgacactagt gacatttgct gaagggtttg ttgtaggagg aggggggctg 19080

ttgtgtgcat tgtaggatgt ttagcagcgt ccttgacctc tacttactag atgctggtaa 19140

cctccccctc ccaagttagg acaataaaaa gtgttcgata ttaccaaatg tctctgagag 19200

cggtagggag aacaaaatca gccccaattg agaatcactg atctatacca tttgataact 19260

tttttgataa aaaaagcacc acatttagac atgctaatgt ttcattatat aagtgtgtgt 19320

tagctgaagc aatgcctttt tcaattttct aaccttagaa aatagctccg gaaactcgtc 19380

gttcaataga agtacccctg aatgagagga tttgtctgca agtggggtcc cagtgtagca 19440

ccaatgagag tgagaagcct agcattttgg ttgaaaaatg catctcaccc ccagaaggta 19500

acaactgaaa gcgctatctc ttggtgttta gagaataaat ctgaatgctc caatgctttt 19560

gtgcattgtt cattaattaa ttcaattctc aaacattttc tgagtctaat aagtgccagg 19620

cattgtacta ggtgctagca gctcaatgaa aatatgagta tgtctctgcc ttgaagaagc 19680

ttacaaacaa cttgaataac aatgcctaat agttatataa aatttaataa tttaaaaaat 19740

gatatacaca tgcacgcgcg cgcacacaca cactcacaca cacacacacg cacacccctc 19800

ctttagatcg agagtcacaa gcttatcttg tgaagtgagt aaggcatgta ttattaccct 19860

gatccatttt atgaggaaga aaggaactgc gtgacttgct gtatgtcttc acatttcagg 19920

cccagtgctt taaaaataaa acacaaaata ctctcttacc cattatgtgt gatacttagt 19980

ttgatatgca ggatatattt gggaaaagat gttgtttaaa gagaaaaata ttatgctggc 20040

ctggctccaa tacaagtcta gacaagatag tacaagacaa ccttaggaaa agctgtcaag 20100

tagtgaggat gatcattgtt ccccaatccc acctctccca atgtccttct ggctcttatg 20160

ttacatacag ggagtagcat atactgatta agagcagggc taccagaaac caactgtcta 20220

actttaaatc ccagctctgc cagctactag ctgtgtaacc ttgggcaaat ttcttagccc 20280

ctctgcacct tagtttcctc atctgtaaac tgggcatgat gatagtatct atctcctaga 20340

attattggga tgattacgta agtcagtata tgtgaggtac ttagaatagt gcctggcaaa 20400

gagtgaatgt tgtgtgtgtg tgtgtgtgtg tgtgtgtgtg tgtacacaca caaaaaacat 20460

atatatataa cacatatata tactatatat aatatatatt atatacatta tatgtaatat 20520

atataatatt atataatata taattatata taatattatg ttgtcccttg gttgggtagg 20580

gctgggcccc atctcttaac ctccagtcct tagctctatg tgccctttgc tctaagccat 20640

tccatttacc tgctttgtac tgatcacaaa cacatgaagt gctgtttgta aaattataaa 20700

attcaggact gccaggagat ttgaaacaac ttacctgccc tattctcctt tccttaggag 20760

ttaacgaaaa aaaaaaaaaa aaaaacggct agatgcagtg gctcacacct gtaatcccag 20820

cactttggga ggccaaggtg ggcgaatcac ctaaggtcag gagtttgaga ccagcctggt 20880

caacatggag aaaaataata ataattagcc aggtgtggtg catgcttgta gtcccagcta 20940

ctcaggaggc tgaggcagga gaatcgcttg aacctgggaa gcagaggttg cagtgagcca 21000

agatcacatg actgcactcc agtctgggtg acagagcaag actctgtctc aaaaaaataa 21060

taattactat actatgacat aaaatggttg atgatggctc agtgtagtac cccttcaagg 21120

atcatgaggg ataacagatt tacattttaa acagaggtgt ttaatttaaa gacattatac 21180

cttaactaaa atggctttat aaagatagag attttaggtc agaaagatac tagggctctt 21240

aaagtcatga cacttaagag gcaactacta agagttaaat taggcttacc tttccccaga 21300

attgcttcag gaggaatgtt tgtttgtttg tttatttaga gacagagtct tgttctgttg 21360

tccaggctgg agtgcagtgg caagatcttg gctcactgca acctccaccc ccctggttta 21420

agcaattctt gtgcctcagc ctcccgagta gctgggacta aagatatgtg ccaccacacc 21480

tggctatttt ttgtattttt agtagagaca gggttttgcc gagttggcca ggctgttctt 21540

gaactcctga cctcaaatga tccacccttg ttggccttcc aaagtgctgg gaatacaggc 21600

gtgagccacc ctgcctggct gaatgtttat tttttaaaaa atgtgtcagt attgatcaaa 21660

agacagaaag ttaaattgta cctcaataat tttttaagac agatatatat aagggttgtt 21720

tgtatatcca cattcattta ggaattgtgt agatattgtc tatgattgaa atagtttttg 21780

atcttttatc tctccctcca gttaggaata ttagttaggg tccctcagtg atagggaaga 21840

tgttttcttt gtatcaggag gtaagagaga aggttgtgaa caaattagat ttgagtttcc 21900

tccatcctgg gtgacttctg ggtaaaatgg ggtgagagat gaagcatttg ctttctgaca 21960

aagcagcatg aagcactctc atcatcccca tgccaaagag tgatttgcta ctttgcatta 22020

caaggaaaga agggtcttaa actcttggcc tggatattct aggtgatcct gagtctgctg 22080

tgactgagct tcaatgcatt tggcacaacc tgagctacat gaagtgttct tggctccctg 22140

gaaggaatac cagtcccgac actaactata ctctctacta ttggtgagta tgtacagtac 22200

aattactgga agactgattg taattgtgtt gggagccttt ggatccaacc tagtcaaaaa 22260

aaaaagaact gtttaatttt ttctgcaaac tcaacaagat ggacagtttg cttcaatgaa 22320

aaatgcatct gtctttggtg tctacttttt aaataaactt tctattttta aatatagatc 22380

ctgtatatta ccagaaaaaa tgtacagaga gatcccatat actccactca gtttctccca 22440

gtggtaacat cttgtgtaac tatagtgcat tatcaaaacc agaaaattgg cattggtaca 22500

atccaaaaac ctcagtccta tttcaacagt tttacatgca ctcgtgtgtg tgtatatgta 22560

gttgtatgca attttatcac atgtgaagat atacaatggt tgcatcacca taaggagttg 22620

ctcttttata gccacactca cctccgtctt aaaccatctc tccttgtaca cccctctccc 22680

ccaatcctct aacctctggc aaccactaat ctgttctcca gctctttaat tttattattt 22740

caagaatgtt actgaaatgg aatcctatag tatataacca cttaagactg gcttaaaaaa 22800

aaaaaactca gcgtaatacc ctggagatct atacaagtag ttgtatatca cgaggccact 22860

cctttttata gctcagtagt attccatgat atggatgtac cagtttttta aaccattcat 22920

tcattaaagg atatctcagt tttttccagt tttttgctat tatgaataaa gctactgtaa 22980

atattcacat acagattttt atatgaacat aacttttcat ttctctggaa taaatgccca 23040

agagtgcaat tgctgggtca tatagtaatg gcttatttag tttttttttt taatgccaaa 23100

ctgttttcta aagaagttag ttcaccttac atttccacta gcaatgcatg agttattgat 23160

gtctactttt aaaaaggaaa atgatatgtc cggggtcaaa aagttgtttg tttgtttatt 23220

tatgtttttg agatagagtc ttgctctgtc acccaggcta gagtgcagtg gtgagatcta 23280

ggctcactgc aacctctgcc tcctgggttc aagtgattct cctgcctcag cttcccaagc 23340

agctggaatt acatgcatgc accaccatgc ctggctaatt tttgtatttt tggtagaaac 23400

agggtttcaa cgtgttggcc aggctggtct cgaactcctg gcctcaagtg atctgcccat 23460

ctcggcctcc caaagtgctg ggattacagg tgtgagccac cacacccagc ctcaaaaagt 23520

agtttagctt cctgcaaaac aactttgata ttttgatatt tgatacttta gtatttggta 23580

tttgtgacta tatctactac aaagtttgtt aaatttctaa gttagtcttc catcattccc 23640

tttagagcct agaattatta gcaacagaac aaggaagaaa gaccagatta atccagttta 23700

attcagtaaa cctttattta gaacactttg tggataaacc actgaatgca cagcagagac 23760

aaaaaacata tctcttctct cctaaagctc aaaatataga ggagaagata gtgcctgata 23820

cagtgacctg cattaaatta agtaagacag tgtatgctga aggctgttgc agagggatag 23880

atagattcct gtgggaagtt aggttggagc ttggggtggt ggtcgagggg aggtggatgg 23940

gtggatggag aggggactga gaggctttga tgaaggagga agactgccta gatgggggag 24000

tttctctgat gctggcctct ggggaagagc attttgagaa atggaggtat ttaatcgttg 24060

tgatgtcagg gtggagaagg ggagacctag cataggcagt ctgcctctac atggccttgt 24120

tccctgactg tcatttgtta agacttcttc tgagagaatg ggatgtggag agccatgtcc 24180

aagacttgtg aagctactgg ataaacatgg cctttcatgc tagagtgtct attttgcctg 24240

atgacatgat ggaaagcatt tttgatacat taaaacactc atggctatat catggaatag 24300

aatgcaaaaa aaaaaaaaaa aaaagtaaca cacatttttg gatgaaaacc tccaaagttg 24360

ccccaggatt cacatccgca ctcctccctg gtggaaggat cctcctggca aagttgggaa 24420

atcctgcagg tggagccaga agttctgtgt ggttatgtct gaggtctgtt tctttttccc 24480

cccctgatgt aaatctcatt tgaggttttg tagtttcacc tgctcactct tggtacctgt 24540

tgtttctcct ttacttaaaa cccttcagtg gctccctggt ggctttaggt taaagtccaa 24600

actctccagc atgacagaca aatcgcttta tgacagacct ggcccctgtc aacctctcca 24660

gactcggttg catctgcctc catgcacccc cgcccctctt gcagcctacc attcgcccgc 24720

agcaaacttc tttcagttcc tcagactgct ttcttcaaat actcaaacac aacactagat 24780

ttcaaacaga atattcaaac agaatagtgg aaacattctg catcctcttg tagcctaggc 24840

ctttcctttt catttggatg cctcttattt gtctttatcc ttatttcagc ccagacacca 24900

tttcctctcc agaaagcctt ccttgacctc tcaagtctag acatgtgtcc cttcctcttg 24960

tctcacggca ccttgcaatt tcttgtgtca tactacccag cacattgtgt tgtcatttcc 25020

tgttacttgg tggtagcctg ctacaggctt cagtttctct tgagagcggg agctgtgtct 25080

tgtttctcct ggtgcctgat atatagtagg tattccataa ataagttgtg aatgtttggt 25140

aggatccaaa gctagagaat attcagggtt gcggtgatga cttatgaggt ataagcaact 25200

gaggaatatt atggttcatg aataaaaaga gagaaggatt gagtagatcc agctccctaa 25260

agaattgcta agaaggggcc aaagaactaa gccttactaa gaaggagtag cctggctgtt 25320

gatgttgtag taaagtttag ggagagctgt tacacaggct tttaagatgt cttgtgagtt 25380

ggcagcatcc atgtgtttat tatgctatta acccctcttg ttttccctgg aatcattatt 25440

ggtaaaaccg cagggaaaac ctttaaagaa tctatgtcta tataagggac tggaaaagaa 25500

gggaagaagt cttaaaactg tatctcattc catggcagac tgggtagaaa attatcccag 25560

ctattagaat cagggtggca ctggaggcag atggtagcct agtccttggg gttcttctaa 25620

gacaaaaata gggaagtgcc ttggggatat actagcaaaa acaaagtctc aatgcaaatt 25680

gttaaaagaa tgtttcctct gggtgaatga attacaaaaa aacctggact gaccaatttg 25740

aaaaagcatt cctcttcctc tgggctgaag caggctctca ccaaagctga gagcagatgg 25800

tacttgggct ggctttcagc cctcagttga tgtctttttg cagggaacaa tctgcacagc 25860

cactggaggt cgctcaagat gcaattccaa gatagtggtg ggtcactagc atccaagggg 25920

gcagatatca aggatcctac ttttttttgc gatagggtct tgctctgttg cccaagctga 25980

agtacagttg gtgtgatcat tgcccactgc ggcttcaacc acccaggccc aagccatccc 26040

cccatgtcag cctcctgagt tgctgggatc acaggtgtgt accaccatgc ccagctaatt 26100

tttgtgtttt ttgtagagat ggggttttgc tatgttgccc aggttgctct cgaactcctg 26160

ggctcaagcc cgtctcggcc tcccaaagtg ctgggattat aggcatgagc caccgcacct 26220

ggcctacttc ttgtcatata cagtcccaga gctggtcatc agcaggggat ctttctgtag 26280

tgtagttctg tgccatccag tataggagcc actggctaca tgtggctagt tacattttaa 26340

tttaaatcaa ttaaaattga atcaaattaa aacttcagtt gcatagtctc gccaaccact 26400

ttcacaagtg atgagtggct cttgtattgg acagcacaga tagcgaccat ttccataatc 26460

ccagaaagtt ctgttggatg gtgctggtat agattctgta tcttatcccc ttaccacccc 26520

acagcttatt ctttctggag ccacactgct tgggttgaaa tcctggctct cttccttact 26580

ttagtgtgat tttgggtaag tcccttctct ctcagcctta gtttcataat ctgtgaaatg 26640

gagatagtaa taatagtgcc tacttcttaa ggttgttgtg agggctagtg tttagagcca 26700

tgactagctg ctagcatatg tagagtaagt gctcaatcca taatagctat tattactatt 26760

ggccaggcgt ggtggctcac gcctgtaatc ccagcacttt gggaggctga ggcaggtgga 26820

tcatgaggtc aggagatcca gaccatcctg gctaacacag tgaaaccccg tctctactaa 26880

aaatacaaaa aaatagccag gcgtggtggc gggcacctgt agtcccagct actcaggagg 26940

ctgaggcagg agaatggtgt gaacccggga ggcggagctt gcagtgagcc gagatcccac 27000

cactgcactc cagtctgggc aacagagcaa gactccatct caaaaaaaaa atactattta 27060

ttattattat tattgttatt atccccactc ctttgcagat gcagtcagta cacagcgctg 27120

gcctcttccc gtctcacaga attagtacct aacccatatc tccttctttc ttgctctcct 27180

ctagatagat cctttctaat caaaaccaca aaagaaaggg agtcaagaca cagtgctcat 27240

tagcaagcac cagaaatggt ttaggtacca gtataaatca catcaaacaa actgccagag 27300

actgttttcc ctaaccccca accataaaat acggagtctt tctttttttt tttttctttt 27360

tttttttttt tggagacaga gtctcattct gtcgccaggc tggagtgcag tggtgcgatc 27420

tcggctcact gcagcctctg cctcccgagt tccagtgatt ctccagcctc aacctcctga 27480

gtagctggga ttacaggcac gtgccaccac acctggctaa tttttgtatt ttcagtagag 27540

acagggtttt accatgttgg ccaggccggt ctggaactcc tgacctcaag tgattcaccc 27600

acctcagcct cctaaagtgc tgggattaca ggcgtgaccc atggcgcctg gcctggagtc 27660

attttttgaa atcaaaataa ttcattgaag ccctagggca atgacttgtg tccctgtagg 27720

gacagtctgg cattgaacag accccggcct gaagagcttc agactagtca tgccagcgaa 27780

ttttagggat tttttttttt tttaccacat taatcatctt ctgagttggg agaatagtct 27840

ttaatttgtc aatttccttc tcatatctgc agtgtgactc tgcagatgaa ttcagtatat 27900

aaattttaac aggaatcacc tgctgtttta ggttaatgtt tcacaagaat tcaagaaatg 27960

ttatctattc agaaccagta ttttcccttc tataattatt tctaaattac aacaaaattg 28020

ttttaaaatt tcattatctg agatccatga gcatatgcag aatactcagg gtacagaaat 28080

gaaaaaggga tcaagaagat tgtgcattcg aggagttcct ttcatcatta aaaacaattt 28140

tacacatggc tttctttagt tatgtaagaa aaatgtcatt aaatttcttc agtttctgtt 28200

tttggccatg ggcggaaaac attccaggac agtctgatcc aaaaactagt catgacaata 28260

acttcatttt tacaacactg ttttcttcat aaaccacagg gcgctgcaac tagggggaaa 28320

agcttatgta ctacctattg aaggaaaaaa gcattttcta tatcgaagca ttcctttgtg 28380

cagcctttga aggaataaat acattagctc tgagtttttt tttttttaaa ctgacttgca 28440

gcagaatctc ctggagaatt gctgttcaaa tgctggttcc taggtcctgg caaaaccttc 28500

taaatcagat tttctgggag agggtttgag aaacctgcat tttaaagtag tacagaggat 28560

tctgaggtaa caggttaagg atttgggact ctttactctt agttcatttt tcttaaaatt 28620

gtcttccttt tctgcaaaac ctttgtgcaa ctccctttgt ggctgatgat gtaagacttg 28680

actctgtctt ctgccagtta ctacaaaaca gttctctgat agcctagact aaaaaaaaaa 28740

gtgtaataaa cacattgtct gcctgcgctg ggctaggtgc tgatgatctt tggggaaagg 28800

tatgtgtcga ggagaaatgg gaaataaggt acctgtaata ctgcttatgg aagacagtta 28860

aagccacaca gtggagtgag acacttagaa agccattgtg ttttagaaca ttgacctggc 28920

agtgattgga gggcactaat aatgaaggtc agggaggtca atctgaagaa gcaatggagc 28980

ctgtagtcat tagggtatga atctcagggt gtgggcaatg gatgtatcat aaaagacgag 29040

gatccatgaa gctttgcaaa ggaagaaaga acagaatttg gtgatagaat acagggaaca 29100

gaggtggggg aaaaggtgat tttaatttaa attacctaat ctaggagccc aaagaaaatg 29160

agctctggct gataatggga aggttggaag aaaaaacagt ttggtggaga agagggtgca 29220

tgatgtgttt tggacactgg atttgaggca atggtagaac actcaaatgg agcagttgga 29280

gatctgggat ttgttctgag gtgagatgtc aggattagga attaccagca tagaaactgt 29340

agttgaagga tgagggagtg ataaactgga tgtgagtgga tgagggagtc agaaagaggg 29400

aagaatgtga gtagtcctcc tactccccct ctgctggaag agagaggagg gaacagaaca 29460

agccaagaaa caaggaagga gcagggcaga gggaccagag gaggaatcgt agacttcagc 29520

agataggcat tttcaagaag catgaaactt ctaatcccag ccttagaatg tcttctctta 29580

tacagaagaa ataggcctcc tgtagtctct atagctgctg acagtaccag tgttcatcaa 29640

aaaggaacaa tctaggctgg gtgcaggggc tcacgcctgt aatcccagca gtttgggagg 29700

ccgaggcggg tggatcactt gtggtcagga gttcgagacc agcctggcca acatgctgaa 29760

cccgtctcca ctaaaaatac aaaaattagc cgggcgtagt agtgtgtgcc tgtaatccca 29820

gctactcagg aggcatgaga ttcactggaa ctcgggaggc ggaggctgca gtcagctgag 29880

atcgtgccac tgtactccag cctgggcaga cagagtgaga ctctgtctca aaaaaaaaaa 29940

aaaaaaaaag cagaaaacag tcttttcttc ctagcatatg aaatgagtca cattgtacaa 30000

tagggttttt ttaaaaaaag ttatcttgcc cattgcaaat attgcttttc atggacatct 30060

cagtcattaa aaagaattct gccttttttt tttttttttt tttttttttt tttgtggaag 30120

ggtcttactc ggtcacccag gctggagtgc agtggtgcaa tctcagttca ctgcaacctc 30180

tgcctcccag gttcaagaga ttctccttcc tcagcctcct gagtaggtgg gattacaagc 30240

acctgccacc acacccggct aatttttgta tttttggtag agacagggtt tcaccatgtt 30300

ggcaaggctg gtttccaact cctgacctca agtgatccgc ctgcctcagc ctccaaaagt 30360

gctgggatta caggtgtgag ccaccgcgcc cagcctgctc ttatttcttg aatactctgt 30420

tgaatataat gcagaagatt gtctcatttt atcttgtagg cacagaagcc tggaaaaaat 30480

tcatcaatgt gaaaacatct ttagagaagg ccaatacttt ggttgttcct ttgatctgac 30540

caaagtgaag gattccagtt ttgaacaaca cagtgtccaa ataatggtca aggataatgc 30600

aggaaaaatt aaaccatcct tcaatatagt gcctttaact tcccgtggta agttttagaa 30660

gtcctctaaa acagtatgga taaaaagtgt gttatatctt ttgcacaatc aatacaaata 30720

ataaaaaatg atcattctgg gtttaagatc ttctgaaaga agggactata ttgatgtata 30780

actttctttg atagatcctt tttaaagaag taaacttgga aattcttttt attgaacaca 30840

tgtgccagat gctttatcct tgtttagagt acactgagag ttagaacagt tagttgtaga 30900

accaacattg gaattgtctt agttcatttt ttgtttggta ttttctggta ttctgttgct 30960

tataacagaa taccagagac tgggtaattt ataaagaaaa ggaatttatt tcttagagtt 31020

ctggaggcag ggaagtggaa ggtcaagggg ccaagtctga tgagtgcctt cttgtttgtg 31080

gggactttct gcaaagtccc aagacagcac agggcatcac atggttaggg agttgagcat 31140

gctagctcag gtctctcttc ctctttgtat acatccacga gtcccactcc catgataatc 31200

cattaatcgg tcaatagatc aatcaattca tgaaggcaga accctcatga cccaattatc 31260

tctttaaagg tccaacctct ccatactgcc acattgggga tgaagtttca acatgagttt 31320

gggagaagat aaacattcaa atcatagcag gaacccaggc catctgactc taaggtctag 31380

tctcttgata cttaaagagg atgatactca taatgatcat atgtagttcc taaataatct 31440

gtctcttaga aaaggcatag acttaaggta gtgtaatcgg gtgatattca aaatatttaa 31500

cattctgtat ggcatgggca ctgatcagtt agaaaaacca gtatagcctt aactgtgggt 31560

gccagctgca tgtcagcctg ttagaaagca ccctcagctt gaacacagat tatctgatta 31620

ggttctaact ccttcacttt gggcaaggca tataaacccc cttcaaatat ttccttatct 31680

gtgaaatgga atatataatc tccctctatc taaaaaaatt gtcataaggt ccaaatgaca 31740

taaagcgtat taacacattg tgtaaattac taaagcaaat tccattagcg taatggttta 31800

tttgttgaaa tatcccaact gggagctcag attctggctt agatagtcat agaagtgaat 31860

tctaataggg taccaaagtc agcttacttt gtaggccaag ggagtgaggt tggagtattt 31920

tgcctagtgg ggtaagctga agcagagccc ttctctttgt ttggcagatt atcttagatg 31980

atcttttaag tcttattttg ctgttttcct ctctggatat taatggtgac ataaaggaag 32040

aaggaagatc cttctcagat agaagatgat atcttcaagt tgttttctgt taattaccca 32100

ttatcttcta ttgcttggtc ttaagtatag ctgactcctg aaaaagtaaa ttgaccaggc 32160

taatgcattt ttaaaaataa attatttttt tctttttctg agacagggtc tctgttgccc 32220

aggctggaat gcagtggcgt gatcacagct cactgcagcc ttgacctccc gggctcaagc 32280

tatcctccca tctcagcctc cctagtacct gggaccacag gcatgtgcca ccacgccagg 32340

ctaatttttg tattttttgt agagacgggg ttttgccatg tttcgcaggc tggtctcaat 32400

ctcctgagct caagcgatct acccaccttg gctcccaaag tattttactc ttaattgtgg 32460

ttataaacac acaacattaa aattaccatc ataaacattt taagtgtaca gttcggtagt 32520

gttaagtata ttcacattgt tatgcagcag ctctttagaa ctttttcatc ttgcaaaacc 32580

gaactctgtt cattaaacat taatttcccc tcccctccgc ccttagcaac cacctttcta 32640

ctttctgttt ctatgaattt gactacttaa gatacctcat aggagtggat tcatacaata 32700

tttgtctttt gtgactggct tattttacct agcataattt ccttgaagtg catttatgtt 32760

gcagtgtgtg tcagagacta agccaatgtt ttaatggtgg tctttgattg gcattaataa 32820

gtgacaacta gggaagcccc ttgtatctgg tgccttatta ctcatcaggt ggaagtacat 32880

ctggatccaa gcctttcttt attctctatg ttgcgtctag atgtcctgat tataccctgg 32940

ccattttcta accagatgca ggtcagagtt catatcatct gctagatacc ccaaatatgt 33000

tccttaaatt cattttacta ggcagtttta aataaacttc ttctataatt gtggctaaat 33060

agaaattcta aaactatgta tctgatcatg aaaggccact aaatgcatca gctctgtttt 33120

gatttaactt tatgaaccct gcatatatct gcagttgtct ctcagtattt gtgagggatt 33180

gcttccagga acttccaagg ataccaaaat ccatggatgc tcaagtccct gataaaaagt 33240

agcatagtac ttgcatataa cctatgcata ttttctcaca tactttaaat catgtctaca 33300

ttacttataa tacctaatac aatgtaaatg ctatgtaaat agttgttata ttatctaggg 33360

aataaatgag aagaaaaaac tctgtacatg ttcagcacag atgctatttt ttaaaaaata 33420

ttttcagttc atggttggtt gaattcatga atgtggaatc catggatatg gagggccgac 33480

tgtgtgtttg tgtgtataat caagctttgg aagcttactt gtgttctatt tttgtttttc 33540

agtgaaacct gatcctccac atattaaaaa cctctccttc cacaatgatg acctatatgt 33600

gcaatgggag aatccacaga attttattag cagatgccta ttttatgaag tagaagtcaa 33660

taacagccaa actgagacac ataatgtttt ctacgtaagg ttttaaaatt attgttttta 33720

tttggctatt tttcttttac ttgaatttct tacattgaaa tatcaatgaa acatagctct 33780

tacatttgtc accttatgat gatgatggta atgaaacttc ttgtctaaaa taaggcatgg 33840

tggaagattt gaagatttat ggtatgatag tatcagctgc aagtttcaag ccatttattc 33900

atctgtaagc ttcttagaaa ggcctattaa acgataattc atttgctcaa cacgttctta 33960

ttaaatatcc accacatgcc aagcactctt ttgtgtgcta gggataaggc agtgaacatg 34020

acacctgaca agtttttttc ttcatgaaac ctacataata gaaaacgtaa gtaaataagc 34080

aagctaattt cagaggatga gtgctgtgaa gaaaataaaa tgggctgatg tagtagagtg 34140

actgggagaa cgagagggaa tattaggtag ggaaatcatc tgaggtaata acatttgaga 34200

tgagaccaga atgatgagca gacgctagcc atgtgaagat ctgggtgtag ggggtcagca 34260

gatacagact ctgaggcaaa aatgagctta gcatgtgtga gagaacagaa aggagggtgg 34320

tgtgaacaaa atgtagcagg aaaagatgtc taagcagtag gcagacatca gatcttgcgg 34380

acactttgga ggctctgata ataccattct gttttcttgg agtttgtggc aactaaagta 34440

atcagatttg agtagtcaat atttgttgga ttatgtgcct gtatgctatt cagaatcaaa 34500

tgcagaacat gctaattaga ggccctgtag gacactgacc agagtgtact ataatctcag 34560

gacatcagtc cagttgttct gctttagggt aaaggtcaca aactagggcc tcacactttt 34620

ttgtgtggaa cacaaagtcg tgttttgttt gtttgtttgt ttactagatt tgaatttttt 34680

gcccagattt taaatcatag tactttaaga gtgtgtgtcc ataagatggc gccttaagac 34740

catgaagact gcaccactca cttgtctgct acctgcctgg accctttgtg tgtttgagtt 34800

ttagagatta gaagtgaagt gttttatatt ggcttttaaa ggagcctagc atctcgcacg 34860

tgggtaaatt ccttggtcca gttgttttag ctcatcatgg aacttccaaa atgcttgcta 34920

aaataagcca gtgttgacag gctataattc caactccaga gggagtgacc aagtccacag 34980

cagaacagaa cagcaaatgc tcttctgggc atttgcatta tgaatccttc tgctgaaata 35040

taataatcat tactagtacc agaaattaag tgccccatga aatgcagcaa agggaaaaat 35100

ctaagggctt tcatgtggaa gggggctggt acttgaattt acaacaaaag gagttatcag 35160

aaagctgaaa tacacttgtt tactccagtc tttactgttc tctcctgtct gaaatcctga 35220

gatgcatgtc tgaaatcctg agatagtatt cattttgcca ctcaattatt ttctgctgtt 35280

taacctatca catgtatgca ttttctctcc actgaaaata tagactccta cagaaaacca 35340

aatgccacat gttctcactt ataagtggga gctggacaat gagaacacat ggacacatgg 35400

aggtgaacaa cacatactgg ggcctgtggg ggctgtggtg ggaggaggga gagcatcagg 35460

aagaacaact aatggatact ggccttaata cctgggtgat gggttgatct gtgcagcaaa 35520

ccactatggc acacttttac ctatgtcaca aacctacaca ccctgcacat gtacctcaga 35580

atttaaaagt tgatattttt taaaaaaaga aaatatagac ttctgtagga aggagtttgg 35640

tgctgtctac tttgtgcagt tcctgctact aaatagcagc taacctgtat tgagtgatta 35700

ctatgtgcta ggtatcttaa taagcactat atctgaattg tttcatttaa tcttcacaat 35760

aaccctaagg atctaggtcc tgttattttg tctccatttt acaagataag aaaacttagt 35820

tactcctaag gtcacatttt tagtaagtgg tagaatcaac atttaaccaa agttgtaacg 35880

ctaagacttg gattcttaac cgctagagaa cacctcaata cttcctctta atagcaatag 35940

ctatcattca ttaagtgttt atggcatgct acaaagatgc tcttctatgt gcttcttaat 36000

acagtagctc atagcaaccc atgaaataga aactatttta acctacattt tattgaaaaa 36060

actaaggctc agagaggtta agtaacttgc tacaagccac acagctaata agtcagtaac 36120

acttcaaagg ttggttggtc tccatgtatt taactttgac caagaatact tagcataatg 36180

tcatgtgcac aatcatcatt taataaatta ttattgaatt gagttacttt tttttttttt 36240

acaaacttct gaactgaaga gaacacatac actatggaag ttatctacct aaatcagaac 36300

tattttctat ttggaattaa ctctgtttgt acacttgagt ttaatattct tcctcctagc 36360

agatgtagaa tgtcatcagt tttttgtgtg ctcacagatt ttgaactctg ctgcttagat 36420

ttttcttttt ttttcctttt tgaggaatta ttttgcctgg tgttacactt aagaggggga 36480

agtaagcggg aacatgaact ggtaccaaca catgggggct gtggtcagag gtagctcatg 36540

acagaattat gaagtaagga acaacttgtc tgtgaccacc ccccaccccc cacccccacc 36600

ttcccagcag actatcttct tggtgccctg gctttccttc tcttagtggt ctctgaagag 36660

ccagtaaact atgcaggggg atttacattt gaacaagagc ctagagacgt aaatctacag 36720

gttgattcgg caagtttgga atttctatat atgggagatg gtccagggga ggagggacat 36780

caatttgcaa cttgatccct cactggtagt tgacgcttgt gattccccaa caggtctacg 36840

attctaaatg gccagtaata gggtatacta gccccctgag tggggttttt tttttctcaa 36900

cctctgtatg aaatattgaa aggatgtccc ttcatcattt gcttggcctc tcagctcttt 36960

acacaggctg gtaggaacta tttagtgaat gacattgaat gtcaaaaata atatctatgt 37020

gttctggtct tggttccaag tttaaacgtc tctttgatag aagttgacta tttaagcaac 37080

agattttcat gtgtttttta ctccattttg gaaatacttg catttaagga aattagtatt 37140

ctctttacat tagacagcaa gtgactttta ttgtgtggag gagaattttg cattacactt 37200

tcttttctcc aacccagtca agttttatat cttatactct caacaaggga cactggtttc 37260

tcgctccctg tctcttttta aaaagttttt actgagttat aatacatgaa caaaattaca 37320

caaatcataa cctatagctc aatgattttt cacaaatgat ttttcaccta tgtaaccagc 37380

accacagaag cccccttcag ttgctgtcct cccaaagagc agctactctc ctggcttcta 37440

atcccataga ttgattttgt tgctacttta aaactttata taaatggaat tatatagaac 37500

gtactttttt ttttcgagac agagtcttgc tctgtcaccc aggctggagt gcagtgcagt 37560

ggcgcaatct cagctcactg caacctccac ctcccgggtt caagcgattc tcctgtctta 37620

gcgtgcggag tagctgggat tacaggcaca tgccatcaag cccagctttt tttttttttt 37680

tgtatttttt agtagagacg aggtttcgcc atgttaggca ggctcctggc tctcgaactc 37740

ctggcctcat gtgatccacc ctcctcagcc tcccaaagtg ctgggattac aggcgtgagc 37800

cactgtgcct ggcccagaat gtactttttt gtgtgtttgc cctttttttc actcaacatt 37860

atgtttatga gattcaccaa tgttgtttct tatagctgta gatggcacgt tctcactaca 37920

ttcattccat tgtgtgaata taccatactt tatccattct cccattgatg gacgtttgga 37980

tagtttccca tttggtttta tgaatggagt tcttatgaac gttcacatac ttgtctcttg 38040

gtacacatgt gcgtacattt gtatggggca tttatatagg catatgtggg tcataaggtt 38100

acatatgatt gtctctagta gataataaca gttttcctgg atggttgtgc cagtttacac 38160

tactaagcag aaattgtgag ttctagatgc ttcatatcct cacaacactt gttgttgtca 38220

ggtaagtgtg tgtattgtta tcttgtggat ttaatttgca tttttctcat gaccagtgat 38280

gttgaacact ttttcagatg tttattcgca acttgcttat cctcttatga agtgcctgtt 38340

caagtctttt gccagttttt ttaaaattgg gttttctgtt tttgccttat tagtagggta 38400

agaaaaaata catatgcaag tcctttgtca gaaattgcaa atatgttctc acactctatg 38460

cattgcctgt ttatttccct ataaggtgtc atttgatcaa caaatgtttt taattttaat 38520

atggatgact ttattaaaaa attctcttta tggttaatat gggaggtttt tttggtcttg 38580

tttaagaaat atttcccttc ttcaaggtca ttaagatatt ctctgttttt atctaaaagc 38640

ttgctatagt tatgtattta ttaattcatt ttatcttaca catttaaatc tgcaatatat 38700

caggaactga ttttgtgaat agtatgaggt agggcgtcaa gaatcttttt ttttttcata 38760

tgcatatgta attgacccag caccatttat taaaaaaggc caaccttttt tcactgtacc 38820

tgatcatctt ttaatgggat aaaaggaaag cttgttagaa atgactaggt gatagaaaat 38880

gggtttttaa acatttagga ctcattagtt tatttattta tttttatttt caaggtccaa 38940

gaggctaaat gtgagaatcc agaatttgag agaaatgtgg aggtcagtaa attcaacatt 39000

agctatttgg gtttagactt agagcagttg ggggtggagc atagcctaaa tatgagctgc 39060

tttgagaaaa acaaattacg ttgttccgtg agttattgac aattttttcg aaaagtcaaa 39120

gataatttga attgcaatcc aagtcttaat catagacaga atttggcaga taattgtatt 39180

atgattcaat aacagtgaaa aagaataaac tggtaatatt ccttgtgtat ttttatttta 39240

tgcctaagaa tacatcttgt ttcatggtcc ctggtgttct tcctgatact ttgaacacag 39300

tcagaataag agtcaaaaca aataagttat gctatgagga tgacaaactc tggagtaatt 39360

ggagccaaga aatgagtata ggtaagagaa cagaattttt attaaaattt aaaacactga 39420

tgtataaaaa ctaagctgaa gcagaaaata gactggggaa agggactgtc atgctctctt 39480

gatgtcagga aaagagtact tacaattgaa atttattttc ttcttaatca ctttcaaacc 39540

acccagggtt aattgacagt atacagttat ttagaaaacc ataatccaaa tggttttatg 39600

ttcttttcta tctcatttcc atttttatta acaaactgaa caaaaatata gaaagcaggc 39660

ataggctgca cacagtgact cacgcctgtg atcccagcac tttgagaggc tgaggcagga 39720

ggatcgcttg agcccaggag ttggagacca gcctgagcaa catggtgaaa ccccatctct 39780

acaaaaaata caaaaattaa ccagatatgg tggtgcacgc ttgtggtctt ggctacccaa 39840

gaggcagaga tgggaagatc tcttgagcct gggaggcgga ggttacagtg agctgagatt 39900

gtgccactgc cctccagcct gggcgacaga gtgagaccgt ctcaaggaag aagaaaaaaa 39960

aaaggcaggc ataataaaat ttgcacattg tacaatacta gagactgtcc tacatgctgg 40020

ttggcagaaa caagagtgga gccatttaat agaaacctgt acctaagttt gaaaaaaatc 40080

atttattaat tgaaaggctg agcaagctag gctcaagtac aggactggaa agacctgtgc 40140

tttatggatg tgtgtgtggt ggggtgggat acacggcttt ataagtgcaa tgtgactcaa 40200

cattgtaatc ttaaatgtgt caaaaaggca tatacaatct taggagtgat aagtgcagca 40260

cccagaaagg aatgctgcag gttggggcac agagctatgg tcacatcaga attgtgcagt 40320

attccattct gagcaccaca ttaaaggagt tgccttggca gactggaatc tatccttgaa 40380

aggataatca ggatgttgaa gaatctcaaa acttggcaag gaacaattgg taaagtaact 40440

ttgaggagtc atgattgctg atttctaata tttgaggtat tgtcaggtac aagaaggaat 40500

aggctttact ttttgtgtct ccaaggggca acacagaacc aacttaagaa ctttttaaac 40560

agctgtatat tggtcaaaca ggttgtcgag gtagccaatt ccctattaat tgagaagctc 40620

aacaagttat gtgccaggca tggttgtatt cgtttattta ttgttgtata acaaattact 40680

ccaaagttta atggcttgaa ccaacataca tttaccatct cacagtttcg gtgagtcagg 40740

agtctagcca cggcttagct ggaggtctca tgaggttgca gtcaagctgc aatgagaact 40800

gaaatatgtc tgaaggcttg actggggaca gagcatccat ttccaagttc actcatatag 40860

ttgttggaaa gcctcagtac ctcactggtt gctggatgga ggctgttgca ggttgaattg 40920

tatctcccaa aaaggtatgt tcgtaggttc caaccccggt acctgtgaat gtaaccttat 40980

ttggaaatag gatttttgca gatataatca agtgaggatg aggtcatgct ggattaaagt 41040

ggacagttaa tctaatgact agtatcctta taaaaagaga agccacacag agacacacag 41100

acacacaggg aagaaagcca tgtgaagatg gaggcagaga ttgcagagat gcagctacaa 41160

ccaaggaaca acaaggattc ccaggagcta ccagaagcta ggaagaggca aagagggatt 41220

cttctctaga gccctcagag ggagcatagc cctgctgaca ccttcatttt ggatttctag 41280

cctccaaaac tgtgagataa tacatttctg ttgttgtaag ccattcagtt tgtggtaatt 41340

tgcttccctt agttccttgc cacttggacc tccccatagg gctacttcct gacatggaaa 41400

ctggctttcc caagtgagta atccaagcaa gaaacagtgt gagagaacac gcaatatggg 41460

aaccatggtc ttttataacc taaccttaga aatgacatcc cattactgct gctgttctgt 41520

tttttagaag tgagtcaatt agtctagccc atgctcaagg agaggggatt acaccaggga 41580

atgaatatca ggaggcagga atcatgtgga gaaatcttag tgactgccta aaacaatggt 41640

gccaggcctt gatgatgctt tctagatgtt catgacctag tgggaaaaat gaatttgtat 41700

atagacaaat gacagtctat atgttagatg tagcaaggca ggctttggaa ttggacagtg 41760
gaactggaat ttgaggcctg atgccattat ttactaaatc cttccggatg acttactaaa 41820
cctctctgag ccttatcttt ctcatctgct taaatgggag ataatgctat cattatagca 41880
tatatatgag ataagcatgc aaaaccacct agtgtggtat cagacatata gtaggtgctt 41940
atttaagaat aatagtagtt gttaggatta ttttaggtat tttagtagtg gtaagtaacc 42000
agtaaaatat gtagtcactg ttaataagat tacatattta atatagcatc tttgttaata 42060
gtatttaaaa ctagttaaga tcttctgatt tacctgagcg ggtggggaaa cccaccccat 42120
gagcatcccc tgggctcacc cagtgcacag agggaggcct cccgtggctg ggcccctctc 42180
agtcagccca tgtggctgcc atgacctgga gcaccacagc cgggggcgcc caccagctca 42240
acaccaccac attcacgtgg cagcacctcc ctgcctggca accgctgctg ttggccagca 42300
ttaggctgca gctcttcttc tacgtgggcc tggccttcat cagcctggac ctctattact 42360
cctccaccag catcaaggag ctggagtaca actacaccgg cgacccgggc accagcaact 42420
gctcggtgtg tgctgtggct ggccagggct gtgtgccact gcccatctgc tcatgcgcct 42480
ggtacttctc actgcctgag ctcttccagg gccctgtgta cccctactac gtgctgacca 42540
acttctacca aaacaaccgg cggtatggag tgtccgcgac aacgcgcagc tgagcgggct 42600
gcccagcacg ctgcaccatc cagtcaatga gtgcacccac tgcgccgcct gcccatcgtg 42660
caccctgcaa tgtcatcacc aacagcctct tcaacgactc ctcgctgtgg caccagtgct 42720
ggcccggcga gccctacgtg gaggtgccgc gctaccgcac tgcacctgca tcacccggta 42780
gaccaactac cccatcaagt tctgcaaccc accactggtc aacggcagcc tggcactggc 42840
cttccatggc acagcacccc tgcccaactg gcgctggctg gtctacgaca agctcagccc 42900
catccccaac aacaacggct tcatcaacca ggacttcgtg gtgtggatgc gcatggcagc 42960
gctgcccacg ttccgcaagc tgttccgcaa gctgtacggg cacatccgcc agggcaacta 43020
ctcagctggg ctgccgcggt gtgtctactg tgtcaacatc acctacaact acctggtgcg 43080
tgcatttggc ggccacaggc tccgcatctt cagcagcatc ttgtggatgg gtggcaggaa 43140
ccccttcctg tgcatcgcct atgtggtggt cagctccctc tgcatcctta ctggctttgt 43200
catgctggtc atctacatta gctaccagga cctaccagaa agatgatgac aatgaggagg 43260
aggggtgatt ccagctttcc aacgaccctt cctgcttctt accaaaattc tgcaagcttt 43320
tttcagtctc tcccctttgc ctctcatcct attccaacct ctcctcactt ttcctccctt 43380
tgtgaatgag ggggccagag aagggaatta cccacttact cttgggggct gctaaactct 43440
gttgcctggt ggcggagggt tgactgcaga gtgaaacatc cttgcaaact ctccccacct 43500
ccttcatgat gctgagatgc catgttaggt tctccaagtc caagagtgga agggatcctt 43560
atagagactc attttcaacc cctaccagag gaagagattg agagacctag caacatcaaa 43620

taactcaatc aagatcaggc agctggcaag tggttcctga cgctaaggct agagcttttg 43680

ccactacagt acagtggttt tcttttcttt gtggggaggg tgggttggga tggagagtga 43740

gacccacaaa ttatctgcag aggcatggag gggtgtggga gaacatattt gttaaatatg 43800

cagatagatg aggaggcacc aaccccagag attctgacac agtaatgctg gggtgagatg 43860

cttgaaactg tgttttaaca aactcctctg gagattcctg tattctctca aatttggaaa 43920

tcactaccct gaacctggtt gggcctgaag cagtcacctg agtccagttt ttcagatagt 43980

aatttgttcc cagggacagt gacaccataa tgttgcaggt ttggtctggc actctgcctt 44040

aaatatagta tgctctaatg acttgtggaa tgaattttta aaaaatcact atctctgggc 44100

tgggcgccat ggctcatgcc tataatccca tcactttggg aggctgaggc gggtggatca 44160

cttgaggtca ggagtacaag accactctgg ccaacatggt gaaaccctct ctctactgaa 44220

atataaaaat aagctgggca tggtggcaga tgcctgtagt tctagctccc gggaggctga 44280

ggcaggagaa tcgcttgaac ccgggaggcg gaggttgcat tgagtcgaga tcgtgccact 44340

gcactccagc ctgggtgaca gagtgagact ctgtctcaaa aaaattaaat taaaaaaaat 44400

cactatctct ggaaaactag ttggtgtaca gatttgtagg ccagggtttg tgtcgattta 44460

tttgatattt tagtataaca gcaaacaggc aaactttacc agatactgtg tagaaatttg 44520

aaaatgtgag gccagtttgt acagttcaga gggaatgctt tttaactgta gaatcagata 44580

gctggaaggg atctttgagg gaaagtaatt tccctagtca catctatgtc tcctggctca 44640

gtcttctttg tcatttaatc tgtgtgtgtg ggtgtgtgcg tgcgcacacg tgcgtactta 44700

caaagggctt cattcatacc ttttctcttg gatctggaaa aaattttttt atgttttcca 44760

atgagataca tttgtagtaa tcatatttga aacaatgcta aggtatatat agtcttatat 44820

gtaaactaga atcttaacat tatttgaatc ttggagataa tgttaaaagt cttagctaaa 44880

atgattccat tgattttatc gattgactaa catcaatcaa tatgtttaaa gttattctaa 44940

gaagcaatat ttttatttaa aaaaaccttt ctttcatggc aaaatagctt aaaagaactt 45000

taaaaaaaaa ccctatgtga tatgttccag tttatttgct aaaagcaaac agttatttgg 45060

tatttgtcag acagaattct tcagtacctg ctttctatta tagctatttt ccagttacca 45120

ctcattcgtt tatactcctt cctttaaagc agcacaagat cttgaagttt tttttttatc 45180

agttaagaaa tattggattg tattggatag tatacatagt ttagtctgtt agttctctga 45240

aagttactga acaatggaca atgtgcatgt cactgacatc cgtctcagat cttctgggac 45300

aactatgatt taagagattc tatctcatta ttacagatac caaaaaaata cttgggtaat 45360

tccagtgtgt tggctttcca actcctgaac actacacaat tttattatca gtttttgtat 45420

ggtgatttta ggctatgaaa acattatttt atgtacatag agaaattttt attatgaatg 45480

tttgagctca ctagcccagc cctcctctat tttgaaaaag agagtttact acatttttta 45540

ctatgtagtt gaaaacaaca tgtattttgt catttttaga atggtcagtc tatttctata 45600

aaattttaaa tgtggctggg cgcagtggct catgcctgta atcccagcaa tttgtgaggc 45660

tgaggcggac agaacacttg aggtcaagag tttaagacca gcctggccca aatagggaaa 45720

ccccgtctct actaaaaata caaaaattaa ctcggtgtgg tggtgtgcgc ctgtagtccc 45780

agctactcag gaggctgagg caggagaatc tcttgaaaca tggaggcgga ggttgcagtg 45840

agccaagatc gtgctattgc actccagcct gggtgacaga gtgagagact ctgtctcaaa 45900

acaaacaaac aaacaaaagt ttaaatgaga ccatcaaatt taagggattc tgatccagat 45960

taaattaatg aatatggttc caggtcctag tctctgagtt tttgaaagag aataaagctt 46020

atgtttgtct tttaaaaaat atcctaattt caggtaatct tttaggtatt tgctcgccta 46080

gggaagttca ggcaccagag gaggactttt tttttttaaa ctattaattt aacagaagat 46140

aagataattg aagagcttgg gaaaataggt tgggggaggg agcagaaata tttgtgatct 46200

tactagctaa cagaattgct gcttggttta ttactaaaag ggttttattt ctcaagtctt 46260

tctgtttgct tttcattctc caggtaagaa gcgcaattcc acactctaca taaccatgtt 46320

actcattgtt ccagtcatcg tcgcaggtgc aatcatagta ctcctgcttt acctaaaaag 46380

gtaaggtagc tgcccaggca ggttttgcag tttctgatag cctgtttgct gtggttatta 46440

cttttagtgc ttgtgtttta tgtctgccca gtgtattaga agcatgttgc ctgtaagtgg 46500

ctttcgggag tatgacatga caaggaagta ttaccacgcc cactttcttt tcttttcttt 46560

tttttttttt tttcctgaga tggagtctct gttgcccagg ctggagcgca gtggtgtgat 46620

ctcggctcac tgcagcctcc acctcctggg ttcaagcaat tctcctgcct cagcctcccg 46680

agtagctgcg attacaggtg cccaccacca cgcccggcta attttttgta tttttagtag 46740

acagggtttc accatgttgg ccaggctcgt ctcaaactcc tgacctcaag tgatccaccc 46800

tcctcagcct cccaaagtgc tgggattata ggcatgagcc actgcaccca gccgctatgc 46860

ccactttcta atgaaaacca tgttggatta gttttccagg gctccctgct caagcactgc 46920

tgcctttgca tgtgtttagg attgttgaat aggtcctatg ttcacataat actctttcaa 46980

atgcctaatc tatcaagtta gtcagtagtt cttagaatga agttttagaa atggagacct 47040

ttactaataa aatgacttgt ctggtattaa tgcaaagtag gctactactt aatattgtgg 47100

gaaagagaac ttcatcaagt tgtcatgaat gtatggtatc cattccttga gaatatttta 47160

ttttctctat ctcattatag gactgttatt tgctcttgta gtaaataact aaggttgaaa 47220

taaggttatt aagttggatc cagaaccaat attttatcat tatttcaaca tttattcaac 47280

atatatatat actctctcta tatatgtata ttttttgagt gcctatttgt gaaccaggta 47340

ctgttctaag tgctaaagtt acagcagtga acaggacaaa gtcctcatct acattctaga 47400

gggaaagata cacacatgca catacacaca cacacacaaa ataaataata aatactataa 47460

tttcacttag aagtataatt gacagtagtg gggtatggct aggaggtgat aggaggtgca 47520

ttttatgtag gatgtcacca agaaagtgac atttgaggag agactcccag taatgtaaag 47580

gagtgaatca ttcagatatt cgaggaaaga gcactccagg ccatgggaac agcaaatgca 47640

aagtccccaa ggctgaaatg cacttggttt gtttggctgg agtggagagt gcagggtaga 47700

gagtggcagc gagggaggac agaggttggc caaccacatc acgcaggcca tggcaaggaa 47760

ctgagatttt attctaagca tgatgaggag cattggagag ttttgagcag agtaggcaca 47820

tggtctgagt tactgtcata aaggattatt gtagttgctg agtgaagaac tgacttgggc 47880

aaaagtggaa gccgatggag tagttcggag gcattgcagt gttttggcag aaagatgagg 47940

ataacctata ctagggggtt agcaatggag atagcaagaa gtggtcagaa atagtatata 48000

ttttgagagt agaattgata agacttgctg ttagttgcct gtctgtggag gtgtgaaggt 48060

aattaaggat tcagagatac ctcttagatt tttggcttga gcaactgagt gaatgctggt 48120

gccatgtact gagacagaga aggctgtgag gagagctggg tttgagggaa agataaggac 48180

ttcagttttg catatgttaa gtttgagatg cttattagat atccaaataa ggatgttaaa 48240

taagcgattt tgtgcatgaa tctggagttg aagggagagg tctgggctga agatctaatt 48300

ttgggaatcc tcggcatgtg aatggattta aagcctaaag accgaaagga gtgctcataa 48360

agtgaatgag atagacaaga agacaaaaaa ctgaatcctg gggcacatcc atggacactc 48420

actgggagat ggaggcaagc tataagattg aaaaatggag agaagtagga aggaaaaaga 48480

aacagaggaa tgtatcctgt tctcagatta ttcttttcaa aattatacag agcaagcgcc 48540

catctggtct gttaaaagta gagtagcata ttatttagat gatagggtat aagtaattca 48600

tttattctga tggtccctgt gctccaccta tgggcaagag tagaacttaa tgcccttaga 48660

acattgattt ttgtgtaccc tattttagaa atttcttcac aactgccttg tcaataaaga 48720

tgacttgtgt accatgtctt ttgtccatgc cttcttttaa tatggtggta tttatgtggg 48780

acactcctat ggactggaag gtttgaatca aatgtctctg ttttcttaaa ggctcaagat 48840

tattatattc cctccaattc ctgatcctgg caagattttt aaagaaatgt ttggagacca 48900

gaatgatgat actctggtaa gaacagattt catggggctt gaaatgtttt tttttttttt 48960

tttttttgga agctatggca ttttttaaaa aagtgtggta aggtatccat aacataaaat 49020

ttattatttt aagcattttt aagtatacag ttcaggctgg gtatggcagc tgacgcctgt 49080

aatcccaaca ctttgggagg ctgaggcagg aggatcactt gagcccagca gtttgagacc 49140

agcctggaca gcatgccact gcacccagct aatttttata tttttttgcg agatctcatt 49200

tccacaaaat atataaaaat tagccgggtg cagcggcatg cacctgtggt cccagctact 49260

caggagattg aggtgggagg atcccttgag cccaggagtt caaggttgca gtgagccatg 49320

atcatgccac tgcattccag cctggatgac agagcgagac tctctaaaaa ataaaatata 49380

tatgtgtgta tatgtgtgtg tgtatatata tatatatata tatatacaca catacagttt 49440

agtgtcatta aatacattta taagttgtac aaccatcgcc actatctctt tccagaaatt 49500

tttcatcatc ccaaatagaa actgtggctg ttaaacaata attccccatc ccctcattcc 49560

tcccacccct ggtagcctct attctacttc ttgtctctat gaattaaata ttctagatac 49620

ctcctatatg tgaaatcatg caataatttg tccttttgtg tctggcttat ttcatttacc 49680

ataatgtttt aaaggttcat tcatgttgta gcatgtatca gaatttcctt ttgaaattgg 49740

ttcttctaaa gtacaattta tttttaaaaa tttgaaggag agctgattta aaatatagta 49800

tctgaggcca agcataccag ttaaaaagtc ttcagaatac tttgctcatc ttatataact 49860

gggctccatt ctctctctgg ttaggagaca tattttatca aaacatgcag gaagaagggc 49920

ctcctttatg aagctagcct aatgagcagg ccttctgact cttggataat ttaaaaaact 49980

tgatgtgggg aaatataaac atagggtaag caattgaaat gcattgtgtt catgttgttt 50040

cttggaaggc tagactccaa agatatagaa atggagcagc cctaccccaa ctgttatagt 50100

tggtttcctg cagattctca tgctagaatt tgtggccaga aaacctaaga cattatggga 50160

acaaaagggt ttgaggcttc taactttagt tggatttatc atccacccta ctgaaataat 50220

aaagacacta aatgaaataa atattattat cattgtgtat atcttaaagg aataataaca 50280

agcatagggc agttaattta gggaagtagt actgcatagt agataatagc ctgatttctg 50340

gagtcagatt gtctggattc atatccctgc tgtgtcattt accatctttt tgaccttgga 50400

taattttctt agcttctctg tgactcaatt ttttcattag taaaatggag gtaacaatag 50460

cacctgcctc atagtgttgt tatgtggatt aacttaatac ccaacagtta attgaatgga 50520

aactgcttag cctacctggt accttgtaag tgcttaataa gtgttagtga ttagtattac 50580

tattgataat aatttatttt tatctttaat tcccaaataa ttattgtaat aatgggtgat 50640

tctcaatggt agtgatgata tttggttagg gaaggaaggc agagaggcaa atatgctatc 50700

agaagaggat tcatttggat cactttttaa ccaggtcatc ttcacagaca gtagataact 50760

ctgccagacc ctgggagtca gggcatgtag acccagaagt taggtacctg atgaggaaat 50820

tgtatgccgt atctggagcg agtatgatag ctatggttct ccaactttag ttgagtcaca 50880

gtcaactgga gtgcttacaa gaaatacaga tttctggacc ctacttccaa ttccctgacc 50940

tgacctgatt tggcaggtca gggacgagtt ctaggactct gcatatttaa taggagccca 51000

cagatgatcc ctgtcctgct aagagagtct gtggaccaca ctttgaaaat cagtgataaa 51060

gatatttacc cctgtctctt acttcatttc accagctgtg atagatgctg gaggttggga 51120

attgtacaag taccctaaca actttcacca gctattgaaa gtctactatg tgtcagacaa 51180

tgtattaagt gctttgccta tattacctta ttgaatcttc ataaccaccc tatggagaag 51240

atgctgttgg tattatttta cagatgagga aatggagccc caatgaggtt aagcaattta 51300

gctgcagtca cacagctagt tggtggtaag gtagaatttg acccagggct gtcttaatgc 51360

tagacttctc ttggtctttc tactaggctg cattgcctct tgtaagatag aatgcttgtt 51420

ctgaagaacc tcaccatctt ctaagacatc tgtatgacag aagtagaggg ttggcatagg 51480

ggatttctga gaaaaagatt tagggttgta ttatgaatag ctttaaatgg gaagatattg 51540

gcatgtcttg tatatattaa tggcactttt catgcactgc tggtgggcct gtaaaatgat 51600

agatatcctt tctggagggc aaactggtaa aatgtaacag gaatctttga gcctatgatt 51660

atacttttag gaaccaattt tgaggaaatg atccaacaat ggcacaagag tggtcaatgc 51720

ggcatttgct tttaacatga aaaattagaa gccaatgaaa cataacaatt gagaattagt 51780

ttaaaaaaat atgttaggtt ttcaaagcat ttaatgttat aacaaaatac tcaaaataca 51840

ggagtaagaa acttaccata aacatgtagt atattacaat cataattttt atcaaaaatg 51900

cattaatata tacatagaac gaggggaagg aaatgggtcc gaatgaatga tcatttctag 51960

gcaaagggat tgtgggtaac tgatattact ctctctctac ctttctaaaa tttccagatt 52020

gcctatagtg agcatgtact acttttataa taaaagaaac cctgtacaag tcattaaggg 52080

aaaaagaaaa tactgtagtc atccaggtct gagagataat aggaacttga acttaggtgg 52140

tagcagagga gattgacgta agagacatta ttaaaggaaa tcaactcatt atgacttggt 52200

gagtctgtgt gggtgatgag aggtagggag agtaaaaaat aatttcaagt tttcaagtct 52260

gagtaataga gtgaatgatg gtaacattga cagcaaaatc agatcaatta tctttctcag 52320

agatgggaaa attgcttttt tcaatttgct gtctagagga taatatgttg tttttgtccc 52380

gatgagtcag ataattctgt aatttggtga cgcaaatata ctgacaatat ggaccattaa 52440

aagcacaagg tatttttgtt ttgtccattg cagaccatta aacaaaatca gttctttaaa 52500

actaaaagta taataaaaat tccagatatg gggaaggtta agagaacaaa tttttatgtc 52560

ggcttttaac caaatgaaaa aaatctgctt tcttcacatg gcatcaattc tcacaaattg 52620

aactgcatat gctgcatact cttttacttc tagcatcatt gaattattga atgaacatag 52680

ccctagaggt catctaatga ggtggatgtt gacatgttta attatttccc tttaattata 52740

aatgttcaaa actcgagatt ctcactcatc tccctctctt tagagatggg gacatgagga 52800

gccacagaga agtttctcaa ggactgtcac tgtgttaagc caatcttgta ttgctataaa 52860

gacatacctg aaactgggta atttataaat aaaagaggtt taattagctc atggttctgc 52920

aggcttcaca ggaactgtgg tgccagcatc tgcttctggt gagtatctca ggaagcttac 52980

agtcatggtg gaaagtgaag gggagcaggc catcttacgt ggtgagagtg ggaacaagag 53040

ggggtaggag gtgccataca cttttaaaca gctcttatga gaactcactg ttgtgatgac 53100

agcattaagc catgagggat ccacccctat gacccaaaca cttcccacca ggccacactt 53160

ccaacgttgg ggattacaat tcagtatgaa atttggcagg gacatatatc caaactgtca 53220

gtcacataga gaggcaactg caaaacctgg actagaaccc atatagccca aagttaccaa 53280

cttccaatcc aaataaataa tcttgtcctt ttttaaacac tgtcttcttc cacactgcac 53340

cattaatagt tgttcttata aaaacttatt tagttatggc catgaataag ttaaatcttc 53400

agtatagttt tccttaaagt tactcgtgtt ttgatgatgt tttgttcatt actatagtaa 53460

tgttttggcc aacagcattt ctttctgttg ctttgctatt tgccttttcc ttgtgactaa 53520

ttgggcttct ggccatattg aggggaggta aggggagttg cagagggaat tgcttctcct 53580

ctctatctgc atttctctta tccataaaaa aaaaatctgt cctccccctg cttcttggaa 53640

accaattctt attttcagca ttgtgccatt tttatattct tgttcagaaa ggagcttttt 53700

aaacatgggg ataacatcca aatactgctg attgttattt tttacttcag tgtgtatctt 53760

ttttttttcc tttttgagat ggagtctcat tctatcactc aggctggagt gcagtggcac 53820

gatcttggct cactgcaaac tttgcctccc aggttcaagt gattctcctg cctcagcctc 53880

ccgagtagct gggactacag atggtgccac catgcctggc taatttttgt aattttagta 53940

gaaatggggt tttgccatgt tggccaggct ggtctcgaac tcctgacctc aggtggtccg 54000

cccgccttgg cctcccaaag tgctgggatt acaggcgtga gccaccacgc ccagcctcaa 54060

tatttatctt taaaagctaa gaatgctttc ctacatagcc aaaatagcat tactgtactt 54120

taaaaattat taattcctta atttcatcta atactcagtc catattcaag tatttccaac 54180

tgcccaaatt gtcaggagac agatattatc cagtcatctc accactgacc actaagacag 54240

tggttgagat tgaccactat atttaaggtg atacaacagc atgtcccctc ctgccatctg 54300

cacttatttc cccattgctt caacacagat aatctgtgta gtgttatgct agcaccttgt 54360

caagtcttca gctaatgctt tttacatcag ttgatatcct tgcttgaatc agtaatttcc 54420

ttaggggttg ggttgaatat tacagttttt ctctttctat catttctaca tttacttttc 54480

tttttttttt cttttgagac aggatcttgc tctgtcaccg aggctggagg gcagtgatgc 54540

aacctctgct cactgtagcc tcgacctcct gagctcaagc agttttcctg cctcagcctc 54600

ctgaatagct gggactatag tcacgtgcca ccacactcag ctaattttta aatttctttt 54660

gtagagacaa ggtctcacta tgttgcccag gctggtcttg aatttctagg ctcaagcgat 54720

cctcttgcct cagcctccca aagtgctggg actacaggca tgagtcaccg tgcctggccc 54780

atttctacac ttattagctg acaggtatct ataaagcgct gctttcagtc atcaactgag 54840

ctctttagtt gttctgaaat tcagttccta ctaaaaaggc aagatgcata tttaatcatt 54900

tccctttatt tatccattaa ggtgaggagt tggattaata gacacctcat agtaccaagt 54960

tattcctctc cataccacca cattcaactt ggtgtttttt tgtttttgct tttttttttt 55020

gagacagcgt ctcactgtgt tgcccaggct ggaatatagt ggtgcaaaca tggttcactg 55080

cagcctcaac ctcctgggct gaggcaatct tctgatctca gcctcccaag tagctgggaa 55140

tacaggtgtg cactaccacg cccagctaat aatttttttt tttttttgta tgaatgggct 55200

tttgccatgt tgcccaggct agtctcaaac tcctgggctc aaatgatctg cctgccttgg 55260

cctcccaaag tgctgggatt acaggcatga gctaccgtgc tgagcctcaa cttgttcttt 55320

ttcaagttgc aatgatttta aggtgtggag atctacaatt cattttgtat aactttctgt 55380

tttgaatgat aatctgaaga gagactgggg catgaaatag gtagacttgt ataccaaagg 55440

ccagtgtttt tgcatggggc taaggtacat gaagctcact tcacttctgt agacaatcca 55500

ctctggaaaa tatcttaacc aaatcttgaa agtaatctta agttgagact tgatatttca 55560

ttgtactacc aaatttatgc ctttaaatta taaaaaccta tataacatgc cgtttttcta 55620

ggctcacata attaccaact aaaagccagt tgtgtaagcc ccctaatcac tgattttctt 55680

catctatgat gtttttcatc tttggccatt actctgttgt tgattacttt gtttttaaaa 55740

ttggcagtaa ggattgaatt ttgggctggt aattgtgtgg atttgtttat ttttttctac 55800

aagtggtttt aaaattagac aggcatataa ttcacagaat tgaataatag gaaagagcca 55860

ttgcatttat ttcttttacc ttacatggac atattcatag aagcagtact caggaaaagg 55920

ccactgtgtt gataaagggt ggaaatgaga gtaattcaaa ttttggggat gaaaaaaggc 55980

aatcagaaag attaatgtat tctgagaagc cctttctgtt gtagtgttca cctcattaaa 56040

acaaattgct tttttcttgt ccagcacagc caagtgttgg tctaatgacc ggaaacaaaa 56100

gtaattttca gggtgcattt ttaatagtta tacagttttt atttgtgaaa aatccatatt 56160

acacacacac acacacacac ttcaccacta tacattcatg cattttttaa attaaaactt 56220

tttttttttt tctgatcact tgggaggatg aggcaagcgg atcacctgag gtcaggagtt 56280

cgagaccaac ctggccaaca tggtgaaacc ccgtctacta aaaatacaaa attgaccagg 56340

catggtgatg catgcctgta atcccagcta cttgggaggc tgaggcagga gaatcgctta 56400

agcctgggag gcggaggttg taatgagccg agattgagcc actgcactcc agcctgggca 56460

acaaaaatga gactctgtcg ccaaaaaaaa aaaaaatata tatatatata tatacgtata 56520

tatgtatata tatgtatata tatatatata tacgtatata cacatatata cgtatatata 56580

ttcttttaaa agcaatatac atttagtata gacaaattag aactacagat agtcaagaga 56640

gaaagacaaa atcactctta atcctgtcac ccagatataa ctatctttaa tggtatgtga 56700

acaacatagg gaaactgaaa tacctttagt gataaatgga gtaatagcaa ccacaataat 56760

aatagttcat acttatggaa cctttactat gtgtcttaat aacactgtca tattgaattg 56820

tatttgaatg taacaaatga gaattaaaag aatttctgaa cttttgatat aaaataaatt 56880

ttaatgatga ttgaaaagat atttttccat atgctctccc catttgctcc ccatttttat 56940

agttgttgaa catctccatt gtgtcctcat atagagccct catttagtct tacttcagtg 57000

tgtggagata cacttgatgc tcactaccat tttttatgtc aacattgctc cagtcatttg 57060

ggttgtttga agctggaaag ttttctcagg aagtattcat gggaccggca tttcctgaat 57120

tatttacaaa ttgatggaat ttgctacatt tatcattgaa agtcattttg gcaaaatata 57180

aaatccatgg ctcataggtc ctgcgttgcg tatcttaaat gtgtcatcca ttttcttctg 57240

gcataatatg ttttgtcaga gtcagcagac aatataattt tcctcctgta ttaaaacact 57300

tggtcttttt ggctggttgt caaatgattt ttttcttcaa agtccaataa ttttaccaga 57360

atatgtgttg gcgttgtgat tttttgcagt gtatggtgtg ctttctcagt atgtaatttt 57420

agatcttttt ttttttaatt ttagtaaaag tttcttcact tacagatttt agttattctc 57480

ttcccatgtt ttctttgacc tttcacatct tttttgtttt ggcacagggt cttgctctgt 57540

tggctgggct ggagtatggt ggcaccatga tagcttattg cagactccaa catctgggct 57600

caagtgatcc tcccaactca gcctcctgaa tagctgggac aggcatgtgc caccatgcct 57660

ggctaatttt taaatttttt ttagagatgc agtcttgctg tgttgcccag tctggtcttg 57720

aactactggc ttcaagttat tcccccacct ggaacttcca gagttcagga attataggtg 57780

tgagccgctg cacccagccc ttccagccct ttcacatcct cttgaataat tttcatccac 57840

cttcatttca tttttgttaa aaaatcctac tttttaatct atttaagatc ttatctgatg 57900

atatgttgtg tttatttgcc ctgtgtttct tctagcttag tcttcaattt tgaaatgatt 57960

attttccttt acttttattt tgttttattt tttgagatgg agtctcactc catcgcccag 58020

gctggagtgc agtggtgcga tcttggctca ctgcaaactc cgcctcccag gttcaagtga 58080

ttctcctgcc tcagcctccc aagtagctgg gattacaggc acatgccacc atgcctggct 58140

aattttgtat tgttattaga gatggggttg caccatgttg gccaggctgg tctcgaaccc 58200

ctgacctcaa gtgatccacc caccttgacc tcccaaagtg ctgggattac aagcatgagc 58260

cactgcgccc agccttcttt tacttttaat tcttctgtga gttttgtcat ccaatttctg 58320

agttgtttat attgactttt aatatattct tctacattga ttactttttt tatatcatgt 58380

aactttttaa atatacttca gctagttttg aaatattagg ttgtagtttt catctatttt 58440

gtggatatat cgttctgtta tgttttaatt ctgtaagagt attattctgt ggctcatttt 58500

tattttattc tttaatttta aacatttttt attttaaaaa taattgtcac tttgggaggc 58560

tgaggcagac agatcacaag gtcaggagtt caagaccagc ttggccaaca tagcaaaacc 58620

ccgtctctac taaaaataca aaaaaattag ccaggcttgg tggcgggtgc ctgtaatccc 58680

agctacttgg gtggctgagg caaggagaat cgcttgaacc tggaggcaga agttgcagtg 58740

agccaagatc acgcctctgc actccagccc gggcaacagt gtgagactcc atctcaaaaa 58800

aaaaaaaaat tattattttt gtagaggtgg ggtttcacca tattgcccag gctggtctca 58860

aattcctggg ctcaagcaat cctcctgcct tggcctccca aagtgctggg attataggcg 58920

tgaacaacca cacttggctt ctgttgttca tttttgtatg aaatcagttt cctgaacttt 58980

tagaaggagg cttgattgag acaagttgtt tctttcccta attttatggc tgtagagctc 59040

catcctctaa tgaattacat tctgaaatct cctggccctt tctgtctacc tttctgcttt 59100

gatctaggct ttcttttttg tcgctcttgt ccctgtcctg actaatttgt aatccagttt 59160

caccagtttc tcctcagagt gcagctttgc cctggaaggg attaaggatt agttttgagt 59220

ttctggaggc ccaggctgct ttcagtctag accacggact ccttgcactc aatcactgtt 59280

tgaaaatgca aacccttccc tttatcagtt gttcataaat ttgcttgctg aagttgagtt 59340

attttacagt tctcagatct ataggatgcc atgttgagtc cgtttatata ctcttccaca 59400

gcttatacca ttttggtcct gtaactatct ctaatttgtc cacattcact cttactttga 59460

ggttcatggg ggaaatgttt tcatcttgtt ttgttgtaga tttcatcttg tgttggtaga 59520

ttttggattt actttcctat tgggggaacc agcccccaat atttcaacgt aagttctttt 59580

ctattttccc taagtgtcag ccggtctgat aaataaaggg aaagagtaca aaagagagaa 59640

attttaaagc tggatgtctg ggggagacat cacatgtcag caggttccat gatgccccct 59700

aagccgtaaa accagcaagt ttttattagc aattttcaaa ggggagggag tgtacgaata 59760

gggtgtgggt cacagagatc acatgcttca agggcaacaa aatatcacaa ggcaaatggg 59820

cagggcaagg tcacaaggcc agggcgaaac tagaatttcg cctggttcct ggtcctgctg 59880

tgcatgcatt gtcattgata aacatcagga aacagggttt gagagcagac aaccattctg 59940

actaaaattt actaggcaga aatttcctaa tcctaataag cctgagggtg ctgcaggaga 60000

ctagggcgtg tttcatctct atctatgact gcataaggca gacactccta gagcagccat 60060

tttagaggcc tcctcctggg aatgcattct tttcccaggg ctgttaattg ctaatattcc 60120

ttactgggga aataattcag tgatatttct cgtacctgtt tttggcaata agagaaatat 60180

gactgtcctg cccggctcca ggcagtcaga cctaatggtt atctcccttg ttccctgaac 60240

attgctgtta tccttttctt ttttcaaggt gcccagattt catattgttc aaacacacat 60300

gctttatgaa caatttgtgc agttaatgca atcatcacag gggcctgagg cgacatacat 60360

cctcagttta tgaagatgac gggattaaga gattaaagta aagataggca taggaagtta 60420

taagagtatt gattggggaa gtgataaatg tctatgaaat cttcacaatt tatgttcaga 60480

gattgcagta aagagaggcg taagaaatta taaaagtatt aatttggaga actaataaat 60540

gtccatgaaa tcttcacaat ttatattctt ctgtcatgac ttcagcaggt ccctccgttc 60600

ggggtccctg acttcacgca acactttcct atttatattt ccaacttgtg tttttttatg 60660

gtgatatttg ggaatattca aaaacgatgc tgctaccacc atcttcttcg cagaattttt 60720

ctgcaaacta tagaccaagg ggcaaattct gcctgccact tgtttttgta cagtctttaa 60780

cctaagaatg gtttttgtgc ttctaaatag ttgggaaaaa aatgtaagaa tactatttca 60840

tggcacatga aattaagatt tcagtgtcat acagtttcgt tggccaggcc cattcattta 60900

tatattgtct gtggctgctt tcatgctaca acagcagagt tgttttatag gcacagagac 60960

tatatggccc ccgaagctga aaatacttct tatctagcca ttttcaggaa aagttagtca 61020

accctggtcc cctataatat agtttttagt ggctaaataa tattccattg tgtgcacata 61080

ttataatgta cttaacaaaa ttcctattgg catttggatg aatcctaaat ttcattgtat 61140

aggcatcatt tcacagctaa gtataagcat atgtattaat attttattac tgtcttagga 61200

taaattgtta caagccaaat tgcatgatca agggggaggg tatatgtatt ttaaactctt 61260

tgactgatac aggctgagta ttcctaatct gaaaatctaa aatccgaaat gctcccaaat 61320

ctgaaacttt ctaacatgac gttcaaagga aatgttcatt gcaacatttg gaatttcaga 61380

tttttgcgtt agtgatgctc agctggtaag tatatagtgc aaatattcca aaagctgaaa 61440

cacttctggt cccaagcatt ttgcataagg gatattctac ctgtattgac aaattgtcat 61500

ccagaaaggt tgtacaaatt aaattcccat taaggaaact ggctaaataa gttatggtgc 61560

attcatgcaa tgaaatatta tgtagaggtt aaaatgaggt agatcacttt tactgatgta 61620

taaaaatata acatacactg ttaggtgaaa cacccaggtt acatatggag agtatgattc 61680

ctttgtgtta aggaaactgg ggcattaaaa tgcacaaaca aattttttca taagaagcat 61740

agattacaac taagttgtgc cagttcatat acctaccagc agtatgttcg actggttgtt 61800

tgaccatact tttgccaata tgatattacc taattttaaa attcttgcca agttaataga 61860

tgagaaaaga gcgtcttaat tttacagtca ggttttgtac tttttcccaa gtttattggc 61920

ttgaattaat tcttttgtga aattcttgtt ccacgtccat ttttccttgt tgtattcatc 61980

ttttggtggt ttgtaaagct ctttacgtat tagatattaa ccctctggct tcttatccat 62040

gttttccttt gtattgtatt tagggtgatt aatgatgtac aaaaggtttt tcctttttta 62100

tgcagtcaaa tcttcctgta tttttaggtg taaagagccc tttccacctt agatttatat 62160

agttgttcct ctacatattt ttctagtact tatttgattt tttttaacat tgagatcttt 62220

aatccatcag ggatttattc atgtaggcta tgaggtaggg tttagcattg ttttcaaatg 62280

cttatattac ccacttgtct tagtgtcatt tattaaataa gccatttttt caaggttata 62340

taattttttc aaattgtttt tattagaaat ctatacattt cacatgtttt aaaatcaagt 62400

ttgaggtgta atttacaata aaatttactc ttttttgagt gttcagttag aagaatgtta 62460

gcaaatatat gcaattttgt aaccactgct ccattaaaaa tatagaatat ttccatcatc 62520

tcctaaaagt tatcttgtag tcctttgcag tcaaccccct ctgttaatcg ccttgcaaac 62580

actgatcagt tttctgtccc taaagtctta caagatgtcc tataaatgaa atcacacagt 62640

acatagcttt ttgagtttgg cttcttttac tcagcataat gtgtttaaga gacatccaca 62700

ctgtggtgtg tgtcagtagt tcatttcctt ttattgctga gcagtattct ataatatggt 62760

tgtaaccaca gtttatttaa ccattcactt gttcaaggac atcagagttg tatctagttt 62820

ttaatctatt attagtgaaa atgctataaa cattcatgta gaagtctttg tgtgacatgt 62880

tttcatttct cttgggtaaa tacctgggag tgggatttct gggtcatgtg gtaagtgtat 62940

gtgttatttt ataaccaagt gtcagactgt tttccataat gattgcacta tcttattttt 63000

accaacaata catagagatc tagtttctct gcgttcttat gagcgcttgg aattgtcggt 63060

atttttaatt ttagccattc tagtaggtgt gtcagggtta tataatttgt ttagaataat 63120

ttgcatattt tgagcagtgt ttcccaactc atcccctcat ctcttaactt gcccagtcca 63180

tcttcagttg cacaacctga ggcagctctt gtttctgttt ttcttctttc catttctttt 63240

ctaagaacat ctgtgcagaa aacgagctta gacataacct aatgcagggt ttggtagact 63300

tttttctgta aagggccaga taataaatga ttttgttttt gcaagccatt cagtctctgt 63360

cacaactcaa ctctgccatt ataacatgaa tgcaggtgaa ggcaatatga aacaaagacc 63420

agcattcagc cggatttgac ctgtggacca tagtttgctg acccctggtt tagtataaca 63480

actttatttt acagatgaga ggaccgagct catgtttcac cctttcctct gatttacaag 63540

taaaagtaaa aaaaaggcaa ggaccaggat attggctttt accataagag acatgcttga 63600

attgggttca agataagtgt gctttatgtg taatttcctg ttctagcaaa tgatgttaga 63660

tgaagctctt aatatagggt ctttcggcaa tttcgattgc agtaagactt taggaacatc 63720

ttatgtttta ccattcatct caatgctact tgactttttg aacctattat gtctctggag 63780

caatgagcat taagaaaata aaggtaataa aggtctcagc aaaacagttg gagtaagaac 63840

ggcatgacat ccttttattc ttcttaatag attttatttc cagtggtgga aatgtccaaa 63900

tttgtttatt tctggagata gtcattgtac atcatgtaac tatctcatga ttcccacctc 63960

catttaatat taattcctga ttgttctgtt tcattaatca ttcagataga taaagattct 64020

taggtcaaaa aaaaaaaaaa aacaggaaat cataccccta cggttccatc cacacaaacc 64080

taaaaataag cattggcact aagtactcag ggaaagaaca ctgtttagat atgtcattgt 64140

gtatgtgttt ttttcctccc ttctagcact ggaagaagta cgacatctat gagaagcaaa 64200

ccaaggagga aaccgactct gtagtgctga tagaaaacct gaagaaagcc tctcagtgat 64260

ggagataatt tatttttacc ttcactgtga ccttgagaag attcttccca ttctccattt 64320

gttatctggg aacttattaa atggaaactg aaactactgc accatttaaa aacaggcagc 64380

tcataagagc cacaggtctt tatgttgagt cgcgcaccga aaaactaaaa ataatgggcg 64440

ctttggagaa gagtgtggag tcattctcat tgaattataa aagccagcag gcttcaaact 64500

aggggacaaa gcaaaaagtg atgatagtgg tggagttaat cttatcaaga gttgtgacaa 64560

cttcctgagg gatctatact tgctttgtgt tctttgtgtc aacatgaaca aattttattt 64620

gtaggggaac tcatttgggg tgcaaatgct aatgtcaaac ttgagtcaca aagaacatgt 64680

agaaaacaaa atggataaaa tctgatatgt attgtttggg atcctattga accatgtttg 64740

tggctattaa aactctttta acagtctggg ctgggtccgg tggctcacgc ctgtaatccc 64800

agcaatttgg gagtccgagg cgggcggatc actcgaggtc aggagttcca gaccagcctg 64860

accaaaatgg tgaaacctcc tctctactaa aactacaaaa attaactggg tgtggtggcg 64920

cgtgcctgta atcccagcta ctcgggaagc tgaggcaggt gaattgtttg aacctgggag 64980

gtggaggttg cagtgagcag agatcacacc actgcactct agcctgggtg acagagcaag 65040

actctgtcta aaaaacaaaa caaaacaaaa caaaacaaaa aaacctctta atattctgga 65100

gtcatcattc ccttcgacag cattttcctc tgctttgaaa gccccagaaa tcagtgttgg 65160

ccatgatgac aactacagaa aaaccagagg cagcttcttt gccaagacct ttcaaagcca 65220

ttttaggctg ttaggggcag tggaggtaga atgactcctt gggtattaga gtttcaacca 65280

tgaagtctct aacaatgtat tttcttcacc tctgctactc aagtagcatt tactgtgtct 65340

ttggtttgtg ctaggccccc gggtgtgaag cacagacccc ttccaggggt ttacagtcta 65400

tttgagactc ctcagttctt gccacttttt tttttaatct ccaccagtca tttttcagac 65460

cttttaactc ctcaattcca acactgattt ccccttttgc attctccctc cttcccttcc 65520

ttgtagcctt ttgactttca ttggaaatta ggatgtaaat ctgctcagga gacctggagg 65580

agcagaggat aattagcatc tcaggttaag tgtgagtaat ctgagaaaca atgactaatt 65640

cttgcatatt ttgtaacttc catgtgaggg ttttcagcat tgatatttgt gcattttcta 65700

aacagagatg aggtggtatc ttcacgtaga acattggtat tcgcttgaga aaaaaagaat 65760

agttgaacct atttctcttt ctttacaaga tgggtccagg attcctcttt tctctgccat 65820

aaatgattaa ttaaatagct tttgtgtctt acattggtag ccagccagcc aaggctctgt 65880

ttatgctttt ggggggcata tattgggttc cattctcacc tatccacaca acatatccgt 65940

atatatcccc tctactctta cttcccccaa atttaaagaa gtatgggaaa tgagaggcat 66000

ttcccccacc ccatttctct cctcacacac agactcatat tactggtagg aacttgagaa 66060

ctttatttcc aagttgttca aacatttacc aatcatatta atacaatgat gctatttgca 66120

attcctgctc ctaggggagg ggagataaga aaccctcact ctctacaggt ttgggtacaa 66180

gtggcaacct gcttccatgg ccgtgtagaa gcatggtgcc ctggcttctc tgaggaagct 66240

ggggttcatg acaatggcag atgtaaagtt attcttgaag tcagattgag gctgggagac 66300

agccgtagta gatgttctac tttgttctgc tgttctctag aaagaatatt tggttttcct 66360

gtataggaat gagattaatt cctttccagg tattttataa ttctgggaag caaaacccat 66420

gcctccccct agccattttt actgttatcc tatttagatg gccatgaaga ggatgctgtg 66480

aaattcccaa caaacattga tgctgacagt catgcagtct gggagtgggg aagtgatctt 66540

ttgttcccat cctcttcttt tagcagtaaa atagctgagg gaaaagggag ggaaaaggaa 66600

gttatgggaa tacctgtggt ggttgtgatc cctaggtctt gggagctctt ggaggtgtct 66660

gtatcagtgg atttcccatc ccctgtggga aattagtagg ctcatttact gttttaggtc 66720

tagcctatgt ggattttttc ctaacatacc taagcaaacc cagtgtcagg atggtaattc 66780

ttattctttc gttcagttaa gtttttccct tcatctgggc actgaaggga tatgtgaaac 66840

aatgttaaca tttttggtag tcttcaacca gggattgttt ctgtttaact tcttatagga 66900

aagcttgagt aaaataaata ttgtcttttt gtatgtca 66938

<210> 33
<211> 42218
<212> DNA
<213> Homo sapiens

<400> 33
gcgagcgcag cggagcctgg agagaaggcg ctgggctgcg agggcgcgag ggcgcgaggg 60

cagggggcaa ccggaccccg cccgcaccca tggcgcccgt cgccgtctgg gccgcgctgg 120

ccgtcggact ggagctctgg gctgcggcgc acgccttgcc cgcccaggtg ggtgactcgc 180

gcggcccacg ggggacagcc gccccgcatg tccacccggc tggtgcgcag ccttcgggtg 240

cccgggccgc gctctcccgg ggcgctgtca cgggctggga ggctgggagt cccagtcgcc 300

gccccccatc cgcatcagac acgcgcgcct ctggggaccc gctggggact ccgggcccgg 360

cacacgtgcg ctcggggcac aactctggcc cccgaagccc ctcttacccg cgtcgtgaca 420

ggggcgcacc ctggttcctc cgcgagcgca ggccggggca tttggggctg agcagcgggt 480

cctgggcaga cccccgctag acccggaccc cctcttccct caccccggcg actgccgcga 540

cagcccttcc ctcccacgcg gtggagaagg ggctgtgccg ggcgctgccc ccactgccca 600

cgccgggcac cctcttcaaa agacttcctt ttcctctggg cgacggtttt ccgatttctt 660

agaaatacca gggtcctgtg tctgaagagc aggaccaggt gtgtgtcagg tccactgagg 720

gcacagctgg agggcgagct gcctgtctgc ttcctccgat gcgtccgggg gctactgcag 780

agtctgtttt ctgcggcctc tggggacagg gcaagcgagg gaggtctggg ctggtaatcg 840

agcaaggcaa atgaagtctc cgggggccgc aggaaaatgg agacagcaga aaccctaggg 900

ctcctattca atatgaacgt cactcccaca gtagcaacgg gcgtacaagg gactgtgcac 960

cctttgcctg tttttgactg tctcctggtc tggtaacata ggataacccc atttttgttt 1020

tactgtctcc atggtttggt aagataagat aaaccatttt ttagagaagg aaaactgagg 1080

ctcagagaac ttgtatttca tggaagggtc tggggggaac tcttcctcca aggggggcct 1140

ctcctgcctt cggcccctgt gccctgaggc tgcggggaag gtgggcgttc atccttcctc 1200

agagccgccc ctgatggaca catggcccat ctgcccccat ctgacatctt gaccgaggag 1260

gaaggtggga ggtggcactg gcttctgtgg tgacataagg tgacttgttg atctgaggcc 1320

agagagtcca gttcttgtgg gtggaagatg ggggctgtca gactagaatt gactcaccga 1380

cagtgggtgt caggagtggg tcctggagaa ctgccccaga gtaagtcctg ggtggccttt 1440

gggtcaggag agtccccact ctagggccga gtgtgaatgg ggacgaccgt ggtccccagc 1500

ttgactttgg ggtcatgcca tatactctgg cctcagaggg ggtcttcctg ccgctgagct 1560

tgggcctgcc tgggtgaact ctggggtcat gtgagcccct ggatgggaga ggggtgtcag 1620

gcacagggct cagcaggaca ccaggctgca gtcctggagt agctggagga ggttccctcc 1680

cacttccttc ccttcctgac cagctcctgc ggcagctcat attccctcct ccctcatccc 1740

tgctccccac gaccccaccc ggactattgc cgcagccttg tagctggtct cccggccctg 1800

ccccctctcc cgctgatcct tggcagagag acccttctca agcatgaggc acctctctgt 1860

ttgcctgggg agcctcctgt ggctcccaat ggttgaggca gaaactcccc tggagatccc 1920

aggccagagt aaggtacctg aactcttcct cgcgcctccc tgcacatgtg ctcccccgcc 1980

tctgtagaac tgatgatggt catcgtgtgt gtcattgtgc aaaaagcatt gtccgtgcca 2040

tcccctctcc aagggacact tcttacccct atccttcaag gccttctgca aatgccattc 2100

tctccaggaa agcctttccg cattctcagt tggatttgat cttctcttcg aactcccagg 2160

cccttttgtt ccctttgagg acagattgca ttctgctttt taatctagct gtgttttatc 2220

cccctcagtg gatggaaggt gtcttcaggg ccagaactgt gggttcctct tccttctcat 2280

cagtagcatc gccatagctc ctgatttctg aacacccact aaacaccagg cactgggcta 2340

ggtactttgt cacaaaattt cactgaaacc tcccttacag caactcccaa gaggtgggtg 2400

gtgccgtttg tctttagaga tgtggaaact gaggcgtttc ccaggatcac agagctgggg 2460

gccggggagc tgggggtggg atttgaatgc aggtctgcct gattcccaag ctcctttcac 2520

tgcaccctcc ccgtgcccgt atctgcactc atgctcccag ccaggccctc acacacagtg 2580

ggttctcagg atggaagtca actgagctgc agaagaagcc cccaggagac ctgagctagc 2640

catggacgca gccgactcac tgccagcagg gcttttgccc cgtttaaaag tcttatttgc 2700

cagctggtat ggaaaacatc attcattcat ttagccattt attcatttga caaagaaatg 2760

ccagtaaacc tggcagtagg tgagtgcttg gtgccctctg atggggatga gcagggtctg 2820

ggctgtctgg ggaggaggat gaggggtctc tgcctgatgg gcctggccca cctacccctg 2880

gaagtcttgg tgctgaggtg ccactatttg cctcctcatc acccagggcc tggggccagt 2940

ttgggttagg ggagaggttg ggggctccca ggctggatgt caaagaggag ttgggtgatc 3000

tggggtacct gctggccctg ggggctcttc cctccttcca ccacgtctct gtctctttgc 3060

cctctgtctg aaatcccagt ggggaccctg tgtgttgact gagggtgtgg tctgttggtc 3120

aggcacgtct tcagagggca atgggtaggt ggccaggcac cctgaggcag ggccattcat 3180

tcattcattc attccccaaa gttcatggac acacctagtc caagcccagc ccagtgcctg 3240

gctctgggga cctcaagatg actccagctc caatcctcta ggagctacac taccaatagg 3300

tcacagacct cacagacctg ggctctgtcc caactgccct tatgagctta gctgagctag 3360

taagcctatg ttctcatctg tggttgtgga aagtattccc tgcctggcct ttgtcacaga 3420

gccatgggtg tgaaaggctg agaacctgcc gggcattctt acgtcagagg tgaccctgcc 3480

atcctggcac ctggcacagt ggctccccgt agcactgtcc ctccccacgg ctgggtgacg 3540

ctttatggtc cttactatgc aatgttttct ggaggagtag cagggacggt tgtgcgtggg 3600

aaggggaaga gttttgccgg agccatggca tgtatgttac acccgactct acaccaactc 3660

acttcttact ttttttctga gatggagtct tgctctgttg ctcaggctgg agtgcagtgg 3720

cgacatctct gctcactgca gtctccatct gcctcttggg ttcaaacgat tctcctgcct 3780

cagcctccag agtagctggg actacaggca tgcaccacca tgcccaacta attttttttt 3840

tttttgtatt tttagtagag tcggggtttc accatattgc ccaggatggt cttggacccc 3900

tgacctcaag tgatcctccc gcctcagcct cccaaagtgc tgggattaca agcatgtgcc 3960

actgtgcctg gcccgttctc acttcttctt ctttttttct atttaatttt tttttattga 4020

gatagagtct tactctgtca cccaggctgg agtgcagtgg cccaatctct gctcactgca 4080

acctccacct cctgggttca agcaattttc ccacctcagc ctcccaagta gctgggatta 4140

caggtgtgtg ccaccatgcc cggctaattt ttcctttttt tttttttttt tagtagagac 4200

cgggttttgc catgttggcc aggctggtct cgaactcctg aactcaagtg atccacctgc 4260

ctcggcctcc cgaagtgctg ggattatagg cgtgagccac cccacccagc cccattctca 4320

cttttaagcc atgcttttct gtcctcagat gtgctgcaca cacacctgct ccagggcctt 4380

tgcacttgct ttccccatca cctccaatac tcctcctgct gacgtctctt ggtctccttc 4440

cctcgcttcc ttctgctgaa gtagacaatc ctgcctgtag gtgctccctg ccaggcactc 4500

tcactccctg acctgctttg tattcctgta taatgctctt gacctattct atttatttca 4560

cttttattat gtcttcccac aagattgtaa gtaccgtgag gacagggact cattcaccac 4620

tgtagactct agcctagaag agggtttttc agtcagtgcc tctgacttta attctttgtt 4680

gtggggctgt cccgtgagct gtaggatgtt tagcgacatc cctggcctct acttattgga 4740

tggcagtagc gccaccctcc ctcgtgacaa aaatatctcc agatattgct aagtgtcctt 4800

tggggttggg ggattgcccc tgattgagaa ccactggcct aggacagtgc ctggcacata 4860

gtaagtgttc aaaaaatatt catctgacct tgaccttgat aatggccctg ttttacagat 4920

gagacggggg tttgaaaagt cagtggtggc agggtggggt tctgtttgcc tccatctctt 4980

ggggtatttt tctgggctcc tcttcccatt gagtggtgtc tgctgaggtt aggggtcctc 5040

tccccaaatt gctcaccccc tcgacatccc tcgtgtagtg agggatgggg ccactcactg 5100

acggtgactc agggccccag aatgcatgtg atttacccag aagcgggcaa ggagggaagc 5160

tggggtttgg ggtgccgggg atgaacagga acaaaggaga ggcgctgtgg ctccccgttc 5220

tggaccctcg tctggccaca gggaagcagc actgatgact ctggggaaag gccccggcct 5280

ctgaatcaga taaacctcaa gtcgctgctc tgctgtttta ctggctgtgt gacctgggca 5340

agtctttcta tccctcggag cctctgtgtt cttatctgta aaatgggagt aatcctagcc 5400

tcacgggctg gctgtcagga ttaaaagtgc tttctaatcc tggcattggc agctatttga 5460

gccattggtg gctttgcagg tctgacccga ctctctgggc catggggtgg tgctcgggcc 5520

taggtgggga cccctgtgga gtcagactgt ctgctctgag ccccagggcc ctgaagcttg 5580

ggtggcagcc ctgcagttgc ccagagttcc tgtctaagtg gctttgttgc ccatcatctc 5640

agaaagcaac ttctttcctc cctgcctttt cctgtctgtg cttctgcccc agtgccccct 5700

ggctgggctc tcaaacccac tgcttgacag cctggtctta ggacactgta ttgtggcaaa 5760

ggctgtgggg tcaggatatc ccctcccctc cctttgccac cccttttagt gagtaacagg 5820

tgctgtgtgt tttggggatg agtgaagggt acatttggtg aagcacagag agagtgcaca 5880

aggcttcagt ttgcaagatg ggcacgggaa gattccaggc catctccaaa acctcttcca 5940

tctccaacct ctgtgctgct gaaggttctg tttacttgtg accctgagaa ggggatgccc 6000

tatgggggct cttctgagat catgctgtcc attttcctgc ctccaagtga cttctaagac 6060

agcatggctc tgaggaagga gcactgactt gggtgttggg agatcacagt tcccatctga 6120

gcaccgtcac tggcttgctg tgctaccttg cttgggttac ttaacctctc tgggcctaag 6180

catccctatc tatgaaacct aacaaccctg gttccaatgc tttcctagaa tattggggaa 6240

gtgtcggctt tgagtatact tgacaaaggc cacagattgg gggtggggta tataaaagtc 6300

tacaggaagg agagcccagg actccccact gtcaatatct tggttcccca gcttctcttc 6360

ttcttgctga cctcaatctc acttgctttg ggtggattga tttcctgact caggctccag 6420

ggccatcaag aattcacggc tgcctttcag ccttcatgaa ggacatggtg gagctcctta 6480

ggcagaactg gggtttgtgg atgtcctgcc tctctgcccc ttggtaccta ggtggcacct 6540

tttcaagttc gggagctaga tcctttcccc ttccccacac tgtaggaaag tccttactgt 6600

aggcaggcgc tgcagagcct gctgtctggg aacccacagg ttcaaggaag ccttgagcag 6660

gaagggcgtt ttcctggaag aagccctttg tcgctggtaa tgggtgcagg ctgctctgac 6720

ccggttctga gtcctgcccc cttccaggcc tagggctttg ggccttgatc acctctgctg 6780

agtagctgac tgcggggctg gggctctgat gctcaggacc cacctctctg ggacccacag 6840

tctttttcca ctgtggcgtg tagtgatgtc acaggtggca gtgatgtcac tgtggtttga 6900

ggtacttggc tgtgagcccc ggaggaggaa gtgtctgttc gctgatgggg ggttggaaga 6960

gatcattgac ttctgcccca agcgtgagcc ccaagtgtgc aggggggagt gcggggggag 7020

ggctgttggc ggcgcatccc agggctctgg ctctgccctt gcatctagcc tgtctttcct 7080

gtgggctgtg acaagccact ctgcatctct gagactccat ttcttcttct tcttcttctt 7140

cttcttcttc ttcttcttct tctccttctc cttctccttc tccttctcct tctccttctc 7200

cttctccttc tccttctcct tctccttctc cttctccttc tccttctcct tctccttctt 7260

cttctgatgg agtctgactc cgttgcccag gctggagtgc agtggcgtga tcttggctca 7320

ctgcaacctc tgcctcccag gttcaagcta ttctccagcc tcaaccttcc aagtaactgg 7380

gattacaggc atgcaccacc acacccggct agtttttgta tttttagtag agatggggtt 7440

tcaccatgtt ggccaggctg gtctcgaact cctgacctca agtgatctgc ccgccttggc 7500

ctcccaaagt gctggaatta caggcgtgag ccactgcgcc cagcttccat ttcttctttg 7560

aaaagtagga ggggttggaa ttgtcaccct ggaggttcat ggttgctaga ttattgattc 7620

tgttgctgga ttcaggagac ctgttagctg gctagttcac agcaagtatt gggtgtttgg 7680

aggggggctg cagagcccct tctcaggccc caggagggcc agctgccctc cctaccccct 7740

cttttggaca ctagtgggca tgttctgctg ggaaaacaga cagtgtaacc tgacttcgag 7800

ggctgcaggc tgaatctctt tcagatgtac tggctccttg agaggcctag cagatctcta 7860

ctctgtgggt ccctttcgga gctggggctc aggtttgacc cagccactct gaccggagac 7920

agcgcagaaa tcaccaggag catttgtttg ttttcctttg ctgtccagac agtggcccac 7980

cgtctgcttc agtctgagcg tggccctgca ctagtgagac tgatgtggcc acaggttcag 8040

agaatcagtg cgcctgagtg ggagagcaga gcgggagggg atttggaggc aggcgggctg 8100

tggacaggga gggggaggga catcttccga gctgtcagcg ggaggccttc ttgaaggtct 8160

ttcaggagca aggccagagg ccgtgggagt gggcggggcc tggggctggg agtcaggggt 8220

ctgaggcctc atcttggccc ggcctctgct tccgggagaa cttggccaat tcccttctcc 8280

tttctgaacc ttggtggccc cagctgtcac ttgagattag caaggacctc cctggtccca 8340

gcaggagtga gtggctttgg gcagggggtg gagtgcagat tgcactgcca cgtcacccag 8400

ctcagaaggc ccaggtgtgg tccagctgtg gacttgattg tgaactcctg gggaagggct 8460

ctggtccttt gcctcagtta gcgggacccc caccagtgcc ctgggtgccc ctgctggccc 8520

ctcctctcgc tgaccccagc cccagtttct ggctgcaact cacagaggcc ccaggctcct 8580

gtggccccat caccagcagc tcctgctgtc atttcctgag cctcgcaagc ccctaatcct 8640

gctccttcct gtcctggccc ccagctccct caagccccac tcctgcatcc cattctccct 8700

ggacatcacc cctcccaccc cttggctcaa tttccttcct gtcgtggctt ggtcctttgg 8760

tcattgccct ggtcttttct tgtccccttc ctgattctgc ccttctccag gcccacacgg 8820

ggaacctcct aaccgctgag ccctgaaagg taggcgggtt taggatgagt ggctacaggg 8880

aggagaatgg tgtggggaga gagatatgga ggaaagaata ttaggggaca ggccgggcac 8940

ggtggctcac gcctgtaatc ccagcacttt gggaggccaa ggcgggcgga tcacttgagg 9000

tcaggagttg gagaccaccc tggccaacat ggtgaaaccc tgtctctact gaaaaaaaaa 9060

aaaaaaatta gccaggcgtg gtggtgtgca cctgtagtcc cagctacttg ggaggctgag 9120

gtatgagaat cacttgaacc ccagaggcgg aggttgccgt gagctgagat cgcaccattg 9180

cactccagtc tgggtgacag aacgagaccc tgtctcaaaa aagcaaaaca aaacaaaaca 9240

aaaaccgaat attagaggac aatgttagga ctgtcccagc tgtcctagct ggagccaaag 9300

gtttgttggg gacagtgaca gcagcacacg aggctgtaag tttagggcca gtttatggag 9360

gagtagtacc aattacattg agaaaactgt agcaactata atttgggagg aattttctaa 9420

atactttaca tgtgttattt aatcttccca tcagccccag gaggtatgtg catttatcag 9480

tcccattcta cagatgaaaa gactgaggtc tatgaactcc tttatttaaa aacaaaacaa 9540

aaccccctca aggctgcctg ctaagagggg aggagtgtgg attcaagcca gcatccttat 9600

tccagtgtcc atgctctcag ccaccacaca cacactccta tcaggagttg gggcagtggg 9660

gagccattat agtttttgag caagagagtg acagggcaaa gatcactgtg gcctcagtgt 9720

gaagcacaga tgagatggag agactggagg cagggaggcc catgaggagg ctgtcaacat 9780

ggttcaggtg acagctctgg gttggtgaag aagcagagaa caggaaggaa ggggcgagag 9840

gcttgaggga ggaggaggaa gcctttggac tgggccgtgg atatagggtg ggaacatagg 9900

aggtgaggag catgctctct ccacctgctt tttgcaacat gcggcctctc tggccttagc 9960

tctgcctgtc ctgagccctg agccctgagc ccaatggacc gattctgttt tttttttgag 10020

atggagtctt gctcttgttg cctaggctgg agtgcagtgg tgtgatcttg gctcactgca 10080

acctctgcct tctgagttca agcgattctc ccacctcagc ctcccaagta gctgggacta 10140

caggcgtgcc ccaccacgcc tggctaattt tttgtatttt ttttagtaga gatggtgttt 10200

cgccatgttg gcccggctgg tcttgaactc ctgacctcag gtgatccacc tgccttggcc 10260

tcccagagtg ctgggattac aggcgggagc caccgtgccc ggccccagtg gactgattct 10320

ggtcagtgtc aaccccattt gtccctggag cttttgcagt cggtcacagg gccagcccgg 10380

tcctggtcac cgctggctgg ttaccccggc tgggttgggc aggagtctgg gcagggctgg 10440

ttgctcatct caggtatgaa attggctgca cccccactag tgtgggggag caggattcca 10500

gggcctgcct ggcgctgcta cccctgtgcc tccagacccc tcgagctcct cccagacctt 10560

tggcttcgtt gactgtgagg tgaaaacaaa agggctgctc tctgtcactt cccctagggg 10620

cgggcagatg ctggcagggc acagagagcc gagggctaag agctgacatt tagcctggct 10680

actcaggaga ctgaggcagg agaatcgctt gaacctggga ggcggagttt gcagtgaacc 10740

gagatcgcgc cattgcactc cagcctgggt gacagggtga gattctgtct ccaaaaaaaa 10800

aaaaaaaaga agagctgaca tttagcgggt gcttcttggg tgcaatgcgc aagagaccga 10860

tgcattaact acacagagtg cccttccttg tcacagctac ctgggggcag gggaacatca 10920

tccccatttt acagaagagg cctttgaagc acagagatta actgacgtac ttagggtttt 10980

gcagctcact agaggcagag ccaagactcc accccaggtg gccaggctcc tgagcgctat 11040

gcctacctca ccctccaatg aggtggtggt ggaggagacc gtgagccgct gtcacctgaa 11100

cactggtgca ctcagtctgc tgctctgagg acgccgcccc ctctctttgt aagtccattt 11160

cctgcccatt tctcatgcta gctccagctc atttcaacta attgaaaatt aaaactttgc 11220

ttctgttgag caattcggca tcgatgagac gccagctgtg ggctgtgtgc tggctgtgtg 11280

tagggtgatg agtagcagaa gcacgggtga cactgagctc tttccactgc cgggcacggt 11340

gccaagcacg ccacgtatac tgacttcatg aatcataaag ccctgtgaag ggggcactgt 11400

ttctgtcgcc attttataga tgataaagtg gaggtggggg aatggcagaa ctgggatcaa 11460

agcccacgtg tctgtgtctg gctcagcgcc catacaaggg tgtcaaggtt gcggggaggc 11520

acgagggaac caataattaa ccaaaactgg tcgggtgtgg gcagtataga gatgcacccg 11580

ggttgtcaca ggagccccta ggagggacct ctgacctccc tgaaatgatg gcagagggaa 11640

ctcttgaaag aagctgagaa gtcagcactg ccaggtcggg ggtggcgggt caggacgttt 11700

gggcagaggc agcagcagta cgggcatgga gaagggggcg gaggagccag gtgctgcttt 11760

gtgttgtgac agcaacaggt gagtgatgaa tacaggtggg agatgctggc aggggctaag 11820

gaggttgcgc aggtggtggc agccgctgga agcttttaag gagagtcagg cttgattgta 11880

ggaagggggc ttaggtccat ctgaaaggga cagaaggctg ggaaccaaga ccaaggagcc 11940

tgcagctggc aggagatgct gagggcagcg aggaggaggg gtcgtgggag atggagcagg 12000

cgacatcagc aggccccttg gagggtgacg gtgggagagg ctggggcgtc tctcattaga 12060

ctcagggttc cttgcaggca gggaccatgc ccggcgtctt tgtaccctgg catttagcac 12120

aaagcctggc tccaagaacc ggataccagc ccagtcttcc tgtctgcccc cagctcattt 12180

ctaaagcaac atcacaggca gcgagtcggg agtgggatgt gtcacccccg ggttgcctgc 12240

ctgtgatgtg gtcagtcctt cctaacgcag atgccattcc tctccgtgac ggcagacggg 12300

cctccgctgt cactggccct ggcgttgctg tgacgacctg gcctgatgta gctgcaccct 12360

gcagtccatg gggctcctgt gaacccccaa ggttgagacc caaatgcctc tgctgcctcc 12420

caccacgccc taggggcccc gcgacccgcc atgcaaatga cgtggtttcc gcattgcttg 12480

aagagggttt cctctgtgtg ggggtccctg ggtccttggg cagatacctc tgcccctccc 12540

cacagctttg gcccctgccc taagaccctg gagacccttt tgcctgggag agtcactgaa 12600

ccacctgccc ctgctcacag tgtgttcaca gaagagtcac tcccctctgt gggctccttg 12660

tctgtaaaat gagggagcag gacaagatca cagggtacaa aactgtcaga gggcacagag 12720

gctgatgttg ctgccatcag ctcctggagt gccgctcact ggctgcatga ccttggacaa 12780

tttgcacgaa atctcccaag cctcaatgtc tgcatctgta aaatggatca tcctgacctc 12840

attgcgttag tgctaggata aaataaagag aaggtatatg aacgtgccca gcactgtgcc 12900

tgcacacagt aggtactttt cctggatctg aacagtctct gttcttccag ctctgtgtaa 12960

aggcttgatc tcgaccccta aagcctgaag cttctccctg tggccacctc cccaccgcat 13020

catcttccgt tctcagcggg tagatccctg cccagggggg accccctggg gaaggctggg 13080

agcatgacct cttcccaagg gtacggcatc taaaagggat ctccggaaca ttgaaaagcc 13140

atgaaggccg ggcacagcgg ctcacgcctg taatcccagc actttgggag gctgagggag 13200

gaggatcgct taaggccagg agttcaagac cagtctggac aacatagcaa gacccggtct 13260

ctttctataa atgaaaaaat aataataaag ccatgaagat ccccaaagaa ggctgtgcta 13320

atggcggccg gaagagcaga gggcaccaca gatgccgtgc tttcagcttt ttccttctcc 13380

tccaggcctc ccttctttcc ttcctctctc gctatttata aataagagaa ggcattgagg 13440

gcccccacct cgggtacttg caggccttgg gcctgggaac aggacaagca gcaagtcagt 13500

tgacccgggg ttgctcaatt ccccaagagc tgacgcaaac tccagaatca gacctgccct 13560

ggccacctgg ccctgcccct taccagctaa gggctccagc agagtcctag tcttgagcga 13620

atcttatacc tgcagccctg tccaccagct gtgcttcctg agtcctccag ggtgaggggg 13680

cgtggggtca gaagagaggg gacggtgtgc agggagggtg gaacctgact gaccgggtca 13740

gccttactct tccagcgggg tcttttcttc ctggagctcg gccctcggca gctctcagaa 13800

gctcaccttc ttgcaggctc tcacagccgc atcgtcctct ggctgggctg ctcacagacc 13860

ccggcccctg ggttttgtcc actcacccag actctgtttt ggggtcacat tacccttcca 13920

gtgttcctct gagcaaagtc cctgtgagtt agcccaaggt ccctgtcaag gaggaccaca 13980

tctggcctgt tgcactcttc tcctttgccc caccgaagcc tctgtcacac tgtcacagca 14040

gcgggcctgc catggtctcc ccatgtaagg cttcctgagg aacgggcctg gtgccagcct 14100

gggggtgaac tgccagcccc aggagtcaca ggtcagcttc cccacactta ccctggaggc 14160

aggatggagg ccccctcctt tgcaaggccc acacggccca tctaacaccc cactaaagcc 14220

gggcagttcg ctgccctggg gtgtgttctc gccttgccag ttcttggcta aaaccaaggc 14280

aggaagagcc cagttcccat gttctgtaac atgggtctat gaccagggcc tttgcaggcc 14340

ccttgcagcg cccccatgga gcccccagga gtctgggaga agatgatgaa cccaagtgag 14400

gacctcccca ggggcctttg ctcctcacaa gaacccctgg aggagacagg gcaaggagta 14460

ccactcccat tttacaggtg acaatgccga ggctcagaga aagtccatga cacccccaaa 14520

tcccccggac ccactaccct gacctcctgg cggtgcacag agcatgccac gcacctctcc 14580

atatgcacat agaccctcta acctaagtca gcgccccttc tacaaggccg gtgtccagca 14640

cacctgggaa aggggacatt tctggccctt tcctctcaga agtcgcctgc tcgagctgcc 14700

ctccctgggg ggaaggggat gggggcagaa gctgagcatg gaagcccctg ctaatccacc 14760

ctctgctctc tccagaggcc caggaccagc ccaactctgc cccacatccc agcccccacc 14820

tcccagctcg tgctgtttct ccacctctca tgtggatggg ccacacacca cccagcaccc 14880

ctgaggtctg gtgagggact gagccttcga cacccatcct taagaaccca gggctggtcc 14940

ttttgcttag agcatcaacc aggcccacac tctcatttta caggcagaga aactgaggca 15000

cagagaacct ggttactgcc cacagcagag gagacacttg agtgaggaca cagaggatct 15060

ttggggtagg ggcgggcctt ggggttgaga cccagtcttc ctggccaggg aggaaacaca 15120

ggactggagg tgaggaggcc tctttgaggc tagtttcttc atctgtaact tgggaataac 15180

aataggactg gcctcagggg gaccagggga ggattcagag aggctatgat gctgggacag 15240

ctcttcgtga tgactggcat gaagcaggct tctagtagat gctctactgc tgctttcgtt 15300

ctcaccctca cccacccgct ctgggcccac tgccctgacc tcctgaccaa gcactgagtg 15360

tgccaagggc atctccctgt ctccccggcc ccgcaccttg tgtcaccctt ttccttagct 15420

cttaccacca ccttgaattc tctattgaac gtgcatgtgt ctccccagct aagatgacaa 15480

cttcccgggg gcagggattt gatttgttca atgctactgt ttacccagca cctacaacac 15540

tgggtgctca gcacgtctgt tgaatgaatg aatgaatgaa tcactgcttt cacctttgcc 15600

cctgactttc tccatggacc acggacaagt gacttgatct ccctgagctg tgaaatgggg 15660

ccattcacac cagcttttta gggtggaact aggattttat acagtgcctg ccatctggcc 15720

tggcacatag tagttgctta gcaaatatcc cattgttttc cttcctcccc agagggccaa 15780

agccaagacc tggaggagca acagggtggg ggtctaaccc ctgggtgaca gagacagctc 15840

ccatctggga tggaggctgc atggtgggag gagctgctga ggttgggcca ttcagctttg 15900

gggcccttgg agtcctgggt ctgtgtggag gggaagggga agctctctgg gccccaagag 15960

aagggaggca ggattggggg tggcaaggat tgcagctctt ctgggatagc cttgggcaca 16020

ctgctttgtc tccctgagca cctgcgaatg tcagtgagaa gaccagacag tgggtctaac 16080

acgcacagtt gcccctggga ggtagcaggt gctcggtacc gatgagctac cgctaactgc 16140

aggtgctacc tgtcttagag gagggagccc aatctggagg aggggaaatg ggagctgtgg 16200

acccagatgg gaggcggctc agagactgtg ggtgggccct gatgagcttc ttatccatag 16260

tcctctatcc gcttgccgcc atcctggccc tggcatggcc caaagtctct gggagacgag 16320

gggaggtggc tttgcctttt tccatctctg ttatctcatc tgtgaaatgg gcacaatcag 16380

acctgcctca ccaattggaa tatagattta ggtgcagttt tcttttttct ttccttttat 16440

ctattctctc tctctctctc tctctctctc tatgtatata tgtatagcta tctattgata 16500

ttttctatca tccatctatt tatcttgtat ctactttcta ttatctctat catctattta 16560

tctaatctat catctatcta gctatctttc tattttatct atctatctat ctgtttgtct 16620

gtctgtttta tctatctagc tattttatct attttatcta tctatctatc tatctatcta 16680

tctatctatc tatctatcta tctatctatt ctatctacct atctatctat ctatctatct 16740

atctatctag ctagctagct agctagctat ctattctatc tacctaccta tctatctatc 16800

tagctatcta tctattctat ctacctatct atctatctat ctatctatct acctacctac 16860

ctacctacat atctatccat cctctccgat tccctcacca ctgggtctca gattccctgc 16920

ctgttaaata aatggtcagt taaccctcaa cagtgatttt caatttttca cttgaggccc 16980

ccatattttg gaagattgta gtttgctgaa cacgctgact gaatcaagta ctatccaact 17040

tatttcctca ttttaacatg tcaaagatcc cctgtggggt gagtggggtg tgaattggag 17100

cctccccaat ccaaggcaca ggaactggaa gcttctgtag ccctgcattt cctagagaat 17160

gcttgccatg ttttggggac ctcctggggg gcccctgtac tgaccagagg ccggctccca 17220

gggctgtggt ttgggcccag ccccctgctc ctggggactg ctgctgtgga gggagtgacc 17280

ttttgttctg ctttgccaag gggctttctg ttcctcattg tttagccatg acaaggctgt 17340

ggattttttt ttttttttct gtccaagaga ataaacaggc ctgagattgc tcaagggagc 17400

cgaccccctc tgccagctac tgggggctgg aggctcacag agccagggag gaagtcctac 17460

cccctctgcc ggctaccggg ggggctggag ccacacagag ccagggagga agtccaggga 17520

cggtcactca cagcaaagcc tcagacaaga gaggaacctg gaggggagga ggtgggaggc 17580

caccactcag ccggcctggg ggccatggga gtccagctgc ctgccgttag tggccaggag 17640

ccctcgtcca ttgcggctct tgagagccca aacttggcca cggcagggct ctttcccatc 17700

aggccctttg agccctgatg ggaaaccctt ccccagccag ggtgggattt cttgctgccg 17760

cctggagccg ccctggccct gtggttctca ggccctggag gagtgagggg ctcccctggt 17820

taccccaagg gcactcgtgg ggatgaagcc cgcgggatgc aggattccca cgagacctgt 17880

gctggccact gtgcggggtg gggggctgcc agggaagagg tgcagcccct gcctgcctca 17940

gggatgcgat ctgagggaag aggcagctgt gtctgggagg ccgttcggtc ggagggggat 18000

aggcacagcc cctgcccttg gggaagcgcc cagcagcagc cttcagggaa ggcagtttgg 18060

gggtttgccg agggccagga agaccctctg ccgcagttag tattcgatgg aatggcttgt 18120

gcagagggag ctgcagaagc tgccatttct ctgggttagg ggctcccaca gcctttggat 18180

gccgggtctc ggccatgagc cccctccgcc acccctcccc caggtcacct tgctcaatcc 18240

aggaggccgg atgatcagag tgtgttctgc aaaggagcgt gagaatcact ggggagattt 18300

cctttttctt tttctttctc tttttttaat ggtacagctt cctgggccca cccctgagat 18360

tctgcacttg tttggggagt gggtgttggc ttcatgacag gcacccctgg gtgctttgga 18420

cacaggcagt gagctccccc cacactgtgt gaacccctga gaaggcttct ccttttgtct 18480

agggtgcctg ggagtctgct ccagcagcaa accggggagg ggaggggtac tgggaccgag 18540

tgtgtgtggc agcaccgtgt ccaggcaaac tgaggctcca gacgggggtt acttcttcag 18600

accctgttat gcagcatggt tcccactgcc cgtgcccaga attcaccata gctgatgggc 18660

ctggggtggg cctccggggc aggccagggt ggaaagtgca tctgtgtttg agctttgcgg 18720

ccctggcgct ggagttagat ccctacagga aaggacattg taacccagcc cgtggttgag 18780

actagcagct gctgagcacc tacgccctgc taggctgtgg ggcaacagca aagggtaacc 18840

cccagcctgc agcctcaggg gggcttccca tctagcgagg ggcccaggga ggccatgagg 18900

agagccatga gggaggaagg tggggactct gggcctgagt gactccttca gggtggggtg 18960

cgggcaggga gccttcagga gggagggtac tgcctgagca gggctcttaa gggacacgtg 19020

gaatttgtta caggaagcac caggccattc caggcataga aatcagctgg ggtgcaaagg 19080

cctgggggtc agaagagtgg cagattgctc catggcagga agccccagct cctctgggtc 19140

gttcccgctg agggattcca gctgttggca ccgaggggtg cagccagggc agggtggtgc 19200

tgcctgccag gctgaggtgg gagacagctg ggtttctagc cctgcttgtc actcgctata 19260

ggtgcagcca aacgctcagc aggcccaaac tctgggcagt cctgactccc aggcagtgct 19320

gtgaggaagg ggaggaggag gcacactgaa cccacctgct acgttggctg gtggaaattt 19380

ggccaggtct gcactggccc agcggtgctg ggtgtcgtga ctggtgtctg ctctgactgg 19440

cttgctgaca gctgaagtcc aggataggtc catggccacg ttgtgctggt ttgttaaata 19500

tttttagtat ctcccctgct cctatatgtt aggtactgga gtccagagag gttaaggcac 19560

ttgcctaagg ccacacagcc gctaagtggt ggagtgagga tttgaaacca gttctttctg 19620

actccctgta tccttatcta tggagaggcc ccatgactcg ttcactaaag gttatggatc 19680

tcgatcttgt actggccaca tgactgcagc tggcagtaca gtggggagtg cgccagccct 19740

ggctctgacc tcctagaact gccctttgtt catttccagg tgacgcatct gtgggggccc 19800

ctgaaggact tggggtgtgt gtgtacaccc tctttgcttg tacagatgtg ttcacaccca 19860

tgtttgccta catgtgaatg tctccattct gagcttgctc tcagcctggt agaggtggct 19920

aattaatgct tgtccctgaa agcccggact gccaacccca gtctttcagg agagagggca 19980

ggactcaggg gcttgctccc gggggtcctg ggaaggcaca atggtgacag tgctgcagct 20040

ctgcactcct ggagggtcac tcagagaccc gagagaggag ggctctgcgt ctgctcctct 20100

gtccagggct gtagcttctc tgggtgcctt tgcttttctc ttttctcccc tctttttttt 20160

tttttttcga gatggagttt tgctctgtca attaggctgg aagtgcagtg gcatgatctc 20220

agctcactgc aacctcctcc tcccaggttc aggggattct cctgcctcag cctccagagt 20280

agctgggatt acaggcacac gccactatgc cgggataatt ttgtattttt agtagagaca 20340

gggttttgcc ttgttggcca ggctggtctc gaactcttga cctcaagtga tccacctgct 20400

tcggcctccc aaagtgctgg gattacaggt gtgagccacc acacctggcc ctctctctct 20460

tttcattcat cacattgttc atctcttccc ggaggcatcc agcacagtct ttgagactgt 20520

gagctgtgaa gtcagacagg acccaggtcc aaacccttct ctgtcactta ccaggtctgg 20580

gaccttgggc aggtgacctg ccctctcagg cctcggtttc ctgttctata aaatggctgc 20640

agtagggaaa ccgtctccat cttcattggg tggttgggaa gtcttgctga gaccacacac 20700

ctgtctcagg tagggtcttc cagaagcaga gcctgaggca gggatctttg tgaaagtgat 20760

ttgctaggga gggctcccag gagaagcagg cagggcaggt caggggaggg ggcttagcag 20820

ggacgttgtt caccagcttc agcccaacac cacgggggtg gctctggacc atgaattgta 20880

ccagggtggg tcccaccttg aggctgtgga gcattcatta tcagccaaca cccacagcag 20940

ctgggggagc ggtgccctgc tggcagcagg ggtctgagtg gggtgcaaca gcacccacca 21000

attcgagcag cagaaacgct tagaacagtg cctggtacct aggcagtgct tcaaaaatgg 21060

caacgattgt catggtcata accaacattc agctgatgag tgccggctct acggtgggtc 21120

ccatgtgaga gattgagggt ctagagatga caaggctgcc acctctgctg tctgcatcac 21180

atgagtgtgt gtgtacacat gtgtgtgcat acatacccac agggggtgga aggacaagca 21240

agtaaccatt ccagaacaat gagatgactg ttagaggggt cataacccac gtttagagag 21300

agccgagagg aggaggagga ggagcttgaa ttagccttgg gtagtcaggg agggcttccc 21360

agaggtggca gcactcatct tgaaagacaa agggatgttt caggtgcagg agggaaggag 21420

aggtatcttc cttcctcagc agagggcagc tttgtgctga gaacccccgt tctctgtgac 21480

caaggccact gtcttctgga tatctgtccc atgcagtgtt aggggtcacc cagcactgga 21540

gaaggccagc agagtcagtg cttctgcctg cagacatgac tagggtacac tgaggtgggg 21600

aggcagggga ggtaaaagaa ggcacgagct ctccttcctg taccctgctc cagggggaga 21660

aacctcccca gccatcatca gtgcagactg gcagggggag ggccaaacat ttgcagggcg 21720

gggacctggg catcaggcat ggcagaaccc aggggcggcc ctgttgatgg cagtcttccc 21780

ttcttccttc caggtggcat ttacacccta cgccccggag cccgggagca catgccggct 21840

cagagaatac tatgaccaga cagctcagat gtgctgcagc aaatgctcgc cgggtgaggg 21900

cagccacggg ggcactcggg gcccatgccc tggaggagcg tgtgtgtaca ggggctgggg 21960

cgcaagagca tagcccttga ttcttactga actcctagtt ctatgcactg gcccatgctc 22020

cctgctgctt ctgggcctgt gaacatgctg ttccctcttc ctggcacact tcccaactcc 22080

ctttccctga ttaactccaa cttgcccttt agacctcagc tgaagatgtc ccctcttcca 22140

ggaaggctgc ctgccccata ctgcccagcc ggtggaggag ggggaggagg agcctaaatt 22200

agctcctggg ggagtcaggg agggctgcct ggaggaggca gcactcagct tgaaagacaa 22260

gtgctgcctc ctttgagctc tcactggcct ctgatgttcc catggaagct ctttccttgc 22320

tgtgggatta tagcccggtc ctgcttcctg ttgcctccac tgtgtgctcc tctagggcag 22380

agattgtggc ttgttttctg ctgaatcccc agcacgtggc gcaatgccat gtaggtgctc 22440

agtgcatatt tgactgactc tgtgaatgct tacccatttc acagatggta gacctgaggc 22500

ccagccacag ttggtggagc taggtagcct gactcctggt gccctccatc tattcatttg 22560

tgtctgtatt cattcatgtc atgattataa ggtatctgct gtgtgttcta agtgctggga 22620

atacacagag agcagggcag acaaaaccct tgccctcgtg gagtctgcat tttaacggtg 22680

gatgcagaca gatacattag gagatgtgtg gtcctgtcta tctgtaagtg ctgtagaaag 22740

acaaggtcag gtaaggcggg ggagacagaa cgggacatac tgttttacac agggtggtct 22800

gggaaggtct ctctgaggag gtgacatttg ggcagagccc tgcaggaggt gagggagcag 22860

gtcatgcatg tatacaggga acagtgtgcc aggaggaagc aacagcatgt gcgaaggcct 22920

tgaagtcgga gcctgtttgg tgagttggag gaacagcagg gccagcatgt gtggcccagg 22980

atgagcgaag gcgagtacgg taggggagga ggaggaagga gtgagcaggg accatggtaa 23040

ggagtttaga ttttattctg tctgggagcc aatggagggc tttgagccgt taccttgaga 23100

tgtaggtggg catatgagta cctgtcaccg tccctagcca ttttaagaca tcctagggaa 23160

agtgactctc ttctttcttt tattaactgg aagggtctct ttaagaagtt actagagagg 23220

ctgaggtggg cggaccaccg gaggccagga gtttgagacc agcctggcca acatggtgga 23280

acccgtctct actaagaata caaaaaaata gctgggcata atggcaggca cctgtaatcc 23340

cagctacttg ggaggctgag gcaggagaat tgcttgagcc tgagaggcag aggttgcagt 23400

gagccgagat tgtgccactg cactctagcc tgggccacag agtgagattc tgtctcaaaa 23460

aaaaaaaaaa aaaaaaaagc agctactgag tcaacttttt tttaaggttt ccgttggtgt 23520

tctctgtccc tggaatgcct gctttccagg cccatgccct tgtccttgat aggataccag 23580

ggactacatt cttactacct gttcccccta tcattcatgc ccctagctgg agaatattag 23640

ccatctatca agaaggaaga aactggcaag ctagttctta ctaaagtgtg attgactgat 23700

aatagggctc cagtggtgga ggttcaagca tctggaaaga gcagagagga atctattttt 23760

gagcagaagg ggttcagaaa tgacgtgata gctggtacaa tactagatgg gagatgattc 23820

tgaggaattg gaactccaca gaatagcctt cccagctggg ctttagaact ctggactttg 23880

tggggacagt ggatgagccc agggtcctgg cagaaggctc gcccagctga gacctctggc 23940

ccttgtttcc tcaggccaac atgcaaaagt cttctgtacc aagacctcgg acaccgtgtg 24000

tgactcctgt gaggacagca catacaccca gctctggaac tgggttcccg agtgcttgag 24060

ctgtggctcc cgctgtagct ctggtgagta ggttcagaga aaaagggggc ccttacaccc 24120

ctgcctccaa cttcccccgg caactccagc ctctttggct tccagctgtc tggagttacc 24180

ccaggctggt tgttggaagt ggcacaggtg cagctgttta cccctaccac tggcattttc 24240

ctcctctgtc tcaccaaagc ctcttcacag ccccacgggg caggcggtgg gagaactgtg 24300

cccacgtgag ggttgaggag gtggtgcgtg ggagagtggt gcgcatgctc gtgctgcgag 24360

gagcaggact gcggggagga gcgaaactgc tgggaagagc gggactgcgg ggaagagcgg 24420

gacttcgggg aggagcggca ctgcggggag gagcaggact gcagggagga gcgggactgt 24480

ggggaggagc gggacttcgg ggaggagcgg gactgcgggg gacgagcgcg actgcggggg 24540

acgagcagga ctgcggagag tagcgggact gcggagagta gcgggactgc ggagagtagc 24600

aggactgccg gtcctgcccc tggactctgg ccggtgttgt gtgtgcccca tgctgaggcg 24660

gtccgccagc ctcctggaga tccgctgtct gagagtgctg ggctgtctgg gagggcagcg 24720

tgggtgtggc ggaggcaggc gtgaccgttt gccgccctct cgctgctcta gaccaggtgg 24780

aaactcaagc ctgcactcgg gaacagaacc gcatctgcac ctgcaggccc ggctggtact 24840

gcgcgctgag caagcaggag gggtgccggc tgtgcgcgcc gctgcgcaag tgccgcccgg 24900

gcttcggcgt ggccagacca ggtacggggt ggggctcagg tccttgggga cgcccatggg 24960

cctctccttt gtagacatcc ttgcagtgtc acgggcatca acccattaat tagtccagca 25020

gggagcactg ttagtggtgg ccaggtgcct gctacccatc cgtctgtcca cctgtccacc 25080

gttcattctt cctgccagca ctcccgatta ggcacctctt atgtactgga accaggagac 25140

ccagagatgg atcagaccaa gccctaagct aggaaagtta tgtgatgctg caagatgaac 25200

tcacataact ctacagccca atcccgtgca tgtgtgtaca ggaatctgtg tgtgtgcatg 25260

tgtgtacagg catctgtgtg tgtgtgtgtg tgtgtgtgtg tgtgtaaggg gtggaggtgc 25320

agacagagct ccttgggccc ctcagacctc tcctagggct ctagtgccaa ggcccagctg 25380

tcccgcagag tgtctgagtg gttgacaagt tcggattgtt ccctgaagga actgaaacat 25440

cagacgtggt gtgcaagccc tgtgccccgg ggacgttctc caacacgact tcatccacgg 25500

atatttgcag gccccaccag atgtgagtag ctgagtcctt tggttctgga ggagcaggga 25560

ggggctgtcc ctgggtgact gtgggtccag gacacagagc agctcaccaa ccaccattgt 25620
ccagactgct ttatctgagg gtggctccca ggataaatgg catggtgggc aggaccttgc 25680
cctggaaggc aggactgggt gggtgcatgg ggaggtgggg gagggctagg agggggtggt 25740
cctcagagag ggcacacatc gtcactctcc tatcctgcct gctggggccc gtgaatgagc 25800
ccagccaccc cagccactct gtcccctgct gcctcctgac caagcctcct cctcctccag 25860
ctgtaacgtg gtggccatcc ctgggaatgc aagcatggat gcagtctgca cgtccacgtc 25920
ccccacccgg agtatggccc caggggcagt acacttaccc cagccagtgt ccacacgatc 25980
ccaacacacg cagccaactc cagaacccag cactgctcca agcacctcct tcctgctccc 26040
aatgggcccc agccccccag ctgaagggag cactggcgac ttcgctcttc cagttggtaa 26100
gtcgcaagaa gtctcattca ttcactcctt ctgtctgcct gtctttctgt ctctctttct 26160
tcctctccca tggttttact gagagcccac ggggggctgg accctgtgcc ctgtcctggg 26220
atacaggtgg cagtgagact gctttctccc cacacctggt gctatcattc aaaatcccta 26280
agtgcgtggg ctggatgcag tggctcaagc ctgtaatccc agtgctttgg gaggccaagg 26340
aggctggagg attgcttgag gctaagagtt tgaggtcagt ctgggtaaca cagcgagact 26400
ccatctctat aaaaaaatta aaaaattatt tgggtgggat ggtgcctatt gtcccagcta 26460
ctcaggaggc tgaggtggga ggatcacatg agcccaggaa tttgaggctg cattgagctc 26520
tgatcgtgcc actgcactcc agcctgggca atgagtgagg tcctatctca aaaacaaaac 26580
aaaaccaaac aaaacccctg gataggggag aggtggggtc agcaattcac ctgaatatgc 26640
agatggtctc cttgctgctg tttctgatga gcccattagg ctaagaagac agcgggctgg 26700
gccaggtcac ctccaggggc ttccattctg gggtagaact gcatcaggcc agaggtctca 26760
aactagtggc ccttaggctg aatgaggcac acagacatga ttgctttgta attggatttg 26820
agaatctccg gcctagactc gcccctcatt tgcaatgacc cgaagcacct tgggtccctg 26880
gcttgcctgg ctggcccctg gtacatttga gtttgttttc tgtagctgtc tgagcttctc 26940
ttttctttct aggactgatt gtgggtgtga cagccttggg tctactaata ataggagtgg 27000
tgaactgtgt catcatgacc caggtgaaaa gtaagagtcc atccttcctt ccttcatcca 27060
cttgttcagg aagcttttgt ggggtgcctc ctgtgagtcc agcactgggc acagccttag 27120
gggtcagaca gagctggata tgggggtcct tgttctgtag gagcttaagg cctggggtga 27180
aggtacctct gctaacttaa ttactaaaag gcaagaggtg atcagggttt tctaaagggt 27240
aggaggagtt caggattcct atccaggaag cctgggggat tcgggcttcc tgcagcaggt 27300
ggtctctgag ctgggtaaga tcttgatggg tggagtctgt gcagggaagg acacgcatcc 27360
tcagtggagg cgcggatggt atagatgggg gactggagaa tggttgggat ggtggcaggt 27420
ggagttgggt gggggcttct gtggaccttg tctggtaggc gattggtccc cacagggctg 27480

ggcctctctg ctggttctat ggggccccat actgccctcc tacttaggac agatgtgcct 27540
gaggaagtca atctcttact tgtcccctct cctctttata gagaagccct tgtgcctgca 27600
gagagaagcc aaggtggtga gtgtctccac tgccctctcc ccctcttccc ctggtctcct 27660
tcccggcgtg ctgggctgtc acagaggaag cagtgaattc tgtcattggt ctgtccagtg 27720
tcaggaggtg tgcagagccc tgggaggtgg aggaaaggcc ggggagtcag gcagccctcc 27780
atgggctaag agaggctgtg agtgtgaaat gctccggcag agagcagatg ccttcgtggc 27840
gttcgtggca ggcacgtggt cgtggaccca cagcgcaacc ttgggcagat gtgaaattgc 27900
tctatggcta cttccttacc atgagatggg ggttgtgtct gctcgatcca gcctgcaggg 27960
tggtgccatt cggtcagctg acaatgatgt ttctccttgc ctccattgtg tttacagagc 28020
actttctcag agagcagtgg ttttcaaact gaattctggg atagcccagt gtcccacaga 28080
gggcccttgg gcgtggggga agtggtcatc tctcaggttt gaggtagagg ctgtggggct 28140
gggccctggg tcctcccaaa gcaactcacc tttgatctgt tttataaagt agggcctgcg 28200
aaggcttttt gttagagaca gaccaacagt gtgtcatatc atagtatttc attcattctc 28260
catcaggaag gcatttggct gcaagtaacc ccaaacctac aggtgacttt agaaaaaatg 28320
gctttatttt tcttgcattg taagaagtca aggggttggt gtctgctgga atggcctcca 28380
ctatcccctt gcatggtgcc agcaggaccc caggctctgt catctttagt gtgttgactt 28440
ttgttctctt gtttgtggcc tcatgtcata agatggtaat catcacttca tatgtcacct 28500
ctaagtttga ggtgggaaga aggggcaggt ggccaggtca gccatggtgc ccttatcagt 28560
aaatcggatg ccttcccaga agccctgggt agacttcttt catctcactg accagacagt 28620
gtcacatgac cacccctagc tgcgagggaa gctgggaaag agggtgtttt gcttttccag 28680
cctctatgga ggaggaggac aagggagggg ggttggaagt gggtatgggg tgagccagaa 28740
catacgtctg tcacagcctt gcacctggta ggcagggcag gttttttacc caatttgaca 28800
gatgagaaaa cagagtctga tgagttttct cgacctcagg ggcaggtaga gaaagaactt 28860
ctcaacctca tggatatttg agacaacatc ttacttaagg gaggccaagg tgggaggatt 28920
gcttgagtct aggagttcaa gaccagcctg ggaaacacag tgagacccta tccctaaaca 28980
aaattaaata aaataaatat ttttaaaaag gatcttggct gggcacggtg gctcactcct 29040
gtaatcccag cactttggaa ggccgaggta gatggatcac ttgaggtcag gagttcaaga 29100
ctagtctggc catcatggta aaaccccatc tctactaaaa acagaaaaat tagccaggcg 29160
tggtggcacg tgcctgtagt ctcagctact cgggaggctg aggcaggaga gtcgcttgaa 29220
cccgggcagt ggaggttgca atgagctgag atcacaccac tgctctccag cctgggcaat 29280
agagcaagac tccatctcaa aagaaaaaat aaaataaaat aaataaataa aatatcttac 29340

ttagttgtgg gggctgtccc gtgctattca gcagcatctt tggtctttat ttacttacat 29400

gccagcctca ttcccactcc agtggtgaca atcaaaaatt ctccagacat caccaagtgt 29460

cccctggagg gcaaaatcac ccctagtgga gaaccactgc tcgagggaac agctcagccc 29520

tgggcagccc aatacccgaa gcttacacca ggtgtttgga atcagataga ttgatttcat 29580

cccctgattt tggtgacaag aaaagagatg cagggaggaa aactgagttg ctcaaggtcg 29640

caccgcagtg tgtctctgga ctcccatctc agtgctcttt gccctgccct acagtatttc 29700

agtggggagg aaattttctc attccccggc gagcgagttt cactgctccc tttctccagc 29760

ttcaattcag tgacactgac ggttgccgcc tggtggcgtg cgtagagcag gtgtctcccc 29820

caagctcttc cgcagcagcc cagctgtcac cttccctttc cccaccaagc gtttcccaat 29880

cctctgtgca ctgaagatgt cctgggtgtg gcctagcagc ctctgcttcc cttttccatt 29940

ttttttttct ttctttcttt tttttttttt gagacagcta tgtcgcccag gctgcagtgc 30000

agtggcgccc tcttggctca ctgcagcctc cgcctcccgg gttcgagtgg tcatcccacc 30060

tcagcctccc aagtagatgg gactatgggc atgcgccacc atgcctggct aatttttata 30120

tttttagtag agacggggtt ttgccatgtt ggccaggctg gtctcaaacc cctgacctca 30180

agtgatctgc ccacctcggt ctcccaaagt actgggatga gaggcataag ccaccacgcc 30240

tggccccttt cctttcactg ttgctctcag tgttcactga atgtgagtga ctcaagtatc 30300

tgttgagtgc cccagtggtg tgtctagtgc agaaaaaaac ccccaaaccc tgatttgtgg 30360

tgtttgctaa tttctgtggt ataaacactc acactgtagc cagtttccaa ctcccaagac 30420

cttcctgaat atggagttgg gaggaaatct gcacaggcct gagcttatcc cagcacagcc 30480

ctggctgagc cctgccaggc tctttgtgac tgagtcccca cgcatccctg tggggcaggg 30540

agcatcatca cccccatttt acagaggagg aaactgagac tctgggaggt tccatttttc 30600

ctgtaggatt agatcattag gtgagtggca tagcccaggt ttgaatcagg tttatgcctg 30660

tgtcccacgc tactcctcaa ccagcagcta ctcctgataa gaggccagtg gagagtcctc 30720

ttgagctggg ctgctgggct tagagtgtgg actcagcacc cacctgctcc tcctgccaca 30780

gaaagaacgt ccagggcatg ggagacagtg gcgttccttt gtttagaaaa caccaacatg 30840

tggccgggcg ccatggctca cgcctgtaat cccagcactt tgggaggccg aggggggcgg 30900

atcacgaggt caggagatcg agaccatcct ggctaacaca gtgaaacccc atctctacta 30960

aaaataaaaa aaattagccg ggcgtggtgg cgggcgcccg tagtcccagc tactccggag 31020

gctgaggcag aatagcgtga acccgggagg tggagcttgc agtgtactga gatcgcacca 31080

ctgcactcca gcctgggcga tagagcgaga ctccatctca aaaaaaaaaa aaaaaaaaac 31140

caatatgtga gcttacccac aatcaccccc atggacacgg acacactcat gcacggaccc 31200

acgtctgata acaggtgcga acatttatcg ggtgttgaac ggatgccagg cactgtgccc 31260

agcgttcttt attctcacag ccatgctgcc acacgcaggc agtatgattc ttgtccccca 31320

ttatatggcc caagaatcat gggcccagag aagttagtaa ctttcccagt gtggcacagc 31380

taatgatcac ggcctgggac acgaacctag gagtgtctct cctcgccacc ccagcgcaca 31440

cactcactac gtcactcaca gatatgcaca catgccccca acatagacac acgtgtgcac 31500

gcttcacacc ctcacgcctg tagacacagg tgtgtgagcc ttgtgaacac acaaacagac 31560

ggacacagca ggaacctgga ggacctggcc cgtggctctg ctgggtctct gcttctgccc 31620

agcagggctg ggccaggagg ggccgggagc tgagggtgct ggctggctgg ctggctggct 31680

ggaattctgt tttttttttt ttttttgaga aagagtcttg ctttgtcgcc caggctggag 31740

tgcagtggca caatcttggc tcactgcaag ctccgcctcc cgggttcaca ccattctcct 31800

gcctcagcct cccaagtagc tgggactaca ggcacccacc agctcgccca gctaattttt 31860

tgtattttta gtagagacgg ggtttcacct tgttagccag gatggtctca atctcctgac 31920

ctcgtgattc ggtgaccacc tcggcctccc aaagtgctgg gattacaggt gtgagccact 31980

gcgcccggcc ctgggatcct gtgttttgaa tgaggctcct cagtactcgg ctctactggg 32040

gtcccagccc aaggaatagg actcagcctg cttctgtgcc acctggggct gcttgaactt 32100

tgcgacttgt ggcttgggag gagggaggtg gccgtgacct ttgggggttt ttgttctgcc 32160

tggctgtagc caccagcaga gggggtgggg cacaggccag aaaaacccct tttgtggggt 32220

tgtgaggagt gacaattggc tgcttctcct ccccttccag gctcagagca gggctggggg 32280

gcagttgtgg gcagtgacca gggtcagacc acctgggcgg aggttcagca tgaacttgca 32340

atgccctcca tctctccaaa actgggggac ccagcccagg gagggtgtgg gggttcctgg 32400

ggaagctggt ctaggcttct gctcctgcca cggaccagct gtgtgatgct gggcacagga 32460

tgcactttct ctgggcctcc gtggcctctt ggggatggct tgcacgagat ccctccagtc 32520

ctgagtgaga ggctgtggcc ttggggaatt aagggtgcag gtggcgctca ggtgtccgag 32580

aagccatggg agccgggggc tgcagggatt ggacagagag gaccctggta ctcgcatctg 32640

ttctcagacc acatctggaa ttgtagctcc ctctggaggg aggcaggagg tctcagcctt 32700

tcttgggggg cggtggcacc tgcgctgctc gctccacccc tgctctcacc tcccgctgca 32760

gtgctggcga gccccatcag cccttcactc atctctaccc tccttctttc tgcctgggac 32820

acttgttttc atcctgggca ggccaggggc cagggcagct gttgggaatg tggcctgtgc 32880

catctccttt tttgctggga tcagaaaaca atcgcttaga attccaaggc aagggtgtga 32940

gcgcctggcc agccagtggg aacagacaac agcctgggag aggaatttcc agcctctctt 33000

cagtgtgcgt gtctggaaat ggggaccttg ccttgagcct ccagagttga aaccccagac 33060

acccaggaaa ggccctttgg gatttagccc agccacagta tgtcctaacc gtgaccttgg 33120

gcaagtaact caatctctcc gtgcctcagt ttccacaaag caaggataac actgggttgt 33180

tggaagaatc aatatagata ttgtctggag ggatgtaggt acagtgcatg gcattgggtg 33240

gcactcaaat gtcagctaat aatattatta ttattctacg ggaagaagac atcaggggaa 33300

gttgcagagc agcctgtggg cggactctgg aacaagaggc tgaggcagtg cagcagaggg 33360

tctcagacgt gagcgctctc tgccccggaa tgattgactg agcgcaaagg tctgcacgct 33420

ttctctgtaa agggccagat ggtaggaatt tcaggctttg tggactgtat ggtctcagtg 33480

acagctactc aaacctgcct ctgtagcaag aaagcagcta catgcataga cagcacacac 33540

ccaactgaga gtggtgtgtt cctatctaat tgtgctactg gacacccaaa cttgagcttc 33600

ccaccattcc atgtgccgca aattattctt tttttttgag atggagtttc actcttgttg 33660

cccagcctgg agtgcagtgg ctcgatctcg cctcaccaca acctctgcct cccaggttca 33720

agcgattctc ctgcctcagc ctcctgagta gctgggatta caggcatgag ccaccacgcc 33780

agctaatttt gtatttttag tagagacggg gttttgccat gttggtcagg ctggtcccca 33840

actcccttcc tcaggtgatc cgcctgcctc agcctcccaa agtgttggga ttacaggcat 33900

gagccaccaa gcctggccac aaattattct taaacatttt tttttcaacc atttaaaaca 33960

tgaaaaccag tggggtgtgg tggtgcacgc ctggtatccc agcactttgg gaggccaggg 34020

taggaggatg gcttgagccc aggagtacaa gaccagcctg ggtaacatag cacaaccctg 34080

tctctacaaa caatcgacaa caaaaaaatt agccaggagc agtgacacgt gcctgtggtc 34140

tcagctactc aggaggttga ggcaggagga tcacttgagc ctggaaaatc gagggctata 34200

gtcagctatg attgtgccac tccactcctg cctgagcaac agggtgagac cctgtctcaa 34260

aaaaaaaaaa aaaaaaaaaa agtgaaaacc attcttagtg gcaggctgta ctgggcctct 34320

gggcaatcat ttgcctagtt ctgatttaac aaactcttgt atggagttta ctatgtaata 34380

ggcattgttt taagcacttt acaaatattc agccatctaa tcttcacaac aaccctatga 34440

ggtaggctat tattctccct ttatagagta aaaaaaaaaa aaaaggcaca gagaggttaa 34500

gtaacttgtc caaggtcaca cagcaagtga gtggtagagt catgatttgc acttgtgtgg 34560

cctgggttta gagtccacac tcttggttgc taggctgggc catgtctccc tgtgcagatg 34620

gggtgaagga aagctgcttt ccttctactc ccttatgcaa aataaggatg aaaatcctgc 34680

cccacctcta agactatttg gtgaacaggg ccaggtattc tctgccctca taggacactc 34740

tagtagagca gctgggtgct aataacagaa acacagaaac cacggagaat cacacctccc 34800

aaaaagtgcc gtgtgtggaa agaacgtgca agggtgggca gaaacgtcac gtaaactgag 34860

aagtgctgat ggcaggaggt gtggatggca gtgggaagga agaggggaag aaagagctgg 34920

ctggtgggct gactgctctc ccctaccacc ccctgcccat ccagcctcac ttgcctgccg 34980

ataaggcccg gggtacacag ggccccgagc agcagcacct gctgatcaca gcgccgagct 35040

ccagcagcag ctccctggag agctcggcca gtgcgttgga cagaagggcg cccactcgga 35100

accagccaca ggcaccaggc gtggaggcca gtggggccgg ggaggcccgg gccagcaccg 35160

ggagctcagg taagaggtgg gagcacacct ggcttcttcc caagcctcct tggtctttct 35220

cacctggttt ctgtcttagc catctcctcc tgagcctccc cgcagggtgg gacgaggcct 35280

gagccacagg gaacttcctt cggttcgctg aacctaagtt ccctcccgcc tttgcccatg 35340

ctgggcctat cacctcaaaa tcctcccttc tgtgggacaa ccccagcttg tccaagtccc 35400

tggtatctgg gggaggagtt ttcctgaaac cttctccctg ctaccacccc cagctggcct 35460

ggctgctcct ccgggctcac catgccctgg cctccctttc acaggactgt cagactgcat 35520

gtacagacat tgtcttctcc tgtctcccac gccaggctgt gggacacctg gtgagcctgg 35580

atcatctcat tcatccctgt atctgcaggc ccaacacggc ccagctactg ataattaatc 35640

ataacaatcg cttctactta tggaaggcta cggaacagca ggcactgtac tgggcacttt 35700

acatgcataa aactgacctg catgtcaatg ctaagagata gttcctgttg ttatcccatt 35760

ttacagatga ggaaactgag ccccagagag gttaaacagc ttcttcaagg gcacatggct 35820

agtaaacaga agagccagac tcacccctag gctgtcgggc tccagagccc tgggattgga 35880

agatgaatga agaaatggtg gctccagggc tccactcact gcagtttgtt gctgggtctc 35940

tttaggtctg aggcactggg actgtgggga ttgtgtccca tttatgcagg ctgcattgtg 36000

ccctggacct ggtctatgac agatgtcact cctggttggc atctctaggg cccacactgg 36060

aggggcccct gtacagggtc tgctggcctg tgcctctctc cctcttacct ggcagtgcca 36120

gccagtgaga attcagggaa tgaccatgaa cttgggtcag tctgagatcc ttgcctggcc 36180

actctggtgc cataagaatt tgggtgggat gcttaaccct gccaagcctt ggttttttca 36240

cctagaggtg agctatagcg cctccttgcc agggctggtg agagatgggt gacattgtgc 36300

ccagctgggc accagaccag ggccaggctc cctctgggcc gcctccaggt ggggactgat 36360

ggctgcagcc cccaccacgc agccctcctc cagctcactc acccccagct gctccccagc 36420

tcactgcgcc cctgcctctt gcctgcactc atgccacccc tgcctgccac acctgttcct 36480

acctgccccc aactgcccac aggagctgac gtggccattt ctgtctgccc catcatagcc 36540

cccagcctcc tcggtgggag gtctcatcag tgcccctcac tccatcctga gaactcccta 36600

tgggagggcc tgggccagtg cctggaagag atcaggggcc ctacacactg ttgaatgaat 36660

gaatgaatga gtgcgctcca attataaact cttagtcttt gcccagttct tggattcgtc 36720

tgattttttt tttttaaatg gggtctcgct cgtcgcctag gctggagtgc agtggcgcag 36780

tcttggctga cttatctgct caccgcagcc tccgcctccc aggttcaagt gatcctccca 36840

cctcagcttc ccgagtaggc gggattacag gcatgtgcca tcacgcctgg ctaacttttg 36900

tacttttagt agagatggag tttcaccatg ttggccagac tggtctcgaa atcctgacct 36960

caggtgatcc atctgcctcg gcctcccgaa gggctgggat tacaggcgtg agccaacatg 37020

cgcagcccat ctttctgatt tcttagctac acctggtgtg gctccctcct tgggccaggg 37080

tggagccctg accatgtctg ccctcccctc tcccctctgc cccttctgct ctgtgctcct 37140

tctcccgagt cccccagccc gtgtccctgg cctctgtctt ctctttctct ccctcccacc 37200

cctaacacct ccctccactg tgggaacctg taaaccccag ggttgtgccc cttcatggtc 37260

ccccatccac ccccgcaatg tctcatgctc gatatacaaa ggccatggtg actttgggtg 37320

acatttgggt gctgtggagg ctcagggtgg aaatttcctt ccggccttgt gatttcaacc 37380

ctcctccccc accacatgct tggggctgtt ttgagcacag caggttgcca gctccatcca 37440

cctcccggct accctatccg agtagttgga gttagggaga accaggctgg ggtgagggca 37500

ctcagcaggc ccctgcagca acagcagcag caactctcat tttctgaggg ggctacttac 37560

tgtatgccag tcccttcata ttcatctcag caaacccacc gtccagtgcc tccccaacca 37620

gttagaaaac tcagttgccc acaggggctg ggcaggaagg tgaggcaaac cttgggctgt 37680

ccttggccgg atctcctgca tctggctccc aagggaagcc ataaatccag atttttaaat 37740

gtaaacgcct gaattttaaa tgttggtaat caattcactt aaaaacatca ccaccaccac 37800

caccaccacc accaacaaaa aaacccgtag acttgtccct gttacaggca ctaggaacac 37860

agcagggaac aatcaaaaag tccctggtct ggccaggcaa ggtggctcat gcctgtaatc 37920

tcagtacttc aggaggccaa ggcaggagga tcacttgagc ccaggagttc gagactagcc 37980

tgggcaacat agcaagaccc ccgtctctac taaaaaaata aaaaaaaaag tccctaccct 38040

cctgggttca gagtctggtt ggggacccca ggagctgggg gctctggaga tcaggagatc 38100

acagaaatgg ggagggaccc agagagtggt ggataggatg ggaagtaaat gtctctagag 38160

agggaggcca gggggtggag ggcgcttcgt ggaggaggtg gcctttgagc taaggcctga 38220

gcactagaga agagctctct aggctgaggg agcggcctgt gcaaaggccc aggggacctg 38280

aagggctcaa ggggctgtag cagggggtgg ggaatgtggc tggaaggaac cccatcaagg 38340

tcttggagcg gcaggagagg gggtgggaga aggcaggctc cagatcagac agggcctggt 38400

aggctgtagc aaggactgtg ggtttttgag cccccaagga agtgatctgc caggttcaag 38460

ggccagctct ggctgctgat gggaaacaga tttcagaggg gtggggttga agccaggaca 38520

gatggaggct gttcacaccc atccagatgg gagtgagggg aggcttccat agcccaccat 38580

gcagcagcag ggcagggtga cccttgcaga agtcatcttt tgtttttgtt tgtttttgag 38640

atggagtttg gctctttcgc ccaggctgga gtgaagtgac gtgatttcgg ctcactgcaa 38700

cctccgtagc ctgggttcaa gctattctcc tgcctcagcc tcccgagtag ctgggattat 38760

aggcacctgc caccataccc ggctaatttt tttttttgta ttttgagtag agacagagtt 38820

tcaccatgtt ggccaggctg gtctcaaact cctgacctca ggcgatccac ctgccttggc 38880

ctcccaaagt gctgggatta caggcgtgag ccaccctgcc tggtccagaa gtcatctttt 38940

gaagggagac aaggcaggaa tgatggatgg gtgtgtgata tgagagaaag atgggtccga 39000

ggctctgggc ccaagcagct gggtggatgg cagcaatggg aactgtgatg agcaggagag 39060

gttttggatg cgagatggga gtagaatcaa gagttaagtt ggaggctgag cacggtggct 39120

cacacctgta atctcagcgc tttgcgaggc tgaggtaggc agattctttg aggtcaggtg 39180

ttcgagacca acccaggcaa cctggcgaaa ccctgtctct acaaaaaatt agcagggtgc 39240

ggtggcctgt agtcccagct attcaggagg ctgatgtggg aggatcactt gaggccggga 39300

ggcagaggtc acagtgagtt gagggagtga cacagcactc ttttgagacc ctgtctcaaa 39360

aaaaaaaaaa aaaaagacag aagagacagg gtctcactat gttgcccagt ctggtcttga 39420

actcctgggc tcaagcgatc ctacaaactt ggcctcccaa gtagacatct gttttatata 39480

attggctcct cccatctctg gggtgattgg ggctgggtag gtagtgatgc tattcttatt 39540

cggcagaggg gaaaatgagg cacatgcagg ttaagtgact tgctcaaggt cacacagcag 39600

agctgggcta gaatcttggt ctcggctcct ggcccagtgc tctttcccat gtgtctgaat 39660

ctgcatcttg ggcaggggtc cctgggcccc actcctggac ccccggactg acccccaccc 39720

catcttgtgc ttagcagatt cttcccctgg tggccatggg acccaggtca atgtcacctg 39780

catcgtgaac gtctgtagca gctctgacca cagctcacag tgctcctccc aagccagctc 39840

cacaatggga gacacagatt ccagcccctc ggagtccccg aaggacgagc aggtcccctt 39900

ctccaaggag gaatgtgcct ttcggtcaca gctggagacg ccagagaccc tgctggggag 39960

caccgaagag aagcccctgc cccttggagt gcctgatgct gggatgaagc ccagttaacc 40020

aggccggtgt gggctgtgtc gtagccaagg tgggctgagc cctggcagga tgaccctgcg 40080

aaggggccct ggtccttcca ggcccccacc actaggactc tgaggctctt tctgggccaa 40140

gttcctctag tgccctccac agccgcagcc tccctctgac ctgcaggcca agagcagagg 40200

cagcgagttg tggaaagcct ctgctgccat ggcgtgtccc tctcggaagg ctggctgggc 40260

atggacgttc ggggcatgct ggggcaagtc cctgactctc tgtgacctgc cccgcccagc 40320

tgcacctgcc agcctggctt ctggagccct tgggtttttt gtttgtttgt ttgtttgttt 40380

gtttgtttct ccccctgggc tctgccccag ctctggcttc cagaaaaccc cagcatcctt 40440

ttctgcagag gggctttctg gagaggaggg atgctgcctg agtcacccat gaagacagga 40500

cagtgcttca gcctgaggct gagactgcgg gatggtcctg gggctctgtg cagggaggag 40560

gtggcagccc tgtagggaac ggggtccttc aagttagctc aggaggcttg gaaagcatca 40620

cctcaggcca ggtgcagtgg ctcacgccta tgatcccagc actttgggag gctgaggcgg 40680

gtggatcacc tgaggttagg agttcgagac cagcctggcc aacatggtaa aaccccatct 40740

ctactaaaaa tacagaaatt agccgggcgt ggtggcgggc acctatagtc ccagctactc 40800

agaagcctga ggctgggaaa tcgtttgaac ccgggaagcg gaggttgcag ggagccgaga 40860

tcacgccact gcactccagc ctgggcgaca gagcgagagt ctgtctcaaa agaaaaaaaa 40920

aagcaccgcc tccaaatgcc aacttgtcct tttgtaccat ggtgtgaaag tcagatgccc 40980

agagggccca ggcaggccac catattcagt gctgtggcct gggcaagata acgcacttct 41040

aactagaaat ctgccaattt tttaaaaaag taagtaccac tcaggccaac aagccaacga 41100

caaagccaaa ctctgccagc cacatccaac cccccacctg ccatttgcac cctccgcctt 41160

cactccggtg tgcctgcagc cccgcgcctc cttccttgct gtcctaggcc acaccatctc 41220

ctttcaggga atttcaggaa ctagagatga ctgagtcctc gtagccatct ctctactcct 41280

acctcagcct agaccctcct cctcccccag aggggtgggt tcctcttccc cactccccac 41340

cttcaattcc tgggccccaa acgggctgcc ctgccacttt ggtacatggc cagtgtgatc 41400

ccaagtgcca gtcttgtgtc tgcgtctgtg ttgcgtgtcg tgggtgtgtg tagccaaggt 41460

cggtaagttg aatggcctgc cttgaagcca ctgaagctgg gattcctccc cattagagtc 41520

agccttcccc ctcccagggc cagggccctg cagaggggaa accagtgtag ccttgcccgg 41580

attctgggag gaagcaggtt gaggggctcc tggaaaggct cagtctcagg agcatgggga 41640

taaaggagaa ggcatgaaat tgtctagcag agcaggggca gggtgataaa ttgttgataa 41700

attccactgg acttgagctt ggcagctgaa ctattggagg gtgggagagc ccagccatta 41760

ccatggagac aagaagggtt ttccaccctg gaatcaagat gtcagactgg ctggctgcag 41820

tgacgtgcac ctgtactcag gaggctgagg ggaggatcac tggagcccag gagtttgagg 41880

ctgcagcgag ctatgatcgc gccactacac tccagcctga gcaacagagt gagaccctgt 41940

ctcttaaaga aaaaaaaagt cagactgctg ggactggcca ggtttctgcc cacattggac 42000

ccacatgagg acatgatgga gcgcacctgc cccctggtgg acagtcctgg gagaacctca 42060

ggcttccttg gcatcacagg gcagagccgg gaagcgatga atttggagac tctgtggggc 42120

cttggttccc ttgtgtgtgt gtgttgatcc caagacaatg aaagtttgca ctgtatgctg 42180

gacggcattc ctgcttatca ataaacctgt ttgtttta 42218

<210> 34
<211> 110237
<212> DNA
<213> Homo sapiens

<400> 34
ccctcctgga caaagcgttt ttcctcagcc ttcaccaccc atggaggagg tgtgattacc 60

tctattcaca gaaaggagac tgggacccaa ccaaggcaag tcatttgctt cagaccacag 120

agcaaagaat cattctgtca ttccagaaat atttgtcagg cgcccctggt gtgtcaggta 180

ggtgcacctt gaacatttgt ttaatttgta cttaataaca ttttcaagag gacattacct 240

ccttaagtga aaaatctgtg aggagttttc tatctttcta agtctttgtg gcacacttat 300

cacatgcata gtactatttc tcaagttacc caggcacaca cagatgtatg gctttcagga 360

agccatacat ctgtgtagct ctgaggccca gccttgttca acacttcact cagaagaatt 420

gctggcctga tgaccaacaa taattgtcaa caacttaaaa cttcattgac ggctcctaaa 480

aaaaatcagt gtgcatgtgt gtgtgtatgt gtgtgtgcgt gtgtgtgtgt gtgtgtgtat 540

gagtgtgtgt ataacattca ttactgcaaa ctggaatgga gtggggctgg aaggcagccc 600

agaagcaagg ctcagagcct ggagctgctg acctcttggg gacactgcag ccctggcctg 660

gcctactttt ctcttctccc atatctggaa aatgaaagta gcctgacgta tccggggacc 720

cggcccaaga ctccacccat tggcagctct tcacttgtat cttttcatat caaaaatggg 780

aggtgacacc cagtttaagg aaaattccaa ggcatttgtc tcgactaatg tgaaagatga 840

ttacagtggc cagaggactg ccaaggctcc ttctcaagct gcttgagtca atgagggtaa 900

ggttttaaag ggcccgccct ggcttccttg ggcctgtagt ttgtgactgt gtcgttttca 960

gagtgggaag gcccagagtt ggccccatgg aattctttct gcttctaggg tgtttgaaga 1020

gatagaaagc caggtaaggg ttcaccgaac ctccaagcaa aaataaattt ccagaagggg 1080

cccctttact ctgaggacat tgaatagcaa caaaaactac cactgaagag tcattttgtc 1140

ccaggccctg tgctggaaac tttgaacacc tgtgttcacc cctccctcat gacactcatg 1200

aagatatacc atggctgtct ttattttcta ggtgggaatc tcaggcttga gggttggggt 1260

ggcttgggga aggtgtgtat ctaagaggta gctcttattt atttttactt ctagagatta 1320

agacacattc tttttcaaga tagacatgga actgtcaaaa aggattaaaa gttaaataag 1380

gataaggtta tgtaaattga tcttatgtaa aaaaaaacac taagatattt gaagactagg 1440

catcattatg tgttatatca tgatttttaa aatgatgcta ccaagagtac ttcagaggtc 1500

caaagctgaa atatatttgt atacttatat atataaagta agggatactt atttaaaacc 1560

tcaaaaaatc agaagtatgt aaaggaagaa ataaaagctc cctcttccct tctgattcta 1620

atttatatca aatttatatc tctatctgta gctcaatttg cctttttaac taaatagggt 1680

gatatcacac atatcattcc ttaacacttt tctctcttaa cgacttctaa tggacatcta 1740

tttgtggcac tgtatatagg gcatactttc ttgaaaaatc cattgcatgg tattttattc 1800

ttgaataaat cacttgcatg gcattttatt gtaagattgt gtcataatgt atttattcag 1860

ttccctggtg atggctttat catgtggctt ttggctgaaa gtatcagaaa gtggcccaaa 1920

ctggcttaca taaatttacc atctcatgta actgaagttc aaagaaagag tggcttctga 1980

gtgggttcca tactactagc ataatatttg gcatttccct gtatgtcccc tcctctgtgt 2040

gttggttttg tttgaagatt ctttcccttt atggtggcaa aatggttttt gtagtggcac 2100

caggctttac aacctcttgt gagggaagag ggagaaatgc catgccttaa tgtgccaaga 2160

aggaggtgtg agatccaccc tggttgaacc ccatggtgcc ggtgtcctct cctagaccag 2220

tggctgccaa gggccatctt ccgaggaagc acctggacta tacatcctcc acaatgggcc 2280

cttagaattg gtccatattt ttcaaaattg caaataatgt gtaaaatcat tgtacttggc 2340

cgggtgtggt agcttatgcc tgtaatccca gcactttggg aggccgaagt ggggggaatc 2400

agctgaggtc aggagttcga gaccaccctg gctgacacgg caaaaccccg tctctagtaa 2460

aaatacaaaa aaattagctg ggcatggtgg cctgtgcctg taatcctagc tactagggag 2520

gctgaagcag gagaactgct tgaacccggg aggtggaggt tgcagtgagc cgagattgtg 2580

ccattgcact ccagcctggg tgacagagca agactctgtg tcaccaaaaa aaaaaaaaaa 2640

gaaaaaaatt cgtacacttt acatatagca ctgtttactt gggcaattat ctgtttagga 2700

taaactagaa ataatattgc taagccaaat agaatatagg ttttaaaagt ttggcgcttt 2760

atcctccaaa acagatttta atagtttaca cttgtaatag atgtacctgg tttcttatac 2820

ctcacttact ggaatattag aaatcgttta gtctctacca aaacatggca tttttctatt 2880

atcttaattc acatttcttt ggttattagt aatcttgaac atgtttttca cgtctattgg 2940

ctttttgctt tttgcttatt gctttttgct tctttctttt ttcttctgtg gactgtttat 3000

tcatgtcttt tgcagtattc tctcttggat ttgtgagaat tgtttattta ttataaacat 3060

taaaccttga ttggtaatac atgttgcaaa tatttttccc taagttttca ttgatttgcc 3120

aaacttagtt tttttttcca agttgtcttt tctatgcaaa agttttcgct tttttgtagg 3180

tgaaactatg agaaattttc ctttaccatt tccgagttta atgtcatgct aagtccttcc 3240

tgtcttggga agatgtgttc acagcccact gactgaaggc ccttcccggc cagttctgag 3300

cttgagctga gtcaaatgta aacagctcct ggggctgtgg gtatagaaac tccctttctc 3360

tttgggtctg gaataccatg atgccttttc cctgtcctca tttttcttaa atgttttcat 3420

ttaatccagg cattatgtac tgggggatca actattcgca aaactcagta aaaggcggat 3480

tcccaaggct ccactttgga tgcagtaggt ctggggccaa agcatgtgag aaaaaaagtg 3540

tcagcattta ttcattccac agctatttca tgagggttca gggcatcatt ctggggacca 3600

cggatacaat acggtgaaca tcatagacaa ggttcttatt ctcatgtagc ctaaattcta 3660

ctgggggaga tggataatag gtgaacaaat aaacaaacaa actcatctgg caccaaaaag 3720

tccactaggc aaagtgctga ctgtgaagaa aagctggggt ggaaagagag gccatggccc 3780

ctgcaaaatg gaataggaac aaagatttaa tcatttgcct ctgaaaaatc tcagtgatga 3840

gttccttccc tcccttccct caacctccag tgtagaggag actgatgatg attgacaggg 3900

aagcactggt ggcttctcag aagtccatgt gcctcttcca ctgccttagt caccccttac 3960

tctgcatttt cacagaatct cagtgaaatt gcttaaacct ctgagtacct gagagatact 4020

cagggctctt tcttggatgc caaatagata aaatattgac tcttaaacat atgtctgaat 4080

tcctcttgcc tttccaaggt aggagaccat accatactca tttgacagca ccatttacgg 4140

tcaacaaatc tctaagctca aagaagagaa gaggcaaaaa gcaaccctac cttatgcaat 4200

taaaaaaaaa tctcccttta tagagtttca ttgacccttt cttaaaaccc cctaaaatgg 4260

acgtcaacaa tgcccctctt ttatgatgag aggatggagg agcaaacagg tcgagggact 4320

tattccaatc gaacgtctca gtagctcagt cccaaggagc tctgccccag tagtgccact 4380

tcaagtccca tgttcttttc atcttgtgag tgatgagcag cacgtggtct ctaggtttgc 4440

aatataattt aggatttact ccttgggttt agttttggta actctcaaag aagttcaaat 4500

attaagaatt ttgctagctg ttttagtaat ctattgctgt gtaactacct cttcccctac 4560

ccccagtcca gaaaacaaca accatcttct cttttctcgt ggttctgtgg gcagttctgc 4620

tccacatgac gtggtctggg gctgcagtcc tgtggatgct tctttgggct ggtgaggttc 4680

catatggtgc tttcacacgt ctggtgcttt ggtgagacag tggggaggct gggctcagat 4740

ggcaagctgt gatgactggg cctatctgcc catgtcatct ctgggcctct ccatatggtc 4800

tttctgcatt ctctccagcc gggtggccta ttccttacat ggtagtttgg ggctcctaaa 4860

agtgcagaca tggaagctgt tcaacctttg tgagatgctg ccccagagct agcctaacat 4920

cccttcctct gccctctatg ggtaaagcag aagcaccaaa ttcaagctgg gaggaaaaca 4980

aacttcctct ttcaaaggcg aaagtgacaa agaatttgca ggccgtttaa atccaccaca 5040

ttgtccagtc aggagcaccc attttctcat tgaccttctg ctaagtgaag aggtagaagg 5100

aattttcaca atggctttta gaaaagtaaa acaatacagg aagggatttt gctgtcattt 5160

aaaacatggt ctgctccaga tggaggattt ggtgattttt attttcttct ttactaaaca 5220

tttctgcatt tcatgaagtt tttcagtgca tcatttttat tatcagaaaa acaatgacat 5280

tttattaaat atatatatat ttaactcatt aattatctaa ctcattgaat atatattttc 5340

attctatatg tatatatatg tgtgtttaca tataaacgtg tgttatgcgt attctttata 5400

catttttctc ttgttttttg agttggacct tggcaaagtc agtggtgaca cctggattac 5460

cttgtgttaa ctttaatcag ttatgtgaca tccatgggat tggcaagtaa aacaagttat 5520

tggtagtggg agtttgaata gatggcttct aaacaccctc ccagacactg taccttcaac 5580

tctctgatct catccaatcc agaccaacac ttctttacta agtttctctg ttaatcagtt 5640

tgcacaggaa aatgtttcca ttcaacacat atgtatgatg agcatgttgt gacactggct 5700

ctgtgtagac atagtgggca ctgaggaaat ctggggatcc tgccattagg agcttgtgag 5760

ctggcgggga gctatggtga tgcacaaaat tataacacat gctaaaagaa cacagttaaa 5820

agagaaataa aatttaaatg ttaaagaggt gattaccagt tatttgaaaa aaaaattcac 5880

ctctttccaa ggagattaga tttctacttt tagaattctt catacaaata cacatatttt 5940

gagttcttga tctcagaacc ccattgcata tattgcatgc aactggtttg caagtttttg 6000

gtacacaccc accccacaca tacctgattg cctactgcac ttcctagaaa tgtcgaggaa 6060

gcctagatga cagaagttaa tttcttccca gggaatgtaa agcatatctt ggcagataag 6120

aacgaaaata caggttaaag acagaagatt tcactgggag tggctatgag gagccattta 6180

aatttctagc agcaaatggg tggcacaatt ggctttaaga tgatcaatct attggcagtg 6240

tatcatagag cttcccaact tatctgtgcc agggagggat caacagtgca cacctgaaac 6300

ttattacaga ctaatttcaa gtgatattgt actgcttcag gtatgatata agaactttat 6360

aacgctttca tttctccctt cctggtcttt gtgttattat tgctgcacat tttccttcta 6420

catatgttat gagacccaca atacattttt aattttgctt cagttatgtt ttgaaatgtt 6480

ttaattaata aaaatatctt acttatgtac ccatgtagtt accatttcta ttgcttcgta 6540

ttcttttttt tttttttgga gatggaatct cactctgtcg cctaggctgg aatgcagtgg 6600

tgtgatctcg gctcactgca agctccgcct cccaggttca caccattctc ctgcctcagc 6660

ctcccaagta gctgggacta caggcgcctg ccaccatgcc tggttaatat tttgtatttt 6720

tagtagagac ggggtttcac cgtgttagcc aggatggtct caatctcctg acctcgtgat 6780

ccacccacct catgtttcca tttggtataa ttttcttttt atctggaggg cttccttcaa 6840

catttcttat agtgcaggtc tgctggtaac aaattatttc agtttataga tgtctgaaaa 6900

tgacttcatt ttatcttcat ttctgaaaga cattttgctt gcataaattt ttataggtct 6960

agaattattt cttttagccc tttaaagata ttgatctagg gtcttttggc ttgcattgtt 7020

tctaatgaga aatctgctgt cattcttttt tctttttttt gctcttcttt ggcagctttt 7080

aagattttct ctttagcagg tattgtaagc atttgattat gatgtacctt agtgtagttg 7140

tcttcatatt ttttgtatta gggcttgttg cccttcttgg atctgtgggt ttacagttac 7200

tgtatttggg gggtttgtag ccattattta ttcaaatttt ttttctatta attcctctct 7260

cataatcccc ccttgaggga cttccattac acatatattt ggatatttta cattgttcta 7320

caattcatta atgctctgct cattttttca atcttctttt ctcaccgctt aattttgggt 7380

agtttctatt gccatgtctt gaaatttact aatttttgtt tttctttctt tctttttatt 7440

ttattttttt tgagagagag tcttgctctg ttgcccaggc tggagtatag tggtgcaatt 7500

tcagctcact gcaacctcca cctcctggat tcaagcaatt ctcacatctc agcctcctga 7560

gtagctggga ttacaggtgc ctgccaccat gctggtctaa tttttgtatt tttagagatg 7620

atggggtttt gccattttgg ccaggctggt cttgaactcc tgacctcagg tgatccaccc 7680

accttggcct cccaaagtgc tgggattata ggtgtgagcc gtggcacccg gccgaagttt 7740

actaatcttt tcttttgtaa tgtgaatttg ccataataca atctaatgtg ttttctcaaa 7800

tctaagatac agctgatttt atctcaatta gttattttac cttaaaaaaa tctcttttgt 7860

gtctctgttc aacatgcttg atctttcctc tagcatcttg gccttataga atagtcataa 7920

taaatgtttt catgtccttg cttgttaatt cttgctctgt gttagttctg catcagtttt 7980

gattgattca tttttctcct cctgttggtt atattttcct gcttctttgc atgcatggta 8040

attttttatt ggatcgtagg cattgtgagt ttgacattca ggttagacct aacatataca 8100

gaacactcca cacaagaaca gtgaaataca catttttatc cactgcatgt ggactatttt 8160

ccagggttga ccatatgtga agacataaaa caatgcttag tacactaaaa aaagagagaa 8220

atcatacaaa gtatattctc tgatcacaac gaaatgaaat tagaaatcaa taaaagaaga 8280

aaatttagga aagtcacaaa tatgtgaaaa ttaaacaata tactcttaaa tatcaaatgg 8340

gtcaaagaag aaattaaaag agaaattaaa aatactgtaa gattaaagaa aatgaaagca 8400

caatacacca agcttatggg atgcagctaa agctatacat agagaaaaaa ttaaagaaat 8460

aaatgagtat attacaaaaa aggatcttaa atgacttatc attccactct aataaactaa 8520

aaaagaagag aatactaaat gccaaatatc agaaggaagt tcataattaa gatcagagta 8580

gaaatagata aaatggggaa tagaaaaaaa tagagaaaat caacaaacca aatgtaggtt 8640

atcttctctg aaaagataaa caaaactggc aaagcattca ctggactgat caaaaattga 8700

aagatgactc aaataactaa aatcaggaat taaagagggg accttacaga aataagaggg 8760

attataaggg aatactacga aaaaattata tgccaactaa ttaaaaagcc tagttgaaag 8820

gcacaaattc ctagaaagac acaactgaaa ctggatcaag aagaaataga taatctgaat 8880

gggactataa taaataaaga cactgaatta gcagtcaaaa aacttcacac acacacacac 8940

acacacacac acacacacac acaagaaaaa aaaaaggaaa aaagcccatg cccagatggt 9000

ttcactggtg aattccacca aacatttaaa agagatgagc agcaatcctt cagaaaacta 9060

tttcaaaaag ttgaaaagga agggacactt gcgaattcat cctagaaagt cagtattacc 9120

ccggtaccaa caccagatga aaacatcata tgaaaagtaa aataaagacc aatatccctg 9180

atgaatacag acatacaaat cctcagcaaa acactggaca tgaaatagag cagctataaa 9240

agaatcatat gccatgacca agtgggattt atcctaagaa tgcaacatta tttcaacatc 9300

caaaaatcaa tcaatgtaat gcaacatatt aatagaataa aggatggaat tgcataatca 9360

tctcaataga tgcagacaaa atgtctgata aaatctgaca ccctttcaag attaaaaaca 9420

acaacaacaa caacaacaaa ccaacaaaaa tcagcagagt agtaatagaa gaggactttc 9480

ttaacctgat aatgggcatc caaaaaaccc cacagctaac attatactta atgatcaaag 9540

accagtagct ttccctctgt gatcaggaag aagagaagga tgtttaatcc taccacttct 9600

attcaacatt gtactaagaa tgctagctag ggaaatttgg caagaagaaa taaaattcat 9660

attggaaaaa accagctcta ttctcaaatg acctggtctt gtatatagaa cattctaagg 9720

aatccactaa aaactattag actactaaac aagctcagta agtttgcaag atacaaaatc 9780

aatatatgaa aattaattgt atttttatat attagcaatg aaaaatttga aaatgaaatt 9840

aagaaaacaa ttctacttac aatagcttca aaaagaataa aatagcataa acttaagaaa 9900

agaagtataa gacttgtaca ctaaaagtat aaaatattat tgaaagtaat tttaaaatcg 9960

ctcaattaat tgaaagacat cccatgttca tgaatcagaa gacttaactt ttttaaagat 10020

ggcagtactc cccaaatgta tcccagattt atcacaatcc ttatgaatat ccaaattgcc 10080

tttattgcag aaattgagaa actgctcata aaatacacaa ggaaatgcaa aggatggaga 10140

atatccaaaa caatcttgaa aaataacaaa gtcaatgact tacactccca ttttaaaact 10200

tactacaaag caacggtatc caagacagta ggatgttctc agagtataaa tataaaaatc 10260

aataaaaata gaattaagaa ttcagaaata tgcccttacg tttatggtca attggttcca 10320

agaaagatgc cagcaattca gtgggggaaa gaataatctt cttaataaaa ggtgatggga 10380

catctggata tccacatgtg aagaaaaaat ttgcatctct atctcatact gtacacaaaa 10440

atttactcaa aaagcaatca tagaactaaa cgtatgatct agaactgcaa aactcttaga 10500

aggaaatata aatataaatc tttgtgacct tgaattgggc aatgcttcct taagtataca 10560

gcgccagagc acaagcaaca acaaaataga taattgcact tcaccaaaat taaatccttt 10620

catgttttaa agtactctct taagaaagtg aaaatatagc tcatggagtg ggataaaata 10680

ttggcaaatt atctgataat gaacttgtat ccataatatg cacaaatctc tgataaatta 10740

acaataaaaa gacaacccaa ttaaaaagtg agcaaaagat ttgaatggac atttcgctga 10800

agaagataga taaagggttg ctaagcacat taaaagatgc tcgacattac tggtcattag 10860

caaacgcaaa gcaaaacata ctaagatgcc atccactaga atgacgggat gaaagaggcc 10920

agaaataata aatgttagtg aggacataga gaaactggta cactcattta ttactggaag 10980

gaatgtaaga tggtacagct acttttaaaa tagtttgtgg ttctttgaaa tgttcaacat 11040

agagttgcca tatgactcag caattccact cctaggtatc tatgtgacat aattaacaac 11100

acaaaaatct gtacaccaat tttcatagca acattattca aaatagccaa aacatagaaa 11160

caacccaaat gtccattaat ggatgaatgg attaacaata tgcagtatat ccaaacaacg 11220

gaatattatt cagccatgga aaggagtgaa gtactaccta tacacagtac aacatagagg 11280

aaccttgaaa gcattatgct aagtgagagg agctagttac agaagcccac acattgtata 11340

attccattta tatggaatgc ttagaataag caaatttata gaatcagaaa gtagtatata 11400

gattgccagg ggctagaaga aaggggaacg aagagtgatt gctaatgagt atgaagtttc 11460

ttttgggggt gatgaaaatg ctctgaaatt ggatagtggt gatgggtgcg caactctatg 11520

aatatactga aaccctttga attgtacact tttaaaaggg taattttatg ctgtgtgaat 11580

gacatctcaa taaaattctc attaaaataa gaatatatga gagtatctat ttctaactgt 11640

agagattttt gcaaatcctg cagaaatatg ctgaattagt tacaaccagc taagggagcc 11700

tgggatggaa gagtttgaac cacacccaat ggcctgcatc tggtgctctc ccaagatcca 11760

tggaaataat cttccaacct gcaatgtacc agggcatcta ccagcctggc tccacggtgg 11820

tttcaggcat tgctctacaa atgatcaagt tttcattcta gagtacgaat gaggattgag 11880

ttagactaac tgtacaaggg acaggaaata acagctcctt ccctgtcctg gggtcagtgg 11940

tttgaaaagc accccatcta tcagtgaata tccagcttcc tgacaaagaa aaggctaggg 12000

ggatttgccc ccaccctgtt tagaaggcga aggcgaatta ggcctgaatg gcgacttgat 12060

tctcatctga atatcagaga aacacatgtg ttgcaggcta cactggaagt cctaatggct 12120

tcagtttgat agtggatttg ggcttaatgg ttgagcaact ccagtggctt cctctcttga 12180

ttacgttggt ggctgtgaga tgaactaccc agtcatcagt catcattatc aatggcccat 12240

ttgcctggga tgctttggaa gaatgaaaga gagtaaaggc agttctactt ttaaagaagt 12300

taaaacatgg gctacagagc attttcgttt attgtttatc aaatgttagc ctgcctcaga 12360

atacctggag ggcacagatt cctaggtccc acctctggag ctgctaagtc agttgatgtg 12420

ggcaaataac ctaacacctt ggcattcact tccaggtttt cttcatttcc tcgtcctccc 12480

tttccttctt caaatgttct cctgttcctt cttggcttta taactatctt tgtctctgct 12540

tcccttccta tacattgcct taattaatcc tttaatttag attaaattaa aggataaata 12600

taaattaaag gataaattaa aggataaatc taatccttta atttaaatat tatttctgga 12660

gatatatttg ccatgtgatc cattttgttt gttagaaaaa cacaagagaa aaaaattgct 12720

gataatcttc tcaaaaagga ttatcttgtt tgttctaaag ccaaggatgt atagtgcatc 12780

ttggaatgtt ttggattgag gtgggacatt tgctttcttt ttgttttcaa acatcattaa 12840

tttacttggt ataagaactt cagaatcagc ataaagtaaa agtagtttct taggaatagt 12900

gtcatgataa ttccatgcac ttaacatgag gtaggtgata acggaagtag tggttttctt 12960

gatttttctt attcaaactg cttcatcaaa caaaacaaaa cagaacaaac gccctggaga 13020

cagagagtct tattgtgcca tagtatttct ctttctctct cttttttaaa tatttcaagc 13080

gagcatgcca tcagcagggc tgtcttcact gagctatttg ttgctgggag atgttgtaga 13140

ctgttgctcg gtcagtgcaa atgagagctg ctagcctcgc tgtaacctcg ctggctgggt 13200

actctcagag attgttaaga gttttaaaat ttttctaatc actaagcatg ttaggaaacc 13260

agagctttgt gtatgaagaa caacttcctc tcagggactg tactcaaggc tgaggtttgg 13320

acctcagcca attttatgaa cgaacaggaa caatgtcaaa aacatcagga ttcctggtca 13380

tgaggaccag agggggccac tccaatggga tggtaagctt ctgtagggct ggcacgtgac 13440

tttgcacccc tgatgctccg catgaaggga ttttctgcac ccagcaagtg ctgactgatt 13500

tcctgaggac acttagtcct actctaaact ctaagaaggt ctagggccac atcaggaaag 13560

tgagattcat gcactgactc atttccacac ttcagaggac actttctttg agtgggtgtt 13620

tggaaaaaaa tgcaaggtag atgtaattct ccacacatca gctgatgact cgtactgaca 13680

gtgtctagaa tagaactttt tttttccttt tgggtaggta ccttctcccc agatggatgg 13740

tgtgttcccc agagtgccga ggtatttctg ggtgaagtaa tattgttccc ttgtgtgtaa 13800

tcctactagt tctctttcat gaagtagctg gaatgtctga gtggtcaagg gtaatccctg 13860

agctggcctt gacacaaaca gatacgggct cttccatcct tgggtccctc tgttctgaca 13920

cctgatgaca cacttaggag ttttcctcta gtacctaaaa ctatagttag taggtactta 13980

gaaacaatta caagctgtgc taatgctttt taaaagtgga aatatcggcc aggtgtggtg 14040

gctcacacct gcaatcccag cactttggga cgccgaggca ggtggatcac gaggtcagga 14100

gttcaagacc agcctgacca atatggtgaa accccatctc tactaaaaat acaaaaatta 14160

gccaggcatg gtgatgggcg cctgtaatcc cagctactca ggaggctgag gcaggagaat 14220

tgcttgaacc tgggaggcag aggttgcagt gagccgagat cacgccactg cactccagct 14280

taagcgactg agcaagactc cgcctcaaaa aaaaaaaagt ggaaatagca atatgtgctt 14340

tagctaaata ttaattttgt ttttgccctg ccttttagct ctgtggtctt gaccaaggcc 14400

tcatagcgac tctgaatctc ttgcgactct gaatctcttg cgactctgaa tctctcgcaa 14460

tctcaatttt cttgtttgca aaactaagat gtagcagtta taatgtgagg cgcaaatgag 14520

aaaggccata caaaaaccct aaccaaaaaa tccagtaaaa tgaagctcag ttaaactaaa 14580

gacagcccgt tgacatcttt tgtgcgtttc ctccttggtt cccaggaatg ctggaaaaag 14640

aggcatttgt tctaaacatg gaaatgcagc aaaataatga ttcttttatg gcctggtggt 14700

ctggtttgac acaatcaata actaaaataa atacacacag cgtacagccc tgtaataata 14760

gaggttgctc ggaatcaact cacagtagta aaaatcctgg ttgagaaatt atccccaaag 14820

atgatatgat tttatcgccc cgtacaaagt taaccagtct tatacataat atggatcttt 14880

tcaaactttt aaatgaagta taatgcatac acagaacagt gaactcatca tgggcaacta 14940

aattaatttt cacaaaatca tattttgtga atatgctatg atttattaat acattgtact 15000

gctgatgaac atttggatag tttctggttt ttggctaata aatatagggc tgctatgagt 15060

atttttgtat acatactttg gttgacattt ctactatttt ctgttggtct tgtacctgtg 15120

agtgaaatta ctgagtcata agatatgtat ttgttcctca gcagtagtta ctgctgccac 15180

acagcatggt tctgctaata tgtacttcca gtgggaatgt ttgacagttc cagctgatgg 15240

aaatctttac cagcgtttaa tattttctct tattcatttt caccattctg gtaagagtgt 15300

cccgcaattt cattctggct ttaattgaat ttccccaatg gctaatgcag ttgaacactt 15360

tttcaaatta ttttttggcc atttaaatat tttcttttgt taaggatcag ttcaaccttt 15420

tgtttgtatt tctattgggt tacctagatt tttcttactg atttgcagtg gtttttaatg 15480

cattctgggt aagaacactt tgtcagcagt atctattcca aatacctgct tcaactctgt 15540

ggattgcatt tcatttccct aatggtgtct ttggatgaac acaccttgat ttatcaatag 15600

tctttctttg attattagaa tattttgtgt ctgttttaca actatttgcc tactccaaag 15660

tcataaaaat tttctcctgt gcatttcttt aaaggcttta ttgttttact tttcacattc 15720

agatctgcag accaacaatt aagatttgga tatggtatta agcagtggct caaggttcaa 15780

ttttcatttc tccatttgac tatccaaatg attcagtgcc atttagagaa atgaatactc 15840

ttttctctct tcagttcagt ggcgtgttgg gcataaatca ggtaactatg taggtgtgta 15900

tgagtctttt tctggactat tcattctatt gcattggcca gtttgtataa attttcactg 15960

aaatgctgtc ctaattacta tagccctgat ataaaatgct atctagtagg gtaagtcttt 16020

actattcctc aagattgcca tagctattat ttgcatttaa tattttcata tgaattttaa 16080

aatcacctgt caattcccac aaaataccaa cagcgatttt gattgggatt gcaattaatt 16140

tataaatcaa tttgggaata actgacaact ttacaacatc aaatctttca attattgaac 16200

atgctacatt gtatagctcc atttataaga tattctttaa cttctcttgg caatttttaa 16260

aatttttcag tgtagggaac ttacatgttt ttcatatttt cagaagaatc actgtctgtg 16320

tcagcaatag ctttattttt aggtggttac tatttttagt gagcttgtac atgaagtaat 16380

ttttgaaaca tttcatttat gacttgttcg ttgatagaaa taaaagtgat tttaatattg 16440

accttttatc cactagcact gctaaacttg tttactgatt ttaataatct gtacatgctt 16500

gtgaatattc cagaaaaaca atcatgccat ctgcaaataa tggcattaat ttttaaaggg 16560

tggtttcact gggtaagatt tcttaattga cagtttagtt ttctttaggt gcttagagta 16620

tgttgttcca atgccttttg gcctccgtac tttctcataa gaattcagtt gttaatcact 16680

tcattgtatt tcctgtatgt gatgggtcgt ttttctctag ctgctctcag gatattttct 16740

ttatcattgg cttttaacaa tttgcttatg atgtgctttc tttgtgatta ttctttgttg 16800

ggttgtattg agcttgttga atatgtaaat taatattttt cactgtttga aaagttgtca 16860

gtcattatta ctttaaatat tttctacgcc tttctccccc tctttttttt tggggatttt 16920

aattacatat attttggcag agccaggggc tctgtttatt tttcaacaga ctttttttct 16980

ttgtgatctt tcaactgtgt aaaaaactca ctagttcttt atctgtcact aatctgctct 17040

gggaatcacc tcgtaaatta aaaaattctc gttactgttc tttccagctg tttttaaacg 17100

ttatctagcc tccctttctc tctcaaagat tttatatttg ttcactcatc aatatcttat 17160

ttaaaaatta tttgaacatg tttctcttta tttttttgag catattgata cacaacatgt 17220

ggacctactc agagtgacct acatttttga agcccatatt ttttaagatg tagaattcca 17280

gttttgcaat tattttccac tcacacatta aatatattat aggtgtatct cagagatatt 17340

gtggatttgc ttccaaacca ctgcagaaaa gtgaatattg caataaagca agtcacaatt 17400

tctttttttt ttggttaccc attgcatgta aaagttatgt ttacactata ctgtagtcta 17460

ttaaatgtat ggtagcttta tgtctaaaaa acaatgtaca tatcttagtt aaaaatactt 17520

tttcacttaa aaatgctaac aatgatctaa ggcttcagta agttgtaatc tttttgctag 17580

tgggaggtct acccttgatg ttgatggctg ctgactgacc agagtggtgg ctgctgaagg 17640

ttggggtagc tgtggcaatt tcttaaaata agacaacaat taaccatgcc acagcgatca 17700

actcttctcc tcttgaaaga tttctctgta gcatgtgatg ctgtttgaca gcattttacc 17760

caaagtagaa cttctttcaa gattggagtt aatcctctca aaccctgaca ctgctttctc 17820

aattcagttt aggtaatatt cataatcctt ttgatatggt ttggctctgt gtccccaccc 17880

aaatctaatg tcaaattgta attctcgtgc atcaggggag ggacctggtg ggaggtgact 17940

ggatcatggg agctgttctc atgatagtga gtgtgagttc tcatgaggtt tgatggttta 18000

aaggtgtggc atgttccccc ttgctctctc tccgtctctc ctgccaccgt gtaagacgtg 18060

ccttgcttcc tcttcacctt cttccatgat tgcaagcttc ctgaggctgc tccagctatg 18120

tggaactgtg ggtcaattaa acctctttcc ttcgtaaatt actcagttct ttctttatag 18180

cagtgtgaaa atggactaat atacccatgt tgtcatttca gcaaatgttc acagcatctt 18240

caccaagagt ggattccatg tcaagaaatc actttctttg ctcaaccata agaaacaact 18300

cctcattcat ccaaatttgg tcatgagact gcagcaattg attccacctt caggctccac 18360

ttttaattct agttctcttg ctattttcac cacatctgca gttacttcct ctactgaagt 18420

cttgaacccc ttaaagtcat ccacgagggt tggagtcaac ttctaaactc ctgttaatgt 18480

tgatatgttg acctcctctc attaaccatg aatgttctta atgacatcta gaatggtgaa 18540

tcctttccag aaagttttcc atttactttt ccagttccat cagaaaaatc actatctatg 18600

gcacctatag ccttacaaaa tgtatttctt aaataataag aattgaaagt caaaattacc 18660

tcttgatcca atggaaattg tgttagcagg catgaaaaaa cattaatctc tttttacatc 18720

ttcatcagag cccttggatg accagttccc ttgccaatga acagtgatat ttttacagga 18780

atcttttttt ttttcccctg agaactagct ctcaacagtg ggcttaaaat attcagtaaa 18840

ccatgctgta aacagatgtg ctgtcattca ggctttgttg ttaagtttat agcgcatagg 18900

tagagtagat gtagcctaag ttttgaaatg agaaatggac gttggcgtca acttaaagtc 18960

accagcttta ttagctccta ataagaaagt cagcttgtcc cttgaagctt caaaaccaga 19020

cgttgacttc ttttctccac tataaaagtc ctagatggca ttttcttcca acagaaggct 19080

gttttgtcta cattgcaaat ctgttgttta gtgtagccac cttcatcaat gatcttacct 19140

agatatgtgt aacttgctgc agcttctaca ttaacactag ctgcttcatc ttgcactttt 19200

gtgttatgga gatggcgtct ttccttcaac ctcatgaacc aacctctgct agcttccaac 19260

ttttcttctg caggtttctc acctctctca gccttcctaa aattgaagag ttagggcctt 19320

gctctgtgtt aggctttgga ttaagagaat gttgggtctg ttttgatcta ttattgacac 19380

tactgaaact ttctccatat caacaataag gctgttttgc tttctgatca tttgtgtgtt 19440

cactggagta gcacttttaa tgtccttcaa gtatttttac tttgcactca caacttttct 19500

aaccatctgg tgcaagaggc ctagctttca tcccatcttg gctttcaaca tgcctaagct 19560

tcatcatttc ttgtttttat taaagtgaga gaattgtgac tcttctttga atgcttagag 19620

gtctttgtag ggctattaat tgacctaatt tcaatattgt tgcatctcag agaataggaa 19680

ggagacagag agatggggga gtagctggtc tgtgggacag tcagaacaca tttattaact 19740

tttctgtctt atgagtgtgg tttgtgtcac ctcaaaacaa ttacgatagt aacatcagat 19800

attgctgatt gcagatcacc ataacatata ataataataa agtttgaaat agtgtgggaa 19860

ttaccaaaat gtgacacaga gatatgaagt taccacatgc tgttggaaaa atggagctga 19920

tagagccaga tgcatggttg ccacaaacct tcaatttgta aaacaaaaca aaaaccccca 19980

cagtatctgt gaggtacaat aaagcaaagt gaaataaaat gaggtctgtt tgtacctcgt 20040

tgtcttctag ttttcactgt ttctatcaag gaggcagttg taagtcttcc tctgaatgcc 20100

tttgtttctc tgactgcttt taaattgttg ctattttgtc tgtggtattc aagagtttta 20160

ttatgatggg tcaaggtgta gttatctttg gtttatcttt tttttttttt ttttgagaca 20220

gagtctcact ctgttgctca ggctggagtg cagtggtacg atctcggctc actgcaagct 20280

ccacctcccg ggttcacgcc attctcctgc ctcagcctcc tgagtagctg ggactacagg 20340

tgcccgccac cacacccggc taattttttt gtatttttag tagagacagg gtttcaccat 20400

gttagccagg atggtctcga tctcctgacc tcatgatccg cctgcctcag cctcccaaag 20460

ggctaggatt acaggcatga gccactgcgc ccagcctatc cttttttttt caattttttt 20520

tttttttttg taatttggcc ttctagtcct ttgtcccctc ctcatcacca ccaccaccta 20580

ccatggggag ttttgggtgc aggaacctaa atccccacaa aggttctatg ttgggggtat 20640

gtaataacaa ataagtcaat cagatcaaat attattttcc caccaacttg gaatttgcta 20700

agccccatga aaatgtggtt caccagtttt gaaaaacgtt tggccactat ctcttcaact 20760

gttgcttctg atttgctctt tctgtcttct tctaagatta attatacata tgttagactt 20820

cttgagtata tcatgcatat ctcttacgct ctttttatta tttttctctc tatgcattgg 20880

ttttgatatt ttctgttaaa ctacctccac atttgcaaaa cctgtggtct gctgtgttta 20940

atcagctgtt aacccatcag ctgaattttc acttcagcta ttatattttt cagttctaga 21000

ttgtctatta gattcctttt tatagatttc aatccattgt tggtattttt tatcctttta 21060

cctgtttagt tcatgttttt ctgcatcttc ttgagggtat taatcacagt tatttacagt 21120

cttagtttgc taactataat ttcttgatta catgtgagct gcttccatat tctgcttttt 21180

tctcttaatt attagccata ttttctcgtc tctgtggtaa atttttaatt ttgccaaaca 21240

atgtgtatga gaggacaata gaggcttcgg ctaatgttat taatgatcag ggagtttctt 21300

cttttctgtt cagtagatgc aatgaggtat tggtaaactc agtccaacca gaattttcca 21360

ttttagtaaa acacattaga acttggttta cttgtgtttc tcaattgaga cttttctggg 21420

cttttcatag agagccttgt gagtctctgt tgccttagtt ccaaaaaaat tgtgggagaa 21480

ccagatatac cctccagaga ttcacagccc agctctcaag tgatcagtct catagagatg 21540

aaaaatctgg caaatgtttt gatggaaaac aggttatgtg cttgaggcag ggcttcttct 21600

agtagttttt tttctttctt tcttttaatc tatcaatctt taacagcatc ttggatagcc 21660

tatgagtgta tgtacttgac aaacattgcc tctttatggt tcacagtagt ttctaaaata 21720

cattcacttt attggccatt atcctttgaa agctgatttt gtaatcttga taaagtggaa 21780

gtggatttta tagtcttgag aaagtggaaa ttaacaaggc agcttcatgg ataatgtatc 21840

cttttcttct ttctgaaaat tatacatact attttatata tttatggggt acatgtgata 21900

ttttgttgca ttcatagaat gtgtaatgat caagtcagag tatgtggagt atctgccatc 21960

ttgagtattt accattttta tacgttggga atatttcaag ttctctcttg taactacttt 22020

gaaatagaca atacactgtt gcttactata gtcaccctac cttgctctcc aatagaacat 22080

atgctttcta tctaactgta tgtttttacc cattgaccaa gctctcttta tccccgtctt 22140

ttacccacac agccttccca gcctctacta tctattattc ttctctctac cttcatggga 22200

ttaacttttt aagctcctat acagaataag aacatgcaaa atttgtcttt ctgtgtctgc 22260

cttgttccac ttaatgtaat gacttccagt tccattcatg ttgctgcaaa tgacataaaa 22320

tcattacttt ttaatggctg aatagaaggt ttttgatatg taataactta aagcttcatg 22380

agattttact ctgccatttc caaagcttcc acatccattt ccagttacct acactatccc 22440

agaactcagg aaatccctat tttgcaaact catcatttgt ttgagacccc cctcaagctt 22500

ctgattgtca gagtagcccc acataaccat taaaagcttt gctgctttct ctttttccag 22560

cagaggtcct ctgtgtgggt caagccaagt cttcagcttg cagccaaaat tggtaatgtt 22620

ggcagagggg cgggaggctg gcaatcatca attctcatct taatcattgt cattgtctct 22680

tctctgaaaa ttttgttttt ccgctcctag tcgttttctt agctctttga ttcttaaaaa 22740

aaatacagct tcttcaattt atccatgttt gtccaatcat tgtagcatta tccagaatat 22800

cctttatcaa ttaaatcctc ccaggaagca gaaggtcttg aaactcttat tttgatattc 22860
atttcctgac caactataga aatctatgtt tcttaagtgc tctttaaacc aattcttttt 22920
gggatttttc cctattcatt tcctcattaa tgagctaaat aacactcggg cctgttctaa 22980
acgtaacatt gaaaattttg cacgcctcat ataaactccg agaacagggc aaaggggagc 23040
atgtttatga cactaattct tctagcagtg aactcttgct gaaaaggtct ggaatttact 23100
ggcaaaacag ggatggtggg agggcagcaa gagaagaaag gatgggggtg gagccgtgtc 23160
caaaggaaga tgcttttccg ctctctagtt ccatgactat gatttttttt ttcagccaga 23220
gtaagtgcat cttgtctata gaggtttact caggattgaa tgaaatttcg ccaagatgta 23280
cttagagaag aaaactcaga ggccagaaag agagattgct ctatacctaa accaaaacta 23340
catatagaca tctccaagac cttgagactt gaaggtgctg attagcagtt ttggaataag 23400
tgggtgatta ttaacttgtt tccacttttg ttcattaaga attgataaga aaataatagt 23460
tgccttagtg gaattgcctg ttattcaagt aactcacgta gaacgtttct gaggatttaa 23520
gttcctatgg ccaaaactcc ccaaggtagt tagtggttga agttggtgaa ggagaaaaaa 23580
aaggggtgga gaatcaaatg aagagcagtc attttaaggg acagaaaaga aaatgtcatc 23640
aatctagctt atgttattga gaaagctagc aaaagtcctt tccggaaaac ccttgtgagt 23700
cttcatttct ttggggtatt ttgaagtaga atgtctagat tagataacag gtatatgttt 23760
aacttttggg gaaaatgtaa atttgtttcc caaagcagct gtaccatttt acattccctc 23820
cagcagtatt tgagttccag ttcatccaca tcctcccttg cacttggaat ggttcaaact 23880
ttccataata gccatcctaa gaggtatata gtggtaccgc attctggatt aatttgcatt 23940
tccccaagga ctagtgatat tgagcatttt ttcgtgtgct tatttgccat ccatatatat 24000
cttccttggt gaaggatctg tttatatttc ttgcccattt ttcaattggc ttatttattt 24060
tcttgtattg ctttttaaga gttctttaca tattctggat gcaagtccag tggtgtcagc 24120
cgccccactc cccagcacct ggtctggccc tacctgcagt gggacctccg cgtgaccctg 24180
gtccccacct cctctgagtg tccgaggaga cacgcgcggg gcagcaggct cccggcagct 24240
gcagtggggc atccccgcgg gaccagtcct cctgcagcag tgtggggtcc agccacgccc 24300
ggttccagaa cctgcctccg tcctgagctt cacctgcagc agaaatacac catgatctgc 24360
cctggggaaa acaaaacaaa acaaaaccaa ccaaccaacc aaccacacat ataaaacttt 24420
aataggaagt ggaatagaag tgactacaaa atactaccat tcaatttcat catgcaagta 24480
ctttaatatt ttcactcact ggcaaaataa agcacatgta actctaaatg tgaaataatt 24540
ctgagtcttt gactaagtct tctaaagaaa gaataaaatg ctggtgaaaa cgtgaaattg 24600
tgtagtgagg gatttatgac cttgtttatt atttttctac agatgttaag atgatcatga 24660
ttaataccaa ttgaagcatt tacagaatgc tatttaggcc caatagtgat gttaaattac 24720

agtatcagag aacctgatag atatggggtc tgattataaa gaaactttat tcaaaatcat 24780

ctttgtcaaa gttttaacac agaagcaaaa gtcaaatatg ttcctacatg agatacaagc 24840

tattcttgct aacagtacaa attctgtcct gtaagtatta gtattttcag gtttttcttt 24900

ttctatgaaa cggttgaaag gaaaacttta gtacgctata actcataggc tactttgtgt 24960

atttatcatt ttataaacaa atgtcttgac aattaagttg tgtatgaagg ccatcatcaa 25020

gtcattaact cttatatcaa aacctattat tattcactga tttagataat tcaatttatg 25080

tgtgcgttac caggttgcag tcagccaaca gacacagaac aggttaatag aaatatatat 25140

atttttctta tcaggttttc ttttattgga acaggttgca tatatataca cacatataca 25200

catatatgtg catatatgta tgtatatttc cattgatata atattctaga aaatgcagac 25260

taatcactag taacagaaaa gcagatcagt catggcctgg ggaggggcaa gagtgacgaa 25320

ttacaaaggg gcgtgagaaa aattttagga tggtggacat attcactatt ttggtggtgg 25380

tgatggtttc atgggcttat acatatgaca atacttaaca gatacgccac ttcacagctg 25440

tttcctaaga gtttaacctt tcagctttag gcccctcccc caacacggtg tcctttctca 25500

ttgccacatc ataaaccagt ctttcccctg agtcacacac atgcaatcct tgcacagcaa 25560

gtcatgatgg tgccctgtgt gtatgtgata gtgatgggaa aaggataaaa caaaacactc 25620

aacttgtgtt atggcaaaaa gagattgcag tgatgataaa gaatgcagga aaggaagcca 25680

atttatctgt taggaaaaaa cttccacttg cttataaatt ctgtttcctc tgatgaagtt 25740

tcattataaa gtgttttctt tttctttaaa aacaagaaga tgagaggaca ttgattactt 25800

aaaaattttt acattaaaga atactggaaa atttcagaga ataaatgtaa tgtataaggc 25860

ccatataagg ccttaggttg aaaacaacat gcacccatga acttgctaag taatttgaga 25920

aatagaacat aattggcatc tttgaagttt tctgtatacc tctctttcta cctccctgct 25980

tccccccaga aatagctgct cttctgaacg tcagtgtttc tgtagtgtta gcatttatgt 26040

ttgcatataa aaaatacatt attttacttc atttaaagat ttttaggccg ggcacggtgg 26100

ctcacgcctg aaatcccagc actttgggag gccgaggcgg gcggatcatg aggttaggag 26160

atcgagacca tcctggccaa catggtgaaa ccccatctct actaaaaata gaaaaattag 26220

ctgggtgtgg tggtacgtgc ctgtaatccc agctactcgg gaggccgagg gaggagaatc 26280

acttgaaccc gggaggcgga gcttgcagtg agccgagatc gtgccactgc actccagcct 26340

ggcgatatag ccagactctg tctcaaaaaa aaagaaaaaa ttttataaaa ataatgcact 26400

attcttcctt ttttcatttt acgttttgtt tttaagctgt attcctgtca atatatgtag 26460

cacaacttag ttttcacact gtgtcctatt cagcgtgtaa atatactgta attgacgcat 26520

tcattttcca ctcaatgaat ttggggactt ttacaggatt ttttgctatt atgaaaaatg 26580

tttctctgag tgttcctcta ctcgcctcat agggtgtgta ctcatgatat tctttagaca 26640

aagtttaagc tgaaccttca gataggcacg tgttcaaact gttttccaaa gtggttgcag 26700

tgatttatat cacatcaaac agagtttcta cttgttgcca acatctggtt tgttagactc 26760

tgaaagttgt ttgcctacct cggaaatgtg aaatagtttc tcatttgtgt ttctaatttc 26820

cttgggtttt taagttaggt atcttttcat ttagtttatt ggcctttcag gtttctattt 26880

ctgtaaaatg cctgtttttg tctttttttg cctatttaat attttttttt ctcttcctaa 26940

ctgattctag gcattctaca tgtttgtgtt actaactctt tataagttat ttgtgttata 27000

aatgtcttcc tcttctggct tgtcttttat ttttttaaat gatgtctttt ggtgagtgga 27060

agtttttaat aataatgtag gcaaatatat taatctttat atattttaaa gcctttgagt 27120

ctttttaaaa cttttaattt taccatcctg acattctctt agataaatat attcttttgt 27180

attttattct taaagatggg agattttgcc ttagtatgag attcatctga agtggatctt 27240

tttttttttt ttttctgcat gggatgaaat aaggaaccaa ttctgtttcc tcagcatatg 27300

ctttgcctgt tgtcccagaa gctgttattg aacaacgtgc attgccacct ctgtcatgta 27360

ctaactttcc ataagtattt gtcaggatag gaaatattac aatgaagttg caaagcttga 27420

aatctcagtg acttagcaca acaaaagttt acgtctcaca gttgttaagt ctaaagttgg 27480

tcattggctc tttttcgtgg ttcttttaga atggtgactt tatcggggat cggggctgct 27540

tccttcctgc aaccctacaa gctaggaatt attttctttc atagcttcac atataagaga 27600

aagataggga tgatggaaac tcacatgagc tcttaactgc cctggactag tggcttggtc 27660

ccaaccataa acaaggaggg gtgtcaaatt tggggagcat atatacatat ttaatgaaca 27720

ttaattgtct gtaccatatc atctaagtgt ggacctcttt ctgtttcctc tattctgttc 27780

ctggtctatt tgtatatcct tgcaataatc tcttaattgc taaagctttg taataagttt 27840

tgatatctgc ttgtacaagt ccctcatcta gaaatattga tgaaagaaaa aagactataa 27900

aatattagga atgaacaggg atgtataact atggacatgg caatatttaa agataagaga 27960

ataccaagag tgactttaag ccaaaacatt tgaaatttta gataaaaaaa caaattccaa 28020

gaaaaacata atgtatcaaa attgactaaa gaaaaaataa aaaaatctga atagtctcat 28080

ataatattaa atatttgatt catattataa ctatcctaca aagaatagtt tgtataaaga 28140

aaatagtata gtttctacaa agaaaatgtt ttacctaaat gatatttgag tgagttctac 28200

caaatattca agcaatagat aatgtcaatc ttatacaaaa tttttagagg ataaaaatta 28260

aaagaaacac attctaatat attatatgaa gctggaatac cattgacacc aaaactagaa 28320

aatcccagta taagaaaata aaatcatatt tcaacttaga ggcacaaatc ttaaaatgaa 28380

ataaaattat cagtgtataa cccgtggaga ttttaaaaag atgagaatcc atgaccgtgt 28440

tacgattata ccagaaatat aaagatggct taatctcagg caatctatta atattatata 28500

tcatatatag agtaagggaa aaactgttat taatagatgc aaaaatagtt tttcataata 28560

tccaatatct attcttgata ctctcttggg aaaccaggaa gagaggaggt cattcttaac 28620

ctgataaatg atgtctacta aaaacctaga agtaaatctt gtactctata aaacactggt 28680

tctctttaaa tcaggagcaa tacaagaatt actattatga attagtttat ttaatgtttt 28740

gctggagttg ctggccaatg cagcaagata agaaaataac aaaagtaaaa agcactgtaa 28800

taaaatgaac agagctatta ttatttgaag ataatatgac ctgtccacat ggtaaaccaa 28860

caaaaaatgc ttgcttttta ctgttgagaa gctgggtcat attcgacagt caagaattct 28920

ggaataagtt gtgtttcatc aaatccctat ttaattgcat agtacactgc agtgaaagta 28980

tgattatgaa agccatataa tagcataaaa actatttgtt aagtcaaaag agattacaaa 29040

gatgtatgta cataggaaaa aaaagagtag gtgggaatgt tgcttaatgc taaagggggt 29100

taggtggcaa gatcatgaat taattttcca tctattcttt atgtcccata tcttctgtgc 29160

tgtggctata ttgtttacaa caaaagtatt tttaaaattt gtactaatga gaacctttgg 29220

aatcttcctg cagatttagt gatgcttgtt gatttgtatc agagagctgt tgaagacaat 29280

acagtttcac ctaacgttta actgcacaat aagtcagatg catttattgg gttgtgactc 29340

atacttagaa cctgacatat aatgaatacc agtaagacat catcttacct tattaaacac 29400

aataacttcc ttattcacta aaataactaa aaaaggcacc gcgcccaaag ttcacagaaa 29460

tatacatcga ggcattgtga tagagtaaaa gagaagaaaa aggagttaaa caaagtctgt 29520

agatccaaat cctagtcttg gttttcccat agtaggcttg gtttcctatt tcaaaatgtg 29580

aatagtgaga aactgccctg tttataacat tgatttgtca tgagggcaaa acaaagaacc 29640

caaaattaga gggcctagaa gagatggtag aagcagtttc cattggatgt tcgtacccat 29700

ccagtggcct ttgtttgagt tcttccaggg atggggcaaa cactgtctct gcattgctga 29760

gtgcttaatc tattgttatg aagatctttt tcacattggc ctcctggacg tctatcccat 29820

ggccttcgtt ccatctctgg gttagggtac catggctcct agcatggtct ggtggctacc 29880

aggtgtacag atatcaatcc tcttagtcta agggcctgtg ggagagccta gctggtcttc 29940

ttcggccatg aacagccttt gttattgttt tctctgcatg ccatgacaag agcgtgtctg 30000

agggcattct ccagtgatga cctcctttgt atcagaaggt acacttccac cagggcaact 30060

acagcctcct accacacatc attcactgtg gacgtcttag tcagtcaaga ctctggcatt 30120

tgtttggttt tgttttccac cagtgctttg ctttttcata tgccctgtcc tgtacttaac 30180

aatatgaggt tagggaacta agaatgggat tttagatttg tctgtgttaa gtctcactat 30240

gttagatttg gcctatggtt ctatcttaat ggcgtctttt gggaccctga tttgctctct 30300

cagtgaattc acagttcatt ttaactccag gactcccaga atgtggtaag cttgcctgct 30360

ctgcccttct gctgtagaaa cctgttctgc acctcccagc tccctcccca acccctggct 30420

tggcctcgag gcattctcct ttcctgcatt caaggcctcc aggaatatta ccacacacca 30480

tctgcacatc gtcgggttct tttggccaat gccaatttga taaatttttt tggcttcagt 30540

gtccagttct gctttctaaa cttgtatttg cttcctttgg aaaatgtatt ccttctttct 30600

accctccctc ctttcctgca ttcctctgct ctgtactctc cacaattgaa gagtgccatg 30660

accatgtcca tgcccagcct ggctgcctct tttctgggag gagaggtcag ctccacacct 30720

gagagccagg atggaaccaa atacctggaa ctgagcctga ggctttccag tagagcatac 30780

tcttctaatt gactcattta ccattgagta tgggtgttta tacatgtatc caactctact 30840

aaccatattc tttgttttgc tcacaagaca gatgtttgca gatacttaca tgaaattctg 30900

attcactaag ttcacagcat ttaactgtgg taatctcacc atctaacaac ctcatcaaac 30960

aaaagattgc atgagcttca ccagacctgg cctgttttta gtcatttcat gctttctctt 31020

ggtgatcgtg gcttccttaa gtgctgaaag actatccctg taatcattca tctagaatct 31080

ttcccattcc ttcatctgtg cttgcaaaat cccttaaact cagtaatggg tgtggaagct 31140

gtctgtctgg aggtatgaag aattaaggga tggcattctc cccggaggtt tgatagaaag 31200

ctggacacag tgcggtctgg aagagtcatg gggatgatac gaattgttca tgtatcattg 31260

cctggaatag gagtatgata agcagccagg cactcaagtc agatccttag ggcctgacca 31320

caaaggttac cagtggccat cggggatggc atggttaatt ctctagtgta aagagacaaa 31380

ggttaaacta acagaatgtg tcacactgga aagcatttgg gagatcaggt caattagcac 31440

tccctgtgtt actgtccaca caagtcaccc agaaagctta gtcaaatata gatttgaatt 31500

cagtggttct ggggtgggcc ctaatgttct gcatttccat cgtgctcctt ggtgatgctc 31560

ttggcttatg ggccacactt tgtgtcccaa gagtttagac tatactgttt tgttttgttt 31620

tttcagatgt agaaactgag atccagtgag atgaagtgat tttcccaaag atacatagat 31680

gaagttgaag cttagctctt taacccaggt ctgctggcgt ccaggttggt gtctctctaa 31740

ttatagaatt gctggactta ggaaagcaga agtctcctgc cctctcttaa tgacccatag 31800

catgttgagg gggagccaag gttagattct agggttgcac atacttgcac aaacatgcac 31860

acacatacac acatgctcat gcacacacat gcacacacat actgtacgtg cttcttccta 31920

ttctcctttt tcaattggga ggtataagaa acacatggat tacagccaca ttaaagatat 31980

tcataaatgc aaatcaattc tgaatgtgta aacttttcca taagaatatt ctgggaaaat 32040

acttgagtta tttctgacct tttatgtata aacacagaca ttctggagtt tgtcaaagac 32100

catgttactg ataaaattgt gttagaatga cgtcttctga tagtttaaat gttaaaagct 32160

ctgggctcgg ctcaggaaat tctatacttt agcattcata aaggacccta gagtttgaga 32220

gattatccat taacttctga tattgaattt ttgttgccaa ctatgactat ttgttttccc 32280

tgaactctgt aggggaacaa cacagctgac aaaacccagg caaaagcgtg tgtgtgggtg 32340

cgtgtgggtg catgtgtgtg gcgtgtgtgt tcacacaggt taactaaaca gtgcagattc 32400

ctccaatttt tctccttttt gagaagtagg aaggagcaag agagaagaaa tcatctggag 32460

tggaaaagag ggctggaaaa gtccttgaac taataatctg ccaattaatc cagctgcttg 32520

gaaatagcct ggaattggaa ggtcttttgg gtgctctctg gaaagggtac caggaagtat 32580

gagacaatgg ctgctaggaa cttgcactgt tcctgagtag ataagacctg ttgtcaagta 32640

aattgcggtg gatccctggt aacacttgaa gagcaatgca agcctgggct ccacctgggc 32700

tctacccctg cctgactaag ggagcttggg gaaagtattt acataatctc aatcagtttt 32760

ctcacctgta aaatgggcct aaagaattta aatttccatg caagtcaaaa ccataatgag 32820

atatcatctc acaccagtca aaatggctat tactaaaaag tcaaaaaata agagatgcca 32880

acaaggttgc agtgaaaagg gaatgcttat attctgcttg tgggaatgta aattagttca 32940

gccactatga aaagcagttt ggtgattgct caaagaactt aaaacacaac taccatttga 33000

ctcagccatc ccattgttgg gtatattccc acaggaatat taatcattct accataagac 33060

atgatgttca ttacatcgct attctcaata gcaaagacat ggaatcaacc taaatgttca 33120

tcagtggtag actggataaa gaaaatgtgg tacatgtaca ccgcggaata ttatgcagcc 33180

atgaaaaaga acaagatcat gtcttttgca gcaacatgga tacaactgga ggccattctt 33240

ctgagaacta acacagaaac agaaaatcaa atactgcatg gtctcactta taagtgagaa 33300

ttaaacactg agttcatacg gacacaaaga aaggaaaaac agacaccagg acctacttga 33360

ggatggagtg tgggaggagg gtgaggattg aaactaccta tagggtacca tacttattac 33420

ctaggttaca aaaaaatctg tagaccaagc cgcctgaata cacaatttgc ctatataaca 33480

gacctgcaca agtacccctg aaactaaaat aaaattaaaa ataaataagt aaatgaatat 33540

ataatttaaa tttccttgaa gaatgcctgg cacctgtaag tgctcacttc tgctatctcc 33600

gttatcatca cagagagaag gcacaggttc agagtcggga aaatcacccc aggatgcttg 33660

agtggggtct gcttgagcaa atggcggtga aaactggaaa gaaggggaag gagcaaagag 33720

gaggtgggct gtgtcgtgct gagtggtgtc aattggctag ggtaaggagt ggcagtggca 33780

gaattgaggc tgcagagcat tgaagcagag gtttgagcag tgcctcttag ggtcagcagg 33840

cggcccatcc aggtgagcac aaagcgctct tggaaagggt agcgggaaca atgacaggaa 33900

agtgtgtgag accagatgtc tgaagacctt gaatgctaga attggaggct tgactcttcc 33960

tccctacgat gcagattttt gagtagcgtt gtgataggct ggactctgta tcatgttgag 34020

aatgttcatc ggagtgcaga gcacaggagg agctatgaga gaaataaaag aggcaggcca 34080

ggtgcggtgg ctcatgcctg taaccccagc actttgggag gccaaggggg tggatcatct 34140

gagttcagga gtttgagacc agcctgacca tcatggtgaa accccatctc tactaaaatt 34200

acaaaaatta gccagacatg gtggcgggca tctataatcc cagctactcg ggctgaggca 34260

cgagaatcac ttgaacctgg gaagcagggg ttgctgtgag ccgagattgt gacattgcac 34320

tccagcctgg gagacagagc aagactgtct cagaaaaaaa aaaaaaaaaa aaaaagagga 34380

ggcttggcag caccagctct cctaggtgga tctggctgca tttgtggaag ccagaggctc 34440

cagctttcat ctccctgagc tgaggggtga tcagtggcca tcccacctcc agggtgtggt 34500

gaagggagac ttccagagtt ctcagggcac atggcaggag tggtggccac catgagaagt 34560

gggccagcat ctgggacacg agcagacctg aataaagagt cctgcagcat tgcctgggct 34620

gactctaggg agattgctct aaggattggg aggtaggcag agagggaaag cttaggccac 34680

cacaggcctg agactgacca gtcaagactc tgatgatggg aacatgataa tgagctggat 34740

tcagccagca ggcttgtcca ggttcaacac aaagtcatga gaggaaagca ccagcttcgg 34800

gaaactttag aggttaccgt gcagtggcgg gtgttggtgt ttcttaaggg tctggcacgt 34860

ttgctgagtg attctggctg tctgcctggc tctgagcttc ctgttcatag ttccccagat 34920

gtgtcacggc cgagggatta aagccctaag aggctgtgac acagccatct ccaaaacccc 34980

actttctcct tcctttgagc ctccgtacca gctggggcgt ccggcaagat gtgagttgtc 35040

actctgctgc ggcacagacc tgaattaaca actctagcta gggctgactt caaaaagcac 35100

tttcgttttt taataaccaa catcagctca gcaggcttca tttgggaaaa gaaaccttgt 35160

cggattaccc cgacattctc cacctcctgg gaggccagcc attcccaaat gccccaagga 35220

tgaagaacgg agacggtaag atgattaatt gcactgcagg ttttttgttt ctgtattttt 35280

ttccctcctt tttttgggtt tacttacaca ttaatgaggt atttaggaaa gacaaatgga 35340

atctgataaa aggtaacgtt atattgtttg tcgaaacaac attttaagga tttcttgaag 35400

ttatatggct ctgtaggcat gtttcacttt aagatgtgtt tctgagtcat attaggagaa 35460

cgactgtgat tatctatgga agattaacta tgaaacatat gggaaatata ctttataact 35520

gtatattact tataaaccat gaatagttac atgacttttg gtatgtggtg agagatgtta 35580

agtgaaaggt gttgtaaatg aagtcgtttt ataagactgt tgttactgta agaattgaga 35640

cttattttat cacttcccat taagcttcta cagtgttaga gaggcataac ccaatgttga 35700

ctgaaggtgc tatgttttct ttttaattta ttttttattt tgtttgagat ggggtctcac 35760

tttgtcaccc agactggagt acaatggcat gatcttggct cactgcaacc tccgcctccc 35820

aggttcaagc gattctcctg cctcagcctc ccgagtagct gggactatag gcgcctgcca 35880

ccatgccctg ctaacttttt gtatttttag tagagatggg ttttcactgt gttagccagg 35940

atggtctcga tctcctgacc tcgtgatcca cctgcctggg cctcccaaag tgctgggatt 36000

acaggcatga gccaccacgc ctggcctgaa tgtgctatgt tattaatggc agcctctatt 36060

ggtttcattt aaaggtataa tgcattgtat tggcaaaact tgtacacgct ttcttcaata 36120

aggacctcaa aggtaatctt aaaagcctga tcatattttt ctcccccctt cagaagcttt 36180

gccaatgttg cacctatagc tatagagaag accatgcact ctcagagggg attataaaca 36240

ttgccttgca tttttatcct tgatctccat aatttatcct tgatctccat ttatccttga 36300

tcctcataat tcctactgat agtgttgttg tgcataagaa tgagaaaatg ggactgaacc 36360

tgaaagatta aggaaagaaa ataacaaaac aaaaaggaaa acctaatgaa gcttatcatt 36420

caattcaatt tataactcct tattgctcat gcaaaatgtg aatttttcgc tgtgttggtg 36480

agggtgtgag gagtggatgg tggggaagga tgcttcaaaa aatacagcta aggaccaact 36540

ggtttggtga gtcatggcat tcacccatga gacagctgga gaacagtgac gtgtgtgtct 36600

ctgtgtgtgt gtgtctgtgt gtgtgtgcat ttgatgccag agattggtgt ttactataaa 36660

tgtgttgatc tgttaggaaa aggaagaaga caaagttgtc cagaaagagc caagaaaact 36720

tcttgagatg aatttgatct gcctaatggt agtgatgagt ttaaaaagat tcttatggcc 36780

tttcctaaca ctacatgcta ttgttaatca ttgaactgac aaaccccgtt gagcacaaga 36840

ctgttagttt acttaggagg aaaacttggc aggaacggtc aaagaatagc gagggtatac 36900

ttctaaaata cgtccttgga acatttacac aaacagcaca agctcattgt caagaataaa 36960

attttaaaaa atacatagag ataatagtaa aagacgagaa gtaaaaacac accactgtga 37020

ggtaaacact gttaacattt tggtaggtgt atttgtctct gagtacattt acattttgaa 37080

gaaatatatt tgtactctac atgcttttcg gttatctgct tttacctcac agcgatggag 37140

catcttgtta tgagaatgag ttttctttta caaaagcttt tccatttcat tgcatagata 37200

gtttattaaa tcctgttggc aggtagttga ttgtttttct cttattaaca gtactacaac 37260

tatgttcttc tcttattaac agtactacaa ctaacaccct tgtagctaaa cctttgcatc 37320

tccttgatgc aaaggttatc tcaacgagtt ttccttgaga taaatcccca gaggtagaat 37380

ggtaggggtc aaagggcatg ggcattttaa aaactctaga tccctgctgc taaattaatc 37440

ctaggaaagt ttaattaatt tatgtgccca ccagcactgt atgaacattt tccaactctc 37500

tcatcagcat tgggttttat aattttttga aaagtgtgct actctgcatg cacacgtatg 37560

tttattgtgg cactattcac aatagcaaag acttggaacc aacccaaatg tccatcagtg 37620

atagactgga ttaagaaaat gtggcacata tacaccatgg aatactatgc agccatgaaa 37680

aaggatgagt tcatgtcctt tgtagggaca tggatgaagc tggaaaccat catcctgagc 37740

aaactatcac aaggacagaa aaccaaacgc cgcatattct cactcgtagg tgggaattga 37800

acaatgagaa cacttggaca cagggtgggg aacatcacac accggggcct gttgtggggt 37860

gggaggaggg gggagggata acattaggag acatacctaa tgtaaatgac aagttaacag 37920

gtgcagcaca ccaacatggc acatgtatac atatgtaaca aacctgcatg ttgtgcacat 37980

gtaccctaga acttaaagta tcacaataaa taaaagaaaa gtgtgctact ctgataatag 38040

gcaaaggtat attttgttgt cttaatttac cttaaggaaa ccctttgcaa aatctgctgg 38100

tttccagttc tgcccctttg ggttcagcct ctcgtctttt ttggttactc attgccaaag 38160

cagtcacctc ttacagtaac ttctgccaac ctaatttctg aggactttga cctcttttga 38220

tgtctctcag aaagtctgct gagagagcga gagaactgga tgcttttgct ctgaattttt 38280

accgattgag taacctgggg taaacctcag tctttgtgat tttgtctcct ttttagctat 38340

aaaatttaga cagaaggcct gtgctagcca cctcacaaga ttgtactggg aatataaaga 38400

ggcaagatag aggcaagctt tttgaataga ggaaaagcaa aaatgcaaaa tggtattatt 38460

attattgttg ttactctaaa gtttagtgat aaatctaacc ttctaaaata tatagtttct 38520

ggagagctgc atgctgaaat tttgtcgtct tctaattcta aacttgggaa gtgtgtgtgt 38580

gtgtgtatgt atgtgtgtgt gtgtgggggg gggtgtgtat aacatatttc tatgcttctt 38640

ggttcttaga ttcagtagca gtgtttagtg tgttacagca gtgcatggat acattttaga 38700

ttaaaaagat ttccattttg ttgcttattt attccattct tgtacaggaa aaccagccat 38760

cctcctccta ttgcattctc atcatccatg ggctttgctc agctccccag tggactgaga 38820

gctcctcaaa gacggggtcc ggtttcatgt gtatacctct ctccagggcc tgctttgggg 38880

tctgacaccc agagggcgga tcaatcaatg tttctcaaat gagtcaatgt aagggaggta 38940

gaaaaatatt tacccagttc tgtggagaaa agaatattcc aaaaaggcac cccagaagat 39000

taaaaaaaaa cctaaatgga gcttttctag ggcaactggt ttatttattt cgtttagttt 39060

gggtgagtct cagtttctat tgagtaaata tttaaagctc tgaaaggata ttaaacaaat 39120

gaatgcaagc caaggatagg accaggaaaa tggagaaccc tgtctttata ggtgcaagtg 39180

tcatggattt ggcatgaata tcaaaaactt taaggaggca tcgattttca ctctgactcc 39240

cagctcagtg ctgggctgca aaaatcagga ccaaactgac atttagtaat ggtttgaact 39300

tggtactgaa taataataat aataataata ataataataa taataataat aaaagctaat 39360

atgtaataca gcacttgcta ggtaggagac ctggttttag gtaccgctca tataaaataa 39420

tcagtactat tattttattt tgcagattaa gaaacggaag cacagagagt gatttgctag 39480

ggttatacca gtagttattg cagagctgga attatgacgc agacaggctc actctggagc 39540

ctgtgggtca gtgagggtag gtcggactga actttggaga caatgtctca tggttttagg 39600

ccactggtga tgtttgtgct gtttatttgg gtatatgact accatttatt gagtcaccta 39660

ctaaaggaac atgcactctt ctgggttctt taagtataat atcttgaatc tttctcgcaa 39720

ccttgaaaca ttattccaac tctaggtcca gacaagctga gaccctgaga agttcagtaa 39780

gtttcccaaa gctgcaggct agtgaggaga gatccatcag cctgcaccaa gtctgtctgc 39840

cttctgagtc catgtgccac actggcctgg acaactgagt gctgatgtat ttggttcttc 39900

catccaaagg tgccaagagt ccagttctac tgcatttgga ggtgttgaac ctggaaaact 39960

cagccacaac aacccctcaa cttggggatc aggcagggct cagcctcagc ttctctgaac 40020

ccacatgggc caatgtgaaa tggatttgat ttttcagacc agatcctccc tggcaggacc 40080

atccaattta tcacaccaac ctagactcga tgggcgttta aaatattgag agatatttcc 40140

ttatatttcc tctctattga ccccccaaat ctgcaccaag gaactccagc gaatgagatg 40200

gagtatggtg agtgctagct tgggggccaa gggcggcttc gtttctcctg ggcgacttat 40260

ctcccttttc tttgtttagt gtttccagtt ataacatgag ggaaaagact ataatttaaa 40320

atggaatttt actttttttg gttaaacttt tccatatgat tttgaaactc tttaaatttt 40380

tttgagtgaa tgacacatgg tttatttgct tttggagtgg taacacaatt taaaaggtga 40440

aagtcttttt ctcttccctt tctgcagacg cttctttccc tcttcctcac caataggcga 40500

gtcttgttaa tagtttctag ttttttcttc cacctctttt gcactcatag tgtgcaccat 40560

tctatacctt gatccatagc tgtatcaaag gcggtgcatt ttctgcctct taatggacag 40620

ttggattgtt tgcaatgttt tgttatttca ggcaatgttg cgaccagtgg cagcctgccc 40680

tcgtaatttt gcagaagtgt ttccaagttg ttggttaaat tcctggaagc agaattgtgg 40740

ggtcagaggc tgtgtacact tgtgattttg atatatattt cagaattacc caccccccca 40800

ccccaccagg tcataccaat taacacccct accaggaatg tttgaaagaa tgcctctcca 40860

tgtgctcgtc aacagtgcct gctaacaagc tggtttttat tagacttcgg cagtctcaaa 40920

ggagaaagct gcaaacttag taagttttct aaattttcct atctcgtatc atgaccatgc 40980

agtgtcagcc ccactgtggt ctctcgtggc aaatacaaca gtctgacagg tactttgttg 41040

actcaggttg ggcatctatt caattggctt aaaaccattt gcgatcacaa cgtttttcat 41100

ttggtgattg tgttttgagg ccttgctgtg tgtccagtca tgggatggca cagggatgcg 41160

tggaccctgc cagggttgca tgaaacagct aggcagccgc gggagttggc acatgtgtgc 41220

tgagcactgt gaaagaaaca cagagacaga aggctgcaga gacagcgagt agggagccac 41280

ggtccgtgtt agcctggtga ttgggacggc tcgaggtgga gtgatgaagt ctgaggcatg 41340

agaaggagcc agccagacag agggcagagg aagcagagca taaaaaccca gcatgagaag 41400

cagaaagcag ctctatgtat ggccaatgaa gagagtggaa gggaggccaa ggtgagctgg 41460

agacagaggc agaggccaga gcatggtggg tgtttagggc tgtggcaagg agtttggctt 41520

ttattgtgag tttcaggttg actgtagtca tgggctgggg aagatggtgc caaggtctgt 41580

aaactcttac cttgtagatg taaattctca cctgaataac accaggaggg catggttcta 41640

tacacaagca cctgtcagac tgtgttgtat ttgccacaag agatcacagt ggggccgctg 41700

ctgcacatag ggctggcgac cagaacactg tatctcttgg ccaaccttgt cctccgtgtg 41760
agacaagtgg agacatttta caaaaatacg ttctacgtct ctctggagta caactcagtt 41820
tacaaagggc tttctcattc attatctcat gcattaataa actgactgat aattttgaca 41880
gattattatt gcatgtccaa cctgtgctaa gccctgtgct aggtgctggg gatgatgaga 41940
ggaagaagga gtggaagggg aaatagaaag atgaagaaaa ttcagagcag cttacagtcc 42000
atctgcgaga tagaatcaaa tgtcaacaat gagaaggaga gcaggaaggg gacagtgaag 42060
aggacccggc cacctctagg ttctggacca gaacacgggt aactctgccc caggggcctg 42120
cttggtgcca gggagtcctt cagggatggg gtctcaggag acagctgcaa gtagggcttc 42180
tctgacatcc ttttctccag gtgtaagtag ggttatcttt ctaactttag aaagatagag 42240
gtgaccaact gagagctttc ctcagggctt ctgagtgggc aaagggcaga actggaactt 42300
ttcctcccac ccctgaaatc tgaggccatg gctctctgtg ctcagaatag aacaaaattc 42360
ctgaagccca accacctggg gaaggcattg aggagagtgt ctctattatg catcgtcttt 42420
catgctggcc accctggttc catgatgatg aggctgcttt gtttatgagt ctgtagtgtt 42480
ttcagggctc tggagaaggg gtgggctctc taccagtctt agggtcttgg tggctctaag 42540
cccagaggtg cttccattgt gtgaagaaac acaggagacc gagacaaaat attgttgatc 42600
agacactcag gcctatttcg gcggcaaaac tgtcctaagt ggtcatttat ggccagcaca 42660
gagtggggat taacgtgtgt gtgtgtgtgt gtgtgtgtgt gagtgtgagt gtgtgtgttg 42720
tgggaggtgt ttgggggagg tctcaggaat tgctggccct gaggtgatac taatggatca 42780
ggtttccatg gacacaggaa catctgggca ggaagtagga agactgcaaa gtcagctcac 42840
ccccttcagt gagcttcacc tcccagtcca gggaaaggag ggcagggctt gcttctgaga 42900
agggactcct ggtgaattcc aggtactcaa cacagaattg ccccaatcag gtgtaacatg 42960
ggatgggggt tgtcaaggcc taatgccagg taaaagagga agcaaaaggt gtgtttacca 43020
tagaaaagtc tagaaatgta gtcataaaac ttgaaatgtg tctgtgttaa gtgtaatgga 43080
aataaatact ttaatagttt tttttaggcg ttgggttttc ctttgtctgt gtacatagat 43140
tgaatgtctg tgtgtccccc aagcccacca ctaaattcct atgttgaaac cctaattcca 43200
gtgatggtgt taggaggtgg catgcttggg agattgttag gtcatgaggg tggagccctg 43260
ataaatggga ttagtgcccc tgtaagaaga ggccagagaa ctagctagct ctctttctac 43320
catgtgagga tacaatgaga agtcagctgt ctgcaaacca gaaagtgggc cctcaccaga 43380
caccggatct gtcagaaccg tgatcttaca cttccagcct ccagaactgt gagacacaaa 43440
tatttgttgt ttaagccacc caatctatga taatttgtta cagcagccca ggctaagaca 43500
ctgcgttcaa tcagcctgtg acaatggtat aactatcttt aaagactttc agtaaggatt 43560
ttagtaagtt tcaggtctat ttcctgaact aatgaacaag tgaatgagcc attgctatag 43620

aataggctca aagcacattt gtgtcttttg tggaaatttg ccttctttta tcctttggtg 43680

agaaattatt agcggtgaat gtacttgtaa gttatccatc ttttctccga aagtgctgaa 43740

cgtcttaacc atgagtctct tcttgtctga gggaggcggt gttgcttttg gtgaccagag 43800

ctgctcagtc atgtatacca ataacatatt ccaacatggg tcacaaaacc aaagcattcc 43860

aaagccatga ttgtgccccc tgaatattga tgtggcttcc ctcattctat tcctggagtt 43920

agtgagaggg tagaacttga ctggaaatcc tggaacaatt ttttgggact tttcattact 43980

tgcgatttta tttgctgtct ccttgatctt gcttgccccg ctctaggctg attagcagcc 44040

agtctcggag gctgcaccaa tgacgctcgg cctccttgcc ctggagcctg ggtacccacc 44100

tcatctgcta ttctagaacc ggccaggatc agcagccatc catctcctcc ccatgatgtc 44160

tgggagcttt tcacattgcc tctgttcctt acacaggctt tatgattttt gatttgtctc 44220

taaggaaact gaggcttaaa aatagcaggt gagtttcttc aagtcgcggc agatcgataa 44280

cagagtcaga atttgaacat gggtctgttg gattctctca gctctgtaca catcattatg 44340

ccaaaatagt aaggaacatg aggaagggaa acactagttt atggaagagc tctttctcac 44400

atgtccttca ggcagtcata tttgggcact ggtttttgag tccattcata tcaagcatgc 44460

cattcaggct ctcttctgcc acttctaaga gttatccaaa ttggtgcatg gtacccataa 44520

actagttttt aaaagagata tgattactga aatgatcagt atgtaatatt gaagcccaaa 44580

tcaatacaaa tgaaatgcat atcataataa agtacaaatt caagtaaagt agggacttgg 44640

tagatgtttc tctggaaagg ttaaacacac tgcgtggaat gcatggggaa acctggtttc 44700

acaaatagta tcagctgtct gctaaagaat ctgtaaaacc acatgcgaag gttcacaggg 44760

gttctttcct gtagggaaaa tatgtgatta ttgtcaacag tgaaggaatg tgtactggaa 44820

cttaattgat ttaaaaagtc tcatgtgaac ctgccatgac aacctctttg aataagtttc 44880

cctcaaggtt taagcatttc tcgagattaa cgtagtgggt ttctgggctg actcacaggg 44940

agaaggggag gttacatttt tgctattgtg ataatgtggg aaattacact cggtatcact 45000

cactgtgctt gaatctttat catcaacctc aaactctggg atttatgtaa ccgcaagatc 45060

agaactctaa gcttcttttt ttctccagtt gaaatttatt ttaagttggt aaggaaatat 45120

gacatatatc tatagaacct aggctcatag gaaggtaaaa aatgctttgg attggaagta 45180

gaggcagacc tttactttag tctggaactg atgtcctgat tgtaaattgt tgttctaggg 45240

aaacccagtg tgtgtgaatg taagcccctg gagttgagtt ccctttcttg gctgggcccc 45300

agtctgccac gtcagtgctg aaaatggaca ttgctgctgc ctcggtgaca tacaaatagg 45360

ggaaatgtct cctgaatgga attactcttt gtcatctagg ccagtagttg tcaaccctgg 45420

ctgcatattg gaatcacctg ggagcctttt aaacactgat gcctggatcc taactccaga 45480

agttctgatg tgatgggaat gcatgcagcc tggcttttta aagtcctcag gtgatcatac 45540

tgtgcagtta gtgtaatgtg ggttgagaac cactgacccg agaagtagac agttgccgag 45600

tcagtaaatt acccaagtaa acaagaagct ggttagacca agcctgtcca tagtgattgc 45660

caaatcctgc ctggtgggga aatttaggac catttcctca gaaaactttg ttactggaag 45720

ttatcaaggg ctctctacct agtaagtgtg ctttcagttt tttaaggcac tgtggatata 45780

agttgtgtaa acgtagccac tttcatttat ttggcaaata tttaaacatc tgctctgttc 45840

aagttatact caatctcagc ctacttttaa gcttattaac tgaacagcag gggagagaac 45900

tcaagcaatg caaagtaggg gctgcaaagt gccatcagag agggggagat gaaaaacctc 45960

aaggtttcag agcagggaga gatagttgcc tgtttctcct ggtcggaggg atgggaaaac 46020

cctaaagaag tggtgcagta tttgggatag gccttggagg ggggagaagg cggaagaaga 46080

agacttgagc tggaggagaa agaagggttg tattctgggt agaggaaact gcacgagcaa 46140

gaaaagaggt agacattagg gaaacttgca aggaaatggg cacatagtaa acatgggtac 46200

atcaagggcg aggagaggaa cacattccaa aaggtaggat caagggagac tgaggagcat 46260

ctggaatgct atactgagtg gtctgggctt tgtttgggag gcagtggagc cttgcatggc 46320

aggttttgac atccaacctt gcagcaaacg gcaaatagga gcttgctcag cagtatttat 46380

atggcgaatc tggattattt agaaggaaat tgtgtttctg tctcactgtg tggcccatgt 46440

aggttgcata gttgcagtat cttcaacttt gcattgacaa ggtgggtgcg ttgcactggg 46500

tcgagatggt tcagatgtcc cccaatgcat tgctgctttc gacatctctc catttctcta 46560

gcttctaaca ctttgtttgt ctctcttcaa ccaccatgct gagaaaaaag tttaagggct 46620

tttcttgttc ccccacgtaa tcttggcaag taataacagt gaaccctgga gatggaaaga 46680

agtgtggatg ttaggagact ggattgctag catggcatta acaatttttg gctatgttga 46740

ctctcaagta aatatggcaa ctgcttttgc ttttcctttt caaaatacaa agataaaatt 46800

taaggggaag tgagattttg gcataaatgg ctgactcttc tgcaaatagc atttctggat 46860

taattttatt acatccaatt gctcttccat gtattccgat aattttattt gtaataaagt 46920

tcataagtgc tgttggtgat tagccaaaaa aaagagtaaa gggaggtcaa cttttgtgta 46980

gttgagatca ctttttctaa gtctgatttt aaattgggtg gtttgtactg tgttagcaat 47040

gttagaggat acaaacaacc ataacaacaa aacagcaaca gcagcaggaa ggtcatccac 47100

ccgtgctgaa aacctccttc ctcttgggtt cacttttcct tctagtgggg gaaatggtga 47160

aatcacgtta gcactttcta ctcttggtcc tggtgtcttc tttatgacct cgtaacattt 47220

tcctagataa aatttgcatg tctctaactt ctgagttcca cttcttactg ttctcatgtt 47280

cattcattat tccattgtga cgtgtttggc tgagtttgaa agactctaat gataagtgga 47340

gaaggaaaaa atgactttta taataaaagg taacacttaa tgacatgaat ctgagacaca 47400

tgactaagta agaaaataaa atattgacgt cttggtttta aatattgggc aacaccatct 47460

catgtttatc ctttttctca tcaaaccgca tgtccaattt tggattagga agacattctt 47520

actgtactta tggtacgcct gcaatcatgg tgcctcctct gtctattttt gggttgacca 47580

aataacaacg caaagcactg agtgccaacc accacaacaa ggacttggtc tgtatttgtc 47640

catatgcttg cacttccccc aaggtggaca ctgtcatccc catgaatgga tgagggaaga 47700

gaagctgaca gaacacagct ggaaagagca gggtgagagg tcactgtggg cctctcccat 47760

cagtgatttg gtgctatttg tggcctctgc aggcatggac atatcctgtt gacattacaa 47820

aataaaatgg atgcaaatat tattgtttag tgtcttccaa tttcttggat ccttaaatag 47880

ctgccaacat cgctcttcag ttatttatgt acacaaagag ctatttctga atatccgtcc 47940

taattttctt ctggcctgtt ccctgcatat taatgtattc caatgaatcc cacccattcc 48000

aaagagaact gaagcaagct tgtggcttgt ttagatgtgg tccaagtctg tgtggtcccc 48060

tcagggaggt gtagtaagga tttctcagaa ccggtgtttg cgtgtgaggg aagcctgctg 48120

agcagagagc gtttacattg cccagtgttc cgagtgcttg agtttcaata gttaccttag 48180

tttattagga ccttggaaga gtaccaaagc tgtgagttcc tgggagtttt ccgtcaaaat 48240

cagtcctctg gcatggtaaa gaatatgttt gtaaaggaac aagaaaaaaa tatataattt 48300

ttctaaatat atatatagga aatgtatata tatatacaca tataatatat atatagacaa 48360

aataaaaata tatatttgat ctctggccct gattcccagc acagagctcc taacttcttg 48420

gaatttcctg ggtgatggga gtcttttgtt ctaatgaagc tcctgggagg agaatggtca 48480

ctagaaagaa cagattagaa gcttgggaat ttcagtccta cccccatcct ctgggaagac 48540

ctggaggctg gagattgagt taatgatcag tgatgttcaa gtaacaaagt cttgtaagaa 48600

tcccagaact ctggggtttg gggagctcca gggctgctga acatgtggag ggtagcccac 48660

ccggagaggg caaggaagcg cacttcccct cccatacacc tcgccctgca tatctcttca 48720

tctggctgtc ttctgtatcc tttattgtat tctttattac atagtaagct gggaaatgtg 48780

tttccctgag gtctgtgagc catcccagca aattatgaaa tccaaggatg gggtcgtagg 48840

aaccctggtt tacagctggt cagtcagaca tataggtgac aacttggcac ttgtatttgg 48900

tatctgaaat ggggggcagt cttgtgggac tgaaccctta acctgaggtc tgtgctaact 48960

ccagttagta tcagaattga attgaattgt gggacactcg gctggtgtca ttcagctggt 49020

gtcagagtat tccttgacat cgcttcaatc cctggtcaca gaagtgtgtg gagtgcaggt 49080

atagaaaagg aaaagtaggg ccgggtgtgg tggctcacgc ctgtaatccc agcactttgg 49140

gaggccaagg tgggaggatc atgaggtcag gagatcaaga ccatcccggc taacatggtg 49200

aaaccctgtc tctactaaaa ataggaaaaa attagccagg cgtggtggca ggcacctgta 49260

gtcccagcta ctcgggaggc tgaggcagga gaatggtatg aacctgggaa gcagagcttg 49320
cggtgagccg agatcgctcc actgctctcc agcttgggca acacagtgag actgtgtctc 49380
aaaaaaaaaa aaaaaaaaaa aaagacaagg aaaaatagtt gttttttatc tatctcacgt 49440
ttattttaga ttactcaaaa taagcatatc tgagcttata tctgctgtgc agagcaacga 49500
aaacatttat gggagggtaa tggcggtaaa cactgtaact tcatgctgac gttaggtggt 49560
aacttctctt cgataattca agctacctag tgataatgta agttctttat tgcacttcaa 49620
aagtatttct ctgtccatgg agggcatttc ttctcaaaac actttgtact tccttatgaa 49680
gttggaatat ttgctatgga aaccaaattg ttcttccttt ggcctgctga aaattagaaa 49740
ataaattttg tgtggtcagt aggttattgt tttaaactta actacatgag attaacttct 49800
ggaactgaga tgccaatgat gcaatttcag aatgcagttg ttgcactcat taaacaccac 49860
agtttgggaa ggatccctga tccacttggt gctcagtggc tttctcagcc taacagggac 49920
atagtttgaa gagtcttaaa caaaagttac ataagaaccg cagatgacta gctgttggtt 49980
ccataggtag attttctttt attatcttag ttttcagctt tattgaggta caactgacaa 50040
acaataattg tgtatattta aggtatacaa catgaggatt tgttgtatgt acacattatg 50100
gaatgatcac cacaatcaag ctaacacatt catcacctta catagttacc tgttttgtgt 50160
atgcatggtg agaacagcta aaatcaactc tcttagcaaa cttcaagtgt acgatacagt 50220
attactcact atagtcacca cgttgtccat taggcccttc agaacttatt catttcatga 50280
tgaaaaatgt atatattttg ataccatttc ccccttccct ccttggcagc caccactcta 50340
ctctctgtaa atcattcttc ttgtcattag tcccctgtca gaactctgtc gtggcccctt 50400
ccaaacctcc ccctgagcta tcaaagcttt catttactgc ctcggtataa ttaattttca 50460
gtgcttattt ggccagatcg gacctacagc cctcagtggt ctttaaagtg aaaaaagtga 50520
aaagtcttta gcatgaaacc tttgatgtgc atgttgtgtg agcgtatgtg tttgagtgtg 50580
tgggtgttca tgtgtagttt catgaacgtg tgtgtgggtt ttcatgtgtg ttgagtcagt 50640
gtgtatcata tgttgtatat gggtgttata tgtatggtgt gaatgtacgt gtttgttgtg 50700
tgtgttttgt gtgtgttgca tgtgctctgt ggctgtgtgt tgcatgaatg tgtgtcgtgt 50760
gtgcattgtg tgcacgtgcc aagtacctgg catgtatttt actaggtgct tagtaaacat 50820
ttgtcacatg caggaaaggc acaattcctg cctccatgaa gctcactgtg taaactcaca 50880
ggtgaattgt caattcctta ggcacagggt ctgcgttgtg ttcatttttc tgtccttcag 50940
tgtcacacag aggcaggcaa ctccggcata aaatgtgggg ctcagatggg aaacaggtat 51000
tgtataatga aacacaaatt tgctctcaat tcaggtttcc tgaagcagaa gcttgttttc 51060
ttcaattcac ctttgcacac actacacaat cccattcact attatgtttg tcatgtatta 51120
tccctccacg ctctgcaggt gggtagggat ttgttggatt tgttcctaga tacacctcaa 51180

gtacctggaa cactgcctag tacatggtgt gcgcacaata aaaaattggt ttaatgatca 51240

aatgtaagag taaatgagtt ccggcacatg tggtccacat ccgggttttt agcagaacat 51300

aggtattatg atgtctgtca cagttcactc taaatctcag ctggtcttgg caagcagctg 51360

cttggggact cttcctcttg ctcctgtcag gccctagtgt taatctgcat cctgcagggt 51420

ttccctctta ggctgtcatc atggagctgg cccccgggag atggctcatc ctgcttggtc 51480

aatggccacg ctgtgccctt gggtccttgc tgcagcctgt gcactgtatg ccattgaccc 51540

aggggtctgg gacatccagc cacccggtgc tgaccaccat ggactttgct gttgcctgca 51600

gatgtccgcc ccccaacccc ctggtttctc tccaggcatc ttctgttcct ctggtttctc 51660

tcgcaacttc tatccccctc ctcggtgcct aggaattctt gtcagcttcc tccgctttgc 51720

cagaggcagc ccttgcagct gtggagccac gtgtcactga gcactgcctt ctggaaagag 51780

actgccattt ccaggctgct cctccaccag cccggtgact cagtttctac tccttcttct 51840

ggagttgacc atcagagctg agctcttgcc ttccttcttt ctccttctgc agatcttccc 51900

tctagctcta caggaagggc ctccctggct gtgtggtcag aaggtctcca ctgtccaagt 51960

ccagcctggc ttactctagg gtttatgggt ccagtcttcc tccatgactc ttgagatgtc 52020

aagcccagtt tggggagttc aaaaatacca ttttctctct ctcaacaaag cctagcacat 52080

tttttaaaac cacattttaa catttctgaa attaggatgc atcttgcaat ggatagtagg 52140

tcctagttat ttggcagcat tttttttctt attgtacata ttacagctaa cgatattttt 52200

gcttaggctg ttgtcttgct atggatttaa actctatact ggagtttaaa gccatgcaac 52260

acttttttgt tctcagcctt catcttcctc acctcaaggt aatagatgcc tctcctttaa 52320

aaatgtgaag ctgccaggca accagaatca agaaaagggc agccctggaa gatatacgtt 52380

tgtttcatac attcaaaaat aggattccca gtacctaaga tcaccctttg aatcaataca 52440

catggggctg gaccacctta aggccatgga ctggtttcca tgctatactt ttaaataagg 52500

tcaaagaaaa aatgtgtcca tctcacaggg gcctggcctt cctggaagaa gaaaacatac 52560

tggtcagtaa ataagaatag ctgggccctt tggcagttat agatgtaatt ttccacgaat 52620

aaggcagaat tgcattccac gcctctgtct catgagaaca taatgagtgt gagacatgga 52680

gagactgcat ttctgagtca ggcctgctga gatacaggcc taaaactttt atactgaagt 52740

cagagttttc tgtgtcagtt ctctctctct ccctgtctcc tatgcacaca tgcgcacaga 52800

ataatgagtg agtggacatg aaatagagaa gtgctgtacc tggcaatagc aactgaatct 52860

aaaaaaaaca gtttactaac aggcgtgagt gaggggcgct cagttatgag ttggtgattt 52920

taagtatcag gatcaggtat gggccaggtt tgttcaagcg aaaaattgta gttaatattc 52980

agaagggcct cgtgaaaccc aagagcaagg acatagccat gtttctggaa gaactgggac 53040

cagccaggga ggttgtctgc cagggtctga aggaatcatt cttcatttct tgtctccatt 53100

ccttgctttt ctctctctct ctttgaaaac tggctttcct tgttttcaga aaacacagtc 53160

actctgcatc tcccaagtga tgttcagagc taaacacttt gttttgactc catctataac 53220

acatatgcat taactccatg tatttcaatg taaaattctt ggggagagca tgtgaatgct 53280

gtagctttgg gtctagcaaa tattcttggc cccaccagtc atggccaagg tgtcttttag 53340

gctcatgcct gtagactgaa agcagggatc agggaaattg gtttacttta gtaagaaata 53400

taggcatgct gagcaaatat caactgagta aacatcaact gaaatacata gaaatcatga 53460

aagatggaat gcagtatact cactctattt ccttaaggta cttctgcatt ccgatgcaat 53520

gtttgttgat tcaaaggata atgtaataaa atgccaaagt tttatcaatc agagacattt 53580

gactgcacat gtaataattc catgctatct ttattatctg tgaaattgtg tttgtgtctt 53640

atgggacaag tgtcatgaaa ctcattttga gaagcaagct gagattttgt aataaataca 53700

tttagctttt ttttggaatg aggcataccc agaggtccat gtttaaaaca aattagagta 53760

aatcaaagta gcatttacca taaatattaa cggtaaatga aaaggctagg ggaaatcttt 53820

ttaaattgta tcaaaataat actttgttta tacataggac agccacagtg gctcacactt 53880

gtaatcccag cactttggga ggccaaggca gacagatcac ctgaggtcag gagttccaga 53940

ccagcctggc caacatggcg aaaccccacc tctactaaaa atacaaaaat gttagctggt 54000

catggtggcg tgcacctata atcccagcta cttgggaggc tgaggcagga gaatctcttg 54060

agccctagag gtggagtttg cagttagccg agatcacgcc attgcactcc agcctgggtg 54120

acagagtgag actgtgtctt aataaaataa aataaaataa aataaaataa aataaaataa 54180

tgtaacatga attcaatgta aaaaaattta gaaatatgcc tacagtgcct catatgggtc 54240

tttcttggtt gataggaatg agacttggca gagattagga aggagatggt gaacaagagg 54300

gagaaggaga gaggtgaaag ggatggaaag gggaagaggg aaagagaaag gggtttatct 54360

tgtgagatca cctaagtaaa gtactcacac actacctcct ctattcttga ggcagggaaa 54420

ggttagacaa gggtgctgtg ggagctggca gggtggacac ctgtattagt ctgtttctgc 54480

actgctgtaa agaaataccc gagactgggc acagtggctc atacctgtgg tctcagcatt 54540

ttgggaggct gaggtgggca gattgcccaa ggtcaggagt tcaagaccag cctgggcaac 54600

atggtgaaac tccatctcta ctaaaaatag aaaaattagc caagcatggt ggtgcgcctg 54660

taatcccagc tactcgggag gctgaggcag gagaatcgct tgaacccagg aggtggagga 54720

tgcagtgagc tgagattgcg ccactgcact ccagcctggg tgacagagca agactccatc 54780

tcccccccac cactgccccc ccaaaaaaga aatacttgag actgggtaat ttataaggaa 54840

aagaggtata attggctcac aattccacat ggctggggaa gcctcgggaa acttataatc 54900

attgtggaag gggaagcaag tgcatctgac atggtggcag gcgggagaag tgaagtgaga 54960

gcacaggaaa aactgccact tttaaaacca tcagatctgg ccggatgtgg tggctcatgc 55020

ctgtaatccc agcactttga gaggccgagg caggtgaatc acttaaggtc aggagtttgg 55080

gaccagcctg gccaacatgg taaaaccctg tctctattaa atacaaaaat tagctgggtg 55140

tggtggctgg cacttgtaat cccagctact cgggaagctg aggcaggaga atcacttgaa 55200

cctgggaggc gggggttgca gtgagctgag attgtgccat tgcactctag cctgggtaac 55260

agagcaagac tctgtctcaa aaaataaaaa aaaataagac ctcctgagac tcacccacta 55320

tcacgagaac agcatggggg aaccaccgcc ataatccaat cacctcccat caggttcctc 55380

ccttgacaca tggagattac aatccaagat gagatttggg tggggacaca gagccaaacc 55440

atatcctcac ccttgcggct tgccaggctg atggcaggga acctctcatg gtggctcttc 55500

ctcggctacc acacaaccgt ctagagtcca ttgattgttt agcacatgct gcctttactc 55560

tgtagagtgt cttgctagga aaaccacagg agagaacaca tgcttgcaac tcccttcatc 55620

atctttgcat caagaataat tcttgtctca acaacaaaca aacaaacgaa accaataacg 55680

aaaattcttc atcatatttg catcaagagt aattctagct tggatttaga ctggcacaaa 55740

caatgtctcc tgttcttctt ggtcgtagct cttgataaac ttattattgt ttcatggact 55800

gggagcttcc tggggaagtg aaggggagag tttctggcat gggttgagga cagtcatgaa 55860

ggtatgttca gaatgaagtc acatgtagca gtcaggatag ataggcaaga tacaaggaag 55920

aagtggggta tctaaaaggg agaaagaaac acagaaagtt gggccttgtt aggcccttcc 55980

agccccactg tggcactcac tgctggggta tcagctgaca tggttaggtg aagtcctcct 56040

ggtggtcttg ggtgtaatat ttcaaagcag tgctcacatt aataaaaaca aggtctaatt 56100

agttgtctgt gcttctaaag gaagactgag gaatgcgaag agggtctgta atacacagtt 56160

tcccaggggc ccatccatga acaaaactct tttgtgatgc ctgggatgca ataagacacg 56220

gaatttgaaa tggttcttcc cagaagactt attgctgagt aggtcacaaa ccattgacga 56280

ggagtggaat acagtcaaga gtgaatgagt agacaatcag gaaaaatcaa agaagaaaat 56340

agtgtcttca gctccaacca ttaccttttt tgagggactg attagaaggt ttagatcagt 56400

ggttcttaac ttcaacagcc ttataatgct ctttttctta ataactattt cataatatcc 56460

cctaagtgaa attcattgac aacataacct acttaagcat gatattaaaa atttaaatgt 56520

attaccttag ctataatgta aagaagatat aaaaggtagt ccatattcag tttcatacat 56580

acatgtgtag acacgactga gctagaagac agaaaggagt tgtcagatgc ttgagctata 56640

cacagagaag tctttaatgc agcagctgca aatgcaggct gaaacagctt gtgcctcgct 56700

ggagagccaa ataacatgag tggcattgct gttggggatg tggttttctt cctcccatgg 56760

tgaaagatct tttataaact tacagaagaa aatcaagttt aacttgtcct caattgacat 56820

ggtagctgct tttctggaaa atttatacgt attaaaactg tgcgaaaaat actttgtgtt 56880

tatatgacaa acagagctag gcttcagact cagattttta tctccattga ggtctggtga 56940

gatgtttgaa aggttttttt ttttttttaa ggatagagga caatttgttg tgctgtgctg 57000

ttctaggtgt tgcaaaatgt ctagtagccc caacttctgc ctggtagatg cacacccaac 57060

caaaaatact cccgcttgta tttaaaactc acttaggtta tagctaattc ccaggcactg 57120

tctgtagaga ttctgctcag gtagggctct agaatgtcca agaggctgca cagctaggca 57180

tgggaaccac ggattcaagc catccctctc tctgactcac cagatccctc tgacatatct 57240

tgggccagtg gctgtccagc ctgggtttta aggagctggg tttcagtgaa gaatgtcagt 57300

aggttcagtt ctcttcccag cccttttaat gcagaggccc ccttgtcagc ctagaaggct 57360

gttctaagct cctgtgcaaa cccctcttgt gaacccttgg gtgtggccat cctggagatg 57420

gttgactggt gatccagatt tcagcctctc ccttctctgc tttgcccagt gtcatgttca 57480

ggcccccggc tttgtcaaaa gagagaaaca cttttccctg ggtctcaatc ctttgaagtt 57540

ccattgggtg agtcagctcc ccacttagct tcagaaggat ccaggacagg ttgtcctaaa 57600

attaatacct gcacattcat ggacagtgca tggtgagagg aggcaaagat ggcagagaca 57660

actataactg gttgtggaaa agagttgagg gagagaagag aaaatattat taaatggaag 57720

agattgaaaa atactgaaat caagtgcaag aacctttaat gggcagagtt aaatgacaca 57780

tttattcatc caataaatat ataaaatgca tctaccacct actaggcttc attcaagaca 57840

atgataatac ggcagtgtat aaaacagacc aaaacctacc ctcatgaagc ttacattcta 57900

gagggggtag acagttaatt aacaagaaca agaagtaata tatatagtaa gtcagatggt 57960

gacacatgct aggaaacccc agtagggact tcaggtaaga cagagatgtg tgtgtgtgac 58020

tgtgtgtgtg tgtgtgtgtg tgtgtgtgtg tgtgtgtgta tccatgcaac atccaatgat 58080

ctgggatacc tatttggcaa caagagagga aaatctcttt ggcaatagtg atttggatgt 58140

tagtagtgta ggaggaatga gaaagccata ctgcaattat caggacagga aatgattcaa 58200

gtttggatag aactttttaa caatgaaaaa tgaagagaaa caaaaccact ttcacatggc 58260

aataattaaa tttctgagtt tacgtaaaga atcttatgaa acttctcatt ccaacctttt 58320

gtttccttta agttctttat tatctttgag tgtttagatt tcaattctcg acagttcttc 58380

acatattgaa tgagtttgct ctcctatcac acagagatca agttatctat gcattttaca 58440

cttggttact ttagacttga gtatttgtgt cgaaataccc tcctttttaa atgtggtttt 58500

ggttgatgct tttggaatga ggaatttatc agctgtggtg actcgtatat actctgttag 58560

ccagaaatac tagcttactc tttacccgaa aactccctat tccaaaatga catcacacta 58620

tgagtttatg cctaatgaag ctccagtaac atttgcttat ttgcatagta gaaatactgt 58680

caggtacacg aattttcagt aattttctca atacctctat ggtattactg aataggtata 58740

tcatgctcat tttatacgtt caaagaggct gagtatataa atacccaaat ataatgaatg 58800

tttggcaggt aatgatcatc tctatcattg actatgttta aatacttttt atgtattaac 58860

ttattctctc aacaacccaa taagctgggg actattgata acattatttt gccatcaagg 58920

gaattgaggc ataagagaag ttaagtaact tgcttagtgc cacacagata gtatattgta 58980

gagctgggat acatttgggg cagttgggct ccattgccta tgctagtaac catcattctg 59040

cacttcctct agttctaaag agcaaatctc aaactcctac atgttttagg gtgattttta 59100

actttacaca caatggcagt ggtcagtggt agaacagtaa aaggcagcaa tgtgaagggg 59160

gaaaacgatc agttttggag agtgtgttgg atcccggttc agttatttta ctcattgggt 59220

aatcttgggc aagttcctta acttctctag gatgtctgtt gctgtctaca aaacaagagt 59280

agtagccagt gtggtggctc actcctgtaa tcccatagtt ttgggaggct gatacaggaa 59340

gatcacttga ggctgagagt ttgtgcccag cttgggccac atagcaagac cctgtctcta 59400

aagaataaaa aagtaaacaa taaataagct gggtgtggtg gtccaggcct gtagtcccag 59460

ctacttggga ggctgaggtg agaggaatac ttgagcccag gagattgagg ctgcagtgag 59520

ccaagatggt gctactgcac tccagcctgg gtgacagagt gagaccttgt ctcaacaaca 59580

aacaaacaaa agaaatcaac aatgaaaccc caaaaaccta caacaacaac aacaacaaca 59640

acaacaacaa caacaacaaa accaaaacaa gaactccttc ccaaagaaaa gtagtaaacc 59700

attcatggtt ataagcatta aatgtgatgt cctattaaag tgtctaaatc atggtcagga 59760

acccgatagg tgcttgaaat ctagaaattt cattcctaca taagggccaa ttattgagca 59820

taggtcagat gaccagttaa gaaagctgcg gtgcagtaaa ttggaaaggg aattggtgat 59880

gttggggaat tcagggcagg atttgtaccc cctaaaggag cagttcaaat ctcaatgatg 59940

acttaggatg taggtcctta tggtgaacct gacaggggct gctgtgctgt gaagtattgc 60000

tttgaaatcg gggtcagggg tgaagcagct gttgacgagg gtgcccctct cttctgacct 60060

cttggcctgc cctttcttct tgctcctatg tagacacttt catgaactac acagtggtct 60120

cagagggctg cagaagcttt atgtagagca gacgttcttg aactttcggt ctaaagacac 60180

cttcgtactc ttaaaaactc tcagaacttt aaagagcttc tgtctacctg gttaattcta 60240

ttgatattaa gcacattaga catgaaaact gagaaattaa aaaaataaat ttttaacaat 60300

tatttaaaaa tgccgataaa ctcattgcct gttaacataa atctctttta taaaaacaat 60360

tatttttaaa aaacgaaagt atcaaaaaga attacctttt tttactttgt ttacattgtt 60420

ctggaaatct ctttaatgca ttgctcaaca gaatacagat aagttctcat atctgcttct 60480

gcattcaatg ttggagaagt tctgcatcat gcggtctctg gaaaagtcca ctgtacgtgt 60540

gtatgaaaaa gacacatacc atgtttgaat tattatgaaa gtagttttga ccttgaagac 60600

tctgaaagga tctcaggaac ctttgagaac tgataatata acgtatcttt ttcttggtaa 60660

taatgttttt gaacattctc agtattccct ctcagcttcc tcctggtgcc tgtgttcaag 60720

caatcacaat tgctaatgat tcttcttgca aaattcttag gtttggcaac cctggaaata 60780

gtagatttga tctttagctg tctccctcac tcttatcttt cctctttcct tgacagctgt 60840

tctttccttt tcactcttgg tggcacaagc ttgggaggtt ttccatccag gctggtgcct 60900

cttaacttct ctctctgcct ctagggcctg acagctgcca ggctgtcctt tatctgacat 60960

tgttcacagt ttacttcctt cccccaaagt cttggttttg actaaaatgt attaattctt 61020

ggttatgttt tataaagata tcaaaaactt tttcaaccaa agtttaaaaa agaatttgca 61080

gggtgggggt gggcatgtgt aaagaatctc aggcatcttc actgatgaac ttcagaaact 61140

gtgtcagtag atttctgatg ccagccagga ttgagtaata ggccctagat ttactttctc 61200

ataagaaaca actaaattac tctacaaaat atgcaaaacc agggttttta agaattggac 61260

atcagctaag gaaggatagt gatccctgag aggcagaaaa caaagccctg tgacttctcc 61320

tgcttactga tgtgagagtt tccaggccat ggcacaggga agcgtgaccc aggcagagtc 61380

agtgagaaga ctacccaaag tcagaaaaag cgcctcccta aaagattaga tgaaatagtg 61440

gacaggagga ggaataattc ctattctcac cagccagact ggaaagtctc gtaattcata 61500

gggatttgtt agaggactca gaagcatcaa gcttcagaag tgggataaaa tgagtcctag 61560

agtgaatggt gctgtggtcc tgtctgccga gtcttaaaag caagatccaa aagaatcaaa 61620

ctattctcaa taactcaaat gcatcctaag acacaaaaat attcagcatc taacaagtta 61680

aaatccacaa tgtttggcat tcaaccaaaa aattaccagg tatgcaaata aggatgataa 61740

taatgcagag gaaaatgagt caaaaccaac caagaactaa tagagatgtt agaattagca 61800

gacaaggaca tggaaacagt tttataaact gtattccata aacacagagg taagtagaga 61860

aacaaaagac gtgaaaatgc ctgcgtctaa cttctagaga taacaactac aatttctgag 61920

gtaaaaaaaa tgactggatg agattaatag cagattagat attgcagaag aatagatcag 61980

tgaatcgaaa gacataacaa tagaaactat aaaaaaaaac cacagaggag gaaaaacaaa 62040

caagtaaaga acagagcatc acgaaacggt gggaaaattt caagtcacca aatacacatg 62100

taattggagt cactcaaggg aaaaagggag tgacaggaaa aatatttgaa gaaataatgg 62160

ccaggagttc tccaagctga actgaaaatt acaaacccac atacccaaga agttcagtga 62220

accttaagaa acttgaggac aaacacatca tttcatcata attaaatttc ttaaaatcag 62280

cgataaagaa aaaatcttag aagcctgagg gaaaaaatac gcctaccaag aaacagcagt 62340

gacacagatg gtagcagatt cctcatagaa acaatgcaag ctagaagagt ttggagcatg 62400

atctctaaca tactaaagga aaaaattgtc aaccaagaat tttatacaca gtgaaaatat 62460

ctttcaaaaa caaagacaca ataaagccat ttcagatata taaaagctga attaatcact 62520

aacagacctg cactacaaaa atgttagagg aaatccttca gacagaagga aaaggatact 62580

agatggagat ctggatctat ccaaaggata gagcaccaga aatgccaaat tattatttaa 62640

atgactttaa atgaaaatta tttaaacaga agtaataatg atgtagtgtg aggtttatag 62700

gatatgtaaa agtaaaatga atgacaaaat agcacaaaag tcacgcaagg gaaaatgaaa 62760

ctatactatt ttagggttcc tgtatgtagg gtgatacagt atcatttata ggaacagttt 62820

tagcactaaa tacctgtttt aaaaagaaga aaattgtcaa attggtgacc atagcctcca 62880

ccttaagtaa ctagaaaaag gaataaatta cacaccaaac aagcaaaaga taagacataa 62940

taaagatcaa agcagaaagc aactaaatag aaaacagaaa tacaatcaag gacatcaatg 63000

acaccaaagc tttgatgtca tttgatgaca cctttgagaa gatcaataaa attgataaac 63060

ctttagctgg attgactgtg gattaaaaag agaagacaga acaatcaatg gtaggaacta 63120

ggtaatatca ctatactaca ccacaaatag taaaagaata ataagggaat actaggaaca 63180

aaattaggca taaaacatat acttttagat gaaatagata aattctttga aggacccaag 63240

ctaccagctt cattcaaaaa gaaatagatg acttgactag ccctctatct actaaagaaa 63300

ttgaatttgt agttaaaaat cttcccacaa ggcgcagtgg ctcacacctg taatcctagc 63360

actttgggag gctgaagcag gaagatcacg aggtcaggag atcaagacca tcctggctaa 63420

cacggtgaaa ccccatcgct actaaaaata caaaaaatta gctggacatg gtggcgggca 63480

cctgtagtcc cagctactag ggaggctgag gcaagagaat ggcatgaacc caggaggcgg 63540

agcttgcagt gagcccatat cgtgccactg cactccagcc tgggcgacag agcaagactc 63600

tgtctcaaaa aaaaaaaaaa aaaaatcttc ccacaaggca aattccaggc ccagataaca 63660

cgtcttgtga attctaccaa acatttaaag aaaatataat agtaattcta ctcaaactct 63720

tccagaaaat tgaataggag taaatgcttc tcaactcatg agttctcccc aactctgtga 63780

ggctaatatt acccagatat caaaaccaaa caggacatta catggaaagt acagaccagt 63840

attcctcatg agcatagata taaaattcta aacaaaatag tagtaaatca aatccaacag 63900

tatataaaaa agctaatgca tcgtgaccaa gtaggtatta tcccaagaat gtaagattgg 63960

cttagcattg gaaaaatcca taaatgtaat tcactatatt agtgaaacta aaaaaagaaa 64020

aacaatcata taattgtctc aacagatgca gaaaaagcac ttggcaaaat ccaacatcta 64080

ttcctggtga aaactcttag caaactgaga aggaaacttc ttcaatccaa taagggatgt 64140

gtacgaaagg tctatagcta acatcatact taatgaaagc tatcctccta ggatcaggaa 64200

caaggcaaga acatccactt tccccacttc tatttaacag tgtattaagg gatctagctg 64260

gtgcaaaaaa aaaaaaatca agatatgaaa taaaaggtat ccagattgga aaagaaaaga 64320

aattctattt gtagacaaca tagtggtcta tgtaggaaac actatggaat ctattaaaaa 64380

tctactagaa ctaataaatg agttttggag gttataagat caatatacaa aaatcagttg 64440
tatactagca atgagcattc aggaattgaa atttaaaaac aatagccttc acaatagcat 64500
ccagtaattt gaaaaacagg aataaatctg gccaaagata tgcaagatct gtacgtgaag 64560
cggtgttgat ggcagagact attctgcccc tgagcccacg atgtgcctgg cactgttctt 64620
gtcggtgcac tcaacccttc ctgtcttagg ctgctctctc tgcagtctgt tcagggtctt 64680
tccaaattac cagctttttc tgggtacttc agatacctgc atcactactt tctttctttt 64740
ctttcttttt tttttttgag acagaatttc gctcttgttg accaggctgg agcgcaatgg 64800
cacagatttc ggctcaccgc aacctccgcc tcctgggttc aagtgattct tctgcctcag 64860
ccttccgagt agctgggatt acaggcatgt gccaccacgc ccagctaatt ttgtattttt 64920
agtagagacg ggttttctcc atgttggtca ggctggtctc gagctcccga gcttaggtga 64980
tccgtccacc tcggcctcca aaagtgctag gattacaggc atgagccacc atgcccggcc 65040
tgcatcacta ctttcaagaa gatagcagag aaaacagaaa aagcaatcaa tactttctca 65100
atcaccatgt tcttcctgag ggtctctcct tctcttgaga ctgcagaagg aagaaactct 65160
catcctagta aacttgtggc tttcctcttc ttttaaactt ttacatgctg ggactcattc 65220
ttcgtgttct taggaaaacc tgggttctgg gtgcatacct acacctgttt ttcacgggca 65280
tcttctaatt tagagactgt ttccctgtgt cattattttc atgcccagat ttagctggga 65340
tattgctagg ttatttacaa gcacacagct aatatgtcaa aggaacaagg cagaggacca 65400
taactcattt ttttttcttg gagaataaac aaaaaggaat gacaaaaatg tagaacatgg 65460
taagatttta caattttatt ggctaatata atgctatcta aaatctgttt tttttaaaat 65520
caaaaggtaa taaacactct gttgctgatt ttatgaacct atgcctggga accacagtgt 65580
tgcctctgct ggatatcact gtgggggcag cagaactttg tgggcggtac ctacaataaa 65640
aaatgcttta cgctgttctt tgtagctgac tgcttctgac attctgagca ttgatcgagt 65700
tgcctatgaa tgttttggga caggggatga agacactcct cacccctaca aagtacatgc 65760
attcctatct ctgaagctct tatatttatt tttactaaaa aattagaata taggcatgaa 65820
tccatgtatt tacaaagtat tatgttaatg aatgtaacaa ttataatgtt attcaaactg 65880
agaatctaat atacagagga tatgtgagag caggtaggat ttgatcaagc aatgaaatac 65940
agaacgaatg atcacctggt tgatcaaaag gaaagaggaa gagtgtgaag aagattcttg 66000
ctcgagtacg gatttaaaac gaccagattg tccattttgg atgaagactg gctttctaga 66060
gaaggacttt tgttctagag tccctgaatt atctaaacct taagcagcta tgtccaatga 66120
acattttgcg gtgaaggaat gtcctatatc tgttctgtct aatatgatag cctctagcaa 66180
tgtgtgggta ttgagctctt gaaatgtggc tagtgcaact gagggattac attttttatt 66240
tcatttaatt taaaataatt taaatagtca tgtttccaga ggctacatga attgcacagc 66300

acagatctgg aggaataaaa gggaatccag tagccacctt ctgagactcc atgcctaagt 66360

cacactcagg ggaactctgc ccaaaatctt gtccagttct gggtcctaag tctttcatgc 66420

cattactggg gaattcactt gataggcttc tattattctc tgtctcagga ggactcaaga 66480

tcagtggctt tggcctctga ggacaaacaa gcttatattc taaatggtat gctgagcttt 66540

tcagcaggtc taactatccc agagtaggaa caactcagca ctgccatcca ggctcttaac 66600

acagtaatct acccgtgata gggcaagaaa caaagaagga atcagtggtc ctatcgcctc 66660

ttctcccaac ccctgggccc gaggcagaga actttcctag ctcccattgt ccctcagggc 66720

aatcatggat aacagtattt tttttttttt tttttttgag acggagtctc actctgtcac 66780

ccaggctaga gtgcagtggc acgatcttgg ctcattgcag cctctgcctc ctgggttcaa 66840

gcgattctct tgccccagcc tcccaagtag ctgggattac aggtgtgcac caccatgcca 66900

agctaatttt ttgtatcttt agtagagacg gggtttcccc atgttggcca ggctggtctc 66960

gaacccctga cctcgtgatc taccctcctc ggcctcccaa agtgctggga ttacaggtgt 67020

gagccaccgt gcccggctgg ataacagtaa ttcttgatgg aaagccattc acctattgct 67080

gagctgttgc caagttgttt gttctgaaga aatacaagag gaatggttgg gtgtgaatgt 67140

aatagttgga cattagggaa atgattaagc aaatatggca ctcctgcatc tgaccagatt 67200

tttcttcttc ctttgctgat agtggcctgc tcttcactct tccaagtatc tttactagtg 67260

gagacttacc tctcatgggc tctgttagtt cccagaaaat cactgaatga caacactaga 67320

aacaaaatca ttctagttaa catttcctca aaagtacata gttatagcta cttcttttac 67380

tcaaaatagt atattcaaaa ctaaacctat ttgaccatct catagctctc agcttttgtt 67440

tgctggtcat aataataatc ttaaggaagc cctttgtaat tggacattgc tcagaggtga 67500

tgctggagaa gggaggatag gtgttgttta attctgagac accttgcatg ctagactgaa 67560

gttatacggt acaagataag ccatggagaa ctgtgacaca ttcctgagta cagtcattgt 67620

tttgatgatc tattattttg ctagaatcta ccctgaaact tgatgactca aaacaacagt 67680

gtattattat ctctcatgat tttgtgggtt gatgaggctc agctaggtgg ttctcatttg 67740

gggcctgtca tgcagttgta gccagacagc cactaactag aattgactgg cctaacatcc 67800

aagatggtgc actgccatgg ctggaatgta ctttggctga cagctggggc tgtggactgg 67860

agcacctgta catggtatac atgtggctcg agcttcctaa cactatggtg gtctcgggac 67920

agtcatggtc cttacctggc aagctctggg catcaagact gagtgttcta agagtcccag 67980

gaagaacctg aaaggctttt tatgattgag ctcagaaatc ccagaaagtc aattccacca 68040

tattctattg gttgaaattg tttgaacaag tcaataagcc agtccagatt caagaggagg 68100

aaaattagac tttacctcta aattagatga gaagcaaaga atttgcagtt atctttagtt 68160

taccacattg atcttattaa aaggaagcca cctagagaaa atacattgac agttatattc 68220

agaaagatta aaaggttgaa aggctccaac acaaaaatct agattttgga aattgcttgt 68280

gtaaaaggaa aagtgacaaa acttagagat caattacaaa aaattgcaga aaaccaaaga 68340

tggtgggact gtgtggaatt acaagggaaa gagtattaca tttaagaata agtttaaaaa 68400

gggttgtatt cttcctacat gtctggcata tgatggagaa cagaggttag aacaggcact 68460

tcttggatga tgggaagggc tgccttttgg gtgaccatag acagttcagt tcctggtgct 68520

tcaaatttta tgtgttaaag caatcagaag ccagtggcat gagagacaga ggccactaat 68580

ctggttctgt taggtgtaac agtacttgga tgtcgccaag tctattggcc tctggaactt 68640

gctgtgctat atctattaac atgggaaaaa ccaggtgcaa gaagcctctt tcactcattt 68700

gccttcactg agcaagattc aagctgcaat tagcacaaac agttggctgg tttgcctgtc 68760

acttatgtct tatcttcctc ctcttattaa ggaggttaag aaatggttta ttgcctatga 68820

ccgtcttatg aaatatgtca cactttctca tcagctctta tttcctgttc acatgtggct 68880

ttctggttgg taaaggtttt attaaatcag aggaatttga acggaaagat aatcatagta 68940

gcccaccacc aattgcctag ggaaagggag agtgttgagt agattgaaat tgagaagttg 69000

tagtgagaaa aagtaaggcg aaatgtagat aagaaaatag ttacttgaaa gtcagcagat 69060

ggaaaatgta tgagaggagc gataaaaaac ctaggctgta gaaggaagtc catctaggac 69120

agtggtttcc aatctttgtt gtggttctgt gagagtttac tggcagtgcc ataaataaca 69180

aaaatatgtg tagcaaatta agaatcacag aggccctctg tgcaaaaatg taattttctt 69240

tatttcttta caattgaagc caactagact taggaattct catttccacc tgatggtaga 69300

aaaagatctc actaatttag agttgttttg tccccactgg ttcaagactc agtctctctg 69360

ggggtagtaa cacaatctct tcagaattct ttccttccaa accctgcatt tgttttttct 69420

gaaggatcaa tggagttctt tgggggtggt ggggctaaag tggaccgagc tgaagaatgg 69480

ttgtcctggt gtcccgggag gggttctggt ccatgagtta tcctctgttc ttgaaaactt 69540

cagctaggac catgcttgtc ccatcagact aggtccaagg gctctttggt cttcttagct 69600

tctgcactgc cccctttgag tgtctgggga ctctcagctg ctttacttga acactctggg 69660

atgcactgga cttttctcaa ggcagcctgt cctcaggccc aggcatatct gctgccaggc 69720

ctgctgtttg gctcaggcca tgttggacac gggggctttg ctatgaccct ggtggtgtcc 69780

agaactaggc tgggaagcca aatggcatcc ctctcttcca gtcttctcca tcaacctctt 69840

cagtcaggag gaggccaggt gatgcagttt ggatatttgt ccctgcccaa atctcatgtt 69900

aaaatgtaat ccccaatgtt ggaggtgggg gctggtggga gttgtttgga tcatgggggt 69960

agatccctca tgaatggctt gggccatccc cttggtgata agcgagcttt tgctctgagt 70020

tcacatgaga tctggtcatt taaaagtgag tggcacctcc cgcacctctc tctcgcttcc 70080

tcctgctctg gccacgtgac gtacctgcac ctccttcacc ttctgtcatg attgttagtt 70140

tcctgaggcc tcctcagaag cccaacagat ggcagtgcca tgcttcctgt atagcctaca 70200

gaaccatgag agaattaaat ctcttttctt ataaatttcc cagtttcagg tattttttta 70260

tagcaatgta ggaatggcct aacacaccag gatttctctt attaggtggt gctgaagagt 70320

ggaagctctg agtcactcct cagaaacctt tcaggtctta gtaaagagca gctccctccc 70380

tctgcttctc cccttcccac ctgctggtca actctgttcc ttggagtggg aacaaaaaca 70440

aatagcttta cattaccata cattcttctg tactccgtgg tttatgatgt aaaccttatg 70500

gtctaagttg tccaatactt tggctttgga ggttcaagga aaaaaaacat aaaatacttc 70560

ttctttcaag cctagcctta atgtttataa tggacagtct ttcaaaatgt cctgccacat 70620

gggccatagg agatgcttta tttttcaggc attggattat gttgtgttgg tcaatatgat 70680

tttgtttcat gtgcattatc agaaactctt cccaaagagg gtcctgggat ctgatcagct 70740

agacaagtgg aggagaaact gaaaaccctc aggggacagg gtctcagtta agtctcctga 70800

atgataagca tttcagaggt gggagggaga caccccggaa ggtgtccaga ggctaacaat 70860

gctatttctg tttcttgtaa gtttttgtcc aagatgtggg ccattggatt gcatcactag 70920

catacaatgt cagtgtaagc agtaggcttt acactaaagc atgttgatgt gctcagagca 70980

tttttgtggt gctgagacca gagcagatag ggatacaggg atgacagtct ccaccttacg 71040

gaagcctcct acccagggga tggagtgaga caggcaggca caccagttat ccaaggaaag 71100

gaaataaagg gaaggagaaa gtcagtgaaa gtggtcagga tggctgagtg agggagaagg 71160

tgacttgaag gtcccagata gaacagtggt aataaggtga gaaagaggag gtcttccaat 71220

tccatttctg tgttgggcgg gtgtttgaga gtcccacctg ggtctagagg cctgcatgtc 71280

tagcaggaca ctgggtagga gtggctgaac ggggacaaca cttaaaaaag acaaaaatta 71340

cggattttat ttctacatgg ttgcacaaat caatttcact ttctacttgt aaactgctta 71400

aaaaccttca ttatgaaaat ggaaggacga atttatgttc accatcatag tgcttctggg 71460

ctggttccac agcatggagc caggcccacc tgctgtagct ttcagaactg ccatttggac 71520

acgtggttga agcagccttc agttggagga ttcacagtaa tggcatcact gtgggagctt 71580

agaggaacaa ctgcttgcaa ccccagctgc actggaatta cctgggagct ttaaaaacac 71640

tgccttttgg gcctcagccc cagagactct gacgtgactg atctgaacac aggagtgtca 71700

tgtcctccgg tgattctgag tggagtcaag gctgtgagac cttctgctgt ataaaggaat 71760

attgagagct gggattgtta aggaattctc ttaatgctac gtcactggcc tctgatgtaa 71820

gcctgctttc tcgaggaaaa ggaaccataa aagcttctgg aaacagattg tttaagaatg 71880

agaaattgtc atcatgccag gttgcaagac agcttttagg gcagatgagc ttctgctgct 71940

cattccttca tcttttcctc cagcctggat ttgtccgggg gcagctgagg ctcaggttac 72000

ctcaattctt gagtttctga agaggcatat ccaggggtgg gttacgagaa caagagcctg 72060

cccagtctga aagggatgca ggaaggggcg gtgtgtacta gaaggaagga agctatcatc 72120

atttattaaa actcttcttg gaggatggcc catgaagacc tccaaacaag ctggaggggc 72180

cagtcacttg ctgaagacta gcgaagtgga gggggaaagc ccgagggagc tgcagactcg 72240

accactgcgc cctcccctcc tctccctgca aggagcccaa ggtgtgatcc tgccaggcgt 72300

tcgttcgctg catgaccttg gccatccttc cattcccctg ggtgtgaatt ctcattggca 72360

aagtgagctg gtgggcataa gtgggtttta agtttaaaaa tttaaaaacc ctgtctgccc 72420

ccaagtgtgg tatcaagatt ttatagtatg acacttaaat tgcttttttc atccgggcgc 72480

gtaacagcaa caatgaaacc agcagataac gcgtgagtag tatcagctct gggcctggca 72540

ctattttata tgtattagct catttttttt aaaaaactgt tttcaaccac tccatgagat 72600

gagtgctctt atgatccctt tttcacagaa gaggaaacgg aggtacaaag aaattagtgc 72660

acaaagccag tcggagagag cccctggcca ggcaccaagc tccagaggtc gctctggcga 72720

gcgtttgctt cgggatctga tgccctggag tcgccaactc aattcgcggg tcgcagccag 72780

gctccatggg ggtagtagag ccaggtcgta gtggctaggt gagttgtctc aactaactct 72840

agtggagccg ccgcagccct ggaggagccg ggccgcgact cgagagcgcc cggcagctct 72900

ccaatgcttt ggaaccggcg ggacccctgc gggctacccg gggcagggcg gtgtccgcag 72960

gggtctgtcc agccgcgcct gctcctccgg gtggagagtt gggacacccg gccagctcgc 73020

tcgcagcccg cggactggcc cagccgagcc gtctccgccc gttccccgcc cccgcagcgg 73080

cggggctaga gccagagccg cgcccggcag ttcccggccg cgagggcggg cgcagcttgt 73140

ggccggcggc cggagccgac tcggagcgcg cggcgccggc cgggaggagc cggagagcgg 73200

ccgggccggg cggtgggggc gccggcctgc cccgcgcgcc ccagggagcg gcaggaatgt 73260

gacaatcgcg cgcccgcgca ccgaagcact cctcgctcgg ctcctagggc tctcgcccct 73320

ctgagctgag ccgggttccg cccggggctg ggatcccatc accctccacg gccgtccgtc 73380

caggtacggg gaagctggag catccgggga ctaggtaggg gtgagacgga ccacagcctt 73440

gacaccttcg agtcccccgc tctgcaggac gccgtgagcg tcctccctaa gttgcacttg 73500

gttgtcagag tgtgttgtgt aagaagggga tggcggtgag gtctgcgtgt gtgtgtttct 73560

tgtgtggagc aagagttaac gttgttcagg tcgtttgaag gaacttgcca gttgttctgg 73620

aggggggttc taggtaggtg tgtgacttcc cgtgtgaatg gctttcaggt tttctatccg 73680

gctacgttct acaggttcag gagcgtccct cttagaggtt aggctcagga ccgtcagtct 73740

ccttaacaag gtaagggctg aggccttaac cacactgggg agggtaactt gttgcccctc 73800

cagataaagg atggtgctag ggaatgtagc tacaagcaag taggcgctga gaagacactc 73860

attatttaca acagtcatgc ccagcacttt acagtttata agcccttgac agcaactggt 73920

cttttaccct ccctacagtc ttgtgaggag ggtgctgtca tcacccctgt tttacagcgg 73980

gggaagcaga agtttaggtg accaacttgt tggaggatga tgtcctgaag gagggtaaga 74040

cctctgcatg cagcccccct ctgtcttccc tcccactcgt cctgcctgcc agggctttct 74100

cctctgcact gccgggagga aggaaggccg ctgggaaggc acgcagatgc tggctcctgc 74160

tgggctgtct ctccactgcc ttatctttgg ggcagggtag cttctcagat ggccacattc 74220

ctgtgattta gctttacatt ctaggaactg aggcaaggga caccatgaac ttgggctctt 74280

gaggtcatgg agcaagggtc cattcgtcac gggaatgtga tggatgtctc agtcgctgcg 74340

gtttggtgcc aggcttctgg acattgaatg tatgaaacag aactccatga ctattgatga 74400

gctgaggaaa gccagaggtg gcatggggac gttcactgtc ctttttgcca tccagtttac 74460

caagcggcta ttggcattgt tttctttctc aatcccttct aatttattgt gtgatatctc 74520

atttgatatt tcatttaata ttctgtattc atatccgatg ttaacgttta tgctagacag 74580

aaaggtagag cctttgagaa tggggtttgc tggggcaggg gtgtggaact ctgatttatt 74640

ttcagtctct ggctagctgc caagctgctg tgtgtgaagc gtctcctgca cccgtccact 74700

ctcatgtggc ctccatgctg ttcaggaaag gatatttgat ctgaattctc acatgacctt 74760

ggcaggccca gagcagcacc cccatttcta ataggccctg cttgtatgtc ataatgtgac 74820

atcaagtttt ttttttatgg ttctgaaacc tgtgacatct tttgagtcaa cctctcacca 74880

ttgttgaaac actgcttaat agtagattca ctaatttatt aaagatttat tgactatcta 74940

ctatgtgcca ggcactgtgg tgagtggtgg ggagacagcg gtgaatacga caagtgaaaa 75000

cctctgtccc cttatggaca tgagtcttct tggtaagaca taggatgtca gcagtctctc 75060

ttcaaaaggg gggtctttga aatccgcccc tttgtacaag ggtgtggatg cactggctac 75120

ctagaggaca ccactcagca gccccaatgg agaggacgag gagatgatgg gcacagatgt 75180

agctctgtgg ttccctagag aatcgcaggg ctgctattct ccaagtttgt ggtgtccctt 75240

gcctcagttc ctaggatgtg gagctctagg aggtggagct acactctgtg gcttggccat 75300

gcacagggac caggcagatg ggaaaccctg tggctgggat gcatcagtgc gagctgagtg 75360

tccacctgca agctgggaca ttatggagaa gacatctcag taataagaga ggcaaagctg 75420

gacaattgcg gtgaaactac catagcatag tgggtggaga agtacagaga tccttatatt 75480

cttatgctac ataatggata tactaggttc ttgccatttt aaaagaagtt gtcaggggca 75540

cctgtggctc acctgccatg gcacagctcc aggatgctac ccatctgttg ccgtgaatgg 75600

gtgcttggtt tgcattttct tcttcttgag gttgtgaggt gggctggtgg cacaagacaa 75660

ggatctgcac tgtcagcagc ctttttctcc tctggttgtc tgctgtgggt ccaggaaatg 75720

ctgctggagc ctcccggcca tgcctagtgc agggagcatg tgggaagccc tggccccgag 75780
ttatctcacc aggtactgaa aggggctgga gctctttaag gtcccagtcg cctgcatgtt 75840
ggattctgtg ctgtctcctc ttcatgcaca tccaggagct cacacatggc ggcacgatgg 75900
agacacctat cacttcccaa gaggactcat gcccacgagc ggacaggggg tctcataaat 75960
taaaagtagg gtaaaaggaa aacaagagcg gagatgaagt ggagtctgaa atgaggcttg 76020
acatgtgcag tgtaaaccct gatgacctag atgctgctaa agttactcaa gtggggccgt 76080
gaatgtggag ggaagcctga gtcttagctc tgcctgcacc ttagcactgc ctgaagcact 76140
cagggaaatg ccaggactgg atgcagagca aacaaactag catctctgga ggcgtgctgg 76200
gcacgggtat tttcaaagcc ccccaggtag ccaaggttga caacataaat acataaatac 76260
tctttcctgt aggcagtggg aaagaggaaa ccctgcaggg cacagccagc agtgtcttca 76320
agatgaaagc aaagtgctca ggaaaaaaag cattcttggc acagagagaa gtgggaagtt 76380
tcttttaaag atccctgaaa aagaggtgag aatagtgagt tttcggtggt ctcttgaaat 76440
gaccctccct gtaagcacag ttcggtaatc atggttcagc gagctgtgaa caggaagagt 76500
ctagccatgc tggtctctgc acatttggcg tcccaaatgt gagaactcat agatccctga 76560
aaatacagcc aatgactaat gactaagagt taaatgaaaa gttagatggt gtcactttcc 76620
atcagtgggt ctcacctcat taaaacccaa cacccccctt aatgcaaata ttttaaaaca 76680
ccccttttat aattttgaaa cgatataata aaacttatct acaacataag tttaaaaaat 76740
caatgtaata cttagatgtt aaggagaaat aaaagcaatt tgtattaaaa ttatgcattt 76800
cagtgtgcag atgcttgagc taagctgaac tacaacctag tgaggtgagc cattgctttc 76860
tatagtctgc aggacagctg gcatggggtg ggaacaggtg agtctcagtg gccatccaaa 76920
tgccactgaa ggatgaatga ctctcggtta aggtcttgat ggtagccaaa ggccattctt 76980
ctctcaattt tcacagtgat tgcattcctg aaaaatttag tgtgtataaa cttgtgccaa 77040
caatatatgt atttcatatg aatgtagaag ggagttgggg ggctaggctt caaacattgt 77100
aaatatgttt ccgtcagtat gaacgtctgt gagacctctg gaggtcatgc aggacaagtc 77160
ttttctgagc agaactgcct gtgttgttcc ggatgtcaag catcgatggc ctccaatgct 77220
aaatgcatcc tgatcaccca gggtcccctt ccccacaggt tccgaaatgc tccctcaagg 77280
gccataccca ctccactgag aaccactgct ttccctgcat tgctgggtag tgagtgatta 77340
aagtgccatt gcaggcgtgg gtgccgagat agcagggcac cggacatgtc taagccagcc 77400
atcaggaatg cacttccgtt gcttgctgta ggatggtaga ggtcgagtta gaaatcagaa 77460
aggttggtgc tttgtgtgtg ggggtgaagg gccacaaggg aactttgagg ggtggcagaa 77520
atggtctaca tcctgaagta ttcctggtgc acacagttgt tgcaatttat ggaactgtcc 77580
acgtaagttt tgtgtgtttc accatgagca atatggtgaa aaatatttac cccaataaaa 77640

aaattataga agtgaaaaca atgtaacgat tcttgtagag aaggcttagt cctctgaaac 77700

tgtctgtgga tcccaatctg agaaacagct gttcttgcct gactgggagg cagtgctcca 77760

gggagaaagg tctctggttc tcagtcaact ttttgacaag tccagtgcaa tagtggcctt 77820

ggcatcgtag accctggtgg ctggagagga gatgtgttgg agttataggg gaggctggga 77880

agcacaaggc agggcattta gaaaggggac aaaggacaag agatgaggcc catccctgtc 77940

tctgggttct ggttctgttg tccaggagcc ctagaaggtt tgtaggggtg agatggaaaa 78000

gtcagacttc aacctagaac ctagtggtca agaggcttgg ggtaggtcca tccccatctg 78060

tggcatctgg tgacggtgat cctgtctttc tgtgtctcat tgccactttt gcccttaaaa 78120

tccacatgcc ttagttgaaa ccaccaatct tgtggctttt cgccttgtat tagctgaata 78180

attgagattt catagcgaca cttgaaacct cttgggaaat ttttacttta tcttcatatc 78240

cttgtgcatc ctcaatgata aaaataaaac tttccttctc ccatttacat gggtaaataa 78300

gccattgata actatgaaca aggctttagg attcctctac tgataggagc attgccaggc 78360

ttggtgtttc ctgggctttc tgagatggtg agtgagcttc tgtttccctg ctgtgggagt 78420

gtttactcat agtttggtag agctgtttct ctgggggaaa atcagaggtg accacattga 78480

acacacagtt gggtttttgg gttgtgtgca catgatagag cgtgggtgac taatctccac 78540

gagatggaaa ccattgtgtc acctttggaa cacactacag tggtccccag cacgctctgt 78600

ggttatttcc acactttcct gctctagctt gggtagggaa gttcctgggt gggctctgtg 78660

atcattaggg gtaggtcact ccctctaatt tgccccacag gtatgagtgt gtggggctct 78720

gatgcttcca tttctcatca gaattgcata gagattatta tggacattta cttaggacag 78780

tgcctctcgg attttacctc ccagggattc cttctgggaa ataaaaagca aaggctcaac 78840

ctagcactcc aaagcagccc actgcaagtg atgcttcttt gaagcctcat gttttgtctt 78900

agatcgtgga gatttttctt tctgcttcat aatatagccg tgtttgctct aatagaaaaa 78960

taataactcc cctgagaaaa tctttggatg aaattagtaa cctagggaga tagacttcag 79020

gctctctggc tgtctgtagc ttctccctgg gaatttgtgg attctaagcc agggcttgtg 79080

tgagtatgga ggtctaaagt tttttccata agtgtgtggt tctatggctt tgtaagtccc 79140

ttttaccttc tttttcaaac tgacattttt gtttttaaca atgatgggga ctttttaata 79200

gctgtatgaa tcttataaca aaggaataac acaggaggtc aaggctgcag tgagctgtaa 79260

tcacaccact gcactgcagt ctgggtaaca gagcaagact ctgtctcaaa acaaacaaac 79320

aaacaaacaa acaaacaaac aaacgggtaa caggaagaca tcataattat gggcttggtt 79380

tcagtactgt aacacattcc ccgtacatgt ttccaccgat tttcagtaga acacatgggc 79440

tgtgacattt catgagctaa agaacctgct ttagcccttg ttttttcagg actcattcat 79500

ggtcacttct cagttatggc aaataagttg gagcctaatt ttctaagtat ctctccatta 79560

catcagatct acacatatgc acatattggt taatatttgg gatgaggggc tgtattaaat 79620

gatgctgtaa atgcatttac tcattactcc atgggaaggt agaagagtgt gttgctatca 79680

ggtggaaatg tagaaagtgc agtgaggatt ttcttgtaaa tctcagattt ggggcttggc 79740

tatgtcaggt ccctggaaga aggaggagtc atttactgcc gtctgttctc tcctcttggg 79800

cctctgtaga gatttggccc tatgccttgg cgggctctgc tctgcagctt ggccagacac 79860

atccctcagc atggcaagaa gtgtggaagg tcaagaggat gtgcccactt ggcctaatcc 79920

taacccacca gcacccctgt tcttaaccat gtccctcatg tacctgggat ccctgaattc 79980

cctccccaaa caccctggac cctaattcca gggcctgtgt ggtcattccc atggactcaa 80040

ctgcctggtt catagactgg ccagggctgt acagaggggg atagacacag gtggacttcg 80100

acacctacct gtgtgcatgc tcagggcccc ttccaatatg gaagggagct ggggagaggc 80160

tagagacaga ggaatggaga ttaatgtagc tagacacagc aggagccatg cggtgggact 80220

gctccgtgca ggtctcatgg tgaggacagc gcagcctatg ttttgacaga catggaatac 80280

caggtgccag gatgagcttc ccccagctct gggcacctct gctgccgctg tccccagaag 80340

tggacctcca gcaggttctg atactatctg gggtgcgcct gctgtgtgcc accttgcctg 80400

tgcctagctt ctatcctaga aggcagggct cacaatgtgg gggaattcct caggaataag 80460

aatggaattc ctcaacattg tggaggaacg ttgtgggaaa ttcctcaaga aaaagaagct 80520

gtttaataaa tgcactgtgg ctacacagca ggaaaaatgc ctccttcccc tgcactgggc 80580

cctaggcctt gaggaagaaa cctgggttcc tgtgacagcc cctctctccc cagctgtcac 80640

ttttcccgtg cgtgcactgg gtatgaaatt ccttgtagag ggacaggaag gtgctgttgg 80700

atgaggtcag tatttcctgg aaggggggct tctgttgggg gccaatatgc tcattgttaa 80760

ttcttgtact taatttctac ttttgtaatt tgtttcacat tacagtttct cattctggga 80820

ctagtaggaa attactttat gcctcaaata tgcattttca ttttgtcttt aatcaacaag 80880

taactttgtg tgtgtgttgc acgtgctgct gtagtcacgt tgtgatacgc cagtgttacc 80940

aaatacgtca tgatcaccaa acagcgatgg gggctgtatt tgggaagatc ttcacaattg 81000

caagcttttg gatgtgattg tcacataaag ccgtggtgtg ctgcagtgaa tggacaaggc 81060

ctccggaagt ggggatgacc agcctaggcc ttgggtcacc ctaggatctg gctgactccc 81120

tgggtctgga catcagcatc tcagcactgc ccgctgtctt tcctggaagc tgctatggag 81180

aggatgttag ccagtccagg ggtttcacac ataacataga tatccttgct tgaaggctga 81240

acagggggaa ccgtatgcta tggcttatta atgtattgag taagttaatc cttttctctt 81300

tcttgtttta ttttatgtgg aaaattcaat ctgaaaaata acagtggcat acacacatct 81360

gtgacttttt aatgttactg gtattttcta aaggtacgtt ttgaatctta ctataagtca 81420

tttaaaagaa ttacaatgga ctttgattaa ctagttctga ataagagagg tttctttacc 81480

tagtatgcct tctggcagta acatttctta gtctctagtc ctattcctgt agttccctct 81540

cctcagggtc atccccaccc ctcaactctt tcgacagcca tctgggtgtg aactctttat 81600

ctaactcctt gtgtaggatt ctaccctctc ttatatgatt ccggccatct ccagacccca 81660

gtgacggttc ccttgcaatc ctattctctg acctacaccc agtaccctta ttccatcctt 81720

tctcttcttt gattctaggt tgggttttca tccccgtgtg tctcatgcaa aaatggtggg 81780

taggtaggta ggtggattcc ttccctccct ccctctctcc ctccttcctt tcttccacaa 81840

gtatttattg agctgttaca acagacatgc aagatgtgta ggatgggcct cctcatgggt 81900

gtgggagagg ggagggtctg ctctgcactg ctttcttccc tggcccaggt agggaaggcc 81960

aacaactagg gagctaccca atgttaccag agccaggggg cccagagcca gcagttcaag 82020

gcacctcggt gcattgcacc agggagagcc tgtgtcactc gtcatgcctg gctttggagg 82080

agcagatgtc tgcaagggcg ctgtgggaag cttctgatca gaatcagctc tgagatggtc 82140

agaaatctca gaagcttcca ggctaggatt ggaagaattg acttccagac ccaggaggtg 82200

ccatagaggt gcatggaggt ggaatggcac ggtgcttata aacggcaagg tagggagcca 82260

gcgctggaca cagagccagc tgaacagtga aggcagaaga gtagggggcg ctgaccctca 82320

tcccaggggt caggcagaag gggccgatgg gatgggtcag gtgctgcaga gatgttcagt 82380

gggctggaga cagaggggtc ttggcatgga gaacctagga gggatagtgg cctgggcagt 82440

gcatctcaga ggagcagtga gcttagaggg cacatggtgg tcgcttcagc aatggatgag 82500

tggtggagac agtaagtata taaattctta agtgaagttt ctggggcaga aaaggaggga 82560

aaatgaggcc attagaagag tgtgggggcc aggtgcagtg gctcatgcct gtaatcccag 82620

cactttggga ggctgaggtg ggtggatcac gaggtcagga gatcgagacc atcctggcta 82680

acacggtgaa accccgtctc tactaaaaat acaaaaaatt agccgggcgt ggtggcaggc 82740

acctgtagtc ccagctactc aggaggctga ggcaggagaa tggcctggat gcgggaggca 82800

gagcttgcag tgagcctaga tcgcaccact gcactccagc ctgggggaca gagcaagact 82860

ccgtctcaaa aaaaaaaaaa aaaaaaaaaa aaaaaaaaaa aaaagaagag tgtggggagt 82920

caccttctct gcaagaagct tcctgggcct cccaaataaa tccatccctc ccctccccct 82980

ccccctctcc cattccatac attttggtgc cattcactat ataattccca gcagctgctt 83040

tcatatctgg ggcatactca caggggtcag tcaggagggc actgtcctag gactgggagc 83100

gggaggatgt gggggtctcc cctttgagtt tctgctatga tgtgattcca attgatgggc 83160

caacacacct ctgatacctt gtgttatgct gtgtactgcc tcaaagtgaa taatagcttg 83220

ggatttagtt tgtgtagtct aaggaaaata ctttgtgttg aactagtcag gctgtgttat 83280

tgatggccat taaaaggcct tgtgcttagg tcttacaagc ttttcaaagg cccaaagaaa 83340

tgattcagac aaaaaaaaaa attagactcc aaaatactaa aagaaaactg caaaaacagg 83400

tgaattaatg gtgaaagatc tgtaggacat agcagtaagg ctaccacaac ccagcttttt 83460

gaggttgtct taaattaatg tgatatgggg tgtaggtatg ttaacatgct tgataatgga 83520

agagtgagga tacctgcttc ttaaacaggg cctgctcatt tttttctggc ctctagtaca 83580

tttctttctg gcaaaactca agctctgttt tcacggagat gatgcactga gccatggcat 83640

ccctaggtgg tttgattcat ttctgcaccc cggtgtctag caaagtgcca cgtgcatgtg 83700

tttaatcaaa atagcttgtg gttgtcaagc tctttgtatt cttccctccc tccctgtgat 83760

ttgatttcct gaaagtatcc tttccccaag gattatggaa catacacttg ttaaacactg 83820

gacccatttt catgatcatt taaatgtcat gacctttctg acaccttgat atggtttggc 83880

tctgtgtccc cacccaaatc tcatctaaat tataatcccc atgtgtccag ggagggatct 83940

ggtgggaggt gattagatca tgggggcagt ttttcctatg ctgttctcat gatagtgagt 84000

gaattctcat gagatctgat ggtttaaaag tgtttggcac ttcccccctt gctctctctc 84060

ctgctgtcat gtaagacgtg cctacttcca cttccaccat gattgtaagt ttcctgaggc 84120

ttccccagcc atgcggagct gtaagtcaat taaatcctct ttcctttata aattacccag 84180

tctcagatat ttctttatag cagcgtgaaa atagactaat acatgcctct tgagatcctc 84240

acagcaattg gtgcctttag acctcttcat tcacacttca ctcatgtgtt cttccttccc 84300

caggtagacg caccctctga agatggtgac tccctcctga gaagctggac cccttggtaa 84360

aagacaaggc cttctccaag gtaacagggc tgagtcaccc cagggtccca aggccggcct 84420

tttctcacaa atagctgaac tcctccactc tcagatccag gggccctgga ctatctccca 84480

ttgtatgcaa aaatatttta tttccgttga cacattactc tttggggcag cttgatggtt 84540

ttattaaatt ttggaaggag gcttcagccc cagtaacagc caatgcctgt ttgctttccc 84600

aggtcacctt gactgtgcta cagtagagac ctgggaaatg atgggttcca gtgctgtggc 84660

caagctctcc ccagtttcaa ccccttccag gccttgctgt ccatttttac tccactgtca 84720

ccacatgttc aaattcttgc tttctgctcg agtcagtact gctcacctcg ttccaagaga 84780

tctgggtcta tgacactgtg ggtgacaccc tctgagcatc ttttccgagc tgtctgtggg 84840

tacctgaacc tgccccccaa cctggccctt gccctacctg ccccgacctc agcgagtctg 84900

gcccagtctg tccctgcttc tcccctctgg ccccaagtgt tctcaaatat gcccccgcct 84960

gcctgtctgt cactgcagct ggcccagccc tcatcctctc tcccatgaac ctctgtggtg 85020

gcctgctctc cctggctccc ctgtctccag tttatgttcc acacaggtgc caaaatgtct 85080

gcaaaaaatg gagatttaac atacactagc ccttggctta aaacctgtac atgcttctct 85140

gccacctgca aatgacagtc ccatttcctc agcaaacctc tgcatgttgt gggaacttat 85200

ggcttctcag cctcccactc ggaacccacc tctggtttat gctccagaac atgatgttgt 85260

tggaccctcc cctccacctc ctccaggctg agttagcctc tgtctcccct cctgccctgg 85320

gcatggctct tccagtagac acccccacat cacagtctaa ctctgtgtcc acattccctg 85380

caactgctga gctcctggag ccaggccctg ggaaccttgt tcctgttttt gttcccagag 85440

ccaaatagat atttgatgta tgattgttga atgcagcagt gagtgaataa agacatgaat 85500

ggttaactga aaacatttta tcaaaactgt tatttgtcat cagagcatgg gccggtttcc 85560

aacagctaac atttaggagt gaccatttgc caggcactgt tctaagccct ttgcatgtgg 85620

ccattcattt cacccacata gcgccatcaa tgacatgctg tcactgcatt gcttgtcaaa 85680

agggggaact gaggcacgga ggctgagctt ttactcaaca tgccatctat gttgagggac 85740

ttctgacttg gatagccgtt caactgcgag cactatggag gccagtcagg cctggccctg 85800

ccttggaatt tctgtgttat tcagggacat aatggggttt gtgttgattt ttgttgggag 85860

tgtgtgggtt aatctaggtg caaccctcac ctccttccct tctgatgtcc tcactcccat 85920

tggtcatgtg acccttaacg cccatcccaa gctcagtgcc aattccttgc cccagggtcc 85980

tggggattga catggatccc aaaagcccgc tgctgagagg acagggccac tcccaccccc 86040

ctgactgttt cccagagcag ctctggggca gaagtaaact tggacccagg gggcataacc 86100

ctttgccctt ttatagtcta ttagaggagg atgaggtctg acatcagctg gttcattgcc 86160

ccttgtgccc cttgagcaca ctggggccca gggagagtca catccctgcc ttgtggtaca 86220

ggccaccggc cctggacctg tgattaaaca ttgtccacaa ttaatttgga attattatct 86280

cgtgatacac tccataaagt tcagtcacac atcttctttt tcttctcccc tgcctaatga 86340

tcagacaagc caggtcttgt gactaaatga accgacagct ggcagtgttc atctgatttt 86400

gcaatgccct tattttgttg ttgataaact aatgcaaata gctggttggc ctggaactgg 86460

tgattgggaa acattttaac ccttctttcc ttcctctcta tcctaaagac ttaaaacaca 86520

cttcacgcag tcacagaaag agctcataga ccagactgcg gggcttcaaa gccttctatc 86580

ttatttaatc atctgtaaat agtttaaagg taaatatggt aataatacta gtattctaga 86640

ctattttggg aagccagctg atgtgaataa agggtcagag ttaaaagata ttgaaggaat 86700

gcagttttac tgctctgagg taggtttcaa ggcttgagcc aggcttagag atctttgtcc 86760

tgatactggg gataaagtga agagaatatg aatgggtatg tctcgcatgg ttagaaaatc 86820

tttttttttc ctgtatttgt ttaaaaagca ttttgtaact taaaaaaata cctatacacc 86880

tttttttttt ttgagatgga gtctcactct gtcactcagg ctggagtgca gtggcaccat 86940

cttggctcac tgcaaccccc gcctcctggg ttcaagcaat tctcccacct cagcctcccc 87000

agtggctggg attacaggca tgcaccacca cacccagcta atttttgtac tttttggtag 87060

agatggagtt tcatcatgtt ggccaggctt gtcttgaagt cctggcctca agtgatctgc 87120

ctgccttggg ctcccaaagt actgggatta caggggtaaa ccaccacacc tgaccataat 87180

acatcttaaa tagaacattt tccagctaat atttaaatga ctgcatgtca ttagaatctg 87240

aatgagtgtt gtgttaaaat attttgtgtt ttcatattta tttacttact tatagctacg 87300

gataagaata taggcaaatt taatctcccg taaaaattaa aaggcagatg ttttcaaagc 87360

acagaattac atggtctact ctagacattg ctcctaaggt tgagcagagg ggataagagc 87420

tttttctctt ttttcctttc ctcttttttt catacaaagt tcgttgtaga tattttagaa 87480

agtatagaaa gccccgacaa ggggaaaccc actcaaccca attttaatgt tgttgtcatt 87540

tcttttagtt tttttgggtg catttctatg tagctatatc ctattgtaca agcaatttag 87600

gatcttgcaa tataaaatga aggcatgacc tttgagctgg ccaaaagtat gggctgattt 87660

ttgcaaatgg gtcctggcag caggggacat aagtggggaa ccaatcccca cacaagcaga 87720

ggtgagctgg cttaggggcg agtgcatagc tgactttgga tgtagactag atttggggaa 87780

atttaaatgg ggtccaagac tggaaatgtg taatgggaca gaaggtagaa atgtttaaaa 87840

tcttaagttg ttgagtttgt tctcaatttt gtgaaaactc tttatccctt tttgttcccc 87900

accccatttt ctaaccagaa gtcaaatatg acaacattca tgatgtagaa aaaccagttc 87960

atcagtgtga cttgtgggtg agggtgggga cagggccggt gttggtatat ttagttttag 88020

agatttgctg gaaaagtaac atttttgctt ttgtctttct ttcgttcctc catttctcca 88080

gaagaatatg aaagtgttac tcagacttat ttgtttcata gctctactga tttcttctct 88140

ggaggctggt aagttaagta tttctttgtg ttcttgtctg ctaagaaaat ctgttattta 88200

gaatacatga agtaccttcc ctaacagagt catttctcat actcgtctct gttgaatctc 88260

tgggcacaga gcctcctggt catagacctg tactgctgga agttcctctc agcatcttcg 88320

tctcattctc tcccatatag agtgggaaac tgaggcctag tgaggctgag agcccaaggt 88380

catgtagcta gttttggaac aaaagctaaa atctaggtct ccaatctagg tccaggcctc 88440

tttccacttg gctgctggtt gaaagtgtga tatgggaaat tctgactttc atttggagga 88500

gatattatgt atagtcttta tgaacccaca gctggaagtc atcaagatac actacttttt 88560

ctgtttcttt ccatttcttc attttctcta atcttttctc tttttgccag gtgctagcta 88620

ctggggatac agcagggaac agtgcacaca gaaatacctg cctgcatagg gtttatattc 88680

ttgtagaaga gagagataac agataagtag acatatattg tgtcatatga tggcaagtat 88740

agggaggaaa aaagagtata aggttttggc attacagtgg gcagaaggtt aatttttgga 88800

atgtggccag aaaaaaacct ccctgagaag ctgacttttg agttgacctt aaggaagggg 88860

aaaagagagc tatttggata tcagattggg gtgtggaaat attccagtca gcatgtgcaa 88920

aggcccagag gccaagagta tttggcaggc tttcagaata aaaatatcag catgtctgga 88980

gcatagtgag agatgaggga agtcataagg gaccaggctg tctgggtaat ggggggatgg 89040

cagagggttg attatatgga atgttgtgcc cttttttagg gtcttggctt gtagtctggc 89100

tgggatggga agacttcagg gttctttgtt taagaccaga gaggtgatct gacttacgtt 89160

ttgattcctg ttttgagaat agatccgagg gagccaaaga gagaaataga gaagccagtg 89220

aagaggcaag ccatgatgat ggctttctct aggcaagaaa tgttgatggc ttgtcaaagg 89280

gtggcaagga gaagtgagaa gtagacaaat tttgaaatat tttgggaaca gaaccaagag 89340

ggatttggag ttgggtcaga tgtgagatgt gagggaaaga gaagaatcaa gaattatctc 89400

agggaatttt ggcctgcgca aacgcaaggt gaaattgcca ttgatgagat ggggaagatc 89460

aggttttcag actggatgtg ttaagtttag ctgtccatta gatgtttaaa aggtgacttt 89520

gactaggcag tctacagcct gagggaaagg tctgggttga atatataaaa ttggagacct 89580

cagcaagcgg ctggtatgag aaccatgaga ctgaatggga tcatggagtg tagttaggaa 89640

agataagaac tttaaggact gagccagaga ttgacttaaa ttccttggtc cagtgagttt 89700

tctccccttt gctgaggggc tccatgtgtg ttggagcata ccttcaacat taaggctgtt 89760

taaggcctta gccttcactt cctgcttgtg tatgacctca gagtcatcca gaaatgagag 89820

cctggtgcct tctatgcaat ttcctgggca tgcacacagc cctgcataca tgcccaccct 89880

tctgggttcc ccaggaatgt gtcagagctt ttcaaagccc tctggaggtt atggctctgg 89940

tattgcagct ttaggcagtt gtaaaaaaca attaacactg agtttttcca gcaaacgcct 90000

tggggatagt gcttttgctg tggagctctt ggtgaggtca ataacaccac caccctatga 90060

atgtgacttt gaggacctgc cagtcaagtc aaagagtggc agtgctctgg aaatgggact 90120

tttcgaggag ctccaaaccc tctctcttct tgcaagcctg ctggttttca aagcctttgt 90180

gtctgcaaag ctcttgatat tcatggctgt tttagagctg ggaagagtag gatggaagtc 90240

atttaagtta taaagctaca aaccctgctg atccgtgatt tggcatgttt ctgtttgttt 90300

ccttattttc tagtaaatgc tcttctggtt actgcaagcc tttgattttt gaaatttttt 90360

gccagtcttt ttgttgcctt tatggaggaa tgatttatga gacccccact ccaccatatt 90420

ggaaatcttg caaattcaat ttctttgaat agaaaatagt attaatagat atagggcaat 90480

ccaggctatc tacttcttct caagggagct ttaatagtct ggaaaatttg ctcatttcat 90540

ttacgttatt aaatttattg gcataacatt gtttataata ttccctcact accctttaca 90600

aattatttaa aaaagagatg tcatctcttt tttttcctga ttagtctggc taaagattta 90660

tcaattttat tgattttgtg aaagaatctg cttttggtat ctttgatttt tcttcattgt 90720

tcttctgttt tctatttcat tgatttcctc tctgatccat attactattt tcttcttact 90780

tctgttatat taattatgtt ttttattttc tgtatcttac catattttga cattctggaa 90840

gatgtttcat atctagggag agactgctcc accaggggct aagcagttcc taggtgtgtt 90900

gtggacagct tacagcttgc ccttcatgtg agactgcttg acccaagatg gtaccccact 90960

cacctcctta tccaactctc acacacccaa accaatgtta ccccagcctc aatcaacaca 91020

gggctaggta ccagagaact agggacagcc tctatgccca aagcctgcaa aaatggttca 91080

aatgggccaa tctgaaactg tttatgctgc cctgccttgc ctttcccatg ggaaccccag 91140

taaagatgct ggcctaggct ttccttttgt ttctgcttct gcctcctaac ccaatctggt 91200

gattctccct gtggttttgc gtggtatggc acaccccttc tgtccgaacc actaagtaat 91260

acaaatcttt caattgcatt agtctctgtt ttgtcactca gtcaccttcg taaatgaaga 91320

cccaggcata gaatctctgc ttgctgctta ctttggaatt actttgttct ttttctagtt 91380

tcttaaggta gaagctcaag tcatcaattt gaaattttta aaaaatatat aggcatttaa 91440

ggctagcagt ttccctctaa gtactgcttt cattgaatgc cataagtttt gatatgttat 91500

gttttcctta tcattcagat acaaatactt tatattcttt ttgatttctt tgactcatga 91560

gttatttagg agtatgttaa ttttcaaata tatagggatt ccttggatat ctttctatta 91620

ttgatttcta atctaattcc attgtcacca gagaataagt tttgtctaat ttgaatcctt 91680

tttaaattac tgagtcttgg gttatgggga agaatatgga aaatttgcta agttttatgt 91740

gtgcacttta aaaagaatgt aatgttctac tgttgttggt tggaattttc tgcaaatatc 91800

aattatgtca aattggttga tagtgttgtt caaatcttct atgttgttac tgattatccg 91860

tttaatttta ttgcttgttc tatcggtcct tgtgagtggg gtttctctag tttcttgcag 91920

aaccagtttc tctagttctt gcagttctgt cagtttttgc ttcatgtatt ttgaagcact 91980

gttagcagct acataaacat ttggtattat tgtgttcctg ttgattaatg acttctttat 92040

ttttttaaaa tttatttatt tatttattca ttcatttttg ggacaaaagt ctctctgtgt 92100

cacccaggct ggagtgcagt ggtgcgatct cggctcacag caacctctgc ctactgggtt 92160

caagtgattc ttgtgcctca gcctcccaag tagctaggat tatgggtgcc caccaccaca 92220

cctggctgat tttttttgta ttttagtaga ggtggagttt caccatgttg gccaggctgg 92280

tctcaaactc ctgacctcag gtgactcacc cgcctcagcc ttgcaaagtg ctgggattac 92340

aggcatgagc caccacgcct ggctgacttc tttattttta tgaaatgccc cttttttctt 92400

gtaatatttt tactgtaaaa tctactttga taataatagt cactcaagct ttaactaatt 92460

aatttttatt aaggtatagt tgacaaaatc gtgtatattt acagtgtaca atgtgatgtt 92520

ttggtatatg tatacattgt gaaatggtta accctagcta attaatcaaa ctttcttttc 92580

atgagtgtta gcctggcaaa tatttttctt ttctttttaa tctattttta cctttattta 92640

taaaatgtct gtatgtaggt gtatgtgttt atgttaggca ccacatagtt gggcctttct 92700

ttagccaatc tgatcatttc cgcctttcaa tgagagtgtt tagactattt gaaattttag 92760

tgtgattatt catatattag ggtttaattc tgggatcttg ctatttgttt ttctatttgt 92820

cccatcaatt ctgttatttt tttctctttt tttgctgcct tttagattaa ttggcatttt 92880

taggattcat tttattttgc tttttggctt tttagtcata actgtttgtt gtgtatgtgt 92940

ttttttaatg actgctctgg ggcttaaata gccatctcta agttatctct gtctgccttc 93000

aggtaacact atacagtttc acatatagta tacaaagtta agagtatata cccatttctc 93060

ctttcttgaa ctttgcccta ttatttccat atatgttact tctttatttg ttttaaactt 93120

tatagcattt tattattttt tatttaaaca ctcagttttc tttaaagaga tttaaataat 93180

aagaaagatg atcttataat tacccatttt tgttgctatt cattcttttg cgtagattca 93240

ttttcatctt ataatattgt gctttttgaa ggaattaaat atttcttgta atgcaggtct 93300

actggtgatg acattttaca gctttggcaa atctgaaaaa gtcagtattt tttcctaaat 93360

tttaaatata ttttcatggg atatggaatt ctaggtagac tttttctttc agtactttaa 93420

cgatattgtt ttgctctctt ctggcttgta ttgtttttgg aaagaaagct gcatcatttt 93480

ttgtctttgt cctctgcaat aattgtgtca tttctcccgg gctacataga agattttatc 93540

tttgtcactg gttttaaaca attttattat tatgtgtttg tagtttgctt tatgtttctt 93600

ttgcttgggg ttcattgtga tatgggggcc tatgtataca tcatttttgt cacatcttga 93660

aaaacttcaa atatgttttc tgttttccac atttttcttc ttcagtgatt catattacat 93720

gtattgaagt tgtcctaagt aaaataattc cctgcccatt ttattctgtt atttttgtct 93780

ctgtgcttaa ttttggataa tttctaatgc tgtgtcttca agttcactca tctttttttt 93840

tcctgcagta tcaaatctgc cgtgaatccc atccattgtt ttttgtaact caacattatt 93900

gttttcatct ctaaaagttt tttaaaatct ctttcatgtc ttcactaatc atgtttgatc 93960

tttcctgtag catcttgaat aaatggaata caatagtaat aaactatttt aatttttaaa 94020

tttattaggc ctattgtctg tgtcatttct gggtcagttt tgactgatcg atttttttcc 94080

tccttatcgt tggttgtatg ttactgcttc tttgcatgcc taattatttt tttggatgtt 94140

agacattgtg gattttacca tgttgggtgc tggatatttt cgtattcccc agaatgttct 94200

tgagctttgt tctgccacat gattgttact tggaaatggt ttaatccttt cgggtcttgc 94260

ttttaagctt aattaggatc aggaaaaagt ttattcttgg cctaattttc cctaatgact 94320

gagtcagaac ccttctgggt actctgcctg atgccttatc attaatgagg ctttccaccc 94380

tggctgttgg gaacagcaac tgtttccagc attgggtgtg ctccatgtgt tactccctcc 94440

agttcgtttg gtagtcctcc ctctagcctt gggtcatttt cttatatgct tacactgact 94500

gatactcagc tgaaaacttg ggggagactc tcagttatcc caagactctt tctgcgtgtc 94560

gttatgcagc tctaagtact tgggtactct gccctgcaaa cttttctacc caggttctca 94620

gctccatctc ctcaactcac tgggctttgc ctgtgttttt tctcggcaat gtcatggcct 94680

agctgtaatt ttggacacct cctttcttct ccgtctttct gggatttctc tcctttgttt 94740

tctgatgttc aatgtcttga atactgttgt ttcatatatt ttgtccattt tttagttgtt 94800

catgtgggaa ggtaaatcca gtttctatta ctccatcttg tactgaaagg catgtctctc 94860

ctcctacatt gtccccactt tttagatgag tgaaaagggg gtgaaggatg ttaaataact 94920

tgccaggggt acagcacaca ggcagtaagt gatgcagctg gacttgaata tgcttctcag 94980

actccccaga taatgatctt atcacaaggc tgtaaagctt ttatatgtaa tgtgaattga 95040

tgtatttaat aatcaatgtg tttcttcgta gatattaaat caagacacac tggcagattt 95100

ttatgtaaat tgcttccacc cttcttcctt ttaaagataa atgcaaggaa cgtgaagaaa 95160

aaataatttt agtgtcatct gcaaatgaaa ttgatgttcg tccctgtcct cttaacccaa 95220

atgaacacaa aggcactata acttggtata aagatgacag caagacacct gtatctacag 95280

aacaagcctc caggattcat caacacaaag agaaactttg gtttgttcct gctaaggtgg 95340

aggattcagg acattactat tgcgtggtaa ggtaagagag gaattagtat gttacaaaca 95400

tgttctagtt tcctgcagtt gttaaggaca gaaatacttt taataatgct taacttacct 95460

attttatttt attttagaaa ttcatcttac tgcctcagaa ttaaaataag tgcaaaattt 95520

gtggagaatg agcctaactt atgttataat gcacaagcca tatttaagca gaaactaccc 95580

gttgcaggag acggaggact tgtgtgccct tatatggagt tttttaaaaa tgaaaataat 95640

gagttaccta aattacagtg gtataaggta attttatttt aaatatgaca tttcactttt 95700

ccagaaaata aaatagttcc ctggacaata gaatgtatct gcaaagtacc tttttatttt 95760

gtatattgtt ttctctttaa tggtgaaact tgatagggaa cctaaaaaga attgtaagac 95820

tgcattcact taatttgaag cttaactaga aatttgtttg ctgttgtttt tatttaattt 95880

aataagatgt aattgagaaa agtactagaa atagatgttt aaagagaagg ctgattataa 95940

gcttaaggta ttgaagatgt aactatttat tacaaaacat actatgtgtg ttttattgaa 96000

gatatggtct tcaataaacc tgctagtttc cataactgtc tgaaaagtgg gttgactagc 96060

tttccatgga acaccccatg ttttggtaac tttaatctac ctagtctgat ggaacttaaa 96120

tggggaatca tcttgtagag cttaagacgt actaaatacg taatatcaat gaagtgcagt 96180

attgttaaga aactatatag tgttaacatt tttttcaaac atgtttttct tgcaaaataa 96240

attggccttc acttcatagt acaagtctca tagacttgaa aacttaatgt cataggaata 96300

tagttaactg tttccaaaaa gccagtatta aaacttacta tatgtaagga acttttccag 96360

tcactttcta aaaatataac atttgctaag gtgaaaaaaa tggagctcct ttggcggtag 96420

attggggaaa gttattggtt attggttttc aatgcttctc tctcccttta tctaggattg 96480

caaacctcta cttcttgaca atatacactt tagtggagtc aaagataggc tcatcgtgat 96540

gaatgtggct gaaaagcata gagggaacta tacttgtcat gcatcctaca catacttggg 96600

caagcaatat cctattaccc gggtaataga atttattact ctaggtgagt catagctcca 96660

gccctaaaag gtttagatct gggaaggtcc agagactgtg attctaatac tactttcatt 96720

atccatgaag aaaccaaatc caagacgacc tcaatgatac cacaaaagtc acatggcttt 96780

agttttgttt ttttgttact aaatactatg taatgttttt ccattcctgc ttcagatgta 96840

gttctaatta aatagagaca ttatgagctg tgttttccaa ctgatttaag agggttgttc 96900

tttggcatgg ctaaaaatgg ttcttactga aagctctggg gctccctctg gctggtatat 96960

gggaatgttc tgagagcctg cacctgggga atctcagtga gtgtcagtgt ctagcagcaa 97020

gcatcggtgt ggacactaat gagctttgga ataagtcaca tttccagaat ctacctcctg 97080

ttcattctgt gttaggtgtt aacctctcta accctgtttc ctcttctgta acaagatgat 97140

gcaatctccc aaggttattg ggaagattag agatatgggg tacacgaaac ttgtgtacct 97200

ggtgcaaaga aatgcttggt gcaaaacagc tatgcaatta ataggtgtag gatgattgtt 97260

cttgttaatt ttcttgtgtc tttcaattct gcgttaacca tgtttcatat tgaaaactat 97320

atcacagcaa aatgaaacaa aatgcacaaa ttgccagggg atagtcagaa tgctcaaact 97380

ttttgtaaat attgagattt tgtaagattt atgtaataaa ataagagtta ttccaatcag 97440

aacttttttg caggttatag gggtgactat tagtagttat gctgggacaa caggtataaa 97500

ttgggactat cccaagcaaa ctcagaatat gatcacccta taaatagctt cacttttcca 97560

gttatataat actttatttt cctaaaaata tttttattaa ataaaagtta attccttgcc 97620

atggtcccac aggcatcagt gaatcccaat tttcaatggt ctttgtatca aaccatgttc 97680

ttgagttttt agcagcctgt cattgggtag ctctggtctg tctttcctga aacatagtaa 97740

cctaaccagc aagtagaact tgagagagaa ctgggcactg gggactattt aagctaggtt 97800

aagtgttttt tttttttttt ttttttttga gatggagtct cgctctgtca cccaggctgg 97860

agtgcagtgg tgcgatctcc gctcactgca agctctgcct cccgggttca tgccattctt 97920

ctgcctcagc ctcccgagta gctggaacta caggcgcccg ccaccacgcc cggctaattt 97980

ttttgtattt ttagtagaga tggggtttca ccgtgttagc caggatggtc tcgatctcct 98040

gacctcgtga tccgcccacc tctgcctcct taagtgctgg gattacaggt gtgagccacc 98100

gcgcccggcc aggttaagtg tgtttttgat attctctgtt tccttttctg atcccatttg 98160

tagaaacaat atactctact cggagtgagg taatattttg ttttctttcc aaggcccaca 98220

ccaatgatgc ctagcacttt ttgttcactc aaggattcaa aaatatttat tgagcattta 98280

ctatgtttca ggaactgtaa taggtataca ttagggaaaa aaacagagaa tatcctacct 98340

tcatggtgtt cacttttttt ttgattgggg gaataccgag tgctcaattt tatagtttct 98400

catagtttct tcatggccac agataaaccc ctgaaaccaa tcttttcata aataagtcag 98460

attaatttct ggggcttctt gatacttttt tgcctatttt aataaattac agcattattc 98520

cataaccctg tagttctcac tattgggtca ttaacacagt cattaaatgc aagcaggtct 98580

agtattgact agcccttctg gccaataaca cccaattcaa ggaagtgact ttccactact 98640

ctcgcctttg ctgacatgct ttcacttaag agaggcattt gtctggggac ccagggtcct 98700

ggtgatgtgt gtgtttttca gtagcacccc actctatgaa ttcggaaggt ctagctctac 98760

cccgggattc acagtgtgct gtggcctctg cctcagcttt cttccttcac tttctgcctc 98820

ctgtctcctg gcatatgtgc tttgaacact tgatggaata tctggccaga agtcatttag 98880

tatgttttgc taagtgttgt cgtcacttga gattctgatc tataagagac tgacaaacct 98940

tatggatgtt tttctttcag aggaaaacaa acccacaagg cctgtgattg tgagcccagc 99000

taatgagaca atggaagtag acttgggtaa gtgggcttca gtgagggtat gctggaatcg 99060

gttttttttt tttaaaacat aagagtaaga taaattgtat ctttactata tataatctaa 99120

gccatttact gtataaatct atcaatggag ggaaagtgag acaaattata tcttaatttt 99180

ttgtatttct tgcctctatg gcaaacatct tgccatgtct gatatctcca gccctacttt 99240

tctgtgaaca cttattacca tgaaaacacc aaccagacac caaggtcaaa aggcctagga 99300

accagcaatg ccattgactg gtacaggatt ggatttcttt cttttttttt ttttcacaga 99360

gttaaaatat ttaatgacaa aaatagggtt tgctgtaaaa gtgaatcaat acagtaggtg 99420

ttacttatct ctgaattaaa caaaaattat atttgacatc ttaaagaact tctgaagaat 99480

tgatcattgt acgacagaca tcagcagtgt tacaatttca ggactggatt tctcatgacc 99540

agatccaagc tttgagtcgg aagagtaagg ctacaggccc caaagatggt agtcattcta 99600

gaaaagtatt tttccaagtt agtgaacatt ttttattcaa gagtaaagaa agaaccccac 99660

aagcatctag ctctgcactg tttagtgcag tagttactag gcgcatggag ttatggacat 99720

gtacaattaa attaagtcaa ttgattcatt tagttaaatt aaaaattagt tcctctgtca 99780

cattagccat atttcaaggg cttaatagcc acatgtgaca agtgactact gtacagacaa 99840

gcacaaatat agaacatttc caacatcact gaaagttcta ttggaaacca ctgatcaaga 99900

gagaaaaagt ggttctttgt gtcagaggac ccaaaatagg ttggccttgg aattgtcctc 99960

tgtaatagta actgcatgac agcagtcaag aaccatacag aggttagaag gtatatgctt 100020

gtgatctgtg aattctgtac ttggccaatt ctgtacttgt tatttatggt ggaaggtggt 100080

agaaagacat tcttagacag aaacaaaaat gtcaaaactc aaaattaaat atgggccaat 100140

aggctctaga acaatgtttc tcaaccatct ttttttgttt taaattgttc ccctttagag 100200

tctaaaaagt cttcttccac ctaattctct ttcctgtctt taaatttcag taccacagtt 100260

atcttttata tttgctgata tactgtggcc ctttggagta ccacaaacca tgtaatatct 100320

aaggtttctt tgcctcttcc ccaagaacca gtttttgccc tgctggccac agcccctgtt 100380

gaaaatgcct gccctagaag tgtgtaattg agagctctga tgcctaggct gcattgatct 100440

ctcaatctac aaaaaaaagc ttcaaattag gaaataatta ttttaatgta ttatattgac 100500

agtggatcag aagcatatag taccctgtca taggattatc ctgaagggtg ttaaacaggc 100560

aataagatcc aataccatta ctcctaaaat gacttagaaa aatagggatt gagagatatt 100620

tccttaactt aataaataac atcagtgcaa gccaatagtc actaatattc ttcatggtga 100680

aatactatag accttctcac taaagccaag agcaagacaa agatgcccat gtaaccacta 100740

ttactgagca cagtctagaa gtgcctacta atgcaattga aggaaaaaaa caaggtatgc 100800

ttaccacaag ggaaaaaaga catttttatt attgtttcct gatcatacca atcccaagaa 100860

aattcccggg aaaaactaga attaattctg tataaattag tcagataata aattaacatt 100920

tagaattaat gttctaaaat ataagacttg tttagcaata accagctaga aaacataata 100980

ggaaacaaat gccatttgca ccagtaaatg tcttagaagt gtccataaaa tatgttagaa 101040

aaactttgaa aatatgtgga atttataaga gaggaaaaaa ctaaaaaaac caaaaccctg 101100

aatattaatc tcagaaaaac tgaaatttaa agcatgcatc cagccaggcg tggtggctca 101160

agcctgtaat tccagcactt tggtaggcca aggtggacag atcacttgag gccaggtgtt 101220

tgagaccagc ctggccaaga tgttgaaaca ccatctctac taaaaataca aaaattagct 101280

gggtgtggtg gtgcacacct gtagttccag ctgctcaaga ggctgaggca tgagagtcac 101340

ttgaacccag gaggagaagg ttacagtgag ccgagattgt gccactgcac ttccagcctg 101400

agtgacagag cgagactgtg tctcattttt aaaaatggaa ttaaataatg gatagaggga 101460

taacagtttt taaaaaggaa ggccgatgag caccaagcta atgaaaagca tgttgattca 101520

ccgtgaattc aagaaatgta gatgaaaact taagtgacat tttcttctct caaaaggcaa 101580

tgtctaacca aaaacacttg ttatctggtg gttttgggga aaatcgtgct cacatacact 101640

attggagaga gggtagactg acacagcctc actaaagaga gggggttagc ttttaaaata 101700

tgtttcttta attcaatctt gcttctagat tttaatactt caggatgtgt acaaataagg 101760

ctattcatta aaatattaat cataatagtg aaaaattagg aaattgtgtt catcaatagg 101820

aaatagttaa tcatagtgta tatgtattat ggaatgctaa gcagccatga cagagtctat 101880

tttggaagac aaatcatgtg ggcccaaatt cccaatccac agtaattaca aaatagtagg 101940

caaaaagacc ccatttcaaa aactgcacat gttcacagag aagataagga atgagacgca 102000

taaggttact aatggtggtt ttctctgggt ggtgggttta gggtaatttt cctttttctt 102060

tgtgcatttc tatatctaaa attttaattc aatagccatt tattaatcta gatagatcag 102120

aaaaaatcaa ttaatgttaa gattaaaaat acattctttg caggatccca gatacaattg 102180

atctgtaatg tcaccggcca gttgagtgac attgcttact ggaagtggaa tgggtcagta 102240

attgatgaag atgacccagt gctaggggaa gactattaca ggtatgtatg ctaagagtta 102300

ttcacatttt ggtgttaaat cccacgtgat attttatata accttgtcgg ttcagtttaa 102360

aagcaagtgt tagttgctcc taacctttgc tgcctttttt tttttttttt tttttttttg 102420

ctaagctaag tagaatttat aagatcttgt acatttaagg taaaaaatac ttgtcattgt 102480

ggtgcaaatt acagaccact tagaagcctt tctcttacgt tatacatgtt gcaacatttc 102540

aacgaagcaa ttatctttca ttattcttca tattcctcaa gtagcttaga agagactcat 102600

taatgagctc aagaatcagg caatccactg ctctcgaaag cctctactgg tttcctactg 102660

ctcatctatg ggacaaggat ctcctggctt cccatgacct ctccttcaca acacctgcaa 102720

agcactttgt catctgcccc aatctcctct tgtttagtgt ctcctctgtg ccccatggag 102780

aacacaatgc atttgtctac atttttcttc ttggaagtgg taattttttt gctttctctg 102840

ctactgagcc tgttactgac agtggtcttg gggttataaa atctcagatt agtgttgcca 102900

gcagtctgaa tgttgtgact gtgattgggc agggtgatta tttcagtggc aattttgtca 102960

caaataagga aatacacagg gtatacaggt cttatttgta gttgatgcaa ttaacttaca 103020

caagtttatt tactctctct ctcgaatagt gtggaaaatc ctgcaaacaa aagaaggagt 103080

accctcatca cagtgcttaa tatatcggaa attgaaagta gattttataa acatccattt 103140

acctgttttg ccaagaatac acatggtata gatgcagcat atatccagtt aatatatcca 103200

ggtaaacaac aaactaattg aaatgcagtt ttgttttact gtagtaagat tccatgactt 103260

tgaagtttga aatgccattt cctctgctta gaacacgctt cacttcctca ttacttgggt 103320

aacttctatt tctcttttac tcttctgctg aactctctct tcttggaagc ttttctgtcc 103380

tgctgcctgc ccttaaccca gctctcggcc caaagagagg aggttagggc ctctgtatgt 103440

acttctgtag caccatatgt cacaggatct atggtacttc gttttctgta cttaactata 103500

aattctgtga tggtggcaac catttatctt agtaatcatt ctatttccac agctaatggc 103560

aagttaggtc tggtacattg tgggtaccca atgaactaca ttgatgatgc atagggtaag 103620

actaaatatc cactttcaaa gaggacatca gaagtcactg ggtttcatga attggtataa 103680

taaaacataa tattgaaagt atttgtgaaa cattttaagg taatttgtgg tttaattaaa 103740

cataggctat tataattgcg tgcatggaag aatatctgaa agactctata aattaacttc 103800

tatgtatgtt taaatactgg cagcatacaa atagaaatta catttaaaag ataacattta 103860

tggtagcatc aaaaatcatc aaatacttaa gaataaatct agcaaaagct gttcaaaacc 103920

tctgtaatac aaactaaaaa atattgagat aaattagaga agaactaaat ggaaggatat 103980

acaatgttca tagattgaaa gactcgatat tatgaagata tcagttcttc cccaaatgaa 104040

tctatggatt ccaaaaaatt tcaatcaaaa tctcagcagt atttgtacaa gtgagagtca 104100

gtgtttgtat gggtgtgttt ttgtggacac ctgtaaaagt catagggaac tgtaaaggac 104160

caagtatagt catggtggtc ttaaataaga aaaataaatc tggaggatta ttactacaga 104220

catcatgatc ttgtatgaag cgacagtact taagaccgta agtgtgaact atcatttggg 104280

ccagctggat atttgtctgg aaaatggtat aacttgattt ctgttttata tccaaatcat 104340

ttaaattgga aaagtaaata aagcttttag attccctttt ctggatgctt ttacatggtt 104400

cctgatattt agagggtact ttctttttct tccctctttc ttcttctcta gccccctccc 104460

catcacctac tccatctgct ccccagcttc ctctttaatt attggataac taagtagaga 104520

aggaggccag gagaatgtgg aaatgtgaaa cttccctaag cctccacctt attcgctttc 104580

caccagtctt taactttacc caatggactc tctgacctca gggaactttt gtaggctttt 104640

gagtgatttt aacttttccc tgttttactg tggaatgctc tatcctgtgg tcttcggtat 104700

tatttcccat gttattgtat attatgctat ttaggtagaa acttcataga atgcacctta 104760

gttttatcat tgctttgtac attagtatcc attacaaagt gataataatg aatgagttaa 104820

tattgtctga atgcaggcca ttcccagata tgggctctct gagacgaaga tatttaattt 104880

gctgtgctca aagttttaat tctttttggt tctaatattt tttctccttt ttttttcttt 104940

ttgctatagt cactaatttc cagaagcaca tgattggtat atgtgtcacg ttgacagtca 105000

taattgtgtg ttctgttttc atctataaaa tcttcaagat tgacattgtg ctttggtaca 105060

gggattcctg ctatgatttt ctcccaataa aaggtataat tttgtattcc atgcagtatt 105120

tcttgttgga gataagggat agttaggaga tgtagaagat gactaagagg gtttttacta 105180

agagggtttc taaaatgaaa agtggcatat acaagaatac tagttattct tacacagaat 105240

tatagtttta gaactcatta ggctgaatat cattgttttc taagttcctg cctcaacatt 105300

catattggta gaataaacag gtatttagag catctgttta taaattcagt tatgcacaag 105360

ccattggtag gccttgctct gtgatgaata ggacagacag agatcatacc ttaatgaaag 105420

aaatgaaatg actagatctt catgcatcag ggaggttctc tctctccttt gtttaaactt 105480

tgcgaaaaaa aaaaaaggaa taccacaata tctaaaaaaa aatgaatgga tagaattcag 105540

ggctgagacc tctggggaag aagatgagga cagggagact ttaaggatgg ctctatacta 105600

agagtatacg aggcagcctc caaatagaag aacaaatttg cagtaaaaca caagaaatag 105660

aaaccttgta agagatgaac actttttaaa gggattatat ttttggtatt gctgcttcag 105720

tcatgccatt gtataaaaaa agatgaatag ttcattatgt aatgaatgtt tgtgatactg 105780

actttatcaa atctcttatt tttgccttgt ggttctaaat acattatgtt tttcctttag 105840

cttcagatgg aaagacctat gacgcatata tactgtatcc aaagactgtt ggggaagggt 105900

ctacctctga ctgtgatatt tttgtgttta aagtcttgcc tgaggtcttg gaaaaacagt 105960

gtggatataa gctgttcatt tatggaaggg atgactacgt tggggaaggt atgtgtgtaa 106020

tggaacagag taaaggctta ttgttgtaaa actacttagt aaaatgtgga ttccatcttt 106080

ctagaagatc gtggcatagg ggtatatgtt tcacaatttt aagaacctct tcagaagtgt 106140

atgatgattt ttacatttat taaatgcatt tacttctctc atattttgtc agagcaataa 106200

ttaagtgatt ttcatttagt tttgaaaaac ttaatgacta gttaaaccca acagttgctt 106260

tacaactgat acaattaaac aggagtggta gagatcaaat gattgaataa taagttttta 106320

aaattccaac aaagttttct gagttctaaa tgaaatattt gtcttttgag ggccaatttt 106380

tgagttagtc tataaaatgg gaggtttgca gtggctcatg ccttttatct cagtacaacc 106440

ctgggaggcc agggtgagag ggtcacttga gcctaggagt ttgaggctgc agtgatccaa 106500

gatcacgcca ctgcgctcca gtgtgagcaa cagaacaaga gactgtctct taaaattaat 106560

taattaaaaa catgggaaac tcctttaatt ttaagtaaga caagaaaaag gcattaggag 106620

ccatacggtt gtgaaaagcc ttgtgtggct ttggttcagg agagaatgat gataaataga 106680

attttactta ctatattgtg cttcctgttt ttcagacatt gttgaggtca ttaatgaaaa 106740

cgtaaagaaa agcagaagac tgattatcat tttagtcaga gaaacatcag gcttcagctg 106800

gctgggtggt tcatctgaag agcaaatagc catgtataat gctcttgttc aggatggaat 106860

taaagttgtc ctgcttgagc tggagaaaat ccaagactat gagaaaatgc cagaatcgat 106920

taaattcatt aagcagaaac atggggctat ccgctggtca ggggacttta cacagggacc 106980

acagtctgca aagacaaggt tctggaagaa tgtcaggtac cacatgccag tccagcgacg 107040

gtcaccttca tctaaacacc agttactgtc accagccact aaggagaaac tgcaaagaga 107100

ggctcacgtg cctctcgggt agcatggaga agttgccaag agttctttag gtgcctcctg 107160

tcttatggcg ttgcaggcca ggttatgcct catgctgact tgcagagttc atggaatgta 107220

actatatcat cctttatccc tgaggtcacc tggaatcaga ttattaaggg aataagccat 107280

gacgtcaata gcagcccagg gcacttcaga gtagagggct tgggaagatc ttttaaaaag 107340

gcagtaggcc cggtgtggtg gctcacgcct ataatcccag cactttggga ggctgaagtg 107400

ggtggatcac cagaggtcag gagttcgaga ccagcccagc caacatggca aaaccccatc 107460

tctactaaaa atacaaaaat gagctaggca tggtggcaca cgcctgtaat cccagctaca 107520

cctgaggctg aggcaggaga attgcttgaa ccggggagac ggaggttgca gtgagccgag 107580

tttgggccac tgcactctag cctggcaaca gagcaagact ccgtctcaaa aaaagggcaa 107640

taaatgccct ctctgaatgt ttgaactgcc aagaaaaggc atggagacag cgaactagaa 107700

gaaagggcaa gaaggaaata gccaccgtct acagatggct tagttaagtc atccacagcc 107760

caagggcggg gctatgcctt gtctggggac cctgtagagt cactgaccct ggagcggctc 107820

tcctgagagg tgctgcaggc aaagtgagac tgacacctca ctgaggaagg gagacatatt 107880

cttggagaac tttccatctg cttgtatttt ccatacacat ccccagccag aagttagtgt 107940

ccgaagaccg aattttattt tacagagctt gaaaactcac ttcaatgaac aaagggattc 108000

tccaggattc caaagttttg aagtcatctt agctttccac aggagggaga gaacttaaaa 108060

aagcaacagt agcagggaat tgatccactt cttaatgctt tcctccctgg catgaccatc 108120

ctgtcctttg ttattatcct gcattttacg tctttggagg aacagctccc tagtggcttc 108180

ctccgtctgc aatgtccctt gcacagccca cacatgaacc atccttccca tgatgccgct 108240

cttctgtcat cccgctcctg ctgaaacacc tcccaggggc tccacctgtt caggagctga 108300

agcccatgct ttcccaccag catgtcactc ccagaccacc tccctgccct gtcctccagc 108360

ttcccctcgc tgtcctgctg tgtgaattcc caggttggcc tggtggccat gtcgcctgcc 108420

cccagcactc ctctgtctct gctcttgcct gcacccttcc tcctcctttg cctaggaggc 108480

cttctcgcat tttctctagc tgatcagaat tttaccaaaa ttcagaacat cctccaattc 108540

cacagtctct gggagacttt ccctaagagg cgacttcctc tccagccttc tctctctggt 108600

caggcccact gcagagatgg tggtgagcac atctgggagg ctggtctccc tccagctgga 108660

attgctgctc tctgagggag aggctgtggt ggctgtctct gtccctcact gccttccagg 108720

agcaatttgc acatgtaaca tagatttatg taatgcttta tgtttaaaaa cattccccaa 108780

ttatcttatt taatttttgc aattattcta attttatata tagagaaagt gacctatttt 108840

ttaaaaaaat cacactctaa gttctattga acctaggact tgagcctcca tttctggctt 108900

ctagtctggt gttctgagta cttgatttca ggtcaataac ggtcccccct cactccacac 108960

tggcacgttt gtgagaagaa atgacatttt gctaggaagt gaccgagtct aggaatgctt 109020

ttattcaaga caccaaattc caaacttcta aatgttggaa ttttcaaaaa ttgtgtttag 109080

attttatgaa aaactcttct actttcatct attctttccc tagaggcaaa catttcttaa 109140

aatgtttcat tttcattaaa aatgaaagcc aaatttatat gccaccgatt gcaggacaca 109200

agcacagttt taagagttgt atgaacatgg agaggacttt tggtttttat atttctcgta 109260

tttaatatgg gtgaacacca acttttattt ggaataataa ttttcctcct aaacaaaaac 109320

acattgagtt taagtctctg actcttgcct ttccacctgc tttctcctgg gcccgctttg 109380

cctgcttgaa ggaacagtgc tgttctggag ctgctgttcc aacagacagg gcctagcttt 109440

catttgacac acagactaca gccagaagcc catggagcag ggatgtcacg tcttgaaaag 109500

cctattagat gttttacaaa tttaattttg cagattattt tagtctgtca tccagaaaat 109560

gtgtcagcat gcatagtgct aagaaagcaa gccaatttgg aaacttaggt tagtgacaaa 109620

attggccaga gagtgggggt gatgatgacc aagaattaca agtagaatgg cagctggaat 109680

ttaaggaggg acaagaatca atggataagc gtgggtggag gaagatccaa acagaaaagt 109740

gcaaagttat tccccatctt ccaagggttg aattctggag gaagaagaca cattcctagt 109800

tccccgtgaa cttcctttga cttattgtcc ccactaaaac aaaacaaaaa acttttaatg 109860

ccttccacat taattagatt ttcttgcagt ttttttatgg cattttttta aagatgccct 109920

aagtgttgaa gaagagtttg caaatgcaac aaaatattta attaccggtt gttaaaactg 109980

gtttagcaca atttatattt tccctctctt gcctttctta tttgcaataa aaggtattga 110040

gccatttttt aaatgacatt tttgataaat tatgtttgta ctagttgatg aaggagtttt 110100

ttttaacctg tttatataat tttgcagcag aagccaaatt ttttgtatat taaagcacca 110160

aattcatgta cagcatgcat cacggatcaa tagactgtac ttattttcca ataaaatttt 110220

caaactttgt actgtta 110237

<210> 35
<211> 15775
<212> DNA
<213> Homo sapiens

<400> 35
caagcttagc ctggccggga aacgggaggc gtggaggccg ggagcagccc ccggggtcat 60

cgccctgcca ccgccgcccg attgctttag cttggaaatt ccggagctga agcggccagc 120

gagggaggat gaccctctcg gcccgggcac cctgtcagtc cggaaataac tgcagcattt 180

gttccggagg ggaaggcgcg aggtttccgg gaaagcagca ccgccccttg gcccccaggt 240

ggctagcgct ataaaggatc acgcgcccca gtcgacgctg agctcctctg ctactcagag 300

ttgcaacctc agcctcgcta tggctcccag cagcccccgg cccgcgctgc ccgcactcct 360

ggtcctgctc ggggctctgt tcccaggtga gtcggggtgg ggattgccgt cgggccagtt 420

ctccgaagcc ccgggaggac cggctcccgg gtcaggtcat gcatgcttag gtagctgttt 480

atgggaagga ggggctagag acagcgattg aaaggcaaca gccagtaggt tcgaatccag 540

accctgcata cctccacgtg tggccttggg ctatagattg cagctttaaa aaagggtagg 600

gggttggaga tggaggggag gggcgggcct cgttttgttg cccaggccgg tcttgaactc 660

cgggggtcta gccttacctc ctgcctcagc ctcccgagta gctgggatga gaggtgtgaa 720

ccaccgcctt gcttggctag attgcgtctc ttacagtttc tcagctgtaa aacgggaaac 780

gttatagcgg ccacctggca gggtatcttg gcccagcgca gcacctggcc ccaggactcg 840

atcatgatgg tttgggaact tggctctgtg ccaacccaac aaggcttaag ggacccccac 900

ccccctcaag atgtatattc tgttcctcat cctctctgcc cctggggaag tccagggctg 960

cttctacttg ggggaattcc agagctgact tatccgtggc ccaaagctga gaagtgggac 1020

gccccagcac accctccccc agctccagcc cagctaggga agagggaagg ggtcagaggg 1080

tctttcatgg tggtgtaagt ttggggaacc aggagggtgg gagattgaca gcttggttaa 1140

cagctcaaca aagcctgaga tccaggccag cacggtagtt catgccagta atcccaacac 1200
tttaggagcc ccaggcgggc gaatcactta aggtcaggag tttgagacca gcctggccaa 1260
catggcaaca tcccgtctct actaaaaata caaaaattag ctggcatggt ggtgggcgcc 1320
tgtgatccca gctgctcggg aggctgaggg aggaaaatcc cttaagccca cgaggctgag 1380
gttgcagtga accaagattg tgccactgca ctccagcctg ggagacatag cgagattctg 1440
tctcaaaaaa caaagcgttc tgatccggac tcagacccag atcgcactgc tttctagctg 1500
agtaaccatt tctctctatg aaatgggaat ggtcccagaa tctcccttgg agaatgtatg 1560
gagccagtgt cctcacaccc ccatccaaga tagaacaaat ctgagacagg aatctttgag 1620
tgaggcagtg ctgggctcag acattttttc ccaccttcgg aggcagcaga atctgaggga 1680
cctgatccaa ataagcccct tctttctttc ttttcttttc tttttttttt tttttttttt 1740
ttgagacgga gtctcactct gtcgcccagg ctggagtgca gtggcgtgat ctcggctcac 1800
tgcaacctct gcctcccagg ttcaaacgat tctcctgcct cagcctccct gagtagctgg 1860
gactacaggc atgtgccatc acacccggct aatcactgtg ttagccagga tggtctcgat 1920
ttcctgacct catgatctgc ccaccctgcc tcccaaagtg ctgggattac atgcgtgagc 1980
cacagtgccc accccgtaag ccccttcttt cttacctgca aggtagccag ttgctaccca 2040
tcctgtgctg agttacttgt attagcaagg gatggggtgg ctatactcac ccaccttaca 2100
gatggggaaa ttgaggccca aagaggggga aactacgtgt ctcagggagt gaggagccag 2160
tctgattcct ggagggctga ctgtctccac ctgacttctt aggagggagg agggcaccaa 2220
cttcacatta aaatctggtt ggacacagtg gctcacacct gtaatcctgg catttgggga 2280
ggcttaggcg ggaggatcac ttgaggccag gagtttgaga ccagccttag caacatagtg 2340
agaccccatc tctacaaaaa tgtttttcag ggccaggcgc ggtggctcac acctataatc 2400
ccagcacttt gggaggctga ggcggcggga ttacctgagg tcaggagttt gagaccagcc 2460
tgaccaacat ggagaaaccc cgtctctact aaaagtacaa aattacccgg gcgtggtggc 2520
gcatgcctgt aatcccagct actcgggagg ctgaggcagg agaatcgctt gaacctggga 2580
ggcggaggtt gcggtgaact gagatcgtgc cattgcactc cagcctgggc aacaagagct 2640
aaactccgtc tcaaaaaaaa aaaaatgttt ttcaaatatt agccgggtat ggtggtgtcc 2700
tgtagtccca gctacttggg aggctgagat gggaggatca cttgagccca ggagttcaag 2760
gttacagtga gctatgattg tgccactgta ttccagcctg ggtaacagag ggagacccgt 2820
ttaaaaaaaa aaaagtgatg gctaaagtcc ttccatggct ccctattgcc ctcagtataa 2880
agaacacatg tggctgggcg tggtggttca cgcctgtaat cccagcactt tgggaggctg 2940
aggcgggcgg atcacttgag gccaggagtt tgagagcagg ctggccgacg tggcgaaacc 3000
ccgtctctat taaaaataca aaaattagct gggcgtggtg gtgcttgcct gtaatcccag 3060

atactctgga ggctgaggca ggagaatcac ttgaacccgg gaggcaaagg ttgcagtgag 3120

ctgagattgc gccactgcac tccagtctga gtgacaaagc gagactccat ctcaaaaaaa 3180

aaaaaaataa aagaacacat ctttagcatg gccttcagtg ctcacgggat cttcctgaat 3240

taatctcccc ctcttcatcc ttgctcactc agctccagcc accctgcccc gggacatctg 3300

tacttgcctg gaacttattt cccttttctc cggacagcca gccctttctc gtcatttaga 3360

tctctgctga aacattaccc tgtcaccaaa gcactgtcta ttctatcacc ctgttttgtt 3420

tttgtcaaag ctcatattaa catcagttat taattatctt gtttgctcat aatttttttt 3480

tttttttttt tggagacaga gtctcgttct gttgctcagg ctggagtgca gtggcacaat 3540

cttggctcac tgtaacctcc acctcccagg ttcaagtgat tcttgtgcct cagcctccca 3600

aatagctagg actacaggca cgtcccacca tgcccagcta atttttgtat ttttagtgga 3660

gacggggctt tgtcatgttg gccaggctga tctcaaattc ctgacctcaa gtgatctgcc 3720

cgccttggcc tcccaaagtg ctgggattac aggcgtgagc caccacaccc ggcctgctca 3780

tgaattttct ctttaacttc cacatcgaag gcaaagtatt gtcttgttaa ggctgtgcct 3840

ccagcaccca gcacaggctg ggcgcacatt cccttgatga acctgatttg taatgcctgt 3900

cgcctcttcc ctcgtttctt ctaggacctg gcaatgccca gacatctgtg tccccctcaa 3960

aagtcatcct gccccgggga ggctccgtgc tggtgacatg cagcacctcc tgtgaccagc 4020

ccaagttgtt gggcatagag accccgttgc ctaaaaagga gttgctcctg cctgggaaca 4080

accggaaggt gtatgaactg agcaatgtgc aagaagatag ccaaccaatg tgctattcaa 4140

actgccctga tgggcagtca acagctaaaa ccttcctcac cgtgtactgt gagtaactga 4200

gcccggaggg ctggactagg cagacccggt gggagagacg tgcaggggca cctgcagagg 4260

cctgggggaa tctttgccac ttgctcgtag ggtcaaggag gggctccttg cagggcaggt 4320

ggggacatcc ttggaaagtc cctttgtgaa tttctttggg tacaattaaa gtatttacag 4380

gctgggtgcg gtggctcatg cctgtaatcc cagcactttg agaggctgag gctggcggat 4440

cacctgagat caggagttta agtttcgcca acatggcgaa accctgtctc tgctgaaaat 4500

acaaaaatca gccgggcatg gtgtcaagcg cctgtaatcc cagctacttg gaaggctgag 4560

gcaggagaac gcttgaacct gagaggcaga gattgcagtg agccgcgatc gtgccagtgc 4620

actccagtct ggataacaga gcaagattcc atctcaagaa aaaaaaaatg ccatctctct 4680

atgcctcact ctttgaacat atgacacggt cctgcttcag acactttaat aaaagatgca 4740

aattaagcca agtgtggtgg cttgtaccta taatcccaac tactccagag gctgaggcag 4800

aaggatggtt tgagcccagg agtttgagac cagcctgggc aacagagtga gaccctgttt 4860

ctttcttttt tttttttttt tttttgagac ggagtctcac tctgtcgccc aggctggagt 4920

gcagtggtgt gatctcggct cactgcaagc tccgcctccc gggttcacgc cattctcctg 4980

cctcagcctc ccgagtagct gggactacag gcgcctgcca ccatgcccgg ctcatttttt 5040

tgtattttta gtagagacgg ggtttcactg tgttagccag gatggtctca atctcctgac 5100

ctagtggtcc gcccgcctcg gcctcccaaa gtgctgggat tacaggtgta agccactgtg 5160

cccatccaag accctgtttc taccggaaaa aaaaagtaaa taatttagct gggcatcgtg 5220

gtgtgcacct gtaatcccag ctgctcctga ggctgtgatg ggaggattgc tttaacccag 5280

gggttcgaat cctaggagtt cgaatccatc ctaggcaaca tagcaaaacc ccatttttat 5340

ttaaaaaaaa aaaaaaagat atgagttaaa actagccctg ggatggcatt tttcacatat 5400

tggtaacaaa caaaagaatt gatggccggg cgcagtggct cacgcctgta atcccagcac 5460

tttgggaggc cgaggcgggc ggatcacaag gtcaggagat cgagaccatc ctggctaaca 5520

tggtgaaacc ccgtctctac taaaactaca aaaaattagc cgggcatggt ggcaggcgcc 5580

tgtggtccca gctactcagg aggctgaggc aggagaatgg catgaacccg ggaggcagag 5640

cttgcagtga gccaagatcg tgccactgca ctccagcctg ggcgacagag caagactcca 5700

tctcaaaaaa aaaaaaaaaa aaaagaattg ataacagctg tgctgccaag gctattggaa 5760

cgtaggaggt cctaggacag tgctgttggg agcataaata agcccaaccc tgtggcggga 5820

aattgggcat cagttctcaa aatgtcatgg gctgggcacg gtggctcacg cctgtaatcc 5880

cagcactttg ggaggctgag ggaggcggat cacttgaggt caggagttcg agaccagcct 5940

gaccaccatg gagaaacccc gtctctatta aaaatacaaa aaaaactagc caggcatagt 6000

ggcacatacc tgtaatccca gctactcggg aggctgaggc aggagaatct cttgaaactg 6060

ggaggcagag gttgcggtga gctgagattg cgccactgca ctccagcttg ggcaacaaga 6120

gcaaaactcc atctcaaaaa aaaaaaaaaa agaaataaaa gaaggtatgt tgaatatgag 6180

tggtatgcca ccctcacatt agggaagggc agtttcgggg aggctgtatt tatgtataaa 6240

atagccctaa aaggaagtgg gagaaatgac aatattagct ggctatgaga agagaggctg 6300

ggaggctgtg ggagagggct tgggtgtgga gaattctttt tgttttttcc tttttttgag 6360

acagagtttc actcttgttg cccaggctgg agtgcaatgg cacgatctca gctcaccgca 6420

accttcacct cctgagttta agtgattctc cggcctcagc ctcccgacta gctgggatta 6480

caggcatggg ccactacgcc tggcaaattt tgtattttta gtagagacag ggtttctcca 6540

tgttggtcag gctggtcttg aactctgacc tcaggtgatc cgcccgcctc ggcctcccaa 6600

agtgctggga ttacaacgtg agccactttg cctggctgag aattcttttt ttgttgttgt 6660

ctttttgaga tggagttttg ctgtgtcccc agcctggagt gcaatggtgt aatctcagct 6720

cactgcaacc tctccctccc gggttcaagc aattctcctg cctcagcctc ccaagtagct 6780

ggaattacag gcgcccagca ccacgcccgg ctaatttttg tatttttagt agagacggga 6840

tttcatcatg ttggccaggc tggtcttgat ctcctgacct tgtgatctgc ccgcctcggc 6900

ctcccaaagt gctgggatta caggcgtgag ccactgcccc cagccgagaa tttctctttg 6960

cgtccttcct actttgggga cttcgaatgg tgggaaagag ttatcaaggc caaaataagg 7020

aattcaaatg aaaacaaaac aaaatcaaag aagaaaaaac agaagagcac tgggcaggct 7080

aggcacgtgg ctcatgccta taatcccagt gatttagaag gccgaagtag aaggatcgct 7140

tggaggccag gagttggaca ccagcctggg caacatagca agaccccata tctacaaaaa 7200

ataaaaaacc taaccaagcg tgctggcata ctagtagtcc cagctactca ggaggctgag 7260

gtgggaggat cacctgagcc tgggaggtcc aggctgtagt gagccgtgat gacaccactg 7320

cactccagcc tgggtgacag agaaagaccc tgtctctaaa aaataaaaac tggccaagta 7380

gctttgggat tagccttggg ttccagtccc agcaaggcct ttaatagctt gggacatgac 7440

ttctgcattt actttgcaat caggtgagac ctcctctgat ggggaaaatg acacggtgag 7500

tgacaaagga tgttctccta tcattgtgtc agggcaagga agcctctggg taaatgatca 7560

aatgatcagc tttgcttctg atttggaggg tgggtgagca gatgctgacc ttcccaggtg 7620

agggaagtcc ccgaacattc ccagcagctt ctggaaaccc cagggaaacc tctttgaagg 7680

tcttttctgc atctctgcct gataggtctt tttttttttt tttttttttt tctttttttt 7740

gacacacagt cttgctctgt cgcccaggct ggatcactgc aacctccatc tcccgggttc 7800

aagcagttct cctgcctcat cctctccagt agctgggatt acaggcacct ggcaccacgc 7860

ctggctaatt tttgtatttt tagtagagac agggtttcgc caagttggcc aggctggtct 7920

cgaactctag acctctggtg atccacccgc ctcagcctcc cgaagtgctg ggattacagg 7980

cttgagccac cacgcgcggc tctgcctgat agctgagagc atagaactcc aggtttgaga 8040

cctggctctg ccacatttct ccctctatga ctgtgggtgc cccactttgc cttagttttt 8100

acctctgtga aatggagcag atggctggca caggtagcaa aggagtaaaa gttatgtggg 8160

agggtggtac ctgagagaga ctctagcttg gtcttgcccc acccctggtg taaacataaa 8220

gaagcctccc tggatggctc aatcttctcc aaaaaggtta gaggtgtaat tcctagagga 8280

ggcgaccact agctgggctt tgaaggatgt gtaggagttc ataaggacag gcattctggg 8340

caggaggaac agcctgggca aaagttggga gcagggagaa atcttgatgg aggcaggagg 8400

aggaggaggt aggttggtgt aggccaggtg cagtggctca cacctgtaat cccagtgctt 8460

tgggaggcca aagcaagagg attgtttgag cccaggagtt cgagaccagc ctgggcaaca 8520

tagcgagacc ctgtctctaa gaaaaaataa aaaaattagg gtacagtggc atatgcttgt 8580

attctcaact actctggagg ctcaagtggg aggatcactt gaacccagga atttttttgt 8640

ttttgttttt gtttttttga gatggagtct cactctgtgg cccaggctgg agtgcagtgg 8700

tgccatcttg gctcactgca gcctcctcca cctcctgggt tcaaccgatt ctgctgcctg 8760

agcctcccga gttgctggga ttaaggtgcc caccatcatg cccagctaat ttttttgtat 8820

ttttagtaga gatagggttt accatgttag ccaggctggt cttgaacgcc tgaccgcaag 8880

agatcctcct gcctcagttt cccaaagtgc tgggattata ggtgtgagcc actgagcctg 8940

gtcaagccca ggaatttgag gttacagtca gctatgattg caccactgca ttccagccca 9000

ggtgacagag agagacactg cccctaaaaa aaaaaaaaaa attgattgat gggaggaagg 9060

gtgaggttgg cagagccttg aatgccaggt ggaggagctg ggactttcct tcttggggtg 9120

atagggagtc atggagggtt tctgagcagg ccagggatta gatagctgaa ggctggattt 9180

actggaagcc aatgagcagt tggctatggt ccttgtccac gcggcccatg ttgtgggcag 9240

tgaccgtatt caagaaggga aggacagaca agtatttgaa tacttcagtg accaggattt 9300

ggtaaaggac tgcaggtcag ggtcaagaag aggtgagagc aggacagact tcctccccgc 9360

tgcaccaggc agctgagctg ggtttcctct aggggctgag gtttgagggt acctcaagtt 9420

ctgcaagagt ctataggagg tggtaagaga gaagagctgg aggtcagagt tttcttgact 9480

atatatatat atattttttt gtttttgttt ttaacagctt aacagctttc tgttttattt 9540

ttagagacag ggtctcaggg tctcactttg tcacccaggc tggagtgcag tggtacaatc 9600

gtagctgact gcagcctcaa actcccaggc tcaagaaatc ctcctcccac ccttagcctc 9660

ctgagtagca gggactacag gtgtgagcca gcaggaagcc cagctggttt tttttttttc 9720

tttggtgttt tttgtttgtt tgtttgagac cggagtttcg ctcttattgc ccaggctgaa 9780

gtgcaatggc aggatcttag ctcaccacaa cctccgaccc ccaggatcaa gctattctct 9840

tgcctcagcc acctgagtag ctgggattac aggcatgcga caccacacaa ggctaatttt 9900

gtatttttag taaagacagg gtttctccat gttggtcagg ctggtctcga actcccaacc 9960

tcagatgatc cacctgcctc ggcctcccaa agtgctgaga ttacaggcat gagccaccgt 10020

gcccggcctt tttttttttt tttttttttt tgagacagag tctcactctg tcgcccaggc 10080

aggagtgcag tggttcgatc tgggctcact gcaagctccg cctcccgggt tcactccatc 10140

ctcctgcctt agcctcctga gtagctggga ctacaggcgc ccaccaccac gcctggctaa 10200

ttttttgtat ttttagtaga gacggggttt caccgtgtta gccaggatgg tcttgatctc 10260

ctgacctcgt gatccgcccg ccttggcctc ccaaagtgct gggattacag gcgtgagcca 10320

ccatgtctgg cctggccagg ctggtcttga actcctgact tccggtgatc catctgttct 10380

ggcctcccaa agtgctggga ttacaggcat aagccaccac gccatgccga agcccagctt 10440

gtttttaatt tttttttttt tttttggaga aatgaggtct tgcaatgttg cccaagctag 10500

ccttgaactc ctggcctcaa atgaccccgc cttggcatcc caaagtactg ggattacaga 10560

tgtgagccac catgccccag ccttgctttc ttgagatacg atttagaata ccataagatt 10620

catccctttt aagcacataa ttcaatgact tctgtacaaa caaccatgac tacaatctaa 10680

ttttaaaata tttcaatcac tctaaaaaag aaacctcctg cttatgtaca gcgactctgt 10740

ctacctctta agtgaattct cctaccttta atagccctat tttacagttc aggaaactga 10800

ggttcagaga gacaaagtca cttacccaca gcaaagaagc aaggctgggt atcaaatgca 10860

ggaccccccc ggtcctgatg cttttttttt tttttttttt ttcctctgag agagactctc 10920

actctgtcac tcagtctaga gtacagtggc gcgatctcag ttcactgcaa tctctgcctc 10980

ctgggctgaa gtaatccttt cctcacaagt aaacctcagc ctctcaagta gctgggacta 11040

caggcacaca acaccacgcc tggctaattt ttgtattttt aggtagagac ggggtttcac 11100

tatattggcc aggctggtct tgaactcctg acctcaggtg atccgcctgc ctcggccccc 11160

caaagtgttg ggattacagg cgtgagccac cacacgcagc ctttttgtta ttagactctg 11220

tcattactga cttttttttt tttttaatag aaacagggtc tttctttccc aggctaaagt 11280

acagtggcat gatcacagtt cactatagcc ttaaactcct gggctcaagt gatcctcctg 11340

cctcagcctc ccaagtagca gggactacag gtgtgcacca ccacacccag ttaaccattc 11400

attcattcat tcattcattt attttgagat ggagtctcgc tctgtcacct aggctggagt 11460

gcagtggcac gatctcagct cactgcaacc tccacctccc aggttcaaga gattctcctg 11520

cctcagcctc ccgagtagct gagactacag gcgtgcacca ccatgccaga ctaatttttg 11580

tatttttaat agagacgggg tttcactctg ttggtcaggc ttatctcgaa ctcctgactt 11640

cgtggatcca ccctccttgg cctcccaaag tgctgggatt aaaggcgtga gccaccgcgc 11700

cctgccaacc tttttttaat ttttcttaga gatgggggtc tccctatgtt gcccaggctt 11760

gtcttgaact cctggcctca agtgaccctc ttgccttggc ctcacaaagt gctaggatta 11820

cagcctgagc catcacacct ggccaacagg tttttttttt tgttttgttt tgttttttaa 11880

agaatgtcta ggccaggctc atttactttc acctgtaatc ccagcacttt gggaggccgg 11940

ggtgggcaga tcacttgagg tcaggaattc gagaccagcc tgggcaacat gctgaaaccc 12000

cgtttctact aaaaatacaa aaattagctg ggtgtggtga cacgtgcctg taatcccagc 12060

tactcaggag gttgaggcag gagaattgct tgaacccagg aggcagaggt tgcagtgagc 12120

caagatcatg ccatcaccct ccagcctggg cgacagaagg agactcagtc taaaaactta 12180

attaattaat taattaaaaa taaaaataca aaaattaacc tggtgtggtg gtgtgtgcct 12240

gtaatctaag ctactcagga ggctgaggca ggagaatccc ctgaatccca gaggcagagg 12300

ttgcagtgag ccaagatcga gccactgttt gcccagtcta gtgcactggg ctgctgaatt 12360

tatttgacca gacacctagc aatagacttt gaagttcttt tccacttttc actctaagat 12420

gctgctgtca tgaataagga atattttgat ccccttcaca aacactcggg gccctcttac 12480

cagttttcac tgaagatctt gacattccta tctgcttagg tgtctgggcg tgtttggggg 12540

agatactgaa gaggtagggc tcccaggcag gtgcagttcg tctgttaggc aggcagcaag 12600

gtccacttca ccagacaccc ccacctctgt tttcctgcag ggactccaga acgggtggaa 12660

ctggcacccc tcccctcttg gcagccagtg ggcaagaacc ttaccctacg ctgccaggtg 12720

gagggtgggg caccccgggc caacctcacc gtggtgctgc tccgtgggga gaaggagctg 12780

aaacgggagc cagctgtggg ggagcccgct gaggtcacga ccacggtgct ggtgaggaga 12840

gatcaccatg gagccaattt ctcgtgccgc actgaactgg acctgcggcc ccaagggctg 12900

gagctgtttg agaacacctc ggccccctac cagctccaga cctttggtga ggattgaaga 12960

agccagcagg gagaaggtgg gggtggggta tcctgcaatg cggtgcctgt ggccacagga 13020

tcttttgaga tgggtgtggc cccggctaag gggtgcatgt gttctaggcg tatgtgacct 13080

aggctgctga gtggccctgg aagaggatct cgcaggaggg ggaatgaaat gccccagaga 13140

agggcttcgg gacgtccatc cctgtctgct cacacctttc ttctctccct agtcctgcca 13200

gcgactcccc cacaacttgt cagcccccgg gtcctagagg tggacacgca ggggaccgtg 13260

gtctgttccc tggacgggct gttcccagtc tcggaggccc aggtccacct ggcactgggg 13320

gaccagaggt tgaaccccac agtcacctat ggcaacgact ccttctcggc caaggcctca 13380

gtcagtgtga ccgcagagga cgagggcacc cagcggctga cgtgtgcagt aatactgggg 13440

aaccagagcc aggagacact gcagacagtg accatctaca gtaagaaggg gcaggggcgg 13500

agtggggctt cttgggggtg tgacctgaac ccggggcggg gctcactgtg tgcctattcc 13560

aggctttccg gcgcccaacg tgattctgac gaagccagag gtctcagaag ggaccgaggt 13620

gacagtgaag tgtgaggccc accctagagc caaggtgacg ctgaatgggg ttccagccca 13680

gccactgggc ccgagggccc agctcctgct gaaggccacc ccagaggaca acgggcgcag 13740

cttctcctgc tctgcaaccc tggaggtggc cggccagctt atacacaaga accagacccg 13800

ggagcttcgt gtcctgtgtg agtggggctg ctggtcaatg gcccctatcc cccaaggccc 13860

aatctccctg aaggtcccat aaggtcttgc ctccaagtcc tgcccccacc cacctccatg 13920

tcatctcatc gtgtttttcc agatggcccc cgactggacg agagggattg tccgggaaac 13980

tggacgtggc cagaaaattc ccagcagact ccaatgtgcc aggcttgggg gaacccattg 14040

cccgagctca agtgtctaaa ggatggcact ttcccactgc ccatcgggga atcagtgact 14100

gtcactcgag atcttgaggg cacctacctc tgtcgggcca ggagcactca aggggaggtc 14160

acccgcaagg tgaccgtgaa tgtgctctgt gagtgagccg gcgggcagag ctgggtgggg 14220

gcaggggcca tggacctaat gcaatcctca ccgcctgttg tatcctcccc acagcccccc 14280

ggtatgagat tgtcatcatc actgtggtag cagccgcagt cataatgggc actgcaggcc 14340

tcagcacgta cctctataac cgccagcgga agatcaagaa atacagacta caacaggccc 14400

aaaaagggac ccccatgaaa ccgaacacac aagccacgcc tccctgaacc tatcccggga 14460

cagggcctct tcctcggcct tcccatattg gtggcagtgg tgccacactg aacagagtgg 14520

aagacatatg ccatgcagct acacctaccg gccctgggac gccggaggac agggcattgt 14580

cctcagtcag atacaacagc atttggggcc atggtacctg cacacctaaa acactaggcc 14640

acgcatctga tctgtagtca catgactaag ccaagaggaa ggagcaagac tcaagacatg 14700

attgatggat gttaaagtct agcctgatga gaggggaagt ggtgggggag acatagcccc 14760

accatgagga catacaactg ggaaatactg aaacttgctg cctattgggt atgctgaggc 14820

cccacagact tacagaagaa gtggccctcc atagacatgt gtagcatcaa aacacaaagg 14880

cccacacttc ctgacggatg ccagcttggg cactgctgtc tactgacccc aacccttgat 14940

gatatgtatt tattcatttg ttattttacc agctatttat tgagtgtctt ttatgtaggc 15000

taaatgaaca taggtctctg gcctcacgga gctcccagtc ctaatcacat tcaaggtcac 15060

caggtacagt tgtacaggtt gtacactgca ggagagtgcc tggcaaaaag atcaaatggg 15120

gctgggactt ctcattggcc aacctgcctt tccccagaag gagtgatttt tctatcggca 15180

caaaagcact atatggactg gtaatggtta caggttcaga gattacccag tgaggcctta 15240

ttcctccctt ccccccaaaa ctgacacctt tgttagccac ctccccaccc acatacattt 15300

ctgccagtgt tcacaatgac actcagcggt catgtctgga catgagtgcc cagggaatat 15360

gcccaagcta tgccttgtcc tcttgtcctg tttgcatttc actgggagct tgcactatgc 15420

agctccagtt tcctgcagtg atcagggtcc tgcaagcagt ggggaagggg gccaaggtat 15480

tggaggactc cctcccagct ttggaagcct catccgcgtg tgtgtgtgtg tgtatgtgta 15540

gacaagctct cgctctgtca cccaggctgg agtgcagtgg tgcaatcatg gttcactgca 15600

gtcttgacct tttgggctca agtgatcctc ccacctcagc ctcctgagta gctgggacca 15660

taggctcaca acaccacacc tggcaaattt gatttttttt ttttttccag agacggggtc 15720

tcgcaacatt gcccagactt cctttgtgtt agttaataaa gctttctcaa ctgcc 15775

<210> 36
<211> 27864
<212> DNA
<213> Homo sapiens

<400> 36
   atgctgacaa tcttcttcct tcccctggcc acctctctgc ccacttgctt cctcagtacc 60
   ttggtccagc tcttcctgca acggcccagg agctcagagc tccacatctg accttctagt 120
   catgaccagg accagggcag cactcctcct gttcacaggt gagcctggac cccaatgaag 180
   tagggctggg gacccaggcc caagggagcc agggccctga actgggggct caggctgggg 240
   ggttaggatc tgggtaggaa gagagactca gtcaagcctg agggggaggc aggcacatag 300
   ggtctgagat ttggagtttg tggagggaga ggatattgat gaaccaattt tgggagagtt 360
   ccagagatgc tggaagagag gccagttgtc tctgtactgc agagattttt aaaataggca 420
   gaatgcgcca acttgtgctc tgtggacagg atgctttggt ccgcaagttt tcctggactc 480
   actctcatag cgcccgaggt gcacgttggg gaaagatcct ttttagagcc tgggtactgc 540
   tctgcagaaa tggagaactg caactcgata gtggatggtg ggcaaggggc atccctggac 600
   cctgggaagg agagaagggg atgagttggg tgtccagaag acccaggcac cccgggcatc 660
   aggctcggag gggagattgg gacgctgggg ccgggggtgg agggcagcca ggcagaagga 720
   agacccttct ccaaagctct cttcccacct ctttcccagc cttagcaact tctctaggtt 780
   tcaacttgga cacagaggag ctgacagcct tccgtgtgga cagcgctggg tttggagaca 840
   gcgtggtcca gtatgccaac tcctggtgag gcccaggtgg tgctggcctt tggctccatc 900
   catcctctcc ctgctcaggg ccccatgccc ccggccctgc cctgttattt gcaaactctc 960
   ctctctgtct ggtgtagcga ctgccctggc taatgaagat ttgccttgaa ggcaggcacg 1020
   gtctcacagc taacatttac agagcagtaa gtgcagtgcc aggctcatca caggtggatg 1080
   ctgatttagt ccacacgaca gcctgtgagt aggaatcagt cgtgcaacaa acacttattt 1140
   gtttttcttt tctttttttc tatacattta aaaatatata gagacagggt ctcactatgt 1200
   tgcctgggtt ggtctcaaac tcctgggctc aagcaatcct cccgcctcag cctcccaaag 1260
   tgctgggatt ccaggtgtga gccaccacac ccagactcaa caaatatttc ttgtctccat 1320
   acgccagaga atccaacaga cagaaatccc ttccacatgg actttaaatt attaaaatcc 1380
   atcttgcaga tgaggaagct gaggctcagg gagggaacgc aaacttgccg gagtggcagc 1440
   tgtgctgcag cgtccacact cttacctaaa gtgttctttg tctcctcgca gggtggtggt 1500
   tggagccccc caaaagataa cagctgccaa ccaaacgggt ggcctctacc agtgtggcta 1560
   cagcactggt gcctgtgagc ccatcggcct gcagggtgag tcaccgcccc tcccgggacc 1620
   cagggccggg ctcccaggct tccctgctcc aggggcccgt ggactcaccg gagtgtcact 1680
   ttcagcttcc actgtgtctg agaccctcac cctcagatat gcttcctggc cccttaaggc 1740
   ctccccgccc atcgcactcc cgcagctctg tcaagacccg acagcttcct tcaccgtcag 1800
   acctccttgt ctcccaggtg gaggtgaccc ctgcccagct cttccacagc cttctctgta 1860
   cccccgagag tgaccatgca catatctgtc cccacagtgc ccccggaggc cgtgaacatg 1920
   tccctgggcc tgtccctggc gtctaccacc agcccttccc agctgctggt gagtggccct 1980
   gggtcacagg aggcttctga gggagggagg gaggagccgg ggccgcgggg ggctgggagt 2040
   ctcctgtagg gtggaggttc cggaatgtga gggtgggagg aagcaggggc agccccccag 2100
   cagcccgctg tgtccccagg cctgcggccc caccgtgcac cacgagtgcg ggaggaacat 2160
   gtacctcacc ggactctgct tcctcctggg ccccacccag ctcacccaga ggctcccggt 2220
   gtccaggcag ggtgagtgtc gggaccacca aggctttgag gagctcacgc acatccaatt 2280
   gggggtgcgg tgggctagag acagtcttgc cagagtggat cagaaagaag ggatctggaa 2340
   aaagagttac cacgtgttgc agtggttcct gacgctgctg cccgcacatc ctgccgatcg 2400
   cccgcacgct gccggacctt tcctgtgacc ttaacctctc caagcctcag tttcttcatc 2460
   tgttggatgg ggataataac acacccagca ctgaaagcaa cacaggatga ttcatggcca 2520
   ggggttagca cagcagctag caccaggcga cagccccatg aaggccagct gttgttattt 2580
   ttagaggaga ggatctattt tcatccaatg ggtcctggga tatgaccaat tggtttgtgc 2640
   cgtagtttag gaaaggtcag tgaaagtgca gtgtgagcaa cgtgtgtgtg tgtacatgtg 2700
   tgtatatgta tgcatgtgta tacatgtgca catgcacaca tgtacatgca tgtgtgtgca 2760
   tgtatgtgtg tgtgcgtgtg cacatgcagg ttgagacgca gggcctgact tccctggtgc 2820
   tgatggggaa gaaagataag gaagcaaaac agtgggcttt ggcaggctga ggcctccccc 2880
   tggtttcaca tttgctctca agcctttggt gagactgaca tctggctgca aatagggtgt 2940
   gccttccagc acctccgtgg gtccagactc catccacagg ggcctgggca cccatcagaa 3000
   tgcccaggac acctgtctct ccggtggagg cctctggggc agaggggatg cttgagaggt 3060
   cacatggaag ggggtttcct ctccaggtgc tggaagatcg tctcttctca ggacagatgc 3120
   taacttcaca caggagggga gtgggggcct gtatttaagc tgagaacaga acaggcccag 3180
   tgtgtggctg ttgcctggga tgatttgttt aaaatattaa caatcaaggc aagagggttg 3240
   tttgaggcct ggagtttgag accagcctgg gcaacatagt gagacctcat ctctacaaaa 3300
   aataaatata aaatacaaag tagcttggca tgctgttaca tacctctagt cctaactact 3360
   ggggaggctg aggcaggaga atcgcttgag cccaggagtt ggaggctgta gggagctttg 3420
   atcacaccac tgcactccag cctgggtgac agagtgagac tccatctcaa ttaaaaaaag 3480
   aaatttaaca aaactaccat tcagcactga gcagtgagga caaacactgg ctagggactg 3540
   gtgccagttt gtgtcattgc tggatggggt gacattgagg gaggaaggag gggtgggtcc 3600
   agggaggggc acagatatcc agggtggtgg aggggtaggc cctctgcttc ctaccgtctg 3660
   aattggagaa atcttcctgg tttttttttt tttctttttt tttagagata gtcttactct 3720
   gtcactgagg ctggagtgca gtggtacaat cacagctcac tgcagcctcc aactcctggg 3780
   ctcaagtgat cctcccacct cagcctccag agtagctggg accacagaga agagctacca 3840
   tgcccagctg atttttaaca atttttagta gatgtgaggt ctaactatgc tgtccaggct 3900
   gatcttgaaa tcctgggctc aagcaattct cctgcctagg cctcccaaag tgcttggatt 3960
   ataggagtaa gtcactgcac ccagtctgac cttcactctt ttgttgatgg ggctgagcag 4020
   cttcatcaca gcctttttca ttttttattt ttttgagatg gagcctcact ctgttgccag 4080
   gttggagtgc tgtggtgcga tctcagctca ctgcaacctc cacctcccag gttcaagcaa 4140
   ttctgcctct gcctcccaag tagttgggac cacaggcgcg tgccaccaca cccacctaat 4200
   ttttgtattt ttagtagaga cggggtttca ccatgtgggc caggatggtc tcgatctcct 4260
   gacctcgtga tccgcccccc tcggcctccc aaagtactgg gattacaggc gtgagccacc 4320
   acgcctggct catcacagcc tctttaggca actttaagag aatgaagggc cttgttccag 4380
   gcaaggggtt agggaagctc tgcccctgat gaggagagga cccagggtgt ggagcctgac 4440
   tcccatcgcc agactagggg cttaggggag gaagggtttt gggagagtga gctcttgtgg 4500
   ccaggaggcc acggtcctgg actccaggag tgtcacttgg aggaccggtg ccacctcctt 4560
   ccccagagtg cccaagacag gagcaggaca ttgtgttcct gatcgatggc tcaggcagca 4620
   tctcctcccg caactttgcc acgatgatga acttcgtgag agctgtgata agccagttcc 4680
   agagacccag cacccaggtg tgcctttggg ggagggaggc tgctgggggt gggtgcttcg 4740
   atcctggtga aatggcctca gccccagccc tgtgtgcttc tcccagtttt ccctgatgca 4800
   gttctccaac aaattccaaa cacacttcac tttcgaggaa ttcaggcgca gctcaaaccc 4860
   cctcagcctg ttggcttctg ttcaccagct gcaagggttt acatacacgg ccaccgccat 4920
   ccaaaatgtc gtgtgagtcc tgatttcttc caggcacagt cccaaagcac ccaggtcttc 4980
   ccttgggcct catctgtctc cacgagaagg ggacaggcag ggaccaaaat ccagcccgtg 5040
   atacccttgc caagctgggg cctctgggtg ggactggggc ctcccaaagg aaaaggcatc 5100
   ttctaatttt cacaagggca ccaggggcta gtgtggtttg gttcacaggc ctctaagacc 5160
   tctcctttcc tgataggcac cgattgttcc atgcctcata tggggcccgt agggatgccg 5220
   ccaaaattct cattgtcatc actgatggga agaaagaagg cgacagcctg gattataagg 5280
   atgtcatccc catggctgat gcagcaggca tcatccgcta tgcaattggg gtagggcgtg 5340
   ggatggcttc ccacttctcc cacggcttcc tctcagggca actccccttt ctgtgtatgt 5400
   tcttttctct ttgagacagg gtcttgctct atcacccagg aagtggtgca atcctagctc 5460
   actgcagcct tgaactcctg ggctccagtg atcctcccac cccagcctcc ccagtagctg 5520
   ggaccacagg tgtgtgccat caagcctggc tattttcttt ttggttgaga tggggtcttg 5580
   ctatgttgcc caggctggtc tcaaattcct ggccttaagc aattctgcca ccttggtctc 5640
   ccaaagcaca gggattacag gcgtgaacca ccgccaacaa catccctttc aaggatagaa 5700
   acaccagctc tctcggctct tactgcctta aggatgaaaa ctctgcccca gactggagac 5760
   catgatcctt tctcctaaac tccctgatgc tgtccgggct tcgtgtttct cctgtgtcca 5820
   ccgggtgtga tcatgttgat cttgtggggt tattggaaga tgttgcaccc agtgcacaca 5880
   ggcacatttg atttattatt tttactgagt tgatcttttc tggggacagg ttggattagc 5940
   ttttcaaaac agaaattctt ggaaagaatt aaatgacatt gcatcgaagc cctcccagga 6000
   acacatattt aaagtggagg actttgatgc tctgaaagat attcaaaacc aactgaagga 6060
   gaagatcttt gccattgagg gtgagtctga agggagctct tcgcttgggg aatcctcagc 6120
   cgttaacacc tttccactta gaacccgagg ctccgtgaaa caggtagaca gcgtctcggt 6180
   tctcctgctt tcccgggacc ccgatagcca tgtctgtcag cttgtcccca ctgacgtccc 6240
   ccagcactgt cagagctgcc ccaaagtggc cccagggatg gccctgctcc ccacagagag 6300
   tgatctcaca ccaccaccgg ctccactgca gaacaaaaag cagtgccagg cccaacccag 6360
   gagacccttc cacccacacc gggccctacc cagcccacat cccaccagcc actcactccc 6420
   ctgggcaagg ggcacacgga cacctggccc cctcgggtct gcttgtagac ctgtgggggg 6480
   ccctgatgag gaccagatcg gtgctgccat cgctgtccac atccatggag cagagggggg 6540
   ccccgaagtc ggagctgatc tggagggcag agcctggtcc ctgtcacagg caccagctct 6600
   ccctgtagcc tccagtctta gcttctccta aagctgaagt gttcttggac ctggcaaagc 6660
   ccgtctccct ccctggcact caagcgtcat gccttccccc aggtacggag accacaagca 6720
   gtagctcctt cgaattggag atggcacagg agggcttcag cgctgtgttc acacctgtga 6780
   gtggggcccc ttaggccgat gatgtgccgt gaggggaggg ggagcaggga aggccagggt 6840
   gggtgtcagg tgggtaagag gcgcaggcgg aaggcatatc tctggtacat gctgtcttcc 6900
   tgctccggcc tctgctcagc cctggaatcc ttttctccca ggatggcccc gttctggggg 6960
   ctgtggggag cttcacctgg tctggaggtg ccttcctgta ccccccaaat atgagcccta 7020
   ccttcatcaa catgtctcag gagaatgtgg acatgaggga ctcttacctg ggtgagaaac 7080
   agccaggggt tggggacagg tgggagatgc actgcccagg gtggggtcca gggttctggg 7140
   gaaggggtat gggggctgtg ctgcccaggg tggggtccag gcttctgggg agggaggatg 7200
   ggcactgtgc tgcccggggt gggttccagg ttctggggag gggggatggg ggctgtgctg 7260
   cctggggtgg gggtccaggg ttctggggag aggggatggg cgctgtgctg cctggggtgg 7320
   gttccagggt tctggggaga gaggatgggg gctgcattgc ccaggtgggg gtccagggtt 7380
   ctggggaggg gagatggtgc tgtgctgccc ggggtggggg tccagggttc tggggagggg 7440
   gaatgggggc ctttgtgctg aggcctgggc ccctcaggtt actccaccga gctggccctc 7500
   tggaaagggg tgcagagcct ggtcctgggg gccccccgct accagcacac cgggaaggct 7560
   gtcatcttca cccaggtgtc caggcaatgg aggatgaagg ccgaagtcac ggggactcag 7620
   gttgggcgtg acaggagcca gaggggagga tgagggtagg ggaggtggct ggggcagagg 7680
   agaggatgga ggggctttga gggccttggg ggaggtcctg gtacctgggg agaggtggga 7740
   cctggcccac agggctgcct ctggcaggga caggcagcat gacccaggct ctgcccttca 7800
   gatcggctcc tacttcgggg cctccctctg ctccgtggac gtagacagcg acggcagcac 7860
   cgacctggtc ctcatcgggg ccccccatta ctacgagcag acccgagggg gccaggtgtc 7920
   tgtgtgtccc ttgcccaggg gggtgagtgg ctgatgggcc tggggtgggt ggggtctggt 7980
   gtgggtggag gggttgcccg ggttgggcct ggcactgctt tttttctgca gtggagaagg 8040
   tggtggtgtg atgctgttct ctacggggag cagggccacc cctggggtcg ctttggggcg 8100
   gctctgacag tgctggggga tgtgaatggg gacaagctga cagacgtggt catcggggcc 8160
   ccaggagagg aggagaaccg gggtgctgtc tacctgtttc acggagtctt gggacccagc 8220
   atcagcccct cccacagcca ggtgaggccg tgtcccattt ctgtcactag agcagcctgc 8280
   ttcttgcctc tcccactctg tcatactgga aaactgtccc tttttacctt ttcctacctc 8340
   ccttgcccag ctctgagcac cttgtagcag tggcgtggtc tcagctcact gcaacctccg 8400
   cctcccaggt tcaagcgatt ctctgcctca gcctccagag tagctgggat tacaggcatg 8460
   caccaccatg tccggttatt ttttgtattt tagtagagac aggctttcgc catgttggct 8520
   aggctggtct tgaactcctg acctcaggtg atctgcctgt ctcggcctcc caaagtgctg 8580
   ggattatagg cgtgagccac catgcccagg cccctgccag ttttacaagg tacacaggtc 8640
   aggcacagaa aacccatttt acagatggaa tctgggacac taggaagaca agggccttgg 8700
   tttgttggag gttcagagtg ggtccgagat ggtgaagggc actccggcct cctgacctct 8760
   aacccggtgt gcagtctccc ggctccctgc tgctcaccac ttaggtccag tcatttcaac 8820
   cttcccttcc actcgcccct ctcctccctg gatgctacat gattttattc ccttcctgcc 8880
   atcaaggtcc caccaaatgc ccatccctgc agccgccctc caccccaagg gagcagggtt 8940
   ccctgagaac gaagggctgc ttttcttggc aaaagtcaag aaagctctgt taaaaaatag 9000
   gcaaaggggc tgctccaggt ggctcacatc tgtaattccg acactttggg aggctgaggc 9060
   aggaggatca cttgaggcca ggagtttcag gccagtctgg gcaacatagg gagaccccat 9120
   ctctagaaaa aattttaaaa attaggcagg cattgtggtg ctgtagtaac tgtagtccca 9180
   attactcggg aggctgaggt gggaggtcac ctgagcccag gaggttgagg ctgcagtgag 9240
   ctatgattgc gtcacttcac ttcagcccag gtgaaagagc acaatcccgt gtcaaaaaaa 9300
   aaaaaaaaaa aaaaaaaagg gcaaaggatt tggatagggt agacgtttct ccaaagaaga 9360
   ggtacaaagg gccaatgggt gcataaaatg attctcaatg tcattagtca tcagcaaatc 9420
   aaaactataa tgagatgcca cttctcacct actagaatgg ctagaaacaa aggcggataa 9480
   tgacaggtgt tggtgaggat gtggagaacc gggcatcctc ccccatgctg gggatgatgt 9540
   aaatccacgc agctgctttg gaaatcagca tggcaggtcc tcaaaacatt aaacataggc 9600
   tgggcacagt ggctcatgcc tgtaacccca gcactttggg aggccgaggc aggtggataa 9660
   cctgaggagt ttgagactag cctggccaac acagtgaaac cccatctcta ctaaaaatac 9720
   aaaaaaaaaa aaaaaattag ctgggcatgg tggtgggtgc ctgtaatccc agctacttgg 9780
   gaggctgagg caggagaatc tattgaacct gggaggtggg ggttgtggtg agccgagatc 9840
   gcggcactgc actccagcct aggcaacaag agcaaaactc cgtctcgaac aaacaaacaa 9900
   acaaaaagtt aaacatagag ttgccatgtg atctggcaat tccacctata aaacatgcta 9960
   agtgagggaa gccaaacaca gaaggccaca tgtattcagg ccaaatgtcc agaacagaca 10020
   gatctgtaga gacagaaagt agatgagcag tttcacaggg ctgggtgtgg gggaaagtca 10080
   ggggcatgag ggtgactgct aatgggtgtg gagtctcttt ttggggtagt ggaaatgttc 10140
   taaaattgat tgtggtagtg gtttcacaac tctctaaaca taccaaaagg cactaagtca 10200
   tatactttaa aggggtgagt tgtgtgctat gtggtctata gctcaaaaaa gctgttacca 10260
   aaaagaaaaa agtgccaagg aaggtccctg cagttctgtc acacaccagc tgtgtgatct 10320
   ttgacaggta actcaccctc tctgtgctca gttttctcac tggtaaaatg gtgatactaa 10380
   tagaaccaac cttattgagc tgctgctgtg aggattaaat gagttagtat gcatgaagtg 10440
   cctggcacac aataggtatc atgttgttat aatctgtttt aattatcatt aagatagttt 10500
   aacaactggc caggcgcggt ggctcccgcc tgtaatccca gcactttggg aggctgaggc 10560
   aggcagatca cttgaggtca ggagttccag actaggctgg ccaacatggg gaaaccccgt 10620
   ctctacccaa aatacaaaaa ttagttgtgc gtaatggcgc atgcctataa tcccagctac 10680
   ttgggaggct gaggcaggaa aatggcttga acccaggaat cggaggttgc agtgagctga 10740
   gattgcgcca ctgcactcca gcctgggtga tagagtgaga ctccatctct aaacaaacaa 10800
   acaaacaaaa aacacatagt ttaacacctg aaggggaatc taaagaagag ggtgaaagac 10860
   tgttgagaat aacaagggcc cacgtgttgt catgaaactg caaacagttc gtaccaattc 10920
   ccacaaccca tcacatgcag gcataccttg ttttatgcgc tttgcagata ctgcattttt 10980
   tacacgctga aggtatgcag cgaccctgca ctgagcaagt ctatcagtgc tgttttccca 11040
   gctgcatgca ctcactttgt gtctccgtgt tactttggta attctcacaa tgtttcaaac 11100
   ttttttgtaa ttatcatgtc tgtgatggtg atctgtgatc agtgatcttc gatgttgcta 11160
   ttgtcctctt tttggggcac catgaagcac gtccgtatat gaaggcgaac ttcataaatg 11220
   tgtgtgttct gactgctcca ccactgatag ctcccctgtc tctctctccc tctcctgtgg 11280
   cctccctatt ccctgcaatg caatgatatt gaaattaagc caaataataa ctctacaatg 11340
   gtctttaagt gttcaactga aaggaagagt cacatgtctc tcactattta tttatttatt 11400
   gaggcaggat ctcactctgt cacccagcct ggagtacact ggtgtgatct tggctcactg 11460
   aaacttcccc ctctggggtt caagtgattc tcctgcctca gccttctgag tagctggcat 11520
   tacaggtggg tgccaccatg cctggctaat tttgtgtttt ttagtagaga tggggtttca 11580
   ccatgttggc caggctagtc tggaattcct gactctaagt gatctgcctg ccttggcctc 11640
   ccaaagcact gggattacag acgtgggcca ccacgtctgg ccacatatct ctcactttag 11700
   atcaaaacct agaaatgatt aagcttggtg aagaagccat gccaaaagcc aagatgggct 11760
   gaaggccttt tgcaccaaac agccaaattc tgaatgcaaa gaaaaagtta ttgaaggaaa 11820
   ttaagagtgt tactccagtg aacatatgaa tgataagaaa gtgaaacagc ctaattgctg 11880
   atatgaagga agtctgagtg atctggatag aagatcaaac cagccacaac atccccttaa 11940
   gccaaagcca aatccagagc aaggtccgaa tgctcttcag ttctatgaag gctgagagac 12000
   gtgaggaagc ttcagaagaa aaattggaag tcagcagagg tggaagtcag cagggttcat 12060
   gaggtttaag gaaagaagca atctccacaa cataaaagta caaggtgaag cagcaagtac 12120
   agatggagaa ggcgcagcaa gttctccaga agatctctga tgaaggtggc tacactcaac 12180
   aacaggtttt cagtgtagat gagatggcct tcgatggaag aagatggcat ctaggacttt 12240
   cagagaagtc aatgcctggc ttcaaagctt caaaggacaa gttgactctc ttgttagagg 12300
   ctaatgcagc tggtaggact tcaagttgta gccagtggtc atttaccatt tggaaaattc 12360
   cagggccctg aagaatgatg ctaaatccac ccttcttgtg ctctagaaat ggaacaaagc 12420
   tggggtgaca gcacatctgt ttatagcatg cttgactgag tgttttaagc ccattctgga 12480
   gacctgctgc tcagaaaaat agatttcttt ccaaagtttg ctgcttattg acagtgcacc 12540
   tgatcaccca agagctctga tggagatgtt caaggagatt agtgttgttt ccatgcctgc 12600
   aaacacaaca cccattctgc agtccatgga ccaaggagta atttcgactt tcaagtctca 12660
   ttatttcaga aatacatttc ataaggctat agctgccata gatagtgatt cttctgatgg 12720
   atctgggcaa agtcaattga aaaccttctg gaaaaaaatt caccatttta gatgccatta 12780
   agaacattca tgattcatca gagggggtca aaatatcaac attaacacga gttcgggaga 12840
   agctgattct gattctcatg gatgactttg agtggctcaa ggcttcaggg gaggaagtga 12900
   ttgcggatgt gtggaaatag agaaccagaa ttagaagtgg agcctgaatt gctgcagtct 12960
   catgatgaaa cttgcacaga tgaggacctg cttcttacag atgaggaaag aaagtgtttt 13020
   tttttttttt cagatggaat ctattcctgg tgaagatgat ataaatattg ttgaaatgac 13080
   aacacaagat taagaatatt acataatccc agcacttagg gaggctgagg tgggcagacc 13140
   acctgaggtc aggagttcaa gaccagcctg gcctacatag ggaaacctct ctactaaaca 13200
   tctctactaa aaaaattagc tgggtgtggt ggcagatgtc tgtaatccca gctactcagg 13260
   aggctgaggc aggagaatcg cttgaacccc tcaggcaaag gttgcagtga gccgagatca 13320
   caccactgca ctctagcctg ggcgacagag tgaaagtgtg tctcaaaaaa aaaaaaaaaa 13380
   agaatattat ataaacgtag ttgataaatc agcatagaat ttgagagaat tgactccaac 13440
   tttctttttt tattttttat tttttattta tttttttatt ttttattttt tttattgatc 13500
   attcttgggt gtttctcgca gagggggatt tcgcagggtc ataggacaat agtggaggga 13560
   aggtcagcag ataaacaagt gaacaaaggt ctctggtttt cccaggcaga ggaccctgcg 13620
   gccttccgca gtgtttgtgt ccctgggtac ttgagattag ggagtggtga tgactcttaa 13680
   cgagcatgct gccttcaagc atctgtttaa caaagcacat cttgcaccgc ccttaatcca 13740
   tttaaccctg agtggacaca gcacatgttt cagagagcac agggttgggg gtaaggtcac 13800
   agatcaagag gatcccaagg cagaagaatt tttcttagta cagaacaaaa tgaaaaatct 13860
   cccatgtcta cttctttcta cacagacacg gcaaccatcc gatttctcaa tcttttcccc 13920
   acctttcccc cctttcgatt ccacaaaacc gccattgtca tcatggcccg ttctcaatga 13980
   gctgttgggt acacctccca gacggggtgg tggccgggca gaggggctcc tcacttccca 14040
   gtaggggcgg ccgggcagag gtgcccctca cctcccggac ggggcggctg gccgggcggg 14100
   gggctgaccc ccccacctcc ctcctggacg aggcagctgg ccgggcgggg ggctgacccc 14160
   cccccccacc tccctcccgg acggggcagc tggccgggca gagggtctcc tcacttccca 14220
   gtaggggcgg ccgggcagag gcgcccctca cctcccggac gactccaact ttcaaagaag 14280
   ctctactgtg gccaaatgtt atcaaccagc atcgcatgct ccagagaaat ttcatgaaag 14340
   gaagagtcca tccaggcagc aaacttcctc acggtcttat tttaggaaat tgccatagcc 14400
   accccaacct ccagcagttg ccactgtgat cagtcagccg tgtcaccatc aggcaagacc 14460
   ctccaccagc aaaaagatta tgccttgcta aaggcttaaa cgattgttag cattttttac 14520
   caatcaaata ttttattttt atttttgaac ttttagattt agagggggta cacctagcct 14580
   ttctagactc cctcagtaat tggtaaattt ctctttttat tttgttatta ttcctttttt 14640
   tcttttcttt tttttaattt ttatttttta gagacagaat ctcatcatgt cactcaggct 14700
   ggagttcagt ggcgcaatca tagctgactg cagcctcgaa ctcctggact gaagagatcc 14760
   tcctgccaaa gactcctgag tagctgggac cacaggcaca aagccagtgt gccctgccca 14820
   ttattattac cttttttttt gagacagagt ctcgctctgt cacccagact ggagcacagt 14880
   ggtgcaatct ccgctcactg caacctctgc ctcccgggtt caagtgattc tcctgcctca 14940
   gcctcctgag cagcttggat tgcaggtatg tgccatcatg cccagctaat ttttgtttat 15000
   ttagtagaaa cggggtttca ccatgttggc caggctggtc tggaactcct gacctcagat 15060
   gacccacccg cctcggcctc ccaaagtgtt gggattacag gtgtgagcca ccgcgtccag 15120
   ccattattat cacttttaat ggctgctagt tccagggtgc acagagattt aaagggcaca 15180
   ttcttccagg gattgggcat aacatgagca ctgggaatga aggtgatagg ttctacaggg 15240
   gaggcagtgc ctgggagggt ggatctgggt gtgggtgcga ccactcccta ccccaccgcg 15300
   gctttttctt gtcctttgaa gagacagagt gacagcaaga aacaaaaagg ggacttgggc 15360
   ctggggttgg acggtctggt gttaggtctg cagtcagcct cttaaaggct acctggggtt 15420
   gacactgtcc ccaacctctc tcctgcatct ttgcaacgag ggaggtgagg ttggacaaac 15480
   tctgaggtcc ctttcagcct taatactcat tgtgatatat tattgactgc tggctgtatc 15540
   ctgggtgctg ttctaagaac cgtaggaatt cctctcctga gcatggctaa actctgagct 15600
   aatagtatca ttatatgaaa gatgaggaaa cggaggcaca gacagattga gtccttgccc 15660
   acggcctcgt ggctcatacg tggaggagtc agaattggaa ctagagactg atcgaatgaa 15720
   tgacactcgg gtcaccagga cacccttcct atctccactc ttacatctgt ttcttagcaa 15780
   tcatctccca actcctacct cctcttttca ggttcttctt ggtgacatct gttcacaact 15840
   caccccttct ctccctttcc gatggtccta cctccatatt ccccttgtta cttatttcca 15900
   acttcttccc ctactttcca tcttgattca cccttctctc ctctggccag cggatcgcgg 15960
   gctcccagct ctcctccagg ctgcagtatt ttgggcaggc actgagcggg ggtcaagacc 16020
   tcacccagga tggactggtg gacctggctg tgggggcccg gggccaggtg ctcctgctca 16080
   ggtgagagca gcctttctca gaggctcccc aggtggtcct aggttcagat gggggtgccc 16140
   accccacgtg gtgctcccag gagccgacgg cctgtcctca gctcggtgct ctgcccgcag 16200
   gaccagacct gtgctctggg tgggggtgag catgcagttc atacctgccg agatccccag 16260
   gtctgcgttt gagtgtcggg agcaggtggt ctctgagcag accctggtac agtccaacat 16320
   ctgcctttac attgacaaac gttctaagaa cctgcttggg agccgtgagt cccctcccct 16380
   ccaacccagg acaccctgac ctctggagtc ccccatccca ggcccctgtc tcccaccctg 16440
   ctcattgtcc acccaaggag ttcctgtctc aacgccgtcc ctgcgaccgc ctacaggtga 16500
   cctccaaagc tctgtgacct tggacctggc cctcgaccct ggccgcctga gtccccgtgc 16560
   caccttccag gaaacaaaga accggagtct gagccgagtc cgagtcctcg ggctgaaggc 16620
   acactgtgaa aacttcaacc tgctgctccc ggtgcgtctg ggcatgaacg tgggtggcgg 16680
   ccgcgctggg gctggcagaa ggcagggcag ggagagaaca ggctgtgttc cggcctccct 16740
   gtggctcagc ccagcacagg accagccatg caggacgtgc ttactgcacg ttagccagtg 16800
   agtgagtgag cgagcaaaca agtgatgaga tcggctgcaa tttccagggg ccacacgatt 16860
   ggatttcagg aaagagaatt gggcaacctg agagagctct ggggcttccg gctgggcttt 16920
   ttttcaggca ttcactggac aggggtttat cgagctgctc ctgggagaca ggccttgccc 16980
   tgggggccag gggcataggt ggccaaaaca gtcattgctg atcgggaggt ctgggggggg 17040
   ggaggaaaaa aacaaagaca aacaagggga gagaacagag agggtgtcag ggaggcctcc 17100
   tgaaggcggt gacgctgagc aggctctgga ggaagctgaa gccgcgcggg agctgggcag 17160
   aggcaggata agaactgcgg atgaggccga gcgcagctct taccctcccc ttaccctccg 17220
   ctccccgcga cgcccgtccc ccagagctgc gtggaggact ctgtgacccc cattaccttg 17280
   cgtctgaact tcacgctggt gggcaagccc ctccttgcct tcagaaacct gcggcctatg 17340
   ctggccgccg atgctcagag atacttcacg gcctccgtga gtcctggcac tgggtctccc 17400
   agagagggtg cacagcgtgg ggcctgggtc tcggagaaaa ccccccgttg ccttcccacg 17460
   cagctaccct ttgagaagaa ctgtggagcc gaccatatct gccaggacaa tctcggcatc 17520
   tccttcagct tcccagggtg agcgccccca ccttagacct gccctactgc cccagcctcc 17580
   ttcctggaat ctgggactcc tgcctctggc tctccctaac attgtctcat cctatagtca 17640
   aaacccaggt gtcttggctg ggcacagtgg ctcacgcctg taatcccagc actttgggag 17700
   gccgaggtgg gaggatcttt tgaggccagg agttagggat cagcctgggc aacagagcga 17760
   gatcccattt ccacaaaaac aaaacaacaa caacaacaac aacaacaaca tcacttgagt 17820
   gtggtagagc atgcctatag tcccagctac ttgggaggct gaagcttaag gattgcttga 17880
   gctctggagt tggaggctgc agtgagccat aatcacacca ctgcactcca gcctgggtga 17940
   aagagcagga ctctgtctct taaaaaaaaa gaagaagaag aagaagaaga agaagaagaa 18000
   cccaggggtc cgtcccctgt ctatctccca aatccccacc caccccattt tatcccagac 18060
   catttctagc ctcagtcaca gaatcatctt ctcctttcct tcacctgata cccagcttga 18120
   agtccctgct ggtggggagt aacctggagc tgaacgcaga agtgatggtg tggaatgacg 18180
   gggaagactc ctacggaacc accatcacct tctcccaccc cgcaggactg tcctaccgct 18240
   acgtggcaga gggccaggtg caccctctgg ggaaggagga ggaggcaggg ctgggcgtta 18300
   gcgtagattc ccgtgcggtt cagaacccgg gctgggcttg gaggtggtag tgccatctgg 18360
   ggcacgcctc tgctcgtgtg ggtgttcaac tttgggcatc gcagtttaag gacactgact 18420
   atcaggaatg atttcctgag aggccactaa tgtggagaca gaggggaacc tgcccaaatt 18480
   tctttctatt ccatattata tcagtattcc tcttttaaat gaaaacatac ctcagccttc 18540
   aaatatttta aaacactgtt ctgtagaagc aaggggttga atttattggt cactctacat 18600
   taaggcttgg cgaccttttt ctataaaggg ctagatagta aatatttcag cttcgtggtc 18660
   tgcgtggtct gtgtcacagc tgctaacctc tggtgttgtg gaatgaaagc agccattgac 18720
   atgaatgagt ttatgagtga atatacctgc gatgcagtaa aactttataa aaataggtag 18780
   tggctgttgg ttgccaatcc ctgctttaaa atgcagaact aggaccacgg ggagtcaaag 18840
   agaaccagat tttttaaaca aagatgttat tatggctggg agtggtggct cacgcctgta 18900
   atcccaggac ttcgggaggt tgaggcaggc ggatcaccgg aggtcaggag tttgagacca 18960
   gcctggccaa catggtgaaa ccccatctct acgaaaaata caaaaattag ctgggtgtgg 19020
   tggcaggtgc ctgtgatccc agctactggg gaggccgagg tgagagaact gcttgaaccc 19080
   ggaagacgga ggttgcagtg agctgagatt gtgccactgc actccagcct gcacaacaga 19140
   gcaagactct gtctaaaaaa aaaaagttat tatgaaaaat ttaaaacatt tacaaaagta 19200
   gaaagtagtt caacaaacct ccccaaaact tgtcacctag attcaacaat tagcagcatt 19260
   ttgccacatt tgtttcatca atttttcccc tttgctaagg tattttcttt atttctttat 19320
   ttttgagaca gggttttgct ctgtcacaca ggctggagtg cagtggcaca atttcagctt 19380
   actgtagcct tgacctcctg ggctcaagcg atcctcccat ctcagcctcc tgagtagcta 19440
   gaaccacatg cacacaccac cacacctggc taatttttta attttttttg tagagttggg 19500
   gtcttaccat gctgcccaaa ctggtctcta acccctgggt tcaagcagtc ctcctatctt 19560
   agcttcccaa agtgttggag ttacagacat gagccactgt gcctgacctg ctaaagtatt 19620
   ttaaaacaaa tctcagacat gggggtcatt gtactcctcc ttattcactc tgctatggtt 19680
   tgaatgcatc acccaatgtt cgtgtgttgg acacttaatc cccaatgcaa cagtgttgaa 19740
   aggtgaaacc ttttaaaggt gattaggtta tgagggcttt gccctcatga atggatgaat 19800
   actattatct caagagtaga ttagttattt caggagtggg tttccttcct tcctttcttc 19860
   tttctttttc tttctttctt ttccttcttt ctttctttcc ttgagagggt cttgctctgt 19920
   tgcccaggct agggtgcagt ggcacgatct tggctcactg cagcctctgc ctcccgaatt 19980
   caagcaattc ttgtgcctca gcctcctgag tagctgggat tacaggtgcc cactgccacg 20040
   cccagctaag ttttgcattt ttagtagaga tggggtttca ccatgttggg cagtctggtc 20100
   ttgaactcct gacctcaaat gatccacccg attcggcctc ccaaagtact aggattacag 20160
   gcgtaagcca cggagcctag cccaggagtg ggtttctaat taaaggatga gtttggcccc 20220
   ctttccctct cactctcaca tgcactgcct tgcacatgca gcacaaaggc cctcaccaga 20280
   tgctggtgcc atgttcttgg acttcccagc ctccagaacc acaaagcttc tattgataca 20340
   ttacctagtt tgcggtaatc tgttatagca gcagggaaca gacaaagaca cactctatag 20400
   ttctgaacaa tgaggacctc ttatatgacc tcaacaccat tatcacaccc aatgaaataa 20460
   gtgattcctt ggtattaaga tcatagtcaa aattgtgtaa ctgtctcaaa aatatctttt 20520
   tacttctggt ctatttgaac gaaaattcga actaggtcca tacatgatgg ttggttgtta 20580
   tttctgctgt ctctcttagt ctaatattcc cagcggaagc gcattttgat taacgtataa 20640
   aagaacacag caggaaaggc catctcttgc atgtgagggg tgatcactgt caatgaaagt 20700
   gctcctagac atatcaacca aatgtaatgg gtggaccttt gctgaatatt gattttaaca 20760
   aatgagctgt aaaataacat ttttggagac aatctaagaa aatagaacat gaccagtata 20820
   ttggtatatt aaggagtaat tgttaatttt gttggctgtg atttgttggt tcttgtggtt 20880
   acatttaaaa tacccttatc tattggtggt atacactgaa gtatttctag atgaaacaat 20940
   atggtgtttg gaatgtgctt taaagtgttc agaaagagaa catagcagaa acaagagtag 21000
   aaaaatgttg gttacttaaa atgagtgatg gttatatggg gttgattttc cagtctctct 21060
   attttttaat gtgttttaaa ttttccatta taagaggcct ttaaaataac aataaaaagg 21120
   tgaacatttt aatacatttg agatttctac tttggaagaa agtttgacca gatagctatg 21180
   ggggagcttt taaaaacgta accttggctg gacatggtgg ctcatacctg taatcctagt 21240
   gttttggggg gacaagatgg gaggattgct tgaggccagg agttcaagac cagcctgggc 21300
   aatatagcga ggtcccattt ataaaaaaat ttttttaaaa ttagccaggc atggtggtgg 21360
   tgtgtgcctg tagtcctagc tactcaggag gctgaggcaa gaggatggct tgaggccaga 21420
   gtttgaggct gcagtgagct gtgattgcat cactgctttc tagcctgggt gacagagtga 21480
   gacctggtct caaaaaacaa acaaacaaac aaaaacataa gcttaaggtg ggctccagga 21540
   agctttatca ctacttcgtg gcgtgtcttt ggaatgctgt tatattaggt tggtgcaaaa 21600
   gtaattgggt ttttgccatt gctttcaatt tcaactaata ctcctctact ttttctcatg 21660
   cctagaaaca agggcagctg cgttccctgc acctgacatg tgacagcgcc ccagttggga 21720
   gccagggcac ctggagcacc agctgcagaa tcaaccacct catcttccgt ggcggcgccc 21780
   aggtcagcct ggcttctgtc ccctcactgc tcccctgccc caccctgtct ttactgctct 21840
   gtgacctctc agttcctttt cctcagatca ccttcttggc tacctttgac gtctccccca 21900
   aggctgtcct gggagaccgg ctgcttctga cagccaatgt gagcaggtga gccgggccag 21960
   gccaggggca gtgcccctca tctccagcct cacaccccat tctcctcctc tggggcctct 22020
   ggcaactgag tctctcctct ttctccagtg agaacaacac tcccaggacc agcaagacca 22080
   ccttccagct ggagctcccg gtgaagtatg ctgtctacac tgtggttagc aggtcagcag 22140
   gtaccccact gcaggaaaaa gggttcttct ctctgacctc aaaaagaaaa aaaaaaaaag 22200
   gccttgaaac gctgccacag agggtgagat aaggtgtttg aaactaaaag gtcaggtgtt 22260
   tcagcagaca ccttccttca gccaatgcct tcctcgaatt tgctgtgtgc caggcagggt 22320
   gctgtggtta ttttccatac attcatttga cattcattga agatttactg agcccccatt 22380
   atgtgtgatc aaaaccagac atgaaccctc gcccttgtgg ggtgtgcctt gctggatgtt 22440
   ctcctgtgcc tggtgtttcc cactctcacc tgcacctgca tgcgtggagg ctcccagcca 22500
   ggtgcacgct ctgagctcgt gtgctggggt gtgccccagg ctctcatacc ccttggcagg 22560
   ggacccacag gcagggctca cctactctag gcatgtgtgg tccacagctt ggccacacat 22620
   ggcaggtgtg atccacagct tggccacaca tggcaggtgt gactctgtgg gttagggcac 22680
   agggagtgcc aggttggggc atttctgggg gaggccaagg tgggaggatt gcttgaggcc 22740
   aggagttcag gatcagcctg ggcaatatag tgagacccta gctctacaaa aaattggcag 22800
   aggaggaatg aggcttcaga gtaggcgtgg tgggctggtg tttggactat gccagctgga 22860
   ggacaggtgc cggaagagtg aagcagtgag gcagtggtga gactggccac ctgtgtgatg 22920
   gtcccagctg cccaccaggt ctccaggctt gtgcccaaaa agggctcagg aggtgttaaa 22980
   ctgagacctc atgaagagtc tgctgtgggc cctccctccc ctccaagccc caggcccgga 23040
   aaagccaagg aaatgggaaa aaagggcccc cggggaggta tgtgggggac tgggaagggg 23100
   caatgctcag tagcatttgg tcaacagcgt cagcctgggc caccccaact ctgtgctgtg 23160
   tcccacagag ccctggcatc ctacatcccc acaaagggat ggaaggaaag cggaggggca 23220
   ggccatggcc cctgctctcc tgcagtcagt ggcagagctc tctgctccgt tcattgtacc 23280
   gactacccgg ccccactggc agcctcctct gcaggcagct atgagctctt tgcctcattt 23340
   tttttttttt aaagcccagg tcttgctctg ttgcccaggc tggagtgcag tggtgtgatc 23400
   acagctcaca gcagccttga actcctcttg ggctcaagcg agcctcccac ctcagcctcc 23460
   caagtagttg agactacaga tgtgtgccac catgcctggc taatttaaaa aattgttttg 23520
   gagagatggg gtcttgctat gttgcccagg ctgatcttga atcctggcct caagtgatcc 23580
   tcccacctca acctcccaaa gtgctgggat tgcaggtgtg agccactgca ctcagccaat 23640
   tagcacttgt ttgaagccca gccctgcttt tctagactct ctctcttttt tttttttttt 23700
   gagaccaagt ctcactgtgt tgcccaggct ggagtgcagt tgtaccatct ctgctcactg 23760
   caacctccgc ctcctggctc caagcaattc tcgtgcctca gcctcccaag tagctgggat 23820
   tacaggcacc tgccaccaca cctggctaat ttttgtattt ttagtagaga tggtgtttct 23880
   ccatgttggc caggctggtc ttgaactccc gacctcaggt gatctgcctg tctcagcctg 23940
   tcaaagtgct gggattatag gcgtgagcac tgtgcctggt ctctaaactc tctctctctg 24000
   tttttttttg agagagagag agtcttgctc tgtcattcag tggcgtgatc tgggctcact 24060
   gcaacctctg tcccctgggc tcaaacaatt ctcctgtctc agcctcctga gtagctggga 24120
   ttacaggtgc acaccatcat gcctggctaa tttttgtatt tttagtagag acagggtttc 24180
   accatgttgg ccagggtgat ctcgaactcc tgatctcagg tgatccgccc atctcagcat 24240
   ctcaaagtgc tgggattaca ggcctgagcc accgcgcctg gcctctaaac tctcttataa 24300
   cctaactcag ccacagccct cattccagga cattccaagg ccccaccgac cacctgtcct 24360
   ctcatgctct agccaatgcc ttctgcagat gccccatggt agttcacatc cacttatgcg 24420
   tcttctctct ccagccacga acaattcacc aaatacctca acttctcaga gtctgaggag 24480
   aaggaaagcc atgtggccat gcacagatac caggcaggtg gtggagacgc aggagactgg 24540
   gctggggtgg gaggctggga gccggagact ggggagggat ttgggctttg gcgtgggctc 24600
   tgccctcagt gccctctgtg caggtcaata acctgggaca gagggacctg cctgtcagca 24660
   tcaacttctg ggtgcctgtg gagctgaacc aggaggctgt gtggatggat gtggaggtct 24720
   cccaccccca ggtacccaag gactgcatgt ggctcctcca cgaatgccct ttctacctgg 24780
   attccttgtg ccccatgtgg gtccctgatg tcccagctga gacacttgtt ctctgcattt 24840
   tcccccagaa cccatccctt cggtgctcct cagagaaaat cgcaccccca gcatctgact 24900
   tcctggcgca cattcagaag aatcccgtgc tggtgaggag ggctctgggc tggccctcac 24960
   tgtaggcccc acatcagagg aatttaaccc aggagttcat gttccatatc catcctgctg 25020
   aagtaccctc ttgcattcgg atatggccgc tgccctcaag tcacaccgca taatgctgcc 25080
   tcccaccttc acactcatct ttctcagccc catgctattt atctgccccc aggactgctc 25140
   cattgctggc tgcctgcggt tccgctgtga cgtcccctcc ttcagcgtcc aggaggagct 25200
   ggatttcacc ctgaagggca acctcagctt tggctgggtc cgccaggtgt gtgggtgcaa 25260
   cgacagagcc cctgccccag actcaggcgg gacctggcat gtctgtgccc atctgcaagc 25320
   cagggcaccc ccagagctct gagcctcccc cagagccagt tcaacaggtt tcccccaacc 25380
   cctttgcaga tattgcagaa gaaggtgtcg gtcgtgagtg tggctgaaat tacgttcgac 25440
   acatccgtgt actcccagct tccaggacag gaggcattta tgagagctca ggtagagacc 25500
   atgtggaggg cagcgaccag gctggaaaga gggcccctag ggctacatct gtggtgctgg 25560
   gtggggggtt tgcaagcctt gggggaggag ggcgaaggcc tctgggcagg atagctgtcc 25620
   ctaagggcac gggtgctgct gtgtctcacc tcttggagca gggcctgggg aaggagggga 25680
   gggagttaaa ggttggggag cctggaggga gtctgggata gtaggaggat gggagtgctc 25740
   tgacagggtc acttccactt cagacgacaa cggtgctgga gaagtacaag gtccacaacc 25800
   ccacccccct catcgtaggc agctccattg ggggtctgtt gctgctggca ctcatcacag 25860
   cggtactgta caaagtgagt gttttatgcc actcttgaca ccaccagcat ctggtcccgc 25920
   tctttttgca gagtgagaag gagctcactt tgaaggcaga ggcacattct tactgggtca 25980
   cttcatatga gaaactgctt cccacctgca atgtcacctg tccccagtgg ccccctgctt 26040
   tgtgattccc aggcttcctc taatatttct ccctttcttt cctgctcttc tccatcattc 26100
   tacgtgtttc ccttgacagc agattatcat ataaaagcac agacctgggt ttgaatgtcg 26160
   acatcaccac gggttctttt tgtctttgac cataggccag tgtctgctcc actctgggcc 26220
   ttgatttctc aatgtgaagg tgattctacc ccagctcata gaattttggc taaatctagg 26280
   ctgggcacgg tggctcatgg ctgtaatccc agcagtttgg gagatcgagg caggtggatc 26340
   acttgaagtc aggagttaag agaccagcct ggcctacctg gtgaaacccc gtctctacta 26400
   aaaatacaaa aattagttgg gcatggtgga ggttgcagtg agccgagatc acgccactgc 26460
   actccagcct gggcaacaga gtgagactcc atctcaaaaa gaaaataatt ttggctaaat 26520
   ctagatgaca tgatgcatat aaagttccta gtgcagtgct ttgaacctag caaggatttc 26580
   agaaatagtg acccctctcc ctcagtcatc tcttttcctc tcttccactg aatgggcttc 26640
   ctgagtttct tcttcgtcct cccccctagg ttggcttctt caagcgtcag tacaaggaaa 26700
   tgatggagga ggcaaatgga caaattgccc cagaaaacgg gacacagacc cccagcccgc 26760
   ccagtgagaa atgatcccct ctttgccttg gacttcttct cccccgcgag ttttccccac 26820
   ttacttaccc tcacctgtca ggcctgacgg ggaggaacca ctgcaccacc gagagaggct 26880
   gggatgggcc tgcttcctgt ctttgggaga aaacgtcttg cttgggaagg ggcctttgtc 26940
   ttgtcaaggt tccaactgga aacccttagg acagggtccc tgctgtgttc cccaaaggac 27000
   ttgacttgca atttctacct agaaatacat ggacaatacc cccaggcctc agtctccctt 27060
   ctcccatgag gcacgaatga tctttctttc ctttcttttt tttttttttt cttttctttt 27120
   tttttttttt gagacggagt ctcgctctgt cacccaggct ggagtgcaat ggcgtgatct 27180
   cggctcactg caacctccgc ctcccgggtt caagtaattc tgctgtctca gcctcctgag 27240
   tagctgggac tacaggcaca cgccacctcg cccggcccga tctttctaaa atacagttct 27300
   gaatatgctg ctcatcccca cctgtcttca acagctcccc attaccctca ggacaatgtc 27360
   tgaactctcc agcttcgcgt gagaagtccc cttccatccc agagggtggg cttcagggcg 27420
   cacagcatga gaggctctgt gcccccatca ccctcgtttc cagtgaatta gtgtcatgtc 27480
   agcatcagct cagggcttca tcgtggggct ctcagttccg atttcccagg ctgaattggg 27540
   agtgagatgc ctgcatgctg ggttctgcac agctggcctc ccgcgttggg caacattgct 27600
   ggctggaagg gaggagcgcc ctctagggag ggacatggcc ccggtgcggc tgcagctcac 27660
   ccagccccag gggcagaaga gacccaacca cttctatttt ttgaggctat gaatatagta 27720
   cctgaaaaaa tgccaagaca tgattatttt tttaaaaagc gtactttaaa tgtttgtgtt 27780
   aataaattaa aacatgcaca aaaagatgca tctaccgctc ttgggaaata tgtcaaaggt 27840
   ctaaaaataa aaaagccttc tgtg 27864

<210> 37
<211> 18501
<212> DNA
<213> Homo sapiens

<400> 37
   gctttaatgg cattaggaca gtgggctggg cctgtttaga aactcatgta cctgactgta 60
   tttctcagcc tcagtctctg tgggatgcag aggttcagtg ctctcaacaa caagggccaa 120
   gaaatccaag tgtccgggcc cctgctccct ccaagggtca aggtaaagaa agcagcaagc 180
   actgtggtga gaagaatcag agggaggcaa tgaaaggaag agaagaagtc atgagggatg 240
   gagaccagaa acacagagag aggtaaggga gtgggggaag ttgagacagt tggcaggaca 300
   gcgcgtccac acccatgcac accaccaaga ttaaccccat aatcctctga tcttgtggaa 360
   gctcatttgt agtcttctag cccaaagaaa taaccgtttt tctgaaatgt taagcatcct 420
   tccaagggta tcagagtgat atggagaaat atggtaagac agttacagaa tcagagataa 480
   cgacactgat tctaaaagca ttattaccaa ggcattgttg cattaagact gttttttctt 540
   ttcttttaga gacagtatcc tgctacgttg cccaggcatg tctcagaccc ctgggtttag 600
   tgatcctcct gcctcagcct ccccaaatgc tgggattaca ggcatgagcc gggctgtttt 660
   taattctaag taatttataa tttacaatta cacctaacaa tggcttcaac aaggggtgtc 720
   caatcttttg ccttccctgg gccacactgg aagaagaaga attgtcttgg gccacacata 780
   aaatacacta acactaacga tacctgatga gctaaaaaaa aaaaaaaaaa aaaaaatccc 840
   ccaaaaatct gataatgttt taagaaagtt tatgaatttg tgttgggctg cattcaaagc 900
   catcttaggc catgggctgg acaagcttag cttagaacca agacgaaatt tatggtttac 960
   tttacaagaa ttttgcaaga gggtaatact aggttaggtt atatctggaa aatatcatca 1020
   agaactacat tcattttaat cttttctctt ttttatccct aatatggtga ctttttttcc 1080
   tggggattgc cacttcatgg ctccaagaaa gctaccacag ctcctgactt cacattacag 1140
   caggggtccc caacctccag gccatagacc aatacacaga ccgataccgg tctgtggcct 1200
   gttaggaagt gggctgcaca gcaggtgact ggcaggtgag caagagaagc ttcatctgta 1260
   tttatagcca ctccccattg ctggcattac tgcctgagct ctgcctcctg tcagattagt 1320
   ggcggcatta gattctcata acagtgtgaa ccctactgtg aactgtgcat gccagggatc 1380
   tgggttgtgt gctccttatg agaatctaat gcctgatgat ctgtcactgt ctcccatcac 1440
   tcccagatgg gactgtctag ttgcgggaaa gcaagctcag ggctcccacg gattctacat 1500
   cattgtgagt tgtataatta tttcattgtg tattacaatg taataataat aatagaaata 1560
   aagtctacaa taaatgcaat gtgcttaaat catcctgaaa ccatctaccc cgccacccca 1620
   tggagaaatt gttttttatg aaactggtcc ctggtgccaa aaatgttggg gactgctaca 1680
   ctccagcatc ccacaccagg aggatgggga tgagatggag aatcactgaa gatctttaat 1740
   cagaagggtg atatgttcat ttgtacttta taaacatttg tgtttgtatt ttataaacat 1800
   tttcttggga tcataacaac agatgcattt tagaagattc ccaaggtatg aagggtaaca 1860
   aatgaagacg tgcctaggag tcacatggaa gccaacactt tgtaagggga ttaaaataaa 1920
   gggaatggca gaggacatca ttatgcacag aggagaaggg cccaaatgtc aagagaaaac 1980
   aagagacaag ggactccaca gagagtaatt gccagaaaaa tttgtaggtc ttttgaagtc 2040
   tgactgttct tcctaaaacg ggggtcctgg gacaccccta cctgtttccc atgagccctc 2100
   ttttctaaaa cataagtagg ttttcttcct ggctaccaaa tggcctgtga ttagcacact 2160
   aggttgggca aaaatagagc caagtgtgtg ttccaaagtg actatggtga agaaaaaagc 2220
   gaaagtctta aaatggactt ccacttctgt gtttgcgtaa ggctaaaagg gaagatgtct 2280
   tagggttgac cggaatggga gtattcctgc ctatgctgca aaagtccctg actcttctgg 2340
   taactgaaac atgagaaatg gcctcggcga gcagcacagc ctcccagtaa agcactggac 2400
   tgaggtggta aagccagatt gcctggacta gaatcccagg actactgttc actagctccg 2460
   gacaagccag ctaatccagg ggtctcaaac atttccccca actttttttt tttgagacag 2520
   agtctcactg tgtcacccag gctggagtac ggtggtgtga tcttggctca ctgcagtttt 2580
   gcctcctagg ttcaagtgat tcccctgcct cagcctctcc agtagctggg attacaggtg 2640
   cccgccacca cgcctggcta atttttgtat tttttaagta gagatatggt tttgccacgt 2700
   tgaccaggct ggtcttgaac tcctgacctc aaacaatcac ttgccttggc ctcccaaagt 2760
   gctgggatta caggacggaa ccactgcact gggccacaaa catttcctat gaagagccag 2820
   atggtcaata tatttcattt ggcaggccat agggtctcca tcacaactcc ttggctctgc 2880
   tgttgtagcc tgaaaacagc cacaagcaaa gttgtaaatg aggagcatga ctgtatgtca 2940
   ataaaacttt atttaaaaca cgacaattgg acttcatata agttttgcat ggcacaaaat 3000
   acccttcatt tgattttctt tagccggtaa gaaatattaa agccattctt ggctctcagg 3060
   ccatacaaaa acaagcggtg gctagatttg acccctgatt taactgcact gggtcccaga 3120
   cacttcacct gtgaattgag agcaatgata cttacagagg tgttttctgg agcagaacaa 3180
   ataagatcag tgatacaaag cacttggcat gaattccagt gcaataagta ttatcaatat 3240
   cttagtaatt acataaacct ctctatctgc tgtttcacgt tctctgcggt gaacccatcc 3300
   atgcttaaaa cttgaaccac acctcagcta acattcacca gacccctgtc tctagctcag 3360
   acctgccctt gatgttcaga cacatcattt tgggttcctg tttcctttca aactcatctg 3420
   cagcctttgc tctcctcacc ttctcccaca actctgttaa ggactaaatt gtgtcacatt 3480
   ccccttcaaa ttcttaggtt gaagccctca ccccatgata tggtttggct gtgtcccaac 3540
   cctgatctca tcttgaattg tagctcccat aattcccacg tgttgtggga ggaaccaggt 3600
   gggagatagt tgtttcatgg ggacgatttc ccccataatg ttctcatggt agtgaatatg 3660
   tctcacgaga tctgacgatt ttatgagggg tttccccttt cacttggttc tcattctctt 3720
   ttgcctgctg ccatgtaaga tgtccctttg ttcttccttc gccttccgca atgattgcga 3780
   ggcctcccca gccacatgga actgtgagtc cattaaacct cttcccttta taaattaccc 3840
   agtcttgggt atgtcattat tagcagcatg agaattgacc aatacacccc agcatgtcag 3900
   aatgtgactg tatttgggga tacaaccttt aaagaggtaa ttagctgaaa ctgaggtcat 3960
   tagggtgggc cccaactcaa tatgactgat atccttatga aaagaggaga tccagacaca 4020
   gacaggcata gagggaaaac tgtgtgaaaa catgaggagg aggcggtatc tgcaagccaa 4080
   ggagagagga ctcaggagga atcaaccctg ctgacacctt gatcttaaac ttccagctga 4140
   taaaactgtg agaaaatata tttctattgt tgtagccacc cagccaatgg tattttgtta 4200
   tggcagctct aacaaactaa acactctgct tcttttcatt gactctcgtc tcaatgacag 4260
   cccccaaatc cacgcagtta ccttggtgca acagttctga agaaaatggc cacaatattc 4320
   ttggacactc cttccactga caggaaggca ggcatattgt atcgcctctc ccctggaacc 4380
   tgggtagggt tgggactact ttgaccaaag aacgtggtag aaatgacttc cacgtagctt 4440
   tcaaggctag gtcaaaaaag gctaggaagc atcttctggt tctcctggga cacgtgttct 4500
   ggaggaaggc agccattgtc ctgggacttt gattcctctg agaccaccat gctggaaagg 4560
   ctgcctgtag gcacttggtg caataccagc ctagttcagc atttgagtca ttccaaccca 4620
   ggcatctgac atgtgaatga aggagctcta gatgactcca attcacagtc ttctagtcac 4680
   cccccagacc tttgagtttt gggtggcaga gacaagcatc tctgtgcctt atcctaattc 4740
   ccaacccaca taatccatga acgtaatgaa atggttgttt tacatgctat gatttgggtg 4800
   gttatacagc aattatgaat tatgaatgtg ggcttcataa agacccttca tgcttcctca 4860
   ttctccagag ccatttggtc tccaagctca gtctaccaag cctggaaatc tctccctaaa 4920
   ctacacctcc tctctagcac actactccag cacaggcaat tattcgacaa gttcaccttg 4980
   atctctggca acttcttacc ctttaagatc tctgcctcca actgggagca ttctcagagc 5040
   catccctttg cagggggact ctagagcaat gttttctgga agcacacacc tgacaagtca 5100
   ctctcctgct taaatagctt ttaagcagaa gaaattgtaa gaaagaaatt tttttttaga 5160
   ccaatatcct tgatgaacat tgctgcaaaa atcctcaata aaatactgac aaaccaaatc 5220
   cagcagcaca tcaaaaagct tatccaccac gaccaagtgg gcttcatccc tgggatgcaa 5280
   ggctggttca atatacgcaa atcaataaat gtaatccagc atataaacag aaccaaagac 5340
   aaaaaccaca tgattatctc aatagatgca gaaaaggcct ttgacaaaat tcaacaaccc 5400
   ttcatgctaa aaactctcaa taaattgggt attgatggga cgtatctcaa aataataaga 5460
   gctatctatg acaaacccac agccaatatc atactgaatg ggcaaaaact agaagcattc 5520
   cctttgaaaa ctggcacaag acagggatgt cctctctcac cactcctatt caacatagtg 5580
   ttagaagttc tggccagggc aattaggcag gagaaggaaa taaagggtat tcaattagga 5640
   aaagaggaag tcaaattgtc cctgtctgca gatgacatga tggtatatct agaaaacccc 5700
   attgtctcag cccaaaatct ccttaagctg ataagcaact tcagcaaagt ctcaggatac 5760
   aaaatcaatg tacaaaaatc acaagcattc ttatacacca ataacagaca aacagagagc 5820
   caaatcatga gtgaactctc attcacaatt gcttcaaaga gaataaaata cttaggaatc 5880
   caacttacaa gggacgtgaa ggacctcttc aaggagaact acaaaccact gctcaatgaa 5940
   ataaaagagg acacaaacaa atggaagaac attccatgct catgggtagg aagaatcaat 6000
   atcgtgaaaa tggccatact gcccaaggta atttacagat tcactgccat ccccatcaag 6060
   ctaccaatga ctttcttcac agaattggaa aaaactactt taaagttcat atggaaccaa 6120
   aaaagagccc gcattgccaa gtcaatccta agccaaaaga acaaagctgg aggcatcacg 6180
   ctacctgact tcaaactata ctacaaggct acagtaacca aaacagcatg gtactggtac 6240
   caaaacagag atatagatca atggaacaga acagagccct cagaaataac gccgcatatc 6300
   tacaactatc tgatctttga caaacctgag aaaaacaagc aatggggaaa ggattcccta 6360
   tttaataaat ggtgctggga aaactggcta gccatatgta gaaagctgaa actggatccc 6420
   ttccttacac cttatacaaa aattaattca agatggatta aagacttaaa tgttagacct 6480
   aaaaccataa aaaccctaga agaaaaccta ggcattacca ttcaggacat aggcatgggc 6540
   aaggacttca tgtctaaaac accaaaagca atggcaacaa aagccaaaat tgacaaatgg 6600
   gatctaatta aactaaagag cttctgcaca gcaaaagaaa ctaccatcag agtgaacagg 6660
   caacctacaa aatgggaaaa agttttcaca acctactcat ctgacaaacg gctaatatcc 6720
   agaatctaca atgaactcaa acaaatttac aagaaaaaaa caaaaaactc tatcaaaaag 6780
   tgggcaaagg atatgaacag acacttctca aaagaagaca tttatgcagc caaaagacac 6840
   atgaaaaaat gctcatcatc actggccatc agagaaatgc aaatcaaaac cacaatgaga 6900
   taccatctca caccagttag aatggcaatc attaaaaagt caggaaaaaa caggtgctgg 6960
   aaaggggaga aataggaaca cttttacact gttggtggga ctgtaaacta gttcaaccat 7020
   tgtggaagtc agtgtggtga ttcctcaggg atctagaact agaaatacca tttgacccag 7080
   ccatcccatt actgggtata tacccaaagg actataaatc atgctgctgt aaagacacat 7140
   gcacacgtat gtttattgca gcactattca caatagcaaa gacttggaac caacccaaat 7200
   gtccaacaat gatagactgg attaagaaaa tgtggcacat atacaccatg gaatactatg 7260
   cagccataaa aaatgatgag ttcatgtcct ttgtagggac atggatgaaa ttggaaatca 7320
   tcattctcag taaactatca caaggacaaa aaaccaaaca ccgcatgttc tcactcatag 7380
   atgggaattg aacaatgagg aaacatggac acaggaaggg gaacatcaca ctctggggac 7440
   tgttgtgggg tgggggaggg ggggagggat agcattggga gatataccta atgctaaatg 7500
   acgagttaat gggtgcagca caccagcatg gcacatgtat acatatgtaa ctaacctgca 7560
   cattgtgcac atgtacccta aaacttaaag tataataata aaaatatata tatatataag 7620
   aaagaaattt taagaagaaa ttctaagttc tttttacagc attcaagacc aaccaagaaa 7680
   ttgcctctgc cgccctctac atacttaatg cttattgctg tctgccttat catctatgct 7740
   ctggtaaaca aaagtaacct gcttttttgt gacccctttc gtgggtattt ttactcccct 7800
   ctgacaagaa tgcattattt tttctccttt atctgagtct ttaagactca gcctacatgt 7860
   tccctcctcc ggatattgac tctagatccg tgaatctgag ttagtggttc cttttagagg 7920
   acctcacaag gagccaggca tctgtctatc actacgtgcc cccaccctat tgtaactaag 7980
   cactgcattc tcacctctct attcaggtgg tccgcagagc ccatgtctga ttgatctcta 8040
   tgtctccagc agccagcaag gaagcacctc tttagagacc tgcacctata caatacctac 8100
   caccttttat ttctcgatat gtgaactcca ttgagaacaa acgagtaaat gtaggtaatg 8160
   tgccttcttc ttttcttttc ttttcttttt ttttttagat ggagtctcgc tctgttgccc 8220
   aggctggagt ttagtggcac aatctcggct cactgtaacc tccgcctccc aggttcaagc 8280
   gattctgccg cctcagccta ccaagtagtt gggattacag gtgcccacca ccacgcccag 8340
   ctaatttttt ttgtattttt agtagaggtg gggtttcatg atgttggcta ggatggtttt 8400
   caactcctga cctcaagtga tccacccacc tcggcctccc aaagtgctag gattacaggt 8460
   gtgagccaca gcgcccagcc agtaatgtgc cttcttaagt tctgtgagcc attctaacaa 8520
   attatcagaa cagaggaagg ggttataaac atccccccac ccccgattta tagccagtca 8580
   gtcagaagta caggtggcca cctgggactt ggattggtgt ctgaagtgag gacagttttg 8640
   ggagagtgag ccctttaact tgtgggatct gacactaact ccaggtagac agcgtcggag 8700
   ctgaattgaa ttgtgagata cccagtggtg tccccagaga actggagaat tgcttgatat 8760
   ggaaaagacc cacacatttg atgccagaag tactgcataa gtcgagaatt gagtttgact 8820
   taatcatcat atttgggctt gattgcgtgg ctgaaactct tcccacttca gtaattgttt 8880
   ctttcatttt catgaaactc tgaagaagga agggctggac attcagattc cttgaccctt 8940
   gacatttgga agcatgaact ccagtctctc acagaaggct agaggtgaag gaacattcag 9000
   acacattggt ttctaagaag agtccgctga caacataccc aaggtgtctt ctgaaaatta 9060
   taagaaatcc tgagtttctg ttaggggatt ggctccagct ccattgtccc tcccccatca 9120
   ttcagtagtc tccgcgaaag cccttagagc cggtgttgct ccacaggaag ccaagaagca 9180
   cacaggaaaa ggagcttagc tgctggtaag ttggggactg acaatttaaa atttatcaat 9240
   ggaactcctg ctctctgaca aacacctcct aaattctaga gcagtgctgt aatggagcat 9300
   ccacttgcca caggtggctt ttgagtgcct atgtttctat cttgatctga aatgtgctta 9360
   agtgtaaaat atacttagtg tagtttaatc attagtatga tcaaaataat acaatttatc 9420
   tcgttaataa ctttcatttt ggtaacgtgt tccaatgatt ttttttattt gcggggttaa 9480
   atgaaacata ttattataat taatttctcc caatgatttt acttttttaa catggctact 9540
   agaaagttgt aagtcacgtg tgtggctggc atttagggct tgcattaccc tgtctctact 9600
   aaaaatgcaa acaacaaact agccaggcgt agtggtggga gcctgtaatc ccagctactc 9660
   aggaggctga ggcaggagaa tcgcttgaac ctaggaggcc gaggttgcag tgagccgata 9720
   tcgtgccatt gcactccagc ctgggcaaga agagtgaaac tctaactcaa agataaataa 9780
   acatgtgaag gagccaagtg tgaggagctt aagaggaaga gcatggtagg cagaaggaac 9840
   agagaaggca gaagccccga gatgacaatg agctgcatgt tcaaggaatc agagcccgtt 9900
   atgggtggag cccagtgtgt cccacgagag tgcagagatg aggtctgagg aatgcacagg 9960
   gcaggtccag gggtacttga gggaacttaa gataaggata agttagaagt ttggaagcca 10020
   ttagaaggtg ttgaaaatag catcaccatt ttattggacc tttttttttt ttttttttga 10080
   gacagaatct tgctctgtcg cccaggctga agtgcagtgg cgcagtctcg gctcactgca 10140
   aactctgcct ctcagggtca agcgattctc ctgcctcagc ctcccaagta gctgggatta 10200
   caggcacccg ccatcacgcc cagctaattt ttgtgttttt ttagtagaga cggggtttca 10260
   tcatgttggg caggttggtc ttgaactcct gacctcaggt gatccaccca cctcagcctc 10320
   ccaaagtgct gggattacag gcatgagcca ctcccggccc ttattggact tttttttttt 10380
   ttttgagaca gagttttgct cttgttgccc aggctggagt gcaatggcac gatctcagct 10440
   cactgcaacc tccgcctccc tggttcaagc gattctcctg cgtcagcctc ccgagcatct 10500
   ggaattacag gcacgtgcca ccacgcccag ctaattttgt atttttagta gagatggggt 10560
   ttcttcatgt tggccaggtt ggtctcgaac tcctgacctc tggtgaccca cctgcctcgg 10620
   cctcccaaag tgctgggatt acaggtgtga gccactgcac ctggccctta ttgaactttt 10680
   taaaagctca tttggtttcc ttctggagat tggattgtag ggcaggggaa agtaggacca 10740
   ggaacaacct atttgcaaag ttggcgcaaa cattcctgcc tgacaggacc atggacacag 10800
   gttgtagaga tagagatggc tctggctgtg cattcagcag attctgtaga tagaattaat 10860
   aggacttgga tgggattgtg gtgagagaaa gtgaaatgaa agataagttc tagtttggaa 10920
   gttttaacaa ctgaatgttt aaactcaaat agacacaaaa tattggaaga gtggcaggtt 10980
   tgggaggatg agacaatcaa ctgtttggtt gagccacgtt aggtttgaaa tgtctacggg 11040
   actcccgtgg ggagaggtta tatcagactg gagcaccaga gagaggccaa ggctgatagt 11100
   ttagatgaaa agagagcatg atattttaag ccctgagact ggataatatc acctatagaa 11160
   agactatata gagataagag aggtggggaa caagtaaaag ctgcgggaca ctcctaaatt 11220
   tagagtcaaa tttagagcag aaaatactag caaaggggac tgaaaagcgg tggccaattg 11280
   agcttcaaat gcaagtgaaa gtgtgttgtg tgtacattta tcatctcatg gcacaggaaa 11340
   aacgtgattt aaggagaagg aagcgatcca atgggaagaa gagatccaat ggatcctcta 11400
   tcacgaagat attgagataa gaaccaatat ggatttgcac ccactgcatt tgcagccttg 11460
   aggtcataag catcctcagg aaaatgcacc aggtcagtgc taaggactgg agtcaataca 11520
   gactggattc aggagaaaaa gtgaggcagg gaatcaaagg gagcaaacac agaaaactcc 11580
   ttccaaaatc tggctgcaaa ttcttttcat agaaagaatt ctctcaggag aaatagggtt 11640
   aacaccagaa aggaatatag tccaagatct ttttggttgt tttttgtttt gttttttggg 11700
   gtggttttta ttttgttttt gtttagtttt gttgttgttt gtttgtttgt ttttgagacg 11760
   gagtcactgt tgcccaggct ggagtgcagt ggtgcgatct cagctcactg caacctccgc 11820
   cttccggtgt tcaagaggtc ttcctgcctc agcttcccaa gtagctggga ttgcaagcgc 11880
   gtgccatcac gtccagctaa tttttgtatt tttagtagag atgaggtttc accatgttgg 11940
   ccagtctggt ctcaaactcc tggcctcaag tgatccactt gcctcagctt ccctaaatgt 12000
   tgggattaca ggtgtgagcc acggcacctg gccaaggatg gtttcttttt aaaggagggg 12060
   gatatgattt atgtaatgtt tatgtaatag tgagaatgat ctagtagaaa taaaatcttg 12120
   agggtttgtt tttggcagaa aatggaagga caaatgcagg gggaaagttt gtgggaagat 12180
   gagagggcat cctgggccaa tgcagggagg gaaattattg gaaagaacaa gaagtcttta 12240
   ttcacagtga cagaggcagg tagactgagg gtcaggggag gaccggctga aaatttcaca 12300
   tctagttatt accattttct cattataatg tcagtcaagg gcatctgctg agagtgggaa 12360
   aaacagagta aatgaggtac tttttggaga aagcaggact gaaataatta aggtgtggga 12420
   tagcgagtgc gctggagaag tagagtaacc taatgaggaa ctgctgagtt ttcatttgaa 12480
   atctgtggtc ataaattcaa ttgcaaccct cgataattgt gaaagtcact tccgctctct 12540
   gagccgtact ttcctacctg ttacacaagg acagcacttg tcccaaagca agggtattgt 12600
   aatcagagat gataaaatat atagtgactg gcacatatta aggactcacc aaatagcaga 12660
   cctcataatc agtacaggta tcatctgtgg ttcgcagttc tcctccttac attacaacta 12720
   gggtggaccc aaaactttgc aatttttgcc ttgttaattt gtcctcacct gtcattgcac 12780
   aatcatcctt ccttgttgca tcaacatttt tttttttctg agacagagtc tcgctctttc 12840
   acccaggctg gagtgctgtg gcacgatctt ggctcactgc aacctctgcc tcccagactc 12900
   aagtgattct cctgcctcag cctcccaagt aactgggatt acaggcaccc accaccatgc 12960
   ccagctaatt tttgtatttt tagtagagac agggtttcac catgttggcc aggctggttt 13020
   caaattcctg gcctcaagtg atccacctgt cttggcctcc caaagtgcta ggattacagg 13080
   cgtgagccac tgcgcccagc ctgtatcaac attttaaaat gaatttgaaa ttatggatag 13140
   atcatgtctt ctaagtgtct tgtagaatgt ttggagtgtg gatagagaaa atctgagtct 13200
   gttgaggaac tatggggtgg aaaaggttta acagcacaat tctgttgtaa tcaatctggc 13260
   tgagctccaa ttctgtctct actctttcta cctggtaacg ttgaacaatt aacgcagccc 13320
   atctaagtct taacttcctc atctgtacaa tggttatact aattatcagg ctgctgcaaa 13380
   agtacttgtg gtttttgata ttattttcaa tggcaaaaac agcgttactt ttgcaccaac 13440
   ctaatatcac taagattaat caatattcac tgtgtctcag gagtgctcag tgttcacaca 13500
   tattaacaca gcccatccac agatcctgtg agaggcgttg atagggttaa aggaaaagct 13560
   cttggtgtgt gggtaatgtt cataagtaga aacaactatt gctatgagca tcgttcttgt 13620
   cctgaccttg aacttcataa tggattgagc cccacgacac agcacacagc ctggtgaggg 13680
   ccaagagttc ccatttgtca tagatatact cagtctagcc taaaagattt ccacatctat 13740
   tctcacttaa tcctcacctg aaagttagac ttcaccataa aattgtcctc gttctgcaga 13800
   gcagaaaact gaggctcaaa gagatatcac tgactaaaaa ctcatagtga ggagacatca 13860
   cagatgagat tagaagagag atgtagctga cttcagagcc cagtatatat atatatttta 13920
   gacagagtct cgctctgtcc cccaggctgg agtgcagtgg cacggctcac tgcaacctcc 13980
   gcctcccggg ttcaagcgat tctcctgcct cagcctcccg agtagctggg actgcaagtg 14040
   catgccacca ggcccggcta attttttgta ttttcggtag agacggggct tcaccgtatt 14100
   gcccagggtg gtctggaact cctgacctca ggcaatccac cccccttggc ctcccaaagt 14160
   gttgggatta caggcgtgag ccaccgtatc tggctgagcc cagtattctt tgtaacagtg 14220
   actctcaatt gggggtgact ttgcctccga ggggaccttt gacaatgttt atggaacctg 14280
   ttgattgtca tttctggaga aggagttgct actggtgtct gtggatagag gccagggggg 14340
   ctgctcaaca tcctactgtg tccagaacag tctgccacaa cgaagaatta cccagaccca 14400
   gatgtcaaag gtgccaaggt tgagaaaccc tgagctacat taaataacgt cccatggaat 14460
   acctcagtga aactcactgg taatagcagg aagaaaagga ggaaataagg aaaaggaaga 14520
   aggctaaaaa tcaccagact gaggcatagc cctatcctag taatcttggg ggaatccctt 14580
   tcaaaggcag gacttccgta tgcaaaggca gtggctctgg agttgttcat ctcggtttcc 14640
   gtatcttgct gcctccactg actggctggg catgatgttg aaaaaaacta cagaagtccc 14700
   cgggccatga attcccccag atgtgatggc tgtttcccaa tgttattatg agcattagat 14760
   gtgaacaagt gctatggaat tgctatttct cttaaagatg ctgaatagcc cagtctgaga 14820
   tgattctcaa attctcatgg ccatagcaaa tcatcctggc ttttacacat acagattcta 14880
   gaatgagatt tatggatata attgtttttc atttttatta taagttcagg ggtatatgtg 14940
   caggtttgtt atacaggtaa acttgtatcc tggtgttttc ttgtacagat tatttcatca 15000
   ctgagatatt aagcctaata cccactactt atttttcctg atcctgtccc tcccctcacc 15060
   ctccaccttc cggtaggccc cagtgttccc ctctttgtgt ccatgtgttc tcatcattta 15120
   cctcccattt gtaaatgaga acatgtggta tttggttttc tgttcctgca ttagtttgct 15180
   aaggataatg aatacaattt taacaggcag cctggatgtt tctgataaag tccacagacc 15240
   acggcataag aaaatgtgct caggtgacgt gggactttgt gttgaactgg atacaaatat 15300
   tacagatgtg atccggacca gaggcaggga ctacatgttt tgggtttttg ttcttaaaag 15360
   aaaaaaacaa aaacaaaaaa acttgatctg agaagctctg aagggtcata ttcttacaat 15420
   gttattgtcc acaatttagt cacgtgtcca ctaccaaagc aatcccttgc aaagtaaaag 15480
   ggattctggt tagtgctaat tagactagtc aggatttact cccaacatgg agaaaagaca 15540
   ccttctctgc agggtgagta actaaacaca attgtggttt ttcaagcaga gaagaatggg 15600
   gaaccgttgc tgggtgggca accagcaata tctgtcaagc gtaagggttt agaagtcaga 15660
   ccatgccaaa ctgaagtcct gaatctatta tctactacct aagtgacctt caacacattt 15720
   cacaattgcc ctgagctttc atttattcta atgaaaacta gaagtaatga atattacata 15780
   gctattacat aggccaaacc ggatcccata tgtagtaatt aacacaatgc atagtctcta 15840
   ataagaactt cacaagtatt aatacaactt attattctta tagtctttat cccacagaca 15900
   ccaaagctat acaaatcggc actgattatt tgcattccaa acagagaaga gctccacatc 15960
   aagcagggac agggaaaagc agagggtaga aagcagagat ggaactctct ttgctgactt 16020
   cctctagtgc agaatcctca ggatataaca tactactaca tgttaccagt gactcctcaa 16080
   aattctcttc aacaataacc tcaagccaaa ccctcaaact ttatcttgag tactagccaa 16140
   gcaataggtc aagggttctc acagtttgag ggacaggagt tgggagactg gaagaacctg 16200
   gggctgaaag agattgcaga gcttgccagc atgtttgcat tgaatggtgg gagacacaga 16260
   tgtgagcctt ctgattctga gtcatgtgcc ttcctcacaa tgctgaggct gcctatgctc 16320
   aattttccat tgtccataca acataagttc agtatgagcc ttgtaatcct cttggtgaag 16380
   tgatcagccc gataagcgaa atgcatattg ataatgatgg gaatatggag gactatccct 16440
   tcccaaagga gcatccaaga gaatgccaat tctatagcac cgttgtcaac aaggcttggt 16500
   agatagagtg ggtctcctgg taggagtggg agctttagtg gaagttgatg tgagctataa 16560
   atacagatgc tgtaagatgg ttccacagct gagaaatggc cattgttgga atgtttcagg 16620
   tgctgctggc aagatggaaa ccaacttctc cactcctctg aatgaatatg aagaagtgtc 16680
   ctatgagtct gctggctaca ctgttctgcg gatcctccca ttggtggtgc ttggggtcac 16740
   ctttgtcctc ggggtcctgg gcaatgggct tgtgatctgg gtggctggat tccggatgac 16800
   acgcacagtc accaccatct gttacctgaa cctggccctg gctgactttt ctttcacggc 16860
   cacattacca ttcctcattg tctccatggc catgggagaa aaatggcctt ttggctggtt 16920
   cctgtgtaag ttaattcaca tcgtggtgga catcaacctc tttggaagtg tcttcttgat 16980
   tggtttcatt gcactggacc gctgcatttg tgtcctgcat ccagtctggg cccagaacca 17040
   ccgcactgtg agtctggcca tgaaggtgat cgtcggacct tggattcttg ctctagtcct 17100
   taccttgcca gttttcctct ttttgactac agtaactatt ccaaatgggg acacatactg 17160
   tactttcaac tttgcatcct ggggtggcac ccctgaggag aggctgaagg tggccattac 17220
   catgctgaca gccagaggga ttatccggtt tgtcattggc tttagcttgc cgatgtccat 17280
   tgttgccatc tgctatgggc tcattgcagc caagatccac aaaaagggca tgattaaatc 17340
   cagccgtccc ttacgggtcc tcactgctgt ggtggcttct ttcttcatct gttggtttcc 17400
   ctttcaactg gttgcccttc tgggcaccgt ctggctcaaa gagatgttgt tctatggcaa 17460
   gtacaaaatc attgacatcc tggttaaccc aacgagctcc ctggccttct tcaacagctg 17520
   cctcaacccc atgctttacg tctttgtggg ccaagacttc cgagagagac tgatccactc 17580
   cctgcccacc agtctggaga gggccctgtc tgaggactca gccccaacta atgacacggc 17640
   tgccaattct gcttcacctc ctgcagagac tgagttacag gcaatgtgag gatggggtca 17700
   gggatatttt gagttctgtt catcctaccc taatgccagt tccagcttca tctacccttg 17760
   agtcatattg aggcattcaa ggatgcacag ctcaagtatt tattcaggaa aaatgctttt 17820
   gtgtccctga tttggggcta agaaatagac agtcaggcta ctaaaatatt agtgttattt 17880
   tttgtttttt gacttctgcc tataccctgg ggtaagtgga gttgggaaat acaagaagag 17940
   aaagaccagt ggggatttgt aagacttaga tgagatagcg cataataagg ggaagacttt 18000
   aaagtataaa gtaaaatgtt tgctgtaggt tttttatagc tattaaaaaa aatcagatta 18060
   tggaagtttt cttctatttt tagtttgcta agagttttct gtttcttttt cttacatcat 18120
   gagtggactt tgcattttat caaatgcatt ttctacatgt attaagatgg tcatattatt 18180
   cttcttcttt tatgtaaatc attataaata atgttcatta agttctgaat gttaaactac 18240
   tcttgaattc ctggaataaa ccacacttag tcctgatgta ctttaaatat ttatatctca 18300
   caggagttgg ttagaatttc tgtgtttatg tttatatact gttatttcac tttttctact 18360
   atccttgcta agttttcata gaaaataagg aacaaagaga aacttgtaat ggtctctgaa 18420
   aaggaattga gaagtaattc ctctgattct gttttctggt gttatatctt tattaaatat 18480
   tcagaaaaat tcaccagtga a 18501

<210> 38
<211> 14156
<212> DNA
<213> Homo sapiens

<400> 38
ctcttttcta agcttgtctc ttaaaaccca ctggacgttg gcacagtgct gggatgacta 60

tggagaccca aatgtctcag aatgtatgtc ccagaaacct gtggctgctt caaccattga 120

cagttttgct gctgctgggt gagtgagggt cattctgaaa tggggcaatt tcagacactc 180

ctactgcctg gactccaggt accagtgtgg taaggagcag gcctgggccc tggaagcagg 240

ggatagattg aaagaggaga gaggaccctg aattcttaag tgttccaatg gttcctaagg 300

tcagaactca gaggtagttt catagtccct ggaaagtcca ttctgcacca aacattcatg 360

tgtactttgt agtcatgagg aggaacagga aatgagataa aggggacata tgaagtgaat 420

acttttataa aagatcaact ggaaacagga tcttgagatg ggtcctggag aaggaagagc 480

ccaagctcac ctccccagca tttcacatac gcagctaaat ttgaagccgt gttctcctgc 540

tcgacgttga tccactctct tctcttttac agcttctgca gacagtcaag ctggtgagta 600

tgccctttgc ttccttgtat tgacagtgtt gtatcctcat aatatgatgc tgtgtttgct 660

ggggcggcgg gggggtatgt ctattccact gaaaatcaag cttgggttca gcatgggcag 720
ttcccccatt ttagtggggt ctgggatttg cttcttagat ccactgaaga cccaagggaa 780
tgaggccgct gcaagtacag atcttgaact acatctacaa taggactctt gggtgcctga 840
cctcccttgg gagctccttt ggcatccaca gtcccttcag ggttatttat tccacacccc 900
tttccactct gcccctcagc agctccccca aaggctgtgc tgaaacttga gcccccgtgg 960
atcaacgtgc tccaggagga ctctgtgact ctgacatgcc agggggctcg cagccctgag 1020
agcgactcca ttcagtggtt ccacaatggg aatctcattc ccacccacac gcagcccagc 1080
tacaggttca aggccaacaa caatgacagc ggggagtaca cgtgccagac tggccagacc 1140
agcctcagcg accctgtgca tctgactgtg ctttccggtc agtggaggaa ggccccaggg 1200
tggacctggg agggccagga cggatgaaat ctgtttacag acagaggttt gcaggaaaga 1260
gtgggcgtgg actgcttact gggaagcact gttgtgagtt gctcattcat tccccatttc 1320
acccaccctc tttgcttagc atgcgtggtg ggggaagggg gaatttctaa taattttctc 1380
agcatgtgtt aggttgtttt tgcctcagtc ttgattgagc aagggggtta cgaggccaca 1440
aacagctgag tgtgggagaa gcagaaggaa atccctactt taaaaaacta ccctcctcct 1500
gtggcaatgc ggtaaagcag agaggttggg ggaaatagac ctctaaaggc tctaaggcag 1560
gaggctgcca gacaggtgtg tggaggtggg agtgtagaat tcagtctgga agatgaggtc 1620
agaggtgagg ggagggtgaa gggtagatca ggcagaggct gataggctgt tgcaactatt 1680
ttgactttta ttcctgagtg agttgagatg gctttaatta aaggagtcac gtgatctgat 1740
ttagctttat gtaaacatct gacagtgttc agtttaatag agtctgtaga gaaaatgctt 1800
ctagatagta tttctttttt ttttccccca agtagctggg actccaggca cacaccacca 1860
tgccaagtga cttgttttta tttattttta tagagacagg gtctcagtat gttgcccagg 1920
ctggtcttca gctcctggcc tcaagtgatc ttccctcctc aggctctcaa agtattggga 1980
ttataggcgt gagccaccac gcctggctta gatactgttt ctacctgctt ccaatgtctg 2040
aaaatataag cacatttcta ttgttgttta cttgagctgc actcttggtg cataaaccct 2100
ggagatggtc acaggtaaac attcagtgtt ttagattctc ctgttcaaca ctgaaagggt 2160
gatttgtttt tgtggtcctt taaggctgct gctgtaccag agagatcctg gctgtggagt 2220
cgctttctgt tgatggtgct ttactgaagg ggaataaata aatattgagt tgacctcaaa 2280
agctgcctaa tgacatcata gctcctctct gtattggctt cccagagaca gccgatagta 2340
attctgactc ttcttatgaa aatgtaaaaa tatagataat aaaaattgag ggctgggtgt 2400
ggtggctcat gcctgtaatc ccagcacttt gggaggctga ggcaggagga tcacctgagg 2460
ttgggatttc gagaccagcc tgaccaacat ggagaaaccc tgtttctact aaaaatacaa 2520
aattagccgg gcatggtggc gcttgcctgt aatcccagct actcgggaga ctgaggcagg 2580

agaattgctt gaacctggga ggtggaggtt gcggtgagcc aagatcacac cactgcactc 2640

cagcctgggc aacaagagtg aaactctgtc tcaaaaaaaa aaaaaaaaaa aaaaaaaatt 2700

gagatctgaa gcattgttag agagaaacta cctagtccca cctatccatt gtacaaagaa 2760

atgcaactga ttagacccaa gagctcagca gttaatggca cagccaaaat cgcaatcctg 2820

actttagttt ctgttttatg ccatctcagt aatggtacct ggcctttcat atacagaata 2880

cttttttttt ttggccaggc acagtggctc atgcctgtaa tcccagcact ttgggaggac 2940

gaggtggatg gatcacttga ggtcgggagt tcgagaccag cctggccaac atggaaaaac 3000

cccatctcta ctaaaaatac aaaattagcc gggcgtgatg gcgcatgcct gtaatccagc 3060

tactcaggag gctgagggag gaggattgct tgaacttggg aggtggagtt tgcagtgagc 3120

cgagatcgcg ccattccact ccagcctggg caataagagc gaaactccat ctcaaaaaaa 3180

aaaaaaagaa aaagaaaact gtttttttaa atgaattttt atttaaataa gatcgaataa 3240

attatattta ttcctatgat acagcaattt gattttcatc tttattaagt ctattgaaac 3300

tttgttaata gatttgtcct ccgaggtagc tgaattttgg aatctgatta ctctgccatc 3360

agtgaaggga aggaattgtt gggagtgagg atgtttgtag gcaaaaagga aaaagtgatt 3420

ttaaatttaa aatttttctt gaatgcccct tttattcaga gtcacaaatg aatactgaat 3480

tcacctgtgt gaattcaagt catgcctttt tatttcttat gtggatggaa gattttatgc 3540

agggtacagt atggtctgaa tgtttctgtg cccccaaaat tcatatgata tgaattaatc 3600

aaatagcaac ccccagggtg atggtattag gaggtgaggt ctttgggtag gtgattaggt 3660

catgagggca gagcccttat aaaagagggc cgagggagct caaaggcctc ttccgccatg 3720

tgaggacaca gtaagaagcc tcagttctgt gaaccaagaa gtaggctctt atcaaagaag 3780

ctgtaagcac cttgatcttg gactttccag cccccagaac agggagaaat aaatttctgt 3840

tgtttataaa ccacccagtt tatagcattt tgttatagca gccagaacag actaagacag 3900

ggtggatgag ggatgattaa ggctctattt tttttttttt ttaaccagca acagcatgtg 3960

gaaaaaaggg gggaagaagg ctctacttct ttgctaatac agttcaaggc tgcccaccct 4020

cacttcccaa agcttgtaca aaatttacca ttaaattagt aatccccaga ccacaaaaac 4080

tgacggcttc aggccctgtc gagtagcctc tacagttact gaaatgcgct catttctcta 4140

ctcaaacgta gtattttaaa ttcctcagaa ttgttttaaa tatctgctct gcatctttga 4200

aacctctgaa aacttctgct tttagaagtc ccacagatat aataccatat attgcctata 4260

agagaatgct cacatctgtc atgaagcatc ttcatttctg tctgccagac atcatgtcaa 4320

gttctgtgag taacgtacct ctgagactga aaaacccttg gaatctatcc ttacaacttt 4380

ttcttatcat atttgtgtct ttcagaatgg ctggtgctcc agacccctca cctggagttc 4440

caggagggag aaaccatcat gctgaggtgc cacagctgga aggacaagcc tctggtcaag 4500

gtcacattct tccagaatgg aaaatcccag aaattctccc atttggatcc caccttctcc 4560

atcccacaag caaaccacag tcacagtggt gattaccact gcacaggaaa cataggctac 4620

acgctgttct catccaagcc tgtgaccatc actgtccaag gtatggggag tctgccaaga 4680

tgtagggagg ggagaagagg ggatggacaa gggctgaggt cacatgggcc tacatggagg 4740

tctgagaaag cccatagcag caaaattggg cactggagca aagaggagtg gggtggaagc 4800

ctggctaagt attgaccaac aagtaggggc cagagcttgg agccctcacg tcccaggtaa 4860

taggtagtca ggctgttgtt tcactttgaa atgtaggccc cagactaaag atggcagtga 4920

agcagagctc cctcattgat gcagaggttc cctaagctcc tgggcattcc taagagctga 4980

ggtttgcctc gtttcttctc atggctcatg ttatagccat tcactccaga aagcctggca 5040

cgtcatggac tgttcaaggc tgtgctccat agagtaatga tgcctccagc tatgcgaggc 5100

tttgggccca ccctccgtac tgtccccagg ggctaagggg aatccttccc tctgctcctg 5160

catgctcacc agtgtgcttt cattcatttg gtggagaaac ctgggtaggg aggaggcaca 5220

agtccagcca cagaaaccct gtgccagtga ggctggggat gtggtgaatc ttgcattggt 5280

gagtgactca gacacagaag agcttcaggt gacaagcact gggacatagc attggaggtg 5340

ggaggtggga cagggagaat acaaacgttg tcattaaaat agtaaccccc catcctgccc 5400

taatgtctgt cttccctagt gcccagcatg ggcagctctt caccaatggg gatcattgtg 5460

gctgtggtca ttgcgactgc tgtagcagcc attgttgctg ctgtagtggc cttgatctac 5520

tgcaggaaaa agcggatttc aggtttgtag ctcctcccag tcccttttgt tatcagtttc 5580

catttggccc agggcctaac cccagacatt gccagaatcc cgctcttagg gctagatatg 5640

cattccgatc taggcccacc ttttatttat tagttcattt attagttcat cctcaacaag 5700

agcactaata ggagtcacct cacagggttg ccatggggat gaaagaggct gatgctctta 5760

caccagtgcc tggtaggcag taacagcaca caaacagggc tgttgccatt ctggaggagg 5820

tggtatgtga gggcagcgct tagctaggga aggaggactc tccaggcact gggccaaggc 5880

agggactgag gctgggtggg gcttatatga ggggggagta gatagggtac tgcaagcctc 5940

tgagaaagag aaataatccc actaacacct cacaaagcac tttgcaattg gttcatagcc 6000

aacacctcag ttactgatga taagtaaata cagagaaaca gagagagaga gaggcagact 6060

gtgagacaca ggggtgtttt taaaaggcag agcaagcctc agacagatgc catggcgtgg 6120

cctttctgac actcctgggc gtccccatgg gtgagctgaa ttctgcctct ggactagccc 6180

ttttccaggt gggagcagcc cctgagcagg ggaactgggg gtgggaggac aggaaacatc 6240

tgccagaggg aaggcctgag ctggtcccat ccaatcctgg ccctggtcct tggtcctgag 6300

gactcaggcc ccactgccta atcctgctaa cctcctgtgt gtccctccca gctctcccag 6360

gagaccctga gtgcagggaa atgggagaga ccctccctga gaaaccaggt gaatacagag 6420

ttgtctcagg gattcagtga tggctcacca gggctgccgg ctggacctga gccagcgaga 6480

aggggcttgt gcgagttcag ctgggagcca gggagggagc agcgatgggg ggatggccag 6540

ggctcaaatc gcttggtcaa ctctgagtct aatttctggg cctaaagagg acctcgctgg 6600

agatgagaag agaaacacca gtcccagata cagaggagag ggcggtgttc aaatttctag 6660

gaccaggaat ctggtgatat ttccagagca agaaatcaga gatactttgg tctttctgtg 6720

gagctttggc agagctggca gaggatgggg ttaggggctg taagactgag gccaatcgga 6780

cgtgggaggc aggagtctaa ggggaaagga ggaggaggcc tggaaacccc tcaatttgct 6840

gaggaatctg cctctgtgga gggatggggt ggagtggcca ggctcagctg tctgtggaac 6900

actaagagga ggtttgggct tgagaaaatt ctggggggct aggggcacta gtgggctcta 6960

ctctgtgggc aaagctcggt tccaatgccc atgcactcaa ggttgactga attttggcct 7020

ggttctgccc ccatcacaca gtggagccct gagcaagtca gtctccctga tggggttcag 7080

tctcctcact gaggaaatga gtgggtggcc agggtgacct ctcaggcctt ctaggtctga 7140

cattcaggtg ggagtatgta aaggaacttc caggaggatg ccaggcaact tccagactgt 7200

agttaaagtc agagcataaa ggaaaccagt gctttctctg ggggcttcca tgacagtagg 7260

aacaggcagg aaaaaggctg tcaaagaccg cacctcagat ggctggagta tgatttagag 7320

cagagccaac agatttaaga gcttgagcaa aatatcactt gttcagctgt gcttgcagca 7380

aggttgcacg tccactggcc aggcccagtg gtgatagggg cctgtgttca caccagggcc 7440

aaagcagatc tggaaaatac aaagtccaag caactggatt atccgtcttg ttgtgtggtt 7500

ctgtctacac tggcataagc acacttcacg aggcactttt gcagccaggc atgggggctg 7560

cataaatggt tttttaaaaa tcctttcgca aaaggcaaag ctgggttgct ggaattgttg 7620

gggaatgtat tagaatggga attcagaatg tggtttgctg cagaatttac agcatggcaa 7680

taaaggctct ctgtgttcag ggggtgagag aagagactag atgcattgtc tcagatgtct 7740

cccgacttcc tgagaaatgc cactttgcaa atactggggc tacttttcac actggccaag 7800

tcagtttatg acaaaaacat gaggcaatta ggtgtgtttt ggacactaac cagagaaagt 7860

ccttccttcc ctcccttcct ccctccctcc ttccctccct ctctcccttc ctgccttcct 7920

tccttccccc ttccctccct ccctttctcc ctccctaagc tccctccctg cctctctttt 7980

tctctctcat gttactttct ctttctgtct tcctttttaa tgccatggag tactatattc 8040

ttcaaagttc caacaaagat gcactacatt tggacaatgt ggaaatggcc ctcagccttc 8100

tcttcctgat gtggtcagca aaaatctgga ctttacttca ctctgatgct gaacaccctg 8160

ctccacgtct tccccagtat ctcagatccc caagctgtct ggtttgcttc taggaaagct 8220

cagcaattcc ctgaaaagag tgtggcccaa gtgactgctt ccctccacca gtgtggccca 8280

tggggaaggc ttgtggctgg gtgaccccaa gggtgggtac atgaggccat atttacttag 8340

cccttggcct tacaggactg tgtcaagggg tcagcagtcc ttgctttgag tctggttatg 8400

gttttgcagc ctcagcatca gcgtgggcag gcccttttca acagcagtgc cctggctaat 8460

ctttttcttt ttccccacag ccaattccac tgatcctgtg aaggctgccc aatttgaggt 8520

gagtaatccc agccatctcc ttttcctcct gccttgtccc ttctctcctg tttcctctca 8580

tttttgcctt tgttaatgca aaattaaaat ggagactggg cctgaaaact cctgagcaaa 8640

caaagccacc caggccttag aaatagcctt atcattgctt aaactgcaaa cataagtgaa 8700

actcaagttg gattgtaact aaaaataggt aatacttaac ttggatcatt tctggtaaat 8760

gtttatgtta gacagaaata agatttaacc ctagccaatc gtaagcagcc aactaacata 8820

attatgtgac taagaacact tcagtaaggt acctcaccca aaagacaatt atgtaactgc 8880

aaacctatca aatttcttta tttttcttcc acattttcca aattaatact tgtctctgac 8940

tttctgtcat taaaacatta gacctctttt ggtttggtgt cacccaattc atgaattgct 9000

tcttattcaa atgaactctt taaaattgta ttgtgcctta gatttttctt taacagtttg 9060

gtgtcagaag taggatataa agtagaatct cccccaaaga tccctaagag caatgggtga 9120

ccaggcatag gtacccatta agcccattgt gttcactggt ttcttcctgc atttgtgagt 9180

cactaggtaa gtccctctca gatatcaggc ttgcaacttg tgtcctgagc tctctgagtt 9240

tatttgagta attttatttc tcatttgatt cagcagttaa tcttggtatt tgacaagttc 9300

atctgtttcc aacaggaact atactaggtc cagtatagtg tactagtttc cagacaaggt 9360

ctgatttctg gattgggatc cagaggtcaa attgggtact tgacagattg aactaggtgt 9420

tcaacagaaa ccgaactggg ggtccagttg gagtcctcag gtaggtaaat ttcagaaggg 9480

caagatatta tggctttgtc agaatgtaca gagtctggga ctcctccatc tgaaacacag 9540

tgatttctat gttcagataa tatgggccca gaacctgtga ctttctagag aaataggtgt 9600

gccttaccta ttgagcctta ccagagatga cttaaagttg caatggtcac agtgaggatg 9660

tttaaatcta gaaaaaattg ttcatttaag ggatgcattg ggaaaaaatg agatcccaaa 9720

tgtctcagaa acaatgataa ataattttga ttggtatgta aaggtttcta aaagaatgat 9780

ttaaaaaaat agtctctcta agtaaatctt tacaatgtgc taataagaaa gtattctaaa 9840

cctattaacc tctttgattg cttgcttttt ccatctgaac ctactcaatg ctaacgtcct 9900

taaaaaatta gatcctcaaa tgagccagga gtatctacaa caactaaatt taactcttgt 9960

tattgcacac atgaactatt ataaaagcaa ccactaaggc taaaaataat aaaaactaat 10020

tggaaatagg ccagataaac taggtgtctc gttagtcact catttaaaat ttaatccagt 10080

ctcaataaga ttgactccta ataataggtg aaaatgcccc aaaactcctt cttggagaag 10140

cttcgtaccc tttctctggg cctttgagtt gtgtaaatgt ttttaaaaca caagcttcag 10200

caaaatgact catcaaaaag aaaaaaggag gaatttttta gaaacaaact ggcaaatgaa 10260

aaatcttaat agtcctctcc acaaatatca ataaaaagtg ccagccatca gaacagctaa 10320

ctttaactag aggctagatc caactaatta gttttatatt attatacctg gcacatggct 10380

aaaatttcag aacaaaagcc atgaatttct gtttgcatct ttctgtatgt ttacatatat 10440

ctacacatgt gtatgctatt tgtacataat attattctac ctccagatgg tatttattag 10500

aaacagtatt aaaggagctc aactggccta gagaaaaata agcacttata taaattacat 10560

attttctcag aagaaaagaa ctttgccaaa atgcttttca acttcatatg acctgagtaa 10620

tctttaataa atgagaatat tggtattaat gaaagtgaat attccaccta gttttaccca 10680

aataagctca tgttgtctgc tatacaattt gtcaaaaaaa taccttgaga tgacaggtag 10740

ttggttttgt taatattatg tttgccactt taactatgtt aaatgaaatg acaggtattc 10800

attaaatatc tacaccattt ccagataaga caaactacta aaacaaaaat aaaaaagtgt 10860

tccacttatc tttttctctc tacctgattg tttcagaatt tggaaactct tattaagtat 10920

ttttattttc aatctgggta tttgcatagg ttcaataaga atctattctc cttgtaacag 10980

gacataattg ggcaagtcct tggcttggct tcttagccta gagaggcttt taaaggtcta 11040

atctgagatt tctcaattac tagatagttt ttaaaaacta aagttgactg ataaaaagtc 11100

agtaaagtct cttggatatc accaaagctg tggctaaaat atattttaaa attactgttt 11160

ttattatacc tatataaata atcaggtcaa atttaatgaa actagattta tttttgcaaa 11220

aaagttaact ttgattttcg gtaaaaatgg aaaataaaaa tatttttctc cagaataaaa 11280

aagattatat tttagtagaa aactattgtg catccattgt caaattctag gcctgttcat 11340

tttctttgaa gttttattat ctccctataa accagattga ctcctgaatt tttgtttttt 11400

agttccacta aaaactaaaa ctgccctttt ccaaagctct gcaaactgaa actggtgact 11460

ttaagcttca gaaaaatcac tgcaacagag cacttatgcc ttaatgccaa tcttaatgcc 11520

tgctgctatc tgggtcactc agagagttta ccagaatgtt taatgaaata accagagaca 11580

ttcaaattgc aaaccagtaa agcacatcac attgcaactg ctatcttcag tctaccattt 11640

aaacttgcct caaactcacc tctagaaatt gtttcaccta gctgccctct aaactcaaaa 11700

actaagatca taatttgctc caaatattaa ctttgttttt ttaatagaag aatactctga 11760

attcctttca agcaatacaa tctaaaatga gcaagttagc ctcaatagca ccccaaaata 11820

gaagttcttg atatcttaac agcttaacaa ggaggagctt gtgttcctac tgatgtcaaa 11880

agaaatggtt gaagatctca gctggcttta tggtgattct agaactgggc aacacttgcc 11940

tccttaaatt agaataaggt ttccaatgag ttacagcaga ggagattggc ttcatagaca 12000

gaaaaaggtc tgaagaaagc agaaacaaag aatttaaagt ggattggcta ttttaaaact 12060

ggttaaagtt gcaaaagaca ggaataggaa aacagaataa taaataactg gttggttaac 12120

atcaggttac tcttttgtaa ggatgccaat tgaaactggc ctgtttggga aattagattt 12180

ttaggttatt aggttattat ctctctctcc tgatttttcc aaaggccaga taagaatgta 12240

gtttctgttt gatgacttga aactttatca tgggtgattc cactttgatt tttagtctgt 12300

tctgtttggg cctagtgcag gagcttagtg caaaacaata gcctcctaaa atttaaaaga 12360

ctttaaagaa catacatgag tttttcatca gataatattt atttgtattc attaatttat 12420

ttgattggtt aagtcttggc tcccgagaat ctttgctcag aggaattttt caatccttgg 12480

ctattattct ccttatagtt attgtattta cctccccggt gtattgaatt atcctatggg 12540

ttttaaatgc tttcctgcag ccacctggac gtcaaatgat tgccatcaga aagagacaac 12600

ttgaagaaac caacaatgac tatgaaacag ctgacggcgg ctacatgact ctgaacccca 12660

gggcacctac tgacgatgat aaaaacatct acctgactct tcctcccaac gaccatgtca 12720

acagtaataa ctaaagagta acgttatgcc atgtggtcat actctcagct tgctgagtgg 12780

atgacaaaaa gaggggaatt gttaaaggaa aatttaaatg gagactggaa aaatcctgag 12840

caaacaaaac cacctggccc ttagaaatag ctttaacttt gcttaaacta caaacacaag 12900

caaaacttca cggggtcata ctacatacaa gcataagcaa aacttaactt ggatcatttc 12960

tggtaaatgc ttatgttaga aataagacaa ccccagccaa tcacaagcag cctactaaca 13020

tataattagg tgactaggga ctttctaaga agatacctac ccccaaaaaa caattatgta 13080

attgaaaacc aaccgattgc ctttattttg cttccacatt ttcccaataa atacttgcct 13140

gtgacatttt gccactggaa cactaaactt catgaattgc gcctcagatt tttcctttaa 13200

catctttttt ttttttgaca gagtctcaat ctgttaccca ggctggagtg cagtggtgct 13260

atcttggctc actgcaaacc cgcctcccag gtttaagcga ttctcatgcc tcagcctccc 13320

agtagctggg attagaggca tgtgccatca tacccagcta atttttgtat tttttatttt 13380

ttttttttag tagagacagg gtttcgcaat gttggccagg ccgatctcga acttctggcc 13440

tctagcgatc tgcccgcctc ggcctcccaa agtgctggga tgaccagcat cagccccaat 13500

gtccagcctc tttaacatct tctttcctat gccctctctg tggatcccta ctgctggttt 13560

ctgccttctc catgctgaga acaaaatcac ctattcactg cttatgcagt cggaagctcc 13620

agaagaacaa agagcccaat taccagaacc acattaagtc tccattgttt tgccttggga 13680

tttgagaaga gaattagaga ggtgaggatc tggtatttcc tggactaaat tccccttggg 13740

gaagacgaag ggatgctgca gttccaaaag agaaggactc ttccagagtc atctacctga 13800

gtcccaaagc tccctgtcct gaaagccaca gacaatatgg tcccaaatga ctgactgcac 13860

cttctgtgcc tcagccgttc ttgacatcaa gaatcttctg ttccacatcc acacagccaa 13920

tacaattagt caaaccactg ttattaacag atgtagcaac atgagaaacg cttatgttac 13980

aggttacatg agagcaatca tgtaagtcta tatgacttca gaaatgttaa aatagactaa 14040

cctctaacaa caaattaaaa gtgattgttt caaggtgatg caattattga tgacctattt 14100

tatttttcta taatgatcat atattacctt tgtaataaaa cattataacc aaaaca 14156

<210> 39
<211> 15540
<212> DNA
<213> Homo sapiens

<400> 39
agaacatttc tttttcactt cccctttcag actccagaat ttgtttgccc tctagggtag 60

aatccgccaa gctttgagag aaggctgtga ctgctgtgct ctgggcgcca gctcgctcca 120

gggagtgatg ggaatcctgt cattcttacc tgtccttgcc actgagagtg actgggctga 180

ctgcaagtcc ccccagcctt ggggtcatat gcttctgtgg acagctgtgc tattcctggg 240

tgagtcaggg cccccaggag ggagagagga acgggaacct cagttgggga taaccctggg 300

caaggatgag gggtggcatg ggaagaccgt caggacaccc aggggaggga aggcaggctg 360

gtgaagatga aaccctgatg actctgctgg ctttggaaag tcatccaatg tggcagagtt 420

cagggttttt tcagtgtctt tcacccaatc ttactgaaca cccagactct tgatgttctg 480

ttgctttcca catcagaacc cgctctgaga aaagtctagt aacctatact tgaatgataa 540

atgaatggcc agagtgtttg ccttctttga aatttatgct tcacaaaaga ccaaatctta 600

ggcaggaggg ccagtgctgc cttgggtaag gggtagggag gaagttgtaa gatgcatttt 660

tgggaggagg tggcagtggc cattctctgt gcccctgaaa ccactttgtg tagcagaagc 720

ctattgtctt catgtgtaag aatattcccc taggcagaag cacagggaga tctgggttat 780

ccagggaaaa gggactctga gaatacaaaa acctgggtaa gtctggagtc atctgttgtg 840

ggaccagaga aatcatgcat gctgcttttg accacacaga ccatgatggg gaagtactgc 900

atctgatcag caagagagac agggtctttg gccaaaatgg agggctcttt tctaaccatg 960

ttctcagctg ttggataggg gacgacgact gattttgctg agtggtttga aaagcctagg 1020

ggaggccaag taggggagac ttactgccag gactatctct ccaagctcag ttcctggggc 1080

ttccaccctc acattctaga ggaaagcata ttgccactca gccaagttgt gtgatgtgat 1140

tactagtgaa acgtgatcag ctctgggggc cctcggctct tggctgtcaa tgcaggatga 1200

tactgggcta caggagaaag aggtaaggaa gacagagtat tcccccatga ataggatgga 1260

atggattaaa gtagaagaga aagtaaagtg aactacctca tttgtcatgt ggggaagttc 1320

aggcctagag aggtgatgtg cttctctacc acctgggcaa gactcctggg aaagatcttt 1380

ggtatttttt cccatttgcc ttttactaag tcatcaaaga tttaccccat tttttccatt 1440

cttattgggg ctgccacatt tacgtcttca tcacttgata actcaatttt tatagcaccc 1500

tctgatgggg tcttgattga tcctgtctcc cctcccctgc atccatgctg cccaccaagg 1560

ccacggcatc ttcaggtcca gctatggctg tgtcagtgca tttaataaat gccttccgtg 1620

ctgctccatt gcccactgca aaaactctaa ttctgccctg cacactgtgt tctccattgc 1680

accccacatg aatcttcttc tacagccagg cagtatgtac actgcacttt ctgtttttac 1740

cacctttgct cctgctgact ctccaaattc tatgtgcagg cactgttcta ggacttggag 1800

acttatcagt gaacatgact gacagccttc ctgaccggaa atacgaggtc aatgaactta 1860

ttgtgaagca ataagtacac cgggtgggag gatatttggt atttactact atttattaac 1920

tgtggaatgg atacataatt ggtgacttct ctctcctttc ctcatctttt cctttttttt 1980

ctctcgctct tcctcttctt ttttactctc tctcccacta caccgtcccc cttgccaatc 2040

ttacatactc aggtttttag ctaccacttc tatgtcaata actcctaaac ctatttctct 2100

tgcttgcttc tgtcaagtat aattttatat ttccactgct ggttggacac ctctaaaatg 2160

attctccagc tcaaaaacaa agcatattca aaatgcaact tatcatctcc tccttacaaa 2220

tgttctcttc ctcctaacat ttgtatttgc gtaagcgcag ccaccctttt cctagttatc 2280

caacttaaat cacccccaca gtctattaaa tgccaacttc tctctgccac ttaaatttct 2340

tctgcatctg tttcctcttc ttcacctcct attgtcagta caactttggg ctctcttatc 2400

tcccacctag attcaaccag tgaatgattt ccagagcaat ggcacatcaa attaatatgg 2460

ctactgctct ctttgaattt atattctact gagggatata aacggtaagt aaacaaacaa 2520

gaaggcataa gtacaaatgg aaaagggcta tgaagatgta aacggggtac tgtgataaga 2580

ttagttgtgg ggaagagaag agagcagccc actttaaaca ggatgttatg taaagagttt 2640

tctgaggggg tgacatttaa gctgagactt tcaacatggt aaggattcag ccatttggtg 2700

atcaaggacg catttcaggg tagagggatt ggcatacaga aaatccccaa atttgaagaa 2760

tgccaggaag gccagtgtta atgaggctca atgagtaggt gaaaagggat ctgagctgag 2820

gctggagaag gaagcacgga cccaatttga tagggcttgc aggccatttc cattttattt 2880

taagaccaaa gaaaggcaac tgaaggcttg taagttgggg agtgatatca tctgatttcg 2940

tttttttttt ttttttaagg aactagcttc tttatggaaa atggattgta gggtgaccat 3000

aagaagtgag gggacaaatt aagatatggg aaaaagctga tggtgactta aactgagtag 3060

tgtctataaa gggggtgaag agaagttagt gggtgcaaga tctaagttgg aagcaggatc 3120

aacaagactt cctggtgtgg aagggactgg attttgggag acagagaggt taaaagatgg 3180

cttgcaggtt tctggcctat gtagctgggt ggataacgaa gatacatatt gacatgggga 3240

agacggagag aaacagaatg tgtccacttg ctatgacaac atgaagccat tggtgatttt 3300

gacaagaaaa gttttagtgg actgctgagg gccgagtcca aattgcagag aattagacag 3360

tgaatgagag gagaagaaat caggacagcc tatgtaagaa aaacaatgga gaaatttagt 3420

tttgaaaggg atgagacagg ccaggcacag tggctcatgc ctgtaatccc aacacttgga 3480

gaggccgagg tgggtggatc acttaaggtc aggagttcaa gaccagcttg gccaacatgg 3540

tgaaacacca tctctactaa aaatacaaaa attagccgga tgtggtggta atacaaaagt 3600

tagccgggcg tggtggtgca tgcctgtaat cccagctgct tgggaagctg aggcaggaga 3660

attgcttgaa tctgggaggc ggaggtcgta gtgcactgag atggtgccgc tgcactccag 3720

cctgggtgaa gcaaacaaca acaacaacaa aaaccgagag aaatggggca gtagctggaa 3780

ttatatgtgg tgtcaaggaa aagttttttt ttgttttttg tttttaatgg aagatactga 3840

aataaattta ccattgatgg gaagatccag ctaagagaga aactggtgat gcagaggaga 3900

aagtgaataa cagaaaggga aatctttgag aggattaaag ggaatgggat ccagagtact 3960

tgctttgaga ctgaccatag acaggaggag agatgctttc tccagtgaaa tgcgaggaag 4020

gagaagacag gtagagatgc agaaaagact ggtagattta gtcgggggac gatgtgagat 4080

cttctgtgta acctcttttg ttttctctgt gaaatatgag gaaaggcatc agctaaaaat 4140

gagagaggac tggaggcaga gggtgggatg agaaagcttg gggaatgagg aaaagatgag 4200

tgtcactact cagagtagca gagcaaattt cttggaaaaa agtgccacaa tggcagttca 4260

gtgtcctttt gaatgcggtg atcgcacatt tactgcgtca acagtttgcc ttcttgtgtg 4320

gtaatttcaa taacacgcag ctgagcaagt aaaggcatgg cgaatacagg tgcatttggt 4380

agagacagag aaaggcaacg aagttaaggg gatttgcaag gaggtaatta taatgatgcc 4440

tcattgaatt caagctagat gggggagggt gggaggtaaa ggcaaacatg tgctcaagga 4500

taggcagtgg taagatggat gctttggggt tcttgataag gtcaaagatt attctggtgg 4560

ctgtacttga gcagagaaag ttcatctcca aagaaagaaa gtttactgat gatgagcagt 4620

tcagggaatg aagaggccag ttgtttgatg ggttatccac atattctgga tcactacagt 4680

catctctttt tttttctttt ttctttttct ctgacctcat tcttccatca caattagttt 4740

ctttcttaat taagctcttt ggcatttgcc ctgttccaat ctatttgaca ctgcactttc 4800

agccccaatt tgaaaattct aagtgacttc ccactgtata ctgaaccaag gagtgaactt 4860

ataaaaaaaa acttaaattt atttgaaatt caccatgtgt catgtagcac actaaatgcc 4920

ttctctggat tatctcattt aatcctaaca accaccctat gaggtagata ctgctgtcat 4980

tatcttcatt tacaagcaag gaaactgaag cagagaaaag ttaagtaatt tgtgaaaggt 5040

cacttagcag tagcggagtc agcatttaaa ctcagtatgt gctttccccc actgccacac 5100

cacctggcac taggagccct ccagagtatg acttctacct tgctttcctg ctttgtccct 5160

cattattgga agtgcttgag gagagaccag aaatcaagta gaagttgggc tgcaacttgc 5220

tgggaatgtt gccaagtact tgctgctcct tcccatggaa taccttttcc ctctcagcca 5280

aaatcctatc agtcctggaa ggtccttgat gaacctgaaa ggtcccatct tctccatagg 5340

attcccctcc ttatgaaccc caaaccctcc caaacacaga gccctgggat cttttctttc 5400

tctgaacctc catagcattt tatccaaatt cctctaatgt catctctttt acatcagatt 5460

acacatgtgc ttatctccac cgttagattc ttgccttcag tggaagtagg gacttaacgt 5520

tcttttccct taggtgaact cagcatgcat tgcagggcag taggaagcca atgcaggttt 5580

gttgattaat tttggatgca aaggaaggaa gtgattggtc caagtgtata tttcgagata 5640

ttggagcatc gggaccagaa tccagttctg taaactttga agccaacact ttctccacat 5700

tgctcccttt ttacctcttg gttctgattc catttccttc cgtacttttg tccctgaaaa 5760

tatacatgca cttggaagct tcttgacttt ctctcagccc agatgcaaga gaaaaccttc 5820

acccatggac agatggacac acacacacat acacacacac acacacacag agctctgatt 5880

cacctaagga cccagctttg cttctttacc caataacagc acccacaaat ctttatctac 5940

cctagcccac actttgcctc cactacggaa gctcttttct taacccagtg gcatccccct 6000

acccctgcat gaagtcacag atctagtgcc cttttgcagt ctcttcttcc ttttatcaga 6060

tatttctaat agcaaaaatc tggaagacct aaatgccaag caaaagagtt gttaaataaa 6120

tcacaggaca gccaaatagt aggatatagt gtagccattt aaaatgacaa catgggcggg 6180

gaacggtggc tcacacctgt aatcccagaa ttttgggagg ctgaggtggg tagatcactt 6240

gaggtcagga gttccagacc agcctggcca acatggtgaa accccttctc tactaaaaat 6300

acaaaactta gctgggtgtg gtggcgcggg cctgtaatcc cagctcttgg gaggctgagg 6360

caggtgaatc acttgaacct gggaggcgga ggttgcagtg agccaagatc gcgttattgc 6420

actccagcct gggtgacaga gtgagaccct gtccccccac aaaataaaat aaaataaaat 6480

aaaataaaat aaaataaaat aaaataaaat gacaacatag aaacagattc atcttctaga 6540

acttgcattg ctcagtgaag aatcaggtta aagaacagtt tttagagtat atcctcattt 6600

acacttatgt atgtctgtat gtgaacagcg tgtgtctgtg tgtgatcaca gaaagaaatc 6660

caacaggatg tatgccaagg tgataatatt ttgctcctgc gtattttcta tttttctgta 6720

gtgatcatgg attacctgtg caatttctac cccctcccaa caaaagaaca aatcttgctc 6780

tcaggataaa ctctggcaaa attatttata ctgatgcata aatcatatgt aaagcccaac 6840

agagacctct ttccagaggc atatttattt taatcagtca agaggggtaa gccaaaggaa 6900

aatgccacaa aacaggtgga agagggtgtt atatactttt atttcatttt tgtatgttac 6960

tgggagttct cagtcctttt aaaagtgcag ctcatccagg cagtcccagg ggatatggcc 7020

acgagggctg tgctgtaggt gaagggcgct ctgggcctgg gcagtctctg ggccaaagtg 7080

ggcaggtgtc agttcagaca ctccactgtg ggctgatctt tgctttctgt cttacagctc 7140

ctgttgctgg gacacctggt aagtactcca cccacagcct ttccttccct cgggtcccct 7200

tttcctctcc tcccagtgcc ctgtctggag gtgctctaaa tgcaccagca actgagccca 7260

gtaccaagtc agggccagga aagggtaatt gctcccgaaa gatcccctct ccctcttcca 7320

ctctcctcct tccttttcct ccttccacgg aggcaagtgg taggcagcca cggtgttcaa 7380

atgggggagc tgtcacagaa aatgagaact ctgatctctc tctcaactgc cttccgctgt 7440

ccaaataaaa ccaaggcttt cctctctcca aataaaattc aggcacatgc aaaatcacat 7500

ggcctgcata tgttgtcccc ctgtgttgct aaatccttga ctgtgcatgt ttctgagagc 7560

tccaaggcct gttccctgac agagtggtct ctgcatacct ctggctacac tttgccgggc 7620

acttctgtgt ttgtgccact cagcctccac cgctccagga ctggccctcc ggcttttctc 7680

cctctctctt tatccttctg aggccagaga ggctcgtgta tgcttgggtg tgaggacggg 7740

ccatcttgac ctcgttttta aagtatacag aatgacatta ggtcagaacg gaaacctgat 7800

atccaactcc catgcctgcc atctccacca gagcccccac ttatcttcag ccagccccag 7860

gggaaattta tccactagtg tctaaatgcc tttgtttatc ggggagaaaa gttagtctcg 7920

aaagagtatg tatgtatggg gtgtctgtgt gtctgtgctg gtggggagag gttggtgggg 7980

agagagagaa agaactggtg aggatatctc aggtaaagta tatgtatttc gagggtgttt 8040

ctcttgcctg ctccctcctt cttccctgtt gccctgtcgg agtcctgggc tccctggggc 8100

cgttgtggtg ggatgtatga gcaaaagcaa ctgccttcag ttgcagaacc attctgggcc 8160

tggctcggct tttggtgccc ctagtaggcc tacaggtgct tttttgtctg agattcaggg 8220

cctctcaagc tcctgggctt cctcttcttc atgctacctc ctctctctgc ccctcagcag 8280

ctcccccaaa ggctgtgctg aaactcgagc cccagtggat caacgtgctc caggaggact 8340

ctgtgactct gacatgccgg gggactcaca gccctgagag cgactccatt cagtggttcc 8400

acaatgggaa tctcattccc acccacacgc agcccagcta caggttcaag gccaacaaca 8460

atgacagcgg ggagtacacg tgccagactg gccagaccag cctcagcgac cctgtgcatc 8520

tgactgtgct ttctggtcag tggaggaagg ccccaggtgg gacctgggag ggccaggacg 8580

gatgaaatct gctttcaggc agaggtttgc aggaaagggg ggtggcctgc ttactgggaa 8640

gtatcgctgt gagttgcctc agcacatatc agtggttgtt tttgcctcag ttctgattga 8700

acagaagaag gtttcaaggc caaaaacagg cagccaagtg tgagagaagc agaaggaaat 8760

ccctactgca taaaacccat ttccatttta atggcagaat tgaaaagcac agaccacaac 8820

tgaatcctag ccctggaaat gactcactat acaacatgat gaattcattt aacccttgag 8880

tttccatttc ttcacctgct ccgtggggca ctaacgcctc cctcagaggc ttctggtgag 8940

aatcagtgtt tccctgcccc cgccccgccc tccatgcccc ttctccacgt tctcactgtg 9000

ctaggtgctc ttctctgtct ttctcttcca ccagcctgtg ggaaacctga gatgaaagtc 9060

gtgtcttacc catctttgta tttccagcat ctgaaactgg gcagagctta ataaatattt 9120

tgctggagag gttgatgatc ttacaaagct cccattgaaa ggtggctctc tgtaaagcaa 9180

agttacaatg agattgtgat gaacattgtc cttgtggctt ttcacttagt cccctccctt 9240

cacctgaaga gcaaattttc ctcaaaagta cacagcaaac gaatgaccca ctggtgacac 9300

tgctgccttt agaccctgct ggaaagaagc tccacattta ttaacattcc cgaagtaaat 9360

ttatcaggta gcattcatca ggtaacattt gttgcacatt catgactttt ctactgtcca 9420

caaaggcata tgtccttatc atatgcggac tcctcggtca cactggattc ttccttccct 9480

cctcgacatg gaagagatgg catcttaggg tctcttgtgt tcttcctgca gaggcctgtc 9540

gggcaggaaa aggctgcagc tgccttcctg ggagaaggag gagatgagtg tatcctgaac 9600

acctattatg tgctaggggc tattgtagat acatgacact atcatgctca ttttcacgaa 9660

tgaggaaact gaggctcaga agacttaaat tatttgccca agagttcata aatgacagag 9720

ccagcattag agtccaggac tgtctgattt cagacctaag ctgttccctc tgcacatcgt 9780

gtcccaccag taaggaagat ctgggtctca gagctgagcc aagacctccc gggtcctctg 9840

cggttttttg tgtctttcag agtggctggt gctccagacc cctcacctgg agttccagga 9900

gggagaaacc atcgtgctga ggtgccacag ctggaaggac aagcctctgg tcaaggtcac 9960

attcttccag aatggaaaat ccaagaaatt ttcccgttcg gatcccaact tctccatccc 10020

acaagcaaac cacagtcaca gtggtgatta ccactgcaca ggaaacatag gctacacgct 10080

gtactcatcc aagcctgtga ccatcactgt ccaaggtatg cggagtctgc caagatgtaa 10140

ggaggggaga agaggggatg gacaagggct gaggtcacat gggcctacat ggaggtctga 10200

gaaaggccac agcgcaaaat tgggcactgg agcaaagagg agtggtgtgg aggcctggct 10260

aagtattgac caatgagcag gagtaggggc cagagcttgg agccctcagg tgataggtga 10320

ccaggctgtt gttccacttt gaaatgcagg ccccagacta aggacggcag cgaagcagag 10380

ctccctcgtt ggtgcagagg ttccctaagc tcctgggcat tcctaagaac tgaggtttgc 10440

ctttattctt ctcatggctc atgttacagc cattcactcc agaaagcctg gcacgtcatg 10500

gaccgttcaa ggctgtgctc catagagtaa tgatgcctcc agctatgcga ggctttgggc 10560

ccaccccttcc cactgcccct gagggctaag ggagcccctt ccctctgttc ctgcctgctc 10620

acaaatgtac ctttattagt ttggtggaga aacctcggta agcaggaggc ataagtccag 10680

ccacagaaac cctgtgcaga tgaggctggg gatgtagtga gtgctgcaga agtgagtgac 10740

tcagacacag aagagcttcg ggtgacaagc actaggacat agcattggat gggggggagg 10800

tgggacaagg agagtactgc ctgtcctgat gtctgtcttc cctagctccc agctcttcac 10860

cgatggggat cattgtggct gtggtcactg ggattgctgt agcggccatt gttgctgctg 10920

tagtggcctt gatctactgc aggaaaaagc ggatttcagg tttgtagctc ctcccggtcc 10980

ctttggttat cagtttccac ttggcccagg ccctaacccc agacattgcc agaatccctc 11040

tctttgggct agatacacat tcagatctag gcccgtattg tattatagtc attcattcgt 11100

ttattagatc attcatttga caagacttga acaaactagc ttatgtgtca gccaatgcgt 11160

gagacatggt gggtgatgcc aagagaagtc cagtccctgc ccttggggag ttctcagcat 11220

tgtgaggaag acagggtcat tataataaga tgggataaga gcaatgatag ggacatgaaa 11280

agcctaggat agaagtacag aggaggctcc atagcccaac aagggctgag gcagttggag 11340

aaggtcttgg ggagagcagt gtagtgtagt gatggagtgc acatagtggg gccagactgc 11400

ctgagttcaa atctagttct acagcatacc aactatatga cccaagtgag tcactccacc 11460

tctctgtgcc tcagctgtct caagtggaaa atggggacac taataggact tacctcagag 11520

ggttgccatg gggatgaaag aggctgatgc tcttacacag tgcctggtag gcagtaacag 11580

cacacaaaca gggctgttgc cattctggag gaggtggtgt gtgagggcag cgcttagcta 11640

gggaaggagg accctccagg cactgggcca aggcagggat tgaagccggg tggggctcat 11700

atgggtgtcg aagggggagt agagagggta ctgcaagcca ccaagaaaga aagaatcctg 11760

ctaacacctc acaaagcact tttcaattgg ttcatagcca acacctcagt tactgatgat 11820

aagtaaatac agagaaacag agagagagag actgagacac aggggtgttt ttaaaaggca 11880

gagcaagcct cagacagatg ccatggcgtg gactttctga cactcctggg catccccatg 11940

ggtgagctga attctgcctc tggaccagcc cttttccagg cgggagcagc ctctgagcag 12000

gggagctggg ggtgggagga caggaaacat ctgccagagt gaaggcctga gctggtccca 12060

tccaaccctg gccctggtcc ttggtcctga ggactcaggc cccaccgcct aatcctacta 12120

acctcctgtg tgcccctccc agctctccca ggataccctg agtgcaggga aatgggagag 12180

accctccctg agaaaccagg tgagtacagg ttgtctcagg gattcagtga tggctcacca 12240

gggctgccgg ctggactgga gccagggaga aggggcttgt gcaagttcag ctgggagcca 12300

gggagggagc agcagtggga ggatggctgg ggctcaaatc gcttggtcag ctcttagtct 12360

aactcctggg cctaaagacg acctctctgg agatgagaag agaaacacca gtcccagata 12420

cagaagagag ggctgtgtcc gaatttctgg taccaggaat ctggtgatat ttccagagca 12480

agaaatcaga gatactttgg tcttcctgtg gagctttggc agagctggca aaggatgggg 12540

ttaggggctg taagactgag gccaatagga cgtgggaggc aggagtctaa ggggaaagga 12600

ggaggaggcc tggaaacccc ttgatttgct gaggaatctg cctctgtgga gggatggggt 12660

ggagtggcca ggctcagctg tctgtggaac actaggagga ggtttgggct tgagaaaatt 12720

ctgggggact aggggcacta gtgggctcta ctctgtgggc aaaactcagt tctgatgccc 12780

atgcactcaa ggttgactga attttggcct ggttctgccc tcatcacaca gtggagccct 12840

gagcaagtca gtctccctga tggggttcag tctcctcact gaggaaatga gtgggtggcc 12900

agggtgacct ctcaggtctt ctagatttga cattctgaga accaggggcc acccaagccc 12960

tggcccaggt gggagtgtgt aaaggaactt ccaggaggat gccaggcaac ttccagactg 13020

tagttaaagt cagagcacaa aggaaaccag tgctttctct gggggcttcc atgacagtaa 13080

gaacaggcag aaaaaagagg ctgtcaaaga ccccacctca gatggctgga gtatgattca 13140

gagcagagcc aacagattta agagcttgag caaaatatca cttgttcagc tgtgcttgca 13200

gcaaggtcgc atgtctgctg gccaggccca gtggtgatag gggcctgtgt tcacaccagg 13260

gccaaaacag atctggaaaa aacaaagtcc aagcaactgg attatccatc ttgttgtgtg 13320

gttctgtctg cactggcata agcacatttc acaaggcact tttgcagcca ggcattgggg 13380

ctgcataaat ggttttttaa aaatcctttc ccaaaaggca aagctgggtt gctgggattc 13440

ttgggaaatg taatagaatg ggaattcaga atgcggttta ctgcagaatt tacagcatgg 13500

caataaaggc tctctatgtt cagggggtga gagaagagac tagatgcatt gtctcagatg 13560

tctcctgact tcctgagaaa tgccactttg cagatatggg gctgcttttc acactggcta 13620

agtcagttca tggcaaaata tgagggaatt cagtgtgttt cagacactaa ccaaagtcca 13680

tccttccttt ctccttccgt ccctcccttc ctccctttct cactcactct ctctttcttt 13740

ttaatgccat ggagtaccat atttttcaaa gttccaacaa agatgcacta catttagaca 13800

atgtggaaat ggccctcagc cttctctccc tgaagtctgt ggccagcaaa aatctggact 13860

ttacttcact ctgatgctga acacccccct cccccacctc ttccccaata tctcagatcc 13920

caagcctccc tggtttgctt ctaggaaagc tcagcaattc cctgaaaaga atgcagctca 13980

agtgactgct ccactttttc accttggcct ttgcggaagg ctgtggctgg gctaccctat 14040

gcatcgatca atgaggtcat atttacccag tgcttggcct agaggcccaa gacagggtca 14100

gcagtgcttg ctttggctct agtttgggcg ttggttttgc agcctcagca tcagcacagg 14160

caggccctct tctccagcag tgctctggct gatatttctt ctttttccca cagccaatcc 14220

cactaatcct gatgaggctg acaaagttgg ggtgagtgat cccagccatc tccccctccc 14280

ttctcccctg ttgccttttc tgttgccttc tttcctaggc cctctctgtg gatccttact 14340

gctggtttct gccttctcta ggctgagaac acaatcacct attcacttct catgcacccg 14400

gatgctctgg aagagcctga tgaccagaac cgtatttagt ctccattgtc ttgcattggg 14460

atttgagaag aaaatcagag agggaagatc tggtatttcc tggcctaaat tccccttggg 14520

gaggacaggg agatgctgca gttccaaaag agaaggtttc ttccagagtc atctacctga 14580

gtcctgaagc tccctgtcct gaaagccaca gacaatatgg tcccaaataa ccgactgcac 14640

cttctgtgct tcagctcttc ttgacatcaa ggctcttccg ttccacatcc acacagccaa 14700

tccaattaat caaaccactg ttattaacag ataatagcaa cttgggaaat gcttatgtta 14760

caggttacgt gagaacaatc atgtaaatct atatgatttc agaaatgtta aaatagacta 14820

acctctacca gcacattaaa agtgattgtt tctgggtgat aaaattattg atgattttta 14880

ttttctttat ttttctataa agatcatata ttacttttat aataaaacat tataaaaaca 14940

acattctgtt taccttttca aggctgtatt ggttggagtg tagactgaac tgcctggggt 15000

ctgtttctct tcagtgatga gactcttagg aaggcaggaa tggataggat agggggagga 15060

gaggagagat ggggatttag aatgtagagt gagtgcccct tttcttaaaa ctgaatacag 15120

tcacgcacca cataatgatg tttagttcaa caacagactg catatatgat ggtgatccca 15180

taaaattata ataccatatt tctattgtac cttttctatt cctatgttta gatatatgag 15240

tacttaccat tgtgttacaa ttgcctaaag tattcagtac agtagcatgc tgtacaggtt 15300

tgtagcctag gggcaatagg ctatacgcta cagcctaggt gtgtagtagg ccacaccatt 15360

taggtttgta taagtacctg ctatgatgtt cacacaacaa aattgcctgc atttctcaaa 15420

atgtatcccc atatttcaac aatgcatgac tgtactcttc tgccaatgac cttgtattct 15480

tgtttccatg tcttcttctc tttcctccta tggcaaataa aacactgttt tgcaacacaa 15540

## Description

L'invention concerne des marqueurs membranaires et leur utilisation dans le cadre de l'identification de cellules infectées constituants le réservoir viral, notamment des cellules infectées par des rétrovirus.

Les traitements antirétroviraux améliorent considérablement le pronostic vital des patients infectés par le virus de l'immunodéficience humaine (VIH) en altérant notamment la capacité du virus à se multiplier.

Ces traitements ne sont cependant pas suffisamment efficaces pour être arrêtés car ils n'éliminent pas les réservoirs cellulaires du virus et leur interruption conduit quasi systématiquement à une réapparition du virus dans le sang, preuve que l'infection persiste.

La persistance de ces réservoirs viraux latents est donc un obstacle majeur au concept de guérison de l'infection par VIH et est devenue une question majeure dans les domaines cliniques et scientifiques.

Des travaux récents ont montré que les lymphocytes T CD4 quiescents une fois infectés chez les patients, constituent les principaux réservoirs de virus à même de persister sous traitements durant des années [Chun et al., 1997. Proc Natl Acad Sci U S A 94, 13193-13197; Finzi et al., 1997 Science 278, 1295-1300; Chomont et al., 2009 Nat Med 15, 893-900.].

Toutefois, aucun marqueur permettant de différencier les cellules réservoir du VIH des cellules non infectées n'a été identifié à ce jour.

En l'absence de marqueurs permettant d'identifier ces réservoirs de virus, différentes stratégies visant à cibler leur persistance en les « purgeant » du virus qu'ils hébergent ont été testées. Elles n'ont cependant pas permis de reproduire chez les patients, leur efficacité observée en laboratoire. Il est possible que les conditions in vitro dans lesquelles ont été testées ces stratégies soient trop éloignées du « contexte patient ».

Aussi, existe-t-il un besoin d'identifier ces cellules afin d'éradiquer complètement l'infection virale.

L'invention se propose de pallier ces manques de l'état de l'art.

Un des buts de l'invention est de proposer l'utilisation de nouveaux marqueurs permettant d'identifier les cellules réservoirs.

Un autre but de l'invention est de fournir des méthodes d'identification et d'éradication des cellules réservoirs du virus.

L'invention concerne l'utilisation d'un marqueur cellulaire, exprimé à la surface des cellules lymphoïdes, et éventuellement myéloïdes, pour la détection de cellules réservoirs d'au moins un lentivirus, notamment un lentivirus, lesdites cellules réservoirs étant des cellules infectées par ledit lentivirus et étant insensibles aux agents thérapeutiques spécifiques dudit lentivirus. L'invention concerne en outre l'utilisation d'un moyen de détection d'un marqueur cellulaire, exprimé à la surface des cellules lymphoïdes, et éventuellement myéloïdes, pour la détection de cellules réservoirs d'au moins un lentivirus, notamment un lentivirus, lesdites cellules réservoirs étant des cellules infectées par ledit lentivirus et étant insensibles aux agents thérapeutiques spécifiques dudit lentivirus.

L'invention repose sur la constatation surprenante faite par les inventeurs que les marqueurs cellulaires, et notamment certains marqueurs des clusters de différentiations CD, sont spécifiquement exprimés, ou exprimés à un certain niveau par rapport au niveau d'expression desdits marqueurs dans une cellule non infectée par un lentivirus, par les cellules réservoirs d'au moins un lentivirus.

Par une approche décrite dans les exemples ci-après, les inventeurs ont mis en évidence que certains marqueurs CD spécifiques étaient particulièrement intéressants pour détecter les cellules réservoirs de lentivirus.

Dans l'invention, on entend par cellules réservoirs des cellules dans lesquelles des formes virales compétentes pour la réplication s'accumulent et persistent avec une cinétique de renouvellement plus lente que celle des formes virales se répliquant activement.

Les marqueurs particulièrement intéressants sont les suivants : le marqueur CD123 (Gene ID: 3563), le marqueur CD87 (Gene ID: 5329), le marqueur CD89 (Gene ID: 2204), le marqueur CD172a (Gene ID: 140885), le marqueur CD112 (Gene ID: 5819), le marqueur CD213a1 (Gene ID: 3597), le marqueur CD120b (Gene ID: 7133), le marqueur CD121a (Gene ID: 3554), le marqueur CD54 (Gene ID: 3383), le marqueur CD11c (Gene ID: 3687), le marqueur FPRL1 (Gene ID: 2358) et le marqueur CD32 (CD32a Gene ID: 2212 ; CD32b Gene ID: 2213).

Le marqueur ou antigène CD123 est également connu sous le nom de sous-unité alpha du récepteur de l'interleukine-3 humain. Il s'agit d'une glycoprotéine transmembranaire de type I et un membre de la superfamille des récepteurs de cytokines. Le CD123 forme un hétérodimère avec l'antigène CD131 (la sous-unité bêta du récepteur de l'interleukine 3) pour former le récepteur de l'interleukine-3. Au sein du récepteur, c'est l'antigène CD123 qui confère la spécificité vis-à-vis de la cytokine. Par exemple chez l'homme, cet antigène est codé par le gène contenu dans la séquence SEQ ID NO : 27. L'antigène est notamment constitué de la séquence d'acides aminés représentée par la séquence SEQ ID NO : 1, codée elle-même par la molécule d'acide nucléique (cDNA) représentée par la séquence SEQ ID NO : 14.

Le marqueur ou antigène CD87 est également connu comme le récepteur de l'urokinase activateur du plasminogène ou uPAR. Le CD87 est un acteur majeur impliqué dans la migration de l'endothélium angiogénique. Par exemple chez l'homme, cet antigène est codé par le gène contenu dans la séquence SEQ ID NO : 28. L'antigène est notamment constitué de la séquence d'acides aminés représentée par la séquence SEQ ID NO : 2, codée elle-même par la molécule d'acide nucléique (cDNA) représentée par la séquence SEQ ID NO : 15.

Le marqueur CD89 est un récepteur des régions constantes Fc des immunoglobulines de classe A (IgA). Récepteur glycoprotéique transmembranaire, il est exprimé à la surface des cellules de lignée myéloïde, telles que les cellules neutrophiles, les monocytes, les macrophages et les éosinophiles, au sein desquelles il participe à la médiation de la réponse immune contre les pathogènes. Par exemple chez l'homme, cet antigène est codé par le gène contenu dans la séquence SEQ ID NO : 29. L'antigène est notamment constitué de la séquence d'acides aminés représentée par la séquence SEQ ID NO : 3, codée elle-même par la molécule d'acide nucléique (cDNA) représentée par la séquence SEQ ID NO : 16.

L'antigène CD172a ou SIRP α (en anglais Signal regulatory protein a) est une glycoprotéine membranaire majoritairement exprimée par les cellules myéloïdes, les cellules souches et les neurones. Cet antigène agit comme un récepteur inhibiteur qui interagit avec l'antigène CD47 visant à réguler la phagocytose des cellules. Par exemple chez l'homme, cet antigène est codé par le gène contenu dans la séquence SEQ ID NO : 30. L'antigène est notamment constitué de la séquence d'acides aminés représentée par la séquence SEQ ID NO : 4, codée elle-même par la molécule d'acide nucléique (cDNA) représentée par la séquence SEQ ID NO : 17.

L'antigène CD112 est une glycoprotéine membranaire également connu sous le nom de nectine-2. Il est également connu sous les noms de PVRL2 (en anglais Poliovirus receptor-related 2) ou HVEB (en anglais herpesvirus entry mediator B), du fait de son implication dans l'entrée des virus de la polio ou de l'herpès Par exemple chez l'homme, cet antigène est codé par le gène contenu dans la séquence SEQ ID NO : 31. L'antigène est notamment constitué de la séquence d'acides aminés représentée par la séquence SEQ ID NO : 5, codée elle-même par la molécule d'acide nucléique (cDNA) représentée par la séquence SEQ ID NO : 18.

L'antigène CD213a1 est aussi connu comme étant la chaine alpha 1 du récepteur à l'interleukine 13 (IL13RA1). Cette sous-unité du récepteur participe à la transduction du signal par la voie JAK/STAT. Par exemple chez l'homme, cet antigène est codé par le gène contenu dans la séquence SEQ ID NO : 32. L'antigène est notamment constitué de la séquence d'acides aminés représentée par la séquence SEQ ID NO : 6, codée elle-même par la molécule d'acide nucléique (cDNA) représentée par la séquence SEQ ID NO : 19.

L'antigène CD120 correspond au récepteur 2 du facteur nécrosant de tumeurs (en anglais tumor necrosis factor receptor 2 -TNFR2). Par exemple chez l'homme, cet antigène est codé par le gène contenu dans la séquence SEQ ID NO : 33. L'antigène est notamment constitué de la séquence d'acides aminés représentée par la séquence SEQ ID NO : 7, codée elle-même par la molécule d'acide nucléique (cDNA) représentée par la séquence SEQ ID NO : 20.

L'antigène CD121a correspond au récepteur de type1 à l'interleukine 1 (IL1R1) et participe à la réponse immune et inflammatoire liée à l'IL1. Par exemple chez l'homme, cet antigène est codé par le gène contenu dans la séquence SEQ ID NO : 34. L'antigène est notamment constitué de la séquence d'acides aminés représentée par la séquence SEQ ID NO : 8, codée elle-même par la molécule d'acide nucléique (cDNA) représentée par la séquence SEQ ID NO : 21.

L'antigène CD54 est également connu sous le nom de molécule d'adhésion intercellulaire 1 (en anglais Intercellular Adhésion Molecule 1 ICAM-1). ICAM-1 est une glycoprotéine de la surface cellulaire exprimée sur les cellules endothéliales et les cellules du système immunitaire interagissant avec les intégrines. ICAM-1 est également utilisé par les rhinovirus pour leur entrée dans les cellules cibles. Par exemple chez l'homme, cet antigène est codé par le gène contenu dans la séquence SEQ ID NO : 35. L'antigène est notamment constitué de la séquence d'acides aminés représentée par la séquence SEQ ID NO : 9, codée elle-même par la molécule d'acide nucléique (cDNA) représentée par la séquence SEQ ID NO : 22.

L'antigène CD11c est une protéine transmembranaire de type I fortement exprimé sur les cellules dendritiques, mais également exprimé par les monocytes, les macrophages les neutrophiles et certaines cellules lymphocytaires B. Par exemple chez l'homme, cet antigène est codé par le gène contenu dans la séquence SEQ ID NO : 36. L'antigène est notamment constitué de la séquence d'acides aminés représentée par la séquence SEQ ID NO : 10, codée elle-même par la molécule d'acide nucléique (cDNA) représentée par la séquence SEQ ID NO : 23.

L'antigène FPRL1 est également connu sous le nom de N-formyl peptide receptor 2. C'est un récepteur de faible affinité pour les N-formyl-methionyl peptides qui sont des chimioattractants des neutrophiles induisant par la suite leur activation. Par exemple chez l'homme, cet antigène est codé par le gène contenu dans la séquence SEQ ID NO : 37. L'antigène est notamment constitué de la séquence d'acides aminés représentée par la séquence SEQ ID NO : 11, codée elle-même par la molécule d'acide nucléique (cDNA) représentée par la séquence SEQ ID NO : 24.

L'antigène CD32 est un récepteur membranaire des immunoglobulines G exprimé par une grande majorité de cellules lymphocytaires B. Le CD32 participe à la régulation de la production des immunoglobulines lors d'une surproduction. Par exemple chez l'homme, cet antigène est codé par le gène contenu dans la séquence SEQ ID NO : 38 ou SEQ ID NO : 39. L'antigène est notamment constitué de la séquence d'acides aminés représentée par la séquence SEQ ID NO : 12 (CD32a) ou SEQ ID NO : 13 (CD32b), codée elles-mêmes respectivement par la molécule d'acide nucléique (cDNA) représentée par la séquence SEQ ID NO : 25 ou SEQ ID NO : 26.

Dans l'invention, lorsqu'il est fait mention des molécules CD (cluster de différentiation) ces molécules seront indifféremment appelées « marqueur », « antigène » ou « molécules ».

Aussi, l'invention concerne avantageusement l'utilisation d'au moins un des antigènes suivants : le marqueur CD123, le marqueur CD87, le marqueur CD89, le marqueur CD172a, le marqueur CD112, le marqueur CD213a1, le marqueur CD120b, le marqueur CD121a, le marqueur CD54, le marqueur CD11c, le marqueur FPRL1 et le marqueur CD32a ou le marqueur CD32b, ou les deux, ou d'un moyen de détection desdits marqueurs susmentionnés, pour la détection de cellules réservoirs d'un lentivirus, lesdites cellules réservoirs étant telles que définies ci-dessus.

Dans l'invention, les lentivirus infectant les cellules réservoirs recherchées sont avantageusement des lentivirus responsables d'une immunodéficience chez les mammifères notamment les singes, notamment les hominidés, en particulier l'homme, et les félins, notamment le chat (i.e. les virus VIH 1 et 2, VIS et VIF respectivement).

Aussi, l'invention concerne avantageusement l'utilisation d'au moins un antigène choisi parmi les antigènes suivants : le marqueur CD32, notamment CD32a ou CD32b, ou les deux, le marqueur CD87, le marqueur CD89, le marqueur CD172a, le marqueur CD112, le marqueur CD213a1, le marqueur CD120b, le marqueur CD121a, le marqueur CD54, le marqueur CD11c, le marqueur FPRL1 et le marqueur CD123, pour la détection de cellules réservoirs d'un virus de l'immunodéficience chez les mammifères, notamment un lentivirus, lesdites cellules réservoirs étant des cellules infectées par ledit virus de l'immunodéficience chez les mammifères et insensibles aux agents thérapeutiques anti-virus de l'immunodéficience chez les mammifères.

Dans l'invention, il est possible d'utiliser l'un des marqueurs susmentionnés, ou une combinaison des marqueurs susmentionnés.

Il est notamment particulièrement avantageux d'utiliser le marqueur CD89, seul ou en combinaison avec l'un au moins des marqueurs susmentionnés pour la sélection susmentionnée.

Aussi, l'invention concerne avantageusement l'utilisation d'au moins le marqueur de différentiation CD89, notamment l'utilisation du seul marqueur CD89, pour la détection de cellules réservoirs d'un virus de l'immunodéficience chez les mammifères, notamment un lentivirus, lesdites cellules réservoirs étant des cellules infectées par ledit virus de l'immunodéficience chez les mammifères et insensibles aux agents thérapeutiques anti-virus de l'immunodéficience chez les mammifères, ces cellules étant responsables du rebond viral après arrêt de la thérapie, notamment la trithérapie ou multithérapie chez l'homme.

Dans l'invention (ci-dessus et ci-dessous), la référence à CD32 concerne le marqueur CD32a ou le marqueur CD32b ou encore les deux marqueurs CD32a et CD32b.

Dans un mode de réalisation avantageux, l'invention concerne l'utilisation susmentionnée, où ledit marqueur CD89 consiste en la protéine de séquence SEQ ID NO: 3.

Comme démontré dans les exemples ci-après, les inventeurs ont identifié le marqueur CD89 comme étant spécifiquement exprimé à la surface des cellules réservoirs, cellules infectées par les lentivirus notamment responsables d'une immunodéficience chez les mammifères.

Dans l'invention, on entend par « insensibles aux agents thérapeutiques » le fait, notamment pour les cellules infectées par le virus de l'immunodéficience, de ne pas répondre significativement à l'effet d'un traitement médicamenteux. L'insensibilité aux agents thérapeutiques peut se caractériser par une absence complète de réponse, due à une latence virale ou à l'inaccessibilité aux agents thérapeutiques, ce qui signifie que l'effet d'un ou plusieurs médicaments est essentiellement le même que celui d'une composition n'ayant aucun agent thérapeutique (véhicule pharmaceutiquement acceptable, excipient, eau, ou encore un placébo). L'insensibilité peut également se caractériser par une réponse qui n'est pas significativement distincte de celle obtenue avec composition n'ayant aucun agent thérapeutique.

Les cellules réservoirs de l'invention sont dites insensibles aux agents thérapeutiques antiviraux, car, avec les thérapies actuelles utilisées pour l'éradication des virus, ces cellules ne sont ni tuées, ni détruites, ni induites dans un processus d'apoptose ou de nécrose, ni reconnues comme faisant partie du non soi par le système immunitaire. Dans le cas particulier des virus de l'immunodéficience tels que le VIH (VIH1 ou VIH2), le VIS ou le VIF, les cellules réservoirs ne sont pas éliminées par les agents antirétroviraux actuellement utilisés (inhibiteurs d'entrée, inhibiteurs de transcriptase inverse, anti protéases, nucléotides modifiés...)

L'invention concerne en outre une méthode de détection de cellules réservoirs de cellules infectées par au moins un virus de l'immunodéficience chez les mammifères ou des cellules réservoirs de lentivirus, ladite méthode comprenant une étape de mise en contact, notamment *in vitro* ou *ex vivo,* de cellules d'un mammifère infecté par ledit virus de l'immunodéficience chez les mammifères et notamment traités avec une thérapie antirétrovirale spécifique dudit virus de l'immunodéficience chez les mammifères, avec un agent permettant la détection des cellules lymphocytaires exprimant à leur surface au moins un des antigènes suivants : le marqueur CD123, le marqueur CD87, le marqueur CD89, le marqueur CD172a, le marqueur CD112, le marqueur CD213a1, le marqueur CD120b, le marqueur CD121a, le marqueur CD54, le marqueur CD11c, le marqueur FPRL1 et le marqueur CD32.

Par « cellules réservoirs de cellules infectées par au moins un virus » on entend dans l'invention une sous-population de cellules infectée par ledit au moins un virus, cette population faisant partie de la population des cellules infectées. Ces cellules réservoirs étant telles que définies précédemment. « Cellules réservoirs de cellules infectées par au moins un virus » signifie également cellules réservoirs d'au moins un virus.

Dans la méthode susmentionnée, il est proposé de détecter les cellules réservoirs de lentivirus, en mettant en contact des cellules lymphocytaires d'un sujet (humain ou animal) avec un agent capable de mettre en évidence spécifiquement des cellules exprimant l'un au moins des marqueurs susmentionnés. Pour ce faire, les méthodes immunologiques classiques d'isolement des cellules lymphocytaires, telle que la cytométrie de flux bien connue de l'homme du métier, peuvent être utilisées.

L'agent permettant la détection dudit marqueur est avantageusement un ou plusieurs anticorps. Cet anticorps, ou ces anticorps si la détection simultanée de plusieurs marqueurs est recherchée, ou encore si plusieurs anticorps du même marqueur sont utilisés, peuvent être couplés à un des agents permettant la détection du complexe immun entre les anticorps et leurs cibles. Par exemple, les anticorps peuvent être couplés à des molécules luminescentes, fluorescentes, ou à des enzymes catalysant une réaction dont les produits sont visibles par l'expérimentateur. Ces techniques de détection des complexes immuns sont très largement connues de l'état de la technique.

Avantageusement, l'invention concerne une méthode de détection de cellules réservoirs de cellules infectées par au moins un virus de l'immunodéficience, notamment un virus, chez les mammifères, ladite méthode comprenant une étape de mise en contact, notamment in vitro ou ex vivo, de cellules d'un mammifère infectées par ledit virus de l'immunodéficience chez les mammifères et traitées avec une thérapie antirétrovirale spécifique dudit virus de l'immunodéficience chez les mammifères, avec un agent permettant la détection des cellules lymphocytaires exprimant à leur surface au moins le marqueur de différentiation CD89.

Dans un mode de réalisation avantageux, l'invention concerne la méthode susmentionnée, où ledit marqueur CD89 consiste en la protéine de séquence SEQ ID NO: 3.

Avantageusement, l'invention concerne une méthode telle que définie ci-dessus, dans laquelle ledit agent permettant la détection des cellules lymphocytaires ou myéloïdes exprimant à sa surface le marqueur de différentiation CD89 est un anticorps anti-CD89.

Dans un mode de réalisation avantageux, l'invention concerne une méthode telle que définie ci-dessus, dans laquelle ledit virus de l'immunodéficience chez les mammifères est le virus de l'immunodéficience humaine VIH, notamment le virus VIH1 ou le virus VIH2, de l'immunodéficience simienne VIS ou de l'immunodéficience féline VIF.

Dans un mode de réalisation avantageux, l'invention concerne une méthode telle que définie ci-dessus, dans laquelle les cellules lymphocytaires exprimant à leur surface le marqueur de différentiation CD89 sont des cellules lymphocytaires TCD4, notamment des cellules TCD4 quiescentes. Ces lymphocytes quiescents sont caractérisés par le fait qu'ils ne se divisent pas, ont une durée de vie longue (semaine-années), un taux de transcription bas et une activité métabolique qui leur est propre.

L'invention concerne en outre une cellule lymphocytaire exprimant à sa surface l'un au moins des marqueurs suivants : le marqueur CD123, le marqueur CD87, le marqueur CD89, le marqueur CD172a, le marqueur CD112, le marqueur CD213a1, le marqueur CD120b, le marqueur CD121a, le marqueur CD54, le marqueur FPRL1 le marqueur CD11c et le marqueur CD32, ladite cellule comprenant, dans son acide désoxyribonucléique nucléaire, le génome d'au moins un virus de l'immunodéficience chez les mammifères, ledit génome d'au moins un virus de l'immunodéficience de mammifère étant génétiquement modifié, et n'existant pas naturellement dans la nature.

Ces cellules sont nouvelles et n'existent pas à l'état naturel. Ces cellules sont des cellules dans lesquelles un lentivirus modifié a été inséré dans le génome. Ces cellules sont des cellules humaines, de singe ou de chat. Ces cellules sont également isolées.

Avantageusement l'invention concerne en outre une cellule lymphocytaire exprimant à sa surface au moins le marqueur de différentiation CD89, ladite cellule comprenant dans son acide désoxyribonucléique nucléaire le génome d'au moins un virus de l'immunodéficience chez les mammifères, ledit génome d'au moins un virus de l'immunodéficience de mammifère étant génétiquement modifié, et n'existant pas à l'état naturel chez les mammifères sans l'intervention de l'homme.

Dans un mode de réalisation avantageux, l'invention concerne la cellule susmentionnée, où ledit marqueur CD89 consiste en la protéine de séquence SEQ ID NO: 3.

Avantageusement, l'invention concerne une cellule susmentionnée, où ledit génome d'au moins un virus de l'immunodéficience de mammifère génétiquement modifié permet l'expression d'une protéine marqueur fluorescente.

En particulier, les cellules susmentionnées sont caractérisées par le fait qu'il s'agit de cellules lymphocytaires réservoirs qui expriment l'antigène CD89 à leur surface, et qui ont intégré dans leur génome un lentivirus responsable d'une immunodéficience, le lentivirus ayant été modifié génétiquement de sorte qu'outre les gènes responsables du cycle vital du virus, un ou plusieurs gènes codant des protéines marqueurs telles que la GFP ou ses dérivées, ont été insérés.
L'invention concerne également un kit, ou trousse, de détection de cellules réservoirs d'un virus de l'immunodéficience chez les mammifères comprenant au moins un agent détectant, ou permettant la détection, l'un au moins des marqueurs suivants : le marqueur CD123, le marqueur CD87, le marqueur CD89, le marqueur CD172a, le marqueur CD112, le marqueur CD213a1, le marqueur CD120b, le marqueur CD121a, le marqueur CD54, le marqueur CD11c, le marqueur FPRL1 et le marqueur CD32, à la surface des cellules et au moins une composition permettant de déterminer la présence d'ADN dudit virus de l'immunodéficience chez les mammifères dans le génome desdites cellules.
Le kit de détection susmentionné comprend donc des moyens de détecter l'expression membranaire d'au moins un des marqueurs susmentionnés, et des moyens de détection de l'insertion dans le génome de la cellule l'ADN d'un lentivirus responsable d'une immunodéficience. Les moyens de détection des marqueurs sont ceux décrits précédemment, notamment des anticorps spécifiques desdits marqueurs. Avantageusement, l'invention concerne également un kit, ou trousse, de détection de cellules réservoirs d'un virus de l'immunodéficience chez les mammifères comprenant au moins un agent détectant le marqueur de différentiation CD89 à la surface des cellules et au moins une composition permettant de déterminer la présence d'ADN dudit virus de l'immunodéficience chez les mammifères dans le génome desdites cellules.
Dans un mode de réalisation avantageux, l'invention concerne le kit susmentionné, où ledit marqueur CD89 consiste en la protéine de séquence SEQ ID NO : 3. Avantageusement, l'invention concerne un kit tel que défini ci-dessus, dans lequel ladite composition permettant de déterminer la présence d'ADN dudit virus de l'immunodéficience chez les mammifères dans le génome desdites cellules comprend des oligonucléotides spécifiques de séquences du génome dudit virus, notamment pour la mise en œuvre d'une réaction de polymérisation en chaine, ou PCR.
Au vu de la connaissance accumulée depuis la découverte du virus VIH, du virus VIS et du virus VIF, l'homme du métier est à même d'identifier des oligonucléotides permettant d'identifier la présence desdits virus dans le génome de la cellule. L'utilisation de couples d'oligonucléotides permettant la mise en œuvre d'une réaction de PCR est particulièrement avantageuse, mais l'homme du métier peut, s'il le souhaite, faire appel à d'autres techniques de biologie moléculaire telles que l'hybridation de Southern (en anglais Southern blot).

Dans un autre aspect, l'invention concerne une méthode de pronostic, notamment *in vitro,* de la rechute d'une immunodéficience chez les mammifères suite à l'arrêt d'un traitement avec des agents thérapeutiques anti-virus responsable de ladite immunodéficience chez les mammifères, ladite méthode comprenant une étape de quantification des cellules lymphocytaires exprimant à leur surface l'un au moins des marqueurs suivants : le marqueur CD123, le marqueur CD87, le marqueur CD89, le marqueur CD172a, le marqueur CD112, le marqueur CD213a1, le marqueur CD120b, le marqueur CD121a, le marqueur CD54, le marqueur CD11c, le marqueur FPRL1 et le marqueur CD32, et présentant dans leur génome de l'ADN d'un virus responsable de ladite immunodéficience chez les mammifères.

La méthode de pronostic de l'invention est basée sur la détection, ou l'absence de détection de cellules réservoirs de lentivirus.

Après l'arrêt du traitement antiviral dirigé contre le VIH, le virus émerge à nouveau et la pathologie (l'immunodéficience) réapparait. Si le patient est traité à la fois avec un traitement antiviral, et un traitement visant à éradiquer les cellules réservoir, il est nécessaire, après l'arrêt du traitement antiviral de déterminer les chances qu'à le patient à voir ré-émerger la maladie, du fait de la réactivation du virus. Aussi, dans le contexte de l'invention, avec les moyens décrits pour détecter les cellules réservoirs, il sera possible de mesurer la quantité de cellules réservoirs résiduelles après les traitements susmentionnés (anti-virus et anti cellules réservoirs). Si le patient dispose d'une très faible quantité de cellules réservoirs, ou s'il ne dispose plus de cellules réservoirs, dans ce cas le pronostic sera favorable : la maladie aura peu de chances de ré-émerger à court terme. A l'inverse, si de nombreuses cellules réservoirs sont encore présentes, le diagnostic ne sera pas favorable, et la rechute du patient sera rapide.

Avantageusement, l'invention concerne par ailleurs une méthode de pronostic, notamment *in vitro,* de la rechute d'une immunodéficience chez les mammifères suite à l'arrêt d'un traitement avec des agents thérapeutiques anti-virus responsable de ladite immunodéficience chez les mammifères, ladite méthode comprenant une étape de quantification des cellules lymphocytaires exprimant à leur surface au moins le marqueur CD89 et présentant dans leur génome de l'ADN d'un virus responsable de ladite immunodéficience chez les mammifères.

Dans un mode de réalisation avantageux, l'invention concerne la méthode susmentionnée, où ledit marqueur CD89 consiste en la protéine de séquence SEQ ID NO: 3.

L'invention concerne également une méthode de diagnostic, notamment *in vitro,* de la rémission complète d'une immunodéficience chez les mammifères après l'arrêt d'un traitement avec des agents thérapeutiques anti-virus responsable de ladite immunodéficience chez les mammifères, ladite méthode comprenant une étape de de détection de l'absence de cellules lymphocytaires exprimant à leur surface l'un au moins des marqueurs suivants : le marqueur CD123, le marqueur CD87, le marqueur CD89, le marqueur CD172a, le marqueur CD112, le marqueur CD213a1, le marqueur CD120b, le marqueur CD121a, le marqueur CD54, le marqueur CD11c, le marqueur FPRL1 et le marqueur CD32, et présentant dans leur génome de l'ADN d'un virus de l'immunodéficience chez les mammifères.

Dans cette méthode de diagnostic, si le patient n'a plus de cellules réservoirs du VIH dans son organisme, il sera possible de poser le diagnostic de rémission complète de l'infection virale, et donc de l'immunodéficience qui est associée. La détection de l'absence de cellules réservoirs est bien évidemment effectuée par les moyens de détection selon l'invention.

Avantageusement, l'invention concerne également une méthode de diagnostic, notamment *in vitro,* de la rémission complète d'une immunodéficience chez les mammifères après l'arrêt d'un traitement avec des agents thérapeutiques anti-virus responsable de ladite immunodéficience chez les mammifères, ladite méthode comprenant une étape de détection de l'absence de cellules lymphocytaires présentant dans leur génome de l'ADN d'un virus de l'immunodéficience chez les mammifères et exprimant à leur surface au moins le marqueur CD89.

Dans un mode de réalisation avantageux, l'invention concerne la méthode susmentionnée, où ledit marqueur CD89 consiste en la protéine de séquence SEQ ID NO: 3.

L'invention concerne également une méthode de détection de cellules réservoirs de cellules infectées par un virus de l'immunodéficience chez les mammifères, ladite méthode comprenant une étape de mise en contact, *in vitro* ou *ex vivo,* de cellules d'un mammifère infectées par ledit virus de l'immunodéficience chez les mammifères, et notamment traitées avec une thérapie antirétrovirale spécifique dudit virus de l'immunodéficience chez les mammifères et/ou une thérapie visant à éradiquer lesdites cellules réservoirs, avec un agent permettant la détection des cellules lymphocytaires exprimant à leur surface un marqueur de différentiation susmentionné, notamment le marqueur CD89.

L'invention concerne par ailleurs une méthode d'évaluation de l'efficacité d'un traitement visant à éradiquer des cellules réservoirs de cellules d'un mammifère infectées par ledit virus de l'immunodéficience chez les mammifères, ladite méthode comprenant une étape de détection in vitro ou ex vivo desdites cellules de réservoir avec un agent permettant la détection de la présente la quantité ou l'absence de cellules lymphocytaires exprimant à leur surface le marqueur de différentiation CD89.

Avantageusement, l'invention concerne la méthode susmentionnée, comprenant en outre une étape de classification desdits mammifères infectés selon la présence, l'absence ou la quantité de cellules réservoir détectées.

Dans cette méthode, les cellules réservoirs sont recherchées à l'issue d'un traitement visant à les détruire. Si les cellules réservoir sont toujours présentes dans un nombre substantiellement identique à celui avant ledit traitement visant à les éradiquer, le traitement n'a alors pas fonctionné. Si par contre le nombre de cellules réservoir a significativement diminué, dans ce cas le traitement est opérationnel, notamment lorsqu'il n'est plus possible d'identifier de cellules réservoir.

Par ailleurs l'invention concerne un agent thérapeutique ciblant spécifiquement les cellules lymphocytaires réservoir des lentivirus telles qu'elles sont identifiées dans la présente invention, notamment les cellules réservoirs des virus responsables d'une immunodéficience chez les mammifères, tels que les virus VIH 1 et 2, VIS ou VIF.

Avantageusement, l'agent thérapeutique est un anticorps ou un agent chimique capable de reconnaître les cellules réservoirs telles qu'identifiées dans la présente invention.

L'agent thérapeutique susmentionné est plus avantageusement un anticorps bifonctionnel capable de reconnaitre d'une part l'un des marqueurs suivants : le marqueur CD123, le marqueur CD87, le marqueur CD89, le marqueur CD172a, le marqueur CD112, le marqueur CD213a1, le marqueur CD120b, le marqueur CD121a, le marqueur CD54, le marqueur CD11c, le marqueur FPRL1 et le marqueur CD32, et d'autre part l'antigène CD3 (protéine associée au récepteur T ou TCR spécifique des lymphocytes T), ou un ou plusieurs autres marqueurs spécifiques des cellules T.

L'invention concerne avantageusement un Anticorps multispécifique reconnaissant d'une part au moins un épitope du marqueur CD89 et au moins un marqueur caractéristique des cellules lymphocytaires, notamment les cellules lymphocytaires, notamment les cellules lymphocytaires T.

Plus avantageusement, l'invention concerne l'anticorps multispécifique susmentionné, où ledit anticorps est un anticorps bispécifique, reconnaissant spécifiquement l'antigène CD89 et l'antigène CD3.

Avantageusement, les anticorps susmentionnés sont dans le cadre de l'invention monoclonaux, notamment humain ou humanisé, de singe ou siminisé, ou de chat ou felinisés.

Par anticorps multispécifique on entend dans l'invention un anticorps capable de reconnaître un premier antigène par son premier CDR et un second antigène par son second CDR.

Avantageusement, l'anticorps défini ci-dessus est un anticorps bifonctionnel, ou bispécifique, reconnaissant l'antigène CD89 et l'antigène CD3.

Par anticorps bifonctionnel, ou bispécifique, on entend dans l'invention, un anticorps recombinant présentant une partie hyper variable reconnaissant un épitope déterminé, et une seconde partie hypervariable reconnaissant un second épitope, différent du premier. Ainsi, un anticorps bifonctionnel, ou bispécifique, est capable de reconnaître simultanément deux protéines différentes. Ces anticorps peuvent également se trouver sous la forme de leurs fragments tels que des scFv bispécifiques, des bi scFv bispécifiques, des minicorps (minibody en anglais) bi spécifiques ou encore des diacorps (diabody en anglais) bispécifiques.

Ces anticorps, ou leurs fragments, bispécifiques sont avantageusement humanisés pour une utilisation en thérapie humaine. L'humanisation, bien connue de l'état de l'art, consiste à remplacer toute la structure de l'anticorps par des molécules de séquence humaine auxquelles sont greffées les régions hypervariables d'intérêt, généralement issues d'anticorps monoclonaux de souris.

Aussi, de manière plus avantageuse, l'invention concerne un anticorps monoclonal, notamment humanisé, ou l'un de ses fragments susmentionnés, capable de reconnaître simultanément, d'une part l'un des marqueurs suivants : le marqueur CD123, le marqueur CD87, le marqueur CD89, le marqueur CD172a, le marqueur CD112, le marqueur CD213a1, le marqueur CD120b, le marqueur CD121a, le marqueur CD54, le marqueur CD11c, le marqueur FPRL1 et le marqueur CD32, et d'autre part le marqueur CD3.

Dans l'invention, les anticorps utilisés peuvent être couplés à des agents actifs tels que des toxines (par exemple la ricine ou le DM1 dérivé maytansine), des radioisotopes (par exemple l'yttrium 90 ou ⁹⁰Y (émetteur pur de rayonnement bêta moins) et l'iode 131 ou ¹³¹I (émetteur bêta moins et gamma)), des agents biologiques, des drogues ou encore des enzymes. L'efficacité thérapeutique repose alors principalement sur l'agent couplé, l'anticorps jouant le rôle de vecteur pour guider l'agent vers la cible.

L'invention concerne également un anticorps bispécifique dirigé d'une part contre l'un des marqueurs suivants : le marqueur CD123, le marqueur CD87, le marqueur CD89, le marqueur CD172a, le marqueur CD112, le marqueur CD213a1, le marqueur CD120b, le marqueur CD121a, le marqueur CD54, le marqueur CD11c, le marqueur FPRL1 et le marqueur CD32, et d'autre part contre un marqueur spécifique des cellules T, notamment le marqueur CD3, pour son utilisation dans le cadre du traitement d'une infection par un lentivirus, notamment une infection liée au VIH ou au VIS.

De manière plus avantageuse, l'invention concerne un anticorps bispécifique dirigé contre le CD89 et le CD3 pour son utilisation dans le cadre du traitement d'une infection par un lentivirus, notamment une infection liée au VIH ou au VIS ou au VIF.

L'invention concerne en outre une composition comprenant au moins un agent reconnaissant les cellules lymphocytaires exprimant le marqueur CD89, pour son utilisation pour l'éradication de cellules réservoirs de cellules infectées par un virus de l'immunodéficience chez les mammifères.

Avantageusement, l'invention concerne la composition susmentionnée, où ledit agent est au moins un anticorps reconnaissant le marqueur CD89, notamment un anticorps monospécifique du marqueur CD89, ou un anticorps multispécifique reconnaissant au moins un épitope du marqueur CD89 et au moins un marqueur caractéristique des cellules lymphocytaires, notamment les cellules lymphocytaires T, pour son utilisation susmentionnée.

Comme indiqué ci-dessus, les anticorps couplés à des agents toxiques peuvent être avantageux pour éradiquer les cellules réservoirs.

L'invention porte également sur une composition comprenant
- d'une part un anticorps bispécifique tel que défini ci-dessus, et
- d'autre part une combinaison d'agents anti-retroviraux, notamment pour son utilisation dans le cadre du traitement d'une infection par un lentivirus, notamment une infection liée au VIH ou au VIS,
- et éventuellement une composition éradiquant les cellules réservoirs.

De manière avantageuse, l'invention concerne une composition pour son utilisation susmentionnée, dans laquelle l'anticorps spécifique et la combinaison d'agents antirétroviraux sont utilisées simultanément, séparément ou étalée dans le temps.

Il est également décrit une méthode de traitement des infections par des lentivirus, notamment des lentivirus provoquant une immunodéficience chez les mammifères, comprenant l'administration à un mammifère infecté, notamment un humain infecté par le VIH, une dose efficace de la composition susmentionnée.

L'invention concerne par ailleurs un anticorps reconnaissant spécifiquement le marqueur CD89 exprimés sur les cellules lymphocytaires réservoir des lentivirus, pour son utilisation dans le cadre du traitement d'une infection par un lentivirus, notamment une infection liée au VIH, au VIS ou au VIF.

Avantageusement, l'anticorps susmentionné est un anticorps anti CD89, éventuellement couplé à un agent cytotoxique, ou un anticorps au moins bispécifique reconnaissant outre le marqueur CD89 et au moins un autre marqueur spécifique des cellules lymphocytaires, notamment le marqueur CD3.

L'invention concerna aussi une composition susmentionnée, pour son utilisation dans le cadre du traitement d'une infection par un lentivirus, notamment une infection liée au VIH, au VIS ou au VIF.

Avantageusement, l'invention concerne une composition pour son utilisation susmentionnée, dans laquelle l'anticorps spécifique et la combinaison d'agents antirétroviraux sont utilisées simultanément, séparément ou étalée dans le temps.

L'invention sera mieux comprise à la lumière des données expérimentales exposées ci-après et des figures qui les illustrent.

### Légende des figures

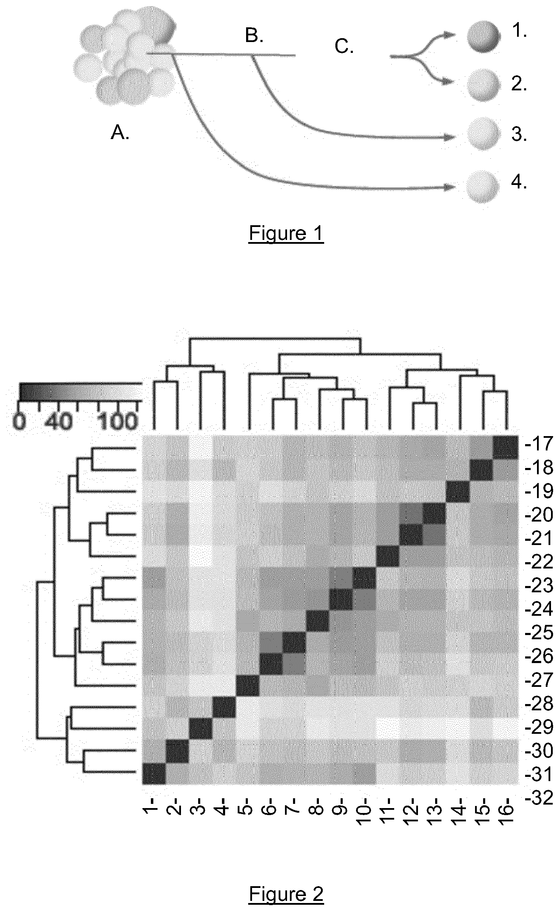

La **Figure 1** représente l'approche expérimentale utilisée par les inventeurs pour obtenir des lymphocytes TCD4 infectés de manière latente. A. : Les cellules mononuclées de sang périphérique (PBMC en anglais) d'individus sains (non infectés par le VIH) sont prélevées à t0. Sans plus de traitements elles constituent les cellules contrôles 4 (Non infectées ; NI). Le reste des PBMC est traité avec la protéine Vpx (VLP-Vpx ; B.). Les cellules qui ne sont pas traitées subséqemment constituent le second témoin 3.de cellules traitées avec Vpx et non infectées.

Enfin les cellules restrantes sont infectées avec un virus VIH permettant l'expression de manière constitutive de la GFP (C.). La fraction des cellules exposée au VIH se divise alors en deux catégories : les cellules exposées non infectées n'exprimant pas la GFP (2.) et les cellules exposées au virus ; infectées et exprimant la GFP (1.).

Les différentes cellules sont triées à l'aide d'un cytomètre de flux en vu de l'extraction de leurs ARN, le séquençage desdits ARN extraits, d'analyses bio-informatique et de la validation des candidats par cytométrie de flux.

La **figure 2** est un graphique coloré (ici en noir et blanc) de regroupement hiérarchique effectuée sur la distance euclidienne et calculée en utilisant les comptages d'expression génique transformés et logarithmiquement régularisés (en anglais "regularized-log transformed gene expression counts"), entre chaque sous-ensembles de cellules 1. à 3. et non infectées (4.). Le graphique représente les résultats pour des PBMC issus de quatre donneurs différents. 1- : Cellules 1. issues de l'individu 2 ; 2- : Cellules 1. issues de l'individu 4 ; 3- : Cellules 1. issues de l'individu 1 ; 4- : Cellules 1. issues de l'individu 3 ; 5- : Cellules 4. issues de l'individu 4 ; 6- : Cellules 3. issues de l'individu 1 ; 7- : Cellules 2. issues de l'individu 1 ; 8- : Cellules 4. issues de l'individu 1 ; 9- : Cellules 3. issues de l'individu 2 ; 10- : Cellules 2. issues de l'individu 2 ; 11- : Cellules 4. issues de l'individu 3 ; 12- : Cellules 3. issues de l'individu 4 ; 13- : Cellules 2. issues de l'individu 4 ; 14- : Cellules 4. issues de l'individu 2 ; 15- : Cellules 3. issues de l'individu 3 ; 16- : Cellules 2. issues de l'individu 3 ; 17- : Cellules 2. issues de l'individu 3 ; 18- : Cellules 3. issues de l'individu 3 ; 19- : Cellules 4. issues de l'individu 2 ; 20- : Cellules 2. issues de l'individu 4 ; 21- : Cellules 3. issues de l'individu 4 ; 22- : Cellules 4. issues de l'individu 3 ; 23- : Cellules 2. issues de l'individu 2 ; 24- : Cellules 3. issues de l'individu 2 ; 25- : Cellules 4. issues de l'individu 1 ; 26- : Cellules 2. issues de l'individu 1 ; 27- : Cellules 3. issues de l'individu 1 ; 28- : Cellules 4. issues de l'individu 4 ; 29- : Cellules 1. issues de l'individu 3 ; 30- : Cellules 1. issues de l'individu 1 ; 31- : Cellules 1. issues de l'individu 4 et 32- : Cellules 1. issues de l'individu 2.

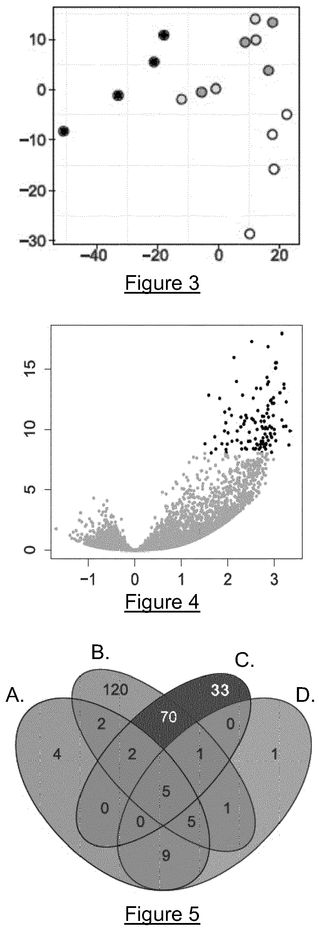

La **figure 3** est un graphique montrant l'analyse en composantes principales des "regularized-log transformed gene expression counts" pour les sous-groupes 1. (points noirs), 2. (points gris foncés), 3. (points gris clairs) et 4.(points blancs) de 4 donneurs sains. Les points de deux premières composantes principales sont présentés. L'axe des abscisses représente PC2 : 17% de la variance et l'axe des ordonnées représente PC1 : 58% de la variance.

La **figure 4** représente un graphique Volcano présentant la significativité (False Discovery Rate, FDR) et la modification d'expression génique entre les cellules TCD4 infectées (1.) et les cellules TCD4 exposées non infectées (2.). Les points noirs indiquent les gènes sélectionnés du fait de leur sur-expression significative dans la fraction 1. comparée aux cellules 2. (FDR <10-8). L'axe des abscisses représente le logarithme de base 2 de la modification d'expression et l'axe des ordonnées représente le logarithme de base 10 de FDR.

La **figure 5** représente un diagramme de Venn montrant les 253 gènes exprimés de manière différentielle entre les 4 groupes et notamment 2. vs 1. (FDR <10-8). Les intersections grises foncées indiquent des gènes sélectionnés pour une analyse ultérieure. A : 4. vs 3. ; B : 4. vs 1. ; C : 2. vs 1. et D : 4. vs 2.

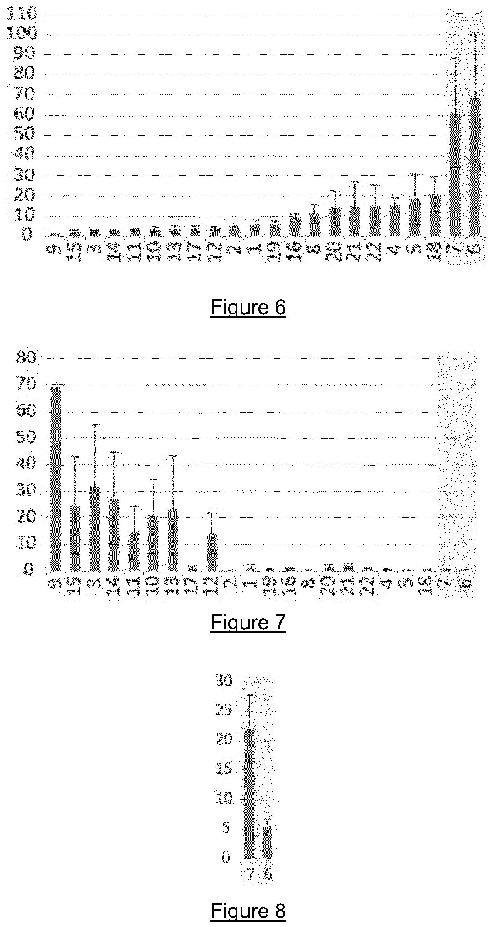

La **figure 6** représente un histogramme montrant les résultats de cytométrie d'augmentation d'expression (axe des abscisses) pour chacun des marqueurs évalués (gènes 1 à 22 sur les 111 candidats potentiels) sur les cellules TCD4 1. et 2.

Les marqueurs sont les suivants : 9 : aqp9, 15 : mucl1, 3 : ca12, 14 : vnn3, 11 : eaat1, 10 : c22orf42, 13 : gpr91, 17 : cd66d, 12 : step1b, 2 : gjb2, 1 : colec12, 19 : cd80, 16 : niacr1, 8 : cd354, 20 : cd116, 21 : scarf1, 22 :llrk2, 4 : cd300c, 5 : clec4d, 18 : tlr2, 7 : cd32 et 6 : fprl1

La **figure 7** représente un histogramme montrant le pourcentage des cellules TCD4 totales 2. exprimant chaque marqueur afin de mettre en évidence l'expression des candidats les plus fiables sur les cellules 1. Les marqueurs sont les suivants : 9 : aqp9, 15 : mucl1, 3 : ca12, 14 : vnn3, 11 : eaat1, 10 : c22orf42, 13 : gpr91, 17 : cd66d, 12 : step1b, 2 : gjb2, 1 : colec12, 19 : cd80, 16 : niacr1, 8 : cd354, 20 : cd116, 21 : scarf1, 22 :llrk2, 4 : cd300c, 5 : clec4d, 18 : tlr2, 7 : cd32 et 6 : fprl1.

La **figure 8** représente un histogramme montrant le pourcentage de cellules TCD4 total 1. exprimant le marqueur #7 (CD32) et le marqueur #6 (fprl1).

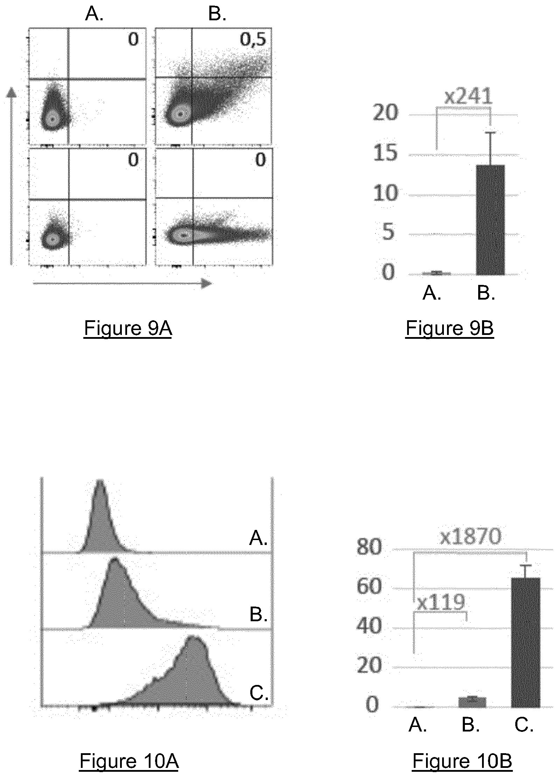

Les **figures 9A et 9B** représentent les profils d'expression du marqueur #7 (CD32) dans le modèle de latence virale développé par les inventeurs.

La figure 9A représente les résultats de cytométrie des cellules TCD4 infectées de façon latente générées comme représenté sur la figure 1. L'expression du marqueur #7 (CD32) a été évaluée par cytométrie en flux à la surface des cellules TCD4 infectées (1.; B.) et des cellules TCD4 quiescentes non infectées 2. (A.) (n=3). Les pourcentages GFP+/marker #7+ (CD32+) sont indiqués sur chaque panel. L'axe des abscisses représente l'intensité de fluorescence de la GFP, et l'axe des ordonnées représente l'intensité de fluorescence de marqueur CD32.

La figure 9B représente un histogramme indiquent le pourcentage de cellules exprimant le marqueur #7+ (CD32+) entre cellules 2. (GFPneg ; A.) et 4 (GFPpos ; B.).

Les **figures 10A et 10B** montrent l'intensité de fluorescence du marqueur #7 (CD32) est corrélée à l'intensité de l'expression de la GFP dans les cellules TCD4 infectées (n = 3).

La figure 10A montre des représentations du nombre de cellules en fonction de l'intensité de fluorescence du marqueur CD32 pour trois sous-groupes des cellules : A. les cellules n'exprimant pas la GFP, B. : les cellules exprimant faiblement la GFP et C : les cellules exprimant fortement la GFP.

La figure 10B représente un graphique le pourcentage de marqueur CD32 sur les cellules TCD4 pour les 3 catégories A. B. et C. décrites à la figure 10A.

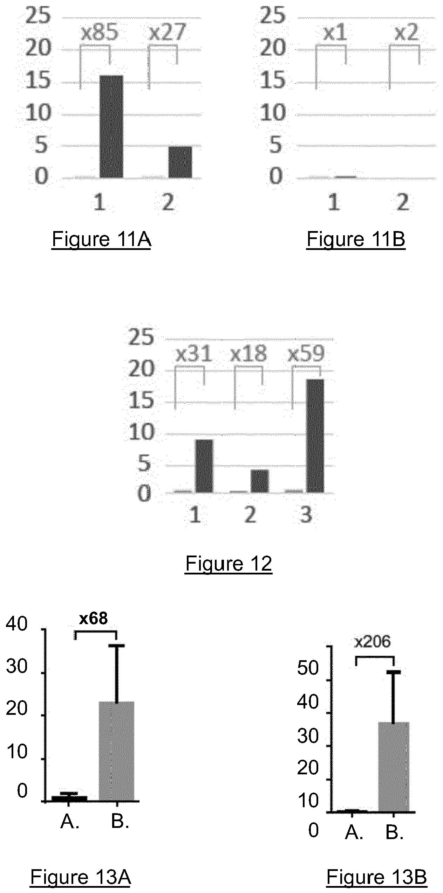

Les **figures 11A et 11B** représentent des graphiques montrant l'expression du marqueur #7 (CD32) à la surface populations de cellules TCD4 non stimulées (11A) ou stimulées par la voie TCR (n=2) (11B). Les barres noires représentent le pourcentage de cellules GFP positives et les barres grises le pourcentage de cellules GFP négatives.

La **figure 12** représente un graphique montrant que l'utilisation de particules virales pseudotypés VSV-G permet de mettre en évidence une induction de l'expression du marqueur #7 (CD32) sur des cellules telles que les TCD8 (n = 2). La moyenne et les IQR sont présentés sur chaque histogramme ainsi que l'augmentation entre les cellules 2. (GFPneg) et 4. (GFP+) lorsque cela est nécessaire.

Les **figures 13** **A et 13B** montrent l'analyse par cytométrie de l'induction du marqueur #7 (CD32) à la surface des cellules TCD4 quiescentes infectées (GFP+) en comparaison avec des cellules non infectées ou traitées avec VLP-Vpx seul.

Des PBMC de donneurs sains (n=3) ont été infectées par les virus SIVmac239 (13A) et VIH-2 (13B). Les résultats de l'analyses FACS sont représentés en % de cellules exprimant le marqueur #7 (CD32) dans les populations non-infectées (GFP- ; A.) et infectées (GFP+ ; B.). Les histogrammes présentent la moyenne et la déviation standard pour les expériences réalisées avec SIVmac239 et VIH-2.

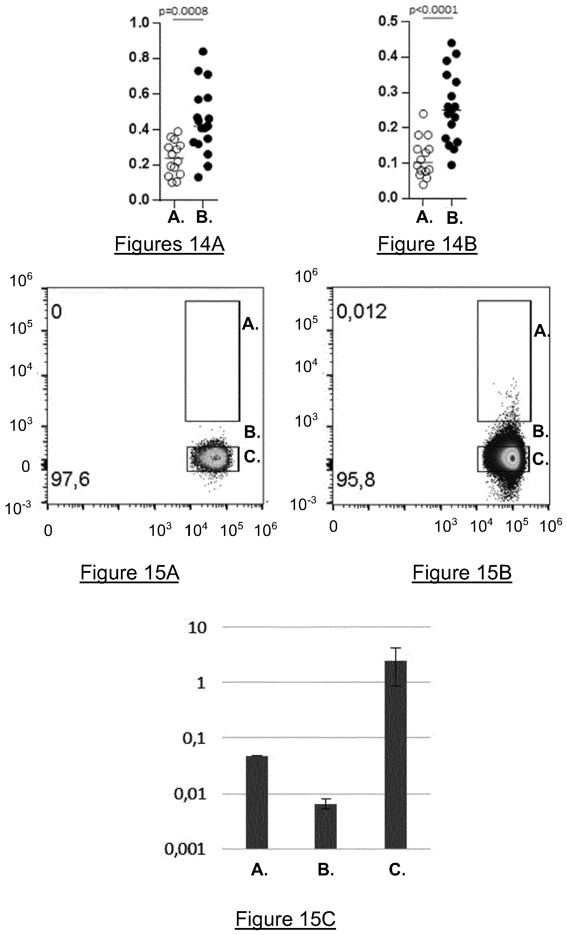

Les **figures 14A et 14B** représentent des histogrammes montrant la comparaison du niveau d'expression *ex vivo* du marqueur #7 (CD32) à la surface des cellules TCD4 de patients infectés par le VIH-1 et viro-supprimés (B) en comparaison de donneurs sains (A).

La figure 14A représente le pourcentage de cellules CD32+ sur le total des cellules TCD4.

La figure 14A représente le pourcentage de cellules CD32+ sur le total des cellules TCD4 immatures DR+.

Les **figures 15A à 15** **C** montrent les résultats d'expression du marqueur CD32 chez un premier patient.

La figure 15A montre des résultats de cytométrie de flux montrant les différents niveaux d'expression ex vivo du marqueur #7 (CD32) sur les lymphoctytes TCD4 (sélection CD3+/CD4+) chez un patient avirémique (n=2), à l'aide d'un isotype. L'axe des abscisses représente le niveau de fluorescence du CD3 et l'axe des ordonnées ne niveau de fluorescence de CD32.

La figure 15B montre des résultats de cytométrie de flux montrant les différents niveaux d'expression ex vivo du marqueur #7 (CD32) sur les lymphoctytes TCD4 (selection CD3+/CD4+) chez un patient avirémique (n=2), à l'aide d'un anticorps anti CD32. A : cellules exprimant fortement le CD32, B : cellules exprimant faiblement le CD32 et C : Cellules n'exprimant pas le CD32. L'axe des abscisses représente l'intensité de fluorescence du marqueur CD3, et l'axe des ordonnées représente l'intensité de fluorescence du marqueur CD32.

La figure 15 C représente un histogramme montrant le nombre de copies d'ADN VIH-1 total par cellule quantifiées par qPCR (normalisation avec le gène de la béta-globine) sur les cellules : A : cellules CD4+ ; B : cellules CD4+ CD32-, C : CD4+ CD32 exprimé fortement.

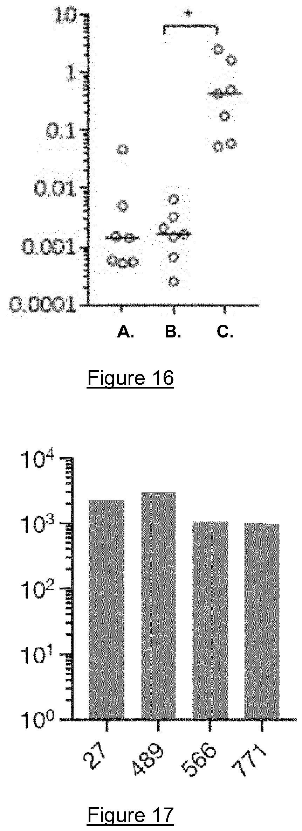

La **figure 16** est un graphique représentant le nombre de copie d'ADN VIH-1 par cellule, quantifié par qPCR, chez 7 patients avirémiques dans les copulations de cellules TCD4 totales (A), les populations TCD4 CD32- (B) et TCD4 CD32+ (C). Un test de Wilcoxon entre les fractions T CD4+ CD32- et CD32+ montre un enrichissement significatif en ADN VIH-1 dans la fraction CD32+ (p=0.0156).

La **Figure 17** est un histogramme montrant le logarithme des unités infectieuses par million des populations de cellules T CD4 CD32+ issues de 4 patients (27, 439, 566 et 771) après réactivation du virus HIV.

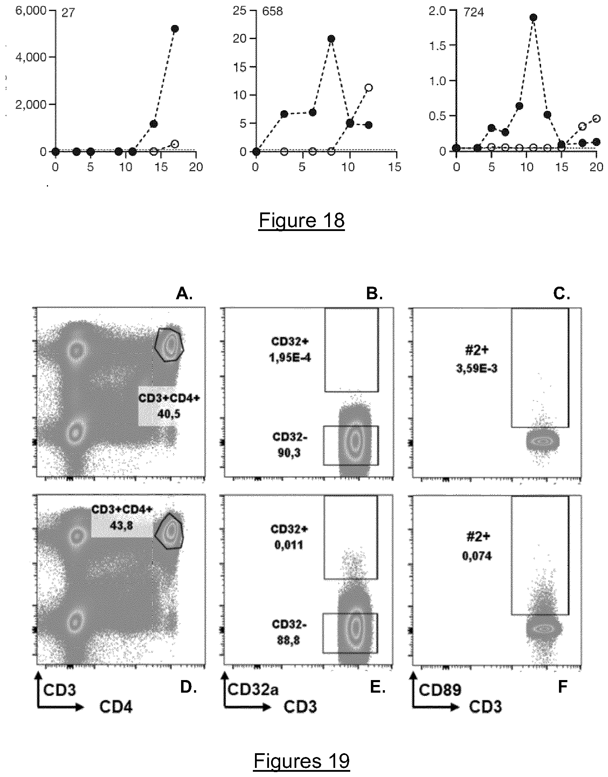

La **Figure 18** représente 3 courbes montrant la réactivation virale au cours du temps (exprimée en jours, axe des abscisses) à partir de populations de cellules T CD4 totales (ronds noirs) ou T CD4 déplétées en cellules CD32 (ronds gris) à partir de trois échantillons de patients différents et indépendants. L'axe des ordonnées représente la quantité de p24 en pg.mL⁻¹.

Les **Figures 19A à 19C** représentent des résultats de cytométrie de flux montrant les contrôles isotypiques (contrôles négatifs) des marquages de surface CD32 et CD89.

La figure 19A représente des résultats de cytométrie de flux sur des cellules de patients et détectées avec les marqueurs CD3 et CD4. La population CD4+CD3+ est marquée par une fenêtre (40,5% de la population).

La figure 19B représente des résultats de cytométrie de flux sur les cellules de la fenêtre de la figure 19A, et les marquages de contrôle isotypique pour le marqueur CD32, détectées à l'aide des marqueurs CD3 et isotype contrôle. Les cellules marquées CD3 et isotype sont détéctées pour leur positivité ou négativité à l'antigène CD32

La figure 19C représente des résultats de cytométrie de flux sur les cellules de la fenêtre du bas (CD32-) de la figure 19B. Les cellules marquées CD3 et isotype sont détectées pour leur positivité ou négativité à l'antigène CD89.

Les **Figures 19D à 19F** représentent des résultats de cytométrie de flux montrant les marquages de surface CD32 et CD89.

La figure 19D représente des résultats de cytométrie de flux sur des cellules de patients et détectées avec les marqueurs CD3 et CD4. La population CD4+CD3+ est marquée par une fenêtre (43,8% de la population).

La figure 19E représente des résultats de cytométrie de flux sur les cellules de la fenêtre de la figure 19A, et détectées à l'aide des marqueurs CD3 et CD32. Les cellules CD32+ (fenêtre du haut) et CD32- (fenêtre du bas - 88,8%) sont indiquées.

La figure 19F représente des résultats de cytométrie de flux sur les cellules de la fenêtre du bas (CD32-) de la figure 19B. Les cellules marquées CD3 et CD89 sont détectées pour leur positivité ou négativité à l'antigène CD89. La fenêtre représente les cellules CD89+ CD32- (0,074%).

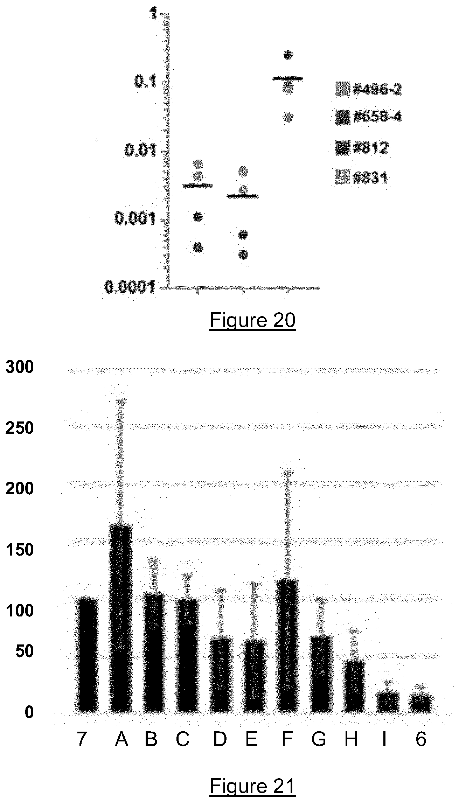

La **Figure 20** est un graphique représentant le nombre de copies d'ADN de VIH par cellule à partir de populations totales de T CD4 (A), de sous-population de cellules T CD4+CD32-CD89- (B) et de sous populations de cellules T CD4+CD32-CD89+(C) de 4 patients indépendants. La quantification des ADN viraux présents dans chacune de ces fractions a été réalisé par qPCR ADN VIH-1. Chez les patients testés, le marqueur CD89 identifie bien un réservoir de cellules infectées (médiane approximative de 0,1 copies d'ADN VIH-1 par cellule T CD4+ CD32a- CD89+)

La **Figure 21** est un histogramme montrant le rapport de marqueurs A, B, C, D, E (CD89), F, G, H et I par rapport au CD32 dans des cellules de patients GFP+ ayant subi une réactivation du VIH. La réactivation du virus est mesurée par RNAseq.

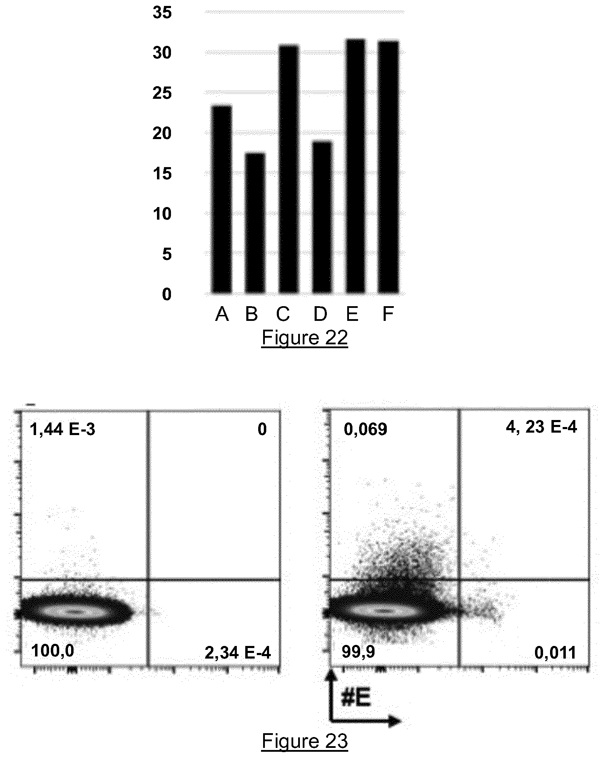

La **Figure 22** est un histogramme montrant le rapport de marqueurs A, B, C, D, E (CD89), dans ces cellules CD32- dans des cellules de patients GFP+ ayant subi une réactivation du VIH. Ces marqueurs sont mutuellement exclusifs avec CD32.

La **Figure 23** montre des résultats de cytométrie de flux de cellules T CD4 de patient (patient 812) marquées avec des anticorps CD32 et CD89 (image de droite bas) ou des isotypes (image de gauche) et la détection des cellules simplement (CD32 ou CD89) et doublement (CD32 et CD89) marquées. On note que les deux populations sont exclusives et qu'aucun double marquage n'est détecté.

### Exemples

### Exemple1 - identification de marqueurs spécifiques des cellules réservoirs

L'invention a pour objectif d'identifier et valider un marqueur spécifique des cellules infectées à l'aide de modèle *in vitro* et l'exploration phénotypique *ex vivo* des cellules primaires de patients infectés par le VIH-1 sous traitement antiviral (anti rétroviral) efficace. La mise en évidence in vitro et ex vivo d'une expression spécifique notamment du marqueur CD32 à la surface des cellules infectées peut être exploitée afin de cibler et d'éliminer le réservoir viral chez les patients infectés par le VIH-1, et ainsi proposer une thérapie efficace permettant d'éradiquer définitivement le virus chez les patients infectés.

### Matériel et méthodes :

### 1. Production virale et VLPs.

Les VLP contenant Vpx et les particules virales ont été produites en suivant le protocole standard de transfection phosphate calcium d'ADN dans les cellules 293T. Les VLP-Vpx ont été produits en co-transfectant 8µg de plasmide pSIV3+ et 2 µg de plasmide pMD2-G VSV-G. Le milieu de culture a été remplacé 16h post-transfection avant de récupérer les VLPs 48h plus tard, les centrifuger les filtrer sur filtre 0.45mm et les concentrer 100X par ultracentrifugation. Les particules virales VIH-1-CMV-eGFP ont été produites en co-transfectant 5µg du plasmide pHRET, 5µg de plasmide de packaging psPAX2 et 2µg de plasmide pMD2-G. Après concentration, le titre p24 du stock viral a été mesuré par ELISA et le titre infectieux (MOI) par titrage sur cellules 293T.

### 2. Infection et « sorting » des lymphocytes T CD4+ quiescents infectés in vitro.

Les cellules mononuclées du sang périphérique de donneurs sains ont été isolées par gradient de densité (Ficoll), puis cultivées en plaque 24 puits en présence de VLP-Vpx pendant 12h à une concentration de 2.10⁶ cellules/puit dans 300µL final de milieu complet (RPMI 10 %SVF). Les cellules ont ensuite été infectées par ajout de VIH-1-CMV-eGFP (1µg p24 équivalent à une MOI de 10X). Comme contrôle, des cellules ont été cultivées exclusivement en présence de VLP-Vpx, de VIH-1-CMV-egFP, ou ont été laissées non traitées. Trois jours post-infection, les cellules T CD4+ quiescentes (CD69- HLA-DR-) infectées (XH+), les cellules T CD4+ quiescentes traitées exclusivement avec du VIH-1-CMV-eGFP (XH-) et les contrôles (X ou NT) ont été isolées à l'aide d'un trieur. Les cellules triées ont été resuspendues dans du tampon RA1 complété en béta-mercaptoéthanol et stockées à -80°C avant extraction de ARN totaux.

### 3. Séquençage des ARN totaux et analyse bio-informatique.

Les ARN totaux issus des fractions XH+, XH-, X et NT ont été extraits en utilisant le GE Healthcare Illustra RNA mini kit. La qualité des ARN a été analysée sur le Bio-analyseur 2100 d'Agilent et par RNA Nanochip. Une libraire Illumina a ensuite été constituée. Les échantillons ont été multiplexés avant séquençage. Une analyse en composantes principales des "regularized-log transformed gene expression counts" pour les différentes fractions.

### 4. Isolement des cellules mononuclées du sang périphérique des patients VIH-1.

Les cellules mononuclées du sang périphérique de patients VIH-1 traités efficacement (charge virale <20 copies d'ARN VIH-1/mL de sang) ont été isolées par gradient de densité (Ficoll).

### 5. Cytométrie de flux et « sorting » des sous-populations de lymphocytes T CD4+.

Les cellules issues des infections *in vitro,* du sang périphérique de donneurs sains et de patients VIH-1 ont été marquées à l'aide d'anticorps anti-CD3, anti-CD4, anti-CD32, anti-HLA-DR, anti-CD69 et analysées par FACS. Les cellules fraiches de patients VIH-1 ont été marquées à l'aide d'anticorps anti-CD3, anti-CD4, anti-CD32, anti-HLA-DR et d'un contrôle isotypique IgG2 afin d'être triées sur SH800 (Sony) en fonction de l'expression du marqueur CD32 (T CD4+ totaux ; T CD4+ CD32- ; T CD4+ CD32low ; T CD4+ CD32+). Pour chaque sous-population, une partie des cellules triées a été conservée à -80°C en culot sec pour une quantification de l'ADN VIH-1 total et une seconde partie a été mise en culture pour des tests d'inductibilté et d'amplification virale.

### 6. Quantification des ADN VIH-1 totaux.

Les ADN des différentes fractions isolées à partir de sang de patients VIH-1 ont été purifiés à l'aide du QIAamp DNA micro kit, Qiagen). La concentration en ADN a été déterminée par qPCR béta-globine (DNA control kit, Roche). Le nombre de copie d'ADN VIH-1 total par cellule a été déterminé par qPCR ultrasensible (Bicentric).

### Descriptif détaillé :

Afin d'identifier des candidats marqueurs, les inventeurs ont développé un modèle *in vitro* permettant pour la première fois d'infecter directement des lymphocytes TCD4 quiescentes issues de donneurs sains. En effet, ces cellules ne sont pas permissives à l'infection par le VIH-1 sans signal d'activation préalable (activation via le TCR ou PHA/IL2). L'identification par les inventeurs de la protéine SAMHD1, responsable de la restriction dans ces cellules, leur a permis de développer un traitement par des VLP contenant la protéine Vpx (codée par le virus SIVmac251) permettant la levée de restriction et l'infection directe, sans recours à un signal d'activation. A l'aide de ce modèle **(****Figure 1****)** où des PBMC de donneurs sains ont été traités par VLP-Vpx puis infectés par un VIH-1-CMV-GFP, les ARN totaux des cellules infectées (GFP+) ont été extraits afin de réaliser des expériences de RNA-seq sur les ARN messagers. Des PBMC traitées par VLP-Vpx mais non-infectées ainsi que des cellules non infectées ont été utilisées comme contrôle. Une analyse statistique et bio-informatique a permis de déterminer l'impact de l'infection par le VIH-1 sur le programme transcriptionnel des lymphocytes TCD4 quiescents.

En effet, les analyses en composante principale PCA **(****Figure 2****)** et le regroupement hiérarchique HC **(****Figure 3****)** ont été réalisées à partir des résultats RNA-seq. Les deux analyses PCA et HC effectuées sur quatre donneurs ont permis de mettre en évidence que les cellules traités VLP-Vpx et infectés (VLP-Vpx GFP+) formaient un cluster distinct des cellules contrôles (VLP-Vpx GFP-, VLP-Vpx seul et non infectés). Ces résultats indiquent que les cellules VLP-Vpx GFP+ de différents donneurs partagent une signature qui les distingue des autres populations. Lors de l'infection, le VIH-1 module donc l'expression génique des lymphocytes TCD4 quiescents et pourrait se traduire par un profil phénotypique les distinguant des cellules non infectées.

Les inventeurs se sont donc intéressés aux gènes exprimés de façon différentielle (gènes DE) et notamment aux gènes up-régulés lors de l'infection latente **(****Figures 4 et 5****).** Ainsi, 22 candidats marqueurs correspondant à des protéines de surface ont été sélectionnés pour validation *in vitro.* En utilisant le modèle *in vitro* sur des PBMC de donneurs sains, l'expression de 22 marqueurs a été testée par analyse FACS **(****Figure 6****)** sur les lymphocytes TCD4 quiescent infectés. Les résultats sont présentés en pourcentage d'expression des marqueurs (#1 à #22) sur les cellules infectées GFP+ et non infectées (GFP-). L'analyse a mis en lumière une expression spécifique du marqueur CD32 (marqueur #7) sur les cellules infectées en comparaison des cellules contrôle **(****Figures 7 et 8****).** L'indentification d'un candidat marqueur s'est donc focalisée sur le marqueur CD32.

La **figure 21** montre que 9 autres marqueurs sont également exprimés à la surface des cellules T CD4 de patients infectés et qui sont susceptible de rebond viral.

La poursuite de l'analyse sur de nouveaux donneurs a permis de confirmer l'induction de l'expression spécifique du marqueur CD32 dans les cellules infectées de manière latente avec enrichissement dans le niveau d'expression de ce marqueur dans les population GFPhigh **(****Figures 9A et 9B et 10A et 10B****).** Les inventeurs ont également montré que l'induction du marqueur CD32 est limitée à la population quiescente. En effet, des cellules stimulées par l'intermédiaire de la voie TCR (anti-CD3/anti-CD28) n'expriment pas le marqueur CD32 **(****Figures 11A et 11B****).** L'utilisation d'un virus pseudotypé par l'enveloppe du virus VSV a permis de montrer que l'induction de ce marqueur sur d'autres populations infectées telles que les CD8 **(****Figure 12****).** De plus, nous avons déterminé si les lentivirus HIV2 et SIV induisent l'expression de CD32 comme le VIH-1. Les **figures 13A et 13B** montrent que SIV ainsi que HIV2 sont capables d'induire l'expression de CD32 à la surface des cellules T CD4 quiescentes infectées en présence de Vpx.

Après avoir validé le marqueur CD32 in vitro, les inventeurs ont cherché à établir sa relevance *ex vivo.* Ainsi, une analyse phénotypique par FACS du niveau d'expression de ce marqueur sur les lymphocytes TCD4 a été réalisée sur les PBMC de patients infectés et traité efficacement avec des antiviraux en comparaison à des donneurs sains. Nous avons pu mettre en évidence une expression significativement plus élevée du CD32 chez les patients **(****Figure 14A et 14B****).**

Enfin, les inventeurs ont exploré le niveau d'enrichissement en ADN-VIH-1 dans les différents sous-groupes de lymphocytes TCD4 exprimant différentiellement le marqueur CD32 (TCD4 totaux, TCD4 CD32-, TCD4 CD32low et TCD4 CD32+) de deux patients viro-supprimés. Après tri de ces différentes populations **(****Figures 15A à 15C****),** les ADN génomiques ont été extraits et les ADN VIH-1 totaux ont été quantifiés par qPCR dans les différentes fractions. Les résultats obtenus chez les deux patients montrent un fort enrichissement en ADN VIH-1 dans les fractions TCD4 exprimant le marqueur CD32 en comparaison avec les TCD4 n'exprimant pas ce marqueur **(****Figure 16****).**

L'ensemble de ces résultats *in vitro* et *ex vivo* ont ainsi permis de valider le CD32 comme marqueur spécifique des cellules infectées par le VIH-1. L'identification du CD32 permet d'établir de nouvelles stratégies visant à cibler directement des cellules infectées par le VIH-1 et de permettre la purge du réservoir viral et la guérison du SIDA.

### Exemple 2

Les sous-populations cellulaires isolées à partir du sang de patients VIH-1 ont été mise en culture en absence ou en présence d'agents activateurs tels que PHA ou des billes anti-CD3, anti-CD28 et anti-CD2 (Miltenyi) en présence d'IL2 (50 UI/mL). Les fractions T CD4⁺ et T CD4⁺ CD32⁻ ont été cultivées en plaque de 24 puits à la concentration de 10⁶ cellules par mL de milieu complet et les surnageant récupérés tous les 2 jours pour test ELISA p24.

Les résultats sont présentés dans le tableau suivant et à la **figure 17** :

| Patient | Unités infectieuses par million (IUPM) de cellules CD4 (95% IC) | IUPM de cellules CD4 CD32a+ (95% IC) |
|---|---|---|
| 27 | 2,2 (0,51 à 9,44) | 4977 (533 à 46400) |
| 489 | 5,5 (1,33 à 23,01) | 16422 (1841 à 146000) |
| 566 | 2,2 (0,51 à 9,44) | 2326 (249 à 21700) |
| 771 | 2,2 (0,51 à 9,44) | 2158 (231 à 20100) |

Cette première expérience a pour but de mettre en évidence que la production de nouvelles particules virales à partir des lymphocytes T CD4+ totaux contenant la fraction CD32+ est plus faible que celle de cellules T CD4+ CD32+, ce qui montre que les réservoirs viraux sont des cellules CD32a+. Les résultats obtenus montrent un enrichissement de 3000x du nombre d'IUPM dans les cellules T CD4+ CD32a+ par rapport aux lymphocytes T CD4+ totaux.

Dans une seconde expérience, des lymphocytes T CD4+ isolés à partir du sang de 3 patients ont été activés polyclonalement (anti-CD3/antiCD28 plus IL2) *in vitro* comme contrôle de production virale. En parallèle et pour ces mêmes patients, des lymphocytes T CD4+ déplétées en cellules exprimant le marqueur CD32a ont également été isolées puis activées dans les mêmes conditions in vitro.

Les fractions T CD4+ CD32low et CD32+ ont été cultivées en plaque de 96 puits à fond rond avant que les surnageants de culture ne soient prélevés tous les 3 jours pour être ajouter sur 2000 cellules MT4C5 pour un test d'amplification virale par ELISA p24 ultrasensible SIMOA. Cette seconde expérience vise à démontrer que le virus dont la production a été induite par activation des fractions T CD4+ CD32+ et T CD4+ CD32low est capable d'établir une infection productive en co-culture.

Cette expérience a pour but de mettre en évidence qu'il est possible d'induire la production de nouvelles particules virales à partir des lymphocytes T CD4+ totaux contenant la fraction CD32+ à l'inverse de cellules T CD4+ déplétée en cellules CD32+.

### Les résultats sont présentés à la figure 18.

Il résulte de la comparaison des cinétiques de réplication virale entre les cellules T CD4+ (ronds noirs) totales et les cellules T CD4+ CD32a- (ronds gris), que la déplétion des cellules T CD4+ CD32a+ entraine un retard considérable dans la réplication virale. Ces résultats confirment que les cellules T CD4+ CD32a+ contribuent de façon significative au réservoir total des cellules infectées et compétentes pour la réplication virale.

### Exemple 3

Dans cet exemple, les inventeurs ont tenté de comprendre comment les populations de cellules T CD4 issues de patents, et déplétées en CD32, peuvent toujours réactiver le virus VIH après activation.

D'autres marqueurs décrits précédemment ont été testés, et ont identifié d'autres réservoirs que ceux exprimant le CD32.

Notamment les inventeurs ont identifié que des populations de cellules T CD4 CD89+ constituaient des réservoirs viraux. Le même protocole que celui de l'exemple 1 a été utilisé.

Chez 4 patients, les marquages à l'aide d'anticorps spécifiques ont permis de mettre en évidence l'expression de CD89 à la surface des lymphocytes T CD4+. Pour démontrer que l'expression du marqueur CD89 à la surface de ces cellules était liée à l'infection de celles-ci, les lymphocytes T CD4+ totaux mais aussi les cellules T CD4+ CD32a- CD89-et T CD4+ CD32a- CD89+ ont été isolées à partir de ces mêmes patients comme décrit précédemment (stratégie de fenêtrage pour un patient représentatif). Les résultats sont présentés aux **Figures 19** **A à 19F.**

Suivant le même protocole qu'à l'exemple 1 la quantification des ADN viraux présents dans chacune de ces fractions a été réalisé par qPCR ADN VIH-1 **(****Figure 20****).** Chez les patients testés, le marqueur CD89 identifie bien un réservoir de cellules infectées (médiane approximative de 0,1 copies d'ADN VIH-1 par cellule T CD4+ CD32a- CD89+).

### Exemple 4

Pour poursuivre l'étude, les inventeurs ont enfin testé si les différents candidats marqueurs étaient coexprimés sur les cellules identifiées comme étant des réservoirs viraux.

Comme le montre la **Figure 22****,** 6 marqueurs, dont le marqueur CD89 sont exprimés à la surface des cellules T CD4 qui n'expriment pas de CD32, ces marqueurs représentant de 17 à 30 % des cellules infectés par le VIH et donc susceptibles de le réactiver.

Afin d'identifier si le marqueur CD89 constitue un autre réservoir que celui identifié pour CD32, les inventeurs ont testé sur des populations cellules de patients la coexpression de CD32 et de CD89.

### Les résultats sont présentés à la Figure 23.

Ces résultats montrent que les deux marqueurs CD32 et CD89 sont mutuellement exclusifs, et identifient chacun un réservoir distinct.

### Exemple 5

Les anticorps de la présente invention, dirigés contre CD32 peuvent être produits par diverses techniques connues de l'homme du métier, notamment celles décrites ci- après.

Des souris Balb/c sont immunisées avec la protéine humaine entière CD32 ou le fragment extracellulaire fusionné au domaine Fc des immunoglobulines humaines. Les souris sont injectées par administration sous-cutanée avec 10µg de la protéine ou du fragment au jour 0, jour 14 et jour 28 en présence d'adjuvant complet de Freund (première injection) ou adjuvant incomplet (deuxième et troisième injections). Les splénocytes de la souris sont fusionnés avec des cellules de myélomes murins (PX63.Ag8.653; ATCC, Rockville, MD) selon le protocole précédemment décrit (Salhi et al. Biochem. J. 2004). Les cellules sont cultivées en plaques de culture (10⁵ cellules par puits) dans un milieu HAT permettant la sélection des hybridomes. Après 12 jours, les surnageants sont récupérés et testés pour leur liaison à CD32 par ELISA. Les cellules sont alors soumises à une étape de sous-clonage par dilution limite, les clones positifs sont ensuite soumis à un deuxième cycle de sous-clonage par dilution limite afin d'isoler après ELISA les clones purifiés présentant l'affinité la plus élevée. Ces clones sont ensuite cultivés à plus grande échelle pour produire les anticorps *in vitro.* Les surnageants sont ensuite purifiés sur colonne de chromatographie d'affinité protéine G.

La technique du phage display utilisant des vecteurs modifiés tels que décrits dans le document WO 2007/074496 ou la sélection sur phage display suivie de celle de Biopanning (Krebber et al, (1997) ; WO 2006/117699) est également une autre alternative à l'obtention d'anticorps de haute affinité dirigés contre CD32.

Le séquençage des hybridomes sélectionnés ou la séquence obtenue déjà connue dans les phages sélectionnés permettent ensuite de cloner les régions variables ou plus particulièrement les CDRs responsables de la liaison spécifique à l'épitope dans un plasmide permettant, après transfection dans des cellules productrices comme des CHO, la production et l'obtention d'anticorps chimériques, humanisés ou humains.

Des anticorps anti-CD3 sont obtenus en procédant à travers les mêmes étapes. La création des anticorps humanisés ou humains monospécifiques anti-CD32 ou bispécifiques anti-CD32/CD3 consiste ensuite à utiliser les séquences obtenues précédemment (hybridomes ayant la meilleure affinité pour l'antigène correspondant (CD32 et CD3 respectivement) ou bactériophages). Dans le cas où les anticorps proviennent d'hybridomes, les CDR sont modifiés par mutagenèse de manière à optimiser les acides aminés importants pour la reconnaissance de l'antigène et les acides aminés charpentes permettant un bon repliement des CDR. L'étape d'humanisation consiste à comparer les séquences issues de l'hybridome murin avec une banque de données de séquences d'anticorps humains « Kabat data base ». Les acides aminés potentiellement immunogènes du fait de leur nature murine sont ensuite modifiés. Après l'humanisation, si les anticorps avaient été obtenus par hybridomes, ou dès obtention de la séquence dans le cas de la technique du phage display, les séquences codantes pour les régions variables lourdes (VH) et légères (VL) humanisées/humaines dirigées contre les deux antigènes (CD32 et CD3) sont clonées en fusion dans un vecteur d'expression eucaryote pour permettre la production en cellules CHO.

L'invention n'est pas limitée aux modes de réalisation présentés et d'autres modes de réalisation apparaîtront clairement à l'homme du métier.

## Revendications

1. Anticorps bispécifique reconnaissant d'une part au moins un épitope du marqueur CD89 et au moins un marqueur caractéristique des cellules lymphocytaires T.

2. Anticorps selon la revendication 1, ou marqueur caractéristique des cellules lymphocytaires T est l'antigène CD3.

3. Anticorps pour son utilisation selon la revendication 1 ou 2, où ledit anticorps est un anticorps monoclonal humanisé.

4. Anticorps selon l'une quelconque des revendications 1 à 3, ledit anticorps étant couplé à des agents actifs tels que des toxines, des radioisotopes, des agents biologiques, des drogues ou encore des enzymes.

5. Composition comprenant
- un anticorps bispécifique tel que défini dans l'une quelconque des revendications 1 à 4, et
- une combinaison d'agents anti-retroviraux.

6. Anticorps selon l'une quelconque des revendications 1 à 4, pour son utilisation pour le traitement d'une infection par un lentivirus.

7. Anticorps pour son utilisation selon la revendication 6, où ladite infection par un lentivirus est une infection liée au virus de l'immunodéficience humaine VIH, de l'immunodéficience simienne VIS ou de l'immunodéficience féline VIF.

8. Composition selon la revendication 5, pour son utilisation pour le traitement d'une infection par un lentivirus.

9. Composition pour son utilisation selon la revendication 8, où l'anticorps bispécifique et la combinaison d'agents antirétroviraux sont utilisés simultanément, séparément ou de manière étalée dans le temps.
